(19) Europäisches Patentamt
European Patent Office
Office européen des brevets

(11) EP 3 736 333 A1

(12) EUROPEAN PATENT APPLICATION

(43) Date of publication:
11.11.2020 Bulletin 2020/46

(21) Application number: 20178501.1

(22) Date of filing: 30.04.2013

(51) Int Cl.:
*C12N 15/113* *(2010.01)* *A61P 35/00* *(2006.01)*
*A61K 31/713* *(2006.01)*

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR

(30) Priority: 02.05.2012 US 201261641588 P

(62) Document number(s) of the earlier application(s) in accordance with Art. 76 EPC:
17187348.2 / 3 272 868
13721578.6 / 2 844 757

(71) Applicant: Arrowhead Pharmaceuticals, Inc.
Pasadena, CA 91105 (US)

(72) Inventors:
- BETTENCOURT, Brian
Cambridge, MA 02139 (US)
- MILSTEIN, Stuart
Cambridge, MA 02142 (US)
- TOUDJARSKA, Ivanka
Cambridge, MA 02142 (US)
- MCDONALD, Earl
Cambridge, MA 02139 (US)
- SCHLABACH, Michael Jr.
Cambridge, MA 02139 (US)
- STEIGMEIER, Frank P.
Cambridge, MA 02139 (US)
- WARMUTH, Markus
Cambridge, MA 02139 (US)
- GAMPA, Kalyani
Cambridge, MA 02139 (US)
- HUESKEN, Dieter
4006 Basel (CH)
- STUMP, Mark
Danvers, MA 01923 (US)
- WEILER, Jan
Cambridge, MA 02139 (US)
- JAGANI, Zainab
Cambridge, MA 02139 (US)

(74) Representative: Arnold & Siedsma
Bezuidenhoutseweg 57
2594 AC The Hague (NL)

Remarks:
The complete document including Reference Tables and the Sequence Listing can be downloaded from the EPO website

# (54) ORGANIC COMPOSITIONS TO TREAT KRAS-RELATED DISEASES

(57) The present disclosure relates to RNAi agents useful in methods of treating KRAS-related diseases such as a proliferative disease, including without limitation a solid or liquid cancer, adenocarcinoma, colorectal cancer, advanced and/or metastatic colorectal cancer, colon cancer, lung, non-small cell lung cancer and lung adenocarcinoma, acute myelogenous lung, bladder, brain, breast, cervical, endometrial, gastric, head and neck, kidney, leukemia, myelodysplastic syndrome, myeloid leukemia, liver, melanoma, ovarian, pancreatic, prostate, testicular, thyroid cancers, and cardio-facio-cutaneous (CFC) syndrome and Noonan syndrome, and similar and related diseases, using a therapeutically effective amount of a RNAi agent to KRAS.

EP 3 736 333 A1

## Description

## BACKGROUND OF THE INVENTION

KRAS is a member of the RAS subfamily of small GTPases. It is involved in cellular signal transduction, growth, differentiation, survival and other cellular functions. Over-expression or hyper-activity of KRAS, along with RAS family members HRAS and NRAS, have been associated with a number of diseases, including various cancers. RAS mutations are found in approximately one-third of all human malignancies. Bos et al. 1989. Cancer Res. 49: 4682-4689. KRAS accounts for most of the RAS mutations found in the majority of human malignancies, including 90% of RAS mutations in lung adenocarcinomas. Forbes et al. 2006. Br. J. Cancer 94: 318-322.

Mutations, hyper-activity and/or over-expression of KRAS in particular has been associated with cancer, including adenocarcinoma, colorectal cancer (including advanced and metastatic colorectal cancer), colon cancer, lung (including non-small cell lung cancer and lung adenocarcinoma), acute myelogenous lung, bladder, brain, breast, cervical, endometrial, gastric, head and neck, kidney, leukemia, myelodysplastic syndrome, myeloid leukemia, liver, melanoma, ovarian, pancreatic, prostate, testicular, thyroid cancers, and cardio-facio-cutaneous (CFC) syndrome and Noonan syndrome, and similar and related diseases.

There exists the need for treatments related to KRAS-related diseases.

## BRIEF SUMMARY OF THE INVENTION

The present disclosure encompasses RNAi (RNA interference) agents to KRAS, which are useful in the treatment of KRAS-related diseases, such as cancer, cardio-facio-cutaneous (CFC) syndrome and Noonan syndrome, and similar and related diseases.

The present disclosure also encompasses a method of treating a human subject having a pathological state mediated at least in part by KRAS expression, the method comprising the step of administering to the subject a therapeutically effective amount of a RNAi agent KRAS.

The method also optionally further comprises the step of administering a second agent. In some embodiments, this second agent is another RNAi agent to KRAS. In other embodiments, the second agent is another treatment, such as one directed to another target, which is also hyper-active, mutated and/or over-expressed in the pathological state.

The present disclosure provides specific RNAi agents and methods that are useful in reducing KRAS levels in a subject, e.g., a mammal, such as a human. The present disclosure specifically provides double-stranded RNAi agents comprising at least 15 to at least 19 or more contiguous nucleotides of KRAS. In particular, the present disclosure provides agents comprising sequences of 15 or more contiguous nucleotides differing by 0, 1, 2 or 3 from those of the RNAi agents provided, e.g., in Tables 1 to 6. The RNAi agents particularly can in one embodiment comprise less than 30 nucleotides per strand, e.g., such as 17 - 23 nucleotides, 15 - 19, 18-22 nucleotides, and/or 19-21 nucleotides, and/or such as those provided, e.g., in Tables 1 to 6, and modified and unmodified variants thereof (e.g., wherein the sense and/or anti-sense or first and/or second strand are modified or unmodified). The present disclosure also provides agents comprising a sense strand and an anti-sense strand, wherein the sense and/or the anti-sense strand comprise sequences of 19 or more contiguous nucleotides differing by 0, 1, 2 or 3 from those of the RNAi agents provided, e.g., in Tables 1 to 6, and modified or unmodified variants thereof. The sense and anti-sense strand can be contiguous, or covalently bounds, e.g., via a loop or linker. The RNAi agents particularly can in one embodiment comprise less than 30 nucleotides per strand, e.g., such as 17 to 23 nucleotides, 18 to 22 nucleotides, and/or 19 to 21 nucleotides, and/or sequences such as those provided, e.g., in Tables 1 to 6.

The double-stranded RNAi agents can have 0, 1 or 2 blunt ends, and/or overhangs of 1, 2, 3 or 4 nucleotides (i.e., 1 to 4 nt) from one or both 3' and/or 5' ends. The double-stranded RNAi agents can also optionally comprise one or two 3' caps and/or one or more modified nucleotides. Modified variants of sequences as provided herein include those that are otherwise identical but contain substitutions of a naturally-occurring nucleotide for a corresponding modified nucleotide.

Furthermore, the RNAi agent can either contain only naturally-occurring nucleotide subunits (e.g., ribonucleotides), or one or more modifications to the sugar, phosphate or base of one or more of the replacement nucleotide subunits, whether they comprise ribonucleotide subunits or deoxyribonucleotide subunits or other related modified variants. In one embodiment, modified variants of the disclosed RNAi agents have a thymidine (as RNA, or, preferably, DNA) replacing a uridine, or have an inosine base. In some embodiments, the modified variants of the disclosed RNAi agents can have a nick in the passenger strand, mismatches between the guide and passenger strand, DNA replacing the RNA of a portion of both the guide and passenger strand (e.g., the seed region), and/or a shortened passenger strand (e.g., 15, 16, 17 or 18 nt). Once a functional guide strand is identified, modifications and variants of the RNAi agent can be readily made. Any combination of modifications which is not mutually exclusive can be (e.g., the combination of base modifications with shortened passenger strand; or nicked passenger strand and base modifications; or DNA replacing part or all of the seed region and base modifications in the remaining RNA; etc.).

In one embodiment, modified variants of the disclosed RNAi agents include RNAi agents with the same sequence (e.g., the same sequence of bases) as disclosed in Tables 1 to 6, but with one or more modifications to one or more of the sugar or phosphate of one or more of the nucleotide subunits. In one embodiment, the modifications improve efficacy, stability (e.g., against nucleases in, for example, blood serum or intestinal fluid), and/or reduce immunogenicity of the RNAi agent. One aspect of the present disclosure relates to a double-stranded oligonucleotide comprising at least one non-natural nucleobase. In certain embodiments, the non-natural nucleobase is difluorotolyl, nitroindolyl, nitropyrrolyl, or nitroimidazolyl. In a particular embodiment, the non-natural nucleobase is difluorotolyl. In certain embodiments, only one of the two oligonucleotide strands contains a non-natural nucleobase. In certain embodiments, both of the oligonucleotide strands contain a non-natural nucleobase.

The RNAi agent(s) can optionally be attached to a ligand selected to improve one or more characteristic, such as, e.g., stability, distribution and/or cellular uptake of the agent, e.g., cholesterol or a derivative thereof. The RNAi agent(s) can be isolated or be part of a pharmaceutical composition used for the methods described herein. Particularly, the pharmaceutical composition can be formulated for delivery to specific tissues (e.g., those afflicted with a KRAS-related disease) or formulated for parenteral administration. The pharmaceutical composition can optionally comprise two or more RNAi agents, each one directed to the same or a different segment of the KRAS mRNA. Optionally, the pharmaceutical composition can further comprise or be used in conjunction with any known treatment for any KRAS-related disease.

The present disclosure further provides methods for reducing the level of KRAS mRNA in a cell, particularly in the case of a disease characterized by over-expression or hyper-activity of KRAS. Cells comprising an alteration such as a mutation, over-expression and/or hyperactivity of KRAS are termed "KRAS-defective" cells. Such methods comprise the step of administering one or more of the RNAi agents of the present disclosure to a cell, as further described below. The present methods utilize the cellular mechanisms involved in RNA interference to selectively degrade the target RNA in a cell and are comprised of the step of contacting a cell with one or more of the RNAi agents of the present disclosure.

The present disclosure further provides methods for reducing the level of KRAS mRNA in a cell, particularly in the case of a disease characterized by over-expression, mutation or hyper-activity of KRAS. Such methods comprise the step of administering one or more of the RNAi agents of the present disclosure to a cell, as further described below. The present methods utilize the cellular mechanisms involved in RNA interference to selectively degrade the target RNA in a cell and are comprised of the step of contacting a cell with one or more of the RNAi agents of the present disclosure.

The present disclosure also encompasses a method of treating a human subject having a pathological state mediated at least in part by KRAS expression, the method comprising the step of administering to the subject a therapeutically effective amount of a RNAi agent targeting KRAS. Additional methods involve preventing, treating, modulating and/or ameliorating a pathological state wherein disease progression (e.g., tumor growth) requires KRAS, although KRAS is not amplified, over-expressed or mis-localized. Such methods comprise the step of administering one or more of the RNAi agents of the present disclosure to a subject, as further described below. The present methods utilize the cellular mechanisms involved in RNA interference to selectively degrade the target RNA in a cell comprise the step of contacting a cell with one or more of the RNAi agents of the present disclosure. Such methods can be performed directly on a cell or can be performed on a mammalian subject by administering to a subject one or more of the RNAi agents/pharmaceutical compositions of the present disclosure. Reduction of target KRAS RNA in a cell results in a reduction in the amount of encoded KRAS protein produced. In an organism, this can result in restoration of balance in a pathway involving KRAS, and/or prevention of KRAS accumulation, and/or a reduction in KRAS activity and/or expression, and/or prevention of KRAS -mediated activation of other genes, and/or amelioration, treatment and/or prevention of a KRAS-related disease. In one embodiment, a reduction in KRAS expression, level or activity can limit tumor growth.

The methods and compositions of the present disclosure, e.g., the methods and KRAS RNAi agent compositions, can be used in any appropriate dosage and/or formulation described herein or known in the art, as well as with any suitable route of administration described herein or known in the art.

The details of one or more embodiments of the present disclosure are set forth in the accompanying drawings and the description below. Elements of the various embodiments (e.g., sequences, modifications, endcaps, combinations of RNAi agents, combination therapy involving a KRAS RNAi agent and another agent, etc.) which are not mutually-exclusive can be combined with each other as described herein and as known or developed in the art. For example, any RNAi agent sequence disclosed herein can be combined with any set of modifications or endcaps disclosed herein. Any combination of modifications, 5' end caps, and/or 3' end caps can be used with any RNAi agent sequence disclosed herein. Any RNAi agent disclosed herein (with any combination of modifications or endcaps or without either modifications or endcaps) can be combined with any other RNAi agent or other treatment composition or method disclosed herein.

Other features, objects, and advantages of the present disclosure will be apparent from this description, the drawings, and from the claims.

BRIEF DESCRIPTION OF THE FIGURE

Figure 1 illustrates various modified nucleotides: U002, U003, U004, U005, C004, C005, A004, A005, G005, and G004, which can be used in the RNAi agents disclosed herein. U002 indicates a 2'- deoxy-thymidine which is DNA. U003 indicates 2'-deoxy uridine. U004 indicates a nucleotide with a Uridine ("U") base with a 2'-O-methyl modification. U005 indicates a U base with a 2'-O-methoxyethyl (MOE) modification. C004 indicates a Cysteine ("C") base with a 2'-O-methyl modification. C005 indicates a C base with 2'-O-methoxyethyl modification. A004 indicates an Adenosine ("A") base with a 2'-O-methyl modification. A005 indicates an A base with 2'-O-methoxyethyl modification. G005 indicates a Guanosine ("G") base with a 2'O-methyl modification. G004 indicates a G base with a 2'O-methyl modification.

## DETAILED DESCRIPTION OF THE INVENTION

The present disclosure encompasses RNAi agents to KRAS, for targeting and inhibition of the KRAS gene, which are useful in treatment of KRAS-related diseases (e.g., diseases associated with mutations in and/or altered expression, level and/or activity of KRAS, and/or diseases which require KRAS, and/or diseases treatable by modulating the expression, level and/or activity of KRAS), such as a proliferative disease, including without limitation a solid or liquid cancer, adenocarcinoma, colorectal cancer, advanced and/or metastatic colorectal cancer, colon cancer, lung, non-small cell lung cancer and lung adenocarcinoma, acute myelogenous lung, bladder, brain, breast, cervical, endometrial, gastric, head and neck, kidney, leukemia, myelodysplastic syndrome, myeloid leukemia, liver or hepatic, melanoma, ovarian, pancreatic, prostate, testicular, thyroid cancers, and cardio-facio-cutaneous (CFC) syndrome and Noonan syndrome, and similar and related diseases. The present disclosure also provides methods of treating a human subject having a pathological state mediated at least in part by KRAS expression or over-expression or hyper-activity, the method comprising the step of administering to the subject a therapeutically effective amount of a RNAi agent to KRAS.

**Various embodiments of the present disclosure include the following.**

In one embodiment, the present disclosure relates to a composition comprising a RNAi agent comprising a sense strand and an antisense strand, wherein the antisense strand comprises at least 15 contiguous nucleotides differing by 0, 1, 2, or 3 nucleotides from the antisense strand of a RNAi agent specific to KRAS (or any set of overlapping RNAi agents specific to KRAS) provided, e.g., in any of Tables 1 to 6. In another embodiment, the present disclosure relates to a composition comprising a RNAi agent comprising a sense and an anti-sense strand, wherein the sense strand comprises at least 15 contiguous nucleotides differing by 0, 1, 2, or 3 nucleotides from the sense strand of a RNAi agent from any sequence provided herein. In another embodiment, the present disclosure relates to a composition comprising a RNAi agent comprising a first strand and a second strand, wherein the first strand comprises at least 15 contiguous nucleotides differing by 0, 1, 2, or 3 nucleotides from the sequence of the first strand, and the second strand comprises at least 15 contiguous nucleotides differing by 0, 1, 2, or 3 nucleotides from the sequence of the second strand of any RNAi agent provided herein.

Particular duplexes include the example unmodified (e.g., "generic") and example modified sequences listed in Table 1. In addition to the described example modifications, other modified variants are contemplated and can be made using the nucleotide sequences provided. In various embodiments, the first and/or second strand are modified or unmodified or a combination of one modified and one unmodified.

**RNAi Agents comprising an antisense and a sense strand described herein**

In one embodiment, the present disclosure relates to a composition comprising a RNAi agent comprising an antisense strand, wherein the antisense strand comprises at least 15 contiguous nucleotides differing by 0, 1, 2, or 3 nucleotides from the antisense strand of a RNAi agent to KRAS selected from any sequence (or overlapping set of sequences) provided in a table herein (e.g., any of Tables 1 to 6). In one embodiment, the present disclosure relates to a composition comprising a RNAi agent comprising a sense strand and an antisense strand, wherein the antisense strand comprises at least 15 contiguous nucleotides differing by 0 nucleotides from the antisense strand of a RNAi agent to KRAS selected from any sequence (or overlapping set of sequences) provided in a table here (e.g., any of Tables 1 to 6). In another embodiment, the present disclosure relates to a composition comprising a RNAi agent comprising a sense and an anti-sense strand, wherein the sequence of the anti-sense strand comprises the sequence of the anti-sense strand of a RNAi agent from any sequence provided herein. In another embodiment, the present disclosure relates to a composition comprising a RNAi agent comprising a first strand and a second strand, wherein the sequence of the first strand is the sequence of the first strand of any RNAi agent provided herein. Particular duplexes include those specific duplexes provided above and as listed in any one or more of Table 1. Additional modified sequences (e.g., sequences comprising one or more modified base) of each of the compositions above are also contemplated as part of the present disclosure.

**TABLE 1. SEQ ID NOs. for RNAi Agents to KRAS**

Provided in Table 1 are the nickname of various KRAS RNAi duplexes; the SEQ ID NOs. of unmodified (e.g., "generic") sense and anti-sense sequences; and the SEQ ID NOs. for example sense and anti-sense strands.

Table 1, below, provides the SEQ ID NOs for the unmodified and an example modified sequence of the sense and an anti-sense strands of various RNAi agents to KRAS. The base composition of each sequence represented by a SEQ ID NO is provided in more detail in Tables 2 and 3. Table 4 shows overlapping groups of these KRAS RNAi agents. Tables 5 and 6 show efficacy of various KRAS RNAi agents.

**Table 1. SEQ ID NOs for unmodified and example modified sequences of a first and a second strand (e.g., sense and anti-sense strand) for RNAi agents to KRAS**

| | UNMODIFIED SEQUENCES | | EXAMPLE MODIFIED | SEQUENCES |
|---|---|---|---|---|
| Duplex | SENSE SEQ ID NO: SEQ ID NO: | ANTI-SENSE SEQ ID NO: | SENSE SEQ ID NO: | ANTI-SENSE SEQ ID NO: |
| AD-35523.3 | 1 | 428 | 855 | 1282 |
| AD-35529.1 | 2 | 429 | 856 | 1283 |
| AD-35535.4 | 3 | 430 | 857 | 1284 |
| AD-35541.4 | 4 | 431 | 858 | 1285 |
| AD-35547.4 | 5 | 432 | 859 | 1286 |
| AD-35553.4 | 6 | 433 | 860 | 1287 |
| AD-35559.4 | 7 | 434 | 861 | 1288 |
| AD-35565.4 | 8 | 435 | 862 | 1289 |
| AD-35524.4 | 9 | 436 | 863 | 1290 |
| AD-35530.4 | 10 | 437 | 864 | 1291 |
| AD-35536.4 | 11 | 438 | 865 | 1292 |
| AD-35542.4 | 12 | 439 | 866 | 1293 |
| AD-35548.4 | 13 | 440 | 867 | 1294 |
| AD-35554.4 | 14 | 441 | 868 | 1295 |
| AD-35560.2 | 15 | 442 | 869 | 1296 |
| AD-35566.4 | 16 | 443 | 870 | 1297 |
| AD-35525.2 | 17 | 444 | 871 | 1298 |
| AD-35531.4 | 18 | 445 | 872 | 1299 |
| AD-35537.4 | 19 | 446 | 873 | 1300 |
| AD-35543.2 | 20 | 447 | 874 | 1301 |
| AD-35549.4 | 21 | 448 | 875 | 1302 |
| AD-35555.4 | 22 | 449 | 876 | 1303 |
| AD-35561.4 | 23 | 450 | 877 | 1304 |
| AD-35567.4 | 24 | 451 | 878 | 1305 |
| AD-35526.4 | 25 | 452 | 879 | 1306 |
| AD-35532.4 | 26 | 453 | 880 | 1307 |
| AD-35538.4 | 27 | 454 | 881 | 1308 |

(continued)

| | UNMODIFIED SEQUENCES | | EXAMPLE MODIFIED | SEQUENCES |
|---|---|---|---|---|
| Duplex | SENSE SEQ ID NO: SEQ ID NO: | ANTI-SENSE SEQ ID NO: | SENSE SEQ ID NO: | ANTI-SENSE SEQ ID NO: |
| AD-35544.4 | 28 | 455 | 882 | 1309 |
| AD-35550.4 | 29 | 456 | 883 | 1310 |
| AD-35556.4 | 30 | 457 | 884 | 1311 |
| AD-35562.4 | 31 | 458 | 885 | 1312 |
| AD-35568.4 | 32 | 459 | 886 | 1313 |
| AD-35527.4 | 33 | 460 | 887 | 1314 |
| AD-35533.4 | 34 | 461 | 888 | 1315 |
| AD-35539.4 | 35 | 462 | 889 | 1316 |
| AD-35545.4 | 36 | 463 | 890 | 1317 |
| AD-35551.4 | 37 | 464 | 891 | 1318 |
| AD-35563.4 | 38 | 465 | 892 | 1319 |
| AD-35528.4 | 39 | 466 | 893 | 1320 |
| AD-35540.4 | 40 | 467 | 894 | 1321 |
| AD-35546.4 | 41 | 468 | 895 | 1322 |
| AD-35552.4 | 42 | 469 | 896 | 1323 |
| AD-35558.4 | 43 | 470 | 897 | 1324 |
| AD-35564.4 | 44 | 471 | 898 | 1325 |
| AD-35570.4 | 45 | 472 | 899 | 1326 |
| AD-35571.4 | 46 | 473 | 900 | 1327 |
| AD-35577.4 | 47 | 474 | 901 | 1328 |
| AD-35583.4 | 48 | 475 | 902 | 1329 |
| AD-35589.4 | 49 | 476 | 903 | 1330 |
| AD-35595.4 | 50 | 477 | 904 | 1331 |
| AD-35601.4 | 51 | 478 | 905 | 1332 |
| AD-35607.4 | 52 | 479 | 906 | 1333 |
| AD-35613.4 | 53 | 480 | 907 | 1334 |
| AD-35572.4 | 54 | 481 | 908 | 1335 |
| AD-35578.4 | 55 | 482 | 909 | 1336 |
| AD-35584.4 | 56 | 483 | 910 | 1337 |
| AD-35590.4 | 57 | 484 | 911 | 1338 |
| AD-35596.4 | 58 | 485 | 912 | 1339 |
| AD-35602.4 | 59 | 486 | 913 | 1340 |
| AD-35608.4 | 60 | 487 | 914 | 1341 |
| AD-35614.4 | 61 | 488 | 915 | 1342 |
| AD-35573.4 | 62 | 489 | 916 | 1343 |

(continued)

| | UNMODIFIED SEQUENCES | | EXAMPLE MODIFIED | SEQUENCES |
|---|---|---|---|---|
| Duplex | SENSE SEQ ID NO: SEQ ID NO: | ANTI-SENSE SEQ ID NO: | SENSE SEQ ID NO: | ANTI-SENSE SEQ ID NO: |
| AD-35579.4 | 63 | 490 | 917 | 1344 |
| AD-35585.4 | 64 | 491 | 918 | 1345 |
| AD-35591.4 | 65 | 492 | 919 | 1346 |
| AD-35597.4 | 66 | 493 | 920 | 1347 |
| AD-35603.4 | 67 | 494 | 921 | 1348 |
| AD-35609.4 | 68 | 495 | 922 | 1349 |
| AD-35615.4 | 69 | 496 | 923 | 1350 |
| AD-35574.4 | 70 | 497 | 924 | 1351 |
| AD-35580.1 | 71 | 498 | 925 | 1352 |
| AD-35586.4 | 72 | 499 | 926 | 1353 |
| AD-35592.4 | 73 | 500 | 927 | 1354 |
| AD-35598.4 | 74 | 501 | 928 | 1355 |
| AD-35604.4 | 75 | 502 | 929 | 1356 |
| AD-35610.4 | 76 | 503 | 930 | 1357 |
| AD-35616.4 | 77 | 504 | 931 | 1358 |
| AD-35575.4 | 78 | 505 | 932 | 1359 |
| AD-35581.4 | 79 | 506 | 933 | 1360 |
| AD-35587.4 | 80 | 507 | 934 | 1361 |
| AD-35593.4 | 81 | 508 | 935 | 1362 |
| AD-35599.4 | 82 | 509 | 936 | 1363 |
| AD-35605.4 | 83 | 510 | 937 | 1364 |
| AD-35611.4 | 84 | 511 | 938 | 1365 |
| AD-35617.4 | 85 | 512 | 939 | 1366 |
| AD-35576.4 | 86 | 513 | 940 | 1367 |
| AD-35588.4 | 87 | 514 | 941 | 1368 |
| AD-35667.1 | 88 | 515 | 942 | 1369 |
| AD-35673.1 | 89 | 516 | 943 | 1370 |
| AD-35679.1 | 90 | 517 | 944 | 1371 |
| AD-35685.1 | 91 | 518 | 945 | 1372 |
| AD-35691.1 | 92 | 519 | 946 | 1373 |
| AD-35557.1 | 93 | 520 | 947 | 1374 |
| AD-35618.1 | 94 | 521 | 948 | 1375 |
| AD-35569.1 | 95 | 522 | 949 | 1376 |
| AD-35534.1 | 96 | 523 | 950 | 1377 |
| AD-35582.1 | 97 | 524 | 951 | 1378 |

(continued)

| | UNMODIFIED SEQUENCES | | EXAMPLE MODIFIED | SEQUENCES |
|---|---|---|---|---|
| Duplex | SENSE SEQ ID NO: SEQ ID NO: | ANTI-SENSE SEQ ID NO: | SENSE SEQ ID NO: | ANTI-SENSE SEQ ID NO: |
| AD-35594.1 | 98 | 525 | 952 | 1379 |
| AD-35600.1 | 99 | 526 | 953 | 1380 |
| AD-35606.1 | 100 | 527 | 954 | 1381 |
| AD-35612.1 | 101 | 528 | 955 | 1382 |
| AD-38127.1 | 102 | 529 | 956 | 1383 |
| AD-38133.1 | 103 | 530 | 957 | 1384 |
| AD-38139.1 | 104 | 531 | 958 | 1385 |
| AD-38145.1 | 105 | 532 | 959 | 1386 |
| AD-38151.1 | 106 | 533 | 960 | 1387 |
| AD-38157.1 | 107 | 534 | 961 | 1388 |
| AD-38163.1 | 108 | 535 | 962 | 1389 |
| AD-38169.1 | 109 | 536 | 963 | 1390 |
| AD-38128.1 | 110 | 537 | 964 | 1391 |
| AD-38134.1 | 111 | 538 | 965 | 1392 |
| AD-38140.1 | 112 | 539 | 966 | 1393 |
| AD-38146.1 | 113 | 540 | 967 | 1394 |
| AD-38152.1 | 114 | 541 | 968 | 1395 |
| AD-38158.1 | 115 | 542 | 969 | 1396 |
| AD-38164.1 | 116 | 543 | 970 | 1397 |
| AD-38170.1 | 117 | 544 | 971 | 1398 |
| AD-38129.1 | 118 | 545 | 972 | 1399 |
| AD-38135.1 | 119 | 546 | 973 | 1400 |
| AD-38141.1 | 120 | 547 | 974 | 1401 |
| AD-38147.1 | 121 | 548 | 975 | 1402 |
| AD-38153.1 | 122 | 549 | 976 | 1403 |
| AD-38159.1 | 123 | 550 | 977 | 1404 |
| AD-38165.1 | 124 | 551 | 978 | 1405 |
| AD-38171.1 | 125 | 552 | 979 | 1406 |
| AD-38130.1 | 126 | 553 | 980 | 1407 |
| AD-38136.1 | 127 | 554 | 981 | 1408 |
| AD-38142.1 | 128 | 555 | 982 | 1409 |
| AD-38148.1 | 129 | 556 | 983 | 1410 |
| AD-38154.1 | 130 | 557 | 984 | 1411 |
| AD-38160.1 | 131 | 558 | 985 | 1412 |
| AD-38166.1 | 132 | 559 | 986 | 1413 |

(continued)

| | UNMODIFIED SEQUENCES | | EXAMPLE MODIFIED | SEQUENCES |
|---|---|---|---|---|
| Duplex | SENSE SEQ ID NO: SEQ ID NO: | ANTI-SENSE SEQ ID NO: | SENSE SEQ ID NO: | ANTI-SENSE SEQ ID NO: |
| AD-38172.1 | 133 | 560 | 987 | 1414 |
| AD-38131.1 | 134 | 561 | 988 | 1415 |
| AD-38137.1 | 135 | 562 | 989 | 1416 |
| AD-38143.1 | 136 | 563 | 990 | 1417 |
| AD-38149.1 | 137 | 564 | 991 | 1418 |
| AD-38155.1 | 138 | 565 | 992 | 1419 |
| AD-38161.1 | 139 | 566 | 993 | 1420 |
| AD-38167.1 | 140 | 567 | 994 | 1421 |
| AD-38173.1 | 141 | 568 | 995 | 1422 |
| AD-38132.1 | 142 | 569 | 996 | 1423 |
| AD-38138.1 | 143 | 570 | 997 | 1424 |
| AD-38144.1 | 144 | 571 | 998 | 1425 |
| AD-38150.1 | 145 | 572 | 999 | 1426 |
| AD-38156.1 | 146 | 573 | 1000 | 1427 |
| AD-38162.1 | 147 | 574 | 1001 | 1428 |
| AD-38168.1 | 148 | 575 | 1002 | 1429 |
| AD-38174.1 | 149 | 576 | 1003 | 1430 |
| AD-39683.1 | 150 | 577 | 1004 | 1431 |
| AD-39689.1 | 151 | 578 | 1005 | 1432 |
| AD-39695.1 | 152 | 579 | 1006 | 1433 |
| AD-39701.1 | 153 | 580 | 1007 | 1434 |
| AD-39707.1 | 154 | 581 | 1008 | 1435 |
| AD-39713.1 | 155 | 582 | 1009 | 1436 |
| AD-39719.1 | 156 | 583 | 1010 | 1437 |
| AD-39725.1 | 157 | 584 | 1011 | 1438 |
| AD-39684.1 | 158 | 585 | 1012 | 1439 |
| AD-39690.1 | 159 | 586 | 1013 | 1440 |
| AD-39696.1 | 160 | 587 | 1014 | 1441 |
| AD-39702.1 | 161 | 588 | 1015 | 1442 |
| AD-39708.1 | 162 | 589 | 1016 | 1443 |
| AD-39714.1 | 163 | 590 | 1017 | 1444 |
| AD-39720.1 | 164 | 591 | 1018 | 1445 |
| AD-39726.1 | 165 | 592 | 1019 | 1446 |
| AD-39685.1 | 166 | 593 | 1020 | 1447 |
| AD-39691.1 | 167 | 594 | 1021 | 1448 |

(continued)

| | UNMODIFIED SEQUENCES | | EXAMPLE MODIFIED | SEQUENCES |
|---|---|---|---|---|
| Duplex | SENSE SEQ ID NO: SEQ ID NO: | ANTI-SENSE SEQ ID NO: | SENSE SEQ ID NO: | ANTI-SENSE SEQ ID NO: |
| AD-39697.1 | 168 | 595 | 1022 | 1449 |
| AD-39703.1 | 169 | 596 | 1023 | 1450 |
| AD-39709.1 | 170 | 597 | 1024 | 1451 |
| AD-39715.1 | 171 | 598 | 1025 | 1452 |
| AD-39721.1 | 172 | 599 | 1026 | 1453 |
| AD-39727.1 | 173 | 600 | 1027 | 1454 |
| AD-39686.1 | 174 | 601 | 1028 | 1455 |
| AD-39692.1 | 175 | 602 | 1029 | 1456 |
| AD-39698.1 | 176 | 603 | 1030 | 1457 |
| AD-39704.1 | 177 | 604 | 1031 | 1458 |
| AD-39710.1 | 178 | 605 | 1032 | 1459 |
| AD-39716.1 | 179 | 606 | 1033 | 1460 |
| AD-39722.1 | 180 | 607 | 1034 | 1461 |
| AD-39728.1 | 181 | 608 | 1035 | 1462 |
| AD-39687.1 | 182 | 609 | 1036 | 1463 |
| AD-39693.1 | 183 | 610 | 1037 | 1464 |
| AD-39699.1 | 184 | 611 | 1038 | 1465 |
| AD-39705.1 | 185 | 612 | 1039 | 1466 |
| AD-39711.1 | 186 | 613 | 1040 | 1467 |
| AD-39717.1 | 187 | 614 | 1041 | 1468 |
| AD-39723.1 | 188 | 615 | 1042 | 1469 |
| AD-39729.1 | 189 | 616 | 1043 | 1470 |
| AD-39688.1 | 190 | 617 | 1044 | 1471 |
| AD-39694.1 | 191 | 618 | 1045 | 1472 |
| AD-39700.1 | 192 | 619 | 1046 | 1473 |
| AD-39706.1 | 193 | 620 | 1047 | 1474 |
| AD-39712.1 | 194 | 621 | 1048 | 1475 |
| AD-39718.1 | 195 | 622 | 1049 | 1476 |
| AD-39724.1 | 196 | 623 | 1050 | 1477 |
| AD-39730.1 | 197 | 624 | 1051 | 1478 |
| AD-39736.1 | 198 | 625 | 1052 | 1479 |
| AD-39742.1 | 199 | 626 | 1053 | 1480 |
| AD-39748.1 | 200 | 627 | 1054 | 1481 |
| AD-39754.1 | 201 | 628 | 1055 | 1482 |
| AD-39760.1 | 202 | 629 | 1056 | 1483 |

(continued)

| | UNMODIFIED SEQUENCES | | EXAMPLE MODIFIED | SEQUENCES |
|---|---|---|---|---|
| Duplex | SENSE SEQ ID NO: SEQ ID NO: | ANTI-SENSE SEQ ID NO: | SENSE SEQ ID NO: | ANTI-SENSE SEQ ID NO: |
| AD-39766.1 | 203 | 630 | 1057 | 1484 |
| AD-39772.1 | 204 | 631 | 1058 | 1485 |
| AD-39731.1 | 205 | 632 | 1059 | 1486 |
| AD-39737.1 | 206 | 633 | 1060 | 1487 |
| AD-39743.1 | 207 | 634 | 1061 | 1488 |
| AD-39749.1 | 208 | 635 | 1062 | 1489 |
| AD-39755.1 | 209 | 636 | 1063 | 1490 |
| AD-39761.1 | 210 | 637 | 1064 | 1491 |
| AD-39767.1 | 211 | 638 | 1065 | 1492 |
| AD-39773.1 | 212 | 639 | 1066 | 1493 |
| AD-39732.1 | 213 | 640 | 1067 | 1494 |
| AD-39738.1 | 214 | 641 | 1068 | 1495 |
| AD-39744.1 | 215 | 642 | 1069 | 1496 |
| AD-39750.1 | 216 | 643 | 1070 | 1497 |
| AD-39756.1 | 217 | 644 | 1071 | 1498 |
| AD-39762.1 | 218 | 645 | 1072 | 1499 |
| AD-39768.1 | 219 | 646 | 1073 | 1500 |
| AD-39774.1 | 220 | 647 | 1074 | 1501 |
| AD-39733.1 | 221 | 648 | 1075 | 1502 |
| AD-39739.1 | 222 | 649 | 1076 | 1503 |
| AD-39745.1 | 223 | 650 | 1077 | 1504 |
| AD-39751.1 | 224 | 651 | 1078 | 1505 |
| AD-39757.1 | 225 | 652 | 1079 | 1506 |
| AD-39763.1 | 226 | 653 | 1080 | 1507 |
| AD-39769.1 | 227 | 654 | 1081 | 1508 |
| AD-39775.1 | 228 | 655 | 1082 | 1509 |
| AD-39734.1 | 229 | 656 | 1083 | 1510 |
| AD-39740.1 | 230 | 657 | 1084 | 1511 |
| AD-39746.1 | 231 | 658 | 1085 | 1512 |
| AD-39752.1 | 232 | 659 | 1086 | 1513 |
| AD-39758.1 | 233 | 660 | 1087 | 1514 |
| AD-39764.1 | 234 | 661 | 1088 | 1515 |
| AD-39770.1 | 235 | 662 | 1089 | 1516 |
| AD-39776.1 | 236 | 663 | 1090 | 1517 |
| AD-39735.1 | 237 | 664 | 1091 | 1518 |

(continued)

| | UNMODIFIED SEQUENCES | | EXAMPLE MODIFIED | SEQUENCES |
|---|---|---|---|---|
| Duplex | SENSE SEQ ID NO: SEQ ID NO: | ANTI-SENSE SEQ ID NO: | SENSE SEQ ID NO: | ANTI-SENSE SEQ ID NO: |
| AD-39741.1 | 238 | 665 | 1092 | 1519 |
| AD-39747.1 | 239 | 666 | 1093 | 1520 |
| AD-39753.1 | 240 | 667 | 1094 | 1521 |
| AD-39759.1 | 241 | 668 | 1095 | 1522 |
| AD-39765.1 | 242 | 669 | 1096 | 1523 |
| AD-39771.1 | 243 | 670 | 1097 | 1524 |
| AD-39778.1 | 244 | 671 | 1098 | 1525 |
| AD-39784.1 | 245 | 672 | 1099 | 1526 |
| AD-39790.1 | 246 | 673 | 1100 | 1527 |
| AD-39796.1 | 247 | 674 | 1101 | 1528 |
| AD-39802.1 | 248 | 675 | 1102 | 1529 |
| AD-39808.1 | 249 | 676 | 1103 | 1530 |
| AD-39814.1 | 250 | 677 | 1104 | 1531 |
| AD-39820.1 | 251 | 678 | 1105 | 1532 |
| AD-39779.1 | 252 | 679 | 1106 | 1533 |
| AD-39785.1 | 253 | 680 | 1107 | 1534 |
| AD-39791.1 | 254 | 681 | 1108 | 1535 |
| AD-39797.1 | 255 | 682 | 1109 | 1536 |
| AD-39803.1 | 256 | 683 | 1110 | 1537 |
| AD-39809.1 | 257 | 684 | 1111 | 1538 |
| AD-39815.1 | 258 | 685 | 1112 | 1539 |
| AD-39821.1 | 259 | 686 | 1113 | 1540 |
| AD-39780.1 | 260 | 687 | 1114 | 1541 |
| AD-39786.1 | 261 | 688 | 1115 | 1542 |
| AD-39792.1 | 262 | 689 | 1116 | 1543 |
| AD-39798.1 | 263 | 690 | 1117 | 1544 |
| AD-39804.1 | 264 | 691 | 1118 | 1545 |
| AD-39810.1 | 265 | 692 | 1119 | 1546 |
| AD-39816.1 | 266 | 693 | 1120 | 1547 |
| AD-39822.1 | 267 | 694 | 1121 | 1548 |
| AD-39781.1 | 268 | 695 | 1122 | 1549 |
| AD-39787.1 | 269 | 696 | 1123 | 1550 |
| AD-39793.1 | 270 | 697 | 1124 | 1551 |
| AD-39799.1 | 271 | 698 | 1125 | 1552 |
| AD-39805.1 | 272 | 699 | 1126 | 1553 |

(continued)

| | UNMODIFIED SEQUENCES | | EXAMPLE MODIFIED | SEQUENCES |
|---|---|---|---|---|
| Duplex | SENSE SEQ ID NO: SEQ ID NO: | ANTI-SENSE SEQ ID NO: | SENSE SEQ ID NO: | ANTI-SENSE SEQ ID NO: |
| AD-39811.1 | 273 | 700 | 1127 | 1554 |
| AD-39817.1 | 274 | 701 | 1128 | 1555 |
| AD-39823.1 | 275 | 702 | 1129 | 1556 |
| AD-39782.1 | 276 | 703 | 1130 | 1557 |
| AD-39788.1 | 277 | 704 | 1131 | 1558 |
| AD-39794.1 | 278 | 705 | 1132 | 1559 |
| AD-39800.1 | 279 | 706 | 1133 | 1560 |
| AD-39806.1 | 280 | 707 | 1134 | 1561 |
| AD-39812.1 | 281 | 708 | 1135 | 1562 |
| AD-39818.1 | 282 | 709 | 1136 | 1563 |
| AD-39824.1 | 283 | 710 | 1137 | 1564 |
| AD-39783.1 | 284 | 711 | 1138 | 1565 |
| AD-39789.1 | 285 | 712 | 1139 | 1566 |
| AD-39795.1 | 286 | 713 | 1140 | 1567 |
| AD-39801.1 | 287 | 714 | 1141 | 1568 |
| AD-39807.1 | 288 | 715 | 1142 | 1569 |
| AD-39813.1 | 289 | 716 | 1143 | 1570 |
| AD-39819.1 | 290 | 717 | 1144 | 1571 |
| AD-39825.1 | 291 | 718 | 1145 | 1572 |
| AD-39831.1 | 292 | 719 | 1146 | 1573 |
| AD-39837.1 | 293 | 720 | 1147 | 1574 |
| AD-39843.1 | 294 | 721 | 1148 | 1575 |
| AD-39849.1 | 295 | 722 | 1149 | 1576 |
| AD-39855.1 | 296 | 723 | 1150 | 1577 |
| AD-39861.1 | 297 | 724 | 1151 | 1578 |
| AD-39867.1 | 298 | 725 | 1152 | 1579 |
| AD-39826.1 | 299 | 726 | 1153 | 1580 |
| AD-39832.1 | 300 | 727 | 1154 | 1581 |
| AD-39838.1 | 301 | 728 | 1155 | 1582 |
| AD-39844.1 | 302 | 729 | 1156 | 1583 |
| AD-39850.1 | 303 | 730 | 1157 | 1584 |
| AD-39856.1 | 304 | 731 | 1158 | 1585 |
| AD-39862.1 | 305 | 732 | 1159 | 1586 |
| AD-39868.1 | 306 | 733 | 1160 | 1587 |
| AD-39827.1 | 307 | 734 | 1161 | 1588 |

(continued)

| | UNMODIFIED SEQUENCES | | EXAMPLE MODIFIED | SEQUENCES |
|---|---|---|---|---|
| Duplex | SENSE SEQ ID NO: SEQ ID NO: | ANTI-SENSE SEQ ID NO: | SENSE SEQ ID NO: | ANTI-SENSE SEQ ID NO: |
| AD-39833.1 | 308 | 735 | 1162 | 1589 |
| AD-39839.1 | 309 | 736 | 1163 | 1590 |
| AD-39845.1 | 310 | 737 | 1164 | 1591 |
| AD-39851.1 | 311 | 738 | 1165 | 1592 |
| AD-39857.1 | 312 | 739 | 1166 | 1593 |
| AD-39863.1 | 313 | 740 | 1167 | 1594 |
| AD-39869.1 | 314 | 741 | 1168 | 1595 |
| AD-39828.1 | 315 | 742 | 1169 | 1596 |
| AD-39834.1 | 316 | 743 | 1170 | 1597 |
| AD-39840.1 | 317 | 744 | 1171 | 1598 |
| AD-39846.1 | 318 | 745 | 1172 | 1599 |
| AD-39852.1 | 319 | 746 | 1173 | 1600 |
| AD-39858.1 | 320 | 747 | 1174 | 1601 |
| AD-39864.1 | 321 | 748 | 1175 | 1602 |
| AD-39870.1 | 322 | 749 | 1176 | 1603 |
| AD-39829.1 | 323 | 750 | 1177 | 1604 |
| AD-39835.1 | 324 | 751 | 1178 | 1605 |
| AD-39841.1 | 325 | 752 | 1179 | 1606 |
| AD-39853.1 | 326 | 753 | 1180 | 1607 |
| AD-39859.1 | 327 | 754 | 1181 | 1608 |
| AD-39865.1 | 328 | 755 | 1182 | 1609 |
| AD-39871.1 | 329 | 756 | 1183 | 1610 |
| AD-39830.1 | 330 | 757 | 1184 | 1611 |
| AD-39836.1 | 331 | 758 | 1185 | 1612 |
| AD-39842.1 | 332 | 759 | 1186 | 1613 |
| AD-39848.1 | 333 | 760 | 1187 | 1614 |
| AD-39854.1 | 334 | 761 | 1188 | 1615 |
| AD-39860.1 | 335 | 762 | 1189 | 1616 |
| AD-39866.1 | 336 | 763 | 1190 | 1617 |
| AD-39992.1 | 337 | 764 | 1191 | 1618 |
| AD-39998.1 | 338 | 765 | 1192 | 1619 |
| AD-40004.1 | 339 | 766 | 1193 | 1620 |
| AD-40010.1 | 340 | 767 | 1194 | 1621 |
| AD-40016.1 | 341 | 768 | 1195 | 1622 |
| AD-40022.1 | 342 | 769 | 1196 | 1623 |

(continued)

| | UNMODIFIED SEQUENCES | | EXAMPLE MODIFIED | SEQUENCES |
|---|---|---|---|---|
| Duplex | SENSE SEQ ID NO: SEQ ID NO: | ANTI-SENSE SEQ ID NO: | SENSE SEQ ID NO: | ANTI-SENSE SEQ ID NO: |
| AD-40028.1 | 343 | 770 | 1197 | 1624 |
| AD-40034.1 | 344 | 771 | 1198 | 1625 |
| AD-39999.1 | 345 | 772 | 1199 | 1626 |
| AD-40005.1 | 346 | 773 | 1200 | 1627 |
| AD-40011.1 | 347 | 774 | 1201 | 1628 |
| AD-40017.1 | 348 | 775 | 1202 | 1629 |
| AD-40029.1 | 349 | 776 | 1203 | 1630 |
| AD-40035.1 | 350 | 777 | 1204 | 1631 |
| AD-39994.1 | 351 | 778 | 1205 | 1632 |
| AD-40000.1 | 352 | 779 | 1206 | 1633 |
| AD-40006.1 | 353 | 780 | 1207 | 1634 |
| AD-40012.1 | 354 | 781 | 1208 | 1635 |
| AD-40018.1 | 355 | 782 | 1209 | 1636 |
| AD-40024.1 | 356 | 783 | 1210 | 1637 |
| AD-40030.1 | 357 | 784 | 1211 | 1638 |
| AD-40036.1 | 358 | 785 | 1212 | 1639 |
| AD-39995.1 | 359 | 786 | 1213 | 1640 |
| AD-40001.1 | 360 | 787 | 1214 | 1641 |
| AD-40007.1 | 361 | 788 | 1215 | 1642 |
| AD-40013.1 | 362 | 789 | 1216 | 1643 |
| AD-40019.1 | 363 | 790 | 1217 | 1644 |
| AD-40025.1 | 364 | 791 | 1218 | 1645 |
| AD-40031.1 | 365 | 792 | 1219 | 1646 |
| AD-40037.1 | 366 | 793 | 1220 | 1647 |
| AD-39996.1 | 367 | 794 | 1221 | 1648 |
| AD-40002.1 | 368 | 795 | 1222 | 1649 |
| AD-40008.1 | 369 | 796 | 1223 | 1650 |
| AD-40014.1 | 370 | 797 | 1224 | 1651 |
| AD-40020.1 | 371 | 798 | 1225 | 1652 |
| AD-40026.1 | 372 | 799 | 1226 | 1653 |
| AD-40032.1 | 373 | 800 | 1227 | 1654 |
| AD-40038.1 | 374 | 801 | 1228 | 1655 |
| AD-39997.1 | 375 | 802 | 1229 | 1656 |
| AD-40009.1 | 376 | 803 | 1230 | 1657 |
| AD-40015.1 | 377 | 804 | 1231 | 1658 |

(continued)

| | UNMODIFIED SEQUENCES | | EXAMPLE MODIFIED | SEQUENCES |
|---|---|---|---|---|
| Duplex | SENSE SEQ ID NO: SEQ ID NO: | ANTI-SENSE SEQ ID NO: | SENSE SEQ ID NO: | ANTI-SENSE SEQ ID NO: |
| AD-40021.1 | 378 | 805 | 1232 | 1659 |
| AD-40027.1 | 379 | 806 | 1233 | 1660 |
| AD-40033.1 | 380 | 807 | 1234 | 1661 |
| AD-40039.1 | 381 | 808 | 1235 | 1662 |
| AD-40045.1 | 382 | 809 | 1236 | 1663 |
| AD-40051.1 | 383 | 810 | 1237 | 1664 |
| AD-40057.1 | 384 | 811 | 1238 | 1665 |
| AD-40063.1 | 385 | 812 | 1239 | 1666 |
| AD-40069.1 | 386 | 813 | 1240 | 1667 |
| AD-40075.1 | 387 | 814 | 1241 | 1668 |
| AD-40081.1 | 388 | 815 | 1242 | 1669 |
| AD-40040.1 | 389 | 816 | 1243 | 1670 |
| AD-40046.1 | 390 | 817 | 1244 | 1671 |
| AD-40052.1 | 391 | 818 | 1245 | 1672 |
| AD-40058.1 | 392 | 819 | 1246 | 1673 |
| AD-40064.1 | 393 | 820 | 1247 | 1674 |
| AD-40070.1 | 394 | 821 | 1248 | 1675 |
| AD-40076.1 | 395 | 822 | 1249 | 1676 |
| AD-40082.1 | 396 | 823 | 1250 | 1677 |
| AD-40041.1 | 397 | 824 | 1251 | 1678 |
| AD-40047.1 | 398 | 825 | 1252 | 1679 |
| AD-40053.1 | 399 | 826 | 1253 | 1680 |
| AD-40059.1 | 400 | 827 | 1254 | 1681 |
| AD-40065.1 | 401 | 828 | 1255 | 1682 |
| AD-40071.1 | 402 | 829 | 1256 | 1683 |
| AD-40077.1 | 403 | 830 | 1257 | 1684 |
| AD-40083.1 | 404 | 831 | 1258 | 1685 |
| AD-40042.1 | 405 | 832 | 1259 | 1686 |
| AD-40048.1 | 406 | 833 | 1260 | 1687 |
| AD-40054.1 | 407 | 834 | 1261 | 1688 |
| AD-40060.1 | 408 | 835 | 1262 | 1689 |
| AD-40066.1 | 409 | 836 | 1263 | 1690 |
| AD-40072.1 | 410 | 837 | 1264 | 1691 |
| AD-40078.1 | 411 | 838 | 1265 | 1692 |
| AD-40084.1 | 412 | 839 | 1266 | 1693 |

(continued)

| | UNMODIFIED SEQUENCES | | EXAMPLE MODIFIED | SEQUENCES |
|---|---|---|---|---|
| Duplex | SENSE SEQ ID NO: SEQ ID NO: | ANTI-SENSE SEQ ID NO: | SENSE SEQ ID NO: | ANTI-SENSE SEQ ID NO: |
| AD-40043.1 | 413 | 840 | 1267 | 1694 |
| AD-40049.1 | 414 | 841 | 1268 | 1695 |
| AD-40055.1 | 415 | 842 | 1269 | 1696 |
| AD-40061.1 | 416 | 843 | 1270 | 1697 |
| AD-40067.1 | 417 | 844 | 1271 | 1698 |
| AD-40073.1 | 418 | 845 | 1272 | 1699 |
| AD-40079.1 | 419 | 846 | 1273 | 1700 |
| AD-40085.1 | 420 | 847 | 1274 | 1701 |
| AD-40044.1 | 421 | 848 | 1275 | 1702 |
| AD-40050.1 | 422 | 849 | 1276 | 1703 |
| AD-40056.1 | 423 | 850 | 1277 | 1704 |
| AD-40062.1 | 424 | 851 | 1278 | 1705 |
| AD-40068.1 | 425 | 852 | 1279 | 1706 |
| AD-40074.1 | 426 | 853 | 1280 | 1707 |
| AD-40080.1 | 427 | 854 | 1281 | 1708 |
| AD-39720.1 | 1716 | 1754 | 1792 | 1830 |
| AD-39706.1 | 1717 | 1755 | 1793 | 1831 |
| AD-39712.1 | 1718 | 1756 | 1794 | 1832 |
| AD-39760.1 | 1719 | 1757 | 1795 | 1833 |
| AD-39732.1 | 1720 | 1758 | 1796 | 1834 |
| AD-35541.4 | 1721 | 1759 | 1797 | 1835 |
| AD-39735.1 or hs_KRAS_321_A22S26 | 1722 | 1760 | 1798 | 1836 |
| AD-39741.1 or hs_KRAS_322_A22S26 | 1723 | 1761 | 1799 | 1837 |
| AD-39778.1 | 1724 | 1762 | 1800 | 1838 |
| AD-39790.1 | 1725 | 1763 | 1801 | 1839 |
| AD-39822.1 | 1726 | 1764 | 1802 | 1840 |
| AD-35609.4 | 1727 | 1765 | 1803 | 1841 |
| AD-35581.4 | 1728 | 1766 | 1804 | 1842 |
| AD-39845.1 | 1729 | 1767 | 1805 | 1843 |
| AD-39858.1 | 1730 | 1768 | 1806 | 1844 |
| AD-39870.1 | 1731 | 1769 | 1807 | 1845 |
| AD-35576.4 | 1732 | 1770 | 1808 | 1846 |
| AD-35588.4 | 1733 | 1771 | 1809 | 1847 |
| AD-35600.1 | 1734 | 1772 | 1810 | 1848 |

(continued)

| | UNMODIFIED SEQUENCES | | EXAMPLE MODIFIED | SEQUENCES |
|---|---|---|---|---|
| Duplex | SENSE SEQ ID NO: SEQ ID NO: | ANTI-SENSE SEQ ID NO: | SENSE SEQ ID NO: | ANTI-SENSE SEQ ID NO: |
| AD-35606.1 | 1735 | 1773 | 1811 | 1849 |
| AD-38151.1 | 1736 | 1774 | 1812 | 1850 |
| AD-38163.1 | 1737 | 1775 | 1813 | 1851 |
| AD-39999.1 | 1738 | 1776 | 1814 | 1852 |
| AD-38159.1 or hs_KRAS_ 528_A22S26 | 1739 | 1777 | 1815 | 1853 |
| AD-38130.1 or hs_KRAS_ 531_A22S26 | 1740 | 1778 | 1816 | 1854 |
| AD-38136.1 | 1741 | 1779 | 1817 | 1855 |
| AD-40036.1 | 1742 | 1780 | 1818 | 1856 |
| AD-40008.1 | 1743 | 1781 | 1819 | 1857 |
| AD-40021.1 | 1744 | 1782 | 1820 | 1858 |
| AD-40077.1 | 1745 | 1783 | 1821 | 1859 |
| AD-40072.1 | 1746 | 1784 | 1822 | 1860 |
| AD-40061.1 | 1747 | 1785 | 1823 | 1861 |
| AD-40068.1 | 1748 | 1786 | 1824 | 1862 |
| AD-38131.1 | 1749 | 1787 | 1825 | 1863 |
| AD-38167.1 | 1750 | 1788 | 1826 | 1864 |
| KRAS1273 31273_A22S26 | 1751 | 1789 | 1827 | 1865 |
| KRAS2892 2892_A37S26 | 1752 | 1790 | 1828 | 1866 |
| KRAS4731 4731_A22S26 | 1753 | 1791 | 1829 | 1867 |
| AD-39741.1 | 2770 | 2760 | | |
| AD-39735.1 | 2771 | 2761 | | |
| AD-38159.1 | 2772 | 2762 | | |
| NA | 2773 | 2763 | | |
| AD-38130.1 | 2774 | 2764 | | |
| NA | 2775 | 2765 | | |
| NA | 2776 | 2766 | | |
| AD-38136.1 | 2777 | 2767 | | |
| AD-40061.1 | 2778 | 2768 | | |
| AD-35588.4 | 2779 | 2769 | | |
| AD-39845.1 | 2790 | 2780 | | |
| AD-39720.1 | 2791 | 2781 | | |
| AD-35581.4 | 2792 | 2782 | | |
| AD-40068.1 | 2793 | 2783 | | |
| AD-39778.1 | 2794 | 2784 | | |

(continued)

| | UNMODIFIED SEQUENCES | | EXAMPLE MODIFIED | SEQUENCES |
|---|---|---|---|---|
| Duplex | SENSE SEQ ID NO: SEQ ID NO: | ANTI-SENSE SEQ ID NO: | SENSE SEQ ID NO: | ANTI-SENSE SEQ ID NO: |
| AD-35606.1 | 2795 | 2785 | | |
| AD-35609.4 | 2796 | 2786 | | |
| AD-38163.1 | 2797 | 2787 | | |
| AD-35600.1 | 2798 | 2788 | | |
| AD-40008.1 | 2799 | 2789 | | |
| AD-40036.1 | 2810 | 2800 | | |
| AD-39790.1 | 2811 | 2801 | | |
| AD-39870.1 | 2812 | 2802 | | |
| AD-38131.1 | 2813 | 2803 | | |
| AD-40077.1 | 2814 | 2804 | | |
| AD-38167.1 | 2815 | 2805 | | |
| AD-38151.1 | 2816 | 2806 | | |
| AD-39858.1 | 2817 | 2807 | | |
| AD-39706.1 | 2818 | 2808 | | |
| AD-40021.1 | 2819 | 2809 | | |
| AD-39760.1 | 2828 | 2820 | | |
| AD-39822.1 | 2829 | 2821 | | |
| AD-39999.1 | 2830 | 2822 | | |
| AD-40072.1 | 2831 | 2823 | | |
| AD-39732.1 | 2832 | 2824 | | |
| AD-35576.4 | 2833 | 2825 | | |
| AD-39712.1 | 2834 | 2826 | | |
| AD-35541.4 | 2835 | 2827 | | |

Table 1 presents the SEQ ID identifiers of an example unmodified first and second strand of each KRAS RNAi agent; the Table also provides an example modified sequence for each KRAS RNAi agent. For example, in Table 1, the unmodified sequences of example KRAS RNAi agent nicknamed AD-35523.3 are SEQ ID NOs: 1 and 428. An example set of modified sequences for this duplex is represented by SEQ ID NOs: 855 and 1282. Also note that the "AD" prefix of the nickname is on occasion replaced by "ND". The name of the RNAi also sometimes has a suffix, such as .1. This indicates a particular variant of a duplex. Thus, AD-35523.1, AD-35523.2, AD-35523.3, ND-35523 and the like all indicate KRAS RNAi agents of the same sequence, although they may differ in how they are modified. In these cases, replacement of a U with a T is considered a modification that does not introduce a mismatch; thus, two otherwise identical sequences wherein one or more U bases are replaced by T are still considered to be identical and the same. Some nicknames of RNAi agents also comprise the suffix "b1", indicating "batch 1." The various KRAS RNAi agents comprising the nickname AD-35523 thus all have the same base sequence (though not necessarily the same modification), and the suffix "b1" (or "b2" or the like) may indicate a particular batch, though these also have the same sequence.

Some RNAi agents in Table 1 have alternative names or nicknames. Some nicknames are derived from their position in the KRAS gene and, often, a code for the format for modifications. It is noted, however, that different methodologies are available for numbering the KRAS gene sequence. The numbering used herein for the KRAS gene is for illustration only. Alternative names of some duplexes are listed below:

**Table 1.1. Alternative Names of some RNAi Agents**

| Duplex Name | Nickname |
|---|---|
| AD-39741.1 | hs_KRAS_322_A22S26 |
| AD-38159.1 | hs_KRAS_528_A22S26 |
| AD-39735.1 | hs_KRAS_321_A22S26 |
| AD-38130.1 | hs_KRAS_531_A22S26 |

NA, not applicable.

Additional alternative names are provided in Tables 1, 2 and 3. For example, hs_KRAS 1273_A22S26, hs_KRAS 2892_A37S26, and hs_KRAS 4731_A22S26 are also sometimes alternatively designated KRAS1273, KRAS2892, and KRAS4731, respectively.

**Table 1.2. Additional Alternative Names of some RNAi Agents**

Additional siRNAs disclosed herein also have alternative names. These names begin with AD. These alternative names, nicknames and sequences of the generic (unmodified) sequences are provided below. The target sequence (DNA) corresponding to the Guide and Sense strand are provided. siRNAs include, for example, double-stranded RNA corresponding to the DNA sequences provided (e.g., as RNA with or without any of the following or combination of any of the following: modification(s), terminal TT or other overhang(s), endcap(s), alternative base (LNA, PNA, etc.), and/or a few DNA bases interspersed among the RNA or other bases, etc.).

| AD nickname | Nickname siRNA | Guide 19mer generic | SEQ ID NO. | Sense 19mer generic | SEQ ID NO. |
|---|---|---|---|---|---|
| AD-39741.1 | hs KRAS 322 A22S26 | ATCAATTACTACTTGCTTC | 2760 | GAAGCAAGTAGTAATT GAT | 2770 |
| AD-39735.1 | hs KRAS 321 A22S26 | TCAATTACTACTTGCTTCC | 2761 | GGAAGCAAGTAGTAAT TGA | 2771 |
| AD-38159.1 | hs KRAS 528 A22S26 | TTTCCTACTAGGACCATAG | 2762 | CTATGGTCCTAGTAGG AAA | 2772 |
| NA | hs KRAS 1273 A22S26 | TAACTTTACATTCATCAGG | 2763 | CCTGATGAATGTAAAGT TA | 2773 |
| AD-38130.1 | hs KRAS 531 A22S26 | TTATTTCCTACTAGGACCA | 2764 | TGGTCCTAGTAGGAAA TAA | 2774 |
| NA | hs KRAS 4731 A22S26 | AATCTAGTTATGACTATTC | 2765 | GAATAGTCATAACTAGA TT | 2775 |
| NA | hs KRAS 2892 A37S26 | TATCTTTATTAAATTCTCC | 2766 | GGAGAATTTAATAAAGA TA | 2776 |
| AD-38136.1 | NA | TTTATTTCCTACTAGGACC | 2767 | GGTCCTAGTAGGAAAT AAA | 2777 |
| AD-40061.1 | NA | AATTCCATAACTTCTTGCT | 2768 | AGCAAGAAGTTATGGA ATT | 2778 |
| AD-35588.4 | NA | TCTATAATGGTGAATATCT | 2769 | AGATATTCACCATTATA GA | 2779 |
| AD-39845.1 | NA | GATTTAGTATTATTTATGG | 2780 | CCATAAATAATACTAAA TC | 2790 |

(continued)

| Additional siRNAs disclosed herein also have alternative names. These names begin with AD. These alternative names, nicknames and sequences of the generic (unmodified) sequences are provided below. The target sequence (DNA) corresponding to the Guide and Sense strand are provided. siRNAs include, for example, double-stranded RNA corresponding to the DNA sequences provided (e.g., as RNA with or without any of the following or combination of any of the following: modification(s), terminal TT or other overhang(s), endcap(s), alternative base (LNA, PNA, etc.), and/or a few DNA bases interspersed among the RNA or other bases, etc.). | | | | | |
|---|---|---|---|---|---|
| **AD nickname** | **Nickname siRNA** | **Guide 19mer generic** | **SEQ ID NO.** | **Sense 19mer generic** | **SEQ ID NO.** |
| AD-39720.1 | NA | AGCTCCAACTACCACAAGT | 2781 | ACTTGTGGTAGTTGGA GCT | 2791 |
| AD-35581.4 | NA | TTATTTATGGCAAATACAC | 2782 | GTGTATTTGCCATAAAT AA | 2792 |
| AD-40068.1 | NA | AATAAAAGGAATTCCATAA | 2783 | TTATGGAATTCCTTTTA TT | 2793 |
| AD-39778.1 | NA | TTCTCCATCAATTACTACT | 2784 | AGTAGTAATTGATGGA GAA | 2794 |
| AD-35606.1 | NA | TTCTCTATAATGGTGAATA | 2785 | TATTCACCATTATAGAG AA | 2795 |
| AD-35609.4 | NA | AAATACACAAAGAAAGCCC | 2786 | GGGCTTTCTTTGTGTAT TT | 2796 |
| AD-38163.1 | NA | TTTAATTTGTTCTCTATAA | 2787 | TTATAGAGAACAAATTA AA | 2797 |
| AD-35600.1 | NA | TCTCTATAATGGTGAATAT | 2788 | ATATTCACCATTATAGA GA | 2798 |
| AD-40008.1 | NA | TCTAGAAGGCAAATCACAT | 2789 | ATGTGATTTGCCTTCTA GA | 2799 |
| AD-40036.1 | NA | AATCACATTTATTTCCTAC | 2800 | GTAGGAAATAAATGTG ATT | 2810 |
| AD-39790.1 | NA | GTTTCTCCATCAATTACTA | 2801 | TAGTAATTGATGGAGAA AC | 2811 |
| AD-39870.1 | hs KRAS 459 A37S26 | ATATCTTCAAATGATTTAG | 2802 | CTAAATCATTTGAAGAT AT | 2812 |
| AD-38131.1 | NA | TTTCAATAAAAGGAATTCC | 2803 | GGAATTCCTTTTATTGA AA | 2813 |
| AD-40077.1 | NA | CTTGCTAAGTCCTGAGCCT | 2804 | AGGCTCAGGACTTAGC AAG | 2814 |
| AD-38167.1 | NA | CTGATGTTTCAATAAAAGG | 2805 | CCTTTTATTGAAACATC AG | 2815 |
| AD-38151.1 | NA | TAATTTGTTCTCTATAATG | 2806 | CATTATAGAGAACAAAT TA | 2816 |
| AD-39858.1 | NA | ATCTTCAAATGATTTAGTA | 2807 | TACTAAATCATTTGAAG AT | 2817 |

(continued)

| Additional siRNAs disclosed herein also have alternative names. These names begin with AD. These alternative names, nicknames and sequences of the generic (unmodified) sequences are provided below. The target sequence (DNA) corresponding to the Guide and Sense strand are provided. siRNAs include, for example, double-stranded RNA corresponding to the DNA sequences provided (e.g., as RNA with or without any of the following or combination of any of the following: modification(s), terminal TT or other overhang(s), endcap(s), alternative base (LNA, PNA, etc.), and/or a few DNA bases interspersed among the RNA or other bases, etc.). | | | | | |
|---|---|---|---|---|---|
| **AD nickname** | **Nickname siRNA** | **Guide 19mer generic** | **SEQ ID NO.** | **Sense 19mer generic** | **SEQ ID NO.** |
| AD-39706.1 | NA | ATCGTCAAGGCACTCTTGC | 2808 | GCAAGAGTGCCTTGACGAT | 2818 |
| AD-40021.1 | NA | TGTCTACTGTTCTAGAAGG | 2809 | CCTTCTAGAACAGTAGACA | 2819 |
| AD-39760.1 | NA | ATTAGCTGTATCGTCAAGG | 2820 | CCTTGACGATACAGCTAAT | 2828 |
| AD-39822.1 | NA | TCGAGAATATCCAAGAGAC | 2821 | GTCTCTTGGATATTCTCGA | 2829 |
| AD-39999.1 | NA | TCTTCAGAGTCCTTAACTC | 2822 | GAGTTAAGGACTCTGAAGA | 2830 |
| AD-40072.1 | NA | ATAACTTCTTGCTAAGTCC | 2823 | GGACTTAGCAAGAAGTTAT | 2831 |
| AD-39732.1 | NA | AATGATTCTGAATTAGCTG | 2824 | CAGCTAATTCAGAATCATT | 2832 |
| AD-35576.4 | NA | TATAATGGTGAATATCTTC | 2825 | GAAGATATTCACCATTATA | 2833 |
| AD-39712.1 | NA | TATCGTCAAGGCACTCTTG | 2826 | CAAGAGTGCCTTGACGATA | 2834 |
| AD-35541.4 | NA | TATTGTTGGATCATATTCG | 2827 | CGAATATGATCCAACAATA | 2835 |

Additional tables (e.g., Tables 1.1 and 1.2) are continuations of Table 1; thus, references to any Table or Tables include references to additional Tables. For example, references to Table 1 also include references to Tables 1.1 and 1.2; and references to Tables 1 to 6 include Tables 1.1, 1.2, 6.1, 6.2, etc.

**Various Embodiments of the Present Disclosure**

Various RNAi agents to KRAS are disclosed in Tables 1 to 6.

Tables 1, 2 and 3 provide the sequence, SEQ ID NOs and relative positions of various RNAi agents to KRAS, including both unmodified sequences (listed in Table 2) and example modified sequences (listed in Table 3).

In the sequences in Table 3, lower-case letters (e.g., c, u) indicate modified nucleotides while upper case letters (e.g., C, U, A, G) indicate unmodified nucleotides. In this Table, example modified versions of each of the sequences are shown. However, the present disclosure also contemplates and encompasses unmodified versions of these sequences and other versions which comprise additional or alternative modifications. Thus, for example, AD-35523.3 can optionally have the unmodified sequences of SEQ ID NO: 1 in the sense strand and SEQ ID NO: 428 in the anti-sense strand. The present disclosure also encompasses alternative modified versions of the duplex comprising the modified sequences of SEQ ID NO: 855 in the sense strand and SEQ ID NO: 1282 in the anti-sense strand. The present disclosure also encompasses different modified variants of SEQ ID NO: 1 in the sense strand and SEQ ID NO: 428 in the anti-sense strand. Similarly, the present disclosure also encompasses any modified variant of any unmodified sequence disclosed herein.

In the sequences in Tables 1 to 3, the modified and unmodified sequences can optionally comprise the sequence "TT", "dTdT", "dTsdT" or "UU" at the 3' end. Thus, in the example above, AD-35523.3 can optionally have the modified sequences of SEQ ID NO: 855 in the sense strand and SEQ ID NO: 1282 in the anti-sense strand. As noted in Table 3, below, SEQ ID NO: 855 and SEQ ID NO: 1282 include a terminal dTdT. dT is 2'-deoxy-thymidine-5'-phosphate and sdT is 2'-deoxy Thymidine 5'-phosphorothioate. In the disclosed sequences, terminal dinucleotide "UU" is 2'-OMe-U 2'-OMe-U, and neither the terminal TT nor the terminal UU are in the inverted/reverse orientation. The terminal dithymidine (or UU) is not part of the KRAS target sequence, but is a modified variant of the dithymidine dinucleotide commonly placed as an overhang to protect the ends of siRNAs from nucleases (see, for example, Elbashir et al. 2001 Nature 411: 494-498; Elbashir et al. 2001 EMBO J. 20: 6877-6888; and Kraynack et al. 2006 RNA 12:163-176). A terminal dinucleotide is known from these references to enhance nuclease resistance but not contribute to target recognition. Thus, the present disclosure also encompasses any modified or any unmodified sequence disclosed herein, wherein the modified sequence comprises a terminal TT, dTdT, sdT, dTsdT, sdTsdT, sdTdT, or the like which may be in either the inverted/reverse orientation or in the same 5' to 3' orientation as the KRAS specific sequence in the duplex. In addition, terminology used herein referring to "the KRAS portion of a RNAi agent sequence" and the like indicate the portion of the sequence of a RNAi agent which is derived from KRAS (thus "the KRAS portion of a RNAi agent sequence" does not include, for example, a terminal dTdT, TT, UU, or the like, but does include the portion of the RNAi agent that corresponds to or is complementary to a portion of the KRAS gene sequence or mRNA sequence.

On any modified or unmodified sequence, a 3' end cap, as is known in the art, can be used instead of or in addition to a terminal dinucleotide to stabilize the end from nuclease degradation provided that the 3' end cap is able to both stablize the RNAi agent (e.g., against nucleases) and not interfere excessively with siRNA activity. Thus, the present disclosure also encompasses any modified or any unmodified sequence disclosed herein, wherein the modified sequence further comprises a terminal 3' end cap.

Table 4 provides sets of overlapping RNAi agents to KRAS.

Activity levels of various KRAS RNAi agents are provided in Tables 5 and 6.

**An RNAi agent comprising an anti-sense strand described herein**

In one particular specific embodiment, the present disclosure relates to a composition comprising a RNAi agent comprising an anti-sense strand, wherein the anti-sense strand comprises at least 15 contiguous nucleotides differing by 0, 1, 2, or 3 nucleotides from the anti-sense strand of a RNAi agent to KRAS selected from those anti-sense strands in the specific duplexes provided herein and as listed, e.g., in Tables 1 to 6.

Various specific embodiments of this embodiment are described below.

In one embodiment, the composition further comprises a second RNAi agent to KRAS. In various embodiments, the second RNAi agent is physically separate from the first, or the two KRAS RNAi agents are physically connected (e.g., covalently linked or otherwise conjugated) or combined in the same pharmaceutical composition, or are both elements in the same treatment regimen.

In one embodiment, the composition comprises a RNAi agent comprising a first and an second strand, wherein the sequence of the first strand and the sequence of the second strand are the sequences of the first and second strand, respectively, of any RNAi agent provided herein. In one embodiment, the composition comprises a RNAi agent comprising a first and an second strand, wherein the sequence of the first strand and the sequence of the second strand are the sequences of the first and second strand, respectively, of any RNAi agent provided herein, further comprising an additional about 6 to 20 nucleotides on one or both strands (e.g., about 6, 7, 8, 9, 10, 11, 12, 13, 14, 15, 16, 17, 18, 19 or 20 nt). In various embodiments, the first and second strands are the sense and anti-sense strands listed in the Tables herein, respectively. In various embodiments, the first and second strands are the anti-sense and sense strands listed in the Tables herein, respectively.

In one embodiment, the antisense strand is about 30 or fewer nt in length.

In one embodiment, the sense strand and the antisense strand form a duplex region of about 15 to about 30 nucleotide pairs in length.

In one embodiment, the antisense strand is about 15 to about 36 nt in length, including about 18 to about 30 nt in length, and further including about 19 to about 21 nt in length and about 19 to about 23 nt in length. In one embodiment, the antisense strand has at least the length selected from about 15 nt, about 16 nt, about 17 nt, about 18 nt, about 19 nt, about 20 nt, about 21 nt, about 22 nt, about 23 nt, about 24 nt, about 25 nt, about 26 nt, about 27 nt, about 28 nt, about 29 nt and about 30 nt.

In one embodiment, the RNAi agent comprises a modification that causes the RNAi agent to have increased stability in a biological sample or environment, e.g., cytoplasm, interstitial fluids, blood serum, lung or intestinal lavage fluid.

In one embodiment, the RNAi agent comprises at least one sugar backbone modification (e.g., phosphorothioate linkage) and/or at least one 2'-modified nucleotide. In one embodiment, all the pyrimidines are 2' O-methyl-modified nucleotides.

In one embodiment, the RNAi agent comprises: at least one 5'-uridine-adenine-3' (5'-ua-3') dinucleotide, wherein the uridine is a 2'-modified nucleotide; and/or at least one 5'-uridine-guanine-3' (5'-ug-3') dinucleotide, wherein the 5'-uridine is a 2'-modified nucleotide; and/or at least one 5'-cytidine-adenine-3' (5'-ca-3') dinucleotide, wherein the 5'-cytidine is a 2'-modified nucleotide; and/or at least one 5'-uridine-uridine-3' (5'-uu-3') dinucleotide, wherein the 5'-uridine is a 2'-modified nucleotide.

In one embodiment, the RNAi agent comprises a 2'-modification selected from the group consisting of: 2'-deoxy, 2'-deoxy-2'-fluoro, 2'-O-methyl, 2'-O-methoxyethyl (2'-O-MOE), 2'-O-aminopropyl (2'-O-AP), 2'-O-dimethylaminoethyl (2'-O-DMAOE), 2'-O-dimethylaminopropyl (2'-O-DMAP), 2'-O-dimethylaminoethyloxyethyl (2-O-DMAEOE), and 2'-O-N-methylacetamido (2'-O-NMA). In one embodiment, all the pyrimidines are 2' O-methyl-modified nucleotides.

In one embodiment, the RNAi agent comprises a blunt end.

In one embodiment, the RNAi agent comprises an overhang having 1 to 4 unpaired nucleotides.

In one embodiment, the RNAi agent comprises an overhang at the 3'-end of the antisense strand of the RNAi agent.

In one embodiment, the RNAi agent is ligated to one or more diagnostic compound, reporter group, cross-linking agent, nuclease-resistance conferring moiety, natural or unusual nucleobase, lipophilic molecule, cholesterol, lipid, lectin, steroid, uvaol, hecigenin, diosgenin, terpene, triterpene, sarsasapogenin, Friedelin, epifriedelanol-derivatized lithocholic acid, vitamin, carbohydrate, dextran, pullulan, chitin, chitosan, synthetic carbohydrate, oligo lactate 15-mer, natural polymer, low- or medium-molecular weight polymer, inulin, cyclodextrin, hyaluronic acid, protein, protein-binding agent, integrin-targeting molecule, polycationic, peptide, polyamine, peptide mimic, and/or transferrin.

In one embodiment, the RNAi agent is capable of inhibiting expression of KRAS by at least about 60% at a concentration of 10 nM in RKO cells *in vitro.*

In one embodiment, the RNAi agent is capable of inhibiting expression of KRAS by at least about 70% at a concentration of 10 nM in RKO cells *in vitro.*

In one embodiment, the RNAi agent is capable of inhibiting expression of KRAS by at least about 80% at a concentration of 10 nM in RKO cells *in vitro.*

In one embodiment, the RNAi agent is capable of inhibiting expression of KRAS by at least about 90% at a concentration of 10 nM in RKO cells *in vitro.*

In one embodiment, the RNAi agent has an EC50 of no more than about 0.1 nM in MiaPaca2 cells.

In one embodiment, the RNAi agent has an EC50 of no more than about 0.01 nM in MiaPaca2 cells.

In one embodiment, the RNAi agent has an EC50 of no more than about 0.001 nM in MiaPaca2 cells.

**A RNAi agent comprising a first and a second strand described herein**

In one particular specific embodiment, the present disclosure relates to a composition comprising a RNAi agent comprising a first strand and a second strand, wherein the sequence of the first strand strand comprises at least 15 contiguous nucleotides, differing by 0, 1, 2, or 3 nucleotides from the sequence of the first strand of a RNAi agent to KRAS selected from the specific duplexes provided herein and listed, e.g., in Table 1.

In one particular specific embodiment, the present disclosure relates to a composition comprising a RNAi agent comprising a first strand and a second strand, wherein the sequence of the first strand and/or second strand comprise at least 15 contiguous nucleotides, differing by 0, 1, 2, or 3 nucleotides from the sequence of the first and/or second strand of a RNAi agent to KRAS selected from the specific duplexes provided herein and listed, e.g., in Table 1.

In one particular specific embodiment, the present disclosure relates to a composition comprising a RNAi agent comprising a first strand and a second strand, wherein the sequence of the first strand and/or second strand comprise the sequence of the first and/or second strand, respectively, of a RNAi agent to KRAS selected from the specific duplexes provided herein and listed, e.g., in Table 1.

In one particular specific embodiment, the present disclosure relates to a composition comprising a RNAi agent comprising a first strand and a second strand, wherein the sequence of the first strand and/or second strand are the sequence of the first and/or second strand, respectively, of a RNAi agent to KRAS selected from the specific duplexes provided herein and listed, e.g., in Table 1.

Various specific embodiments of these embodiments are described below.

In one embodiment, the composition further comprises a second RNAi agent to KRAS. In various embodiments, the second RNAi agent is physically separate from the first, or the two are physically connected (e.g., covalently linked or otherwise conjugated).

In one embodiment, the antisense strand is about 30 or fewer nt in length.

In one embodiment, the sense strand and the antisense strand form a duplex region of about 15 to about 30 nt pairs in length.

In one embodiment, the antisense strand is about 15 to about 36 nt in length including about 18 to about 23 nt in length, and including about 19 to about 21 nt in length and about 19 to about 23 nt in length. In one embodiment, the antisense strand has at least the length selected from about 15 nt, about 16 nt, about 17 nt, about 18 nt, about 19 nt, about 20 nt, about 21 nt, about 22 nt, about 23 nt, about 24 nt, about 25 nt, about 26 nt, about 27 nt, about 28 nt, about 29 nt and about 30 nt.

In one embodiment, the RNAi agent comprises a modification that causes the RNAi agent to have increased stability in a biological sample or environment, e.g., blood serum or intestinal lavage fluid.

In one embodiment, the RNAi agent comprises at least one sugar backbone modification (e.g., phosphorothioate linkage) and/or at least one 2'-modified nucleotide. In one embodiment, all the pyrimidines are 2' O-methyl-modified nucleotides.

In one embodiment, the RNAi agent comprises: at least one 5'-uridine-adenine-3' (5'-ua-3') dinucleotide, wherein the uridine is a 2'-modified nucleotide; and/or at least one 5'-uridine-guanine-3' (5'-ug-3') dinucleotide, wherein the 5'-uridine is a 2'-modified nucleotide; and/or at least one 5'-cytidine-adenine-3' (5'-ca-3') dinucleotide, wherein the 5'-cytidine is a 2'-modified nucleotide; and/or at least one 5'-uridine-uridine-3' (5'-uu-3') dinucleotide, wherein the 5'-uridine is a 2'-modified nucleotide.

In one embodiment, the RNAi agent comprises a 2'-modification selected from the group consisting of: 2'-deoxy, 2'-deoxy-2'-fluoro, 2'-O-methyl, 2'-O-methoxyethyl (2'-O-MOE), 2'-O-aminopropyl (2'-O-AP), 2'-O-dimethylaminoethyl (2'-O-DMAOE), 2'-O-dimethylaminopropyl (2'-O-DMAP), 2'-O-dimethylaminoethyloxyethyl (2-O-DMAEOE), and 2'-O-N-methylacetamido (2'-O-NMA).

In one embodiment, the RNAi agent comprises a blunt end.

In one embodiment, the RNAi agent comprises an overhang having 1 to 4 unpaired nucleotides.

In one embodiment, the RNAi agent comprises an overhang at the 3'-end of the antisense strand of the RNAi agent.

In one embodiment, the RNAi agent is ligated to one or more diagnostic compound, reporter group, cross-linking agent, nuclease-resistance conferring moiety, natural or unusual nucleobase, lipophilic molecule, cholesterol, lipid, lectin, steroid, uvaol, hecigenin, diosgenin, terpene, triterpene, sarsasapogenin, Friedelin, epifriedelanol-derivatized lithocholic acid, vitamin, carbohydrate, dextran, pullulan, chitin, chitosan, synthetic carbohydrate, oligo lactate 15-mer, natural polymer, low- or medium-molecular weight polymer, inulin, cyclodextrin, hyaluronic acid, protein, protein-binding agent, integrin-targeting molecule, polycationic, peptide, polyamine, peptide mimic, and/or transferrin.

In one embodiment, the RNAi agent is capable of inhibiting expression of KRAS by at least about 60% at a concentration of 10 nM in RKO cells *in vitro.*

In one embodiment, the RNAi agent is capable of inhibiting expression of KRAS by at least about 70% at a concentration of 10 nM in RKO cells *in vitro.*

In one embodiment, the RNAi agent is capable of inhibiting expression of KRAS by at least about 80% at a concentration of 10 nM in RKO cells *in vitro.*

In one embodiment, the RNAi agent is capable of inhibiting expression of KRAS by at least about 90% at a concentration of 10 nM in RKO cells *in vitro.*

In one embodiment, the RNAi agent has an EC50 of no more than about 0.1 nM in MiaPaca2 cells.

In one embodiment, the RNAi agent has an EC50 of no more than about 0.01 nM in MiaPaca2 cells.

In one embodiment, the RNAi agent has an EC50 of no more than about 0.001 nM in MiaPaca2 cells.

## A method of treatment using a RNAi agent described herein

In one particular specific embodiment, the present disclosure relates to a method of treating a KRAS-related disease in an individual, comprising the step of administering to the individual a therapeutically effective amount of a composition comprising a RNAi agent comprising at least an antisense strand, wherein the antisense strand comprises at least 15 to 19 contiguous nucleotides differing by 0, 1, 2, or 3 nucleotides from the antisense strand of a RNAi agent to KRAS selected from the specific duplexes provided herein and as listed, e.g., in Table 1.

In another embodiment, the present disclosure relates to such a method, wherein the composition comprising a RNAi agent further comprises a sense strand, wherein the sense strand comprises at least 15 contiguous nucleotides differing by 0, 1, 2, or 3 nucleotides from the sense strand of a RNAi agent to KRAS selected from the specific duplexes provided herein and as listed, e.g., in Table 1.

In one embodiment of the method, the RNAi agent comprises at least an anti-sense strand, and/or comprises a sense and an anti-sense strand, wherein the sequence of the sense and/or anti-sense strand is the sequence of the sense and/or the anti-sense strand of a RNAi agent to KRAS selected from those specific duplex provided herein and as listed, e.g., in Table 1, wherein the composition further comprises a pharmaceutically effective formulation.

In one embodiment of the method, the RNAi agent comprises at least an anti-sense strand, and/or comprises a sense and an anti-sense strand, wherein the sequence of the sense and/or anti-sense strand comprises the sequence of the sense and/or the anti-sense strand of a RNAi agent to KRAS selected from those specific duplex provided herein and as listed, e.g., in Table 1, wherein the composition further comprises a pharmaceutically effective formulation.

Various particular specific embodiments of these embodiments are described below.

In one embodiment, the KRAS-related disease is a proliferative disease, or cardio-facio-cutaneous (CFC) syndrome or Noonan syndrome.

In one embodiment, the KRAS-related disease is a proliferative disease, including without limitation a solid or liquid cancer, adenocarcinoma, colorectal cancer, advanced and/or metastatic colorectal cancer, colon cancer, lung, non-small cell lung cancer and lung adenocarcinoma, acute myelogenous lung, bladder, brain, breast, cervical, endometrial, gastric, head and neck, kidney, leukemia, myelodysplastic syndrome, myeloid leukemia, liver, melanoma, ovarian, pancreatic, prostate, testicular, thyroid cancers, or cardio-facio-cutaneous (CFC) syndrome or Noonan syndrome.

In one embodiment, the method further comprises the administration of an additional treatment. In one embodiment, the additional treatment is a therapeutically effective amount of a composition.

In one embodiment, the additional treatment is a method (or procedure).

In one embodiment, the additional treatment and the RNAi agent can be administered in any order, or can be administered simultaneously.

In one embodiment, the method further comprises the step of administering an additional treatment for a proliferative disease, cardio-facio-cutaneous (CFC) syndrome or Noonan syndrome.

In one embodiment, the method further comprises the step of administering an additional treatment or therapy selected from the list of an additional antagonist to a KRAS-related disease.

In one embodiment, the composition comprises a second RNAi agent to KRAS. In various embodiments, the second RNAi agent is physically separate from the first, or the two are physically connected (e.g., covalently linked or otherwise conjugated).

In one embodiment, the method further comprises the step of administering an additional RNAi agent which comprises at least 15 contiguous nucleotides differing by 0, 1, 2, or 3 nucleotides from the antisense strand of a RNAi agent to KRAS selected from the specific duplexes provided herein and as listed, e.g., in Table 1.

**A method of inhibiting the expression of KRAS using a RNAi agent described herein**

In one particular specific embodiment, the present disclosure relates to a method of inhibiting the expression of KRAS in an individual, comprising the step of administering to the individual a therapeutically effective amount of a composition comprising a RNAi agent of the present disclosure.

In one embodiment of the method, the RNAi agent comprises at least an anti-sense strand, and/or comprises a sense and an anti-sense strand, wherein the anti-sense strand comprises at least 15 contiguous nucleotides differing by 0, 1, 2, or 3 nucleotides from the anti-sense strand of a RNAi agent to KRAS selected from those specific duplex provided herein and as listed, e.g., in Table 1.

In one embodiment of the method, the RNAi agent comprises at least an anti-sense strand, and/or comprises a sense and an anti-sense strand, wherein the sequence of the sense and/or anti-sense strand is the sequence of the sense and/or the anti-sense strand of a RNAi agent to KRAS selected from those specific duplex provided herein and as listed, e.g., in Table 1, wherein the composition is in a pharmaceutically effective formulation.

In one embodiment of the method, the RNAi agent comprises at least an anti-sense strand, and/or comprises a sense and an anti-sense strand, wherein the sequence of the sense and/or anti-sense strand comprises the sequence of the sense and/or the anti-sense strand of a RNAi agent to KRAS selected from those specific duplex provided herein and as listed, e.g., in Table 1, wherein the composition is in a pharmaceutically effective formulation.

Various particular embodiments of these embodiments are described below.

In one embodiment, the individual is afflicted with or susceptible to a KRAS-related disease.

In one embodiment, the KRAS-related disease is a proliferative disease, or cardio-facio-cutaneous (CFC) syndrome or Noonan syndrome.

In one embodiment, the KRAS-related disease is a proliferative disease, including without limitation a solid or liquid cancer, e.g., adenocarcinoma, colorectal cancer, advanced and/or metastatic colorectal cancer, colon cancer, lung, non-small cell lung cancer and lung adenocarcinoma, acute myelogenous lung, bladder, brain, breast, cervical, endometrial, gastric, head and neck, kidney, leukemia, myelodysplastic syndrome, myeloid leukemia, liver, melanoma, ovarian, pancreatic, prostate, testicular, thyroid cancers, or cardio-facio-cutaneous (CFC) syndrome or Noonan syndrome.

In one embodiment, the method further comprises the administration of an additional treatment. In one embodiment, the additional treatment is a therapeutically effective amount of a composition. In one embodiment, the additional treatment is a therapeutically relevant method (or procedure).

In one embodiment, the additional treatment and the RNAi agent can be administered in any order or can be administered simultaneously.

In one embodiment, the method further comprises the step of administering an additional treatment for a proliferative disease, cardio-facio-cutaneous (CFC) syndrome or Noonan syndrome.

In one embodiment, the composition comprises a second RNAi agent to KRAS. In various embodiments, the second RNAi agent is physically separate from the first, or the two are physically connected (e.g., covalently linked or otherwise conjugated).

In one embodiment, the method further comprises the step of administering an additional RNAi agent which comprises at least 15 contiguous nucleotides differing by 0, 1, 2, or 3 nucleotides from the antisense strand of a RNAi agent to KRAS selected from the specific duplexes provided herein and as listed, e.g., in Table 1.

**Pharmaceutical compositions of a RNAi agent to KRAS**

In one particular specific embodiment, the present disclosure relates to a composition comprising a RNAi agent of the present disclosure. In one embodiment, the RNAi agent comprises at least an anti-sense strand, and/or comprises a sense and an anti-sense strand, wherein the anti-sense strand comprises at least 15 contiguous nucleotides differing by 0, 1, 2, or 3 nucleotides from the anti-sense strand of a RNAi agent to KRAS selected from those specific duplex provided herein and as listed, e.g., in Table 1, wherein the composition is in a pharmaceutically effective formulation.

In one embodiment, the RNAi agent comprises at least an anti-sense strand, and/or comprises a sense and an anti-sense strand, wherein the sequence of the sense and/or anti-sense strand is the sequence of the sense and/or the anti-sense strand of a RNAi agent to KRAS selected from those specific duplex provided herein and as listed, e.g., in Table 1, wherein the composition is in a pharmaceutically effective formulation.

In one embodiment, the RNAi agent comprises at least an anti-sense strand, and/or comprises a sense and an anti-sense strand, wherein the sequence of the sense and/or anti-sense strand comprises the sequence of the sense and/or the anti-sense strand of a RNAi agent to KRAS selected from those specific duplex provided herein and as listed, e.g., in Table 1, wherein the composition is in a pharmaceutically effective formulation.

In one embodiment, the present disclosure pertains to the use of a RNAi agent in the manufacture of a medicament for treatment of a KRAS-related disease, wherein the RNAi agent comprises a sense strand and an antisense strand, wherein the antisense strand comprises at least 15 contiguous nucleotides differing by 0, 1, 2, or 3 nucleotides from the antisense strand of a RNAi agent to KRAS selected from those specific duplex provided herein and as listed, e.g., in Table 1.

Specific Embodiments of RNAi Agents to KRAS comprising mismatches from the disclosed sequences

Various specific embodiments of a RNAi agent to KRAS are disclosed herein. The present disclosure encompasses the example modified sequences provided in Tables 1 to 6, and the corresponding unmodified sequences and other modified sequences (e.g., modified and unmodified variants). Specific embodiments of the present disclosure include RNAi agents which comprise sequences differing by 0, 1, 2, or 3 nt (nucleotides) or bp [basepair(s)] (e.g., with 0, 1, 2 or 3 mismatches) from any of the RNAi agents listed in Table 1, and modified and unmodified variants thereof. As described in additional detail below, a mismatch is defined herein as a difference between the base sequence (e.g., A instead of G) or length when two sequences are maximally aligned and compared. In addition, as described in more detail below, an "unmodified variant" is a variant in which the base sequence is identical, but none of the bases are modified; this includes, for example, a sequence identical to the corresponding portion of the wild-type KRAS mRNA or gene. A "modified variant" contains one or more modifications (or one or more fewer or different modifications) to a nucleotide, sugar, phosphate or backbone, and/or addition of one or more moieties; but without a change, substitution, addition, or deletion to the base sequence. A particular sequence and its modified or unmodified variants have 0 mismatches among them.

In one particular embodiment, the present disclosure comprises a RNAi agent comprising a sense and an antisense strand, wherein the sense and/or anti-sense strand comprises at least 15 contiguous nucleotides differing by 0, 1, 2, or 3 nt from the sequence of the sense and/or anti-sense strand of: any of the RNAi agents listed in Tables 1 to 6, and modified and unmodified variants thereof.

In another particular embodiment, the RNAi agent comprises a sense strand comprising at least 15 contiguous nucleotides differing by 0, 1, 2, or 3 nt from the sense strand of any of the RNAi agents listed in Tables 1 to 6, and modified and unmodified variants thereof.

Other embodiments

Various particular specific embodiments of this disclosure are described below.

In one embodiment, the disclosure pertains to a composition according to any of the embodiments described herein, for use in a method of treating a KRAS-related disease in an individual, the method comprising the step of administering to the individual a therapeutically effective amount of a composition according to any of the claims.

One embodiment of the disclosure is the use of a composition according to any of these embodiments, in the manufacture of a medicament for treatment of an KRAS-related disease.

In one embodiment, the KRAS-related disease is a proliferative disease, or cardio-facio-cutaneous (CFC) syndrome or Noonan syndrome..

In one embodiment, the KRAS-related disease is a proliferative disease, including without limitation a solid or liquid cancer, adenocarcinoma, colorectal cancer, advanced and/or metastatic colorectal cancer, colon cancer, lung, non-small cell lung cancer and lung adenocarcinoma, acute myelogenous lung, bladder, brain, breast, cervical, endometrial, gastric, head and neck, kidney, leukemia, myelodysplastic syndrome, myeloid leukemia, liver, melanoma, ovarian, pancreatic, prostate, testicular, thyroid cancers, or cardio-facio-cutaneous (CFC) syndrome or Noonan syndrome.

In one embodiment, the disclosure pertains to the composition of any of the above embodiments, for use in the treatment of an KRAS-related disease.

Definitions

For convenience, the meaning of certain terms and phrases used in the specification, examples, and appended claims, are provided below. If there is an apparent discrepancy between the usage of a term in other parts of this specification and its definition provided in this section, the definition in this section shall prevail.

KRAS

By "KRAS" is meant the gene, mRNA, and/or protein, or any nucleic acid encoding the protein, or any mutant, variant or modified version thereof, designated by the Approved Gene Symbol: KRAS; Cytogenetic location: 12p12.1; Genomic coordinates (GRCh37): 12:25,358,179 - 25,403,853 (from NCBI), which also variously designated: KRas, K-ras, K-RAS, GTPase KRas, and V-Ki-ras2 Kirsten rat sarcoma viral oncogene homolog, C-K-RAS; K-RAS2A; K-RAS2B; K-RAS4A; K-RAS4B; KI-RAS; KRAS2; RASK2; v-Ki-ras2 Kirsten rat sarcoma viral oncogene homolog [Source: MGI Symbol; Acc: MGI: 96680]; OMIM: 190070 MGI: 96680 HomoloGene: 37990 GeneCards: KRAS Gene; Species Human: Entrez 3845; Ensembl ENSG00000133703; UniProt P01116; RefSeq (mRNA) NM_004985.3; RefSeq (protein) NP _004976.2; Location (UCSC) Chr 12: 25.36 - 25.4 Mb; Species-Mouse: Entrez-16653; Ensembl-ENSMUSG00000030265; UniProt-O70564; RefSeq (mRNA)-NM_021284.6; RefSeq (protein)-NP_067259.4; Location (UCSC)-Chr 6: 145.17 - 145.2 Mb.

The Ras subfamily

The Ras subfamily (an abbreviation of RAt Sarcoma) is a protein subfamily of small GTPases that are involved in cellular signal transduction and other processes. Activation of Ras signaling causes cell growth, differentiation and survival.

Since Ras communicates signals from outside the cell to the nucleus, mutations in ras genes can cause permanent activation and inappropriate transmission inside the cell, even in the absence of extracellular signals. Because these signals result in cell growth and division, dysregulated or otherwise aberrant Ras signaling can ultimately lead to oncogenesis and cancer. Goodsell et al. 1999. Oncologist 4. 3: 263-4. Activating mutations in Ras are found in a third of all human tumors and up to 90% in specific tumor types. Downward. 2003. Nat. Rev. Cancer 3. 1: 11-22; Bos et al. 1989 Cancer Res. 49: 4682-4689; and Forbes et al. 2006 Br. J. Cancer 94: 318-322

The ras genes were first identified as the transforming oncogenes [Malumbres et al. 2003. Nat. Rev. Cancer 3. 6: 459-65] responsible for the cancer-causing activities of the Harvey (the HRAS oncogene) and Kirsten (KRAS) sarcoma viruses, by Scolnick and colleagues at the National Institutes of Health (NIH). Chang et al. 1982. Proc. Natl. Acad. Sci. U.S.A. 79. 16: 4848-52. These viruses were discovered originally in rats during the 1960s by Jennifer Harvey [Harvey. 1964. Nature 204. 4963: 1104-5] and Werner Kirsten [Kirsten et al. 1970. Bibl Haematol. 36: 246-9], respectively. In 1982, activated and transforming human KRAS genes were discovered in human cancer cells [Santos et al. 1982. Nature 298. 5872: 343-7], [Parada et al. 1982. Nature 297. 5866: 474-8]. Subsequent studies identified a third human KRAS gene, designated NRAS, for its initial identification in human neuroblastoma cells.

The three human KRAS genes encode highly related 188 to 189 amino acid proteins, designated H-Ras, N-Ras and K-Ras4A and K-Ras4B (the two K-Ras proteins arise from alternative gene splicing). For additional information pertaining to KRAS cloning and isoforms, see, for example, Der et al. 1982 Proc. Nat. Acad. Sci. 79: 3637-3640; Chang et al. 1982 Proc. Nat. Acad. Sci. 79: 4848-4852; McCoy et al. 1983 Nature 302: 79-81; McGrath et al. 1983 Nature 304: 501-506; and Carta et al. 2006 Am. J. Hum. Genet. 79: 129-135.

Ras function

Ras proteins function as binary molecular switches that control intracellular signaling networks. Ras-regulated signal pathways, control such processes as actin cytoskeletal integrity, proliferation, differentiation, cell adhesion, apoptosis, and cell migration. Ras and ras-related proteins are often deregulated in cancers, leading to increased invasion and metastasis and decreased apoptosis.

Ras activates several pathways, of which the mitogen-activated protein (MAP) kinase cascade has been well-studied. This cascade transmits signals downstream and results in the transcription of genes involved in cell growth and division. There is a separate AKT pathway that inhibits apoptosis.

In various embodiments, the RNAi agents can be used to treat KRAS-related diseases in which any one or more of these pathways or functions is altered. In various embodiments, the present disclosure encompasses KRAS RNAi agents that modulate or alter Ras-regulated signal pathways, including the MAP kinase cascade, and/or modulate or alter processes such as actin cytoskeletal integrity, proliferation, differentiation, cell adhesion, apoptosis, and cell migration. Ras and ras-related proteins are often deregulated in cancers, leading to increased invasion and metastasis and decreased apoptosis.

Activation and deactivation

Ras is a G protein, or a guanosine-nucleotide-binding protein. Specifically, it is a single-subunit small GTPase, which is related in structure to the $G_\alpha$ subunit of heterotrimeric G proteins (large GTPases). G proteins function as binary signaling switches with "on" and "off" states. In the "off" state it is bound to the nucleotide guanosine diphosphate (GDP), while in the "on" state, Ras is bound to guanosine triphosphate (GTP), which has an extra phosphate group as compared to GDP. This extra phosphate holds the two switch regions in a "loaded-spring" configuration (specifically the Thr-35 and Gly-60). When released, the switch regions relax which causes a conformational change into the inactivate state. Hence, activation and deactivation of Ras and other small G proteins are controlled by cycling between the active GTP-bound and inactive GDP-bound forms.

Critical regions of KRAS for oncogenic activity include codons 12, 13, 59, 60, 61, 63 and 153. Grimmond et al. 1992 Urol. Res. 20:121-126; Burmer et al. 1989 Proc. Natl. Acad. Sci. 86: 2403-2407. Mutations sometimes appear affecting these codons. Some KRAS mutations cause RAS to accumulate in the GTP-bound state by impairing intrinsic GTPase activity and conferring resistance to GTPase-activating proteins. Zenker et al. 2007 J. Med. Genet. 44: 131-135. In various embodiments, the sequence of the KRAS RNAi agent encompasses the portion of the coding segment corresponding to any of the above-listed codons. In various embodiments, the KRAS RNAi agent has a mismatch to the wild-type (wt) KRAS sequence, such that the sequence of the KRAS RNAi agent matches the sequence of a mutated portion of the KRAS gene. In various embodiments, the disclosure encompasses the administration to a patient of a KRAS RNAi agent with a sequence corresponding to that of a particular mutation found in the patient, optionally combined with the administration of a KRAS RNAi agent with a sequence encompassing the wt sequence. In some embodiments, the KRAS RNAi agents correspond to different portions of the KRAS mRNA. In some embodiments, the patient may have one or more mutations corresponding to particular codons of KRAS (e.g., codons 12, 13 and/or 59), but the remainder of the KRAS gene is wild type, and thus the patient can be administered a KRAS RNAi agent with a wild-type sequence which does not span the mutation, and/or administered a KRAS RNAi agent with a sequence corresponding to the one or more mutations.

In some embodiments, the disclosure encompasses a method involving the steps of identifying a patient with a KRAS-related disease, isolating a nucleic acid encoding or corresponding to the KRAS protein or gene from the patient, determining the sequence of the nucleic acid to determine if there is a mutation in the KRAS gene, and, if the sequence indicates a mutation in KRAS, producing a KRAS RNAi agent with a sequence which corresponds to the mutation, and administering the KRAS RNAi agent in a therapeutically effective amount to treat or ameliorate the KRAS-related disease. In some embodiments, the KRAS RNAi agent is administered along with or as part of a treatment regimen with another disease treatment appropriate to the KRAS-related disease.

The process of RAS exchanging the bound nucleotide is facilitated by guanine nucleotide exchange factors (GEFs) and GTPase activating proteins (GAPs). As per its classification, Ras has an intrinsic GTPase activity, which means that the protein on its own will hydrolyze a bound GTP molecule into GDP. However this process is too slow for efficient function, and hence the GAP for Ras, RasGAP, may bind to and stabilize the catalytic machinery of Ras, supplying additional catalytic residues ("arginine finger") such that a water molecule is optimally positioned for nucleophilic attack on the gamma-phosphate of GTP. An inorganic phosphate is released and the Ras molecule is now bound to a GDP. Since the GDP-bound form is "off" or "inactive" for signaling, GTPase Activating Protein inactivates Ras by activating its GTPase activity. Thus, GAPs accelerate Ras inactivation.

GEFs catalyze a "push and pull" reaction which releases GDP from Ras. They insert close to the P-loop and magnesium cation binding site and inhibit the interaction of these with the gamma phosphate anion. Acidic (negative) residues in switch II "pull" a lysine in the P-loop away from the GDP, which "pushes" switch I away from the guanine. The contacts holding GDP in place are broken and it is released into the cytoplasm. Because intracellular GTP is abundant relative to GDP (approximately 10 fold more). GTP predominantly re-enters the nucleotide binding pocket of Ras and reloads the spring. Thus GEFs facilitate Ras activation. Well known GEFs include Son of Sevenless (Sos) and cdc25 which include the RasGEF domain.

The balance between GEF and GAP activity determines the guanine nucleotide status of Ras, thereby regulating Ras activity.

In the GTP-bound conformation, Ras has high affinity for numerous effectors which allow it to carry out its functions. These include PI3K. Other small GTPases may bind adaptors such as arfaptin or second messenger systems such as adenylyl cyclase. The Ras binding domain is found in many effectors and invariably binds to one of the switch regions, because these change conformation between the active and inactive forms. However, they may also bind to the rest of the protein surface.

In various embodiments, the present disclosure encompasses KRAS RNAi agents that modulate the amount of KRAS and/or the amount of activated KRAS in a cell. In various other embodiments, the present disclosure encompasses a composition or pharmaceutical composition comprising and/or a method of treatment involving one or more KRAS RNAi agents and a second agent (e.g., another RNAi agent, antibody, or small molecule) which targets a factor which interacts with KRAS (e.g., PI3K, GAP, etc.).

**Ras in cancer**

Mutations in the Ras family of proto-oncogenes are very common. A pharmacological approach that curtails Ras activity can inhibit certain cancer types. Ras point mutations are the single most common abnormality of human proto-oncogenes. Pathologic Basis of Disease 8th ed. 2010. p. 282. Ras inhibitor trans-farnesylthiosalicylic acid (FTS, Salirasib) exhibits profound anti-oncogenic effects in many cancer cell lines. Rotblat et al. 2008. Methods Enzymol 439: 467-89; and Blum et al. 2005. Cancer Research 65. 3: 999-1006.

In various embodiments, KRAS RNAi agents disclosed herein can be introduced into tumor cells with a point mutation in KRAS or other mutation causing an over-expression or hyper-activity of KRAS. Such cells are termed "KRAS-defective" cells.

Inappropriate activation of the gene has been shown to play a key role in signal transduction, proliferation and malignant transformation. Lodish et al. 2000. Chapter 25. Cancer. Molecular cell biology. 4th ed. San Francisco: W.H. Freeman.

Mutations in a number of different genes as well as KRAS itself can also have this effect. Oncogenes such as p210BCR-ABL or the growth receptor erbB are upstream of Ras, so if they are constitutively activated their signals will transduce through Ras.

The tumour suppressor gene NF1 encodes a Ras-GAP - its mutation in neurofibromatosis will mean that Ras is less likely to be inactivated. RNAi agents targeting KRAS can thus be used in conjunction with other agents (e.g., other RNAi agents) which target other factors which effect KRAS and its various pathways.

Finally, Ras oncogenes can be activated by point mutations so that the GTPase reaction can no longer be stimulated by GAP - this increases the half life of active Ras-GTP mutants.

In various embodiments, the KRAS RNAi agent can be administered to patients having a KRAS-related diseases involving a mutation outside KRAS, such as in p210BCR-ABL, erbB, or NF1. In various other embodiments, the present disclosure encompasses a composition or pharmaceutical composition or treatment method involving both one or more KRAS RNAi agents and one or more agents (e.g., a RNAi agent, antibody or small molecule) which targets another factor which causes inappropriate activation of KRAS, such as in p210BCR-ABL, erbB, or NF1.

**Constitutively active Ras**

Constitutively active Ras ($Ras^D$) is one which contains mutations that prevent GTP hydrolysis, thus locking Ras in a permanently 'On' state.

The most common mutations are found at residue G12 in the P-loop and the catalytic residue Q61.

The glycine to valine mutation at residue 12 renders the GTPase domain of Ras insensitive to inactivation by GAP and thus stuck in the "on state". Ras requires a GAP for inactivation as it is a relatively poor catalyst on its own, as opposed to other G-domain-containing proteins such as the alpha subunit of heterotrimeric G proteins.

Residue 61 is responsible for stabilizing the transition state for GTP hydrolysis. Because enzyme catalysis in general is achieved by lowering the energy barrier between substrate and product, mutation of Q61 to K necessarily reduces the rate of intrinsic Ras GTP hydrolysis to physiologically meaningless levels.

In various embodiments, the present disclosure pertains to a method of treatment of a patient having or susceptible to a KRAS-related disease, wherein the KRAS is constitutively active.

**KRAS sequences in various species**

The sequence of KRAS gene has determined for various species, including Human (HGNC: 6407; NM_004985.3); Mouse (MGI:96680); and Rat (RGD:2981). A RNAi agent specific to KRAS can be designed such that the sequence thereof completely matches that of the mRNA corresponding to the human KRAS gene and the homologous gene from a test animal. This is advantageous when developing a therapeutic RNAi agent since the same RNAi agent can be used in both a test animal (e.g., rat, mouse, or cynomolgus monkey, etc.) and a human.

The KRAS sequence in cynomolgus monkey (*Macaca fascicularis,* or "cyno") has been partially determined. Shown below are the partial cyno sequence and comparisons beween portions of the cyno and human KRAS sequences. NM_004985 KRAS, v-Ki-ras2 Kirsten rat sarcoma viral oncogene homolog consensus -- target length = 5312, consensus length = 4474, 23 contigs (longest is 775) 113 mismatches, 1651.87410068346 reads, coverage: max = 96, mean = 13.158; consensus consistency = 99.41 %, target/consensus conservation 97.47%.

KRAS cynomolgus sequence:

```
NNNNNNNNNN NNNNNNNNNN NNNNNNNNNN NNNNNNNNNN NNNNNNNNNN NNNNNNNNNN   60
NNNNNNNNNN NNNNNNNNNN NNNNNNNNNN NNNNNNNNNN NNNNNNNNNN NNAGGCACTG  120
AAGGCGGCGG CGGGGCCAGA GGCTCAGCGG CTCCCCGGGG CGGGAGAGAG NNNNNNNNNN  180
NNNNNNNNNN NNNNNNNNNN NNNNAGTTGG AGCTGGTGGG GTAGGCAAGA GTGCCTTGAC  240
NNNNNNGCTA ATTCAGAATC ATTTTGTGGA CGAATATGAT CCAACAATAG AGGATTCCTA  300
CAGGAAGCAA GTAGTAATTG ATGGAGAAAC CTGTCTCTTG GATATTCTCG ACACAGCAGG  360
TCAAGAGGAG TACAGTGCAA TGAGGGACCA GTACATGAGG ACTGGGGAGG GCTTTCTTTG  420
TGTATTTGCC ATAAATAATA CTAAATCATT TGAAGATATT CACCATTATA GAGAACAAAT  480
TAAAAGAGTT AAGGACTCTG AAGATGTACC TATGGTCCTA GTAGGAAATA AATGTGATTT  540
GCCTTCTAGA ACAGTAGACA CAAAACAGGC TCAGGACTTA GCAAGAAGTT ACGGAATTCC  600
TTTTATTGAA ACATCAGCAA AGACAAGACA GGGTGTTGAT GATGCCTTCT ATACATTAGT  660
TCGAGAAATT CGAAAACATA AAGAAAAGAT GAGCAAAGAT GGTAAAAAGA AGAAAAAGAA  720
GTCAAAGACA AAGTGTGTAA TTATGTAAAT ACAATTTGTA CTTTTTTCTT AAGGCATACT  780
AGTAAAAGTG GTAATTTTTG TACATTACAC TAAATTATTA GCATTTGTTT TAGCATTACC  840
TAATTTTTTT CCTGCTCCAT GCAGACTGTT AGCTTTTACC TTAAATGCTT ATTTTAAAAT  900
GACAGTGGAA GCTTTTTTTT CCTCTAAGTG CCAGTATTCC CAGAGTTTTG ATTTTTGAAC  960
TAGCAATGCC TGTGAAAAAG AAACTGAATA CCTAAGATTT CTGTCTTGGG GTTTTTGGTG 1020
CNNNNNNNNN NNNNNNNNNN NNNNNNNNNN NNNNNNNNNN NNNNNNNNNN NNNNNNAATT 1080
AATGAAGCTT CTGAATCATC CCTATTCTGT GTTTTATCTA GTCACATAAA TGGATTAATT 1140
ACTAATTTCA GTTGAGACCT TCTAATTGGT TTTTACTGAA ACATTGAGGG AACACAAANN 1200
NNNNNNNNNN NNNNNNNNNN NNNNTCTAGG CATCATGTCC TATAGTTTGT CATCCCTGAT 1260
GAATGTAAAG TTACACTGTT CACAAAGGTT TTGTTCCCTT TCCACTGCTA TTAGTCATGG 1320
```

TCACTCTCCC CGNNNNNNNN NNNNNNNNNN NNNNNNNNNN NNNNNTGGAA AAAAATTACA 1380
AGTCAATGGA AACTATTATA AGGCCGTTTC CTTTTCACAT TAGATAAATT ACTATAAAGA 1440
CTCCTAATAG CTTTTCCTGT TAAGGCAGAC CCAGTATGAA ATGGGGATTN NNNNNNNNNN 1500
NNNNNNNNNN NNNNNNNNNN NNNNNNNNNA ATTTTTATAA TAATTGAAAT AATTTTAACA 1560
TGTATAAAAA ATTCTCATAG GAATTAAATG TAGTCTCCCT GTGTCAGATT GCTCTTTCAT 1620
AGTATAACTT TAAATCTTTT CTTCAACTTC AGTCTTTGAA GGTAGTTTTA ATTCTGCTTG 1680
TGACATTAAA AGATTATTTG GGCCAGTTAT AGCTTAGTAG GTGTTGAAGA GACCAAGGTT 1740
GCATGGCCAG GCCCTGTGTG AACCTTTGAG CTTTCATAGA GAGTTTCACA GCATGGACTG 1800
TGTCCCCATG GTCATCCAGT GTTGTCATGC ATTGGTTAGT CAAAATGGGG AGGGACTAGG 1860
ACAGTTTGGA TAGGTCAACA AGATACATTC TCACTCTGTG GTGGTCCTGC TGACAAATCA 1920
AGAGCATTGC TTTTGTTTTT TAAGAAAATG AACTCTTTTT TAAAAATTAC TTTTAAATAT 1980
TAACTCAAAA GTTGAGATTT TGGGGGGGTG GTATGCCAAG ACATTAATTT TTTTNNNNNN 2040
NNNNNNNNNN NNNAAGTTTT ACAATCTCTA GGTTTGGCTA GTTCTCTTAA CACTGGTTAA 2100
ATTAACATTT CATAAACACT TTTCAAGNNN NNNNNNNNNN NNNNNNNNNN NNNNNNNNNN 2160
NNNNNNNNNN NNNNNNNNNN NNNATTTAAA ATGTTACTTA TTTTAAAATA TATGAAATGA 2220
GATGGCATGG TGAGGTGAAA GTATCACTGG ACTANNAAGA AGGTGACTTA GGTTCTAGAT 2280
AGTTGTCTTT TAGGACTCTG ATTTTGAGGA CATCACTTAC TATCCATTTC TTCATGTTAA 2340
NNNNNNNNNN NNNNNNNNNN NNNNNNNNNN TTTTTACAAC TATGTGATTT ATATTCCGTT 2400
TACATGAGGA TACACTTATT TGTCAAGCTC AGCACAATCT AAAAATTTTT AACCTATGTT 2460
ACACCATCTT CAGTGCCAAT CTTGGGCAAA ATTGTGCAAG AGGTGAAGTT TATATTTGAA 2520
TATCCATTCT CGTTTTAGGA CTCTCCTTCC ATGTTAGTGT CATCTTGCCT GCCTACCTTC 2580
CACATGCCCC ATGACTTGAT GCAGTTTTAA TACTTCTAAT TCCCCTAACC ATAAGATTTA 2640
CTGCTGCTAT GGATATCTCC ATGAAGTTTT CCCNNNNNNN NNNNNNNNNN NNNNNNNNNN 2700
NNNNNNNNNN NNNNNNNNNN NGAGAATCTG ACAGATACCA TAATGGGATT TGACCTAATC 2760
ACTAATTTTC AGGTGGTGGC CGATCCTTTG AAAATATCTC TGCTGCCCAA TCCATTAGCG 2820
ACAGTAGGAT TTTTCAGACC TGGTATGAAT AGACAGAACC CTATCCAGTG GAAGGAGAAT 2880
TTAATAAAGA TAGTACTGAA ATAATTCCTA AGGTAATCTA CAACTAGGAC TACTCCTGGT 2940
AACAGTAATA CATTCCATTG TTTTAGTAAC CAGAAATNNN NNNNNNNNNN NNNNNNNNNN 3000
NNNNNNNNNN NNNNNNNNNN NNNNNNNNNN NNNNNNNNNN NNNNNNNNNN NNCCAGGCTG 3060
GAATGCAGTG GCGCCATCTC AGCTCACTGC AACCTCCATC TCCCAGGTTC AAGCGATTCT 3120
CGTGCCTCGG CATCCTGAGT AGCTGGGATT ACAGGCATGT GCCNCCACAC TCAACTAATT 3180
TTTGTAGTTT TAGGAGAGAC AGGGTTTCAC CCTATTGGCC AGGCTGGTCT CGAACTCCTG 3240
ACCTCAAGTG ATTCACCCAC CTNNNCCTCA TAAAGCCGTT TTGCAGAACT CATTTATTCA 3300
GCAAATATTT ATTGAGTGCC TACCAGATGC CAGTCATTTC ACAAGGCACT GGGTATATGG 3360

TATCCCCAAA CAAGAGACAT AATCCTGGTC CTTAGCTAGT GCTAGCNNNN NNNNNNNNNN 3420
NNNNNNNNNN NNNNNNNNNN NNNNNNNNNN NNNNNNNNNN NNNNNNNNNN NNNNNNNNNN 3480
NNNNGGGGTT TGGTCCCTGT GCCTGCTCTA TAATTGTTTT GCTATGATTC CAGTGAAANN 3540
NNNNNNNNNN NNNNNNNNNN NNNNNNNNNN NNNNNNNNNN NNNNNNNNNN NNNNNNNNNN 3600
NNNNNNNNNN NNNNNNNNNN NNNNNNNNNN NNNNNNNNNN NNTTACTGTA CACATTAAGG 3660
TGTATGTCAG ATATCCATAT TGAANNNNNN NNNNNNNNNN NNNNNNNNNN NNNNNNNNAT 3720
AATTAAAATA TACTTAAAAA TTAATAGTTT TATCTGGGTA CAAACAAACA GGTGCCTGAA 3780
CTAGTTCACA GACAAGGAAA CTTCTATGTA AAAATCAGTA TGATTTNNNN NNNNNNNNGT 3840
GAAACTACAG ATCTTTGGAA CATTGTTTAG ATAGGGTGTT AAGACTTACA CAGTACCACG 3900
TTTCTACACA CAGAAAGAAA TGGCCATACT TCAGGAACTG CAGTGCTAAT GAGGGGATAT 3960
TTAGGCCTCT TGAATTTTTG ATGTAGATGG GCATTTTTTT AAGGTANNTG GTAATTACCT 4020
TTATGTGAAC TTTGAATGGT TTAACAAAAG ATTTGTTTTT GTAGAGATTT TAAAGGGGGA 4080
GAATGCTAGA AATAAATGTT ATCTAANNNN NNNCGCCTTA AAGATAAAAA TCCTTGTTGA 4140
AGTTTTTTAA AAAAAAGCTA AATTACATAG TCTTAGGCAT TAACATGTTT GTGGAAGAAT 4200
ATAGCACACA TATATTGTAT CATTTGAGTG AATATTCCCA AATAGGCATT CTAGGCTCTA 4260
TTTAACTGAG TCACACTGCA TAGGAATTTA GAACCTAACT TTTATAGGTT ATCAAAACTG 4320
TTGCCTCCAT TGCACAATTT TGTCCTAATA CATACATGGA GACTTTGTGG GGCATGTTAA 4380
GTTACAGTTT GCACAAGTTC ATCTCATTTG TATTCCATTG ATTTTTTTTT TCTTCTAAAC 4440
ATTTTTTCTT CAAACAGTAT ATAACTTTTT TTAGGGGATT TTTTTTNNNN NNNNNNNNNN 4500
NNTCTGACGA TTTCCATTTG TCAAAAAGTA ATGATTTCTT GATAATTATG TAGTAATGTT 4560
TTTAAGAACC CAGCAGTTAC CTTAAAGCTG AATTTATATT TAATAACTTC TGTGTTAATA 4620
CTGGATAGCA TGAATTCTGC ATTGAGAAAC TGAATAGNNN NNNNNNNNNN NNNNNNNNNN 4680
NNNNNNNNNN NNNNNNNNCA TGAGTTCTTG AAGAATAGTC ATAACTAGAT TAAGATCTGT 4740
GTTTTAGTTT AATAGTTTGA AGTGCCTGTT TGGGATAATG ATAGGTAATT TAGATGAATT 4800
TAGGGGGAAA AAAAGTTATC TGCAGAAATG TTGAGGGCCC ATCTCTCCCC ACACACCCAC 4860
ATAGAGCTAA CTGGGTTACA GTGTTTTATC CGAAAGTTTC CAATTCCACT GTCTTGTGTT 4920
TTCATGTTGA AAATACTTTT GCATTTTTCC TTTGAGTGCC AATTTCTTAC TAGTACTATT 4980
TCTTAATGTA ACATGTTTAC CTGGAATGTA TTTTAACTAT TTTTGTATAG TGTAAACTGA 5040
AACATGCACA TTTTGTACAT TGTGCTTTCT TTTGTGGGAC ATATGCAGTG TGATCCAGTT 5100
GTTTTCCATC ATTTGGTTGC GCTGACCTAG GAATGTTGGT CATATCAAAC ATTAAAAATG 5160
ACCACTCTTT TAATTGAAAT TAACTTTTAA ATGTTTATAG GAGTATGTGC TGTGAAGTGA 5220
TCTAAAATTT GTAATATTTT TGTCATGAAC TGTACTACTC CTAATTATTG TAATGTAATA 5280

**[00184]** AAAATAGTTA CAGTGACAAA AAAAAAAANN NN 5312 [SEQ ID NO: 1709].

Comparisons between portions of the human sequence and the cyno sequence are presented below.

```
Human 113 AGGCACTGAAGGCGGCGGCGGGGCCAGAGGCTCAGCGGCTCCCAGGTGCGGGAGAGAGGC 172
          ||||||||||||||||||||||||||||||||||||||||||| || |||||||||||
Cyno  113 AGGCACTGAAGGCGGCGGCGGGGCCAGAGGCTCAGCGGCTCCCCGGGGCGGGAGAGAGNN 172

Human 173 CTGCTGAAAATGACTGAATATAAACTTGTGGTAGTTGGAGCTGGTGGCGTAGGCAAGAGT 232
                                          ||||||||||||||| ||||||||||||
Cyno  173 NNNNNNNNNNNNNNNNNNNNNNNNNNNNNNNNAGTTGGAGCTGGTGGGGTAGGCAAGAGT 232

Human 233 GCCTTGACGATACAGCTAATTCAGAATCATTTTGTGGACGAATATGATCCAACAATAGAG 292
          ||||||||      ||||||||||||||||||||||||||||||||||||||||||||||
Cyno  233 GCCTTGACNNNNNNGCTAATTCAGAATCATTTTGTGGACGAATATGATCCAACAATAGAG 292

Human 293 GATTCCTACAGGAAGCAAGTAGTAATTGATGGAGAAACCTGTCTCTTGGATATTCTCGAC 352
          ||||||||||||||||||||||||||||||||||||||||||||||||||||||||||||
Cyno  293 GATTCCTACAGGAAGCAAGTAGTAATTGATGGAGAAACCTGTCTCTTGGATATTCTCGAC 352

Human 353 ACAGCAGGTCAAGAGGAGTACAGTGCAATGAGGGACCAGTACATGAGGACTGGGGAGGGC 412
          ||||||||||||||||||||||||||||||||||||||||||||||||||||||||||||
Cyno  353 ACAGCAGGTCAAGAGGAGTACAGTGCAATGAGGGACCAGTACATGAGGACTGGGGAGGGC 412

Human 413 TTTCTTTGTGTATTTGCCATAAATAATACTAAATCATTTGAAGATATTCACCATTATAGA 472
          ||||||||||||||||||||||||||||||||||||||||||||||||||||||||||||
Cyno  413 TTTCTTTGTGTATTTGCCATAAATAATACTAAATCATTTGAAGATATTCACCATTATAGA 472

Human 473 GAACAAATTAAAAGAGTTAAGGACTCTGAAGATGTACCTATGGTCCTAGTAGGAAATAAA 532
          ||||||||||||||||||||||||||||||||||||||||||||||||||||||||||||
Cyno  473 GAACAAATTAAAAGAGTTAAGGACTCTGAAGATGTACCTATGGTCCTAGTAGGAAATAAA 532

Human 533 TGTGATTTGCCTTCTAGAACAGTAGACACAAAACAGGCTCAGGACTTAGCAAGAAGTTAT 592
          ||||||||||||||||||||||||||||||||||||||||||||||||||||||||||| 
Cyno  533 TGTGATTTGCCTTCTAGAACAGTAGACACAAAACAGGCTCAGGACTTAGCAAGAAGTTAC 592

Human 593 GGAATTCCTTTTATTGAAACATCAGCAAAGACAAGACAGGGTGTTGATGATGCCTTCTAT 652
          ||||||||||||||||||||||||||||||||||||||||||||||||||||||||||||
Cyno  593 GGAATTCCTTTTATTGAAACATCAGCAAAGACAAGACAGGGTGTTGATGATGCCTTCTAT 652

Human 653 ACATTAGTTCGagaaattcgaaaacataaagaaaagatgagcaaagatggtaaaaagaaG 712
          ||||||||||||||||||||||||||||||||||||||||||||||||||||||||||||
Cyno  653 ACATTAGTTCGAGAAATTCGAAAACATAAAGAAAAGATGAGCAAAGATGGTAAAAAGAAG 712

Human 713 aaaaagaagtcaaagacaaagTGTGTAATTATGTAAATACAATTTGTACTTTTTTCTTAA 772
          ||||||||||||||||||||||||||||||||||||||||||||||||||||||||||||
Cyno  713 AAAAAGAAGTCAAAGACAAAGTGTGTAATTATGTAAATACAATTTGTACTTTTTTCTTAA 772

Human 773 GGCATACTAGTACAAGTGGTAATTTTTGTACATTACACTAAATTATTAGCATTTGTTTA 832
```

```
           |||||||||||| |||||||||||||||||||||||||||||||||||||||||||||||
Cyno  773  GGCATACTAGTAAAAGTGGTAATTTTTGTACATTACACTAAATTATTAGCATTTGTTTTA 832

Human 833  GCATTACCTAAtttttttCCTGCTCCATGCAGACTGTTAGCTTTTACCTTAAATGCTTAT 892
           ||||||||||||||||||||||||||||||||||||||||||||||||||||||||||||
Cyno  833  GCATTACCTAATTTTTTTCCTGCTCCATGCAGACTGTTAGCTTTTACCTTAAATGCTTAT 892

Human 893  TTTAAAATGACAGTGGAAGttttttttCCTCTAAGTGCCAGTATTCCCAGAGTTTTGGT 952
           ||||||||||||||||||| |||||||||||||||||||||||||||||||||||||| |
Cyno  893  TTTAAAATGACAGTGGAAGCTTTTTTTTCCTCTAAGTGCCAGTATTCCCAGAGTTTTGAT 952

Human 953  TTTGAACTAGCAATGCCTGTGAAAAAGAAACTGAATACCTAAGATTTCTGTCTTGGGGT 1012
           ||||||||||||||||||||||||||||||||||||||||||||||||||||||||||||
Cyno  953  TTTGAACTAGCAATGCCTGTGAAAAAGAAACTGAATACCTAAGATTTCTGTCTTGGGGT 1012

Human 1013 TTTTGGTGCATGCAGTTGATTACTTCTTATTTTTCTTACCAATTGTGAATGTTGGTGTGA 1072
           |||||||||
Cyno  1013 TTTTGGTGCNNNNNNNNNNNNNNNNNNNNNNNNNNNNNNNNNNNNNNNNNNNNNNNNNNN 1072

Human 1073 AACAAATTAATGAAGCTTTTGAATCATCCCTATTCTGTGTTTTATCTAGTCACATAAATG 1132
               |||||||||||||| |||||||||||||||||||||||||||||||||||||||||
Cyno  1073 NNNNAATTAATGAAGCTTCTGAATCATCCCTATTCTGTGTTTTATCTAGTCACATAAATG 1132

Human 1133 GATTAATTACTAATTTCAGTTGAGACCTTCTAATTGGTTTTTACTGAAACATTGAGGGAA 1192
           ||||||||||||||||||||||||||||||||||||||||||||||||||||||||||||
Cyno  1133 GATTAATTACTAATTTCAGTTGAGACCTTCTAATTGGTTTTTACTGAAACATTGAGGGAA 1192

Human 1193 CACAAATTTATGGGCTTCCTGATGATGATTCTTCTAGGCATCATGTCCTATAGTTTGTCA 1252
           ||||||                      ||||||||||||||||||||||||||||
Cyno  1193 CACAAANNNNNNNNNNNNNNNNNNNNNNNNNNNNTCTAGGCATCATGTCCTATAGTTTGTCA 1252

Human 1253 TCCCTGATGAATGTAAAGTTACACTGTTCACAAAGGTTTTGTCTCCTTTCCACTGCTATT 1312
           ||||||||||||||||||||||||||||||||||||||||||  ||||||||||||||||
Cyno  1253 TCCCTGATGAATGTAAAGTTACACTGTTCACAAAGGTTTTGTTCCCTTTCCACTGCTATT 1312

Human 1313 AGTCATGGTCACTCTCCCCAAAATATTATATTTTTTCTATaaaaagaaaaaaatggaaaA 1372
           |||||||||||||||||||                              |||||||
Cyno  1313 AGTCATGGTCACTCTCCCCGNNNNNNNNNNNNNNNNNNNNNNNNNNNNNNNNNNTGGAAAA 1372

Human 1373 aaaTTACAAGGCAATGGAAACTATTATAAGGCCATTTCCTTTTCACATTAGATAAATTAC 1432
           |||||||||| |||||||||||||||||||||| ||||||||||||||||||||||||||
Cyno  1373 AAATTACAAGTCAATGGAAACTATTATAAGGCCGTTTCCTTTTCACATTAGATAAATTAC 1432

Human 1433 TATAAAGACTCCTAATAGCTTTTCCTGTTAAGGCAGACCCAGTATGAAATGGGGATTATT 1492
           |||||||||||||||||||||||||||||||||||||||||||||||||||||||||
Cyno  1433 TATAAAGACTCCTAATAGCTTTTCCTGTTAAGGCAGACCCAGTATGAAATGGGGATTNNN 1492
```

```
Human 1493 ATAGCAACCATTTTGGGGCTATATTTACATGCTACTAAATTTTTATAATAATTGAAAAGA 1552
                                                |||||||||||||||||||  |
Cyno  1493 NNNNNNNNNNNNNNNNNNNNNNNNNNNNNNNNNNNNNAATTTTTATAATAATTGAAATAA 1552

Human 1553 TTTTAACAAGTATAAAAAATTCTCATAGGAATTAAATGTAGTCTCCCTGTGTCAGACTGC 1612
           |||||||| |||||||||||||||||||||||||||||||||||||||||||||||| |||
Cyno  1553 TTTTAACATGTATAAAAAATTCTCATAGGAATTAAATGTAGTCTCCCTGTGTCAGATTGC 1612

Human 1613 TCTTTCATAGTATAACTTTAAATCTTTTCTTCAACTTGAGTCTTTGAAGATAGTTTTAAT 1672
           ||||||||||||||||||||||||||||||||||||| ||||||||||| ||||||||||
Cyno  1613 TCTTTCATAGTATAACTTTAAATCTTTTCTTCAACTTCAGTCTTTGAAGGTAGTTTTAAT 1672

Human 1673 TCTGCTTGTGACATTAAAAGATTATTTGGGCCAGTTATAGCTTATTAGGTGTTGAAGAGA 1732
           |||||||||||||||||||||||||||||||||||||||||||| |||||||||||||||
Cyno  1673 TCTGCTTGTGACATTAAAAGATTATTTGGGCCAGTTATAGCTTAGTAGGTGTTGAAGAGA 1732

Human 1733 CCAAGGTTGCAAGGCCAGGCCCTGTGTGAACCTTTGAGCTTTCATAGAGAGTTTCACAGC 1792
           ||||||||||| ||||||||||||||||||||||||||||||||||||||||||||||||
Cyno  1733 CCAAGGTTGCATGGCCAGGCCCTGTGTGAACCTTTGAGCTTTCATAGAGAGTTTCACAGC 1792

Human 1793 ATGGACTGTGTCCCCACGGTCATCCAGTGTTGTCATGCATTGGTTAGTCAAAATGGGGAG 1852
           |||||||||||||||| |||||||||||||||||||||||||||||||||||||||||||
Cyno  1793 ATGGACTGTGTCCCCATGGTCATCCAGTGTTGTCATGCATTGGTTAGTCAAAATGGGGAG 1852

Human 1853 GGACTAGGGCAGTTTGGATAGCTCAACAAGATACAATCTCACTCTGTGGTGGTCCTGCTG 1912
           |||||||| |||||||||||| ||||||||||||| ||||||||||||||||||||||||
Cyno  1853 GGACTAGGACAGTTTGGATAGGTCAACAAGATACATTCTCACTCTGTGGTGGTCCTGCTG 1912

Human 1913 ACAAATCAAGAGCATTGCTTTTGTTTCTTAAGAAAACAAACTCTTTTTAAAAATTACTT 1972
           |||||||||||||||||||||||||| |||||||||  ||||||||||||||||||||||
Cyno  1913 ACAAATCAAGAGCATTGCTTTTGTTTTTTAAGAAAATGAACTCTTTTTAAAAATTACTT 1972

Human 1973 TTAAATATTAACTCAAAAGTTGAGATTTTGGGGTGGTGGTGTGCCAAGACATTAAttttT 2032
           ||||||||||||||||||||||||||||||||| |||||| |||||||||||||||||||
Cyno  1973 TTAAATATTAACTCAAAAGTTGAGATTTTGGGGGGGTGGTATGCCAAGACATTAATTTTT 2032

Human 2033 tttttAAACAATGAAGTGAAAAAGTTTTACAATCTCTAGGTTTGGCTAGTTCTCTTAACA 2092
           ||                   |||||||||||||||||||||||||||||||||||||||
Cyno  2033 TTNNNNNNNNNNNNNNNNNNNAAGTTTTACAATCTCTAGGTTTGGCTAGTTCTCTTAACA 2092

Human 2093 CTGGTTAAATTAACATTGCATAAACACTTTTCAAG 2127 [SEQ ID NO: 1710]
           ||||||||||||||||| |||||||||||||||||
Cyno  2093 CTGGTTAAATTAACATTTCATAAACACTTTTCAAG 2127 [SEQ ID NO: 1711]
```

```
Human 2184  atttaaaatgttacttattttaaaataaatGAAGTGAGATGGCATGGTGAGGTGAAAGTA 2243
            |||||||||||||||||||||||||| |||||| ||||||||||||||||||||||||||
Cyno  2184  ATTTAAAATGTTACTTATTTTAAAATATATGAAATGAGATGGCATGGTGAGGTGAAAGTA 2243

Human 2244  TCACTGGACTAGGAAGAAGGTGACTTAGGTTCTAGATAGGTGTCTTTTAGGACTCTGATT 2303
            |||||||||||  |||||||||||||||||||||||||| ||||||||||||||||||||
Cyno  2244  TCACTGGACTANNAAGAAGGTGACTTAGGTTCTAGATAGTTGTCTTTTAGGACTCTGATT 2303

Human 2304  TTGAGGACATCACTTACTATCCATTTCTTCATGTTAAAAGAAGTCATCTCAAACTCTTAG 2363
            |||||||||||||||||||||||||||||||||||||
Cyno  2304  TTGAGGACATCACTTACTATCCATTTCTTCATGTTAANNNNNNNNNNNNNNNNNNNNNNN 2363

Human 2364  ttttttttttttACAACTATGTAATTTATATTCCATTTACATAAGGATACACTTATTTGT 2423
                   ||||||||||||||| ||||||||||| ||||||| |||||||||||||||||
Cyno  2364  NNNNNNNTTTTTACAACTATGTGATTTATATTCCGTTTACATGAGGATACACTTATTTGT 2423

Human 2424  CAAGCTCAGCACAATCTGTAAATTTTTAACCTATGTTACACCATCTTCAGTGCCAGTCTT 2483
            |||||||||||||||||  |||||||||||||||||||||||||||||||||||| ||||
Cyno  2424  CAAGCTCAGCACAATCTAAAAATTTTTAACCTATGTTACACCATCTTCAGTGCCAATCTT 2483

Human 2484  GGGCAAAATTGTGCAAGAGGTGAAGTTTATATTTGAATATCCATTCTCGTTTTAGGACTC 2543
            ||||||||||||||||||||||||||||||||||||||||||||||||||||||||||||
Cyno  2484  GGGCAAAATTGTGCAAGAGGTGAAGTTTATATTTGAATATCCATTCTCGTTTTAGGACTC 2543

Human 2544  TTCTTCCATATTAGTGTCATCTTGCCTCCCTACCTTCCACATGCCCCATGACTTGATGCA 2603
            | ||||||| ||||||||||||||||| ||||||||||||||||||||||||||||||||
Cyno  2544  TCCTTCCATGTTAGTGTCATCTTGCCTGCCTACCTTCCACATGCCCCATGACTTGATGCA 2603

Human 2604  GTTTAATACTTGTAATTCCCCTAACCATAAGATTTACTGCTGCTGTGGATATCTCCATG 2663
            ||||||||||| |||||||||||||||||||||||||||||||| |||||||||||||||
Cyno  2604  GTTTAATACTTCTAATTCCCCTAACCATAAGATTTACTGCTGCTATGGATATCTCCATG 2663

Human 2664  AAGTTTTCCCACTGAGTCACATCAGAAATGCCCTACATCTTATTTCCTCAGGGCTCAAGA 2723
            ||||||||||                                           ||
Cyno  2664  AAGTTTTCCCNNNNNNNNNNNNNNNNNNNNNNNNNNNNNNNNNNNNNNNNNNNNNNNNNNGA 2723

Human 2724  GAATCTGACAGATACCATAAAGGGATTTGACCTAATCACTAATTTTCAGGTGGTGGCTGA 2783
            |||||||||||||||||||| |||||||||||||||||||||||||||||||||||| ||
Cyno  2724  GAATCTGACAGATACCATAATGGGATTTGACCTAATCACTAATTTTCAGGTGGTGGCCGA 2783

Human 2784  TGCTTTGAACATCTCTTTGCTGCCCAATCCATTAGCGACAGTAGGATTTTTCAAACCTGG 2843
            | ||||||| || ||| |||||||||||||||||||||||||||||||||||| ||||||
Cyno  2784  TCCTTTGAAAATATCTCTGCTGCCCAATCCATTAGCGACAGTAGGATTTTTCAGACCTGG 2843

Human 2844  TATGAATAGACAGAACCCTATCCAGTGGAAGGAGAATTTAATAAAGATAGTGCTGAAAGA 2903
            ||||||||||||||||||||||||||||||||||||||||||||||||||| |||||| |
```

```
Cyno  2844 TATGAATAGACAGAACCCTATCCAGTGGAAGGAGAATTTAATAAAGATAGTACTGAAATA 2903

Human 2904 ATTCCTTAGGTAATCTATAACTAGGACTACTCCTGGTAACAGTAATACATTCCATTGTTT 2963
           |||||| |||||||||| ||||||||||||||||||||||||||||||||||||||||||
Cyno  2904 ATTCCTAAGGTAATCTACAACTAGGACTACTCCTGGTAACAGTAATACATTCCATTGTTT 2963

Human 2964 TAGTAACCAGAAAT 2977 [SEQ ID NO: 1712]
           ||||||||||||||
Cyno  2964 TAGTAACCAGAAAT 2977 [SEQ ID NO: 1713]


Human 3053 CCAGGCTGGAATGCAGTGGCGCCATCTCAGCTCACTGCAACCTCCATCTCCCAGGTTCAA 3112
           ||||||||||||||||||||||||||||||||||||||||||||||||||||||||||||
Cyno  3053 CCAGGCTGGAATGCAGTGGCGCCATCTCAGCTCACTGCAACCTCCATCTCCCAGGTTCAA 3112

Human 3113 GCGATTCTCGTGCCTCGGCCTCCTGAGTAGCTGGGATTACAGGCGTGTGCCACTACACTC 3172
           ||||||||||||||||||| |||||||||||||||||||||||| |||||| | ||||||
Cyno  3113 GCGATTCTCGTGCCTCGGCATCCTGAGTAGCTGGGATTACAGGCATGTGCCNCCACACTC 3172

Human 3173 AACTAATTTTTGTATTTTTAGGAGAGACGGGGTTTCACCCTGTTGGCCAGGCTGGTCTCG 3232
           |||||||||||||| ||||||||||||| |||||||||||| ||||||||||||||||||
Cyno  3173 AACTAATTTTTGTAGTTTTAGGAGAGACAGGGTTTCACCCTATTGGCCAGGCTGGTCTCG 3232

Human 3233 AACTCCTGACCTCAAGTGATTCACCCACCTTGGCCTCATAAACCTGTTTTGCAGAACTCA 3292
           ||||||||||||||||||||||||||||||   ||||||||| | |||||||||||||||
Cyno  3233 AACTCCTGACCTCAAGTGATTCACCCACCTNNNCCTCATAAAGCCGTTTTGCAGAACTCA 3292

Human 3293 TTTATTCAGCAAATATTTATTGAGTGCCTACCAGATGCCAGTCACCGCACAAGGCACTGG 3352
           ||||||||||||||||||||||||||||||||||||||||||||   |||||||||||||
Cyno  3293 TTTATTCAGCAAATATTTATTGAGTGCCTACCAGATGCCAGTCATTTCACAAGGCACTGG 3352

Human 3353 GTATATGGTATCCCCAAACAAGAGACATAATCCCGGTCCTTAGGTAGTGCTAG 3405
           ||||||||||||||||||||||||||||||||| ||||||||| |||||||||
Cyno  3353 GTATATGGTATCCCCAAACAAGAGACATAATCCTGGTCCTTAGCTAGTGCTAG 3405

Human 3643 TTACTGTACACATTAAGGTGTATGTCAGATATTCATATTGACCCAAATGTGTAATATTCC 3702
           |||||||||||||||||||||||||||||||| ||||||||
Cyno  3643 TTACTGTACACATTAAGGTGTATGTCAGATATCCATATTGAANNNNNNNNNNNNNNNNNN 3702

Human 3703 AGTTTTCTCTGCATAAGTAATTAAAATATACTTAAAAATTAATAGTTTTATCTGGGTACA 3762
                            |||||||||||||||||||||||||||||||||||||||||||
Cyno  3703 NNNNNNNNNNNNNNNNATAATTAAAATATACTTAAAAATTAATAGTTTTATCTGGGTACA 3762

Human 3763 AATAAACAGGTGCCTGAACTAGTTCACAGACAAGGAAACTTCTATGTAAAAATCACTATG 3822
           || |||||||||||||||||||||||||||||||||||||||||||||||||||| ||||
```

```
Cyno  3763 AACAAACAGGTGCCTGAACTAGTTCACAGACAAGGAAACTTCTATGTAAAAATCAGTATG 3822

Human 3823 ATTTCTGAATTGCTATGTGAAACTACAGATCTTTGGAACACTGTTTAGGTAGGGTGTTAA 3882
           ||||            |||||||||||||||||||||||| ||||||| |||||||||||
Cyno  3823 ATTTNNNNNNNNNNNNGTGAAACTACAGATCTTTGGAACATTGTTTAGATAGGGTGTTAA 3882

Human 3883 GACTTACACAGTACCTCGTTTCTACACAGAGAAAGAAATGGCCATACTTCAGGAACTGCA 3942
           ||||||||||||||| |||||||||||| |||||||||||||||||||||||||||||||
Cyno  3883 GACTTACACAGTACCACGTTTCTACACACAGAAAGAAATGGCCATACTTCAGGAACTGCA 3942

Human 3943 GTGCTTATGAGGGGATATTTAGGCCTCTTGAATTTTTGATGTAGATGGGCAtttttttAA 4002
           ||||| ||||||||||||||||||||||||||||||||||||||||||||||||||||||
Cyno  3943 GTGCTAATGAGGGGATATTTAGGCCTCTTGAATTTTTGATGTAGATGGGCATTTTTTTAA 4002

Human 4003 GGTAGTGGTTAATTACCTTTATGTGAACTTTGAATGGTTTAACAAAAGATTTGTTTTTGT 4062
           ||||   | |||||||||||||||||||||||||||||||||||||||||||||||||||
Cyno  4003 GGTANNTGGTAATTACCTTTATGTGAACTTTGAATGGTTTAACAAAAGATTTGTTTTTGT 4062

Human 4063 AGAGATTTTAAAGGGGGAGAATTCTAGAAATAAATGTTACCTAATTATTACAGCCTTAAA 4122
           |||||||||||||||||||||| |||||||||||||||| ||||        ||||||||
Cyno  4063 AGAGATTTTAAAGGGGGAGAATGCTAGAAATAAATGTTATCTAANNNNNNNCGCCTTAAA 4122

Human 4123 GACAAAAATCCTTGTTGAAGtttttttaaaaaaaGCTAAATTACATAGACTTAGGCATTA 4182
           || ||||||||||||||||||||||| ||||||||||||||||||||| |||||||||||
Cyno  4123 GATAAAAATCCTTGTTGAAGTTTTTTAAAAAAAAGCTAAATTACATAGTCTTAGGCATTA 4182

Human 4183 ACATGTTTGTGGAAGAATATAGCAGACGTATATTGTATCATTTGAGTGAATGTTCCCAAG 4242
           |||||||||||||||||||||||| || ||||||||||||||||||||||| ||||||| 
Cyno  4183 ACATGTTTGTGGAAGAATATAGCACACATATATTGTATCATTTGAGTGAATATTCCCAAA 4242

Human 4243 TAGGCATTCTAGGCTCTATTTAACTGAGTCACACTGCATAGGAATTTAGAACCTAACTTT 4302
           ||||||||||||||||||||||||||||||||||||||||||||||||||||||||||||
Cyno  4243 TAGGCATTCTAGGCTCTATTTAACTGAGTCACACTGCATAGGAATTTAGAACCTAACTTT 4302

Human 4303 TATAGGTTATCAAAACTGTTGTCACCATTGCACAATTTTGTCCTAATATATACATAGAAA 4362
           ||||||||||||||||||||| | |||||||||||||||||||||||| |||||| || |
Cyno  4303 TATAGGTTATCAAAACTGTTGCCTCCATTGCACAATTTTGTCCTAATACATACATGGAGA 4362

Human 4363 CTTTGTGGGGCATGTTAAGTTACAGTTTGCACAAGTTCATCTCATTTGTATTCCATTGAT 4422
           ||||||||||||||||||||||||||||||||||||||||||||||||||||||||||||
Cyno  4363 CTTTGTGGGGCATGTTAAGTTACAGTTTGCACAAGTTCATCTCATTTGTATTCCATTGAT 4422

Human 4423 tttttttttcttctaaacatttttcttcaaacagtatataactttttttaggggatttT 4482
           ||||||||||||||||||||||||||||||||||||||||||||||||||||||||||||
Cyno  4423 TTTTTTTTTCTTCTAAACATTTTTCTTCAAACAGTATATAACTTTTTTTAGGGGATTTT 4482
```

```
Human 4483 tttttAGACAGCAAAAACTATCTGAAGATTTCCATTTGTCAAAAAGTAATGATTTCTTGA 4542
           ||||                |||||  ||||||||||||||||||||||||||||||||||
Cyno  4483 TTTTNNNNNNNNNNNNNNNNTCTGACGATTTCCATTTGTCAAAAAGTAATGATTTCTTGA 4542

Human 4543 TAATTGTGTAGTAATGTTTTTTAGAACCCAGCAGTTACCTTAAAGCTGAATTTATATTTA 4602
           ||||| ||||||||||||||| ||||||||||||||||||||||||||||||||||||||
Cyno  4543 TAATTATGTAGTAATGTTTTTAAGAACCCAGCAGTTACCTTAAAGCTGAATTTATATTTA 4602

Human 4603 GTAACTTCTGTGTTAATACTGGATAGCATGAATTCTGCATTGAGAAACTGAATAGCTGTC 4662
            ||||||||||||||||||||||||||||||||||||||||||||||||||||||     
Cyno  4603 ATAACTTCTGTGTTAATACTGGATAGCATGAATTCTGCATTGAGAAACTGAATAGNNNNN 4662

Human 4663 ATAAAATGAAACTTTCTTTCTAAAGAAAGATACTCACATGAGTTCTTGAAGAATAGTCAT 4722
                                               ||||||||||||||||||||||||
Cyno  4663 NNNNNNNNNNNNNNNNNNNNNNNNNNNNNNNNNNNNCATGAGTTCTTGAAGAATAGTCAT 4722

Human 4723 AACTAGATTAAGATCTGTGTTTTAGTTTAATAGTTTGAAGTGCCTGTTTGGGATAATGAT 4782
           ||||||||||||||||||||||||||||||||||||||||||||||||||||||||||||
Cyno  4723 AACTAGATTAAGATCTGTGTTTTAGTTTAATAGTTTGAAGTGCCTGTTTGGGATAATGAT 4782

Human 4783 AGGTAATTTAGATGAATTTAGGGGaaaaaaaaGTTATCTGCAGATATGTTGAGGGCCCAT 4842
           |||||||||||||||||||||||| ||||||||||||||||||| |||||||||||||||
Cyno  4783 AGGTAATTTAGATGAATTTAGGGGGAAAAAAAGTTATCTGCAGAAATGTTGAGGGCCCAT 4842

Human 4843 CTCTCCCCCCACACCCCCACAGAGCTAACTGGGTTACAGTGTTTTATCCGAAAGTTTCCA 4902
           |||||||| ||||||| || ||||||||||||||||||||||||||||||||||||||||
Cyno  4843 CTCTCCCCACACACCCACATAGAGCTAACTGGGTTACAGTGTTTTATCCGAAAGTTTCCA 4902

Human 4903 ATTCCACTGTCTTGTGTTTTCATGTTGAAAATACTTTTGCATTTTTCCTTTGAGTGCCAA 4962
           ||||||||||||||||||||||||||||||||||||||||||||||||||||||||||||
Cyno  4903 ATTCCACTGTCTTGTGTTTTCATGTTGAAAATACTTTTGCATTTTTCCTTTGAGTGCCAA 4962

Human 4963 TTTCTTACTAGTACTATTTCTTAATGTAACATGTTTACCTGGAATGTATTTTAACTATTT 5022
           ||||||||||||||||||||||||||||||||||||||||||||||||||||||||||||
Cyno  4963 TTTCTTACTAGTACTATTTCTTAATGTAACATGTTTACCTGGAATGTATTTTAACTATTT 5022

Human 5023 TTGTATAGTGTAAACTGAAACATGCACATTTTGTACATTGTGCTTTCTTTTGTGGGACAT 5082
           ||||||||||||||||||||||||||||||||||||||||||||||||||||||||||||
Cyno  5023 TTGTATAGTGTAAACTGAAACATGCACATTTTGTACATTGTGCTTTCTTTTGTGGGACAT 5082

Human 5083 ATGCAGTGTGATCCAGTTGTTTTCCATCATTTGGTTGCGCTGACCTAGGAATGTTGGTCA 5142
           ||||||||||||||||||||||||||||||||||||||||||||||||||||||||||||
Cyno  5083 ATGCAGTGTGATCCAGTTGTTTTCCATCATTTGGTTGCGCTGACCTAGGAATGTTGGTCA 5142

Human 5143 TATCAAACATTAAAAATGACCACTCTTTTAATTGAAATTAACTTTTAAATGTTTATAGGA 5202
           ||||||||||||||||||||||||||||||||||||||||||||||||||||||||||||
```

```
Cyno  5143 TATCAAACATTAAAAATGACCACTCTTTTAATTGAAATTAACTTTTAAATGTTTATAGGA 5202

Human 5203 GTATGTGCTGTGAAGTGATCTAAAATTTGTAATATTTTTGTCATGAACTGTACTACTCCT 5262
           ||||||||||||||||||||||||||||||||||||||||||||||||||||||||||||
Cyno  5203 GTATGTGCTGTGAAGTGATCTAAAATTTGTAATATTTTTGTCATGAACTGTACTACTCCT 5262

Human 5263 AATTATTGTAATGTAATAAAAATAGTTACAGTGACaaaaaaaaaaA 5308
           ||||||||||||||||||||||||||||||||||||||||||||||
Cyno  5263 AATTATTGTAATGTAATAAAAATAGTTACAGTGACAAAAAAAAAAA 5308
```

Human [SEQ ID NO: 1714]

Cyno [SEQ ID NO: 1715]

The lowercase letters in the KRAS sequence are an NCBI convention for low-complexity sequences. The matching algorithm frequently excludes these when calculating match scores, as they tend to match against large numbers of things in the genome.

In one embodiment, the KRAS RNAi agent of the present disclosure comprises a sequence which is identical in the all three of human, rat and cyno KRAS mRNAs. This sequence identity across species facilitates animal testing prior to human testing, since the sequence of the RNAi does not need to be modified due to species-specific differences in the target sequence. In one embodiment, the KRAS RNAi agent comprises a sequence which is identical in the human, mouse and cyno KRAS mRNAs. In various embodiments, the KRAS RNAi agent comprises a sequence which is identical in human and mouse, human and rat, and/or human and cyno.

### Additional embodiments of a RNAi agent to KRAS

The present disclosure pertains to RNAi agents which target human KRAS. In one embodiment, the KRAS RNAi agent comprises a sequence which does not match that of any other mRNA or gene. In one embodiment, the KRAS RNAi agent comprises a sequence which differs from all other known non-KRAS mRNAs or genes by at least 0, 1, 2 or 3 nucleotides. An increase in the number of mismatches between the KRAS RNAi sequence and that of any other gene can decrease the amount of "off-target effects" or the RNAi agent targeting a non-target gene (e.g., a gene that is not KRAS).

In one embodiment, the KRAS RNAi agent of the present disclosure is administered to a patient in need thereof (e.g., a patient suffering from a KRAS-related disease).

The patient can also be administered more than one RNAi agent specific to KRAS. In one embodiment, the KRAS RNAi agent(s) of the present disclosure can optionally be administered along with one or more additional pharmaceutical agent appropriate for that disease. In one embodiment, the KRAS RNAi agent(s) of the present disclosure can be optionally administered along with any other appropriate additional treatment, wherein the additional treatment can be a composition (e.g., another agent that can treat a KRAS-related disease such as a cancer treatment) or a method (e.g., surgery to remove a tumor if the KRAS-related disease is a cancer).

The RNAi agent(s) and additional disease treatment(s) can be administered in any order, simultaneously, concurrently, separately, or sequentially, or in one or multiple doses over time.

### Additional Definitions

For convenience, the meaning of certain terms and phrases used in the specification, examples, and appended claims, are provided below. If there is an apparent discrepancy between the usage of a term in other parts of this specification and its definition provided in this section, the definition in this section shall prevail.

As used throughout this disclosure, articles such as "a" and "an" refer to one or more than one (at least one) of the grammatical object of the article.

### RNAi Agent

In one embodiment, the present disclosure pertains to a KRAS RNAi agent or other composition comprising at least an antisense nucleic acid sequence complementary to a KRAS nucleic acid (or portion thereof), or pertains to a recombinant expression vector encoding an shRNA or composition comprising the antisense nucleic acid that can function as an RNAi as defined below. As used herein, an "antisense" nucleic acid comprises a nucleotide sequence complementary to a "sense" nucleic acid encoding the KRAS protein (e.g., complementary to the coding strand of a double-stranded DNA, complementary to an mRNA or complementary to the coding strand of a KRAS gene or nucleic acid).

RNAi agents include, as non-limiting examples, siRNAs (small interfering RNAs), dsRNAs (double stranded RNAs), shRNAs (short hairpin RNAs) and miRNAs (micro RNAs).

In various embodiments, the present disclosure pertains to any RNAi agent comprising a RNA sequence corresponding to any DNA sequences disclosed herein (e.g., wherein the DNA nucleotides are replaced by the corresponding RNA nucleotide, for example, with T in DNA replaced by U in RNA, and with ribose instead of deoxyribose in the sugar-phosphate backbone).

In one embodiment, the RNAi comprises a single strand. This single-stranded RNAi agent oligonucleotide or polynucleotide can comprise the sense or antisense strand, as described by Sioud 2005 J. Mol. Biol. 348:1079-1090, and references therein. Thus the disclosure encompasses RNAi agents with a single strand comprising either the sense or antisense strand of an RNAi agent described herein. The disclosure also encompasses RNAi agents comprising a single strand, wherein the single strand comprises the sequences of both the antisense and sense strands of any RNAi agent disclosed herein, e.g., wherein the strands are contiguous, connected by a loop or otherwise linked. Examples of such molecules include those with a hairpin between the sense and anti-sense sequences (e.g., shRNA).

The RNAi agent(s) of the present disclosure target (e.g., bind to, anneal to, hybridize, etc.) the KRAS mRNA. The use of the RNAi agent specific to KRAS results in a decrease of KRAS activity, level and/or expression, e.g., a "knock-down" (KD) or "knock-out" of the target gene or target sequence. In one embodiment, in the case of a disease state characterized by over-expression or hyper-activity of KRAS, administration of a RNAi agent to KRAS knocks down the KRAS target enough to provide a more normal or therapeutic level of KRAS activity or expression.

In one embodiment, the RNAi comprises a single strand (such as an shRNA, as described herein).

In various embodiments, one or both strands contain one or more nicks, i.e., a break or missing bond in the phosphate backbone, such that at least one nucleotide subunit is not covalently linked to the adjacent nucleotide subunit in any given sequence. In some embodiments, the passenger strand is nicked (see, for example, WO 2007/107162). In various embodiments, one or both strands contain one or more gaps, e.g., wherein at least one entire nucleotide subunit is absent from the disclosed sequence. Where a sense or antisense sequence contains a gap, that strand is envisioned to comprise two separate oligonucleotides.

Particularly useful siRNAs include those which can bind specifically to those regions of the KRAS mRNA that have one or more of the following qualities: binding in the coding segment of KRAS; binding at or near the junction of the 5' untranslated region and the start of the coding segment; binding at or near the translational start site of the mRNA; binding at, across or near junctions of exons and introns; little or no binding to the mRNAs or transcripts of other genes (little or no "off-target effects"); binding to the KRAS mRNA in or near a region or regions that is not double-stranded or a stem region, e.g., those in a loop or single-stranded portion; eliciting little or no immunogenicity; binding in a segment of the KRAS mRNA sequence which is conserved among various animal species (including human, mouse, rat, cyno, etc.), as the presence of a conserved sequence facilitates testing using various laboratory animals; binding to double-stranded region(s) of the mRNA; binding to an AT-rich region (e.g., at least about 50, 51, 52, 53, 54, 55, 56, 57, 58, 59, or 60% AT-rich); and/or lacking particular sequences known or suspected to decrease siRNA activity, e.g., the presence of a GG sequence at the 5' end, which may decrease separation of the double-stranded portion of the siRNA. In one embodiment, the RNAi agent specific to KRAS can be a double-stranded RNA having any one or more of these qualities.

The term "double-stranded RNA" or "dsRNA," as used herein, refers to a RNAi agent comprising a first and a second strand; e.g., a composition that includes an RNA molecule or complex of molecules having a hybridized duplex region (i.e., a region where the nucleotide bases from the first strand and the second strand are paired) that comprises two anti-parallel and substantially complementary nucleic acid strands, which will be referred to as having "sense" and "antisense" orientations with respect to a target RNA. The antisense strand, with respect to the mRNA target, is also called the "guide" strand, and the sense strand is also called the "passenger" or "anti-guide" strand. The passenger strand can include at least one or more of the following: one or more extra nucleotides (e.g., a bulge or 1 nt loop) compared to the other strand, a nick, a gap, a mismatch, etc., compared to the other strand. In various embodiments, the RNAi agent comprises a first strand and a second strand. In various embodiments, and as used herein and as is clear by context, terminology referring to the first strand refers to the sense strand and the second strand refers to the anti-sense strand as listed in any Table herein. In other embodiments, and as used herein and as is clear by context, the first strand refers to the anti-sense strand, and the second strand refers to the sense strand as listed in any Table herein.

The duplex region can be of any length that permits specific degradation of a desired target RNA through a RISC pathway, but will typically range from 9 to 36 base pairs ("bp") in length, e.g., 15-30 bp in length. Considering a duplex between 9 and 36 bp, the duplex can be any length in this range, for example, 9, 10, 11, 12, 13, 14, 15, 16, 17, 18, 19, 20, 21, 22, 23, 24, 25, 26, 27, 28, 29, 30, 31, 32, 33, 34, 35, or 36 bp and any sub-range therebetween, including, but not limited to 15-30 bp, 15-26 bp, 15-23 bp, 15-22 bp, 15-21 bp, 15-20 bp, 15-19 bp, 15-18 bp, 15-17 bp, 18-30 bp, 18-26 bp, 18-23 bp, 18-22 bp, 18-21 bp, 18-20 bp, 19-30 bp, 19-26 bp, 19-23 bp, 19-22 bp, 19-21 bp, 19-20 bp, 19 bp, 20-30 bp, 20-26 bp, 20-25 bp, 20-24 bp, 20-23 bp, 20-22 bp, 20-21 bp, 20 basepairs, 21-30 bp, 21-26 bp, 21-25 bp, 21-24 bp, 21-23 bp, 21-22 bp, 21 bp, 22 bp, or 23 bp. The dsRNAs generated in the cell by processing with Dicer and similar enzymes are generally in the range of about 19 to about 22 bp in length. One strand of the duplex region of a dsRNA comprises a sequence that is substantially complementary to a region of a target RNA. The two strands forming the duplex structure can be from a single RNA molecule having at least one self-complementary duplex region, or can be formed from two or more separate RNA molecules that hybridize to form the duplex. Where the duplex region is formed from two self-complementary regions of a single molecule, the molecule can have a duplex region separated by a single-stranded chain of nucleotides (herein referred to as a "hairpin loop", e.g., such as found in an shRNA construct) between the 3'-end of one strand and the 5'-end of the respective other strand forming the duplex structure. The hairpin loop can comprise at least one unpaired nucleotide; in some embodiments the hairpin loop can comprise at least 3, at least 4, at least 5, at least 6, at least 7, at least 8, at least 9, at least 10, at least 20, at least 23 or more unpaired nucleotides. Where the two substantially complementary strands of a dsRNA are comprised by separate RNA molecules, those molecules need not, but can, be covalently connected. Where the two strands are connected covalently by a hairpin loop, the construct is generally referred to herein and in the art as a "shRNA". Where the two strands are connected covalently by means other than a hairpin loop, the connecting structure is referred to as a "linker."

**RNAi Agents to KRAS comprising mismatches from the disclosed sequences**

Various specific embodiments of a RNAi agent to KRAS are disclosed herein; example sequences are provided in Tables 1, 2 and 3. Specific embodiments of the present disclosure include RNAi agents which comprise sequences differing by 0, 1, 2, or 3 nt (nucleotides) or bp [basepair(s)] (e.g., with 0, 1, 2 or 3 mismatches) from any of the RNAi agents listed in Tables 1 to 6, and modified and unmodified variants thereof.

A mismatch is defined herein as a difference between the base sequence or length when two sequences are maximally aligned and compared. A mismatch is defined as a position wherein the base of one sequence does not match the base of the other sequence. Thus, a mismatch is counted, for example, if a position in one sequence has a particular base (e.g., A), and the corresponding position on the other sequence has a different base (e.g., G). Substitution of A, for example, with T, C, G or U would constitute a mismatch. Substitution of G with T, A, C or U would also constitute a mismatch. Substitution of C with T, G, A or U would also constitute a mismatch. Substitution of U with A, C or G would constitute a mismatch. Note, however, that on a given strand, a U can be replaced by T (either as RNA or, preferably, DNA, e.g., 2'-deoxy-thymidine); the replacement of a U with a T is not a mismatch as used herein, as either U or T can pair with A on the opposite strand. The RNAi agent can thus comprise one or more DNA bases, e.g., T. In some cases, in a portion or portions of the RNAi agent, DNA can be used in place of RNA (e.g., in the seed region), to form a DNA-RNA hybrid. See, for example, Yamato et al. 2011 cancer Gene Ther. 18: 587-597. No mismatch is counted between a DNA portion(s) of the RNAi agent and the corresponding target mRNA if basepairing occurs (e.g., between A, G, C, or T in the DNA portion, and the corresponding U, C, G, or A, respectively in the mRNA).

A mismatch is also counted, e.g., if a position in one sequence has a base (e.g., A), and the corresponding position on the other sequence has no base (e.g., that position is an abasic nucleotide, which comprises a phosphate-sugar backbone but no base). A single-stranded nick in either sequence (or in the sense or anti-sense strand) is not counted as mismatch. Thus, as a non-limiting example, no mismatch would be counted if one sequence comprises the sequence AG, but the other sequence comprises the sequence AG with a single-stranded nick between the A and the G. A nucleotide modification in the sugar or phosphate is also not considered a mismatch. Thus, if one sequence comprises a C, and the other sequence comprises a modified C (e.g., 2'-modification) at the same position, no mismatch would be counted.

Thus, no mismatches are counted if modifications are made to the sugar, phosphate, or backbone of the RNAi agent without modifying the base. Thus, a sequence of GGACGAAUAUGAUCCAACA (SEQ ID NO: 1) as an RNA and the same sequence as a PNA (peptide nucleic acid) (or TNA or GNA or FANA) have 0 mismatches from each other.

It is also noted that the sequences of the RNAi agents in Tables 1 to 6 include sequences which comprise modifications, as detailed in Table 3. It is noted that dTdT (2'-deoxy-thymidine-5'-phosphate and 2'-deoxy-thymidine-5'-phosphate), or in some cases, TT or UU, is added as a cap or extension to both 3'-ends, but this cap or extension is not included in the calculation of the total number of mismatches. In addition, in Table 3, a modified sequence can comprise one or more modifications from the corresponding unmodified sequence. In this case, lowercase "c" represents 2'-O-methylcytidine-5'-phosphate, and lowercase "u" represents 2'-O-methyluridine-5'-phosphate. Uppercase "A", "C", "G" and "U" represent the un-modified adenosine-5'-phosphate, cytidine-5'-phosphate, guanosine-5'-phosphate, and uridine-5'-phosphate, respectively. The substitution of modified c for unmodified C does not count as a mismatch in numbering the 0, 1, 2, or 3 mismatches between sequences. This nomenclature is used for all sequences in Tables 1 to 6. Thus, an equal number of mismatches would be calculated (a) between a test sequence and a modified sequence, and (b) between the same test sequence and the corresponding unmodified sequence from the KRAS gene, and (c) between a modified sequence and a differently modified sequence which have the same base sequence.

In one particular embodiment, the present disclosure comprises a RNAi agent comprising a anti-sense strand comprising at least 15 to 19 contiguous nucleotides differing by 0, 1, 2, or 3 nt from the sequence of the anti-sense strand of: any of the RNAi agents listed in Tables 1 to 6, and modified and unmodified variants thereof.

The present disclosure pertains to "modified and unmodified variants" of the disclosed sequences.

An "unmodified variant" of a particular sequence is the corresponding portion of KRAS without any modifications. Example modified sequences are listed in Tables 1, 2 and 3. The "unmodified variants" of the sequences of Tables 1 to 6 have the identical sequence, without base modifications or terminal dTdT. A given sequence and an "unmodified variant" of it differ by 0 nt (and have no mismatches).

A "modified variant" of a particular sequence comprises one or more (or one or more fewer) modifications to the backbone, sugar, phosphate or base, but do not have any base substitutions (e.g., G for C, or A for G); thus a given sequence and a modified variant thereof differ by 0 nt (and have no mismatches). As another example, a given sequence as a RNA and the same sequence as a PNA are modified variants of each other and differ by 0 nt (and have no mismatches). Similarly, the same sequence (with no base substitutions) as a locked nucleic acid (LNA), Morpholino, threose nucleic acid (TNA), or glycol nucleic acid (GNA) would be a modified variant which has 0 mismatches.

As detailed below, substituting a single nucleotide at a given position with a modified version of the same nucleotide would produce a modified variant (with 0 mismatches).

In another particular embodiment, the RNAi agent comprises a sense strand comprising at least 15 contiguous nucleotides differing by 0, 1, 2, or 3 nt from the sense strand of any of the RNAi agents listed in Tables 1 to 6and modified and unmodified variants thereof.

RNAi agents to KRAS of the present disclosure can be used in RNA interference.

**Modifications of RNAi Agent Sequences**

The present disclosure encompasses both unmodified sequences and example modified sequences, such as those disclosed in Tables 1, 2 and 3.

The present disclosure further encompasses any other modification of a disclosed sequence (e.g., a modified variant).

For example, the disclosure encompasses a RNAi agent with a substitution of a single nucleotide at a given position with a modified version of the same nucleotide. Thus a nucleotide (A, G, C or U) can be replaced by the corresponding 5-fluorouracil, 5-bromouracil, 5-chlorouracil, 5-iodouracil, hypoxanthine, xantine, 4-acetylcytosine, 5-(carboxyhydroxylmethyl) uracil, 5-carboxymethylaminomethyl-2-thiouridine, 5-carboxymethylaminomethyluracil, dihydrouracil, beta-D-galactosylqueosine, inosine, N6-isopentenyladenine, 1-methylguanine, 1-methylinosine, 2,2-dimethylguanine, 2-methyladenine, 2-methylguanine, 3-methylcytosine, 5-methylcytosine, N6-adenine, 7-methylguanine, 5-methylaminomethyluracil, 5-methoxyaminomethyl-2-thiouracil, beta-D-mannosylqueosine, 5'-methoxycarboxymethyluracil, 5-methoxyuracil, 2-methylthio-N6-isopentenyladenine, uracil-5-oxyacetic acid (v), wybutoxosine, pseudouracil, queosine, 2-thiocytosine, 5-methyl-2-thiouracil, 2-thiouracil, 4-thiouracil, 5-methyluracil, uracil-5- oxyacetic acid methylester, uracil-5-oxyacetic acid (v), 5-methyl-2-thiouracil, 3-(3-amino-3-N-2-carboxypropyl) uracil, (acp3) w, or 2,6-diaminopurine.

Additional modified variants include the addition of any other moiety (e.g., a radiolabel or other tag or conjugate) to the RNAi agent; provided that the base sequence is identical, the addition of other moieties produces a "modified variant" (with no mismatches).

Various sets of modifications can be used. These include the following formats, which are used in various screens disclosed herein.

A22S26

A22 indicates that All UA as 2'-OMe-U A and all CA as 2'-OMe-C A
S26 indicates that All U as 2'-OMe-U and all C as 2'-OMe-C
All 3' overhangs as 2'-OMe-U 2'-OMe-U

In addition to these modifications and patterns (e.g, formats) for modifications, other modifications or sets of modifications of the sequences provided can be generated using common knowledge of nucleic acid modification.

**RNA Interference**

RNA interference (RNAi) is a post-transcriptional, targeted gene-silencing technique that uses double-stranded RNA (dsRNA) to degrade messenger RNA (mRNA) containing the same sequence as the dsRNA. The process of RNAi occurs when ribonuclease III (Dicer) cleaves the longer dsRNA into shorter fragments called siRNAs. siRNAs (small interfering RNAs) produced by Dicer are typically about 21 to 23 nucleotides long and comprise about 19 base pair duplexes (though artificial siRNAs or RNAi agents can be shorter or longer, and/or blunt-ended, and/or comprises one or more endcaps). The smaller RNA segments then mediate the degradation of the target mRNA. Dicer has also been implicated in the excision of 21- and 22-nucleotide small temporal RNAs (stRNAs) from precursor RNA of conserved structure that are implicated in translational control. Hutvagner et al. 2001 Science 293: 834. The RNAi response also features an endonuclease complex, commonly referred to as an RNA-induced silencing complex (RISC), which mediates cleavage of single-stranded mRNA complementary to the anti-sense strand of the RNAi agent. Cleavage of the target RNA takes place in the middle of the region complementary to the anti-sense strand of the siRNA duplex.

In one aspect, an RNA interference agent includes a single-stranded RNA that interacts with a target RNA sequence to direct the cleavage of the target RNA. Without wishing to be bound by theory, the present disclosure contemplates a long double-stranded RNA introduced into plants and invertebrate cells is broken down into siRNA by a Type III endonuclease known as Dicer. Sharp et al. 2001 Genes Dev. 15:485. Dicer, a ribonuclease-III-like enzyme, processes the dsRNA into 19-23 base pair short interfering RNAs with characteristic two base 3' overhangs. Bernstein, et al. 2001 Nature 409:363. The siRNAs are then incorporated into an RNA-induced silencing complex (RISC) where one or more helicases unwind the siRNA duplex, enabling one of the now unpaired siRNA strands to act as a "guide" strand to guide target recognition. Nykanen, et al. 2001 Cell 107:309. Upon binding of the antisense guide strand to the appropriate target mRNA, one or more endonucleases within the RISC cleaves the target to induce silencing. Elbashir, et al. 2001 Genes Dev. 15:188. Thus, in one aspect the present disclosure relates to a single-stranded RNA that promotes the formation of a RISC complex to effect silencing of the target gene.

Kits for RNAi synthesis are commercially available, e.g., from New England Biolabs and Ambion.

The RNAi agent(s) of the present disclosure target (e.g., bind to, anneal to, etc.) the KRAS mRNA. The use of the RNAi agent to KRAS results in a decrease of KRAS activity, level and/or expression, e.g., a "knock-down" or "knock-out" of the target gene or target sequence. Particularly, in one embodiment, in the case of a disease state characterized by over-expression or hyper-activity of KRAS, administration of a RNAi agent to KRAS knocks down the KRAS target enough to restore a normal level of KRAS activity.

A suitable RNAi agent can be selected by any process known in the art or conceivable by one of ordinary skill in the art. For example, the selection criteria can include one or more of the following steps: initial analysis of the KRAS gene sequence and design of RNAi agents; this design can take into consideration sequence similarity across species (human, cynomolgus, mouse, etc.) and dissimilarity to other (non-KRAS) genes; screening of RNAi agents *in vitro* (e.g., at 10 nM in RKO cells); determination of EC50 in RKO cells; determination of viability of cells treated with RNAi agents, including insensitive cells which do not require KRAS for survival, or sensitive cells, which do require KRAS for survival; testing with human PBMC (peripheral blood mononuclear cells), e.g., to test levels of TNF-alpha to estimate immunogenicity, wherein immunostimulatory sequences are less desired; testing in human whole blood assay, wherein fresh human blood is treated with an RNAi agent and cytokine/chemokine levels are determined [e.g., TNF-alpha (tumor necrosis factor-alpha) and/or MCP1 (monocyte chemotactic protein 1)], wherein Immunostimulatory sequences are less desired; determination of gene knockdown *in vivo* using subcutaneous tumors in test animals; KRAS target gene modulation analysis, e.g., using a pharmacodynamic (PD) marker, for example, other factors whose expression is affected by KRAS, wherein KRAS knockdown leads to a dose-dependent reduction of abundance of those components; and optimization of specific modifications of the RNAi agents.

The dsRNA molecules (RNAi agents) described herein are thus useful in RNA interference of KRAS.

### Features of a RNAi Agent: Sense strand, Antisense Strand and (Optional) Overhangs

In various embodiments, the RNAi agents comprise a first strand and a second strand, e.g., a sense strand and an antisense strand (or an antisense and a sense strand) and, optionally, one or both ends of the duplex containing unpaired nucleotides referred to herein as overhangs.

The term "antisense strand" refers to the strand of a RNAi agent which includes a region that is substantially complementary to a target sequence. As used herein, the term "region of complementarity" refers to the region on the antisense strand that is substantially complementary to a sequence, for example a target sequence, as defined herein. Where the region of complementarity is not fully complementary to the target sequence, the mismatches may be in the internal or terminal regions of the molecule. Generally, the most tolerated mismatches are in the terminal regions, e.g., within 5, 4, 3, or 2 nucleotides of the 5' and/or 3' terminus.

The term "sense strand," as used herein, refers to the strand of a RNAi agent that includes a region that is substantially complementary to a region of the antisense strand as that term is defined herein.

The sequence of a gene may vary from individual to individual, especially at wobble positions within the coding segment, or in the untranslated region; individuals may also differ from each other in coding sequence, resulting in additional differences in mRNA. The sequence of the sense and antisense strands of the RNAi agent can thus be designed to correspond to that of an individual patient, if and where needed. RNAi agents can also be modified in sequence to reduce immunogenicity, binding to undesired mRNAs (e.g., "off-target effects") or to increase stability in the blood. These sequence variants are independent of chemical modification of the bases or 5' or 3' or other end-caps of the RNAi agents.

The RNAi agents can also have overhangs of 0, 1, or 2 overhangs; in the case of a 0 nt overhang, they are blunt-ended. A RNAi agent can have 0, 1 or 2 blunt ends. In a "blunt-ended RNAi agent" both strands terminate in a base-pair; thus a blunt-ended molecule lacks either 3' or 5' single-stranded nucleotide overhangs.

The RNAi agents can comprise overhang(s), blunt end(s), and/or 5' and 3' endcap(s).

As used herein, the term "overhang" or "nucleotide overhang" refer to at least one unpaired nucleotide that protrudes from the end of at least one of the two strands of the duplex structure of a RNAi agent. For example, when a 3'-end of one strand of a dsRNA extends beyond the 5'-end of the other strand, or vice versa, the unpaired nucleotide(s) form the overhang. A dsRNA can comprise an overhang of at least one nucleotide; alternatively the overhang can comprise at least two nucleotides, at least three nucleotides, at least four nucleotides, at least five nucleotides or more. An overhang can comprise or consist of a nucleotide/nucleoside analog, including a deoxynucleotide/nucleoside. The overhang(s) may be on the sense strand, the antisense strand or any combination thereof. Furthermore, the nucleotide(s) of an overhang can be present on the 5' end, 3' end or both ends of either an antisense or sense strand of a dsRNA.The RNAi agent can also optionally comprise a cap. The term "Cap" and the like include a chemical moiety attached to the end of a double-stranded nucleotide duplex, but is used herein to exclude a chemical moiety that is a nucleotide or nucleoside. A "3' Cap" is attached at the 3' end of a nucleotide or oligonucleotide and protects the molecule from degradation, e.g., from nucleases, such as those in blood serum or intestinal fluid. A 3' cap can replace a TT or UU dinucleotide at the end of a blunt-ended RNAi agent. In one embodiment, 3' end caps are as disclosed in, for example, WO 2005/021749 and WO 2007/128477. A "5' cap" is attached at the 5' end of a nucleotide or oligonucleotide. A cap should not interfere (or unduly interfere) with RNAi activity.

The present disclosure thus contemplates a RNAi agent specific to KRAS comprising an antisense strand (which may be contiguous or connected via a linker or loop) in a RNAi agent. In a more specific embodiment, an RNAi agent comprises an antisense strand and a sense strand which together comprise a double-stranded or complementary region. In one embodiment, it can also optionally comprise one or two overhangs and/or one or two caps. The RNAi agent is used to induce RNA interference of the target gene, KRAS.

**Target and complementary sequences**

The RNAi agents of the present disclosure target (e.g., specifically bind to, anneal to, etc.) the mRNA encoding the gene KRAS. The use of the RNAi agent specific to KRAS results in a decrease of KRAS activity, level and/or expression, e.g., a "knock-down" or "knock-out" of the target gene or target sequence. Particularly in one embodiment, in the case of a disease state characterized by over-expression or hyper-activity of KRAS, administration of a RNAi agent to KRAS knocks down the KRAS gene enough to restore a normal level of KRAS activity or expression.

In one embodiment, the first or second strand of the RNAi comprises a sequence complementary to that of the target nucleic acid, KRAS.

As used herein, the term "strand comprising a sequence" refers to an oligonucleotide comprising a chain of nucleotides that is described by the sequence referred to using the standard nucleotide nomenclature.

As used herein, "target sequence" or "target gene" refer to a contiguous portion of the nucleotide sequence of an mRNA molecule formed during the transcription of a gene, e.g., a KRAS gene, including mRNA that is a product of RNA processing of a primary transcription product. The target portion of the sequence will be at least long enough to serve as a substrate for iRNA-directed cleavage at or near that portion. For example, the target sequence will generally be from 9-36 nucleotides ("nt") in length, e.g., 15-30 nt in length, including all sub-ranges therebetween. As non-limiting examples, the target sequence can be from 15-30 nt, 15-26 nt, 15-23 nt, 15-22 nt, 15-21 nt, 15-20 nt, 15-19 nt, 15-18 nt, 15-17 nt, 18-30 nt, 18-26 nt, 18-23 nt, 18-22 nt, 18-21 nt, 18-20 nt, 19-30 nt, 19-26 nt, 19-23 nt, 19-22 nt, 19-21 nt, 19-20 nt, 19 nt, 20-30 nt, 20-26 nt, 20-25 nt, 20-24 nt, 20-23 nt, 20-22 nt, 20-21 nt, 20 nt, 21-30 nt, 21-26 nt, 21-25 nt, 21-24 nt, 21-23 nt, or 21-22 nt, 21 nt, 22 nt, or 23 nt. The sense and antisense strands of the RNAi comprise a sequence complementary to that of the target nucleic acid, KRAS.

As used herein, and unless otherwise indicated, the term "complementary" refers to the ability of an oligonucleotide or polynucleotide comprising a first nucleotide sequence to hybridize and form a duplex structure under certain conditions with an oligonucleotide or polynucleotide comprising a second nucleotide sequence. Such conditions can, for example, be stringent, e.g., 400 mM NaCl, 40 mM PIPES pH 6.4, 1 mM EDTA, 50°C or 70°C for 12-16 hours followed by washing. Other conditions, such as physiologically relevant conditions as may be encountered inside an organism, can apply. The skilled person will be able to determine the set of conditions most appropriate for a test of complementarity of two sequences in accordance with the ultimate application of the hybridized nucleotides.

Complementary sequences within a RNAi agent, e.g., within a dsRNA as described herein, include base-paired oligonucleotides or polynucleotides comprising a first nucleotide sequence to an oligonucleotide or polynucleotide comprising a second nucleotide sequence over the entire length of one or both nucleotide sequences. Such sequences can be referred to as "fully complementary" with respect to each other herein. However, where a first sequence is referred to as "substantially complementary" with respect to a second sequence herein, the two sequences can be fully complementary, or they may form one or more, but generally not more than 5, 4, 3 or 2 mismatched base pairs upon hybridization for a duplex up to 30 base pairs, while retaining the ability to hybridize under the conditions most relevant to their ultimate application, e.g., inhibition of gene expression via a RISC pathway. However, where two oligonucleotides are designed to form, upon hybridization, one or more single-stranded overhangs, such overhangs shall not be regarded as mismatches with regard to the determination of complementarity. For example, a dsRNA comprising one oligonucleotide 21 nucleotides in length and another oligonucleotide 23 nucleotides in length, wherein the longer oligonucleotide comprises a sequence of 21 nucleotides that is fully complementary to the shorter oligonucleotide, may yet be referred to as "fully complementary" for the purposes described herein. The term "overhang" describes an unpaired nucleotide at the 3' or 5' end of a double-stranded nucleotide duplex, as described above. In one embodiment, the overhang is 1 to 4 nt long and is on the 3' end.

"Complementary" sequences, as used herein, may also include, or be formed entirely from, non-Watson-Crick base pairs and/or base pairs formed from non-natural and modified nucleotides, in as far as the above requirements with respect to their ability to hybridize are fulfilled. Such non-Watson-Crick base pairs includes, but are not limited to, Wobble or Hoogstein base pairing. The terms "complementary," "fully complementary" and "substantially complementary" herein may furthermore be used with respect to the base matching between the sense strand and the antisense strand of a dsRNA, or between the antisense strand of a RNAi agent and a target sequence, as will be understood from the context of their use. As used herein, a polynucleotide that is "substantially complementary to at least part of" a messenger RNA (mRNA) refers to a polynucleotide that is substantially complementary to a contiguous portion of the mRNA of interest (e.g., an mRNA encoding KRAS). For example, a polynucleotide is complementary to at least a part of a KRAS mRNA if the sequence is substantially complementary to a non-interrupted portion of an mRNA encoding KRAS.

Thus, the RNAi agent of the present disclosure is complimentary or substantially complimentary to a target sequence in the target KRAS and is double-stranded, comprising a sense and an antisense strand (which can be contiguous, linked via a loop, or otherwise joined), where the double-stranded region an be 9 to 36 bp long (particularly for example, 19-22 bp or 19-23 bp long), and can furthermore optionally comprise a 3' or 5' overhang, and the RNAi agent can furthermore comprise a 3' cap. The RNAi agent mediates RNA interference, down-regulating or inhibiting the level, expression and/or activity of KRAS, and/or establishing or re-establishing an approximately normal level of KRAS and/or KRAS activity, or other biological function related to KRAS.

Thus, the RNAi agent of the present disclosure is complimentary or substantially complimentary to a target sequence in the target KRAS and is double-stranded, comprising a sense and an antisense strand (which can be contiguous, linked via a loop, or otherwise joined), where the double-stranded region an be 9 to 36 bp long (particularly for example, 19-22 bp or 19-23 bp long), and can furthermore optionally comprise a 3' or 5' overhang, and the RNAi agent can furthermore comprise a 3' cap. The RNAi agent mediates RNA interference, down-regulating or inhibiting the level, expression and/or activity of KRAS, and/or establishing or re-establishing an approximately normal level of KRAS activity or expression.

The term "double-stranded RNA" or "dsRNA," as used herein, refers to an RNAi agent comprising a first and a second strand; e.g., a composition that includes an RNA molecule or complex of molecules having a hybridized duplex region that comprises two anti-parallel and substantially complementary nucleic acid strands, which will be referred to as having "sense" and "antisense" orientations with respect to a target RNA. The antisense strand, with respect to the mRNA target, is also called the "guide" strand, and the sense strand is also called the "passenger" strand. As used herein, depending on the context, the "first" strand can be the guide or antisense strand, and the "second" strand can be the passenger or sense strand. Also as used herein, again depending on the context, the "first" strand can be the passenger or sense strand, and the "second" strand can be the guide or antisense. The passenger strand can include at least one or more of the following: one or more extra nucleotides (e.g., a bulge or 1 nt loop) compared to the other strand, a nick, a gap, etc., compared to the other strand. In various embodiments, the first strand is the sense strand and the second strand is the anti-sense strand. In other embodiments, the first strand is the anti-sense strand, and the second strand is the sense strand.

The duplex region can be of any length that permits loading into the RISC complex and subsequent specific degradation of a desired target RNA through a RISC pathway, but will typically range from 9 to 36 base pairs ("bp") in length, e.g., 15-30 base pairs in length. Considering a duplex between 9 and 36 base pairs, the duplex can be any length in this range, for example, 9, 10, 11, 12, 13, 14, 15, 16, 17, 18, 19, 20, 21, 22, 23, 24, 25, 26, 27, 28, 29, 30, 31, 32, 33, 34, 35, or 36 bp and any sub-range therebetween, including, but not limited to 15-30 base pairs, 15-26 bp, 15-23 bp, 15-22 bp, 15-21 bp, 15-20 bp, 15-19 bp, 15-18 bp, 15-17 bp, 18-30 bp, 18-26 bp, 18-23 bp, 18-22 bp, 18-21 bp, 18-20 bp, 19-30 bp, 19-26 bp, 19-23 bp, 19-22 bp, 19-21 bp, 19-20 bp, 19 bp, 20-30 bp, 20-26 bp, 20-25 bp, 20-24 bp, 20-23 bp, 20-22 bp, 20-21 bp, 20 bp, 21-30 bp, 21-26 bp, 21-25 bp, 21-24 bp, 21-23 bp, 21-22 bp, 21 bp, 22 bp, or 23 bp.

The dsRNAs generated in the cell by processing with Dicer and similar enzymes are generally in the range of about 19 to about 22 base pairs in length, although artificial RNAi agents can be synthesized or made by any method known in the art. One strand of the duplex region of a dsRNA comprises a sequence that is substantially complementary to a region of a target RNA. The two strands forming the duplex structure can be from a single RNA molecule having at least one self-complementary duplex region, or can be formed from two or more separate RNA molecules that hybridize to form the duplex. Where the duplex region is formed from two self-complementary regions of a single molecule, the molecule can have a duplex region separated by a single stranded chain of nucleotides (herein referred to as a "hairpin loop", e.g., such as found in an shRNA construct) between the 3'-end of one strand and the 5'-end of the respective other strand forming the duplex structure. The hairpin loop can comprise at least one unpaired nucleotide; in some embodiments the hairpin loop can comprise at least 3, at least 4, at least 5, at least 6, at least 7, at least 8, at least 9, at least 10, at least 20, at least 23 or more unpaired nucleotides. Where the two substantially complementary strands of a dsRNA are comprised by separate RNA molecules, those molecules need not, but can be covalently connected. Where the two strands are connected covalently by a hairpin loop, the construct is generally referred to herein and in the art as a "shRNA". Where the two strands are connected covalently by means other than a hairpin loop, the connecting structure is referred to as a "linker." The term "siRNA" is also used herein to refer to a dsRNA as described above.

**RNAi Agents Lowering or Normalizing KRAS Level, Expression And/Or Activity**

RNAi agents for targeting KRAS include those which bind to a KRAS sequence provided herein and which work to reduce KRAS through a RNAi mechanism. Example RNAi agents (e.g., siRNAs) to KRAS are provided, e.g., in Table 1.

Any method known in the art can be use to measure changes in KRAS activity, level, and/or expression induced by a KRAS RNAi agent. Measurements can be performed at multiple timepoints, prior to, during and after administration of the RNAi agent, to determine the effect of the RNAi agent.

The RNAi agents of the present disclosure silence, inhibit the expression of, down-regulate the expression of, and/or suppress the expression of KRAS, such that an approximately normal level of KRAS activity or expression is restored.

In addition, in various embodiments, depending on the disease condition and biological context, it is acceptable to use the RNAi agents of the present disclosure to establish a level of KRAS expression, activity and/or level which is below the normal level, or above the normal level, depending on the therapeutic outcome that is desired.

Any method known in the art can be use to measure changes in KRAS activity, level and/or expression induced by a KRAS siRNA. Measurements can be performed at multiple timepoints, prior to, during and after administration of the siRNA, to determine the effect of the siRNA.

The terms "silence," "inhibit the expression of," "down-regulate the expression of," "suppress the expression of," and the like, in so far as they refer to a KRAS gene, herein refer to the at least partial suppression of the expression of a KRAS gene, as manifested by a reduction of the amount of KRAS mRNA which may be isolated from or detected in a first cell or group of cells in which a KRAS gene is transcribed and which has or have been treated such that the expression of a KRAS gene is inhibited, as compared to a second cell or group of cells substantially identical to the first cell or group of cells but which has or have not been so treated (control cells). The degree of inhibition is usually expressed in terms of

$$\frac{(\text{mRNA in control cells}) - (\text{mRNA in treated cells})}{(\text{mRNA in control cells})} \bullet 100\%$$

Equation 1

Alternatively, the degree of inhibition may be given in terms of a reduction of a parameter that is functionally linked to KRAS gene expression, e.g., the amount of protein encoded by a KRAS gene, alteration in expression of a protein whose expression is dependent on KRAS, etc. In principle, KRAS gene silencing may be determined in any cell expressing KRAS, either constitutively or by genomic engineering, and by any appropriate assay. However, when a reference or control is needed in order to determine whether a given RNAi agent inhibits the expression of KRAS by a certain degree and therefore is encompassed by the instant disclosure, the assays provided in the Examples below shall serve as such reference.

For example, in certain instances, expression of KRAS is suppressed by at least about 10%, 15%, 20%, 25%, 30%, 35%, 40%, 45%, or 50% by administration of a RNAi agent featured in the present disclosure. In some embodiments, KRAS is suppressed by at least about 60%, 70%, or 80% by administration of a RNAi agent featured in the present disclosure. In some embodiments, KRAS is suppressed by at least about 85%, 90%, or 95% or more by administration of a RNAi agent, as described herein. In one embodiment, the degree of KRAS suppression is determined by loss of full length KRAS mRNA in a treated cell compared to an untreated cell. In one embodiment, the degree of KRAS suppression is determined with a phenotypic assay that monitors loss of proliferative activity and/or cell death. Other embodiments are as provided in the Examples.

The ability of a RNAi agent to suppress KRAS can be first tested *in vitro* (e.g., using test cells such as RKO).

RNAi agents which can suppress KRAS *in vitro* can then be tested for immunostimulation using, for example, a PBMC (peripheral blood mononuclear cell) assay. RNAi agents can also be tested in animal tests. Test and control animals include those which over-express or under-express KRAS, as described in, for example, Hummer et al. 2005 J. Am. Soc. Nephrol. 16: 3160-3166; Randrianarison etal. 2007 Am. J. Physiol. Lung Cell. Mol. Physiol. 294: 409-416; Cao et al. 2006 Am. J. Physiol. Renal Physiol., and references cited therein. RNAi agents which suppress or alter the level, activity and/or expression of KRAS can be used in medicaments to treat various KRAS-related diseases.

By "lower" in the context of KRAS or a symptom of a KRAS-related disease is meant a statistically significant decrease in such level. The decrease can be, for example, at least 10%, at least 20%, at least 30%, at least 40% or more. If, for a particular disease, or for an individual suffering from a particular disease, the levels or expression of KRAS are elevated, treatment with a KRAS RNAi agent of the present disclosure can particularly reduce the level or expression of KRAS to a level considered in the literature as within the range of normal for an individual without such disorder, or to a level that reduces or ameliorates symptoms of a disease. The level or expression of KRAS can be measured by evaluation of mRNA (e.g., via Northern blots or PCR), or protein (e.g., Western blots). The effect of a RNAi agent on KRAS expression can be determined by measuring KRAS gene transcription rates (e.g., via Northern blots; or reverse transcriptase polymerase chain reaction or real-time polymerase chain reaction).

As used herein, "down-regulates" refers to any statistically significant decrease in a biological activity and/or expression of KRAS, including full blocking of the activity (i.e., complete inhibition) and/or expression. For example, "down-regulation" can refer to a decrease of at least about 10, 20, 30, 40, 50, 60, 70, 80, 90 or 100 % in KRAS level, activity and/or expression.

As used herein, the term "inhibit" or "inhibiting" KRAS refers to any statistically significant decrease in biological level, activity and/or expression of KRAS, including full blocking of the activity and/or expression. For example, "inhibition" can refer to a decrease of at least about 10, 20, 30, 40, 50, 60, 70, 80, 90 or 100 % in KRAS level, activity and/or expression. As used herein, the term "inhibit" similarly refers to a significant decrease in level, activity and/or expression, while referring to any other biological agent or composition.

By "level", it is meant that the KRAS RNAi agent can alter the level of KRAS, e.g., the level of KRAS mRNA or the level of KRAS protein, or the level of activity of KRAS.

Some diseases, such as types of a proliferative disease, including without limitation a solid or liquid cancer, adenocarcinoma, colorectal cancer, advanced and/or metastatic colorectal cancer, colon cancer, lung, non-small cell lung cancer and lung adenocarcinoma, acute myelogenous lung, bladder, brain, breast, cervical, endometrial, gastric, head and neck, kidney, leukemia, myelodysplastic syndrome, myeloid leukemia, liver, melanoma, ovarian, pancreatic, prostate, testicular, thyroid cancers, and cardio-facio-cutaneous (CFC) syndrome and Noonan syndrome, and similar and related diseases, are characterized by a KRAS mutation or excessive KRAS abundance or activity. Particularly in one embodiment, in the case of a disease characterized by over-expression and/or hyper-activity of KRAS, administration of a RNAi agent to KRAS reduces the level, expression and/or activity of KRAS. Thus, in various embodiments, administration of a RNAi agent to KRAS particularly establishes or re-establishes a normal or approximately normal level of KRAS activity, expression and/or level.

By "normal" or "approximately normal" in terms of level, expression and/or activity, is meant at least: about 50%, about 60%, about 70%, about 80%, about 90%, and/or about 100%; and/or no more than: about 100%, about 120%, about 130%, about 140%, or about 150% of the level, expression or activity of KRAS in a healthy cell, tissue, or organ. This can be measured using, for example, lung or kidney homogenates, as described in Gambling et al. 2004 Kidney Intl. 65: 1774-1781. Particularly in one embodiment, administration of the appropriate amount of the appropriate KRAS RNAi agent restores KRAS level, activity and/or expression to about 50% to about 150%, more particularly about 60% to about 140%, more particularly to about 70% to about 130%, more particularly to about 80% to about 120%, more particularly to about 90% to about 110%, and most particularly to about 100% of that of a healthy cell, tissue or organ. Administration of a KRAS RNAi to a patient with a KRAS-related disease thus particularly restores the level, activity, and/or expression of KRAS and the level of $Na^+$ reabsorption to an approximately normal level, as determined by direct measurements of KRAS mRNA or protein levels, or indirect determinations. In addition, the preferred target amount of KRAS level, expression and/or activity after KRAS RNAi agent administration can be calculated to take into account any other perturbations in a KRAS-related pathway. For example, if another factor in a KRAS-related pathway is either over- or under-expressed, KRAS level, expression or activity may be modulated to attain a more normal state.

In addition, in various embodiments, depending on the disease condition and biological context, it is acceptable to use the RNAi agents of the present disclosure to establish a level of KRAS expression, activity and/or level which is below the normal level, or above the normal level.

**Types of RNAi Agents and Modification Thereof**

The use of RNAi agents or compositions comprising an antisense nucleic acid to down-modulate the expression of a particular protein in a cell is well known in the art. A RNAi agent comprises a sequence complementary to, and is capable of hydrogen bonding to, the coding strand of another nucleic acid (e.g., an mRNA). Thus, in various embodiments, the RNAi agents of the present disclosure encompass any RNAi agents which target (e.g., are complementary, capable of hybridizing or hydrogen bonding to, etc.) any sequence presented, e.g., in any of Tables 1 to 6.

Once a functional guide strand has been identified, many variations to the guide and/or passenger strand can be made. For example, the RNAi agent may have modifications internally, or at one or both ends. The modifications at the ends can help stabilize the RNAi agent, protecting it from degradation by nucleases in the blood. The RNAi agents may optionally be directed to regions of the KRAS mRNA known or predicted to be near or at splice sites of the gene.

A RNAi agent can be constructed using chemical synthesis and enzymatic ligation reactions using procedures known in the art. For example, RNAi agent can be chemically synthesized using naturally-occurring nucleotides or variously modified nucleotides designed to decrease off-target effects, and/or increase the biological stability of the molecules or to increase the physical stability of the duplex formed between the antisense and sense nucleic acids, e.g., phosphorothioate derivatives and acridine substituted nucleotides can be used.

"G," "C," "A," "T" and "U" each generally stand for a nucleotide that contains guanine, cytosine, adenine, thymidine and uracil as a base, respectively. However, the terms "ribonucleotide", "deoxynucleotide", or "nucleotide" can also refer to a modified nucleotide or a surrogate replacement moiety. The skilled person is well aware that guanine, cytosine, adenine, and uracil may be replaced by other moieties without substantially altering the base pairing properties of an oligonucleotide comprising a nucleotide bearing such replacement moiety. For example, without limitation, a nucleotide comprising inosine as its base may base pair with nucleotides containing adenine, cytosine, or uracil. Hence, nucleotides containing uracil, guanine, or adenine may be replaced in the nucleotide sequences of dsRNA featured in the present disclosure by a nucleotide containing, for example, inosine. In another example, adenine and cytosine anywhere in the oligonucleotide can be replaced with guanine and uracil, respectively to form Wobble base pairing with the target mRNA. Sequences containing such replacement moieties are suitable for the compositions and methods featured in the present disclosure.

The skilled artisan will recognize that the term "RNA molecule" or "ribonucleic acid molecule" encompasses not only RNA molecules as expressed or found in nature (i.e., are naturally occurring), but also non-naturally occurring analogs and derivatives of RNA comprising one or more ribonucleotide/ribonucleoside analogs or derivatives as described herein or as known in the art.The RNA can be modified in the nucleobase structure or in the ribose-phosphate backbone structure, e.g., as described herein below. However, the molecules comprising ribonucleoside analogs or derivatives must retain the ability to form a duplex. As non-limiting examples, an RNA molecule can also include at least one modified ribonucleoside, including but not limited to a 2'-O-methyl modified nucleotide, a nucleoside comprising a 5' phosphorothioate group, a terminal nucleoside linked to a cholesteryl derivative or dodecanoic acid bisdecylamide group, a locked nucleoside, an abasic nucleoside, a 2'-deoxy-2'-fluoro modified nucleoside, a 2'-amino-modified nucleoside, 2'-alkyl-modified nucleoside, morpholino nucleoside, an unlocked ribonucleotide (e.g., an acyclic nucleotide monomer, as described in WO 2008/147824), a phosphoramidate or a non-natural base comprising nucleoside, or any combination thereof. Alternatively, an RNA molecule can comprise at least two modified ribonucleosides, at least 3, at least 4, at least 5, at least 6, at least 7, at least 8, at least 9, at least 10, at least 15, at least 20 or more, up to the entire length of the dsRNA molecule. The modifications need not be the same for each of such a plurality of modified ribonucleosides in an RNA molecule. In one embodiment, modified RNAs contemplated for use in methods and compositions described herein are peptide nucleic acids (PNAs) that have the ability to form the required duplex structure and that permit or mediate the specific degradation of a target RNA via a RISC pathway.

Examples of modified nucleotides which can be used to generate the RNAi agent include 5-fluorouracil, 5-bromouracil, 5-chlorouracil, 5-iodouracil, hypoxanthine, xantine, 4-acetylcytosine, 5-(carboxyhydroxylmethyl) uracil, 5-carboxymethylaminomethyl-2-thiouridine, 5-carboxymethylaminomethyluracil, dihydrouracil, beta-D-galactosylqueosine, inosine, N6-isopentenyladenine, 1-methylguanine, 1-methylinosine, 2,2-dimethylguanine, 2-methyladenine, 2-methylguanine, 3-methylcytosine, 5-methylcytosine, N6-adenine, 7-methylguanine, 5-methylaminomethyluracil, 5-methoxyaminomethyl-2-thiouracil, beta-D-mannosylqueosine, 5'-methoxycarboxymethyluracil, 5-methoxyuracil, 2-methylthio-N6-isopentenyladenine, uracil-5-oxyacetic acid (v), wybutoxosine, pseudouracil, queosine, 2-thiocytosine, 5-methyl-2-thiouracil, 2-thiouracil, 4-thiouracil, 5-methyluracil, uracil-5- oxyacetic acid methylester, uracil-5-oxyacetic acid (v), 5-methyl-2-thiouracil, 3-(3-amino-3-N-2-carboxypropyl) uracil, (acp3)w, and 2,6-diaminopurine.

A "modified variant" of a sequence disclosed herein includes any variant comprising the same sequence, but with a modification in the base, sugar, phosphate or backbone (but not a base substitution, e.g., A for G, or C for U). Thus, a modified variant can comprise any modified nucleotide described above (e.g., 5-fluorouracil, 5-bromouracil, 5-chlorouracil, 5-iodouracil, hypoxanthine, xantine, 4-acetylcytosine, 5-(carboxyhydroxylmethyl) uracil, etc.). When a base is replaced by a corresponding modified base (e.g., A for modified A), these modified nucleotides do not constitute a mismatch or base difference. Thus a given sequence with a U at a particular position and a modified variant comprising a 5-fluorouracil, 5-bromouracil, 5-chlorouracil, or 5-iodouracil at the same sequence would differ by 0 nt (or have no mismatches); however, a given sequence with a C at a particular position and a different sequence with a 5-fluorouracil (wherein the two sequences are otherwise identical) would differ by 1 nt (1 mismatch).

Replacing the 3'-terminal nucleotide overhanging segments of a 21-mer siRNA duplex having two-nucleotide 3'-overhangs with deoxyribonucleotides does not have an adverse effect on RNAi activity. Replacing up to four nucleotides on each end of the siRNA with deoxyribonucleotides has been well tolerated, whereas complete substitution with deoxyribonucleotides results in no RNAi activity. International PCT Publication No. WO 00/44914, and Beach et al. International PCT Publication No. WO 01/68836 preliminarily suggest that siRNA may include modifications to either the phosphate-sugar backbone or the nucleoside to include at least one of a nitrogen or sulfur heteroatom. Kreutzer et al. Canadian Patent Application No. 2,359,180, also describe certain chemical modifications for use in dsRNA constructs in order to counteract activation of double-stranded RNA-dependent protein kinase PKR, specifically 2'-amino or 2'-O-methyl nucleotides, and nucleotides containing a 2'-O or 4'-C methylene bridge. Additional 3'-terminal nucleotide overhangs include dT (deoxythimidine), 2'-O,4'-C-ethylene thymidine (eT), and 2-hydroxyethyl phosphate (hp). 4-thiouracil and 5-bromouracil substitutions can also be made. Parrish et al. 2000 Molecular Cell 6: 1077-1087.

Those skilled in the art will appreciate that it is possible to synthesize and modify the siRNA as desired, using any conventional method known in the art (see Henschel et al. 2004 DEQOR: a web-based tool for the design and quality control of siRNAs. Nucleic Acids Research 32 (Web Server Issue): W113-W120). In addition, if the RNAi agent is a shRNA, it will be apparent to those skilled in the art that there are a variety of regulatory sequences (for example, constitutive or inducible promoters, tissue- specific promoters or functional fragments thereof, etc.) which are useful for shRNA expression construct/vector.

There are several examples in the art describing sugar, base, phosphate and backbone modifications that can be introduced into nucleic acid molecules with significant enhancement in their nuclease stability and efficacy. For example, oligonucleotides are modified to enhance stability and/or enhance biological activity by modification with nuclease resistant groups, for example, 2'-amino, 2'-C-allyl, 2'-flouro, 2'-O-methyl, 2'-O-allyl, 2'-H, nucleotide base modifications (for a review see Usman and Cedergren 1992 TIBS. 17: 34; Usman et al. 1994 Nucleic Acids Symp. Ser. 31: 163; Burgin et al. 1996 Biochemistry 35: 14090). Sugar modification of nucleic acid molecules are extensively described in the art.

In various embodiments, the RNAi agent comprises a 2'-modification selected from the group consisting of: 2'-deoxy, 2'-deoxy-2'-fluoro, 2'-O-methyl, 2'-O-methoxyethyl (2'-O-MOE), 2'-O-aminopropyl (2'-O-AP), 2'-O-dimethylaminoethyl (2-O-DMAOE), 2'-O-dimethylaminopropyl (2'-O-DMAP), 2'-O-dimethylaminoethyloxyethyl (2'-O-DMAEOE), and 2'-O-N-methylacetamido (2'-O-NMA).

Additional modifications and conjugations of RNAi agents have been described. Soutschek et al. 2004 Nature 432: 173-178 presented conjugation of cholesterol to the 3'-end of the sense strand of a siRNA molecule by means of a pyrrolidine linker, thereby generating a covalent and irreversible conjugate. Chemical modifications (including conjugation with other molecules) of RNAi agents may also be made to improve the *in vivo* pharmacokinetic retention time and efficiency.

In various embodiments, the RNAi agent to KRAS comprises at least one 5'-uridine-adenine-3' (5'-ua-3') dinucleotide, wherein the uridine is a 2'-modified nucleotide; at least one 5'-uridine-guanine-3' (5'-ug-3') dinucleotide, wherein the 5'-uridine is a 2'-modified nucleotide; at least one 5'-cytidine-adenine-3' (5'-ca-3') dinucleotide, wherein the 5'-cytidine is a 2'-modified nucleotide; and/or at least one 5'-uridine-uridine-3' (5'-uu-3') dinucleotide, wherein the 5'-uridine is a 2'-modified nucleotide. In certain embodiments, the RNAi agent can comprise a non-natural nucleobase, wherein the non-natural nucleobase is difluorotolyl, nitroindolyl, nitropyrrolyl, or nitroimidazolyl. In a particular embodiment, the non-natural nucleobase is difluorotolyl. In certain embodiments, only one of the two oligonucleotide strands contains a non-natural nucleobase. In certain embodiments, both of the oligonucleotide strands contain a non-natural nucleobase.

In another embodiment, the RNAi comprises a gap or contains mismatch comprising an abasic nucleotide.

In another embodiment, the RNAi agent has a single-stranded nick (e.g., a break or missing bond in the backbone). In various embodiments, a single-stranded nick can be in either the sense or anti-sense strand, or both.

This nick can be, for example, in the sense strand, producing a small internally segmented interfering RNA, or sisiRNA, which may have less off-target effects than the corresponding RNAi agent without a nick. See, for example, WO 2007/107162 to Wengels and Kjems.

The antisense nucleic acid or RNAi agent can also have an alternative backbone such as locked nucleic acids (LNA), Morpholinos, peptidic nucleic acids (PNA), threose nucleic acid (TNA), or glycol nucleic acid (GNA), or FANA and/or it can be labeled (e.g., radiolabeled or otherwise tagged). FANA are described in Dowler et al. 2006 Nucl. Acids Res. 34: 1669-1675.

One or both strands can comprise an alternative backbone.

In yet another embodiment, the RNAi agent employed by the methods of the present disclosure can include an $\alpha$-anomeric nucleic acid molecule. An $\alpha$-anomeric nucleic acid molecule forms specific double-stranded hybrids with complementary RNA in which, contrary to the usual β-units, the strands run parallel to each other. Gaultier et al. 1987 Nucleic Acids. Res. 15: 6625-6641.

The antisense nucleic acid molecule can also comprise a 2'-o-methylribonucleotide (Inoue et al. 1987 Nucleic Acids Res. 15: 6131-6148) or a chimeric RNA-DNA analogue (Inoue et al. 1987 FEBS Lett. 215: 327-330).

Other modifications and/or other changes can be made to the RNAi agent. A portion of the RNAi agent can be double-stranded DNA, while another portion is double-stranded RNA, forming a DNA-RNA chimera (See, for example, Yamato et al. 2011. Cancer Gene Ther. 18: 587-597). Mismatches between the guide and passenger stand can also be introduced, though some positions may be better suited than others (See, for example, U.S. Patent App. No. 2009/0209626 to Khvorova). The passenger strand can also be shortened, to as short as 15 or 16 nt, while the guide strand remains 19 nt or longer (See, for example, Sun et al. 2008 Nature Biotech. 26: 1379-1382; and Chu and Rana 2008 RNA 14: 1714-1719). This can increase incorporation of the guide strand into the RNA-induced Silence Complex (RISC), and decrease incorporation of the passenger strand, than reducing off-target effects. In some cases, the passenger strand may be more amenable to modification (e.g., single-stranded nicking, nucleotide modifications, and shortening) than the guide strand.

These and many other modifications can be made once a functional guide strand is identified.

**Pharmaceutical Compositions of RNAi Agents**

As used here, a "pharmaceutical composition" comprises a pharmaceutically effective amount of one or more KRAS RNAi agent, a pharmaceutically acceptable carrier, and, optionally, an additional disease treatment which works synergistically with the RNAi agent. As used herein, "pharmacologically effective amount," "therapeutically effective amount" or simply "effective amount" refers to that amount of a RNAi agent effective to produce the intended pharmacological, therapeutic or preventive result. For example, if a given clinical treatment is considered effective where there is at least a 10% reduction in a measurable parameter associated with a disease or disorder, a therapeutically effective amount of a drug for the treatment of that disease or disorder is the amount necessary to effect at least a 10% reduction in that parameter. In this embodiment, a therapeutically effective amount of a RNAi agent targeting KRAS can reduce KRAS protein levels by at least 10%. In additional embodiments, a given clinical treatment is considered effective where there is at least a 15, 20, 25, 30, 35, 40, 45, 50, 55, 60, 65, 70, 75, 80, 85, 90 or 95% reduction in a measurable parameter associated with a disease or disorder, and the therapeutically effective amount of a drug for the treatment of that disease or disorder is the amount necessary to effect at least a 15, 20, 25, 30, 35, 40, 45, 50, 55, 60, 65, 70, 75, 80, 85, 90 or 95% reduction, respectively, in that parameter.

The term "pharmaceutically acceptable carrier" refers to a carrier for administration of a therapeutic agent. Such carriers include, but are not limited to, lipid nanoparticles, saline, buffered saline, dextrose, water, glycerol, ethanol, and combinations thereof. The term specifically excludes cell culture medium. Any appropriate phamaceutical carrier known in the art can be used in conjunction with the RNAi agents disclosed herein.

**Pharmaceutical Composition Comprising a RNAi Agent to KRAS**

Additional components of a pharmaceutical composition comprising a RNAi Agent to KRAS are contemplated to aid in delivery, stability, efficacy, or reduction of immunogenicity.

Liposomes have been used previously for drug delivery (e.g., delivery of a chemotherapeutic). Liposomes (e.g., cationic liposomes) are described in PCT publications WO02/100435A1, WO03/015757A1, WO04029213A2; and WO/2011/076807; U.S. Pat. Nos. 5,962,016; 5,030,453; and 6,680,068; and U.S. Patent Application 2004/0208921. A process of making liposomes is also described in WO04/002453A1. Furthermore, neutral lipids have been incorporated into cationic liposomes (e.g., Farhood et al. 1995), as well as PEGylated lipids.

Cationic liposomes have been used to deliver RNAi agent to various cell types (Sioud and Sorensen 2003; U.S. Patent Application 2004/0204377; Duxbury et al., 2004; Donze and Picard, 2002).

Use of neutral liposomes disclosed in Miller et al. 1998, and U.S. Patent Application 2003/0012812.

As used herein, the term "SNALP" refers to a stable nucleic acid-lipid particle. A SNALP represents a vesicle of lipids coating a reduced aqueous interior comprising a nucleic acid such as an iRNA or a plasmid from which an iRNA is transcribed. SNALPs are described, e.g., in U.S. Patent Application Publication Nos. 20060240093, 20070135372, and in International Application No. WO 2009082817.

Chemical transfection using lipid-based, amine-based and polymer-based techniques, is disclosed in products from Ambion Inc., Austin, Tex.; and Novagen, EMD Biosciences, Inc, an Affiliate of Merck KGaA, Darmstadt, Germany); Ovcharenko D (2003) "Efficient delivery of siRNAs to human primary cells." Ambion TechNotes 10 (5): 15-16). Additionally, Song et al. (Nat Med. published online (Feb 10, 2003) doi: 10.1038/nm828) and others [Caplen et al. 2001 Proc. Natl. Acad. Sci. (USA), 98: 9742-9747; and McCaffrey et al. Nature 414: 34-39] disclose that liver cells can be efficiently transfected by injection of the siRNA into a mammal's circulatory system.

A variety of molecules have been used for cell-specific RNAi agent delivery. See, for example, WO/2011/076807. For example, the nucleic acid-condensing property of protamine has been combined with specific antibodies to deliver siRNAs. Song et al. 2005 Nat Biotech. 23: 709-717. The self-assembly PEGylated polycation polyethylenimine (PEI) has also been used to condense and protect siRNAs. Schiffelers et al. 2004 Nucl. Acids Res. 32: el49, 141-1 10.

The RNAi agents of the present disclosure can be delivered via, for example, Lipid nanoparticles (LNP); neutral liposomes (NL); polymer nanoparticles; double-stranded RNA binding motifs (dsRBMs); or via modification of the RNAi agent (e.g., covalent attachment to the dsRNA) or by any method known in the art for delivery of a RNAi agent comprising nucleic acids.

Lipid nanoparticles (LNP) are self-assembling cationic lipid based systems. These can comprise, for example, a neutral lipid (the liposome base); a cationic lipid (for siRNA loading); cholesterol (for stabilizing the liposomes); and PEG-lipid (for stabilizing the formulation, charge shielding and extended circulation in the bloodstream).

The cationic lipid can comprise, for example, a headgroup, a linker, a tail and a cholesterol tail. The LNP can have, for example, good tumor delivery, extended circulation in the blood, small particles (e.g., less than 100 nm), and stability in the tumor microenvironment (which has low pH and is hypoxic).

**Neutral liposomes (NL) are non-cationic lipid based particles.**

Polymer nanoparticles are self-assembling polymer-based particles.

Double-stranded RNA binding motifs (dsRBMs) are self-assembling RNA binding proteins, which will need modifications.

In various embodiments, the RNAi agent to KRAS is packaged as a monotherapy into a delivery vehicle, or may be further ligated to one or more diagnostic compound, reporter group, cross-linking agent, nuclease-resistance conferring moiety, natural or unusual nucleobase, lipophilic molecule, cholesterol, lipid, lectin, steroid, uvaol, hecigenin, diosgenin, terpene, triterpene, sarsasapogenin, Friedelin, epifriedelanol-derivatized lithocholic acid, vitamin, carbohydrate, dextran, pullulan, chitin, chitosan, synthetic carbohydrate, oligo lactate 15-mer, natural polymer, low- or medium-molecular weight polymer, inulin, cyclodextrin, hyaluronic acid, protein, protein-binding agent, integrin-targeting molecule, polycationic, peptide, polyamine, peptide mimic, and/or transferrin.

The RNAi agents of the present disclosure can be prepared in a pharmaceutical composition comprising various components appropriate for the particular method of administration of the RNAi agent.

## KRAS-Related Diseases

The present disclosure encompasses RNAi agents to KRAS and administration of the RNAi agents to humans and non-human animals to treat various KRAS-related diseases.

By "KRAS-related disease" is meant any disease related to a dysfunction in the level, expression and/or activity of KRAS, and/or any disease which can be treated and/or ameliorated by modulating the level, expression and/or activity of KRAS. In particular, it includes a proliferative disease, including without limitation a solid or liquid cancer, e.g., adenocarcinoma, colorectal cancer, advanced and/or metastatic colorectal cancer, colon cancer, lung, non-small cell lung cancer and lung adenocarcinoma, acute myelogenous lung, bladder, brain, breast, cervical, endometrial, gastric, head and neck, kidney, leukemia, myelodysplastic syndrome, myeloid leukemia, liver, melanoma, ovarian, pancreatic, prostate, testicular, thyroid cancers, and cardio-facio-cutaneous (CFC) syndrome and Noonan syndrome, and similar and related diseases.

For additional information on KRAS-related diseases, see, for example: Bos. 1989. Cancer Res. 49. 17: 4682-9; Engelman et al. 2008. Nature Med. 14: 1351-1356; Haigis et al. 2008 Nature Gen. 40: 600-608; Karapetis et al. 2008 N. E. J. Med. 359: 1757-1765; Kiaris et al. 1995. Int. J. Oncol. 7: 413-421; Liu et al. 1987 Nature 330: 186-188; Riely et al. 2008 Clin. Cancer Res. 14: 5731; and Riely et al. 2009. Proc. Am. Thorac. Soc. 6: 201-205. KRAS mutation, hyperactivity or over-expression is also associated with cardio-facio-cutaneous (CFC) syndrome and Noonan syndrome. Adachi et al. 2012 Seizure: Eur. J. Epilepsy 21: 55-60; Kim et al. 2011. J. Korean Soc. Neonatol. 18: 374-378.

RNAi agents to KRAS can thus be used to treat KRAS-related diseases, particularly those diseases associated with altered expression, activity and/or levels of KRAS.

## Use of RNAi Agents for Treatment of KRAS-Related Diseases

The RNAi agents to KRAS described herein can be formulated into pharmaceutical compositions which can be administered to humans or non-human animals. These compositions can comprise one or more RNAi agents, and, optionally, additional treatments useful for treating KRAS-related diseases. They can be administered as part of an early/preventative treatment, and can be administered in a therapeutically-effective dosage. The pharmaceutical composition can comprise a pharmaceutical carrier and can be administered by any method known in the art. These various aspects of the present disclosure are described in additional detail below.

RNAi agents to KRAS can be administered to humans and non-human animals for treatment of KRAS-related diseases.

In one embodiment of the present disclosure, the compositions comprising a KRAS RNAi agent can be administered to non-human animals. For example, the compositions can be given to chickens, turkeys, livestock animals (such as sheep, pigs, horses, cattle, etc.), companion animals (e.g., cats and dogs) and can have efficacy in treatment of proliferative disease, cardio-facio-cutaneous (CFC) syndrome or Noonan syndrome, and similar diseases. In each case, the RNAi agent to KRAS would be selected to match the sequence of the KRAS of the genome of the animal, and to, particularly, contain at least 1 nt mismatch from all other genes in that animal's genome. The RNAi agents of the present disclosure can thus be used in treatment of KRAS-related diseases in humans and non-human animals.

As used herein in the context of KRAS expression, the terms "treat," "treatment," and the like, refer to relief from or alleviation of pathological processes mediated by KRAS expression. In the context of the present disclosure insofar as it relates to any of the other conditions recited herein below (other than pathological processes mediated by KRAS expression), the terms "treat," "treatment," and the like mean to relieve or alleviate at least one symptom associated with such condition, or to slow or reverse the progression or anticipated progression of such condition, such as slowing the progression of a lipid disorder, such as atherosclerosis.

By "treatment" is also meant prophylaxis, therapy, cure, or any other change in a patient's condition indicating improvement or absence of degradation of physical condition. By "treatment" is meant treatment of KRAS-related disease (e.g., a proliferative disease, including without limitation a solid or liquid cancer, adenocarcinoma, colorectal cancer, advanced and/or metastatic colorectal cancer, colon cancer, lung, non-small cell lung cancer and lung adenocarcinoma, acute myelogenous lung, bladder, brain, breast, cervical, endometrial, gastric, head and neck, kidney, leukemia, myelodysplastic syndrome, myeloid leukemia, liver, melanoma, ovarian, pancreatic, prostate, testicular, thyroid cancers, or cardio-facio-cutaneous (CFC) syndrome or Noonan syndrome), or any appropriate treatment of any other ailment the patient has. As used herein, the terms "treatment" and "treat" refer to both prophylactic and preventative treatment and curative or disease-modifying treatment, including treatment of patients at risk of contracting a disease or suspected of having a disease, as well as patients already ill or diagnosed as suffering from a condition. The terms "treatment" and "treat" also refer to the maintenance and/or promotion of health in an individual not suffering from a disease but who may be susceptible to developing an unhealthy condition, such as nitrogen imbalance or muscle loss. In one embodiment, "treatment" does not encompass prevention of a disease state. Thus, the present disclosure is useful for suppressing expression of KRAS and/or treating a KRAS-related disease in an individual afflicted by a KRAS-related disease, or an individual susceptible to a KRAS-related disease. An individual "afflicted" by a KRAS-related disease has demonstrated detectable symptoms characteristics of the disease, or had otherwise been shown clinically to have been exposed to or to carry KRAS-related disease pathogens or markers. As non-limiting examples, an individual afflicted by a KRAS-related disease can show outward symptoms; or can show no outward symptoms but can be shown with a clinical test to carry protein markers associated with a KRAS-related disease, or proteins or genetic material associated with a pathogen in the blood.

Early treatment of some KRAS-related diseases may be more efficacious if administered early rather than later. Thus, in one particular embodiment, the RNAi agent to KRAS is administered early, prior to disease manifestation, and/or as a preventative agent, rather than administered after disease establishment.

Treatments of KRAS-related diseases can comprise various treatments, comprising a KRAS RNAi agent, and optionally further comprising an additional treatment, which can be a method (or procedure), or an additional composition (e.g., an agent or additional RNAi agent).

**Dosages and Effective Amounts of RNAi Agents**

The RNAi agents of the present disclosure are administered in a dosage of a therapeutically effective amount to a patient in need thereof.

An "effective amount" or a "therapeutically effective amount" is an amount that treats a disease or medical condition of an individual, or, more generally, provides a nutritional, physiological or medical benefit to an individual. As used herein, the phrases "therapeutically effective amount" and "prophylactically effective amount" refer to an amount that provides a therapeutic benefit in the treatment, prevention, or management of pathological processes mediated by KRAS expression or an overt symptom of pathological processes mediated by KRAS expression. The specific amount that is therapeutically effective can be readily determined by an ordinary medical practitioner, and may vary depending on factors known in the art, such as, for example, the type of pathological processes mediated by KRAS expression, the patient's history and age, the stage of pathological processes mediated by KRAS expression, and the administration of other agents that inhibit pathological processes mediated by KRAS expression.

In various embodiments of the present disclosure, the patient is at least about 1, 3, 6, or 9 months, or 1, 5, 10, 20, 30, 40, 50, 55, 60, 65, 70, or 75 years of age. In various embodiments, the patient is no more than about 1, 3, 6, or 9 months, or 1, 5, 10, 20, 30, 40, 50, 55, 60, 65, 70, 75, 80, 90, or 100 years of age. In various embodiments the patient has a body weight of at least about 5, 10, 15, 20, 30, 40, 50, 60, 70, 80, 90, 100, 120, 140, 160, 180, 200, 220, 240, 260, 280, 300, 320, 340, 360, 380 or 400 lbs. In various embodiments, the patient has a body weight of no more than about 5, 10, 15, 20, 30, 40, 50, 60, 70, 80, 90, 100, 120, 140, 160, 180, 200, 220, 240, 260, 280, 300, 320, 340, 360, 380 or 400 lbs.

In various embodiments of the present disclosure, the dosage [measuring only the active ingredient(s)] can be at least about 1, 5, 10, 25, 50, 100, 200, 250, 300, 250, 400, 450, 500, 550, 600, 650, 700, 750, 800, 850, 900, 950 or 1000 ng, 1, 5, 10, 25, 50, 100, 200, 250, 300, 250, 400, 450, 500, 550, 600, 650, 700, 750, 800, 850, 900, 950 or 1000 micrograms, 1, 5, 10, 25, 50, 100, 200, 250, 300, 250, 400, 450, 500, 550, 600, 650, 700, 750, 800, 850, 900, 950 or 1000 mg. In various embodiments, the dosage can be no more than about 10, 25, 50, 100, 200, 250, 300, 250, 400, 450, 500, 550, 600, 650, 700, 750, 800, 850, 900, 950 or 1000 mg. In various embodiments, the dosage can be administered at least more than once a day, daily, more than once a weekly, weekly, bi-weekly, monthly, and/or every 2, 3, 4, 5, 6, 7, 8, 9, 10, 11 or 12 months, or a combination thereof.

In various embodiments, the dosage is correlated to the body weight or body surface area of the individual. The actual dosage level can be varied to obtain an amount of active agent which is effective for a particular patient, composition and mode of administration, without being toxic to the patient. The selected dose will depend on a variety of pharmacokinetic factors, including the activity of the particular RNAi agent employed, the route of administration, the rate of excretion of the RNAi agent, the duration of the treatment, other drugs, compounds and/or materials used in combination with the RNAi agent, the age, sex, weight, condition, general health and prior medical history of the patient, and like factors well known in the medical arts. A physician or veterinarian having ordinary skill in the art can readily determine the effective amount of the RNAi agent required. A suitable dose will be that amount which is the lowest dose effective to produce a therapeutic effect, or a dose low enough to produce a therapeutic effect without causing side effects.

RNAi Agents to KRAS in Combination with Other Therapies

In addition to a therapeutically-effective dosage of one or more RNAi agents to KRAS, the pharmaceutical compositions of the present disclosure can comprise or be used in conjunction with an additional disease treatment which works synergistically with the RNAi agent.

In addition, many tumors contain mutations in both KRAS and one or more other genes. For example, some lung cancer or colorectal tumor cells or other types of cancer can have mutations in both KRAS and one or more of: EGFR, GLUT1, PI3K, RAF, and APC. Haigis et al. 2008 Nature Gen. 40: 600-608; Karapetis et al. 2008 N. E. J. Med. 359: 1757-1765; and Yun et al. 2009 Science 325: 1555-1559. Effective combination therapy can comprise a KRAS RNAi agent combined with an agent which targets the other mutated hyper-active or overexpressed protein, or which is an approved therapy for the cancer type being treated.

RNAi agents to KRAS can thus be effective when used in a regimen involving other disease treatments. Some of these treatments are not effective when the patient has an activating mutation in KRAS. However, the RNAi agent to KRAS can reduce the overall amount of KRAS, improving the efficacy of disease treatments which are not effective in the presence of an activating KRAS mutation.

Furthermore, proliferation, survival and/or apoptosis resistance of KRAS-defective tumor cells may require another factor such as NF-kappa-B or I-kappa-B kinase TBK1. Meylan et al. 2009 Nature 462: 104-107; and Barbie et al. 2009 Nature 462: 108-112. Thus, in some embodiments, effective combination therapy can comprise administration of a KRAS RNAi agent and administration (or co-administration) of a treatment which targets the other factor (e.g., a siRNA, antibody or small molecule which targets NF-kappa-B or TBK1).

Thus, the KRAS RNAi agent can be used in a combination therapy along with any other therapeutic composition or method or therapy, including those which specifically target another factor, or which are otherwise known to be effective in treatment of a KRAS-related disease.

In the treatment of these KRAS-related diseases, the RNAi agent(s) and additional disease treatment(s) can be administered in any order, simultaneously or sequentially, or in multiple doses over time. Administration of the RNAi agent and the additional treatment can be, for example, simultaneous, concurrent, separate or sequential.

Simultaneous administration may, e.g., take place in the form of one fixed combination with two or more active ingredients, or by simultaneously administering two or more active ingredients that are formulated independently. Sequential use (administration) preferably means administration of one (or more) components of a combination at one time point, other components at a different time point, that is, in a chronically staggered manner, preferably such that the combination shows more efficiency than the single compounds administered independently (especially showing synergism). Separate use (administration) preferably means administration of the components of the combination independently of each other at different time points, preferably meaning that the components (a) and (b) are administered such that no overlap of significant measurable blood levels of both compounds are present in an overlapping manner (at the same time).

Also combinations of two or more of sequential, separate and simultaneous administration are possible, preferably such that the combination component-drugs show a joint therapeutic effect that exceeds the effect found when the combination component-drugs are used independently at time intervals so large that no mutual effect on their therapeutic efficiency can be found, a synergistic effect being especially preferred.

The term "delay of progression" as used herein means administration of the combination to patients being in a pre-stage or in an early phase, of the first manifestation or a relapse of the disease to be treated, in which patients, e.g., a pre-form of the corresponding disease is diagnosed or which patients are in a condition, e.g., during a medical treatment or a condition resulting from an accident, under which it is likely that a corresponding disease will develop.

"Jointly therapeutically active" or "joint therapeutic effect" means that the compounds may be given separately (in a chronically staggered manner, especially a sequence-specific manner) in such time intervals that they preferably, in the warm-blooded animal, especially human, to be treated, still show a (preferably synergistic) interaction (joint therapeutic effect). Whether this is the case, can inter alia be determined by following the blood levels, showing that both compounds are present in the blood of the human to be treated at least during certain time intervals.

The present disclosure thus encompasses a combination therapy comprising both a KRAS RNAi agent and one or more additional therapies for KRAS-related diseases known in the art.

The use of known treatments for any of KRAS-related diseases is within the capabilities of one of ordinary skill in the art. Any such additional treatment can be used in conjunction with a RNAi agent to KRAS.

The dosages of the additional treatments and RNAi agents can be easily determined by one of ordinary skill in the art, and as described herein.

**Embodiments comprising one or more efficacious RNAi agents to KRAS**

In various embodiments of the present disclosure, the present disclosure comprises a RNAi agent demonstrating at least about 40, 50, 60, 70, 80, 90 or 95% knockdown (KD) (i.e., no more than about 60, 50, 40, 30, 20, 10, or 5% residual gene activity, respectively) of the KRAS gene at an *in vitro* concentration of 10 or 0.1 nM as assayed in RKO cells.

In various embodiments of the present disclosure, the composition comprises one or more RNAi agents capable of a Fold-Change at 10 nM at 24 hr of < 0.05, < 0.10, < 0.20, < 0.30, < 0.40 (indicating a KRAS gene knock-down of at least 95, 90, 80, 70, or 60%, respectively, at a concentration of 10 nM at 24 hrs in RKO cells). RNAi agents capable of these levels of activity are disclosed in the Tables herein.

**Embodiments comprising one or more efficacious RNAi agents to KRAS**

In various embodiments of the present disclosure, the composition comprises one or more RNAi agents capable of a Fold-Change at 0.1 nM at 120 hr of < 0.30, < 0.20, or < 0.10 (indicating a KRAS gene knock-down of at least 70, 80, or 90%, respectively, at a concentration of 0.1 nM at 120 hrs in RKO cells). RNAi agents capable of these levels of activity are disclosed in the Tables herein.

**Various embodiments comprising one or more RNAi agents to KRAS with low EC50**

In various embodiments of the present disclosure, the composition comprises one or more RNAi agents capable of mediating a 50% gene knockdown (EC50) of KRAS at a low concentration in RKO cells. For many of the RNAi agents listed herein, an estimated EC50 is calculated. Cells are treated at 10nM, 1nM, 0.1nM, 0.01nM, and 0.001nM of RNAi agent, and the data fit to a curve. The indicated EC50 is an estimated EC50 calculated from available data that is expected to give 50% gene knock-down of KRAS in RKO cells. In various embodiments of the present disclosure, the composition comprises one or more RNAi agents capable of mediating a 50% gene knockdown (EC50) at 100, 75, 50, 25, 10, 5, 2.5, 1, 0.75, 0.5, 0.25, 0.2, 0.12, 0.1, 0.09, 0.08, 0.07, 0.06, 0.05, 0.04, 0.03, or 0.02 nM or less in RKO cells. RNAi agents capable of these levels of activity are disclosed in the Tables herein.

**Specific embodiments of RNAi agents to KRAS**

In one embodiment, the present disclosure pertains to: a composition comprising any one or more of: a RNAi agent comprising a sense strand and an anti-sense strand, wherein the anti-sense strand comprises at least 15 contiguous nucleotides differing by 0, 1, 2, or 3 nucleotides from the anti-sense strand of: any RNAi agent from any of Tables 1 to 6 and modified or unmodified variants thereof.

In one embodiment, the composition comprises any one or more of: a RNAi agent comprising a sense strand and an anti-sense strand, wherein the anti-sense strand comprises at least 15 contiguous nucleotides differing by 0 nucleotides from the anti-sense strand of any RNAi agent from any of Tables 1 to 6, and modified and unmodified variants thereof.

In one embodiment, the present disclosure pertains to a composition comprising any one or more of: any RNAi agent from any of Tables 1 to 6, and modified and unmodified variants thereof.

In one embodiment, the present disclosure pertains to a composition comprising any one or more of: a RNAi agent comprising an anti-sense strand comprising the sequence of the anti-sense strand of any RNAi agent from any of Tables 1 to 6, and modified and unmodified variants thereof.

In one embodiment, the present disclosure pertains to a composition comprising any one or more of: a RNAi agent comprises an anti-sense strand consisting of the sequence of the anti-sense strand of any RNAi agent from any of Tables 1 to 6, and modified and unmodified variants thereof.

In one embodiment, the present disclosure pertains to a composition comprising any one or more of: a RNAi agent comprising a first strand and a second strand, wherein the sequence of the first strand is the second of the first strand of any RNAi agent from any of Tables 1 to 6, and modified and unmodified variants thereof.

In one embodiment, the present disclosure pertains to a composition comprising any one or more of: a RNAi agent comprising a first and a second strand, wherein the sequences of the first and the second strand are the sequences of the first and second strand of any RNAi agent from any of Tables 1 to 6, and modified and unmodified variants thereof.

In one embodiment, the present disclosure pertains to a composition comprising any one or more of: a RNAi agent comprising a first strand and a second strand, wherein the sequence of the first strand is the second of the first strand of any RNAi agent from any of Tables 1 to 6, and modified and unmodified variants thereof, wherein the first and/or second strand further comprise up to about 20 additional nucleotides.

Specific embodiments of RNAi agents to KRAS

In one embodiment, the present disclosure pertains to: a composition comprising any one or more of: a RNAi agent comprising a first strand and a second strand, wherein the first strand comprises at least 15 contiguous nucleotides differing by 0, 1, 2, or 3 nucleotides from the first strand of: any RNAi agent from any of Tables 1 to 6 or modified or unmodified variants thereof.

In one embodiment, the present disclosure pertains to: a composition comprising any one or more of: a RNAi agent comprising a first strand and a second strand, wherein the first strand comprises at least 15 contiguous nucleotides from the first strand of any RNAi agent from any of Tables 1 to 6, or modified and unmodified variants thereof.

In one embodiment, the present disclosure pertains to a composition comprising any one or more of: a RNAi agent comprising a first strand and a second strand, wherein the sequence of the first strand is the sequence of a first strand of any RNAi agent from any of Tables 1 to 6, or modified and unmodified variants thereof.

In one embodiment, the present disclosure pertains to a composition comprising any one or more of: a RNAi agent comprising a first and a second strand, wherein the sequences of the first and/or second strands comprise the sequences of a first and/or second strands of any RNAi agent from any of Tables 1 to 6, or modified and unmodified variants thereof.

In one embodiment, the present disclosure pertains to a composition comprising any one or more of: a RNAi agent comprising a first and a second strand, wherein the sequences of the first and the second strand are the sequences of the first and second strand of any RNAi agent from any of Tables 1 to 6, or modified and unmodified variants thereof.

In one embodiment, the present disclosure pertains to a composition comprising any one or more of: a RNAi agent comprising a first and a second strand, wherein the sequence of the first and/or second strand is the sequence of a first and/or second strand of any RNAi agent from any of Tables 1 to 6, or modified and unmodified variants thereof, wherein the first and/or second strand further comprise up to about 20 additional nucleotides.

**Specific embodiments comprising at least 15 contiguous nucleotides differing from 0 to 3 nt from RNAi agents disclosed herein**

In one embodiment, the present disclosure pertains to: a composition comprising any one or more of: a RNAi agent comprising a first and a second strand, wherein the first strand comprises at least 15 contiguous nucleotides (nt) differing by 0, 1, 2, or 3 nt from a first strand of: any RNAi agent from any of Tables 1 to 6 or modified or unmodified variants thereof.

Thus, in various embodiments, the present disclosure pertains to a composition comprising any one or more of the following:

A RNAi agent comprising a first and a second strand, wherein the first strand comprises at least 15 contiguous nt differing by 0, 1, 2, or 3 nt from a first strand of: AD-35523.3 (SEQ ID NO: 428), or a modified or unmodified variant thereof.

A RNAi agent comprising a first and a second strand, wherein the first strand comprises at least 15 contiguous nt differing by 0, 1, 2, or 3 nt from a first strand of: AD-35529.1 (SEQ ID NO: 429), or a modified or unmodified variant thereof.

A RNAi agent comprising a first and a second strand, wherein the first strand comprises at least 15 contiguous nt differing by 0, 1, 2, or 3 nt from a first strand of: AD-35535.4 (SEQ ID NO: 430), or a modified or unmodified variant thereof.

A RNAi agent comprising a first and a second strand, wherein the first strand comprises at least 15 contiguous nt differing by 0, 1, 2, or 3 nt from a first strand of: AD-35541.4 (SEQ ID NO: 431), or a modified or unmodified variant thereof.

A RNAi agent comprising a first and a second strand, wherein the first strand comprises at least 15 contiguous nt differing by 0, 1, 2, or 3 nt from a first strand of: AD-35547.4 (SEQ ID NO: 432), or a modified or unmodified variant thereof.

A RNAi agent comprising a first and a second strand, wherein the first strand comprises at least 15 contiguous nt differing by 0, 1, 2, or 3 nt from a first strand of: AD-35553.4 (SEQ ID NO: 433), or a modified or unmodified variant thereof.

A RNAi agent comprising a first and a second strand, wherein the first strand comprises at least 15 contiguous nt differing by 0, 1, 2, or 3 nt from a first strand of: AD-35559.4 (SEQ ID NO: 434), or a modified or unmodified variant thereof.

A RNAi agent comprising a first and a second strand, wherein the first strand comprises at least 15 contiguous nt differing by 0, 1, 2, or 3 nt from a first strand of: AD-35565.4 (SEQ ID NO: 435), or a modified or unmodified variant thereof.

A RNAi agent comprising a first and a second strand, wherein the first strand comprises at least 15 contiguous nt differing by 0, 1, 2, or 3 nt from a first strand of: AD-35524.4 (SEQ ID NO: 436), or a modified or unmodified variant thereof.

A RNAi agent comprising a first and a second strand, wherein the first strand comprises at least 15 contiguous nt differing by 0, 1, 2, or 3 nt from a first strand of: AD-35530.4 (SEQ ID NO: 437), or a modified or unmodified variant thereof.

A RNAi agent comprising a first and a second strand, wherein the first strand comprises at least 15 contiguous nt differing by 0, 1, 2, or 3 nt from a first strand of: AD-35536.4 (SEQ ID NO: 438), or a modified or unmodified variant thereof.

A RNAi agent comprising a first and a second strand, wherein the first strand comprises at least 15 contiguous nt differing by 0, 1, 2, or 3 nt from a first strand of: AD-35542.4 (SEQ ID NO: 439), or a modified or unmodified variant thereof.

A RNAi agent comprising a first and a second strand, wherein the first strand comprises at least 15 contiguous nt differing by 0, 1, 2, or 3 nt from a first strand of: AD-35548.4 (SEQ ID NO: 440), or a modified or unmodified variant thereof.

A RNAi agent comprising a first and a second strand, wherein the first strand comprises at least 15 contiguous nt differing by 0, 1, 2, or 3 nt from a first strand of: AD-35554.4 (SEQ ID NO: 441), or a modified or unmodified variant thereof.

A RNAi agent comprising a first and a second strand, wherein the first strand comprises at least 15 contiguous nt differing by 0, 1, 2, or 3 nt from a first strand of: AD-35560.2 (SEQ ID NO: 442), or a modified or unmodified variant thereof.

A RNAi agent comprising a first and a second strand, wherein the first strand comprises at least 15 contiguous nt differing by 0, 1, 2, or 3 nt from a first strand of: AD-35566.4 (SEQ ID NO: 443), or a modified or unmodified variant thereof.

A RNAi agent comprising a first and a second strand, wherein the first strand comprises at least 15 contiguous nt differing by 0, 1, 2, or 3 nt from a first strand of: AD-35525.2 (SEQ ID NO: 444), or a modified or unmodified variant thereof.

A RNAi agent comprising a first and a second strand, wherein the first strand comprises at least 15 contiguous nt differing by 0, 1, 2, or 3 nt from a first strand of: AD-35531.4 (SEQ ID NO: 445), or a modified or unmodified variant thereof.

A RNAi agent comprising a first and a second strand, wherein the first strand comprises at least 15 contiguous nt differing by 0, 1, 2, or 3 nt from a first strand of: AD-35537.4 (SEQ ID NO: 446), or a modified or unmodified variant thereof.

A RNAi agent comprising a first and a second strand, wherein the first strand comprises at least 15 contiguous nt differing by 0, 1, 2, or 3 nt from a first strand of: AD-35543.2 (SEQ ID NO: 447), or a modified or unmodified variant thereof.

A RNAi agent comprising a first and a second strand, wherein the first strand comprises at least 15 contiguous nt differing by 0, 1, 2, or 3 nt from a first strand of: AD-35549.4 (SEQ ID NO: 448), or a modified or unmodified variant thereof.

A RNAi agent comprising a first and a second strand, wherein the first strand comprises at least 15 contiguous nt differing by 0, 1, 2, or 3 nt from a first strand of: AD-35555.4 (SEQ ID NO: 449), or a modified or unmodified variant thereof.

A RNAi agent comprising a first and a second strand, wherein the first strand comprises at least 15 contiguous nt differing by 0, 1, 2, or 3 nt from a first strand of: AD-35561.4 (SEQ ID NO: 450), or a modified or unmodified variant thereof.

A RNAi agent comprising a first and a second strand, wherein the first strand comprises at least 15 contiguous nt differing by 0, 1, 2, or 3 nt from a first strand of: AD-35567.4 (SEQ ID NO: 451), or a modified or unmodified variant thereof.

A RNAi agent comprising a first and a second strand, wherein the first strand comprises at least 15 contiguous nt differing by 0, 1, 2, or 3 nt from a first strand of: AD-35526.4 (SEQ ID NO: 452), or a modified or unmodified variant thereof.

A RNAi agent comprising a first and a second strand, wherein the first strand comprises at least 15 contiguous nt differing by 0, 1, 2, or 3 nt from a first strand of: AD-35532.4 (SEQ ID NO: 453), or a modified or unmodified variant thereof.

A RNAi agent comprising a first and a second strand, wherein the first strand comprises at least 15 contiguous nt differing by 0, 1, 2, or 3 nt from a first strand of: AD-35538.4 (SEQ ID NO: 454), or a modified or unmodified variant thereof.

A RNAi agent comprising a first and a second strand, wherein the first strand comprises at least 15 contiguous nt differing by 0, 1, 2, or 3 nt from a first strand of: AD-35544.4 (SEQ ID NO: 455), or a modified or unmodified variant thereof.

A RNAi agent comprising a first and a second strand, wherein the first strand comprises at least 15 contiguous nt differing by 0, 1, 2, or 3 nt from a first strand of: AD-35550.4 (SEQ ID NO: 456), or a modified or unmodified variant thereof.

A RNAi agent comprising a first and a second strand, wherein the first strand comprises at least 15 contiguous nt differing by 0, 1, 2, or 3 nt from a first strand of: AD-35556.4 (SEQ ID NO: 457), or a modified or unmodified variant thereof.

A RNAi agent comprising a first and a second strand, wherein the first strand comprises at least 15 contiguous nt differing by 0, 1, 2, or 3 nt from a first strand of: AD-35562.4 (SEQ ID NO: 458), or a modified or unmodified variant thereof.

A RNAi agent comprising a first and a second strand, wherein the first strand comprises at least 15 contiguous nt differing by 0, 1, 2, or 3 nt from a first strand of: AD-35568.4 (SEQ ID NO: 459), or a modified or unmodified variant thereof.

A RNAi agent comprising a first and a second strand, wherein the first strand comprises at least 15 contiguous nt differing by 0, 1, 2, or 3 nt from a first strand of: AD-35527.4 (SEQ ID NO: 460), or a modified or unmodified variant thereof.
A RNAi agent comprising a first and a second strand, wherein the first strand comprises at least 15 contiguous nt differing by 0, 1, 2, or 3 nt from a first strand of: AD-35533.4 (SEQ ID NO: 461), or a modified or unmodified variant thereof.
A RNAi agent comprising a first and a second strand, wherein the first strand comprises at least 15 contiguous nt differing by 0, 1, 2, or 3 nt from a first strand of: AD-35539.4 (SEQ ID NO: 462), or a modified or unmodified variant thereof.
A RNAi agent comprising a first and a second strand, wherein the first strand comprises at least 15 contiguous nt differing by 0, 1, 2, or 3 nt from a first strand of: AD-35545.4 (SEQ ID NO: 463), or a modified or unmodified variant thereof.
A RNAi agent comprising a first and a second strand, wherein the first strand comprises at least 15 contiguous nt differing by 0, 1, 2, or 3 nt from a first strand of: AD-35551.4 (SEQ ID NO: 464), or a modified or unmodified variant thereof.
A RNAi agent comprising a first and a second strand, wherein the first strand comprises at least 15 contiguous nt differing by 0, 1, 2, or 3 nt from a first strand of: AD-35563.4 (SEQ ID NO: 465), or a modified or unmodified variant thereof.
A RNAi agent comprising a first and a second strand, wherein the first strand comprises at least 15 contiguous nt differing by 0, 1, 2, or 3 nt from a first strand of: AD-35528.4 (SEQ ID NO: 466), or a modified or unmodified variant thereof.
A RNAi agent comprising a first and a second strand, wherein the first strand comprises at least 15 contiguous nt differing by 0, 1, 2, or 3 nt from a first strand of: AD-35540.4 (SEQ ID NO: 467), or a modified or unmodified variant thereof.
A RNAi agent comprising a first and a second strand, wherein the first strand comprises at least 15 contiguous nt differing by 0, 1, 2, or 3 nt from a first strand of: AD-35546.4 (SEQ ID NO: 468), or a modified or unmodified variant thereof.
A RNAi agent comprising a first and a second strand, wherein the first strand comprises at least 15 contiguous nt differing by 0, 1, 2, or 3 nt from a first strand of: AD-35552.4 (SEQ ID NO: 469), or a modified or unmodified variant thereof.
A RNAi agent comprising a first and a second strand, wherein the first strand comprises at least 15 contiguous nt differing by 0, 1, 2, or 3 nt from a first strand of: AD-35558.4 (SEQ ID NO: 470), or a modified or unmodified variant thereof.
A RNAi agent comprising a first and a second strand, wherein the first strand comprises at least 15 contiguous nt differing by 0, 1, 2, or 3 nt from a first strand of: AD-35564.4 (SEQ ID NO: 471), or a modified or unmodified variant thereof.
A RNAi agent comprising a first and a second strand, wherein the first strand comprises at least 15 contiguous nt differing by 0, 1, 2, or 3 nt from a first strand of: AD-35570.4 (SEQ ID NO: 472), or a modified or unmodified variant thereof.
A RNAi agent comprising a first and a second strand, wherein the first strand comprises at least 15 contiguous nt differing by 0, 1, 2, or 3 nt from a first strand of: AD-35571.4 (SEQ ID NO: 473), or a modified or unmodified variant thereof.
A RNAi agent comprising a first and a second strand, wherein the first strand comprises at least 15 contiguous nt differing by 0, 1, 2, or 3 nt from a first strand of: AD-35577.4 (SEQ ID NO: 474), or a modified or unmodified variant thereof.
A RNAi agent comprising a first and a second strand, wherein the first strand comprises at least 15 contiguous nt differing by 0, 1, 2, or 3 nt from a first strand of: AD-35583.4 (SEQ ID NO: 475), or a modified or unmodified variant thereof.
A RNAi agent comprising a first and a second strand, wherein the first strand comprises at least 15 contiguous nt differing by 0, 1, 2, or 3 nt from a first strand of: AD-35589.4 (SEQ ID NO: 476), or a modified or unmodified variant thereof.
A RNAi agent comprising a first and a second strand, wherein the first strand comprises at least 15 contiguous nt differing by 0, 1, 2, or 3 nt from a first strand of: AD-35595.4 (SEQ ID NO: 477), or a modified or unmodified variant thereof.
A RNAi agent comprising a first and a second strand, wherein the first strand comprises at least 15 contiguous nt differing by 0, 1, 2, or 3 nt from a first strand of: AD-35601.4 (SEQ ID NO: 478), or a modified or unmodified variant thereof.
A RNAi agent comprising a first and a second strand, wherein the first strand comprises at least 15 contiguous nt differing by 0, 1, 2, or 3 nt from a first strand of: AD-35607.4 (SEQ ID NO: 479), or a modified or unmodified variant thereof.
A RNAi agent comprising a first and a second strand, wherein the first strand comprises at least 15 contiguous nt differing by 0, 1, 2, or 3 nt from a first strand of: AD-35613.4 (SEQ ID NO: 480), or a modified or unmodified variant thereof.
A RNAi agent comprising a first and a second strand, wherein the first strand comprises at least 15 contiguous nt differing by 0, 1, 2, or 3 nt from a first strand of: AD-35572.4 (SEQ ID NO: 481), or a modified or unmodified variant thereof.
A RNAi agent comprising a first and a second strand, wherein the first strand comprises at least 15 contiguous nt differing by 0, 1, 2, or 3 nt from a first strand of: AD-35578.4 (SEQ ID NO: 482), or a modified or unmodified variant thereof.
A RNAi agent comprising a first and a second strand, wherein the first strand comprises at least 15 contiguous nt differing by 0, 1, 2, or 3 nt from a first strand of: AD-35584.4 (SEQ ID NO: 483), or a modified or unmodified variant thereof.
A RNAi agent comprising a first and a second strand, wherein the first strand comprises at least 15 contiguous nt differing by 0, 1, 2, or 3 nt from a first strand of: AD-35590.4 (SEQ ID NO: 484), or a modified or unmodified variant thereof.
A RNAi agent comprising a first and a second strand, wherein the first strand comprises at least 15 contiguous nt differing by 0, 1, 2, or 3 nt from a first strand of: AD-35596.4 (SEQ ID NO: 485), or a modified or unmodified variant thereof.
A RNAi agent comprising a first and a second strand, wherein the first strand comprises at least 15 contiguous nt differing by 0, 1, 2, or 3 nt from a first strand of: AD-35602.4 (SEQ ID NO: 486), or a modified or unmodified variant thereof.
A RNAi agent comprising a first and a second strand, wherein the first strand comprises at least 15 contiguous nt differing by 0, 1, 2, or 3 nt from a first strand of: AD-35608.4 (SEQ ID NO: 487), or a modified or unmodified variant thereof.
A RNAi agent comprising a first and a second strand, wherein the first strand comprises at least 15 contiguous nt differing by 0, 1, 2, or 3 nt from a first strand of: AD-35614.4 (SEQ ID NO: 488), or a modified or unmodified variant thereof.

A RNAi agent comprising a first and a second strand, wherein the first strand comprises at least 15 contiguous nt differing by 0, 1, 2, or 3 nt from a first strand of: AD-35573.4 (SEQ ID NO: 489), or a modified or unmodified variant thereof.
A RNAi agent comprising a first and a second strand, wherein the first strand comprises at least 15 contiguous nt differing by 0, 1, 2, or 3 nt from a first strand of: AD-35579.4 (SEQ ID NO: 490), or a modified or unmodified variant thereof.
A RNAi agent comprising a first and a second strand, wherein the first strand comprises at least 15 contiguous nt differing by 0, 1, 2, or 3 nt from a first strand of: AD-35585.4 (SEQ ID NO: 491), or a modified or unmodified variant thereof.
A RNAi agent comprising a first and a second strand, wherein the first strand comprises at least 15 contiguous nt differing by 0, 1, 2, or 3 nt from a first strand of: AD-35591.4 (SEQ ID NO: 492), or a modified or unmodified variant thereof.
A RNAi agent comprising a first and a second strand, wherein the first strand comprises at least 15 contiguous nt differing by 0, 1, 2, or 3 nt from a first strand of: AD-35597.4 (SEQ ID NO: 493), or a modified or unmodified variant thereof.
A RNAi agent comprising a first and a second strand, wherein the first strand comprises at least 15 contiguous nt differing by 0, 1, 2, or 3 nt from a first strand of: AD-35603.4 (SEQ ID NO: 494), or a modified or unmodified variant thereof.
A RNAi agent comprising a first and a second strand, wherein the first strand comprises at least 15 contiguous nt differing by 0, 1, 2, or 3 nt from a first strand of: AD-35609.4 (SEQ ID NO: 495), or a modified or unmodified variant thereof.
A RNAi agent comprising a first and a second strand, wherein the first strand comprises at least 15 contiguous nt differing by 0, 1, 2, or 3 nt from a first strand of: AD-35615.4 (SEQ ID NO: 496), or a modified or unmodified variant thereof.
A RNAi agent comprising a first and a second strand, wherein the first strand comprises at least 15 contiguous nt differing by 0, 1, 2, or 3 nt from a first strand of: AD-35574.4 (SEQ ID NO: 497), or a modified or unmodified variant thereof.
A RNAi agent comprising a first and a second strand, wherein the first strand comprises at least 15 contiguous nt differing by 0, 1, 2, or 3 nt from a first strand of: AD-35580.1 (SEQ ID NO: 498), or a modified or unmodified variant thereof.
A RNAi agent comprising a first and a second strand, wherein the first strand comprises at least 15 contiguous nt differing by 0, 1, 2, or 3 nt from a first strand of: AD-35586.4 (SEQ ID NO: 499), or a modified or unmodified variant thereof.
A RNAi agent comprising a first and a second strand, wherein the first strand comprises at least 15 contiguous nt differing by 0, 1, 2, or 3 nt from a first strand of: AD-35592.4 (SEQ ID NO: 500), or a modified or unmodified variant thereof.
A RNAi agent comprising a first and a second strand, wherein the first strand comprises at least 15 contiguous nt differing by 0, 1, 2, or 3 nt from a first strand of: AD-35598.4 (SEQ ID NO: 501), or a modified or unmodified variant thereof.
A RNAi agent comprising a first and a second strand, wherein the first strand comprises at least 15 contiguous nt differing by 0, 1, 2, or 3 nt from a first strand of: AD-35604.4 (SEQ ID NO: 502), or a modified or unmodified variant thereof.
A RNAi agent comprising a first and a second strand, wherein the first strand comprises at least 15 contiguous nt differing by 0, 1, 2, or 3 nt from a first strand of: AD-35610.4 (SEQ ID NO: 503), or a modified or unmodified variant thereof.
A RNAi agent comprising a first and a second strand, wherein the first strand comprises at least 15 contiguous nt differing by 0, 1, 2, or 3 nt from a first strand of: AD-35616.4 (SEQ ID NO: 504), or a modified or unmodified variant thereof.
A RNAi agent comprising a first and a second strand, wherein the first strand comprises at least 15 contiguous nt differing by 0, 1, 2, or 3 nt from a first strand of: AD-35575.4 (SEQ ID NO: 505), or a modified or unmodified variant thereof.
A RNAi agent comprising a first and a second strand, wherein the first strand comprises at least 15 contiguous nt differing by 0, 1, 2, or 3 nt from a first strand of: AD-35581.4 (SEQ ID NO: 506), or a modified or unmodified variant thereof.
A RNAi agent comprising a first and a second strand, wherein the first strand comprises at least 15 contiguous nt differing by 0, 1, 2, or 3 nt from a first strand of: AD-35587.4 (SEQ ID NO: 507), or a modified or unmodified variant thereof.
A RNAi agent comprising a first and a second strand, wherein the first strand comprises at least 15 contiguous nt differing by 0, 1, 2, or 3 nt from a first strand of: AD-35593.4 (SEQ ID NO: 508), or a modified or unmodified variant thereof.
A RNAi agent comprising a first and a second strand, wherein the first strand comprises at least 15 contiguous nt differing by 0, 1, 2, or 3 nt from a first strand of: AD-35599.4 (SEQ ID NO: 509), or a modified or unmodified variant thereof.
A RNAi agent comprising a first and a second strand, wherein the first strand comprises at least 15 contiguous nt differing by 0, 1, 2, or 3 nt from a first strand of: AD-35605.4 (SEQ ID NO: 510), or a modified or unmodified variant thereof.
A RNAi agent comprising a first and a second strand, wherein the first strand comprises at least 15 contiguous nt differing by 0, 1, 2, or 3 nt from a first strand of: AD-35611.4 (SEQ ID NO: 511), or a modified or unmodified variant thereof.
A RNAi agent comprising a first and a second strand, wherein the first strand comprises at least 15 contiguous nt differing by 0, 1, 2, or 3 nt from a first strand of: AD-35617.4 (SEQ ID NO: 512), or a modified or unmodified variant thereof.
A RNAi agent comprising a first and a second strand, wherein the first strand comprises at least 15 contiguous nt differing by 0, 1, 2, or 3 nt from a first strand of: AD-35576.4 (SEQ ID NO: 513), or a modified or unmodified variant thereof.
A RNAi agent comprising a first and a second strand, wherein the first strand comprises at least 15 contiguous nt differing by 0, 1, 2, or 3 nt from a first strand of: AD-35588.4 (SEQ ID NO: 514), or a modified or unmodified variant thereof.
A RNAi agent comprising a first and a second strand, wherein the first strand comprises at least 15 contiguous nt differing by 0, 1, 2, or 3 nt from a first strand of: AD-35667.1 (SEQ ID NO: 515), or a modified or unmodified variant thereof.
A RNAi agent comprising a first and a second strand, wherein the first strand comprises at least 15 contiguous nt differing by 0, 1, 2, or 3 nt from a first strand of: AD-35673.1 (SEQ ID NO: 516), or a modified or unmodified variant thereof.
A RNAi agent comprising a first and a second strand, wherein the first strand comprises at least 15 contiguous nt differing by 0, 1, 2, or 3 nt from a first strand of: AD-35679.1 (SEQ ID NO: 517), or a modified or unmodified variant thereof.

A RNAi agent comprising a first and a second strand, wherein the first strand comprises at least 15 contiguous nt differing by 0, 1, 2, or 3 nt from a first strand of: AD-35685.1 (SEQ ID NO: 518), or a modified or unmodified variant thereof.

A RNAi agent comprising a first and a second strand, wherein the first strand comprises at least 15 contiguous nt differing by 0, 1, 2, or 3 nt from a first strand of: AD-35691.1 (SEQ ID NO: 519), or a modified or unmodified variant thereof.

A RNAi agent comprising a first and a second strand, wherein the first strand comprises at least 15 contiguous nt differing by 0, 1, 2, or 3 nt from a first strand of: AD-35557.1 (SEQ ID NO: 520), or a modified or unmodified variant thereof.

A RNAi agent comprising a first and a second strand, wherein the first strand comprises at least 15 contiguous nt differing by 0, 1, 2, or 3 nt from a first strand of: AD-35618.1 (SEQ ID NO: 521), or a modified or unmodified variant thereof.

A RNAi agent comprising a first and a second strand, wherein the first strand comprises at least 15 contiguous nt differing by 0, 1, 2, or 3 nt from a first strand of: AD-35569.1 (SEQ ID NO: 522), or a modified or unmodified variant thereof.

A RNAi agent comprising a first and a second strand, wherein the first strand comprises at least 15 contiguous nt differing by 0, 1, 2, or 3 nt from a first strand of: AD-35534.1 (SEQ ID NO: 523), or a modified or unmodified variant thereof.

A RNAi agent comprising a first and a second strand, wherein the first strand comprises at least 15 contiguous nt differing by 0, 1, 2, or 3 nt from a first strand of: AD-35582.1 (SEQ ID NO: 524), or a modified or unmodified variant thereof.

A RNAi agent comprising a first and a second strand, wherein the first strand comprises at least 15 contiguous nt differing by 0, 1, 2, or 3 nt from a first strand of: AD-35594.1 (SEQ ID NO: 525), or a modified or unmodified variant thereof.

A RNAi agent comprising a first and a second strand, wherein the first strand comprises at least 15 contiguous nt differing by 0, 1, 2, or 3 nt from a first strand of: AD-35600.1 (SEQ ID NO: 526), or a modified or unmodified variant thereof.

A RNAi agent comprising a first and a second strand, wherein the first strand comprises at least 15 contiguous nt differing by 0, 1, 2, or 3 nt from a first strand of: AD-35606.1 (SEQ ID NO: 527), or a modified or unmodified variant thereof.

A RNAi agent comprising a first and a second strand, wherein the first strand comprises at least 15 contiguous nt differing by 0, 1, 2, or 3 nt from a first strand of: AD-35612.1 (SEQ ID NO: 528), or a modified or unmodified variant thereof.

A RNAi agent comprising a first and a second strand, wherein the first strand comprises at least 15 contiguous nt differing by 0, 1, 2, or 3 nt from a first strand of: AD-38127.1 (SEQ ID NO: 529), or a modified or unmodified variant thereof.

A RNAi agent comprising a first and a second strand, wherein the first strand comprises at least 15 contiguous nt differing by 0, 1, 2, or 3 nt from a first strand of: AD-38133.1 (SEQ ID NO: 530), or a modified or unmodified variant thereof.

A RNAi agent comprising a first and a second strand, wherein the first strand comprises at least 15 contiguous nt differing by 0, 1, 2, or 3 nt from a first strand of: AD-38139.1 (SEQ ID NO: 531), or a modified or unmodified variant thereof.

A RNAi agent comprising a first and a second strand, wherein the first strand comprises at least 15 contiguous nt differing by 0, 1, 2, or 3 nt from a first strand of: AD-38145.1 (SEQ ID NO: 532), or a modified or unmodified variant thereof.

A RNAi agent comprising a first and a second strand, wherein the first strand comprises at least 15 contiguous nt differing by 0, 1, 2, or 3 nt from a first strand of: AD-38151.1 (SEQ ID NO: 533), or a modified or unmodified variant thereof.

A RNAi agent comprising a first and a second strand, wherein the first strand comprises at least 15 contiguous nt differing by 0, 1, 2, or 3 nt from a first strand of: AD-38157.1 (SEQ ID NO: 534), or a modified or unmodified variant thereof.

A RNAi agent comprising a first and a second strand, wherein the first strand comprises at least 15 contiguous nt differing by 0, 1, 2, or 3 nt from a first strand of: AD-38163.1 (SEQ ID NO: 535), or a modified or unmodified variant thereof.

A RNAi agent comprising a first and a second strand, wherein the first strand comprises at least 15 contiguous nt differing by 0, 1, 2, or 3 nt from a first strand of: AD-38169.1 (SEQ ID NO: 536), or a modified or unmodified variant thereof.

A RNAi agent comprising a first and a second strand, wherein the first strand comprises at least 15 contiguous nt differing by 0, 1, 2, or 3 nt from a first strand of: AD-38128.1 (SEQ ID NO: 537), or a modified or unmodified variant thereof.

A RNAi agent comprising a first and a second strand, wherein the first strand comprises at least 15 contiguous nt differing by 0, 1, 2, or 3 nt from a first strand of: AD-38134.1 (SEQ ID NO: 538), or a modified or unmodified variant thereof.

A RNAi agent comprising a first and a second strand, wherein the first strand comprises at least 15 contiguous nt differing by 0, 1, 2, or 3 nt from a first strand of: AD-38140.1 (SEQ ID NO: 539), or a modified or unmodified variant thereof.

A RNAi agent comprising a first and a second strand, wherein the first strand comprises at least 15 contiguous nt differing by 0, 1, 2, or 3 nt from a first strand of: AD-38146.1 (SEQ ID NO: 540), or a modified or unmodified variant thereof.

A RNAi agent comprising a first and a second strand, wherein the first strand comprises at least 15 contiguous nt differing by 0, 1, 2, or 3 nt from a first strand of: AD-38152.1 (SEQ ID NO: 541), or a modified or unmodified variant thereof.

A RNAi agent comprising a first and a second strand, wherein the first strand comprises at least 15 contiguous nt differing by 0, 1, 2, or 3 nt from a first strand of: AD-38158.1 (SEQ ID NO: 542), or a modified or unmodified variant thereof.

A RNAi agent comprising a first and a second strand, wherein the first strand comprises at least 15 contiguous nt differing by 0, 1, 2, or 3 nt from a first strand of: AD-38164.1 (SEQ ID NO: 543), or a modified or unmodified variant thereof.

A RNAi agent comprising a first and a second strand, wherein the first strand comprises at least 15 contiguous nt differing by 0, 1, 2, or 3 nt from a first strand of: AD-38170.1 (SEQ ID NO: 544), or a modified or unmodified variant thereof.

A RNAi agent comprising a first and a second strand, wherein the first strand comprises at least 15 contiguous nt differing by 0, 1, 2, or 3 nt from a first strand of: AD-38129.1 (SEQ ID NO: 545), or a modified or unmodified variant thereof.

A RNAi agent comprising a first and a second strand, wherein the first strand comprises at least 15 contiguous nt differing by 0, 1, 2, or 3 nt from a first strand of: AD-38135.1 (SEQ ID NO: 546), or a modified or unmodified variant thereof.

A RNAi agent comprising a first and a second strand, wherein the first strand comprises at least 15 contiguous nt differing by 0, 1, 2, or 3 nt from a first strand of: AD-38141.1 (SEQ ID NO: 547), or a modified or unmodified variant thereof.

A RNAi agent comprising a first and a second strand, wherein the first strand comprises at least 15 contiguous nt differing by 0, 1, 2, or 3 nt from a first strand of: AD-38147.1 (SEQ ID NO: 548), or a modified or unmodified variant thereof.

A RNAi agent comprising a first and a second strand, wherein the first strand comprises at least 15 contiguous nt differing by 0, 1, 2, or 3 nt from a first strand of: AD-38153.1 (SEQ ID NO: 549), or a modified or unmodified variant thereof.

A RNAi agent comprising a first and a second strand, wherein the first strand comprises at least 15 contiguous nt differing by 0, 1, 2, or 3 nt from a first strand of: AD-38159.1 (SEQ ID NO: 550), or a modified or unmodified variant thereof.

A RNAi agent comprising a first and a second strand, wherein the first strand comprises at least 15 contiguous nt differing by 0, 1, 2, or 3 nt from a first strand of: AD-38165.1 (SEQ ID NO: 551), or a modified or unmodified variant thereof.

A RNAi agent comprising a first and a second strand, wherein the first strand comprises at least 15 contiguous nt differing by 0, 1, 2, or 3 nt from a first strand of: AD-38171.1 (SEQ ID NO: 552), or a modified or unmodified variant thereof.

A RNAi agent comprising a first and a second strand, wherein the first strand comprises at least 15 contiguous nt differing by 0, 1, 2, or 3 nt from a first strand of: AD-38130.1 (SEQ ID NO: 553), or a modified or unmodified variant thereof.

A RNAi agent comprising a first and a second strand, wherein the first strand comprises at least 15 contiguous nt differing by 0, 1, 2, or 3 nt from a first strand of: AD-38136.1 (SEQ ID NO: 554), or a modified or unmodified variant thereof.

A RNAi agent comprising a first and a second strand, wherein the first strand comprises at least 15 contiguous nt differing by 0, 1, 2, or 3 nt from a first strand of: AD-38142.1 (SEQ ID NO: 555), or a modified or unmodified variant thereof.

A RNAi agent comprising a first and a second strand, wherein the first strand comprises at least 15 contiguous nt differing by 0, 1, 2, or 3 nt from a first strand of: AD-38148.1 (SEQ ID NO: 556), or a modified or unmodified variant thereof.

A RNAi agent comprising a first and a second strand, wherein the first strand comprises at least 15 contiguous nt differing by 0, 1, 2, or 3 nt from a first strand of: AD-38154.1 (SEQ ID NO: 557), or a modified or unmodified variant thereof.

A RNAi agent comprising a first and a second strand, wherein the first strand comprises at least 15 contiguous nt differing by 0, 1, 2, or 3 nt from a first strand of: AD-38160.1 (SEQ ID NO: 558), or a modified or unmodified variant thereof.

A RNAi agent comprising a first and a second strand, wherein the first strand comprises at least 15 contiguous nt differing by 0, 1, 2, or 3 nt from a first strand of: AD-38166.1 (SEQ ID NO: 559), or a modified or unmodified variant thereof.

A RNAi agent comprising a first and a second strand, wherein the first strand comprises at least 15 contiguous nt differing by 0, 1, 2, or 3 nt from a first strand of: AD-38172.1 (SEQ ID NO: 560), or a modified or unmodified variant thereof.

A RNAi agent comprising a first and a second strand, wherein the first strand comprises at least 15 contiguous nt differing by 0, 1, 2, or 3 nt from a first strand of: AD-38131.1 (SEQ ID NO: 561), or a modified or unmodified variant thereof.

A RNAi agent comprising a first and a second strand, wherein the first strand comprises at least 15 contiguous nt differing by 0, 1, 2, or 3 nt from a first strand of: AD-38137.1 (SEQ ID NO: 562), or a modified or unmodified variant thereof.

A RNAi agent comprising a first and a second strand, wherein the first strand comprises at least 15 contiguous nt differing by 0, 1, 2, or 3 nt from a first strand of: AD-38143.1 (SEQ ID NO: 563), or a modified or unmodified variant thereof.

A RNAi agent comprising a first and a second strand, wherein the first strand comprises at least 15 contiguous nt differing by 0, 1, 2, or 3 nt from a first strand of: AD-38149.1 (SEQ ID NO: 564), or a modified or unmodified variant thereof.

A RNAi agent comprising a first and a second strand, wherein the first strand comprises at least 15 contiguous nt differing by 0, 1, 2, or 3 nt from a first strand of: AD-38155.1 (SEQ ID NO: 565), or a modified or unmodified variant thereof.

A RNAi agent comprising a first and a second strand, wherein the first strand comprises at least 15 contiguous nt differing by 0, 1, 2, or 3 nt from a first strand of: AD-38161.1 (SEQ ID NO: 566), or a modified or unmodified variant thereof.

A RNAi agent comprising a first and a second strand, wherein the first strand comprises at least 15 contiguous nt differing by 0, 1, 2, or 3 nt from a first strand of: AD-38167.1 (SEQ ID NO: 567), or a modified or unmodified variant thereof.

A RNAi agent comprising a first and a second strand, wherein the first strand comprises at least 15 contiguous nt differing by 0, 1, 2, or 3 nt from a first strand of: AD-38173.1 (SEQ ID NO: 568), or a modified or unmodified variant thereof.

A RNAi agent comprising a first and a second strand, wherein the first strand comprises at least 15 contiguous nt differing by 0, 1, 2, or 3 nt from a first strand of: AD-38132.1 (SEQ ID NO: 569), or a modified or unmodified variant thereof.

A RNAi agent comprising a first and a second strand, wherein the first strand comprises at least 15 contiguous nt differing by 0, 1, 2, or 3 nt from a first strand of: AD-38138.1 (SEQ ID NO: 570), or a modified or unmodified variant thereof.

A RNAi agent comprising a first and a second strand, wherein the first strand comprises at least 15 contiguous nt differing by 0, 1, 2, or 3 nt from a first strand of: AD-38144.1 (SEQ ID NO: 571), or a modified or unmodified variant thereof.

A RNAi agent comprising a first and a second strand, wherein the first strand comprises at least 15 contiguous nt differing by 0, 1, 2, or 3 nt from a first strand of: AD-38150.1 (SEQ ID NO: 572), or a modified or unmodified variant thereof.

A RNAi agent comprising a first and a second strand, wherein the first strand comprises at least 15 contiguous nt differing by 0, 1, 2, or 3 nt from a first strand of: AD-38156.1 (SEQ ID NO: 573), or a modified or unmodified variant thereof.

A RNAi agent comprising a first and a second strand, wherein the first strand comprises at least 15 contiguous nt differing by 0, 1, 2, or 3 nt from a first strand of: AD-38162.1 (SEQ ID NO: 574), or a modified or unmodified variant thereof.

A RNAi agent comprising a first and a second strand, wherein the first strand comprises at least 15 contiguous nt differing by 0, 1, 2, or 3 nt from a first strand of: AD-38168.1 (SEQ ID NO: 575), or a modified or unmodified variant thereof.

A RNAi agent comprising a first and a second strand, wherein the first strand comprises at least 15 contiguous nt differing by 0, 1, 2, or 3 nt from a first strand of: AD-38174.1 (SEQ ID NO: 576), or a modified or unmodified variant thereof.

A RNAi agent comprising a first and a second strand, wherein the first strand comprises at least 15 contiguous nt differing by 0, 1, 2, or 3 nt from a first strand of: AD-39683.1 (SEQ ID NO: 577), or a modified or unmodified variant thereof.

A RNAi agent comprising a first and a second strand, wherein the first strand comprises at least 15 contiguous nt differing by 0, 1, 2, or 3 nt from a first strand of: AD-39689.1 (SEQ ID NO: 578), or a modified or unmodified variant thereof.

A RNAi agent comprising a first and a second strand, wherein the first strand comprises at least 15 contiguous nt differing by 0, 1, 2, or 3 nt from a first strand of: AD-39695.1 (SEQ ID NO: 579), or a modified or unmodified variant thereof.

A RNAi agent comprising a first and a second strand, wherein the first strand comprises at least 15 contiguous nt differing by 0, 1, 2, or 3 nt from a first strand of: AD-39701.1 (SEQ ID NO: 580), or a modified or unmodified variant thereof.

A RNAi agent comprising a first and a second strand, wherein the first strand comprises at least 15 contiguous nt differing by 0, 1, 2, or 3 nt from a first strand of: AD-39707.1 (SEQ ID NO: 581), or a modified or unmodified variant thereof.

A RNAi agent comprising a first and a second strand, wherein the first strand comprises at least 15 contiguous nt differing by 0, 1, 2, or 3 nt from a first strand of: AD-39713.1 (SEQ ID NO: 582), or a modified or unmodified variant thereof.

A RNAi agent comprising a first and a second strand, wherein the first strand comprises at least 15 contiguous nt differing by 0, 1, 2, or 3 nt from a first strand of: AD-39719.1 (SEQ ID NO: 583), or a modified or unmodified variant thereof.

A RNAi agent comprising a first and a second strand, wherein the first strand comprises at least 15 contiguous nt differing by 0, 1, 2, or 3 nt from a first strand of: AD-39725.1 (SEQ ID NO: 584), or a modified or unmodified variant thereof.

A RNAi agent comprising a first and a second strand, wherein the first strand comprises at least 15 contiguous nt differing by 0, 1, 2, or 3 nt from a first strand of: AD-39684.1 (SEQ ID NO: 585), or a modified or unmodified variant thereof.

A RNAi agent comprising a first and a second strand, wherein the first strand comprises at least 15 contiguous nt differing by 0, 1, 2, or 3 nt from a first strand of: AD-39690.1 (SEQ ID NO: 586), or a modified or unmodified variant thereof.

A RNAi agent comprising a first and a second strand, wherein the first strand comprises at least 15 contiguous nt differing by 0, 1, 2, or 3 nt from a first strand of: AD-39696.1 (SEQ ID NO: 587), or a modified or unmodified variant thereof.

A RNAi agent comprising a first and a second strand, wherein the first strand comprises at least 15 contiguous nt differing by 0, 1, 2, or 3 nt from a first strand of: AD-39702.1 (SEQ ID NO: 588), or a modified or unmodified variant thereof.

A RNAi agent comprising a first and a second strand, wherein the first strand comprises at least 15 contiguous nt differing by 0, 1, 2, or 3 nt from a first strand of: AD-39708.1 (SEQ ID NO: 589), or a modified or unmodified variant thereof.

A RNAi agent comprising a first and a second strand, wherein the first strand comprises at least 15 contiguous nt differing by 0, 1, 2, or 3 nt from a first strand of: AD-39714.1 (SEQ ID NO: 590), or a modified or unmodified variant thereof.

A RNAi agent comprising a first and a second strand, wherein the first strand comprises at least 15 contiguous nt differing by 0, 1, 2, or 3 nt from a first strand of: AD-39720.1 (SEQ ID NO: 591), or a modified or unmodified variant thereof.

A RNAi agent comprising a first and a second strand, wherein the first strand comprises at least 15 contiguous nt differing by 0, 1, 2, or 3 nt from a first strand of: AD-39726.1 (SEQ ID NO: 592), or a modified or unmodified variant thereof.

A RNAi agent comprising a first and a second strand, wherein the first strand comprises at least 15 contiguous nt differing by 0, 1, 2, or 3 nt from a first strand of: AD-39685.1 (SEQ ID NO: 593), or a modified or unmodified variant thereof.

A RNAi agent comprising a first and a second strand, wherein the first strand comprises at least 15 contiguous nt differing by 0, 1, 2, or 3 nt from a first strand of: AD-39691.1 (SEQ ID NO: 594), or a modified or unmodified variant thereof.

A RNAi agent comprising a first and a second strand, wherein the first strand comprises at least 15 contiguous nt differing by 0, 1, 2, or 3 nt from a first strand of: AD-39697.1 (SEQ ID NO: 595), or a modified or unmodified variant thereof.

A RNAi agent comprising a first and a second strand, wherein the first strand comprises at least 15 contiguous nt differing by 0, 1, 2, or 3 nt from a first strand of: AD-39703.1 (SEQ ID NO: 596), or a modified or unmodified variant thereof.

A RNAi agent comprising a first and a second strand, wherein the first strand comprises at least 15 contiguous nt differing by 0, 1, 2, or 3 nt from a first strand of: AD-39709.1 (SEQ ID NO: 597), or a modified or unmodified variant thereof.

A RNAi agent comprising a first and a second strand, wherein the first strand comprises at least 15 contiguous nt differing by 0, 1, 2, or 3 nt from a first strand of: AD-39715.1 (SEQ ID NO: 598), or a modified or unmodified variant thereof.

A RNAi agent comprising a first and a second strand, wherein the first strand comprises at least 15 contiguous nt differing by 0, 1, 2, or 3 nt from a first strand of: AD-39721.1 (SEQ ID NO: 599), or a modified or unmodified variant thereof.

A RNAi agent comprising a first and a second strand, wherein the first strand comprises at least 15 contiguous nt differing by 0, 1, 2, or 3 nt from a first strand of: AD-39727.1 (SEQ ID NO: 600), or a modified or unmodified variant thereof.

A RNAi agent comprising a first and a second strand, wherein the first strand comprises at least 15 contiguous nt differing by 0, 1, 2, or 3 nt from a first strand of: AD-39686.1 (SEQ ID NO: 601), or a modified or unmodified variant thereof.

A RNAi agent comprising a first and a second strand, wherein the first strand comprises at least 15 contiguous nt differing by 0, 1, 2, or 3 nt from a first strand of: AD-39692.1 (SEQ ID NO: 602), or a modified or unmodified variant thereof.

A RNAi agent comprising a first and a second strand, wherein the first strand comprises at least 15 contiguous nt differing by 0, 1, 2, or 3 nt from a first strand of: AD-39698.1 (SEQ ID NO: 603), or a modified or unmodified variant thereof.

A RNAi agent comprising a first and a second strand, wherein the first strand comprises at least 15 contiguous nt differing by 0, 1, 2, or 3 nt from a first strand of: AD-39704.1 (SEQ ID NO: 604), or a modified or unmodified variant thereof.

A RNAi agent comprising a first and a second strand, wherein the first strand comprises at least 15 contiguous nt differing by 0, 1, 2, or 3 nt from a first strand of: AD-39710.1 (SEQ ID NO: 605), or a modified or unmodified variant thereof.

A RNAi agent comprising a first and a second strand, wherein the first strand comprises at least 15 contiguous nt differing by 0, 1, 2, or 3 nt from a first strand of: AD-39716.1 (SEQ ID NO: 606), or a modified or unmodified variant thereof.

A RNAi agent comprising a first and a second strand, wherein the first strand comprises at least 15 contiguous nt differing by 0, 1, 2, or 3 nt from a first strand of: AD-39722.1 (SEQ ID NO: 607), or a modified or unmodified variant thereof.

A RNAi agent comprising a first and a second strand, wherein the first strand comprises at least 15 contiguous nt differing by 0, 1, 2, or 3 nt from a first strand of: AD-39728.1 (SEQ ID NO: 608), or a modified or unmodified variant thereof.

A RNAi agent comprising a first and a second strand, wherein the first strand comprises at least 15 contiguous nt differing by 0, 1, 2, or 3 nt from a first strand of: AD-39687.1 (SEQ ID NO: 609), or a modified or unmodified variant thereof.

A RNAi agent comprising a first and a second strand, wherein the first strand comprises at least 15 contiguous nt differing by 0, 1, 2, or 3 nt from a first strand of: AD-39693.1 (SEQ ID NO: 610), or a modified or unmodified variant thereof.

A RNAi agent comprising a first and a second strand, wherein the first strand comprises at least 15 contiguous nt differing by 0, 1, 2, or 3 nt from a first strand of: AD-39699.1 (SEQ ID NO: 611), or a modified or unmodified variant thereof.

A RNAi agent comprising a first and a second strand, wherein the first strand comprises at least 15 contiguous nt differing by 0, 1, 2, or 3 nt from a first strand of: AD-39705.1 (SEQ ID NO: 612), or a modified or unmodified variant thereof.

A RNAi agent comprising a first and a second strand, wherein the first strand comprises at least 15 contiguous nt differing by 0, 1, 2, or 3 nt from a first strand of: AD-39711.1 (SEQ ID NO: 613), or a modified or unmodified variant thereof.

A RNAi agent comprising a first and a second strand, wherein the first strand comprises at least 15 contiguous nt differing by 0, 1, 2, or 3 nt from a first strand of: AD-39717.1 (SEQ ID NO: 614), or a modified or unmodified variant thereof.

A RNAi agent comprising a first and a second strand, wherein the first strand comprises at least 15 contiguous nt differing by 0, 1, 2, or 3 nt from a first strand of: AD-39723.1 (SEQ ID NO: 615), or a modified or unmodified variant thereof.

A RNAi agent comprising a first and a second strand, wherein the first strand comprises at least 15 contiguous nt differing by 0, 1, 2, or 3 nt from a first strand of: AD-39729.1 (SEQ ID NO: 616), or a modified or unmodified variant thereof.

A RNAi agent comprising a first and a second strand, wherein the first strand comprises at least 15 contiguous nt differing by 0, 1, 2, or 3 nt from a first strand of: AD-39688.1 (SEQ ID NO: 617), or a modified or unmodified variant thereof.

A RNAi agent comprising a first and a second strand, wherein the first strand comprises at least 15 contiguous nt differing by 0, 1, 2, or 3 nt from a first strand of: AD-39694.1 (SEQ ID NO: 618), or a modified or unmodified variant thereof.

A RNAi agent comprising a first and a second strand, wherein the first strand comprises at least 15 contiguous nt differing by 0, 1, 2, or 3 nt from a first strand of: AD-39700.1 (SEQ ID NO: 619), or a modified or unmodified variant thereof.

A RNAi agent comprising a first and a second strand, wherein the first strand comprises at least 15 contiguous nt differing by 0, 1, 2, or 3 nt from a first strand of: AD-39706.1 (SEQ ID NO: 620), or a modified or unmodified variant thereof.

A RNAi agent comprising a first and a second strand, wherein the first strand comprises at least 15 contiguous nt differing by 0, 1, 2, or 3 nt from a first strand of: AD-39712.1 (SEQ ID NO: 621), or a modified or unmodified variant thereof.

A RNAi agent comprising a first and a second strand, wherein the first strand comprises at least 15 contiguous nt differing by 0, 1, 2, or 3 nt from a first strand of: AD-39718.1 (SEQ ID NO: 622), or a modified or unmodified variant thereof.

A RNAi agent comprising a first and a second strand, wherein the first strand comprises at least 15 contiguous nt differing by 0, 1, 2, or 3 nt from a first strand of: AD-39724.1 (SEQ ID NO: 623), or a modified or unmodified variant thereof.

A RNAi agent comprising a first and a second strand, wherein the first strand comprises at least 15 contiguous nt differing by 0, 1, 2, or 3 nt from a first strand of: AD-39730.1 (SEQ ID NO: 624), or a modified or unmodified variant thereof.

A RNAi agent comprising a first and a second strand, wherein the first strand comprises at least 15 contiguous nt differing by 0, 1, 2, or 3 nt from a first strand of: AD-39736.1 (SEQ ID NO: 625), or a modified or unmodified variant thereof.

A RNAi agent comprising a first and a second strand, wherein the first strand comprises at least 15 contiguous nt differing by 0, 1, 2, or 3 nt from a first strand of: AD-39742.1 (SEQ ID NO: 626), or a modified or unmodified variant thereof.

A RNAi agent comprising a first and a second strand, wherein the first strand comprises at least 15 contiguous nt differing by 0, 1, 2, or 3 nt from a first strand of: AD-39748.1 (SEQ ID NO: 627), or a modified or unmodified variant thereof.

A RNAi agent comprising a first and a second strand, wherein the first strand comprises at least 15 contiguous nt differing by 0, 1, 2, or 3 nt from a first strand of: AD-39754.1 (SEQ ID NO: 628), or a modified or unmodified variant thereof.

A RNAi agent comprising a first and a second strand, wherein the first strand comprises at least 15 contiguous nt differing by 0, 1, 2, or 3 nt from a first strand of: AD-39760.1 (SEQ ID NO: 629), or a modified or unmodified variant thereof.

A RNAi agent comprising a first and a second strand, wherein the first strand comprises at least 15 contiguous nt differing by 0, 1, 2, or 3 nt from a first strand of: AD-39766.1 (SEQ ID NO: 630), or a modified or unmodified variant thereof.

A RNAi agent comprising a first and a second strand, wherein the first strand comprises at least 15 contiguous nt differing by 0, 1, 2, or 3 nt from a first strand of: AD-39772.1 (SEQ ID NO: 631), or a modified or unmodified variant thereof.

A RNAi agent comprising a first and a second strand, wherein the first strand comprises at least 15 contiguous nt differing by 0, 1, 2, or 3 nt from a first strand of: AD-39731.1 (SEQ ID NO: 632), or a modified or unmodified variant thereof.

A RNAi agent comprising a first and a second strand, wherein the first strand comprises at least 15 contiguous nt differing by 0, 1, 2, or 3 nt from a first strand of: AD-39737.1 (SEQ ID NO: 633), or a modified or unmodified variant thereof.

A RNAi agent comprising a first and a second strand, wherein the first strand comprises at least 15 contiguous nt differing by 0, 1, 2, or 3 nt from a first strand of: AD-39743.1 (SEQ ID NO: 634), or a modified or unmodified variant thereof.

A RNAi agent comprising a first and a second strand, wherein the first strand comprises at least 15 contiguous nt differing by 0, 1, 2, or 3 nt from a first strand of: AD-39749.1 (SEQ ID NO: 635), or a modified or unmodified variant thereof.

A RNAi agent comprising a first and a second strand, wherein the first strand comprises at least 15 contiguous nt differing by 0, 1, 2, or 3 nt from a first strand of: AD-39755.1 (SEQ ID NO: 636), or a modified or unmodified variant thereof.

A RNAi agent comprising a first and a second strand, wherein the first strand comprises at least 15 contiguous nt differing by 0, 1, 2, or 3 nt from a first strand of: AD-39761.1 (SEQ ID NO: 637), or a modified or unmodified variant thereof.

A RNAi agent comprising a first and a second strand, wherein the first strand comprises at least 15 contiguous nt differing by 0, 1, 2, or 3 nt from a first strand of: AD-39767.1 (SEQ ID NO: 638), or a modified or unmodified variant thereof.

A RNAi agent comprising a first and a second strand, wherein the first strand comprises at least 15 contiguous nt differing by 0, 1, 2, or 3 nt from a first strand of: AD-39773.1 (SEQ ID NO: 639), or a modified or unmodified variant thereof.

A RNAi agent comprising a first and a second strand, wherein the first strand comprises at least 15 contiguous nt differing by 0, 1, 2, or 3 nt from a first strand of: AD-39732.1 (SEQ ID NO: 640), or a modified or unmodified variant thereof.

A RNAi agent comprising a first and a second strand, wherein the first strand comprises at least 15 contiguous nt differing by 0, 1, 2, or 3 nt from a first strand of: AD-39738.1 (SEQ ID NO: 641), or a modified or unmodified variant thereof.

A RNAi agent comprising a first and a second strand, wherein the first strand comprises at least 15 contiguous nt differing by 0, 1, 2, or 3 nt from a first strand of: AD-39744.1 (SEQ ID NO: 642), or a modified or unmodified variant thereof.

A RNAi agent comprising a first and a second strand, wherein the first strand comprises at least 15 contiguous nt differing by 0, 1, 2, or 3 nt from a first strand of: AD-39750.1 (SEQ ID NO: 643), or a modified or unmodified variant thereof.

A RNAi agent comprising a first and a second strand, wherein the first strand comprises at least 15 contiguous nt differing by 0, 1, 2, or 3 nt from a first strand of: AD-39756.1 (SEQ ID NO: 644), or a modified or unmodified variant thereof.

A RNAi agent comprising a first and a second strand, wherein the first strand comprises at least 15 contiguous nt differing by 0, 1, 2, or 3 nt from a first strand of: AD-39762.1 (SEQ ID NO: 645), or a modified or unmodified variant thereof.

A RNAi agent comprising a first and a second strand, wherein the first strand comprises at least 15 contiguous nt differing by 0, 1, 2, or 3 nt from a first strand of: AD-39768.1 (SEQ ID NO: 646), or a modified or unmodified variant thereof.

A RNAi agent comprising a first and a second strand, wherein the first strand comprises at least 15 contiguous nt differing by 0, 1, 2, or 3 nt from a first strand of: AD-39774.1 (SEQ ID NO: 647), or a modified or unmodified variant thereof.

A RNAi agent comprising a first and a second strand, wherein the first strand comprises at least 15 contiguous nt differing by 0, 1, 2, or 3 nt from a first strand of: AD-39733.1 (SEQ ID NO: 648), or a modified or unmodified variant thereof.

A RNAi agent comprising a first and a second strand, wherein the first strand comprises at least 15 contiguous nt differing by 0, 1, 2, or 3 nt from a first strand of: AD-39739.1 (SEQ ID NO: 649), or a modified or unmodified variant thereof.

A RNAi agent comprising a first and a second strand, wherein the first strand comprises at least 15 contiguous nt differing by 0, 1, 2, or 3 nt from a first strand of: AD-39745.1 (SEQ ID NO: 650), or a modified or unmodified variant thereof.

A RNAi agent comprising a first and a second strand, wherein the first strand comprises at least 15 contiguous nt differing by 0, 1, 2, or 3 nt from a first strand of: AD-39751.1 (SEQ ID NO: 651), or a modified or unmodified variant thereof.

A RNAi agent comprising a first and a second strand, wherein the first strand comprises at least 15 contiguous nt differing by 0, 1, 2, or 3 nt from a first strand of: AD-39757.1 (SEQ ID NO: 652), or a modified or unmodified variant thereof.

A RNAi agent comprising a first and a second strand, wherein the first strand comprises at least 15 contiguous nt differing by 0, 1, 2, or 3 nt from a first strand of: AD-39763.1 (SEQ ID NO: 653), or a modified or unmodified variant thereof.

A RNAi agent comprising a first and a second strand, wherein the first strand comprises at least 15 contiguous nt differing by 0, 1, 2, or 3 nt from a first strand of: AD-39769.1 (SEQ ID NO: 654), or a modified or unmodified variant thereof.

A RNAi agent comprising a first and a second strand, wherein the first strand comprises at least 15 contiguous nt differing by 0, 1, 2, or 3 nt from a first strand of: AD-39775.1 (SEQ ID NO: 655), or a modified or unmodified variant thereof.

A RNAi agent comprising a first and a second strand, wherein the first strand comprises at least 15 contiguous nt differing by 0, 1, 2, or 3 nt from a first strand of: AD-39734.1 (SEQ ID NO: 656), or a modified or unmodified variant thereof.

A RNAi agent comprising a first and a second strand, wherein the first strand comprises at least 15 contiguous nt differing by 0, 1, 2, or 3 nt from a first strand of: AD-39740.1 (SEQ ID NO: 657), or a modified or unmodified variant thereof.

A RNAi agent comprising a first and a second strand, wherein the first strand comprises at least 15 contiguous nt differing by 0, 1, 2, or 3 nt from a first strand of: AD-39746.1 (SEQ ID NO: 658), or a modified or unmodified variant thereof.

A RNAi agent comprising a first and a second strand, wherein the first strand comprises at least 15 contiguous nt differing by 0, 1, 2, or 3 nt from a first strand of: AD-39752.1 (SEQ ID NO: 659), or a modified or unmodified variant thereof.

A RNAi agent comprising a first and a second strand, wherein the first strand comprises at least 15 contiguous nt differing by 0, 1, 2, or 3 nt from a first strand of: AD-39758.1 (SEQ ID NO: 660), or a modified or unmodified variant thereof.

A RNAi agent comprising a first and a second strand, wherein the first strand comprises at least 15 contiguous nt differing by 0, 1, 2, or 3 nt from a first strand of: AD-39764.1 (SEQ ID NO: 661), or a modified or unmodified variant thereof.

A RNAi agent comprising a first and a second strand, wherein the first strand comprises at least 15 contiguous nt differing by 0, 1, 2, or 3 nt from a first strand of: AD-39770.1 (SEQ ID NO: 662), or a modified or unmodified variant thereof.

A RNAi agent comprising a first and a second strand, wherein the first strand comprises at least 15 contiguous nt differing by 0, 1, 2, or 3 nt from a first strand of: AD-39776.1 (SEQ ID NO: 663), or a modified or unmodified variant thereof.

A RNAi agent comprising a first and a second strand, wherein the first strand comprises at least 15 contiguous nt differing by 0, 1, 2, or 3 nt from a first strand of: AD-39735.1 (SEQ ID NO: 664), or a modified or unmodified variant thereof.

A RNAi agent comprising a first and a second strand, wherein the first strand comprises at least 15 contiguous nt differing by 0, 1, 2, or 3 nt from a first strand of: AD-39741.1 (SEQ ID NO: 665), or a modified or unmodified variant thereof.

A RNAi agent comprising a first and a second strand, wherein the first strand comprises at least 15 contiguous nt differing by 0, 1, 2, or 3 nt from a first strand of: AD-39747.1 (SEQ ID NO: 666), or a modified or unmodified variant thereof.

A RNAi agent comprising a first and a second strand, wherein the first strand comprises at least 15 contiguous nt differing by 0, 1, 2, or 3 nt from a first strand of: AD-39753.1 (SEQ ID NO: 667), or a modified or unmodified variant thereof.

A RNAi agent comprising a first and a second strand, wherein the first strand comprises at least 15 contiguous nt differing by 0, 1, 2, or 3 nt from a first strand of: AD-39759.1 (SEQ ID NO: 668), or a modified or unmodified variant thereof.

A RNAi agent comprising a first and a second strand, wherein the first strand comprises at least 15 contiguous nt differing by 0, 1, 2, or 3 nt from a first strand of: AD-39765.1 (SEQ ID NO: 669), or a modified or unmodified variant thereof.

A RNAi agent comprising a first and a second strand, wherein the first strand comprises at least 15 contiguous nt differing by 0, 1, 2, or 3 nt from a first strand of: AD-39771.1 (SEQ ID NO: 670), or a modified or unmodified variant thereof.

A RNAi agent comprising a first and a second strand, wherein the first strand comprises at least 15 contiguous nt differing by 0, 1, 2, or 3 nt from a first strand of: AD-39778.1 (SEQ ID NO: 671), or a modified or unmodified variant thereof.

A RNAi agent comprising a first and a second strand, wherein the first strand comprises at least 15 contiguous nt differing by 0, 1, 2, or 3 nt from a first strand of: AD-39784.1 (SEQ ID NO: 672), or a modified or unmodified variant thereof.

A RNAi agent comprising a first and a second strand, wherein the first strand comprises at least 15 contiguous nt differing by 0, 1, 2, or 3 nt from a first strand of: AD-39790.1 (SEQ ID NO: 673), or a modified or unmodified variant thereof.

A RNAi agent comprising a first and a second strand, wherein the first strand comprises at least 15 contiguous nt differing by 0, 1, 2, or 3 nt from a first strand of: AD-39796.1 (SEQ ID NO: 674), or a modified or unmodified variant thereof.

A RNAi agent comprising a first and a second strand, wherein the first strand comprises at least 15 contiguous nt differing by 0, 1, 2, or 3 nt from a first strand of: AD-39802.1 (SEQ ID NO: 675), or a modified or unmodified variant thereof.

A RNAi agent comprising a first and a second strand, wherein the first strand comprises at least 15 contiguous nt differing by 0, 1, 2, or 3 nt from a first strand of: AD-39808.1 (SEQ ID NO: 676), or a modified or unmodified variant thereof.

A RNAi agent comprising a first and a second strand, wherein the first strand comprises at least 15 contiguous nt differing by 0, 1, 2, or 3 nt from a first strand of: AD-39814.1 (SEQ ID NO: 677), or a modified or unmodified variant thereof.

A RNAi agent comprising a first and a second strand, wherein the first strand comprises at least 15 contiguous nt differing by 0, 1, 2, or 3 nt from a first strand of: AD-39820.1 (SEQ ID NO: 678), or a modified or unmodified variant thereof.

A RNAi agent comprising a first and a second strand, wherein the first strand comprises at least 15 contiguous nt differing by 0, 1, 2, or 3 nt from a first strand of: AD-39779.1 (SEQ ID NO: 679), or a modified or unmodified variant thereof.

A RNAi agent comprising a first and a second strand, wherein the first strand comprises at least 15 contiguous nt differing by 0, 1, 2, or 3 nt from a first strand of: AD-39785.1 (SEQ ID NO: 680), or a modified or unmodified variant thereof.

A RNAi agent comprising a first and a second strand, wherein the first strand comprises at least 15 contiguous nt differing by 0, 1, 2, or 3 nt from a first strand of: AD-39791.1 (SEQ ID NO: 681), or a modified or unmodified variant thereof.

A RNAi agent comprising a first and a second strand, wherein the first strand comprises at least 15 contiguous nt differing by 0, 1, 2, or 3 nt from a first strand of: AD-39797.1 (SEQ ID NO: 682), or a modified or unmodified variant thereof.

A RNAi agent comprising a first and a second strand, wherein the first strand comprises at least 15 contiguous nt differing by 0, 1, 2, or 3 nt from a first strand of: AD-39803.1 (SEQ ID NO: 683), or a modified or unmodified variant thereof.

A RNAi agent comprising a first and a second strand, wherein the first strand comprises at least 15 contiguous nt differing by 0, 1, 2, or 3 nt from a first strand of: AD-39809.1 (SEQ ID NO: 684), or a modified or unmodified variant thereof.

A RNAi agent comprising a first and a second strand, wherein the first strand comprises at least 15 contiguous nt differing by 0, 1, 2, or 3 nt from a first strand of: AD-39815.1 (SEQ ID NO: 685), or a modified or unmodified variant thereof.

A RNAi agent comprising a first and a second strand, wherein the first strand comprises at least 15 contiguous nt differing by 0, 1, 2, or 3 nt from a first strand of: AD-39821.1 (SEQ ID NO: 686), or a modified or unmodified variant thereof.

A RNAi agent comprising a first and a second strand, wherein the first strand comprises at least 15 contiguous nt differing by 0, 1, 2, or 3 nt from a first strand of: AD-39780.1 (SEQ ID NO: 687), or a modified or unmodified variant thereof.

A RNAi agent comprising a first and a second strand, wherein the first strand comprises at least 15 contiguous nt differing by 0, 1, 2, or 3 nt from a first strand of: AD-39786.1 (SEQ ID NO: 688), or a modified or unmodified variant thereof.

A RNAi agent comprising a first and a second strand, wherein the first strand comprises at least 15 contiguous nt differing by 0, 1, 2, or 3 nt from a first strand of: AD-39792.1 (SEQ ID NO: 689), or a modified or unmodified variant thereof.

A RNAi agent comprising a first and a second strand, wherein the first strand comprises at least 15 contiguous nt differing by 0, 1, 2, or 3 nt from a first strand of: AD-39798.1 (SEQ ID NO: 690), or a modified or unmodified variant thereof.

A RNAi agent comprising a first and a second strand, wherein the first strand comprises at least 15 contiguous nt differing by 0, 1, 2, or 3 nt from a first strand of: AD-39804.1 (SEQ ID NO: 691), or a modified or unmodified variant thereof.

A RNAi agent comprising a first and a second strand, wherein the first strand comprises at least 15 contiguous nt differing by 0, 1, 2, or 3 nt from a first strand of: AD-39810.1 (SEQ ID NO: 692), or a modified or unmodified variant thereof.

A RNAi agent comprising a first and a second strand, wherein the first strand comprises at least 15 contiguous nt differing by 0, 1, 2, or 3 nt from a first strand of: AD-39816.1 (SEQ ID NO: 693), or a modified or unmodified variant thereof.

A RNAi agent comprising a first and a second strand, wherein the first strand comprises at least 15 contiguous nt differing by 0, 1, 2, or 3 nt from a first strand of: AD-39822.1 (SEQ ID NO: 694), or a modified or unmodified variant thereof.

A RNAi agent comprising a first and a second strand, wherein the first strand comprises at least 15 contiguous nt differing by 0, 1, 2, or 3 nt from a first strand of: AD-39781.1 (SEQ ID NO: 695), or a modified or unmodified variant thereof.

A RNAi agent comprising a first and a second strand, wherein the first strand comprises at least 15 contiguous nt differing by 0, 1, 2, or 3 nt from a first strand of: AD-39787.1 (SEQ ID NO: 696), or a modified or unmodified variant thereof.

A RNAi agent comprising a first and a second strand, wherein the first strand comprises at least 15 contiguous nt differing by 0, 1, 2, or 3 nt from a first strand of: AD-39793.1 (SEQ ID NO: 697), or a modified or unmodified variant thereof.

A RNAi agent comprising a first and a second strand, wherein the first strand comprises at least 15 contiguous nt differing by 0, 1, 2, or 3 nt from a first strand of: AD-39799.1 (SEQ ID NO: 698), or a modified or unmodified variant thereof.

A RNAi agent comprising a first and a second strand, wherein the first strand comprises at least 15 contiguous nt differing by 0, 1, 2, or 3 nt from a first strand of: AD-39805.1 (SEQ ID NO: 699), or a modified or unmodified variant thereof.

A RNAi agent comprising a first and a second strand, wherein the first strand comprises at least 15 contiguous nt differing by 0, 1, 2, or 3 nt from a first strand of: AD-39811.1 (SEQ ID NO: 700), or a modified or unmodified variant thereof.

A RNAi agent comprising a first and a second strand, wherein the first strand comprises at least 15 contiguous nt differing by 0, 1, 2, or 3 nt from a first strand of: AD-39817.1 (SEQ ID NO: 701), or a modified or unmodified variant thereof.

A RNAi agent comprising a first and a second strand, wherein the first strand comprises at least 15 contiguous nt differing by 0, 1, 2, or 3 nt from a first strand of: AD-39823.1 (SEQ ID NO: 702), or a modified or unmodified variant thereof.

A RNAi agent comprising a first and a second strand, wherein the first strand comprises at least 15 contiguous nt differing by 0, 1, 2, or 3 nt from a first strand of: AD-39782.1 (SEQ ID NO: 703), or a modified or unmodified variant thereof.

A RNAi agent comprising a first and a second strand, wherein the first strand comprises at least 15 contiguous nt differing by 0, 1, 2, or 3 nt from a first strand of: AD-39788.1 (SEQ ID NO: 704), or a modified or unmodified variant thereof.

A RNAi agent comprising a first and a second strand, wherein the first strand comprises at least 15 contiguous nt differing by 0, 1, 2, or 3 nt from a first strand of: AD-39794.1 (SEQ ID NO: 705), or a modified or unmodified variant thereof.

A RNAi agent comprising a first and a second strand, wherein the first strand comprises at least 15 contiguous nt differing by 0, 1, 2, or 3 nt from a first strand of: AD-39800.1 (SEQ ID NO: 706), or a modified or unmodified variant thereof.

A RNAi agent comprising a first and a second strand, wherein the first strand comprises at least 15 contiguous nt differing by 0, 1, 2, or 3 nt from a first strand of: AD-39806.1 (SEQ ID NO: 707), or a modified or unmodified variant thereof.

A RNAi agent comprising a first and a second strand, wherein the first strand comprises at least 15 contiguous nt differing by 0, 1, 2, or 3 nt from a first strand of: AD-39812.1 (SEQ ID NO: 708), or a modified or unmodified variant thereof.

A RNAi agent comprising a first and a second strand, wherein the first strand comprises at least 15 contiguous nt differing by 0, 1, 2, or 3 nt from a first strand of: AD-39818.1 (SEQ ID NO: 709), or a modified or unmodified variant thereof.

A RNAi agent comprising a first and a second strand, wherein the first strand comprises at least 15 contiguous nt differing by 0, 1, 2, or 3 nt from a first strand of: AD-39824.1 (SEQ ID NO: 710), or a modified or unmodified variant thereof.

A RNAi agent comprising a first and a second strand, wherein the first strand comprises at least 15 contiguous nt differing by 0, 1, 2, or 3 nt from a first strand of: AD-39783.1 (SEQ ID NO: 711), or a modified or unmodified variant thereof.

A RNAi agent comprising a first and a second strand, wherein the first strand comprises at least 15 contiguous nt differing by 0, 1, 2, or 3 nt from a first strand of: AD-39789.1 (SEQ ID NO: 712), or a modified or unmodified variant thereof.

A RNAi agent comprising a first and a second strand, wherein the first strand comprises at least 15 contiguous nt differing by 0, 1, 2, or 3 nt from a first strand of: AD-39795.1 (SEQ ID NO: 713), or a modified or unmodified variant thereof.

A RNAi agent comprising a first and a second strand, wherein the first strand comprises at least 15 contiguous nt differing by 0, 1, 2, or 3 nt from a first strand of: AD-39801.1 (SEQ ID NO: 714), or a modified or unmodified variant thereof.

A RNAi agent comprising a first and a second strand, wherein the first strand comprises at least 15 contiguous nt differing by 0, 1, 2, or 3 nt from a first strand of: AD-39807.1 (SEQ ID NO: 715), or a modified or unmodified variant thereof.

A RNAi agent comprising a first and a second strand, wherein the first strand comprises at least 15 contiguous nt differing by 0, 1, 2, or 3 nt from a first strand of: AD-39813.1 (SEQ ID NO: 716), or a modified or unmodified variant thereof.

A RNAi agent comprising a first and a second strand, wherein the first strand comprises at least 15 contiguous nt differing by 0, 1, 2, or 3 nt from a first strand of: AD-39819.1 (SEQ ID NO: 717), or a modified or unmodified variant thereof.

A RNAi agent comprising a first and a second strand, wherein the first strand comprises at least 15 contiguous nt differing by 0, 1, 2, or 3 nt from a first strand of: AD-39825.1 (SEQ ID NO: 718), or a modified or unmodified variant thereof.

A RNAi agent comprising a first and a second strand, wherein the first strand comprises at least 15 contiguous nt differing by 0, 1, 2, or 3 nt from a first strand of: AD-39831.1 (SEQ ID NO: 719), or a modified or unmodified variant thereof.

A RNAi agent comprising a first and a second strand, wherein the first strand comprises at least 15 contiguous nt differing by 0, 1, 2, or 3 nt from a first strand of: AD-39837.1 (SEQ ID NO: 720), or a modified or unmodified variant thereof.

A RNAi agent comprising a first and a second strand, wherein the first strand comprises at least 15 contiguous nt differing by 0, 1, 2, or 3 nt from a first strand of: AD-39843.1 (SEQ ID NO: 721), or a modified or unmodified variant thereof.
A RNAi agent comprising a first and a second strand, wherein the first strand comprises at least 15 contiguous nt differing by 0, 1, 2, or 3 nt from a first strand of: AD-39849.1 (SEQ ID NO: 722), or a modified or unmodified variant thereof.
A RNAi agent comprising a first and a second strand, wherein the first strand comprises at least 15 contiguous nt differing by 0, 1, 2, or 3 nt from a first strand of: AD-39855.1 (SEQ ID NO: 723), or a modified or unmodified variant thereof.
A RNAi agent comprising a first and a second strand, wherein the first strand comprises at least 15 contiguous nt differing by 0, 1, 2, or 3 nt from a first strand of: AD-39861.1 (SEQ ID NO: 724), or a modified or unmodified variant thereof.
A RNAi agent comprising a first and a second strand, wherein the first strand comprises at least 15 contiguous nt differing by 0, 1, 2, or 3 nt from a first strand of: AD-39867.1 (SEQ ID NO: 725), or a modified or unmodified variant thereof.
A RNAi agent comprising a first and a second strand, wherein the first strand comprises at least 15 contiguous nt differing by 0, 1, 2, or 3 nt from a first strand of: AD-39826.1 (SEQ ID NO: 726), or a modified or unmodified variant thereof.
A RNAi agent comprising a first and a second strand, wherein the first strand comprises at least 15 contiguous nt differing by 0, 1, 2, or 3 nt from a first strand of: AD-39832.1 (SEQ ID NO: 727), or a modified or unmodified variant thereof.
A RNAi agent comprising a first and a second strand, wherein the first strand comprises at least 15 contiguous nt differing by 0, 1, 2, or 3 nt from a first strand of: AD-39838.1 (SEQ ID NO: 728), or a modified or unmodified variant thereof.
A RNAi agent comprising a first and a second strand, wherein the first strand comprises at least 15 contiguous nt differing by 0, 1, 2, or 3 nt from a first strand of: AD-39844.1 (SEQ ID NO: 729), or a modified or unmodified variant thereof.
A RNAi agent comprising a first and a second strand, wherein the first strand comprises at least 15 contiguous nt differing by 0, 1, 2, or 3 nt from a first strand of: AD-39850.1 (SEQ ID NO: 730), or a modified or unmodified variant thereof.
A RNAi agent comprising a first and a second strand, wherein the first strand comprises at least 15 contiguous nt differing by 0, 1, 2, or 3 nt from a first strand of: AD-39856.1 (SEQ ID NO: 731), or a modified or unmodified variant thereof.
A RNAi agent comprising a first and a second strand, wherein the first strand comprises at least 15 contiguous nt differing by 0, 1, 2, or 3 nt from a first strand of: AD-39862.1 (SEQ ID NO: 732), or a modified or unmodified variant thereof.
A RNAi agent comprising a first and a second strand, wherein the first strand comprises at least 15 contiguous nt differing by 0, 1, 2, or 3 nt from a first strand of: AD-39868.1 (SEQ ID NO: 733), or a modified or unmodified variant thereof.
A RNAi agent comprising a first and a second strand, wherein the first strand comprises at least 15 contiguous nt differing by 0, 1, 2, or 3 nt from a first strand of: AD-39827.1 (SEQ ID NO: 734), or a modified or unmodified variant thereof.
A RNAi agent comprising a first and a second strand, wherein the first strand comprises at least 15 contiguous nt differing by 0, 1, 2, or 3 nt from a first strand of: AD-39833.1 (SEQ ID NO: 735), or a modified or unmodified variant thereof.
A RNAi agent comprising a first and a second strand, wherein the first strand comprises at least 15 contiguous nt differing by 0, 1, 2, or 3 nt from a first strand of: AD-39839.1 (SEQ ID NO: 736), or a modified or unmodified variant thereof.
A RNAi agent comprising a first and a second strand, wherein the first strand comprises at least 15 contiguous nt differing by 0, 1, 2, or 3 nt from a first strand of: AD-39845.1 (SEQ ID NO: 737), or a modified or unmodified variant thereof.
A RNAi agent comprising a first and a second strand, wherein the first strand comprises at least 15 contiguous nt differing by 0, 1, 2, or 3 nt from a first strand of: AD-39851.1 (SEQ ID NO: 738), or a modified or unmodified variant thereof.
A RNAi agent comprising a first and a second strand, wherein the first strand comprises at least 15 contiguous nt differing by 0, 1, 2, or 3 nt from a first strand of: AD-39857.1 (SEQ ID NO: 739), or a modified or unmodified variant thereof.
A RNAi agent comprising a first and a second strand, wherein the first strand comprises at least 15 contiguous nt differing by 0, 1, 2, or 3 nt from a first strand of: AD-39863.1 (SEQ ID NO: 740), or a modified or unmodified variant thereof.
A RNAi agent comprising a first and a second strand, wherein the first strand comprises at least 15 contiguous nt differing by 0, 1, 2, or 3 nt from a first strand of: AD-39869.1 (SEQ ID NO: 741), or a modified or unmodified variant thereof.
A RNAi agent comprising a first and a second strand, wherein the first strand comprises at least 15 contiguous nt differing by 0, 1, 2, or 3 nt from a first strand of: AD-39828.1 (SEQ ID NO: 742), or a modified or unmodified variant thereof.
A RNAi agent comprising a first and a second strand, wherein the first strand comprises at least 15 contiguous nt differing by 0, 1, 2, or 3 nt from a first strand of: AD-39834.1 (SEQ ID NO: 743), or a modified or unmodified variant thereof.
A RNAi agent comprising a first and a second strand, wherein the first strand comprises at least 15 contiguous nt differing by 0, 1, 2, or 3 nt from a first strand of: AD-39840.1 (SEQ ID NO: 744), or a modified or unmodified variant thereof.
A RNAi agent comprising a first and a second strand, wherein the first strand comprises at least 15 contiguous nt differing by 0, 1, 2, or 3 nt from a first strand of: AD-39846.1 (SEQ ID NO: 745), or a modified or unmodified variant thereof.
A RNAi agent comprising a first and a second strand, wherein the first strand comprises at least 15 contiguous nt differing by 0, 1, 2, or 3 nt from a first strand of: AD-39852.1 (SEQ ID NO: 746), or a modified or unmodified variant thereof.
A RNAi agent comprising a first and a second strand, wherein the first strand comprises at least 15 contiguous nt differing by 0, 1, 2, or 3 nt from a first strand of: AD-39858.1 (SEQ ID NO: 747), or a modified or unmodified variant thereof.
A RNAi agent comprising a first and a second strand, wherein the first strand comprises at least 15 contiguous nt differing by 0, 1, 2, or 3 nt from a first strand of: AD-39864.1 (SEQ ID NO: 748), or a modified or unmodified variant thereof.
A RNAi agent comprising a first and a second strand, wherein the first strand comprises at least 15 contiguous nt differing by 0, 1, 2, or 3 nt from a first strand of: AD-39870.1 (SEQ ID NO: 749), or a modified or unmodified variant thereof.

A RNAi agent comprising a first and a second strand, wherein the first strand comprises at least 15 contiguous nt differing by 0, 1, 2, or 3 nt from a first strand of: AD-39829.1 (SEQ ID NO: 750), or a modified or unmodified variant thereof.

A RNAi agent comprising a first and a second strand, wherein the first strand comprises at least 15 contiguous nt differing by 0, 1, 2, or 3 nt from a first strand of: AD-39835.1 (SEQ ID NO: 751), or a modified or unmodified variant thereof.

A RNAi agent comprising a first and a second strand, wherein the first strand comprises at least 15 contiguous nt differing by 0, 1, 2, or 3 nt from a first strand of: AD-39841.1 (SEQ ID NO: 752), or a modified or unmodified variant thereof.

A RNAi agent comprising a first and a second strand, wherein the first strand comprises at least 15 contiguous nt differing by 0, 1, 2, or 3 nt from a first strand of: AD-39853.1 (SEQ ID NO: 753), or a modified or unmodified variant thereof.

A RNAi agent comprising a first and a second strand, wherein the first strand comprises at least 15 contiguous nt differing by 0, 1, 2, or 3 nt from a first strand of: AD-39859.1 (SEQ ID NO: 754), or a modified or unmodified variant thereof.

A RNAi agent comprising a first and a second strand, wherein the first strand comprises at least 15 contiguous nt differing by 0, 1, 2, or 3 nt from a first strand of: AD-39865.1 (SEQ ID NO: 755), or a modified or unmodified variant thereof.

A RNAi agent comprising a first and a second strand, wherein the first strand comprises at least 15 contiguous nt differing by 0, 1, 2, or 3 nt from a first strand of: AD-39871.1 (SEQ ID NO: 756), or a modified or unmodified variant thereof.

A RNAi agent comprising a first and a second strand, wherein the first strand comprises at least 15 contiguous nt differing by 0, 1, 2, or 3 nt from a first strand of: AD-39830.1 (SEQ ID NO: 757), or a modified or unmodified variant thereof.

A RNAi agent comprising a first and a second strand, wherein the first strand comprises at least 15 contiguous nt differing by 0, 1, 2, or 3 nt from a first strand of: AD-39836.1 (SEQ ID NO: 758), or a modified or unmodified variant thereof.

A RNAi agent comprising a first and a second strand, wherein the first strand comprises at least 15 contiguous nt differing by 0, 1, 2, or 3 nt from a first strand of: AD-39842.1 (SEQ ID NO: 759), or a modified or unmodified variant thereof.

A RNAi agent comprising a first and a second strand, wherein the first strand comprises at least 15 contiguous nt differing by 0, 1, 2, or 3 nt from a first strand of: AD-39848.1 (SEQ ID NO: 760), or a modified or unmodified variant thereof.

A RNAi agent comprising a first and a second strand, wherein the first strand comprises at least 15 contiguous nt differing by 0, 1, 2, or 3 nt from a first strand of: AD-39854.1 (SEQ ID NO: 761), or a modified or unmodified variant thereof.

A RNAi agent comprising a first and a second strand, wherein the first strand comprises at least 15 contiguous nt differing by 0, 1, 2, or 3 nt from a first strand of: AD-39860.1 (SEQ ID NO: 762), or a modified or unmodified variant thereof.

A RNAi agent comprising a first and a second strand, wherein the first strand comprises at least 15 contiguous nt differing by 0, 1, 2, or 3 nt from a first strand of: AD-39866.1 (SEQ ID NO: 763), or a modified or unmodified variant thereof.

A RNAi agent comprising a first and a second strand, wherein the first strand comprises at least 15 contiguous nt differing by 0, 1, 2, or 3 nt from a first strand of: AD-39992.1 (SEQ ID NO: 764), or a modified or unmodified variant thereof.

A RNAi agent comprising a first and a second strand, wherein the first strand comprises at least 15 contiguous nt differing by 0, 1, 2, or 3 nt from a first strand of: AD-39998.1 (SEQ ID NO: 765), or a modified or unmodified variant thereof.

A RNAi agent comprising a first and a second strand, wherein the first strand comprises at least 15 contiguous nt differing by 0, 1, 2, or 3 nt from a first strand of: AD-40004.1 (SEQ ID NO: 766), or a modified or unmodified variant thereof.

A RNAi agent comprising a first and a second strand, wherein the first strand comprises at least 15 contiguous nt differing by 0, 1, 2, or 3 nt from a first strand of: AD-40010.1 (SEQ ID NO: 767), or a modified or unmodified variant thereof.

A RNAi agent comprising a first and a second strand, wherein the first strand comprises at least 15 contiguous nt differing by 0, 1, 2, or 3 nt from a first strand of: AD-40016.1 (SEQ ID NO: 768), or a modified or unmodified variant thereof.

A RNAi agent comprising a first and a second strand, wherein the first strand comprises at least 15 contiguous nt differing by 0, 1, 2, or 3 nt from a first strand of: AD-40022.1 (SEQ ID NO: 769), or a modified or unmodified variant thereof.

A RNAi agent comprising a first and a second strand, wherein the first strand comprises at least 15 contiguous nt differing by 0, 1, 2, or 3 nt from a first strand of: AD-40028.1 (SEQ ID NO: 770), or a modified or unmodified variant thereof.

A RNAi agent comprising a first and a second strand, wherein the first strand comprises at least 15 contiguous nt differing by 0, 1, 2, or 3 nt from a first strand of: AD-40034.1 (SEQ ID NO: 771), or a modified or unmodified variant thereof.

A RNAi agent comprising a first and a second strand, wherein the first strand comprises at least 15 contiguous nt differing by 0, 1, 2, or 3 nt from a first strand of: AD-39999.1 (SEQ ID NO: 772), or a modified or unmodified variant thereof.

A RNAi agent comprising a first and a second strand, wherein the first strand comprises at least 15 contiguous nt differing by 0, 1, 2, or 3 nt from a first strand of: AD-40005.1 (SEQ ID NO: 773), or a modified or unmodified variant thereof.

A RNAi agent comprising a first and a second strand, wherein the first strand comprises at least 15 contiguous nt differing by 0, 1, 2, or 3 nt from a first strand of: AD-40011.1 (SEQ ID NO: 774), or a modified or unmodified variant thereof.

A RNAi agent comprising a first and a second strand, wherein the first strand comprises at least 15 contiguous nt differing by 0, 1, 2, or 3 nt from a first strand of: AD-40017.1 (SEQ ID NO: 775), or a modified or unmodified variant thereof.

A RNAi agent comprising a first and a second strand, wherein the first strand comprises at least 15 contiguous nt differing by 0, 1, 2, or 3 nt from a first strand of: AD-40029.1 (SEQ ID NO: 776), or a modified or unmodified variant thereof.

A RNAi agent comprising a first and a second strand, wherein the first strand comprises at least 15 contiguous nt differing by 0, 1, 2, or 3 nt from a first strand of: AD-40035.1 (SEQ ID NO: 777), or a modified or unmodified variant thereof.

A RNAi agent comprising a first and a second strand, wherein the first strand comprises at least 15 contiguous nt differing by 0, 1, 2, or 3 nt from a first strand of: AD-39994.1 (SEQ ID NO: 778), or a modified or unmodified variant thereof.

A RNAi agent comprising a first and a second strand, wherein the first strand comprises at least 15 contiguous nt differing by 0, 1, 2, or 3 nt from a first strand of: AD-40000.1 (SEQ ID NO: 779), or a modified or unmodified variant thereof.

A RNAi agent comprising a first and a second strand, wherein the first strand comprises at least 15 contiguous nt differing by 0, 1, 2, or 3 nt from a first strand of: AD-40006.1 (SEQ ID NO: 780), or a modified or unmodified variant thereof.

A RNAi agent comprising a first and a second strand, wherein the first strand comprises at least 15 contiguous nt differing by 0, 1, 2, or 3 nt from a first strand of: AD-40012.1 (SEQ ID NO: 781), or a modified or unmodified variant thereof.

A RNAi agent comprising a first and a second strand, wherein the first strand comprises at least 15 contiguous nt differing by 0, 1, 2, or 3 nt from a first strand of: AD-40018.1 (SEQ ID NO: 782), or a modified or unmodified variant thereof.

A RNAi agent comprising a first and a second strand, wherein the first strand comprises at least 15 contiguous nt differing by 0, 1, 2, or 3 nt from a first strand of: AD-40024.1 (SEQ ID NO: 783), or a modified or unmodified variant thereof.

A RNAi agent comprising a first and a second strand, wherein the first strand comprises at least 15 contiguous nt differing by 0, 1, 2, or 3 nt from a first strand of: AD-40030.1 (SEQ ID NO: 784), or a modified or unmodified variant thereof.

A RNAi agent comprising a first and a second strand, wherein the first strand comprises at least 15 contiguous nt differing by 0, 1, 2, or 3 nt from a first strand of: AD-40036.1 (SEQ ID NO: 785), or a modified or unmodified variant thereof.

A RNAi agent comprising a first and a second strand, wherein the first strand comprises at least 15 contiguous nt differing by 0, 1, 2, or 3 nt from a first strand of: AD-39995.1 (SEQ ID NO: 786), or a modified or unmodified variant thereof.

A RNAi agent comprising a first and a second strand, wherein the first strand comprises at least 15 contiguous nt differing by 0, 1, 2, or 3 nt from a first strand of: AD-40001.1 (SEQ ID NO: 787), or a modified or unmodified variant thereof.

A RNAi agent comprising a first and a second strand, wherein the first strand comprises at least 15 contiguous nt differing by 0, 1, 2, or 3 nt from a first strand of: AD-40007.1 (SEQ ID NO: 788), or a modified or unmodified variant thereof.

A RNAi agent comprising a first and a second strand, wherein the first strand comprises at least 15 contiguous nt differing by 0, 1, 2, or 3 nt from a first strand of: AD-40013.1 (SEQ ID NO: 789), or a modified or unmodified variant thereof.

A RNAi agent comprising a first and a second strand, wherein the first strand comprises at least 15 contiguous nt differing by 0, 1, 2, or 3 nt from a first strand of: AD-40019.1 (SEQ ID NO: 790), or a modified or unmodified variant thereof.

A RNAi agent comprising a first and a second strand, wherein the first strand comprises at least 15 contiguous nt differing by 0, 1, 2, or 3 nt from a first strand of: AD-40025.1 (SEQ ID NO: 791), or a modified or unmodified variant thereof.

A RNAi agent comprising a first and a second strand, wherein the first strand comprises at least 15 contiguous nt differing by 0, 1, 2, or 3 nt from a first strand of: AD-40031.1 (SEQ ID NO: 792), or a modified or unmodified variant thereof.

A RNAi agent comprising a first and a second strand, wherein the first strand comprises at least 15 contiguous nt differing by 0, 1, 2, or 3 nt from a first strand of: AD-40037.1 (SEQ ID NO: 793), or a modified or unmodified variant thereof.

A RNAi agent comprising a first and a second strand, wherein the first strand comprises at least 15 contiguous nt differing by 0, 1, 2, or 3 nt from a first strand of: AD-39996.1 (SEQ ID NO: 794), or a modified or unmodified variant thereof.

A RNAi agent comprising a first and a second strand, wherein the first strand comprises at least 15 contiguous nt differing by 0, 1, 2, or 3 nt from a first strand of: AD-40002.1 (SEQ ID NO: 795), or a modified or unmodified variant thereof.

A RNAi agent comprising a first and a second strand, wherein the first strand comprises at least 15 contiguous nt differing by 0, 1, 2, or 3 nt from a first strand of: AD-40008.1 (SEQ ID NO: 796), or a modified or unmodified variant thereof.

A RNAi agent comprising a first and a second strand, wherein the first strand comprises at least 15 contiguous nt differing by 0, 1, 2, or 3 nt from a first strand of: AD-40014.1 (SEQ ID NO: 797), or a modified or unmodified variant thereof.

A RNAi agent comprising a first and a second strand, wherein the first strand comprises at least 15 contiguous nt differing by 0, 1, 2, or 3 nt from a first strand of: AD-40020.1 (SEQ ID NO: 798), or a modified or unmodified variant thereof.

A RNAi agent comprising a first and a second strand, wherein the first strand comprises at least 15 contiguous nt differing by 0, 1, 2, or 3 nt from a first strand of: AD-40026.1 (SEQ ID NO: 799), or a modified or unmodified variant thereof.

A RNAi agent comprising a first and a second strand, wherein the first strand comprises at least 15 contiguous nt differing by 0, 1, 2, or 3 nt from a first strand of: AD-40032.1 (SEQ ID NO: 800), or a modified or unmodified variant thereof.

A RNAi agent comprising a first and a second strand, wherein the first strand comprises at least 15 contiguous nt differing by 0, 1, 2, or 3 nt from a first strand of: AD-40038.1 (SEQ ID NO: 801), or a modified or unmodified variant thereof.

A RNAi agent comprising a first and a second strand, wherein the first strand comprises at least 15 contiguous nt differing by 0, 1, 2, or 3 nt from a first strand of: AD-39997.1 (SEQ ID NO: 802), or a modified or unmodified variant thereof.

A RNAi agent comprising a first and a second strand, wherein the first strand comprises at least 15 contiguous nt differing by 0, 1, 2, or 3 nt from a first strand of: AD-40009.1 (SEQ ID NO: 803), or a modified or unmodified variant thereof.

A RNAi agent comprising a first and a second strand, wherein the first strand comprises at least 15 contiguous nt differing by 0, 1, 2, or 3 nt from a first strand of: AD-40015.1 (SEQ ID NO: 804), or a modified or unmodified variant thereof.

A RNAi agent comprising a first and a second strand, wherein the first strand comprises at least 15 contiguous nt differing by 0, 1, 2, or 3 nt from a first strand of: AD-40021.1 (SEQ ID NO: 805), or a modified or unmodified variant thereof.

A RNAi agent comprising a first and a second strand, wherein the first strand comprises at least 15 contiguous nt differing by 0, 1, 2, or 3 nt from a first strand of: AD-40027.1 (SEQ ID NO: 806), or a modified or unmodified variant thereof.

A RNAi agent comprising a first and a second strand, wherein the first strand comprises at least 15 contiguous nt differing by 0, 1, 2, or 3 nt from a first strand of: AD-40033.1 (SEQ ID NO: 807), or a modified or unmodified variant thereof.

A RNAi agent comprising a first and a second strand, wherein the first strand comprises at least 15 contiguous nt differing by 0, 1, 2, or 3 nt from a first strand of: AD-40039.1 (SEQ ID NO: 808), or a modified or unmodified variant thereof.

A RNAi agent comprising a first and a second strand, wherein the first strand comprises at least 15 contiguous nt differing by 0, 1, 2, or 3 nt from a first strand of: AD-40045.1 (SEQ ID NO: 809), or a modified or unmodified variant thereof.

A RNAi agent comprising a first and a second strand, wherein the first strand comprises at least 15 contiguous nt differing by 0, 1, 2, or 3 nt from a first strand of: AD-40051.1 (SEQ ID NO: 810), or a modified or unmodified variant thereof.

A RNAi agent comprising a first and a second strand, wherein the first strand comprises at least 15 contiguous nt differing by 0, 1, 2, or 3 nt from a first strand of: AD-40057.1 (SEQ ID NO: 811), or a modified or unmodified variant thereof.

A RNAi agent comprising a first and a second strand, wherein the first strand comprises at least 15 contiguous nt differing by 0, 1, 2, or 3 nt from a first strand of: AD-40063.1 (SEQ ID NO: 812), or a modified or unmodified variant thereof.

A RNAi agent comprising a first and a second strand, wherein the first strand comprises at least 15 contiguous nt differing by 0, 1, 2, or 3 nt from a first strand of: AD-40069.1 (SEQ ID NO: 813), or a modified or unmodified variant thereof.

A RNAi agent comprising a first and a second strand, wherein the first strand comprises at least 15 contiguous nt differing by 0, 1, 2, or 3 nt from a first strand of: AD-40075.1 (SEQ ID NO: 814), or a modified or unmodified variant thereof.

A RNAi agent comprising a first and a second strand, wherein the first strand comprises at least 15 contiguous nt differing by 0, 1, 2, or 3 nt from a first strand of: AD-40081.1 (SEQ ID NO: 815), or a modified or unmodified variant thereof.

A RNAi agent comprising a first and a second strand, wherein the first strand comprises at least 15 contiguous nt differing by 0, 1, 2, or 3 nt from a first strand of: AD-40040.1 (SEQ ID NO: 816), or a modified or unmodified variant thereof.

A RNAi agent comprising a first and a second strand, wherein the first strand comprises at least 15 contiguous nt differing by 0, 1, 2, or 3 nt from a first strand of: AD-40046.1 (SEQ ID NO: 817), or a modified or unmodified variant thereof.

A RNAi agent comprising a first and a second strand, wherein the first strand comprises at least 15 contiguous nt differing by 0, 1, 2, or 3 nt from a first strand of: AD-40052.1 (SEQ ID NO: 818), or a modified or unmodified variant thereof.

A RNAi agent comprising a first and a second strand, wherein the first strand comprises at least 15 contiguous nt differing by 0, 1, 2, or 3 nt from a first strand of: AD-40058.1 (SEQ ID NO: 819), or a modified or unmodified variant thereof.

A RNAi agent comprising a first and a second strand, wherein the first strand comprises at least 15 contiguous nt differing by 0, 1, 2, or 3 nt from a first strand of: AD-40064.1 (SEQ ID NO: 820), or a modified or unmodified variant thereof.

A RNAi agent comprising a first and a second strand, wherein the first strand comprises at least 15 contiguous nt differing by 0, 1, 2, or 3 nt from a first strand of: AD-40070.1 (SEQ ID NO: 821), or a modified or unmodified variant thereof.

A RNAi agent comprising a first and a second strand, wherein the first strand comprises at least 15 contiguous nt differing by 0, 1, 2, or 3 nt from a first strand of: AD-40076.1 (SEQ ID NO: 822), or a modified or unmodified variant thereof.

A RNAi agent comprising a first and a second strand, wherein the first strand comprises at least 15 contiguous nt differing by 0, 1, 2, or 3 nt from a first strand of: AD-40082.1 (SEQ ID NO: 823), or a modified or unmodified variant thereof.

A RNAi agent comprising a first and a second strand, wherein the first strand comprises at least 15 contiguous nt differing by 0, 1, 2, or 3 nt from a first strand of: AD-40041.1 (SEQ ID NO: 824), or a modified or unmodified variant thereof.

A RNAi agent comprising a first and a second strand, wherein the first strand comprises at least 15 contiguous nt differing by 0, 1, 2, or 3 nt from a first strand of: AD-40047.1 (SEQ ID NO: 825), or a modified or unmodified variant thereof.

A RNAi agent comprising a first and a second strand, wherein the first strand comprises at least 15 contiguous nt differing by 0, 1, 2, or 3 nt from a first strand of: AD-40053.1 (SEQ ID NO: 826), or a modified or unmodified variant thereof.

A RNAi agent comprising a first and a second strand, wherein the first strand comprises at least 15 contiguous nt differing by 0, 1, 2, or 3 nt from a first strand of: AD-40059.1 (SEQ ID NO: 827), or a modified or unmodified variant thereof.

A RNAi agent comprising a first and a second strand, wherein the first strand comprises at least 15 contiguous nt differing by 0, 1, 2, or 3 nt from a first strand of: AD-40065.1 (SEQ ID NO: 828), or a modified or unmodified variant thereof.

A RNAi agent comprising a first and a second strand, wherein the first strand comprises at least 15 contiguous nt differing by 0, 1, 2, or 3 nt from a first strand of: AD-40071.1 (SEQ ID NO: 829), or a modified or unmodified variant thereof.

A RNAi agent comprising a first and a second strand, wherein the first strand comprises at least 15 contiguous nt differing by 0, 1, 2, or 3 nt from a first strand of: AD-40077.1 (SEQ ID NO: 830), or a modified or unmodified variant thereof.

A RNAi agent comprising a first and a second strand, wherein the first strand comprises at least 15 contiguous nt differing by 0, 1, 2, or 3 nt from a first strand of: AD-40083.1 (SEQ ID NO: 831), or a modified or unmodified variant thereof.

A RNAi agent comprising a first and a second strand, wherein the first strand comprises at least 15 contiguous nt differing by 0, 1, 2, or 3 nt from a first strand of: AD-40042.1 (SEQ ID NO: 832), or a modified or unmodified variant thereof.

A RNAi agent comprising a first and a second strand, wherein the first strand comprises at least 15 contiguous nt differing by 0, 1, 2, or 3 nt from a first strand of: AD-40048.1 (SEQ ID NO: 833), or a modified or unmodified variant thereof.

A RNAi agent comprising a first and a second strand, wherein the first strand comprises at least 15 contiguous nt differing by 0, 1, 2, or 3 nt from a first strand of: AD-40054.1 (SEQ ID NO: 834), or a modified or unmodified variant thereof.

A RNAi agent comprising a first and a second strand, wherein the first strand comprises at least 15 contiguous nt differing by 0, 1, 2, or 3 nt from a first strand of: AD-40060.1 (SEQ ID NO: 835), or a modified or unmodified variant thereof.

A RNAi agent comprising a first and a second strand, wherein the first strand comprises at least 15 contiguous nt differing by 0, 1, 2, or 3 nt from a first strand of: AD-40066.1 (SEQ ID NO: 836), or a modified or unmodified variant thereof.

A RNAi agent comprising a first and a second strand, wherein the first strand comprises at least 15 contiguous nt differing by 0, 1, 2, or 3 nt from a first strand of: AD-40072.1 (SEQ ID NO: 837), or a modified or unmodified variant thereof.
A RNAi agent comprising a first and a second strand, wherein the first strand comprises at least 15 contiguous nt differing by 0, 1, 2, or 3 nt from a first strand of: AD-40078.1 (SEQ ID NO: 838), or a modified or unmodified variant thereof.
A RNAi agent comprising a first and a second strand, wherein the first strand comprises at least 15 contiguous nt differing by 0, 1, 2, or 3 nt from a first strand of: AD-40084.1 (SEQ ID NO: 839), or a modified or unmodified variant thereof.
A RNAi agent comprising a first and a second strand, wherein the first strand comprises at least 15 contiguous nt differing by 0, 1, 2, or 3 nt from a first strand of: AD-40043.1 (SEQ ID NO: 840), or a modified or unmodified variant thereof.
A RNAi agent comprising a first and a second strand, wherein the first strand comprises at least 15 contiguous nt differing by 0, 1, 2, or 3 nt from a first strand of: AD-40049.1 (SEQ ID NO: 841), or a modified or unmodified variant thereof.
A RNAi agent comprising a first and a second strand, wherein the first strand comprises at least 15 contiguous nt differing by 0, 1, 2, or 3 nt from a first strand of: AD-40055.1 (SEQ ID NO: 842), or a modified or unmodified variant thereof.
A RNAi agent comprising a first and a second strand, wherein the first strand comprises at least 15 contiguous nt differing by 0, 1, 2, or 3 nt from a first strand of: AD-40061.1 (SEQ ID NO: 843), or a modified or unmodified variant thereof.
A RNAi agent comprising a first and a second strand, wherein the first strand comprises at least 15 contiguous nt differing by 0, 1, 2, or 3 nt from a first strand of: AD-40067.1 (SEQ ID NO: 844), or a modified or unmodified variant thereof.
A RNAi agent comprising a first and a second strand, wherein the first strand comprises at least 15 contiguous nt differing by 0, 1, 2, or 3 nt from a first strand of: AD-40073.1 (SEQ ID NO: 845), or a modified or unmodified variant thereof.
A RNAi agent comprising a first and a second strand, wherein the first strand comprises at least 15 contiguous nt differing by 0, 1, 2, or 3 nt from a first strand of: AD-40079.1 (SEQ ID NO: 846), or a modified or unmodified variant thereof.
A RNAi agent comprising a first and a second strand, wherein the first strand comprises at least 15 contiguous nt differing by 0, 1, 2, or 3 nt from a first strand of: AD-40085.1 (SEQ ID NO: 847), or a modified or unmodified variant thereof.
A RNAi agent comprising a first and a second strand, wherein the first strand comprises at least 15 contiguous nt differing by 0, 1, 2, or 3 nt from a first strand of: AD-40044.1 (SEQ ID NO: 848), or a modified or unmodified variant thereof.
A RNAi agent comprising a first and a second strand, wherein the first strand comprises at least 15 contiguous nt differing by 0, 1, 2, or 3 nt from a first strand of: AD-40050.1 (SEQ ID NO: 849), or a modified or unmodified variant thereof.
A RNAi agent comprising a first and a second strand, wherein the first strand comprises at least 15 contiguous nt differing by 0, 1, 2, or 3 nt from a first strand of: AD-40056.1 (SEQ ID NO: 850), or a modified or unmodified variant thereof.
A RNAi agent comprising a first and a second strand, wherein the first strand comprises at least 15 contiguous nt differing by 0, 1, 2, or 3 nt from a first strand of: AD-40062.1 (SEQ ID NO: 851), or a modified or unmodified variant thereof.
A RNAi agent comprising a first and a second strand, wherein the first strand comprises at least 15 contiguous nt differing by 0, 1, 2, or 3 nt from a first strand of: AD-40068.1 (SEQ ID NO: 852), or a modified or unmodified variant thereof.
A RNAi agent comprising a first and a second strand, wherein the first strand comprises at least 15 contiguous nt differing by 0, 1, 2, or 3 nt from a first strand of: AD-40074.1 (SEQ ID NO: 853), or a modified or unmodified variant thereof.
A RNAi agent comprising a first and a second strand, wherein the first strand comprises at least 15 contiguous nt differing by 0, 1, 2, or 3 nt from a first strand of: AD-40080.1 (SEQ ID NO: 854), or a modified or unmodified variant thereof.
A RNAi agent comprising a first and a second strand, wherein the first strand comprises at least 15 contiguous nt differing by 0, 1, 2, or 3 nt from a first strand of: AD-39720.1 (SEQ ID NO: 1754), or a modified or unmodified variant thereof.
A RNAi agent comprising a first and a second strand, wherein the first strand comprises at least 15 contiguous nt differing by 0, 1, 2, or 3 nt from a first strand of: AD-39706.1 (SEQ ID NO: 1755), or a modified or unmodified variant thereof.

A RNAi agent comprising a first and a second strand, wherein the first strand comprises at least 15 contiguous nt differing by 0, 1, 2, or 3 nt from a first strand of: AD-39712.1 (SEQ ID NO: 1756), or a modified or unmodified variant thereof.

A RNAi agent comprising a first and a second strand, wherein the first strand comprises at least 15 contiguous nt differing by 0, 1, 2, or 3 nt from a first strand of: AD-39760.1 (SEQ ID NO: 1757), or a modified or unmodified variant thereof.

A RNAi agent comprising a first and a second strand, wherein the first strand comprises at least 15 contiguous nt differing by 0, 1, 2, or 3 nt from a first strand of: AD-39732.1 (SEQ ID NO: 1758), or a modified or unmodified variant thereof.

A RNAi agent comprising a first and a second strand, wherein the first strand comprises at least 15 contiguous nt differing by 0, 1, 2, or 3 nt from a first strand of: AD-35541.4 (SEQ ID NO: 1759), or a modified or unmodified variant thereof.

A RNAi agent comprising a first and a second strand, wherein the first strand comprises at least 15 contiguous nt differing by 0, 1, 2, or 3 nt from a first strand of: AD-39735.1 or hs_KRAS_ 321_A22S26 (SEQ ID NO: 1760), or a modified or unmodified variant thereof.

A RNAi agent comprising a first and a second strand, wherein the first strand comprises at least 15 contiguous nt differing by 0, 1, 2, or 3 nt from a first strand of: AD-39741.1, or modified or unmodified variants thereof.

A RNAi agent comprising a first and a second strand, wherein the first strand comprises at least 15 contiguous nt differing by 0, 1, 2, or 3 nt from a first strand of: hs_KRAS_ 322_A22S26 (SEQ ID NO: 1761), or a modified or unmodified variant thereof.

A RNAi agent comprising a first and a second strand, wherein the first strand comprises at least 15 contiguous nt differing by 0, 1, 2, or 3 nt from a first strand of: AD-39778.1 (SEQ ID NO: 1762), or a modified or unmodified variant thereof.

A RNAi agent comprising a first and a second strand, wherein the first strand comprises at least 15 contiguous nt differing by 0, 1, 2, or 3 nt from a first strand of: AD-39790.1 (SEQ ID NO: 1763), or a modified or unmodified variant thereof.

A RNAi agent comprising a first and a second strand, wherein the first strand comprises at least 15 contiguous nt differing by 0, 1, 2, or 3 nt from a first strand of: AD-39822.1 (SEQ ID NO: 1764), or a modified or unmodified variant thereof.

A RNAi agent comprising a first and a second strand, wherein the first strand comprises at least 15 contiguous nt differing by 0, 1, 2, or 3 nt from a first strand of: AD-35609.4 (SEQ ID NO: 1765), or a modified or unmodified variant thereof.

A RNAi agent comprising a first and a second strand, wherein the first strand comprises at least 15 contiguous nt differing by 0, 1, 2, or 3 nt from a first strand of: AD-35581.4 (SEQ ID NO: 1766), or a modified or unmodified variant thereof.

A RNAi agent comprising a first and a second strand, wherein the first strand comprises at least 15 contiguous nt differing by 0, 1, 2, or 3 nt from a first strand of: AD-39845.1 (SEQ ID NO: 1767), or a modified or unmodified variant thereof.

A RNAi agent comprising a first and a second strand, wherein the first strand comprises at least 15 contiguous nt differing by 0, 1, 2, or 3 nt from a first strand of: AD-39858.1 (SEQ ID NO: 1768), or a modified or unmodified variant thereof.

A RNAi agent comprising a first and a second strand, wherein the first strand comprises at least 15 contiguous nt differing by 0, 1, 2, or 3 nt from a first strand of: AD-39870.1 (SEQ ID NO: 1769), or a modified or unmodified variant thereof.

A RNAi agent comprising a first and a second strand, wherein the first strand comprises at least 15 contiguous nt differing by 0, 1, 2, or 3 nt from a first strand of: AD-35576.4 (SEQ ID NO: 1770), or a modified or unmodified variant thereof.

A RNAi agent comprising a first and a second strand, wherein the first strand comprises at least 15 contiguous nt differing by 0, 1, 2, or 3 nt from a first strand of: AD-35588.4 (SEQ ID NO: 1771), or a modified or unmodified variant thereof.

A RNAi agent comprising a first and a second strand, wherein the first strand comprises at least 15 contiguous nt differing by 0, 1, 2, or 3 nt from a first strand of: AD-35600.1 (SEQ ID NO: 1772), or a modified or unmodified variant thereof.

A RNAi agent comprising a first and a second strand, wherein the first strand comprises at least 15 contiguous nt differing by 0, 1, 2, or 3 nt from a first strand of: AD-35606.1 (SEQ ID NO: 1773), or a modified or unmodified variant thereof.

A RNAi agent comprising a first and a second strand, wherein the first strand comprises at least 15 contiguous nt differing by 0, 1, 2, or 3 nt from a first strand of: AD-38151.1 (SEQ ID NO: 1774), or a modified or unmodified variant thereof.

A RNAi agent comprising a first and a second strand, wherein the first strand comprises at least 15 contiguous nt differing by 0, 1, 2, or 3 nt from a first strand of: AD-38163.1 (SEQ ID NO: 1775), or a modified or unmodified variant thereof.

A RNAi agent comprising a first and a second strand, wherein the first strand comprises at least 15 contiguous nt differing by 0, 1, 2, or 3 nt from a first strand of: AD-39999.1 (SEQ ID NO: 1776), or a modified or unmodified variant thereof.

A RNAi agent comprising a first and a second strand, wherein the first strand comprises at least 15 contiguous nt differing by 0, 1, 2, or 3 nt from a first strand of: AD-38159.1 or hs_KRAS_ 528_A22S26 (SEQ ID NO: 1777), or a modified or unmodified variant thereof.

A RNAi agent comprising a first and a second strand, wherein the first strand comprises at least 15 contiguous nt differing by 0, 1, 2, or 3 nt from a first strand of: AD-38130.1 or hs_KRAS_ 531_A22S26 (SEQ ID NO: 1778), or a modified or unmodified variant thereof.

A RNAi agent comprising a first and a second strand, wherein the first strand comprises at least 15 contiguous nt differing by 0, 1, 2, or 3 nt from a first strand of: AD-38136.1 (SEQ ID NO: 1779), or a modified or unmodified variant thereof.

A RNAi agent comprising a first and a second strand, wherein the first strand comprises at least 15 contiguous nt differing by 0, 1, 2, or 3 nt from a first strand of: AD-40036.1 (SEQ ID NO: 1780), or a modified or unmodified variant thereof.

A RNAi agent comprising a first and a second strand, wherein the first strand comprises at least 15 contiguous nt differing by 0, 1, 2, or 3 nt from a first strand of: AD-40008.1 (SEQ ID NO: 1781), or a modified or unmodified variant thereof.

A RNAi agent comprising a first and a second strand, wherein the first strand comprises at least 15 contiguous nt differing by 0, 1, 2, or 3 nt from a first strand of: AD-40021.1 (SEQ ID NO: 1782), or a modified or unmodified variant thereof.

A RNAi agent comprising a first and a second strand, wherein the first strand comprises at least 15 contiguous nt differing by 0, 1, 2, or 3 nt from a first strand of: AD-40077.1 (SEQ ID NO: 1783), or a modified or unmodified variant thereof.

A RNAi agent comprising a first and a second strand, wherein the first strand comprises at least 15 contiguous nt differing by 0, 1, 2, or 3 nt from a first strand of: AD-40072.1 (SEQ ID NO: 1784), or a modified or unmodified variant thereof.

A RNAi agent comprising a first and a second strand, wherein the first strand comprises at least 15 contiguous nt differing by 0, 1, 2, or 3 nt from a first strand of: AD-40061.1 (SEQ ID NO: 1785), or a modified or unmodified variant thereof.

A RNAi agent comprising a first and a second strand, wherein the first strand comprises at least 15 contiguous nt differing by 0, 1, 2, or 3 nt from a first strand of: AD-40068.1 (SEQ ID NO: 1786), or a modified or unmodified variant thereof.

A RNAi agent comprising a first and a second strand, wherein the first strand comprises at least 15 contiguous nt differing by 0, 1, 2, or 3 nt from a first strand of: AD-38131.1 (SEQ ID NO: 1787), or a modified or unmodified variant thereof.

A RNAi agent comprising a first and a second strand, wherein the first strand comprises at least 15 contiguous nt differing by 0, 1, 2, or 3 nt from a first strand of: AD-38167.1 (SEQ ID NO: 1788), or a modified or unmodified variant thereof.

A RNAi agent comprising a first and a second strand, wherein the first strand comprises at least 15 contiguous nt differing by 0, 1, 2, or 3 nt from a first strand of: hs_KRAS 1273_A22S26 (SEQ ID NO: 1789), or a modified or unmodified variant thereof.

A RNAi agent comprising a first and a second strand, wherein the first strand comprises at least 15 contiguous nt differing by 0, 1, 2, or 3 nt from a first strand of: hs_KRAS 2892_A37S26 (SEQ ID NO: 1790), or a modified or unmodified variant thereof.

A RNAi agent comprising a first and a second strand, wherein the first strand comprises at least 15 contiguous nt differing by 0, 1, 2, or 3 nt from a first strand of: hs_KRAS 4731_A22S26 (SEQ ID NO: 1791), or a modified or unmodified variant thereof.

**Specific embodiments comprising at least 15 contiguous nucleotides of any RNAi agent disclosed herein**

In one embodiment, the present disclosure pertains to: a composition comprising any one or more of: a RNAi agent comprising a first strand and a second strand, wherein the first strand comprises at least 15 contiguous nucleotides (differing by 0 nucleotides) from a first strand of: any RNAi agent from any of Tables 1 to 6 or modified or unmodified variants thereof.

Thus, in various embodiments, the present disclosure pertains to a composition comprising any one or more of the following:

A RNAi agent comprising a first strand and a second strand, wherein the first strand comprises at least 15 contiguous nucleotides from a first strand of: AD-35523.3 (SEQ ID NO: 428), or a modified or unmodified variant thereof.

A RNAi agent comprising a first strand and a second strand, wherein the first strand comprises at least 15 contiguous nucleotides from a first strand of: AD-35529.1 (SEQ ID NO: 429), or a modified or unmodified variant thereof.

A RNAi agent comprising a first strand and a second strand, wherein the first strand comprises at least 15 contiguous nucleotides from a first strand of: AD-35535.4 (SEQ ID NO: 430), or a modified or unmodified variant thereof.

A RNAi agent comprising a first strand and a second strand, wherein the first strand comprises at least 15 contiguous nucleotides from a first strand of: AD-35541.4 (SEQ ID NO: 431), or a modified or unmodified variant thereof.

A RNAi agent comprising a first strand and a second strand, wherein the first strand comprises at least 15 contiguous nucleotides from a first strand of: AD-35547.4 (SEQ ID NO: 432), or a modified or unmodified variant thereof.

A RNAi agent comprising a first strand and a second strand, wherein the first strand comprises at least 15 contiguous nucleotides from a first strand of: AD-35553.4 (SEQ ID NO: 433), or a modified or unmodified variant thereof.

A RNAi agent comprising a first strand and a second strand, wherein the first strand comprises at least 15 contiguous nucleotides from a first strand of: AD-35559.4 (SEQ ID NO: 434), or a modified or unmodified variant thereof.

A RNAi agent comprising a first strand and a second strand, wherein the first strand comprises at least 15 contiguous nucleotides from a first strand of: AD-35565.4 (SEQ ID NO: 435), or a modified or unmodified variant thereof.

A RNAi agent comprising a first strand and a second strand, wherein the first strand comprises at least 15 contiguous nucleotides from a first strand of: AD-35524.4 (SEQ ID NO: 436), or a modified or unmodified variant thereof.

A RNAi agent comprising a first strand and a second strand, wherein the first strand comprises at least 15 contiguous nucleotides from a first strand of: AD-35530.4 (SEQ ID NO: 437), or a modified or unmodified variant thereof.

A RNAi agent comprising a first strand and a second strand, wherein the first strand comprises at least 15 contiguous nucleotides from a first strand of: AD-35536.4 (SEQ ID NO: 438), or a modified or unmodified variant thereof.

A RNAi agent comprising a first strand and a second strand, wherein the first strand comprises at least 15 contiguous nucleotides from a first strand of: AD-35542.4 (SEQ ID NO: 439), or a modified or unmodified variant thereof.

A RNAi agent comprising a first strand and a second strand, wherein the first strand comprises at least 15 contiguous nucleotides from a first strand of: AD-35548.4 (SEQ ID NO: 440), or a modified or unmodified variant thereof.

A RNAi agent comprising a first strand and a second strand, wherein the first strand comprises at least 15 contiguous nucleotides from a first strand of: AD-35554.4 (SEQ ID NO: 441), or a modified or unmodified variant thereof.

A RNAi agent comprising a first strand and a second strand, wherein the first strand comprises at least 15 contiguous nucleotides from a first strand of: AD-35560.2 (SEQ ID NO: 442), or a modified or unmodified variant thereof.

A RNAi agent comprising a first strand and a second strand, wherein the first strand comprises at least 15 contiguous nucleotides from a first strand of: AD-35566.4 (SEQ ID NO: 443), or a modified or unmodified variant thereof.

A RNAi agent comprising a first strand and a second strand, wherein the first strand comprises at least 15 contiguous nucleotides from a first strand of: AD-35525.2 (SEQ ID NO: 444), or a modified or unmodified variant thereof.

A RNAi agent comprising a first strand and a second strand, wherein the first strand comprises at least 15 contiguous nucleotides from a first strand of: AD-35531.4 (SEQ ID NO: 445), or a modified or unmodified variant thereof.

A RNAi agent comprising a first strand and a second strand, wherein the first strand comprises at least 15 contiguous nucleotides from a first strand of: AD-35537.4 (SEQ ID NO: 446), or a modified or unmodified variant thereof.

A RNAi agent comprising a first strand and a second strand, wherein the first strand comprises at least 15 contiguous nucleotides from a first strand of: AD-35543.2 (SEQ ID NO: 447), or a modified or unmodified variant thereof.

A RNAi agent comprising a first strand and a second strand, wherein the first strand comprises at least 15 contiguous nucleotides from a first strand of: AD-35549.4 (SEQ ID NO: 448), or a modified or unmodified variant thereof.

A RNAi agent comprising a first strand and a second strand, wherein the first strand comprises at least 15 contiguous nucleotides from a first strand of: AD-35555.4 (SEQ ID NO: 449), or a modified or unmodified variant thereof.

A RNAi agent comprising a first strand and a second strand, wherein the first strand comprises at least 15 contiguous nucleotides from a first strand of: AD-35561.4 (SEQ ID NO: 450), or a modified or unmodified variant thereof.

A RNAi agent comprising a first strand and a second strand, wherein the first strand comprises at least 15 contiguous nucleotides from a first strand of: AD-35567.4 (SEQ ID NO: 451), or a modified or unmodified variant thereof.

A RNAi agent comprising a first strand and a second strand, wherein the first strand comprises at least 15 contiguous nucleotides from a first strand of: AD-35526.4 (SEQ ID NO: 452), or a modified or unmodified variant thereof.

A RNAi agent comprising a first strand and a second strand, wherein the first strand comprises at least 15 contiguous nucleotides from a first strand of: AD-35532.4 (SEQ ID NO: 453), or a modified or unmodified variant thereof.

A RNAi agent comprising a first strand and a second strand, wherein the first strand comprises at least 15 contiguous nucleotides from a first strand of: AD-35538.4 (SEQ ID NO: 454), or a modified or unmodified variant thereof.

A RNAi agent comprising a first strand and a second strand, wherein the first strand comprises at least 15 contiguous nucleotides from a first strand of: AD-35544.4 (SEQ ID NO: 455), or a modified or unmodified variant thereof.

A RNAi agent comprising a first strand and a second strand, wherein the first strand comprises at least 15 contiguous nucleotides from a first strand of: AD-35550.4 (SEQ ID NO: 456), or a modified or unmodified variant thereof.

A RNAi agent comprising a first strand and a second strand, wherein the first strand comprises at least 15 contiguous nucleotides from a first strand of: AD-35556.4 (SEQ ID NO: 457), or a modified or unmodified variant thereof.

A RNAi agent comprising a first strand and a second strand, wherein the first strand comprises at least 15 contiguous nucleotides from a first strand of: AD-35562.4 (SEQ ID NO: 458), or a modified or unmodified variant thereof.

A RNAi agent comprising a first strand and a second strand, wherein the first strand comprises at least 15 contiguous nucleotides from a first strand of: AD-35568.4 (SEQ ID NO: 459), or a modified or unmodified variant thereof.

A RNAi agent comprising a first strand and a second strand, wherein the first strand comprises at least 15 contiguous nucleotides from a first strand of: AD-35527.4 (SEQ ID NO: 460), or a modified or unmodified variant thereof.

A RNAi agent comprising a first strand and a second strand, wherein the first strand comprises at least 15 contiguous nucleotides from a first strand of: AD-35533.4 (SEQ ID NO: 461), or a modified or unmodified variant thereof.

A RNAi agent comprising a first strand and a second strand, wherein the first strand comprises at least 15 contiguous nucleotides from a first strand of: AD-35539.4 (SEQ ID NO: 462), or a modified or unmodified variant thereof.

A RNAi agent comprising a first strand and a second strand, wherein the first strand comprises at least 15 contiguous nucleotides from a first strand of: AD-35545.4 (SEQ ID NO: 463), or a modified or unmodified variant thereof.

A RNAi agent comprising a first strand and a second strand, wherein the first strand comprises at least 15 contiguous nucleotides from a first strand of: AD-35551.4 (SEQ ID NO: 464), or a modified or unmodified variant thereof.

A RNAi agent comprising a first strand and a second strand, wherein the first strand comprises at least 15 contiguous nucleotides from a first strand of: AD-35563.4 (SEQ ID NO: 465), or a modified or unmodified variant thereof.

A RNAi agent comprising a first strand and a second strand, wherein the first strand comprises at least 15 contiguous nucleotides from a first strand of: AD-35528.4 (SEQ ID NO: 466), or a modified or unmodified variant thereof.

A RNAi agent comprising a first strand and a second strand, wherein the first strand comprises at least 15 contiguous nucleotides from a first strand of: AD-35540.4 (SEQ ID NO: 467), or a modified or unmodified variant thereof.

A RNAi agent comprising a first strand and a second strand, wherein the first strand comprises at least 15 contiguous nucleotides from a first strand of: AD-35546.4 (SEQ ID NO: 468), or a modified or unmodified variant thereof.

A RNAi agent comprising a first strand and a second strand, wherein the first strand comprises at least 15 contiguous nucleotides from a first strand of: AD-35552.4 (SEQ ID NO: 469), or a modified or unmodified variant thereof.

A RNAi agent comprising a first strand and a second strand, wherein the first strand comprises at least 15 contiguous nucleotides from a first strand of: AD-35558.4 (SEQ ID NO: 470), or a modified or unmodified variant thereof.

A RNAi agent comprising a first strand and a second strand, wherein the first strand comprises at least 15 contiguous nucleotides from a first strand of: AD-35564.4 (SEQ ID NO: 471), or a modified or unmodified variant thereof.

A RNAi agent comprising a first strand and a second strand, wherein the first strand comprises at least 15 contiguous nucleotides from a first strand of: AD-35570.4 (SEQ ID NO: 472), or a modified or unmodified variant thereof.

A RNAi agent comprising a first strand and a second strand, wherein the first strand comprises at least 15 contiguous nucleotides from a first strand of: AD-35571.4 (SEQ ID NO: 473), or a modified or unmodified variant thereof.

A RNAi agent comprising a first strand and a second strand, wherein the first strand comprises at least 15 contiguous nucleotides from a first strand of: AD-35577.4 (SEQ ID NO: 474), or a modified or unmodified variant thereof.

A RNAi agent comprising a first strand and a second strand, wherein the first strand comprises at least 15 contiguous nucleotides from a first strand of: AD-35583.4 (SEQ ID NO: 475), or a modified or unmodified variant thereof.

A RNAi agent comprising a first strand and a second strand, wherein the first strand comprises at least 15 contiguous nucleotides from a first strand of: AD-35589.4 (SEQ ID NO: 476), or a modified or unmodified variant thereof.

A RNAi agent comprising a first strand and a second strand, wherein the first strand comprises at least 15 contiguous nucleotides from a first strand of: AD-35595.4 (SEQ ID NO: 477), or a modified or unmodified variant thereof.

A RNAi agent comprising a first strand and a second strand, wherein the first strand comprises at least 15 contiguous nucleotides from a first strand of: AD-35601.4 (SEQ ID NO: 478), or a modified or unmodified variant thereof.

A RNAi agent comprising a first strand and a second strand, wherein the first strand comprises at least 15 contiguous nucleotides from a first strand of: AD-35607.4 (SEQ ID NO: 479), or a modified or unmodified variant thereof.

A RNAi agent comprising a first strand and a second strand, wherein the first strand comprises at least 15 contiguous nucleotides from a first strand of: AD-35613.4 (SEQ ID NO: 480), or a modified or unmodified variant thereof.

A RNAi agent comprising a first strand and a second strand, wherein the first strand comprises at least 15 contiguous nucleotides from a first strand of: AD-35572.4 (SEQ ID NO: 481), or a modified or unmodified variant thereof.

A RNAi agent comprising a first strand and a second strand, wherein the first strand comprises at least 15 contiguous nucleotides from a first strand of: AD-35578.4 (SEQ ID NO: 482), or a modified or unmodified variant thereof.

A RNAi agent comprising a first strand and a second strand, wherein the first strand comprises at least 15 contiguous nucleotides from a first strand of: AD-35584.4 (SEQ ID NO: 483), or a modified or unmodified variant thereof.

A RNAi agent comprising a first strand and a second strand, wherein the first strand comprises at least 15 contiguous nucleotides from a first strand of: AD-35590.4 (SEQ ID NO: 484), or a modified or unmodified variant thereof.

A RNAi agent comprising a first strand and a second strand, wherein the first strand comprises at least 15 contiguous nucleotides from a first strand of: AD-35596.4 (SEQ ID NO: 485), or a modified or unmodified variant thereof.

A RNAi agent comprising a first strand and a second strand, wherein the first strand comprises at least 15 contiguous nucleotides from a first strand of: AD-35602.4 (SEQ ID NO: 486), or a modified or unmodified variant thereof.

A RNAi agent comprising a first strand and a second strand, wherein the first strand comprises at least 15 contiguous nucleotides from a first strand of: AD-35608.4 (SEQ ID NO: 487), or a modified or unmodified variant thereof.

A RNAi agent comprising a first strand and a second strand, wherein the first strand comprises at least 15 contiguous nucleotides from a first strand of: AD-35614.4 (SEQ ID NO: 488), or a modified or unmodified variant thereof.

A RNAi agent comprising a first strand and a second strand, wherein the first strand comprises at least 15 contiguous nucleotides from a first strand of: AD-35573.4 (SEQ ID NO: 489), or a modified or unmodified variant thereof.

A RNAi agent comprising a first strand and a second strand, wherein the first strand comprises at least 15 contiguous nucleotides from a first strand of: AD-35579.4 (SEQ ID NO: 490), or a modified or unmodified variant thereof.

A RNAi agent comprising a first strand and a second strand, wherein the first strand comprises at least 15 contiguous nucleotides from a first strand of: AD-35585.4 (SEQ ID NO: 491), or a modified or unmodified variant thereof.

A RNAi agent comprising a first strand and a second strand, wherein the first strand comprises at least 15 contiguous nucleotides from a first strand of: AD-35591.4 (SEQ ID NO: 492), or a modified or unmodified variant thereof.

A RNAi agent comprising a first strand and a second strand, wherein the first strand comprises at least 15 contiguous nucleotides from a first strand of: AD-35597.4 (SEQ ID NO: 493), or a modified or unmodified variant thereof.

A RNAi agent comprising a first strand and a second strand, wherein the first strand comprises at least 15 contiguous nucleotides from a first strand of: AD-35603.4 (SEQ ID NO: 494), or a modified or unmodified variant thereof.

A RNAi agent comprising a first strand and a second strand, wherein the first strand comprises at least 15 contiguous nucleotides from a first strand of: AD-35609.4 (SEQ ID NO: 495), or a modified or unmodified variant thereof.

A RNAi agent comprising a first strand and a second strand, wherein the first strand comprises at least 15 contiguous nucleotides from a first strand of: AD-35615.4 (SEQ ID NO: 496), or a modified or unmodified variant thereof.

A RNAi agent comprising a first strand and a second strand, wherein the first strand comprises at least 15 contiguous nucleotides from a first strand of: AD-35574.4 (SEQ ID NO: 497), or a modified or unmodified variant thereof.

A RNAi agent comprising a first strand and a second strand, wherein the first strand comprises at least 15 contiguous nucleotides from a first strand of: AD-35580.1 (SEQ ID NO: 498), or a modified or unmodified variant thereof.

A RNAi agent comprising a first strand and a second strand, wherein the first strand comprises at least 15 contiguous nucleotides from a first strand of: AD-35586.4 (SEQ ID NO: 499), or a modified or unmodified variant thereof.

A RNAi agent comprising a first strand and a second strand, wherein the first strand comprises at least 15 contiguous nucleotides from a first strand of: AD-35592.4 (SEQ ID NO: 500), or a modified or unmodified variant thereof.

A RNAi agent comprising a first strand and a second strand, wherein the first strand comprises at least 15 contiguous nucleotides from a first strand of: AD-35598.4 (SEQ ID NO: 501), or a modified or unmodified variant thereof.

A RNAi agent comprising a first strand and a second strand, wherein the first strand comprises at least 15 contiguous nucleotides from a first strand of: AD-35604.4 (SEQ ID NO: 502), or a modified or unmodified variant thereof.

A RNAi agent comprising a first strand and a second strand, wherein the first strand comprises at least 15 contiguous nucleotides from a first strand of: AD-35610.4 (SEQ ID NO: 503), or a modified or unmodified variant thereof.

A RNAi agent comprising a first strand and a second strand, wherein the first strand comprises at least 15 contiguous nucleotides from a first strand of: AD-35616.4 (SEQ ID NO: 504), or a modified or unmodified variant thereof.

A RNAi agent comprising a first strand and a second strand, wherein the first strand comprises at least 15 contiguous nucleotides from a first strand of: AD-35575.4 (SEQ ID NO: 505), or a modified or unmodified variant thereof.

A RNAi agent comprising a first strand and a second strand, wherein the first strand comprises at least 15 contiguous nucleotides from a first strand of: AD-35581.4 (SEQ ID NO: 506), or a modified or unmodified variant thereof.

A RNAi agent comprising a first strand and a second strand, wherein the first strand comprises at least 15 contiguous nucleotides from a first strand of: AD-35587.4 (SEQ ID NO: 507), or a modified or unmodified variant thereof.

A RNAi agent comprising a first strand and a second strand, wherein the first strand comprises at least 15 contiguous nucleotides from a first strand of: AD-35593.4 (SEQ ID NO: 508), or a modified or unmodified variant thereof.

A RNAi agent comprising a first strand and a second strand, wherein the first strand comprises at least 15 contiguous nucleotides from a first strand of: AD-35599.4 (SEQ ID NO: 509), or a modified or unmodified variant thereof.

A RNAi agent comprising a first strand and a second strand, wherein the first strand comprises at least 15 contiguous nucleotides from a first strand of: AD-35605.4 (SEQ ID NO: 510), or a modified or unmodified variant thereof.

A RNAi agent comprising a first strand and a second strand, wherein the first strand comprises at least 15 contiguous nucleotides from a first strand of: AD-35611.4 (SEQ ID NO: 511), or a modified or unmodified variant thereof.

A RNAi agent comprising a first strand and a second strand, wherein the first strand comprises at least 15 contiguous nucleotides from a first strand of: AD-35617.4 (SEQ ID NO: 512), or a modified or unmodified variant thereof.

A RNAi agent comprising a first strand and a second strand, wherein the first strand comprises at least 15 contiguous nucleotides from a first strand of: AD-35576.4 (SEQ ID NO: 513), or a modified or unmodified variant thereof.

A RNAi agent comprising a first strand and a second strand, wherein the first strand comprises at least 15 contiguous nucleotides from a first strand of: AD-35588.4 (SEQ ID NO: 514), or a modified or unmodified variant thereof.

A RNAi agent comprising a first strand and a second strand, wherein the first strand comprises at least 15 contiguous nucleotides from a first strand of: AD-35667.1 (SEQ ID NO: 515), or a modified or unmodified variant thereof.

A RNAi agent comprising a first strand and a second strand, wherein the first strand comprises at least 15 contiguous nucleotides from a first strand of: AD-35673.1 (SEQ ID NO: 516), or a modified or unmodified variant thereof.

A RNAi agent comprising a first strand and a second strand, wherein the first strand comprises at least 15 contiguous nucleotides from a first strand of: AD-35679.1 (SEQ ID NO: 517), or a modified or unmodified variant thereof.

A RNAi agent comprising a first strand and a second strand, wherein the first strand comprises at least 15 contiguous nucleotides from a first strand of: AD-35685.1 (SEQ ID NO: 518), or a modified or unmodified variant thereof.

A RNAi agent comprising a first strand and a second strand, wherein the first strand comprises at least 15 contiguous nucleotides from a first strand of: AD-35691.1 (SEQ ID NO: 519), or a modified or unmodified variant thereof.

A RNAi agent comprising a first strand and a second strand, wherein the first strand comprises at least 15 contiguous nucleotides from a first strand of: AD-35557.1 (SEQ ID NO: 520), or a modified or unmodified variant thereof.

A RNAi agent comprising a first strand and a second strand, wherein the first strand comprises at least 15 contiguous nucleotides from a first strand of: AD-35618.1 (SEQ ID NO: 521), or a modified or unmodified variant thereof.

A RNAi agent comprising a first strand and a second strand, wherein the first strand comprises at least 15 contiguous nucleotides from a first strand of: AD-35569.1 (SEQ ID NO: 522), or a modified or unmodified variant thereof.

A RNAi agent comprising a first strand and a second strand, wherein the first strand comprises at least 15 contiguous nucleotides from a first strand of: AD-35534.1 (SEQ ID NO: 523), or a modified or unmodified variant thereof.

A RNAi agent comprising a first strand and a second strand, wherein the first strand comprises at least 15 contiguous nucleotides from a first strand of: AD-35582.1 (SEQ ID NO: 524), or a modified or unmodified variant thereof.

A RNAi agent comprising a first strand and a second strand, wherein the first strand comprises at least 15 contiguous nucleotides from a first strand of: AD-35594.1 (SEQ ID NO: 525), or a modified or unmodified variant thereof.

A RNAi agent comprising a first strand and a second strand, wherein the first strand comprises at least 15 contiguous nucleotides from a first strand of: AD-35600.1 (SEQ ID NO: 526), or a modified or unmodified variant thereof.

A RNAi agent comprising a first strand and a second strand, wherein the first strand comprises at least 15 contiguous nucleotides from a first strand of: AD-35606.1 (SEQ ID NO: 527), or a modified or unmodified variant thereof.

A RNAi agent comprising a first strand and a second strand, wherein the first strand comprises at least 15 contiguous nucleotides from a first strand of: AD-35612.1 (SEQ ID NO: 528), or a modified or unmodified variant thereof.

A RNAi agent comprising a first strand and a second strand, wherein the first strand comprises at least 15 contiguous nucleotides from a first strand of: AD-38127.1 (SEQ ID NO: 529), or a modified or unmodified variant thereof.

A RNAi agent comprising a first strand and a second strand, wherein the first strand comprises at least 15 contiguous nucleotides from a first strand of: AD-38133.1 (SEQ ID NO: 530), or a modified or unmodified variant thereof.

A RNAi agent comprising a first strand and a second strand, wherein the first strand comprises at least 15 contiguous nucleotides from a first strand of: AD-38139.1 (SEQ ID NO: 531), or a modified or unmodified variant thereof.

A RNAi agent comprising a first strand and a second strand, wherein the first strand comprises at least 15 contiguous nucleotides from a first strand of: AD-38145.1 (SEQ ID NO: 532), or a modified or unmodified variant thereof.

A RNAi agent comprising a first strand and a second strand, wherein the first strand comprises at least 15 contiguous nucleotides from a first strand of: AD-38151.1 (SEQ ID NO: 533), or a modified or unmodified variant thereof.

A RNAi agent comprising a first strand and a second strand, wherein the first strand comprises at least 15 contiguous nucleotides from a first strand of: AD-38157.1 (SEQ ID NO: 534), or a modified or unmodified variant thereof.

A RNAi agent comprising a first strand and a second strand, wherein the first strand comprises at least 15 contiguous nucleotides from a first strand of: AD-38163.1 (SEQ ID NO: 535), or a modified or unmodified variant thereof.

A RNAi agent comprising a first strand and a second strand, wherein the first strand comprises at least 15 contiguous nucleotides from a first strand of: AD-38169.1 (SEQ ID NO: 536), or a modified or unmodified variant thereof.

A RNAi agent comprising a first strand and a second strand, wherein the first strand comprises at least 15 contiguous nucleotides from a first strand of: AD-38128.1 (SEQ ID NO: 537), or a modified or unmodified variant thereof.

A RNAi agent comprising a first strand and a second strand, wherein the first strand comprises at least 15 contiguous nucleotides from a first strand of: AD-38134.1 (SEQ ID NO: 538), or a modified or unmodified variant thereof.

A RNAi agent comprising a first strand and a second strand, wherein the first strand comprises at least 15 contiguous nucleotides from a first strand of: AD-38140.1 (SEQ ID NO: 539), or a modified or unmodified variant thereof.

A RNAi agent comprising a first strand and a second strand, wherein the first strand comprises at least 15 contiguous nucleotides from a first strand of: AD-38146.1 (SEQ ID NO: 540), or a modified or unmodified variant thereof.

A RNAi agent comprising a first strand and a second strand, wherein the first strand comprises at least 15 contiguous nucleotides from a first strand of: AD-38152.1 (SEQ ID NO: 541), or a modified or unmodified variant thereof.

A RNAi agent comprising a first strand and a second strand, wherein the first strand comprises at least 15 contiguous nucleotides from a first strand of: AD-38158.1 (SEQ ID NO: 542), or a modified or unmodified variant thereof.

A RNAi agent comprising a first strand and a second strand, wherein the first strand comprises at least 15 contiguous nucleotides from a first strand of: AD-38164.1 (SEQ ID NO: 543), or a modified or unmodified variant thereof.

A RNAi agent comprising a first strand and a second strand, wherein the first strand comprises at least 15 contiguous nucleotides from a first strand of: AD-38170.1 (SEQ ID NO: 544), or a modified or unmodified variant thereof.

A RNAi agent comprising a first strand and a second strand, wherein the first strand comprises at least 15 contiguous nucleotides from a first strand of: AD-38129.1 (SEQ ID NO: 545), or a modified or unmodified variant thereof.

A RNAi agent comprising a first strand and a second strand, wherein the first strand comprises at least 15 contiguous nucleotides from a first strand of: AD-38135.1 (SEQ ID NO: 546), or a modified or unmodified variant thereof.

A RNAi agent comprising a first strand and a second strand, wherein the first strand comprises at least 15 contiguous nucleotides from a first strand of: AD-38141.1 (SEQ ID NO: 547), or a modified or unmodified variant thereof.

A RNAi agent comprising a first strand and a second strand, wherein the first strand comprises at least 15 contiguous nucleotides from a first strand of: AD-38147.1 (SEQ ID NO: 548), or a modified or unmodified variant thereof.

A RNAi agent comprising a first strand and a second strand, wherein the first strand comprises at least 15 contiguous nucleotides from a first strand of: AD-38153.1 (SEQ ID NO: 549), or a modified or unmodified variant thereof.

A RNAi agent comprising a first strand and a second strand, wherein the first strand comprises at least 15 contiguous nucleotides from a first strand of: AD-38159.1 (SEQ ID NO: 550), or a modified or unmodified variant thereof.

A RNAi agent comprising a first strand and a second strand, wherein the first strand comprises at least 15 contiguous nucleotides from a first strand of: AD-38165.1 (SEQ ID NO: 551), or a modified or unmodified variant thereof.

A RNAi agent comprising a first strand and a second strand, wherein the first strand comprises at least 15 contiguous nucleotides from a first strand of: AD-38171.1 (SEQ ID NO: 552), or a modified or unmodified variant thereof.

A RNAi agent comprising a first strand and a second strand, wherein the first strand comprises at least 15 contiguous nucleotides from a first strand of: AD-38130.1 (SEQ ID NO: 553), or a modified or unmodified variant thereof.

A RNAi agent comprising a first strand and a second strand, wherein the first strand comprises at least 15 contiguous nucleotides from a first strand of: AD-38136.1 (SEQ ID NO: 554), or a modified or unmodified variant thereof.

A RNAi agent comprising a first strand and a second strand, wherein the first strand comprises at least 15 contiguous nucleotides from a first strand of: AD-38142.1 (SEQ ID NO: 555), or a modified or unmodified variant thereof.

A RNAi agent comprising a first strand and a second strand, wherein the first strand comprises at least 15 contiguous nucleotides from a first strand of: AD-38148.1 (SEQ ID NO: 556), or a modified or unmodified variant thereof.

A RNAi agent comprising a first strand and a second strand, wherein the first strand comprises at least 15 contiguous nucleotides from a first strand of: AD-38154.1 (SEQ ID NO: 557), or a modified or unmodified variant thereof.

A RNAi agent comprising a first strand and a second strand, wherein the first strand comprises at least 15 contiguous nucleotides from a first strand of: AD-38160.1 (SEQ ID NO: 558), or a modified or unmodified variant thereof.

A RNAi agent comprising a first strand and a second strand, wherein the first strand comprises at least 15 contiguous nucleotides from a first strand of: AD-38166.1 (SEQ ID NO: 559), or a modified or unmodified variant thereof.

A RNAi agent comprising a first strand and a second strand, wherein the first strand comprises at least 15 contiguous nucleotides from a first strand of: AD-38172.1 (SEQ ID NO: 560), or a modified or unmodified variant thereof.

A RNAi agent comprising a first strand and a second strand, wherein the first strand comprises at least 15 contiguous nucleotides from a first strand of: AD-38131.1 (SEQ ID NO: 561), or a modified or unmodified variant thereof.

A RNAi agent comprising a first strand and a second strand, wherein the first strand comprises at least 15 contiguous nucleotides from a first strand of: AD-38137.1 (SEQ ID NO: 562), or a modified or unmodified variant thereof.

A RNAi agent comprising a first strand and a second strand, wherein the first strand comprises at least 15 contiguous nucleotides from a first strand of: AD-38143.1 (SEQ ID NO: 563), or a modified or unmodified variant thereof.

A RNAi agent comprising a first strand and a second strand, wherein the first strand comprises at least 15 contiguous nucleotides from a first strand of: AD-38149.1 (SEQ ID NO: 564), or a modified or unmodified variant thereof.

A RNAi agent comprising a first strand and a second strand, wherein the first strand comprises at least 15 contiguous nucleotides from a first strand of: AD-38155.1 (SEQ ID NO: 565), or a modified or unmodified variant thereof.

A RNAi agent comprising a first strand and a second strand, wherein the first strand comprises at least 15 contiguous nucleotides from a first strand of: AD-38161.1 (SEQ ID NO: 566), or a modified or unmodified variant thereof.

A RNAi agent comprising a first strand and a second strand, wherein the first strand comprises at least 15 contiguous nucleotides from a first strand of: AD-38167.1 (SEQ ID NO: 567), or a modified or unmodified variant thereof.

A RNAi agent comprising a first strand and a second strand, wherein the first strand comprises at least 15 contiguous nucleotides from a first strand of: AD-38173.1 (SEQ ID NO: 568), or a modified or unmodified variant thereof.

A RNAi agent comprising a first strand and a second strand, wherein the first strand comprises at least 15 contiguous nucleotides from a first strand of: AD-38132.1 (SEQ ID NO: 569), or a modified or unmodified variant thereof.

A RNAi agent comprising a first strand and a second strand, wherein the first strand comprises at least 15 contiguous nucleotides from a first strand of: AD-38138.1 (SEQ ID NO: 570), or a modified or unmodified variant thereof.

A RNAi agent comprising a first strand and a second strand, wherein the first strand comprises at least 15 contiguous nucleotides from a first strand of: AD-38144.1 (SEQ ID NO: 571), or a modified or unmodified variant thereof.

A RNAi agent comprising a first strand and a second strand, wherein the first strand comprises at least 15 contiguous nucleotides from a first strand of: AD-38150.1 (SEQ ID NO: 572), or a modified or unmodified variant thereof.

A RNAi agent comprising a first strand and a second strand, wherein the first strand comprises at least 15 contiguous nucleotides from a first strand of: AD-38156.1 (SEQ ID NO: 573), or a modified or unmodified variant thereof.

A RNAi agent comprising a first strand and a second strand, wherein the first strand comprises at least 15 contiguous nucleotides from a first strand of: AD-38162.1 (SEQ ID NO: 574), or a modified or unmodified variant thereof.

A RNAi agent comprising a first strand and a second strand, wherein the first strand comprises at least 15 contiguous nucleotides from a first strand of: AD-38168.1 (SEQ ID NO: 575), or a modified or unmodified variant thereof.

A RNAi agent comprising a first strand and a second strand, wherein the first strand comprises at least 15 contiguous nucleotides from a first strand of: AD-38174.1 (SEQ ID NO: 576), or a modified or unmodified variant thereof.

A RNAi agent comprising a first strand and a second strand, wherein the first strand comprises at least 15 contiguous nucleotides from a first strand of: AD-39683.1 (SEQ ID NO: 577), or a modified or unmodified variant thereof.

A RNAi agent comprising a first strand and a second strand, wherein the first strand comprises at least 15 contiguous nucleotides from a first strand of: AD-39689.1 (SEQ ID NO: 578), or a modified or unmodified variant thereof.

A RNAi agent comprising a first strand and a second strand, wherein the first strand comprises at least 15 contiguous nucleotides from a first strand of: AD-39695.1 (SEQ ID NO: 579), or a modified or unmodified variant thereof.

A RNAi agent comprising a first strand and a second strand, wherein the first strand comprises at least 15 contiguous nucleotides from a first strand of: AD-39701.1 (SEQ ID NO: 580), or a modified or unmodified variant thereof.

A RNAi agent comprising a first strand and a second strand, wherein the first strand comprises at least 15 contiguous nucleotides from a first strand of: AD-39707.1 (SEQ ID NO: 581), or a modified or unmodified variant thereof.

A RNAi agent comprising a first strand and a second strand, wherein the first strand comprises at least 15 contiguous nucleotides from a first strand of: AD-39713.1 (SEQ ID NO: 582), or a modified or unmodified variant thereof.

A RNAi agent comprising a first strand and a second strand, wherein the first strand comprises at least 15 contiguous nucleotides from a first strand of: AD-39719.1 (SEQ ID NO: 583), or a modified or unmodified variant thereof.

A RNAi agent comprising a first strand and a second strand, wherein the first strand comprises at least 15 contiguous nucleotides from a first strand of: AD-39725.1 (SEQ ID NO: 584), or a modified or unmodified variant thereof.

A RNAi agent comprising a first strand and a second strand, wherein the first strand comprises at least 15 contiguous nucleotides from a first strand of: AD-39684.1 (SEQ ID NO: 585), or a modified or unmodified variant thereof.

A RNAi agent comprising a first strand and a second strand, wherein the first strand comprises at least 15 contiguous nucleotides from a first strand of: AD-39690.1 (SEQ ID NO: 586), or a modified or unmodified variant thereof.

A RNAi agent comprising a first strand and a second strand, wherein the first strand comprises at least 15 contiguous nucleotides from a first strand of: AD-39696.1 (SEQ ID NO: 587), or a modified or unmodified variant thereof.

A RNAi agent comprising a first strand and a second strand, wherein the first strand comprises at least 15 contiguous nucleotides from a first strand of: AD-39702.1 (SEQ ID NO: 588), or a modified or unmodified variant thereof.

A RNAi agent comprising a first strand and a second strand, wherein the first strand comprises at least 15 contiguous nucleotides from a first strand of: AD-39708.1 (SEQ ID NO: 589), or a modified or unmodified variant thereof.

A RNAi agent comprising a first strand and a second strand, wherein the first strand comprises at least 15 contiguous nucleotides from a first strand of: AD-39714.1 (SEQ ID NO: 590), or a modified or unmodified variant thereof.

A RNAi agent comprising a first strand and a second strand, wherein the first strand comprises at least 15 contiguous nucleotides from a first strand of: AD-39720.1 (SEQ ID NO: 591), or a modified or unmodified variant thereof.

A RNAi agent comprising a first strand and a second strand, wherein the first strand comprises at least 15 contiguous nucleotides from a first strand of: AD-39726.1 (SEQ ID NO: 592), or a modified or unmodified variant thereof.

A RNAi agent comprising a first strand and a second strand, wherein the first strand comprises at least 15 contiguous nucleotides from a first strand of: AD-39685.1 (SEQ ID NO: 593), or a modified or unmodified variant thereof.

A RNAi agent comprising a first strand and a second strand, wherein the first strand comprises at least 15 contiguous nucleotides from a first strand of: AD-39691.1 (SEQ ID NO: 594), or a modified or unmodified variant thereof.

A RNAi agent comprising a first strand and a second strand, wherein the first strand comprises at least 15 contiguous nucleotides from a first strand of: AD-39697.1 (SEQ ID NO: 595), or a modified or unmodified variant thereof.

A RNAi agent comprising a first strand and a second strand, wherein the first strand comprises at least 15 contiguous nucleotides from a first strand of: AD-39703.1 (SEQ ID NO: 596), or a modified or unmodified variant thereof.

A RNAi agent comprising a first strand and a second strand, wherein the first strand comprises at least 15 contiguous nucleotides from a first strand of: AD-39709.1 (SEQ ID NO: 597), or a modified or unmodified variant thereof.

A RNAi agent comprising a first strand and a second strand, wherein the first strand comprises at least 15 contiguous nucleotides from a first strand of: AD-39715.1 (SEQ ID NO: 598), or a modified or unmodified variant thereof.

A RNAi agent comprising a first strand and a second strand, wherein the first strand comprises at least 15 contiguous nucleotides from a first strand of: AD-39721.1 (SEQ ID NO: 599), or a modified or unmodified variant thereof.

A RNAi agent comprising a first strand and a second strand, wherein the first strand comprises at least 15 contiguous nucleotides from a first strand of: AD-39727.1 (SEQ ID NO: 600), or a modified or unmodified variant thereof.

A RNAi agent comprising a first strand and a second strand, wherein the first strand comprises at least 15 contiguous nucleotides from a first strand of: AD-39686.1 (SEQ ID NO: 601), or a modified or unmodified variant thereof.

A RNAi agent comprising a first strand and a second strand, wherein the first strand comprises at least 15 contiguous nucleotides from a first strand of: AD-39692.1 (SEQ ID NO: 602), or a modified or unmodified variant thereof.

A RNAi agent comprising a first strand and a second strand, wherein the first strand comprises at least 15 contiguous nucleotides from a first strand of: AD-39698.1 (SEQ ID NO: 603), or a modified or unmodified variant thereof.

A RNAi agent comprising a first strand and a second strand, wherein the first strand comprises at least 15 contiguous nucleotides from a first strand of: AD-39704.1 (SEQ ID NO: 604), or a modified or unmodified variant thereof.

A RNAi agent comprising a first strand and a second strand, wherein the first strand comprises at least 15 contiguous nucleotides from a first strand of: AD-39710.1 (SEQ ID NO: 605), or a modified or unmodified variant thereof.

A RNAi agent comprising a first strand and a second strand, wherein the first strand comprises at least 15 contiguous nucleotides from a first strand of: AD-39716.1 (SEQ ID NO: 606), or a modified or unmodified variant thereof.

A RNAi agent comprising a first strand and a second strand, wherein the first strand comprises at least 15 contiguous nucleotides from a first strand of: AD-39722.1 (SEQ ID NO: 607), or a modified or unmodified variant thereof.

A RNAi agent comprising a first strand and a second strand, wherein the first strand comprises at least 15 contiguous nucleotides from a first strand of: AD-39728.1 (SEQ ID NO: 608), or a modified or unmodified variant thereof.

A RNAi agent comprising a first strand and a second strand, wherein the first strand comprises at least 15 contiguous nucleotides from a first strand of: AD-39687.1 (SEQ ID NO: 609), or a modified or unmodified variant thereof.

A RNAi agent comprising a first strand and a second strand, wherein the first strand comprises at least 15 contiguous nucleotides from a first strand of: AD-39693.1 (SEQ ID NO: 610), or a modified or unmodified variant thereof.

A RNAi agent comprising a first strand and a second strand, wherein the first strand comprises at least 15 contiguous nucleotides from a first strand of: AD-39699.1 (SEQ ID NO: 611), or a modified or unmodified variant thereof.

A RNAi agent comprising a first strand and a second strand, wherein the first strand comprises at least 15 contiguous nucleotides from a first strand of: AD-39705.1 (SEQ ID NO: 612), or a modified or unmodified variant thereof.

A RNAi agent comprising a first strand and a second strand, wherein the first strand comprises at least 15 contiguous nucleotides from a first strand of: AD-39711.1 (SEQ ID NO: 613), or a modified or unmodified variant thereof.

A RNAi agent comprising a first strand and a second strand, wherein the first strand comprises at least 15 contiguous nucleotides from a first strand of: AD-39717.1 (SEQ ID NO: 614), or a modified or unmodified variant thereof.

A RNAi agent comprising a first strand and a second strand, wherein the first strand comprises at least 15 contiguous nucleotides from a first strand of: AD-39723.1 (SEQ ID NO: 615), or a modified or unmodified variant thereof.

A RNAi agent comprising a first strand and a second strand, wherein the first strand comprises at least 15 contiguous nucleotides from a first strand of: AD-39729.1 (SEQ ID NO: 616), or a modified or unmodified variant thereof.

A RNAi agent comprising a first strand and a second strand, wherein the first strand comprises at least 15 contiguous nucleotides from a first strand of: AD-39688.1 (SEQ ID NO: 617), or a modified or unmodified variant thereof.

A RNAi agent comprising a first strand and a second strand, wherein the first strand comprises at least 15 contiguous nucleotides from a first strand of: AD-39694.1 (SEQ ID NO: 618), or a modified or unmodified variant thereof.

A RNAi agent comprising a first strand and a second strand, wherein the first strand comprises at least 15 contiguous nucleotides from a first strand of: AD-39700.1 (SEQ ID NO: 619), or a modified or unmodified variant thereof.

A RNAi agent comprising a first strand and a second strand, wherein the first strand comprises at least 15 contiguous nucleotides from a first strand of: AD-39706.1 (SEQ ID NO: 620), or a modified or unmodified variant thereof.

A RNAi agent comprising a first strand and a second strand, wherein the first strand comprises at least 15 contiguous nucleotides from a first strand of: AD-39712.1 (SEQ ID NO: 621), or a modified or unmodified variant thereof.

A RNAi agent comprising a first strand and a second strand, wherein the first strand comprises at least 15 contiguous nucleotides from a first strand of: AD-39718.1 (SEQ ID NO: 622), or a modified or unmodified variant thereof.

A RNAi agent comprising a first strand and a second strand, wherein the first strand comprises at least 15 contiguous nucleotides from a first strand of: AD-39724.1 (SEQ ID NO: 623), or a modified or unmodified variant thereof.

A RNAi agent comprising a first strand and a second strand, wherein the first strand comprises at least 15 contiguous nucleotides from a first strand of: AD-39730.1 (SEQ ID NO: 624), or a modified or unmodified variant thereof.

A RNAi agent comprising a first strand and a second strand, wherein the first strand comprises at least 15 contiguous nucleotides from a first strand of: AD-39736.1 (SEQ ID NO: 625), or a modified or unmodified variant thereof.

A RNAi agent comprising a first strand and a second strand, wherein the first strand comprises at least 15 contiguous nucleotides from a first strand of: AD-39742.1 (SEQ ID NO: 626), or a modified or unmodified variant thereof.

A RNAi agent comprising a first strand and a second strand, wherein the first strand comprises at least 15 contiguous nucleotides from a first strand of: AD-39748.1 (SEQ ID NO: 627), or a modified or unmodified variant thereof.

A RNAi agent comprising a first strand and a second strand, wherein the first strand comprises at least 15 contiguous nucleotides from a first strand of: AD-39754.1 (SEQ ID NO: 628), or a modified or unmodified variant thereof.

A RNAi agent comprising a first strand and a second strand, wherein the first strand comprises at least 15 contiguous nucleotides from a first strand of: AD-39760.1 (SEQ ID NO: 629), or a modified or unmodified variant thereof.

A RNAi agent comprising a first strand and a second strand, wherein the first strand comprises at least 15 contiguous nucleotides from a first strand of: AD-39766.1 (SEQ ID NO: 630), or a modified or unmodified variant thereof.

A RNAi agent comprising a first strand and a second strand, wherein the first strand comprises at least 15 contiguous nucleotides from a first strand of: AD-39772.1 (SEQ ID NO: 631), or a modified or unmodified variant thereof.

A RNAi agent comprising a first strand and a second strand, wherein the first strand comprises at least 15 contiguous nucleotides from a first strand of: AD-39731.1 (SEQ ID NO: 632), or a modified or unmodified variant thereof.

A RNAi agent comprising a first strand and a second strand, wherein the first strand comprises at least 15 contiguous nucleotides from a first strand of: AD-39737.1 (SEQ ID NO: 633), or a modified or unmodified variant thereof.

A RNAi agent comprising a first strand and a second strand, wherein the first strand comprises at least 15 contiguous nucleotides from a first strand of: AD-39743.1 (SEQ ID NO: 634), or a modified or unmodified variant thereof.

A RNAi agent comprising a first strand and a second strand, wherein the first strand comprises at least 15 contiguous nucleotides from a first strand of: AD-39749.1 (SEQ ID NO: 635), or a modified or unmodified variant thereof.

A RNAi agent comprising a first strand and a second strand, wherein the first strand comprises at least 15 contiguous nucleotides from a first strand of: AD-39755.1 (SEQ ID NO: 636), or a modified or unmodified variant thereof.

A RNAi agent comprising a first strand and a second strand, wherein the first strand comprises at least 15 contiguous nucleotides from a first strand of: AD-39761.1 (SEQ ID NO: 637), or a modified or unmodified variant thereof.

A RNAi agent comprising a first strand and a second strand, wherein the first strand comprises at least 15 contiguous nucleotides from a first strand of: AD-39767.1 (SEQ ID NO: 638), or a modified or unmodified variant thereof.

A RNAi agent comprising a first strand and a second strand, wherein the first strand comprises at least 15 contiguous nucleotides from a first strand of: AD-39773.1 (SEQ ID NO: 639), or a modified or unmodified variant thereof.

A RNAi agent comprising a first strand and a second strand, wherein the first strand comprises at least 15 contiguous nucleotides from a first strand of: AD-39732.1 (SEQ ID NO: 640), or a modified or unmodified variant thereof.

A RNAi agent comprising a first strand and a second strand, wherein the first strand comprises at least 15 contiguous nucleotides from a first strand of: AD-39738.1 (SEQ ID NO: 641), or a modified or unmodified variant thereof.

A RNAi agent comprising a first strand and a second strand, wherein the first strand comprises at least 15 contiguous nucleotides from a first strand of: AD-39744.1 (SEQ ID NO: 642), or a modified or unmodified variant thereof.

A RNAi agent comprising a first strand and a second strand, wherein the first strand comprises at least 15 contiguous nucleotides from a first strand of: AD-39750.1 (SEQ ID NO: 643), or a modified or unmodified variant thereof.

A RNAi agent comprising a first strand and a second strand, wherein the first strand comprises at least 15 contiguous nucleotides from a first strand of: AD-39756.1 (SEQ ID NO: 644), or a modified or unmodified variant thereof.

A RNAi agent comprising a first strand and a second strand, wherein the first strand comprises at least 15 contiguous nucleotides from a first strand of: AD-39762.1 (SEQ ID NO: 645), or a modified or unmodified variant thereof.

A RNAi agent comprising a first strand and a second strand, wherein the first strand comprises at least 15 contiguous nucleotides from a first strand of: AD-39768.1 (SEQ ID NO: 646), or a modified or unmodified variant thereof.

A RNAi agent comprising a first strand and a second strand, wherein the first strand comprises at least 15 contiguous nucleotides from a first strand of: AD-39774.1 (SEQ ID NO: 647), or a modified or unmodified variant thereof.

A RNAi agent comprising a first strand and a second strand, wherein the first strand comprises at least 15 contiguous nucleotides from a first strand of: AD-39733.1 (SEQ ID NO: 648), or a modified or unmodified variant thereof.

A RNAi agent comprising a first strand and a second strand, wherein the first strand comprises at least 15 contiguous nucleotides from a first strand of: AD-39739.1 (SEQ ID NO: 649), or a modified or unmodified variant thereof.

A RNAi agent comprising a first strand and a second strand, wherein the first strand comprises at least 15 contiguous nucleotides from a first strand of: AD-39745.1 (SEQ ID NO: 650), or a modified or unmodified variant thereof.

A RNAi agent comprising a first strand and a second strand, wherein the first strand comprises at least 15 contiguous nucleotides from a first strand of: AD-39751.1 (SEQ ID NO: 651), or a modified or unmodified variant thereof.

A RNAi agent comprising a first strand and a second strand, wherein the first strand comprises at least 15 contiguous nucleotides from a first strand of: AD-39757.1 (SEQ ID NO: 652), or a modified or unmodified variant thereof.

A RNAi agent comprising a first strand and a second strand, wherein the first strand comprises at least 15 contiguous nucleotides from a first strand of: AD-39763.1 (SEQ ID NO: 653), or a modified or unmodified variant thereof.

A RNAi agent comprising a first strand and a second strand, wherein the first strand comprises at least 15 contiguous nucleotides from a first strand of: AD-39769.1 (SEQ ID NO: 654), or a modified or unmodified variant thereof.

A RNAi agent comprising a first strand and a second strand, wherein the first strand comprises at least 15 contiguous nucleotides from a first strand of: AD-39775.1 (SEQ ID NO: 655), or a modified or unmodified variant thereof.

A RNAi agent comprising a first strand and a second strand, wherein the first strand comprises at least 15 contiguous nucleotides from a first strand of: AD-39734.1 (SEQ ID NO: 656), or a modified or unmodified variant thereof.

A RNAi agent comprising a first strand and a second strand, wherein the first strand comprises at least 15 contiguous nucleotides from a first strand of: AD-39740.1 (SEQ ID NO: 657), or a modified or unmodified variant thereof.

A RNAi agent comprising a first strand and a second strand, wherein the first strand comprises at least 15 contiguous nucleotides from a first strand of: AD-39746.1 (SEQ ID NO: 658), or a modified or unmodified variant thereof.

A RNAi agent comprising a first strand and a second strand, wherein the first strand comprises at least 15 contiguous nucleotides from a first strand of: AD-39752.1 (SEQ ID NO: 659), or a modified or unmodified variant thereof.

A RNAi agent comprising a first strand and a second strand, wherein the first strand comprises at least 15 contiguous nucleotides from a first strand of: AD-39758.1 (SEQ ID NO: 660), or a modified or unmodified variant thereof.

A RNAi agent comprising a first strand and a second strand, wherein the first strand comprises at least 15 contiguous nucleotides from a first strand of: AD-39764.1 (SEQ ID NO: 661), or a modified or unmodified variant thereof.

A RNAi agent comprising a first strand and a second strand, wherein the first strand comprises at least 15 contiguous nucleotides from a first strand of: AD-39770.1 (SEQ ID NO: 662), or a modified or unmodified variant thereof.

A RNAi agent comprising a first strand and a second strand, wherein the first strand comprises at least 15 contiguous nucleotides from a first strand of: AD-39776.1 (SEQ ID NO: 663), or a modified or unmodified variant thereof.

A RNAi agent comprising a first strand and a second strand, wherein the first strand comprises at least 15 contiguous nucleotides from a first strand of: AD-39735.1 (SEQ ID NO: 664), or a modified or unmodified variant thereof.

A RNAi agent comprising a first strand and a second strand, wherein the first strand comprises at least 15 contiguous nucleotides from a first strand of: AD-39741.1 (SEQ ID NO: 665), or a modified or unmodified variant thereof.

A RNAi agent comprising a first strand and a second strand, wherein the first strand comprises at least 15 contiguous nucleotides from a first strand of: AD-39747.1 (SEQ ID NO: 666), or a modified or unmodified variant thereof.

A RNAi agent comprising a first strand and a second strand, wherein the first strand comprises at least 15 contiguous nucleotides from a first strand of: AD-39753.1 (SEQ ID NO: 667), or a modified or unmodified variant thereof.

A RNAi agent comprising a first strand and a second strand, wherein the first strand comprises at least 15 contiguous nucleotides from a first strand of: AD-39759.1 (SEQ ID NO: 668), or a modified or unmodified variant thereof.

A RNAi agent comprising a first strand and a second strand, wherein the first strand comprises at least 15 contiguous nucleotides from a first strand of: AD-39765.1 (SEQ ID NO: 669), or a modified or unmodified variant thereof.

A RNAi agent comprising a first strand and a second strand, wherein the first strand comprises at least 15 contiguous nucleotides from a first strand of: AD-39771.1 (SEQ ID NO: 670), or a modified or unmodified variant thereof.

A RNAi agent comprising a first strand and a second strand, wherein the first strand comprises at least 15 contiguous nucleotides from a first strand of: AD-39778.1 (SEQ ID NO: 671), or a modified or unmodified variant thereof.

A RNAi agent comprising a first strand and a second strand, wherein the first strand comprises at least 15 contiguous nucleotides from a first strand of: AD-39784.1 (SEQ ID NO: 672), or a modified or unmodified variant thereof.

A RNAi agent comprising a first strand and a second strand, wherein the first strand comprises at least 15 contiguous nucleotides from a first strand of: AD-39790.1 (SEQ ID NO: 673), or a modified or unmodified variant thereof.

A RNAi agent comprising a first strand and a second strand, wherein the first strand comprises at least 15 contiguous nucleotides from a first strand of: AD-39796.1 (SEQ ID NO: 674), or a modified or unmodified variant thereof.

A RNAi agent comprising a first strand and a second strand, wherein the first strand comprises at least 15 contiguous nucleotides from a first strand of: AD-39802.1 (SEQ ID NO: 675), or a modified or unmodified variant thereof.

A RNAi agent comprising a first strand and a second strand, wherein the first strand comprises at least 15 contiguous nucleotides from a first strand of: AD-39808.1 (SEQ ID NO: 676), or a modified or unmodified variant thereof.

A RNAi agent comprising a first strand and a second strand, wherein the first strand comprises at least 15 contiguous nucleotides from a first strand of: AD-39814.1 (SEQ ID NO: 677), or a modified or unmodified variant thereof.

A RNAi agent comprising a first strand and a second strand, wherein the first strand comprises at least 15 contiguous nucleotides from a first strand of: AD-39820.1 (SEQ ID NO: 678), or a modified or unmodified variant thereof.

A RNAi agent comprising a first strand and a second strand, wherein the first strand comprises at least 15 contiguous nucleotides from a first strand of: AD-39779.1 (SEQ ID NO: 679), or a modified or unmodified variant thereof.

A RNAi agent comprising a first strand and a second strand, wherein the first strand comprises at least 15 contiguous nucleotides from a first strand of: AD-39785.1 (SEQ ID NO: 680), or a modified or unmodified variant thereof.

A RNAi agent comprising a first strand and a second strand, wherein the first strand comprises at least 15 contiguous nucleotides from a first strand of: AD-39791.1 (SEQ ID NO: 681), or a modified or unmodified variant thereof.

A RNAi agent comprising a first strand and a second strand, wherein the first strand comprises at least 15 contiguous nucleotides from a first strand of: AD-39797.1 (SEQ ID NO: 682), or a modified or unmodified variant thereof.

A RNAi agent comprising a first strand and a second strand, wherein the first strand comprises at least 15 contiguous nucleotides from a first strand of: AD-39803.1 (SEQ ID NO: 683), or a modified or unmodified variant thereof.

A RNAi agent comprising a first strand and a second strand, wherein the first strand comprises at least 15 contiguous nucleotides from a first strand of: AD-39809.1 (SEQ ID NO: 684), or a modified or unmodified variant thereof.

A RNAi agent comprising a first strand and a second strand, wherein the first strand comprises at least 15 contiguous nucleotides from a first strand of: AD-39815.1 (SEQ ID NO: 685), or a modified or unmodified variant thereof.

A RNAi agent comprising a first strand and a second strand, wherein the first strand comprises at least 15 contiguous nucleotides from a first strand of: AD-39821.1 (SEQ ID NO: 686), or a modified or unmodified variant thereof.

A RNAi agent comprising a first strand and a second strand, wherein the first strand comprises at least 15 contiguous nucleotides from a first strand of: AD-39780.1 (SEQ ID NO: 687), or a modified or unmodified variant thereof.

A RNAi agent comprising a first strand and a second strand, wherein the first strand comprises at least 15 contiguous nucleotides from a first strand of: AD-39786.1 (SEQ ID NO: 688), or a modified or unmodified variant thereof.

A RNAi agent comprising a first strand and a second strand, wherein the first strand comprises at least 15 contiguous nucleotides from a first strand of: AD-39792.1 (SEQ ID NO: 689), or a modified or unmodified variant thereof.

A RNAi agent comprising a first strand and a second strand, wherein the first strand comprises at least 15 contiguous nucleotides from a first strand of: AD-39798.1 (SEQ ID NO: 690), or a modified or unmodified variant thereof.

A RNAi agent comprising a first strand and a second strand, wherein the first strand comprises at least 15 contiguous nucleotides from a first strand of: AD-39804.1 (SEQ ID NO: 691), or a modified or unmodified variant thereof.

A RNAi agent comprising a first strand and a second strand, wherein the first strand comprises at least 15 contiguous nucleotides from a first strand of: AD-39810.1 (SEQ ID NO: 692), or a modified or unmodified variant thereof.

A RNAi agent comprising a first strand and a second strand, wherein the first strand comprises at least 15 contiguous nucleotides from a first strand of: AD-39816.1 (SEQ ID NO: 693), or a modified or unmodified variant thereof.

A RNAi agent comprising a first strand and a second strand, wherein the first strand comprises at least 15 contiguous nucleotides from a first strand of: AD-39822.1 (SEQ ID NO: 694), or a modified or unmodified variant thereof.

A RNAi agent comprising a first strand and a second strand, wherein the first strand comprises at least 15 contiguous nucleotides from a first strand of: AD-39781.1 (SEQ ID NO: 695), or a modified or unmodified variant thereof.

A RNAi agent comprising a first strand and a second strand, wherein the first strand comprises at least 15 contiguous nucleotides from a first strand of: AD-39787.1 (SEQ ID NO: 696), or a modified or unmodified variant thereof.

A RNAi agent comprising a first strand and a second strand, wherein the first strand comprises at least 15 contiguous nucleotides from a first strand of: AD-39793.1 (SEQ ID NO: 697), or a modified or unmodified variant thereof.

A RNAi agent comprising a first strand and a second strand, wherein the first strand comprises at least 15 contiguous nucleotides from a first strand of: AD-39799.1 (SEQ ID NO: 698), or a modified or unmodified variant thereof.

A RNAi agent comprising a first strand and a second strand, wherein the first strand comprises at least 15 contiguous nucleotides from a first strand of: AD-39805.1 (SEQ ID NO: 699), or a modified or unmodified variant thereof.

A RNAi agent comprising a first strand and a second strand, wherein the first strand comprises at least 15 contiguous nucleotides from a first strand of: AD-39811.1 (SEQ ID NO: 700), or a modified or unmodified variant thereof.

A RNAi agent comprising a first strand and a second strand, wherein the first strand comprises at least 15 contiguous nucleotides from a first strand of: AD-39817.1 (SEQ ID NO: 701), or a modified or unmodified variant thereof.

A RNAi agent comprising a first strand and a second strand, wherein the first strand comprises at least 15 contiguous nucleotides from a first strand of: AD-39823.1 (SEQ ID NO: 702), or a modified or unmodified variant thereof.

A RNAi agent comprising a first strand and a second strand, wherein the first strand comprises at least 15 contiguous nucleotides from a first strand of: AD-39782.1 (SEQ ID NO: 703), or a modified or unmodified variant thereof.

A RNAi agent comprising a first strand and a second strand, wherein the first strand comprises at least 15 contiguous nucleotides from a first strand of: AD-39788.1 (SEQ ID NO: 704), or a modified or unmodified variant thereof.

A RNAi agent comprising a first strand and a second strand, wherein the first strand comprises at least 15 contiguous nucleotides from a first strand of: AD-39794.1 (SEQ ID NO: 705), or a modified or unmodified variant thereof.

A RNAi agent comprising a first strand and a second strand, wherein the first strand comprises at least 15 contiguous nucleotides from a first strand of: AD-39800.1 (SEQ ID NO: 706), or a modified or unmodified variant thereof.

A RNAi agent comprising a first strand and a second strand, wherein the first strand comprises at least 15 contiguous nucleotides from a first strand of: AD-39806.1 (SEQ ID NO: 707), or a modified or unmodified variant thereof.

A RNAi agent comprising a first strand and a second strand, wherein the first strand comprises at least 15 contiguous nucleotides from a first strand of: AD-39812.1 (SEQ ID NO: 708), or a modified or unmodified variant thereof.

A RNAi agent comprising a first strand and a second strand, wherein the first strand comprises at least 15 contiguous nucleotides from a first strand of: AD-39818.1 (SEQ ID NO: 709), or a modified or unmodified variant thereof.

A RNAi agent comprising a first strand and a second strand, wherein the first strand comprises at least 15 contiguous nucleotides from a first strand of: AD-39824.1 (SEQ ID NO: 710), or a modified or unmodified variant thereof.

A RNAi agent comprising a first strand and a second strand, wherein the first strand comprises at least 15 contiguous nucleotides from a first strand of: AD-39783.1 (SEQ ID NO: 711), or a modified or unmodified variant thereof.

A RNAi agent comprising a first strand and a second strand, wherein the first strand comprises at least 15 contiguous nucleotides from a first strand of: AD-39789.1 (SEQ ID NO: 712), or a modified or unmodified variant thereof.

A RNAi agent comprising a first strand and a second strand, wherein the first strand comprises at least 15 contiguous nucleotides from a first strand of: AD-39795.1 (SEQ ID NO: 713), or a modified or unmodified variant thereof.

A RNAi agent comprising a first strand and a second strand, wherein the first strand comprises at least 15 contiguous nucleotides from a first strand of: AD-39801.1 (SEQ ID NO: 714), or a modified or unmodified variant thereof.

A RNAi agent comprising a first strand and a second strand, wherein the first strand comprises at least 15 contiguous nucleotides from a first strand of: AD-39807.1 (SEQ ID NO: 715), or a modified or unmodified variant thereof.

A RNAi agent comprising a first strand and a second strand, wherein the first strand comprises at least 15 contiguous nucleotides from a first strand of: AD-39813.1 (SEQ ID NO: 716), or a modified or unmodified variant thereof.

A RNAi agent comprising a first strand and a second strand, wherein the first strand comprises at least 15 contiguous nucleotides from a first strand of: AD-39819.1 (SEQ ID NO: 717), or a modified or unmodified variant thereof.

A RNAi agent comprising a first strand and a second strand, wherein the first strand comprises at least 15 contiguous nucleotides from a first strand of: AD-39825.1 (SEQ ID NO: 718), or a modified or unmodified variant thereof.

A RNAi agent comprising a first strand and a second strand, wherein the first strand comprises at least 15 contiguous nucleotides from a first strand of: AD-39831.1 (SEQ ID NO: 719), or a modified or unmodified variant thereof.

A RNAi agent comprising a first strand and a second strand, wherein the first strand comprises at least 15 contiguous nucleotides from a first strand of: AD-39837.1 (SEQ ID NO: 720), or a modified or unmodified variant thereof.

A RNAi agent comprising a first strand and a second strand, wherein the first strand comprises at least 15 contiguous nucleotides from a first strand of: AD-39843.1 (SEQ ID NO: 721), or a modified or unmodified variant thereof.

A RNAi agent comprising a first strand and a second strand, wherein the first strand comprises at least 15 contiguous nucleotides from a first strand of: AD-39849.1 (SEQ ID NO: 722), or a modified or unmodified variant thereof.

A RNAi agent comprising a first strand and a second strand, wherein the first strand comprises at least 15 contiguous nucleotides from a first strand of: AD-39855.1 (SEQ ID NO: 723), or a modified or unmodified variant thereof.

A RNAi agent comprising a first strand and a second strand, wherein the first strand comprises at least 15 contiguous nucleotides from a first strand of: AD-39861.1 (SEQ ID NO: 724), or a modified or unmodified variant thereof.

A RNAi agent comprising a first strand and a second strand, wherein the first strand comprises at least 15 contiguous nucleotides from a first strand of: AD-39867.1 (SEQ ID NO: 725), or a modified or unmodified variant thereof.

A RNAi agent comprising a first strand and a second strand, wherein the first strand comprises at least 15 contiguous nucleotides from a first strand of: AD-39826.1 (SEQ ID NO: 726), or a modified or unmodified variant thereof.

A RNAi agent comprising a first strand and a second strand, wherein the first strand comprises at least 15 contiguous nucleotides from a first strand of: AD-39832.1 (SEQ ID NO: 727), or a modified or unmodified variant thereof.

A RNAi agent comprising a first strand and a second strand, wherein the first strand comprises at least 15 contiguous nucleotides from a first strand of: AD-39838.1 (SEQ ID NO: 728), or a modified or unmodified variant thereof.

A RNAi agent comprising a first strand and a second strand, wherein the first strand comprises at least 15 contiguous nucleotides from a first strand of: AD-39844.1 (SEQ ID NO: 729), or a modified or unmodified variant thereof.

A RNAi agent comprising a first strand and a second strand, wherein the first strand comprises at least 15 contiguous nucleotides from a first strand of: AD-39850.1 (SEQ ID NO: 730), or a modified or unmodified variant thereof.

A RNAi agent comprising a first strand and a second strand, wherein the first strand comprises at least 15 contiguous nucleotides from a first strand of: AD-39856.1 (SEQ ID NO: 731), or a modified or unmodified variant thereof.

A RNAi agent comprising a first strand and a second strand, wherein the first strand comprises at least 15 contiguous nucleotides from a first strand of: AD-39862.1 (SEQ ID NO: 732), or a modified or unmodified variant thereof.

A RNAi agent comprising a first strand and a second strand, wherein the first strand comprises at least 15 contiguous nucleotides from a first strand of: AD-39868.1 (SEQ ID NO: 733), or a modified or unmodified variant thereof.

A RNAi agent comprising a first strand and a second strand, wherein the first strand comprises at least 15 contiguous nucleotides from a first strand of: AD-39827.1 (SEQ ID NO: 734), or a modified or unmodified variant thereof.

A RNAi agent comprising a first strand and a second strand, wherein the first strand comprises at least 15 contiguous nucleotides from a first strand of: AD-39833.1 (SEQ ID NO: 735), or a modified or unmodified variant thereof.

A RNAi agent comprising a first strand and a second strand, wherein the first strand comprises at least 15 contiguous nucleotides from a first strand of: AD-39839.1 (SEQ ID NO: 736), or a modified or unmodified variant thereof.

A RNAi agent comprising a first strand and a second strand, wherein the first strand comprises at least 15 contiguous nucleotides from a first strand of: AD-39845.1 (SEQ ID NO: 737), or a modified or unmodified variant thereof.

A RNAi agent comprising a first strand and a second strand, wherein the first strand comprises at least 15 contiguous nucleotides from a first strand of: AD-39851.1 (SEQ ID NO: 738), or a modified or unmodified variant thereof.

A RNAi agent comprising a first strand and a second strand, wherein the first strand comprises at least 15 contiguous nucleotides from a first strand of: AD-39857.1 (SEQ ID NO: 739), or a modified or unmodified variant thereof.

A RNAi agent comprising a first strand and a second strand, wherein the first strand comprises at least 15 contiguous nucleotides from a first strand of: AD-39863.1 (SEQ ID NO: 740), or a modified or unmodified variant thereof.

A RNAi agent comprising a first strand and a second strand, wherein the first strand comprises at least 15 contiguous nucleotides from a first strand of: AD-39869.1 (SEQ ID NO: 741), or a modified or unmodified variant thereof.

A RNAi agent comprising a first strand and a second strand, wherein the first strand comprises at least 15 contiguous nucleotides from a first strand of: AD-39828.1 (SEQ ID NO: 742), or a modified or unmodified variant thereof.

A RNAi agent comprising a first strand and a second strand, wherein the first strand comprises at least 15 contiguous nucleotides from a first strand of: AD-39834.1 (SEQ ID NO: 743), or a modified or unmodified variant thereof.

A RNAi agent comprising a first strand and a second strand, wherein the first strand comprises at least 15 contiguous nucleotides from a first strand of: AD-39840.1 (SEQ ID NO: 744), or a modified or unmodified variant thereof.

A RNAi agent comprising a first strand and a second strand, wherein the first strand comprises at least 15 contiguous nucleotides from a first strand of: AD-39846.1 (SEQ ID NO: 745), or a modified or unmodified variant thereof.

A RNAi agent comprising a first strand and a second strand, wherein the first strand comprises at least 15 contiguous nucleotides from a first strand of: AD-39852.1 (SEQ ID NO: 746), or a modified or unmodified variant thereof.

A RNAi agent comprising a first strand and a second strand, wherein the first strand comprises at least 15 contiguous nucleotides from a first strand of: AD-39858.1 (SEQ ID NO: 747), or a modified or unmodified variant thereof.

A RNAi agent comprising a first strand and a second strand, wherein the first strand comprises at least 15 contiguous nucleotides from a first strand of: AD-39864.1 (SEQ ID NO: 748), or a modified or unmodified variant thereof.

A RNAi agent comprising a first strand and a second strand, wherein the first strand comprises at least 15 contiguous nucleotides from a first strand of: AD-39870.1 (SEQ ID NO: 749), or a modified or unmodified variant thereof.

A RNAi agent comprising a first strand and a second strand, wherein the first strand comprises at least 15 contiguous nucleotides from a first strand of: AD-39829.1 (SEQ ID NO: 750), or a modified or unmodified variant thereof.

A RNAi agent comprising a first strand and a second strand, wherein the first strand comprises at least 15 contiguous nucleotides from a first strand of: AD-39835.1 (SEQ ID NO: 751), or a modified or unmodified variant thereof.

A RNAi agent comprising a first strand and a second strand, wherein the first strand comprises at least 15 contiguous nucleotides from a first strand of: AD-39841.1 (SEQ ID NO: 752), or a modified or unmodified variant thereof.

A RNAi agent comprising a first strand and a second strand, wherein the first strand comprises at least 15 contiguous nucleotides from a first strand of: AD-39853.1 (SEQ ID NO: 753), or a modified or unmodified variant thereof.

A RNAi agent comprising a first strand and a second strand, wherein the first strand comprises at least 15 contiguous nucleotides from a first strand of: AD-39859.1 (SEQ ID NO: 754), or a modified or unmodified variant thereof.

A RNAi agent comprising a first strand and a second strand, wherein the first strand comprises at least 15 contiguous nucleotides from a first strand of: AD-39865.1 (SEQ ID NO: 755), or a modified or unmodified variant thereof.

A RNAi agent comprising a first strand and a second strand, wherein the first strand comprises at least 15 contiguous nucleotides from a first strand of: AD-39871.1 (SEQ ID NO: 756), or a modified or unmodified variant thereof.

A RNAi agent comprising a first strand and a second strand, wherein the first strand comprises at least 15 contiguous nucleotides from a first strand of: AD-39830.1 (SEQ ID NO: 757), or a modified or unmodified variant thereof.

A RNAi agent comprising a first strand and a second strand, wherein the first strand comprises at least 15 contiguous nucleotides from a first strand of: AD-39836.1 (SEQ ID NO: 758), or a modified or unmodified variant thereof.

A RNAi agent comprising a first strand and a second strand, wherein the first strand comprises at least 15 contiguous nucleotides from a first strand of: AD-39842.1 (SEQ ID NO: 759), or a modified or unmodified variant thereof.

A RNAi agent comprising a first strand and a second strand, wherein the first strand comprises at least 15 contiguous nucleotides from a first strand of: AD-39848.1 (SEQ ID NO: 760), or a modified or unmodified variant thereof.

A RNAi agent comprising a first strand and a second strand, wherein the first strand comprises at least 15 contiguous nucleotides from a first strand of: AD-39854.1 (SEQ ID NO: 761), or a modified or unmodified variant thereof.

A RNAi agent comprising a first strand and a second strand, wherein the first strand comprises at least 15 contiguous nucleotides from a first strand of: AD-39860.1 (SEQ ID NO: 762), or a modified or unmodified variant thereof.

A RNAi agent comprising a first strand and a second strand, wherein the first strand comprises at least 15 contiguous nucleotides from a first strand of: AD-39866.1 (SEQ ID NO: 763), or a modified or unmodified variant thereof.

A RNAi agent comprising a first strand and a second strand, wherein the first strand comprises at least 15 contiguous nucleotides from a first strand of: AD-39992.1 (SEQ ID NO: 764), or a modified or unmodified variant thereof.

A RNAi agent comprising a first strand and a second strand, wherein the first strand comprises at least 15 contiguous nucleotides from a first strand of: AD-39998.1 (SEQ ID NO: 765), or a modified or unmodified variant thereof.

A RNAi agent comprising a first strand and a second strand, wherein the first strand comprises at least 15 contiguous nucleotides from a first strand of: AD-40004.1 (SEQ ID NO: 766), or a modified or unmodified variant thereof.

A RNAi agent comprising a first strand and a second strand, wherein the first strand comprises at least 15 contiguous nucleotides from a first strand of: AD-40010.1 (SEQ ID NO: 767), or a modified or unmodified variant thereof.

A RNAi agent comprising a first strand and a second strand, wherein the first strand comprises at least 15 contiguous nucleotides from a first strand of: AD-40016.1 (SEQ ID NO: 768), or a modified or unmodified variant thereof.

A RNAi agent comprising a first strand and a second strand, wherein the first strand comprises at least 15 contiguous nucleotides from a first strand of: AD-40022.1 (SEQ ID NO: 769), or a modified or unmodified variant thereof.

A RNAi agent comprising a first strand and a second strand, wherein the first strand comprises at least 15 contiguous nucleotides from a first strand of: AD-40028.1 (SEQ ID NO: 770), or a modified or unmodified variant thereof.

A RNAi agent comprising a first strand and a second strand, wherein the first strand comprises at least 15 contiguous nucleotides from a first strand of: AD-40034.1 (SEQ ID NO: 771), or a modified or unmodified variant thereof.

A RNAi agent comprising a first strand and a second strand, wherein the first strand comprises at least 15 contiguous nucleotides from a first strand of: AD-39999.1 (SEQ ID NO: 772), or a modified or unmodified variant thereof.

A RNAi agent comprising a first strand and a second strand, wherein the first strand comprises at least 15 contiguous nucleotides from a first strand of: AD-40005.1 (SEQ ID NO: 773), or a modified or unmodified variant thereof.

A RNAi agent comprising a first strand and a second strand, wherein the first strand comprises at least 15 contiguous nucleotides from a first strand of: AD-40011.1 (SEQ ID NO: 774), or a modified or unmodified variant thereof.

A RNAi agent comprising a first strand and a second strand, wherein the first strand comprises at least 15 contiguous nucleotides from a first strand of: AD-40017.1 (SEQ ID NO: 775), or a modified or unmodified variant thereof.

A RNAi agent comprising a first strand and a second strand, wherein the first strand comprises at least 15 contiguous nucleotides from a first strand of: AD-40029.1 (SEQ ID NO: 776), or a modified or unmodified variant thereof.

A RNAi agent comprising a first strand and a second strand, wherein the first strand comprises at least 15 contiguous nucleotides from a first strand of: AD-40035.1 (SEQ ID NO: 777), or a modified or unmodified variant thereof.

A RNAi agent comprising a first strand and a second strand, wherein the first strand comprises at least 15 contiguous nucleotides from a first strand of: AD-39994.1 (SEQ ID NO: 778), or a modified or unmodified variant thereof.

A RNAi agent comprising a first strand and a second strand, wherein the first strand comprises at least 15 contiguous nucleotides from a first strand of: AD-40000.1 (SEQ ID NO: 779), or a modified or unmodified variant thereof.

A RNAi agent comprising a first strand and a second strand, wherein the first strand comprises at least 15 contiguous nucleotides from a first strand of: AD-40006.1 (SEQ ID NO: 780), or a modified or unmodified variant thereof.

A RNAi agent comprising a first strand and a second strand, wherein the first strand comprises at least 15 contiguous nucleotides from a first strand of: AD-40012.1 (SEQ ID NO: 781), or a modified or unmodified variant thereof.

A RNAi agent comprising a first strand and a second strand, wherein the first strand comprises at least 15 contiguous nucleotides from a first strand of: AD-40018.1 (SEQ ID NO: 782), or a modified or unmodified variant thereof.

A RNAi agent comprising a first strand and a second strand, wherein the first strand comprises at least 15 contiguous nucleotides from a first strand of: AD-40024.1 (SEQ ID NO: 783), or a modified or unmodified variant thereof.

A RNAi agent comprising a first strand and a second strand, wherein the first strand comprises at least 15 contiguous nucleotides from a first strand of: AD-40030.1 (SEQ ID NO: 784), or a modified or unmodified variant thereof.

A RNAi agent comprising a first strand and a second strand, wherein the first strand comprises at least 15 contiguous nucleotides from a first strand of: AD-40036.1 (SEQ ID NO: 785), or a modified or unmodified variant thereof.

A RNAi agent comprising a first strand and a second strand, wherein the first strand comprises at least 15 contiguous nucleotides from a first strand of: AD-39995.1 (SEQ ID NO: 786), or a modified or unmodified variant thereof.

A RNAi agent comprising a first strand and a second strand, wherein the first strand comprises at least 15 contiguous nucleotides from a first strand of: AD-40001.1 (SEQ ID NO: 787), or a modified or unmodified variant thereof.

A RNAi agent comprising a first strand and a second strand, wherein the first strand comprises at least 15 contiguous nucleotides from a first strand of: AD-40007.1 (SEQ ID NO: 788), or a modified or unmodified variant thereof.

A RNAi agent comprising a first strand and a second strand, wherein the first strand comprises at least 15 contiguous nucleotides from a first strand of: AD-40013.1 (SEQ ID NO: 789), or a modified or unmodified variant thereof.

A RNAi agent comprising a first strand and a second strand, wherein the first strand comprises at least 15 contiguous nucleotides from a first strand of: AD-40019.1 (SEQ ID NO: 790), or a modified or unmodified variant thereof.

A RNAi agent comprising a first strand and a second strand, wherein the first strand comprises at least 15 contiguous nucleotides from a first strand of: AD-40025.1 (SEQ ID NO: 791), or a modified or unmodified variant thereof.

A RNAi agent comprising a first strand and a second strand, wherein the first strand comprises at least 15 contiguous nucleotides from a first strand of: AD-40031.1 (SEQ ID NO: 792), or a modified or unmodified variant thereof.

A RNAi agent comprising a first strand and a second strand, wherein the first strand comprises at least 15 contiguous nucleotides from a first strand of: AD-40037.1 (SEQ ID NO: 793), or a modified or unmodified variant thereof.

A RNAi agent comprising a first strand and a second strand, wherein the first strand comprises at least 15 contiguous nucleotides from a first strand of: AD-39996.1 (SEQ ID NO: 794), or a modified or unmodified variant thereof.

A RNAi agent comprising a first strand and a second strand, wherein the first strand comprises at least 15 contiguous nucleotides from a first strand of: AD-40002.1 (SEQ ID NO: 795), or a modified or unmodified variant thereof.

A RNAi agent comprising a first strand and a second strand, wherein the first strand comprises at least 15 contiguous nucleotides from a first strand of: AD-40008.1 (SEQ ID NO: 796), or a modified or unmodified variant thereof.

A RNAi agent comprising a first strand and a second strand, wherein the first strand comprises at least 15 contiguous nucleotides from a first strand of: AD-40014.1 (SEQ ID NO: 797), or a modified or unmodified variant thereof.

A RNAi agent comprising a first strand and a second strand, wherein the first strand comprises at least 15 contiguous nucleotides from a first strand of: AD-40020.1 (SEQ ID NO: 798), or a modified or unmodified variant thereof.

A RNAi agent comprising a first strand and a second strand, wherein the first strand comprises at least 15 contiguous nucleotides from a first strand of: AD-40026.1 (SEQ ID NO: 799), or a modified or unmodified variant thereof.

A RNAi agent comprising a first strand and a second strand, wherein the first strand comprises at least 15 contiguous nucleotides from a first strand of: AD-40032.1 (SEQ ID NO: 800), or a modified or unmodified variant thereof.

A RNAi agent comprising a first strand and a second strand, wherein the first strand comprises at least 15 contiguous nucleotides from a first strand of: AD-40038.1 (SEQ ID NO: 801), or a modified or unmodified variant thereof.

A RNAi agent comprising a first strand and a second strand, wherein the first strand comprises at least 15 contiguous nucleotides from a first strand of: AD-39997.1 (SEQ ID NO: 802), or a modified or unmodified variant thereof.

A RNAi agent comprising a first strand and a second strand, wherein the first strand comprises at least 15 contiguous nucleotides from a first strand of: AD-40009.1 (SEQ ID NO: 803), or a modified or unmodified variant thereof.

A RNAi agent comprising a first strand and a second strand, wherein the first strand comprises at least 15 contiguous nucleotides from a first strand of: AD-40015.1 (SEQ ID NO: 804), or a modified or unmodified variant thereof.

A RNAi agent comprising a first strand and a second strand, wherein the first strand comprises at least 15 contiguous nucleotides from a first strand of: AD-40021.1 (SEQ ID NO: 805), or a modified or unmodified variant thereof.

A RNAi agent comprising a first strand and a second strand, wherein the first strand comprises at least 15 contiguous nucleotides from a first strand of: AD-40027.1 (SEQ ID NO: 806), or a modified or unmodified variant thereof.

A RNAi agent comprising a first strand and a second strand, wherein the first strand comprises at least 15 contiguous nucleotides from a first strand of: AD-40033.1 (SEQ ID NO: 807), or a modified or unmodified variant thereof.

A RNAi agent comprising a first strand and a second strand, wherein the first strand comprises at least 15 contiguous nucleotides from a first strand of: AD-40039.1 (SEQ ID NO: 808), or a modified or unmodified variant thereof.

A RNAi agent comprising a first strand and a second strand, wherein the first strand comprises at least 15 contiguous nucleotides from a first strand of: AD-40045.1 (SEQ ID NO: 809), or a modified or unmodified variant thereof.

A RNAi agent comprising a first strand and a second strand, wherein the first strand comprises at least 15 contiguous nucleotides from a first strand of: AD-40051.1 (SEQ ID NO: 810), or a modified or unmodified variant thereof.

A RNAi agent comprising a first strand and a second strand, wherein the first strand comprises at least 15 contiguous nucleotides from a first strand of: AD-40057.1 (SEQ ID NO: 811), or a modified or unmodified variant thereof.

A RNAi agent comprising a first strand and a second strand, wherein the first strand comprises at least 15 contiguous nucleotides from a first strand of: AD-40063.1 (SEQ ID NO: 812), or a modified or unmodified variant thereof.

A RNAi agent comprising a first strand and a second strand, wherein the first strand comprises at least 15 contiguous nucleotides from a first strand of: AD-40069.1 (SEQ ID NO: 813), or a modified or unmodified variant thereof.

A RNAi agent comprising a first strand and a second strand, wherein the first strand comprises at least 15 contiguous nucleotides from a first strand of: AD-40075.1 (SEQ ID NO: 814), or a modified or unmodified variant thereof.

A RNAi agent comprising a first strand and a second strand, wherein the first strand comprises at least 15 contiguous nucleotides from a first strand of: AD-40081.1 (SEQ ID NO: 815), or a modified or unmodified variant thereof.

A RNAi agent comprising a first strand and a second strand, wherein the first strand comprises at least 15 contiguous nucleotides from a first strand of: AD-40040.1 (SEQ ID NO: 816), or a modified or unmodified variant thereof.

A RNAi agent comprising a first strand and a second strand, wherein the first strand comprises at least 15 contiguous nucleotides from a first strand of: AD-40046.1 (SEQ ID NO: 817), or a modified or unmodified variant thereof.

A RNAi agent comprising a first strand and a second strand, wherein the first strand comprises at least 15 contiguous nucleotides from a first strand of: AD-40052.1 (SEQ ID NO: 818), or a modified or unmodified variant thereof.

A RNAi agent comprising a first strand and a second strand, wherein the first strand comprises at least 15 contiguous nucleotides from a first strand of: AD-40058.1 (SEQ ID NO: 819), or a modified or unmodified variant thereof.

A RNAi agent comprising a first strand and a second strand, wherein the first strand comprises at least 15 contiguous nucleotides from a first strand of: AD-40064.1 (SEQ ID NO: 820), or a modified or unmodified variant thereof.

A RNAi agent comprising a first strand and a second strand, wherein the first strand comprises at least 15 contiguous nucleotides from a first strand of: AD-40070.1 (SEQ ID NO: 821), or a modified or unmodified variant thereof.

A RNAi agent comprising a first strand and a second strand, wherein the first strand comprises at least 15 contiguous nucleotides from a first strand of: AD-40076.1 (SEQ ID NO: 822), or a modified or unmodified variant thereof.

A RNAi agent comprising a first strand and a second strand, wherein the first strand comprises at least 15 contiguous nucleotides from a first strand of: AD-40082.1 (SEQ ID NO: 823), or a modified or unmodified variant thereof.

A RNAi agent comprising a first strand and a second strand, wherein the first strand comprises at least 15 contiguous nucleotides from a first strand of: AD-40041.1 (SEQ ID NO: 824), or a modified or unmodified variant thereof.

A RNAi agent comprising a first strand and a second strand, wherein the first strand comprises at least 15 contiguous nucleotides from a first strand of: AD-40047.1 (SEQ ID NO: 825), or a modified or unmodified variant thereof.

A RNAi agent comprising a first strand and a second strand, wherein the first strand comprises at least 15 contiguous nucleotides from a first strand of: AD-40053.1 (SEQ ID NO: 826), or a modified or unmodified variant thereof.

A RNAi agent comprising a first strand and a second strand, wherein the first strand comprises at least 15 contiguous nucleotides from a first strand of: AD-40059.1 (SEQ ID NO: 827), or a modified or unmodified variant thereof.

A RNAi agent comprising a first strand and a second strand, wherein the first strand comprises at least 15 contiguous nucleotides from a first strand of: AD-40065.1 (SEQ ID NO: 828), or a modified or unmodified variant thereof.

A RNAi agent comprising a first strand and a second strand, wherein the first strand comprises at least 15 contiguous nucleotides from a first strand of: AD-40071.1 (SEQ ID NO: 829), or a modified or unmodified variant thereof.

A RNAi agent comprising a first strand and a second strand, wherein the first strand comprises at least 15 contiguous nucleotides from a first strand of: AD-40077.1 (SEQ ID NO: 830), or a modified or unmodified variant thereof.

A RNAi agent comprising a first strand and a second strand, wherein the first strand comprises at least 15 contiguous nucleotides from a first strand of: AD-40083.1 (SEQ ID NO: 831), or a modified or unmodified variant thereof.

A RNAi agent comprising a first strand and a second strand, wherein the first strand comprises at least 15 contiguous nucleotides from a first strand of: AD-40042.1 (SEQ ID NO: 832), or a modified or unmodified variant thereof.

A RNAi agent comprising a first strand and a second strand, wherein the first strand comprises at least 15 contiguous nucleotides from a first strand of: AD-40048.1 (SEQ ID NO: 833), or a modified or unmodified variant thereof.

A RNAi agent comprising a first strand and a second strand, wherein the first strand comprises at least 15 contiguous nucleotides from a first strand of: AD-40054.1 (SEQ ID NO: 834), or a modified or unmodified variant thereof.

A RNAi agent comprising a first strand and a second strand, wherein the first strand comprises at least 15 contiguous nucleotides from a first strand of: AD-40060.1 (SEQ ID NO: 835), or a modified or unmodified variant thereof.

A RNAi agent comprising a first strand and a second strand, wherein the first strand comprises at least 15 contiguous nucleotides from a first strand of: AD-40066.1 (SEQ ID NO: 836), or a modified or unmodified variant thereof.

A RNAi agent comprising a first strand and a second strand, wherein the first strand comprises at least 15 contiguous nucleotides from a first strand of: AD-40072.1 (SEQ ID NO: 837), or a modified or unmodified variant thereof.

A RNAi agent comprising a first strand and a second strand, wherein the first strand comprises at least 15 contiguous nucleotides from a first strand of: AD-40078.1 (SEQ ID NO: 838), or a modified or unmodified variant thereof.

A RNAi agent comprising a first strand and a second strand, wherein the first strand comprises at least 15 contiguous nucleotides from a first strand of: AD-40084.1 (SEQ ID NO: 839), or a modified or unmodified variant thereof.

A RNAi agent comprising a first strand and a second strand, wherein the first strand comprises at least 15 contiguous nucleotides from a first strand of: AD-40043.1 (SEQ ID NO: 840), or a modified or unmodified variant thereof.

A RNAi agent comprising a first strand and a second strand, wherein the first strand comprises at least 15 contiguous nucleotides from a first strand of: AD-40049.1 (SEQ ID NO: 841), or a modified or unmodified variant thereof.

A RNAi agent comprising a first strand and a second strand, wherein the first strand comprises at least 15 contiguous nucleotides from a first strand of: AD-40055.1 (SEQ ID NO: 842), or a modified or unmodified variant thereof.

A RNAi agent comprising a first strand and a second strand, wherein the first strand comprises at least 15 contiguous nucleotides from a first strand of: AD-40061.1 (SEQ ID NO: 843), or a modified or unmodified variant thereof.

A RNAi agent comprising a first strand and a second strand, wherein the first strand comprises at least 15 contiguous nucleotides from a first strand of: AD-40067.1 (SEQ ID NO: 844), or a modified or unmodified variant thereof.

A RNAi agent comprising a first strand and a second strand, wherein the first strand comprises at least 15 contiguous nucleotides from a first strand of: AD-40073.1 (SEQ ID NO: 845), or a modified or unmodified variant thereof.

A RNAi agent comprising a first strand and a second strand, wherein the first strand comprises at least 15 contiguous nucleotides from a first strand of: AD-40079.1 (SEQ ID NO: 846), or a modified or unmodified variant thereof.

A RNAi agent comprising a first strand and a second strand, wherein the first strand comprises at least 15 contiguous nucleotides from a first strand of: AD-40085.1 (SEQ ID NO: 847), or a modified or unmodified variant thereof.

A RNAi agent comprising a first strand and a second strand, wherein the first strand comprises at least 15 contiguous nucleotides from a first strand of: AD-40044.1 (SEQ ID NO: 848), or a modified or unmodified variant thereof.

A RNAi agent comprising a first strand and a second strand, wherein the first strand comprises at least 15 contiguous nucleotides from a first strand of: AD-40050.1 (SEQ ID NO: 849), or a modified or unmodified variant thereof.

A RNAi agent comprising a first strand and a second strand, wherein the first strand comprises at least 15 contiguous nucleotides from a first strand of: AD-40056.1 (SEQ ID NO: 850), or a modified or unmodified variant thereof.

A RNAi agent comprising a first strand and a second strand, wherein the first strand comprises at least 15 contiguous nucleotides from a first strand of: AD-40062.1 (SEQ ID NO: 851), or a modified or unmodified variant thereof.

A RNAi agent comprising a first strand and a second strand, wherein the first strand comprises at least 15 contiguous nucleotides from a first strand of: AD-40068.1 (SEQ ID NO: 852), or a modified or unmodified variant thereof.

A RNAi agent comprising a first strand and a second strand, wherein the first strand comprises at least 15 contiguous nucleotides from a first strand of: AD-40074.1 (SEQ ID NO: 853), or a modified or unmodified variant thereof.

A RNAi agent comprising a first strand and a second strand, wherein the first strand comprises at least 15 contiguous nucleotides from a first strand of: AD-40080.1 (SEQ ID NO: 854), or a modified or unmodified variant thereof.

A RNAi agent comprising a first strand and a second strand, wherein the first strand comprises at least 15 contiguous nucleotides from a first strand of: AD-39720.1 (SEQ ID NOs: 1754), or a modified or unmodified variant thereof.

A RNAi agent comprising a first strand and a second strand, wherein the first strand comprises at least 15 contiguous nucleotides from a first strand of: AD-39706.1 (SEQ ID NOs: 1755), or a modified or unmodified variant thereof.

A RNAi agent comprising a first strand and a second strand, wherein the first strand comprises at least 15 contiguous nucleotides from a first strand of: AD-39712.1 (SEQ ID NOs: 1756), or a modified or unmodified variant thereof.

A RNAi agent comprising a first strand and a second strand, wherein the first strand comprises at least 15 contiguous nucleotides from a first strand of: AD-39760.1 (SEQ ID NOs: 1757), or a modified or unmodified variant thereof.

A RNAi agent comprising a first strand and a second strand, wherein the first strand comprises at least 15 contiguous nucleotides from a first strand of: AD-39732.1 (SEQ ID NOs: 1758), or a modified or unmodified variant thereof.

A RNAi agent comprising a first strand and a second strand, wherein the first strand comprises at least 15 contiguous nucleotides from a first strand of: AD-35541.4 (SEQ ID NOs: 1759), or a modified or unmodified variant thereof.

A RNAi agent comprising a first strand and a second strand, wherein the first strand comprises at least 15 contiguous nucleotides from a first strand of: AD-39735.1 or hs_KRAS_321_A22S26 (SEQ ID NOs: 1760), or a modified or unmodified variant thereof.

A RNAi agent comprising a first strand and a second strand, wherein the first strand comprises at least 15 contiguous nucleotides from a first strand of: AD-39741.1, or modified or unmodified variants thereof.

hs_KRAS_322_A22S26 (SEQ ID NOs: 1761), or a modified or unmodified variant thereof.

A RNAi agent comprising a first strand and a second strand, wherein the first strand comprises at least 15 contiguous nucleotides from a first strand of: AD-39778.1 (SEQ ID NOs: 1762), or a modified or unmodified variant thereof.

A RNAi agent comprising a first strand and a second strand, wherein the first strand comprises at least 15 contiguous nucleotides from a first strand of: AD-39790.1 (SEQ ID NOs: 1763), or a modified or unmodified variant thereof.

A RNAi agent comprising a first strand and a second strand, wherein the first strand comprises at least 15 contiguous nucleotides from a first strand of: AD-39822.1 (SEQ ID NOs: 1764), or a modified or unmodified variant thereof.

A RNAi agent comprising a first strand and a second strand, wherein the first strand comprises at least 15 contiguous nucleotides from a first strand of: AD-35609.4 (SEQ ID NOs: 1765), or a modified or unmodified variant thereof.

A RNAi agent comprising a first strand and a second strand, wherein the first strand comprises at least 15 contiguous nucleotides from a first strand of: AD-35581.4 (SEQ ID NOs: 1766), or a modified or unmodified variant thereof.

A RNAi agent comprising a first strand and a second strand, wherein the first strand comprises at least 15 contiguous nucleotides from a first strand of: AD-39845.1 (SEQ ID NOs: 1767), or a modified or unmodified variant thereof.

A RNAi agent comprising a first strand and a second strand, wherein the first strand comprises at least 15 contiguous nucleotides from a first strand of: AD-39858.1 (SEQ ID NOs: 1768), or a modified or unmodified variant thereof.

A RNAi agent comprising a first strand and a second strand, wherein the first strand comprises at least 15 contiguous nucleotides from a first strand of: AD-39870.1 (SEQ ID NOs: 1769), or a modified or unmodified variant thereof.

A RNAi agent comprising a first strand and a second strand, wherein the first strand comprises at least 15 contiguous nucleotides from a first strand of: AD-35576.4 (SEQ ID NOs: 1770), or a modified or unmodified variant thereof.

A RNAi agent comprising a first strand and a second strand, wherein the first strand comprises at least 15 contiguous nucleotides from a first strand of: AD-35588.4 (SEQ ID NOs: 1771), or a modified or unmodified variant thereof.

A RNAi agent comprising a first strand and a second strand, wherein the first strand comprises at least 15 contiguous nucleotides from a first strand of: AD-35600.1 (SEQ ID NOs: 1772), or a modified or unmodified variant thereof.

A RNAi agent comprising a first strand and a second strand, wherein the first strand comprises at least 15 contiguous nucleotides from a first strand of: AD-35606.1 (SEQ ID NOs: 1773), or a modified or unmodified variant thereof.
A RNAi agent comprising a first strand and a second strand, wherein the first strand comprises at least 15 contiguous nucleotides from a first strand of: AD-38151.1 (SEQ ID NOs: 1774), or a modified or unmodified variant thereof.

A RNAi agent comprising a first strand and a second strand, wherein the first strand comprises at least 15 contiguous nucleotides from a first strand of: AD-38163.1 (SEQ ID NOs: 1775), or a modified or unmodified variant thereof.

A RNAi agent comprising a first strand and a second strand, wherein the first strand comprises at least 15 contiguous nucleotides from a first strand of: AD-39999.1 (SEQ ID NOs: 1776), or a modified or unmodified variant thereof.

A RNAi agent comprising a first strand and a second strand, wherein the first strand comprises at least 15 contiguous nucleotides from a first strand of: AD-38159.1 or hs_KRAS_528_A22S26 (SEQ ID NOs: 1777), or a modified or unmodified variant thereof.

A RNAi agent comprising a first strand and a second strand, wherein the first strand comprises at least 15 contiguous nucleotides from a first strand of: AD-38130.1 or hs_KRAS_531_A22S26 (SEQ ID NOs: 1778), or a modified or unmodified variant thereof.

A RNAi agent comprising a first strand and a second strand, wherein the first strand comprises at least 15 contiguous nucleotides from a first strand of: AD-38136.1 (SEQ ID NOs: 1779), or a modified or unmodified variant thereof.

A RNAi agent comprising a first strand and a second strand, wherein the first strand comprises at least 15 contiguous nucleotides from a first strand of: AD-40036.1 (SEQ ID NOs: 1780), or a modified or unmodified variant thereof.

A RNAi agent comprising a first strand and a second strand, wherein the first strand comprises at least 15 contiguous nucleotides from a first strand of: AD-40008.1 (SEQ ID NOs: 1781), or a modified or unmodified variant thereof.

A RNAi agent comprising a first strand and a second strand, wherein the first strand comprises at least 15 contiguous nucleotides from a first strand of: AD-40021.1 (SEQ ID NOs: 1782), or a modified or unmodified variant thereof.

A RNAi agent comprising a first strand and a second strand, wherein the first strand comprises at least 15 contiguous nucleotides from a first strand of: AD-40077.1 (SEQ ID NOs: 1783), or a modified or unmodified variant thereof.

A RNAi agent comprising a first strand and a second strand, wherein the first strand comprises at least 15 contiguous nucleotides from a first strand of: AD-40072.1 (SEQ ID NOs: 1784), or a modified or unmodified variant thereof.

A RNAi agent comprising a first strand and a second strand, wherein the first strand comprises at least 15 contiguous nucleotides from a first strand of: AD-40061.1 (SEQ ID NOs: 1785), or a modified or unmodified variant thereof.

A RNAi agent comprising a first strand and a second strand, wherein the first strand comprises at least 15 contiguous nucleotides from a first strand of: AD-40068.1 (SEQ ID NOs: 1786), or a modified or unmodified variant thereof.

A RNAi agent comprising a first strand and a second strand, wherein the first strand comprises at least 15 contiguous nucleotides from a first strand of: AD-38131.1 (SEQ ID NOs: 1787), or a modified or unmodified variant thereof.

A RNAi agent comprising a first strand and a second strand, wherein the first strand comprises at least 15 contiguous nucleotides from a first strand of: AD-38167.1 (SEQ ID NOs: 1788), or a modified or unmodified variant thereof.

A RNAi agent comprising a first strand and a second strand, wherein the first strand comprises at least 15 contiguous nucleotides from a first strand of: hs_KRAS 1273_A22S26 (SEQ ID NOs: 1789), or a modified or unmodified variant thereof.

A RNAi agent comprising a first strand and a second strand, wherein the first strand comprises at least 15 contiguous nucleotides from a first strand of: hs_KRAS 2892_A37S26 (SEQ ID NOs: 1790), or a modified or unmodified variant thereof.

A RNAi agent comprising a first strand and a second strand, wherein the first strand comprises at least 15 contiguous nucleotides from a first strand of: hs_KRAS 4731_A22S26 (SEQ ID NOs: 1791), or a modified or unmodified variant thereof.

**Specific embodiments comprising a first strand of a RNAi agent disclosed herein**

In one embodiment, the present disclosure pertains to a composition comprising any one or more of: a RNAi agent comprising a first and a second strand, wherein the sequence of the first strand is the sequence of a first strand of any RNAi agent from any of Tables 1 to 6, or modified and unmodified variants thereof.

Thus, in various embodiments, the present disclosure pertains to a composition comprising any one or more of the following:

A RNAi agent comprising a first and a second strand, wherein the sequence of the first strand is the sequence of a first strand of: AD-35523.3 (SEQ ID NO: 428), or a modified or unmodified variant thereof.

A RNAi agent comprising a first and a second strand, wherein the sequence of the first strand is the sequence of a first strand of: AD-35529.1 (SEQ ID NO: 429), or a modified or unmodified variant thereof.

A RNAi agent comprising a first and a second strand, wherein the sequence of the first strand is the sequence of a first strand of: AD-35535.4 (SEQ ID NO: 430), or a modified or unmodified variant thereof.

A RNAi agent comprising a first and a second strand, wherein the sequence of the first strand is the sequence of a first strand of: AD-35541.4 (SEQ ID NO: 431), or a modified or unmodified variant thereof.

A RNAi agent comprising a first and a second strand, wherein the sequence of the first strand is the sequence of a first strand of: AD-35547.4 (SEQ ID NO: 432), or a modified or unmodified variant thereof.

A RNAi agent comprising a first and a second strand, wherein the sequence of the first strand is the sequence of a first strand of: AD-35553.4 (SEQ ID NO: 433), or a modified or unmodified variant thereof.

A RNAi agent comprising a first and a second strand, wherein the sequence of the first strand is the sequence of a first strand of: AD-35559.4 (SEQ ID NO: 434), or a modified or unmodified variant thereof.

A RNAi agent comprising a first and a second strand, wherein the sequence of the first strand is the sequence of a first strand of: AD-35565.4 (SEQ ID NO: 435), or a modified or unmodified variant thereof.

A RNAi agent comprising a first and a second strand, wherein the sequence of the first strand is the sequence of a first strand of: AD-35524.4 (SEQ ID NO: 436), or a modified or unmodified variant thereof.

A RNAi agent comprising a first and a second strand, wherein the sequence of the first strand is the sequence of a first strand of: AD-35530.4 (SEQ ID NO: 437), or a modified or unmodified variant thereof.

A RNAi agent comprising a first and a second strand, wherein the sequence of the first strand is the sequence of a first strand of: AD-35536.4 (SEQ ID NO: 438), or a modified or unmodified variant thereof.

A RNAi agent comprising a first and a second strand, wherein the sequence of the first strand is the sequence of a first strand of: AD-35542.4 (SEQ ID NO: 439), or a modified or unmodified variant thereof.

A RNAi agent comprising a first and a second strand, wherein the sequence of the first strand is the sequence of a first strand of: AD-35548.4 (SEQ ID NO: 440), or a modified or unmodified variant thereof.

A RNAi agent comprising a first and a second strand, wherein the sequence of the first strand is the sequence of a first strand of: AD-35554.4 (SEQ ID NO: 441), or a modified or unmodified variant thereof.

A RNAi agent comprising a first and a second strand, wherein the sequence of the first strand is the sequence of a first strand of: AD-35560.2 (SEQ ID NO: 442), or a modified or unmodified variant thereof.

A RNAi agent comprising a first and a second strand, wherein the sequence of the first strand is the sequence of a first strand of: AD-35566.4 (SEQ ID NO: 443), or a modified or unmodified variant thereof.

A RNAi agent comprising a first and a second strand, wherein the sequence of the first strand is the sequence of a first strand of: AD-35525.2 (SEQ ID NO: 444), or a modified or unmodified variant thereof.

A RNAi agent comprising a first and a second strand, wherein the sequence of the first strand is the sequence of a first strand of: AD-35531.4 (SEQ ID NO: 445), or a modified or unmodified variant thereof.

A RNAi agent comprising a first and a second strand, wherein the sequence of the first strand is the sequence of a first strand of: AD-35537.4 (SEQ ID NO: 446), or a modified or unmodified variant thereof.

A RNAi agent comprising a first and a second strand, wherein the sequence of the first strand is the sequence of a first strand of: AD-35543.2 (SEQ ID NO: 447), or a modified or unmodified variant thereof.

A RNAi agent comprising a first and a second strand, wherein the sequence of the first strand is the sequence of a first strand of: AD-35549.4 (SEQ ID NO: 448), or a modified or unmodified variant thereof.

A RNAi agent comprising a first and a second strand, wherein the sequence of the first strand is the sequence of a first strand of: AD-35555.4 (SEQ ID NO: 449), or a modified or unmodified variant thereof.

A RNAi agent comprising a first and a second strand, wherein the sequence of the first strand is the sequence of a first strand of: AD-35561.4 (SEQ ID NO: 450), or a modified or unmodified variant thereof.

A RNAi agent comprising a first and a second strand, wherein the sequence of the first strand is the sequence of a first strand of: AD-35567.4 (SEQ ID NO: 451), or a modified or unmodified variant thereof.

A RNAi agent comprising a first and a second strand, wherein the sequence of the first strand is the sequence of a first strand of: AD-35526.4 (SEQ ID NO: 452), or a modified or unmodified variant thereof.

A RNAi agent comprising a first and a second strand, wherein the sequence of the first strand is the sequence of a first strand of: AD-35532.4 (SEQ ID NO: 453), or a modified or unmodified variant thereof.

A RNAi agent comprising a first and a second strand, wherein the sequence of the first strand is the sequence of a first strand of: AD-35538.4 (SEQ ID NO: 454), or a modified or unmodified variant thereof.

A RNAi agent comprising a first and a second strand, wherein the sequence of the first strand is the sequence of a first strand of: AD-35544.4 (SEQ ID NO: 455), or a modified or unmodified variant thereof.

A RNAi agent comprising a first and a second strand, wherein the sequence of the first strand is the sequence of a first strand of: AD-35550.4 (SEQ ID NO: 456), or a modified or unmodified variant thereof.

A RNAi agent comprising a first and a second strand, wherein the sequence of the first strand is the sequence of a first strand of: AD-35556.4 (SEQ ID NO: 457), or a modified or unmodified variant thereof.

A RNAi agent comprising a first and a second strand, wherein the sequence of the first strand is the sequence of a first strand of: AD-35562.4 (SEQ ID NO: 458), or a modified or unmodified variant thereof.

A RNAi agent comprising a first and a second strand, wherein the sequence of the first strand is the sequence of a first strand of: AD-35568.4 (SEQ ID NO: 459), or a modified or unmodified variant thereof.

A RNAi agent comprising a first and a second strand, wherein the sequence of the first strand is the sequence of a first strand of: AD-35527.4 (SEQ ID NO: 460), or a modified or unmodified variant thereof.

A RNAi agent comprising a first and a second strand, wherein the sequence of the first strand is the sequence of a first strand of: AD-35533.4 (SEQ ID NO: 461), or a modified or unmodified variant thereof.

A RNAi agent comprising a first and a second strand, wherein the sequence of the first strand is the sequence of a first strand of: AD-35539.4 (SEQ ID NO: 462), or a modified or unmodified variant thereof.

A RNAi agent comprising a first and a second strand, wherein the sequence of the first strand is the sequence of a first strand of: AD-35545.4 (SEQ ID NO: 463), or a modified or unmodified variant thereof.

A RNAi agent comprising a first and a second strand, wherein the sequence of the first strand is the sequence of a first strand of: AD-35551.4 (SEQ ID NO: 464), or a modified or unmodified variant thereof.

A RNAi agent comprising a first and a second strand, wherein the sequence of the first strand is the sequence of a first strand of: AD-35563.4 (SEQ ID NO: 465), or a modified or unmodified variant thereof.

A RNAi agent comprising a first and a second strand, wherein the sequence of the first strand is the sequence of a first strand of: AD-35528.4 (SEQ ID NO: 466), or a modified or unmodified variant thereof.

A RNAi agent comprising a first and a second strand, wherein the sequence of the first strand is the sequence of a first strand of: AD-35540.4 (SEQ ID NO: 467), or a modified or unmodified variant thereof.

A RNAi agent comprising a first and a second strand, wherein the sequence of the first strand is the sequence of a first strand of: AD-35546.4 (SEQ ID NO: 468), or a modified or unmodified variant thereof.

A RNAi agent comprising a first and a second strand, wherein the sequence of the first strand is the sequence of a first strand of: AD-35552.4 (SEQ ID NO: 469), or a modified or unmodified variant thereof.

A RNAi agent comprising a first and a second strand, wherein the sequence of the first strand is the sequence of a first strand of: AD-35558.4 (SEQ ID NO: 470), or a modified or unmodified variant thereof.

A RNAi agent comprising a first and a second strand, wherein the sequence of the first strand is the sequence of a first strand of: AD-35564.4 (SEQ ID NO: 471), or a modified or unmodified variant thereof.

A RNAi agent comprising a first and a second strand, wherein the sequence of the first strand is the sequence of a first strand of: AD-35570.4 (SEQ ID NO: 472), or a modified or unmodified variant thereof.

A RNAi agent comprising a first and a second strand, wherein the sequence of the first strand is the sequence of a first strand of: AD-35571.4 (SEQ ID NO: 473), or a modified or unmodified variant thereof.

A RNAi agent comprising a first and a second strand, wherein the sequence of the first strand is the sequence of a first strand of: AD-35577.4 (SEQ ID NO: 474), or a modified or unmodified variant thereof.

A RNAi agent comprising a first and a second strand, wherein the sequence of the first strand is the sequence of a first strand of: AD-35583.4 (SEQ ID NO: 475), or a modified or unmodified variant thereof.

A RNAi agent comprising a first and a second strand, wherein the sequence of the first strand is the sequence of a first strand of: AD-35589.4 (SEQ ID NO: 476), or a modified or unmodified variant thereof.

A RNAi agent comprising a first and a second strand, wherein the sequence of the first strand is the sequence of a first strand of: AD-35595.4 (SEQ ID NO: 477), or a modified or unmodified variant thereof.

A RNAi agent comprising a first and a second strand, wherein the sequence of the first strand is the sequence of a first strand of: AD-35601.4 (SEQ ID NO: 478), or a modified or unmodified variant thereof.

A RNAi agent comprising a first and a second strand, wherein the sequence of the first strand is the sequence of a first strand of: AD-35607.4 (SEQ ID NO: 479), or a modified or unmodified variant thereof.

A RNAi agent comprising a first and a second strand, wherein the sequence of the first strand is the sequence of a first strand of: AD-35613.4 (SEQ ID NO: 480), or a modified or unmodified variant thereof.

A RNAi agent comprising a first and a second strand, wherein the sequence of the first strand is the sequence of a first strand of: AD-35572.4 (SEQ ID NO: 481), or a modified or unmodified variant thereof.

A RNAi agent comprising a first and a second strand, wherein the sequence of the first strand is the sequence of a first strand of: AD-35578.4 (SEQ ID NO: 482), or a modified or unmodified variant thereof.

A RNAi agent comprising a first and a second strand, wherein the sequence of the first strand is the sequence of a first strand of: AD-35584.4 (SEQ ID NO: 483), or a modified or unmodified variant thereof.

A RNAi agent comprising a first and a second strand, wherein the sequence of the first strand is the sequence of a first strand of: AD-35590.4 (SEQ ID NO: 484), or a modified or unmodified variant thereof.

A RNAi agent comprising a first and a second strand, wherein the sequence of the first strand is the sequence of a first strand of: AD-35596.4 (SEQ ID NO: 485), or a modified or unmodified variant thereof.

A RNAi agent comprising a first and a second strand, wherein the sequence of the first strand is the sequence of a first strand of: AD-35602.4 (SEQ ID NO: 486), or a modified or unmodified variant thereof.

A RNAi agent comprising a first and a second strand, wherein the sequence of the first strand is the sequence of a first strand of: AD-35608.4 (SEQ ID NO: 487), or a modified or unmodified variant thereof.

A RNAi agent comprising a first and a second strand, wherein the sequence of the first strand is the sequence of a first strand of: AD-35614.4 (SEQ ID NO: 488), or a modified or unmodified variant thereof.

A RNAi agent comprising a first and a second strand, wherein the sequence of the first strand is the sequence of a first strand of: AD-35573.4 (SEQ ID NO: 489), or a modified or unmodified variant thereof.

A RNAi agent comprising a first and a second strand, wherein the sequence of the first strand is the sequence of a first strand of: AD-35579.4 (SEQ ID NO: 490), or a modified or unmodified variant thereof.

A RNAi agent comprising a first and a second strand, wherein the sequence of the first strand is the sequence of a first strand of: AD-35585.4 (SEQ ID NO: 491), or a modified or unmodified variant thereof.

A RNAi agent comprising a first and a second strand, wherein the sequence of the first strand is the sequence of a first strand of: AD-35591.4 (SEQ ID NO: 492), or a modified or unmodified variant thereof.

A RNAi agent comprising a first and a second strand, wherein the sequence of the first strand is the sequence of a first strand of: AD-35597.4 (SEQ ID NO: 493), or a modified or unmodified variant thereof.

A RNAi agent comprising a first and a second strand, wherein the sequence of the first strand is the sequence of a first strand of: AD-35603.4 (SEQ ID NO: 494), or a modified or unmodified variant thereof.

A RNAi agent comprising a first and a second strand, wherein the sequence of the first strand is the sequence of a first strand of: AD-35609.4 (SEQ ID NO: 495), or a modified or unmodified variant thereof.

A RNAi agent comprising a first and a second strand, wherein the sequence of the first strand is the sequence of a first strand of: AD-35615.4 (SEQ ID NO: 496), or a modified or unmodified variant thereof.

A RNAi agent comprising a first and a second strand, wherein the sequence of the first strand is the sequence of a first strand of: AD-35574.4 (SEQ ID NO: 497), or a modified or unmodified variant thereof.

A RNAi agent comprising a first and a second strand, wherein the sequence of the first strand is the sequence of a first strand of: AD-35580.1 (SEQ ID NO: 498), or a modified or unmodified variant thereof.

A RNAi agent comprising a first and a second strand, wherein the sequence of the first strand is the sequence of a first strand of: AD-35586.4 (SEQ ID NO: 499), or a modified or unmodified variant thereof.

A RNAi agent comprising a first and a second strand, wherein the sequence of the first strand is the sequence of a first strand of: AD-35592.4 (SEQ ID NO: 500), or a modified or unmodified variant thereof.

A RNAi agent comprising a first and a second strand, wherein the sequence of the first strand is the sequence of a first strand of: AD-35598.4 (SEQ ID NO: 501), or a modified or unmodified variant thereof.

A RNAi agent comprising a first and a second strand, wherein the sequence of the first strand is the sequence of a first strand of: AD-35604.4 (SEQ ID NO: 502), or a modified or unmodified variant thereof.

A RNAi agent comprising a first and a second strand, wherein the sequence of the first strand is the sequence of a first strand of: AD-35610.4 (SEQ ID NO: 503), or a modified or unmodified variant thereof.

A RNAi agent comprising a first and a second strand, wherein the sequence of the first strand is the sequence of a first strand of: AD-35616.4 (SEQ ID NO: 504), or a modified or unmodified variant thereof.

A RNAi agent comprising a first and a second strand, wherein the sequence of the first strand is the sequence of a first strand of: AD-35575.4 (SEQ ID NO: 505), or a modified or unmodified variant thereof.

A RNAi agent comprising a first and a second strand, wherein the sequence of the first strand is the sequence of a first strand of: AD-35581.4 (SEQ ID NO: 506), or a modified or unmodified variant thereof.

A RNAi agent comprising a first and a second strand, wherein the sequence of the first strand is the sequence of a first strand of: AD-35587.4 (SEQ ID NO: 507), or a modified or unmodified variant thereof.

A RNAi agent comprising a first and a second strand, wherein the sequence of the first strand is the sequence of a first strand of: AD-35593.4 (SEQ ID NO: 508), or a modified or unmodified variant thereof.

A RNAi agent comprising a first and a second strand, wherein the sequence of the first strand is the sequence of a first strand of: AD-35599.4 (SEQ ID NO: 509), or a modified or unmodified variant thereof.

A RNAi agent comprising a first and a second strand, wherein the sequence of the first strand is the sequence of a first strand of: AD-35605.4 (SEQ ID NO: 510), or a modified or unmodified variant thereof.

A RNAi agent comprising a first and a second strand, wherein the sequence of the first strand is the sequence of a first strand of: AD-35611.4 (SEQ ID NO: 511), or a modified or unmodified variant thereof.

A RNAi agent comprising a first and a second strand, wherein the sequence of the first strand is the sequence of a first strand of: AD-35617.4 (SEQ ID NO: 512), or a modified or unmodified variant thereof.

A RNAi agent comprising a first and a second strand, wherein the sequence of the first strand is the sequence of a first strand of: AD-35576.4 (SEQ ID NO: 513), or a modified or unmodified variant thereof.

A RNAi agent comprising a first and a second strand, wherein the sequence of the first strand is the sequence of a first strand of: AD-35588.4 (SEQ ID NO: 514), or a modified or unmodified variant thereof.

A RNAi agent comprising a first and a second strand, wherein the sequence of the first strand is the sequence of a first strand of: AD-35667.1 (SEQ ID NO: 515), or a modified or unmodified variant thereof.

A RNAi agent comprising a first and a second strand, wherein the sequence of the first strand is the sequence of a first strand of: AD-35673.1 (SEQ ID NO: 516), or a modified or unmodified variant thereof.

A RNAi agent comprising a first and a second strand, wherein the sequence of the first strand is the sequence of a first strand of: AD-35679.1 (SEQ ID NO: 517), or a modified or unmodified variant thereof.

A RNAi agent comprising a first and a second strand, wherein the sequence of the first strand is the sequence of a first strand of: AD-35685.1 (SEQ ID NO: 518), or a modified or unmodified variant thereof.

A RNAi agent comprising a first and a second strand, wherein the sequence of the first strand is the sequence of a first strand of: AD-35691.1 (SEQ ID NO: 519), or a modified or unmodified variant thereof.

A RNAi agent comprising a first and a second strand, wherein the sequence of the first strand is the sequence of a first strand of: AD-35557.1 (SEQ ID NO: 520), or a modified or unmodified variant thereof.

A RNAi agent comprising a first and a second strand, wherein the sequence of the first strand is the sequence of a first strand of: AD-35618.1 (SEQ ID NO: 521), or a modified or unmodified variant thereof.

A RNAi agent comprising a first and a second strand, wherein the sequence of the first strand is the sequence of a first strand of: AD-35569.1 (SEQ ID NO: 522), or a modified or unmodified variant thereof.

A RNAi agent comprising a first and a second strand, wherein the sequence of the first strand is the sequence of a first strand of: AD-35534.1 (SEQ ID NO: 523), or a modified or unmodified variant thereof.

A RNAi agent comprising a first and a second strand, wherein the sequence of the first strand is the sequence of a first strand of: AD-35582.1 (SEQ ID NO: 524), or a modified or unmodified variant thereof.

A RNAi agent comprising a first and a second strand, wherein the sequence of the first strand is the sequence of a first strand of: AD-35594.1 (SEQ ID NO: 525), or a modified or unmodified variant thereof.

A RNAi agent comprising a first and a second strand, wherein the sequence of the first strand is the sequence of a first strand of: AD-35600.1 (SEQ ID NO: 526), or a modified or unmodified variant thereof.

A RNAi agent comprising a first and a second strand, wherein the sequence of the first strand is the sequence of a first strand of: AD-35606.1 (SEQ ID NO: 527), or a modified or unmodified variant thereof.

A RNAi agent comprising a first and a second strand, wherein the sequence of the first strand is the sequence of a first strand of: AD-35612.1 (SEQ ID NO: 528), or a modified or unmodified variant thereof.

A RNAi agent comprising a first and a second strand, wherein the sequence of the first strand is the sequence of a first strand of: AD-38127.1 (SEQ ID NO: 529), or a modified or unmodified variant thereof.

A RNAi agent comprising a first and a second strand, wherein the sequence of the first strand is the sequence of a first strand of: AD-38133.1 (SEQ ID NO: 530), or a modified or unmodified variant thereof.

A RNAi agent comprising a first and a second strand, wherein the sequence of the first strand is the sequence of a first strand of: AD-38139.1 (SEQ ID NO: 531), or a modified or unmodified variant thereof.

A RNAi agent comprising a first and a second strand, wherein the sequence of the first strand is the sequence of a first strand of: AD-38145.1 (SEQ ID NO: 532), or a modified or unmodified variant thereof.

A RNAi agent comprising a first and a second strand, wherein the sequence of the first strand is the sequence of a first strand of: AD-38151.1 (SEQ ID NO: 533), or a modified or unmodified variant thereof.

A RNAi agent comprising a first and a second strand, wherein the sequence of the first strand is the sequence of a first strand of: AD-38157.1 (SEQ ID NO: 534), or a modified or unmodified variant thereof.

A RNAi agent comprising a first and a second strand, wherein the sequence of the first strand is the sequence of a first strand of: AD-38163.1 (SEQ ID NO: 535), or a modified or unmodified variant thereof.

A RNAi agent comprising a first and a second strand, wherein the sequence of the first strand is the sequence of a first strand of: AD-38169.1 (SEQ ID NO: 536), or a modified or unmodified variant thereof.

A RNAi agent comprising a first and a second strand, wherein the sequence of the first strand is the sequence of a first strand of: AD-38128.1 (SEQ ID NO: 537), or a modified or unmodified variant thereof.

A RNAi agent comprising a first and a second strand, wherein the sequence of the first strand is the sequence of a first strand of: AD-38134.1 (SEQ ID NO: 538), or a modified or unmodified variant thereof.

A RNAi agent comprising a first and a second strand, wherein the sequence of the first strand is the sequence of a first strand of: AD-38140.1 (SEQ ID NO: 539), or a modified or unmodified variant thereof.

A RNAi agent comprising a first and a second strand, wherein the sequence of the first strand is the sequence of a first strand of: AD-38146.1 (SEQ ID NO: 540), or a modified or unmodified variant thereof.

A RNAi agent comprising a first and a second strand, wherein the sequence of the first strand is the sequence of a first strand of: AD-38152.1 (SEQ ID NO: 541), or a modified or unmodified variant thereof.

A RNAi agent comprising a first and a second strand, wherein the sequence of the first strand is the sequence of a first strand of: AD-38158.1 (SEQ ID NO: 542), or a modified or unmodified variant thereof.

A RNAi agent comprising a first and a second strand, wherein the sequence of the first strand is the sequence of a first strand of: AD-38164.1 (SEQ ID NO: 543), or a modified or unmodified variant thereof.

A RNAi agent comprising a first and a second strand, wherein the sequence of the first strand is the sequence of a first strand of: AD-38170.1 (SEQ ID NO: 544), or a modified or unmodified variant thereof.

A RNAi agent comprising a first and a second strand, wherein the sequence of the first strand is the sequence of a first strand of: AD-38129.1 (SEQ ID NO: 545), or a modified or unmodified variant thereof.

A RNAi agent comprising a first and a second strand, wherein the sequence of the first strand is the sequence of a first strand of: AD-38135.1 (SEQ ID NO: 546), or a modified or unmodified variant thereof.

A RNAi agent comprising a first and a second strand, wherein the sequence of the first strand is the sequence of a first strand of: AD-38141.1 (SEQ ID NO: 547), or a modified or unmodified variant thereof.

A RNAi agent comprising a first and a second strand, wherein the sequence of the first strand is the sequence of a first strand of: AD-38147.1 (SEQ ID NO: 548), or a modified or unmodified variant thereof.

A RNAi agent comprising a first and a second strand, wherein the sequence of the first strand is the sequence of a first strand of: AD-38153.1 (SEQ ID NO: 549), or a modified or unmodified variant thereof.

A RNAi agent comprising a first and a second strand, wherein the sequence of the first strand is the sequence of a first strand of: AD-38159.1 (SEQ ID NO: 550), or a modified or unmodified variant thereof.

A RNAi agent comprising a first and a second strand, wherein the sequence of the first strand is the sequence of a first strand of: AD-38165.1 (SEQ ID NO: 551), or a modified or unmodified variant thereof.

A RNAi agent comprising a first and a second strand, wherein the sequence of the first strand is the sequence of a first strand of: AD-38171.1 (SEQ ID NO: 552), or a modified or unmodified variant thereof.

A RNAi agent comprising a first and a second strand, wherein the sequence of the first strand is the sequence of a first strand of: AD-38130.1 (SEQ ID NO: 553), or a modified or unmodified variant thereof.

A RNAi agent comprising a first and a second strand, wherein the sequence of the first strand is the sequence of a first strand of: AD-38136.1 (SEQ ID NO: 554), or a modified or unmodified variant thereof.

A RNAi agent comprising a first and a second strand, wherein the sequence of the first strand is the sequence of a first strand of: AD-38142.1 (SEQ ID NO: 555), or a modified or unmodified variant thereof.

A RNAi agent comprising a first and a second strand, wherein the sequence of the first strand is the sequence of a first strand of: AD-38148.1 (SEQ ID NO: 556), or a modified or unmodified variant thereof.

A RNAi agent comprising a first and a second strand, wherein the sequence of the first strand is the sequence of a first strand of: AD-38154.1 (SEQ ID NO: 557), or a modified or unmodified variant thereof.

A RNAi agent comprising a first and a second strand, wherein the sequence of the first strand is the sequence of a first strand of: AD-38160.1 (SEQ ID NO: 558), or a modified or unmodified variant thereof.

A RNAi agent comprising a first and a second strand, wherein the sequence of the first strand is the sequence of a first strand of: AD-38166.1 (SEQ ID NO: 559), or a modified or unmodified variant thereof.

A RNAi agent comprising a first and a second strand, wherein the sequence of the first strand is the sequence of a first strand of: AD-38172.1 (SEQ ID NO: 560), or a modified or unmodified variant thereof.

A RNAi agent comprising a first and a second strand, wherein the sequence of the first strand is the sequence of a first strand of: AD-38131.1 (SEQ ID NO: 561), or a modified or unmodified variant thereof.

A RNAi agent comprising a first and a second strand, wherein the sequence of the first strand is the sequence of a first strand of: AD-38137.1 (SEQ ID NO: 562), or a modified or unmodified variant thereof.

A RNAi agent comprising a first and a second strand, wherein the sequence of the first strand is the sequence of a first strand of: AD-38143.1 (SEQ ID NO: 563), or a modified or unmodified variant thereof.

A RNAi agent comprising a first and a second strand, wherein the sequence of the first strand is the sequence of a first strand of: AD-38149.1 (SEQ ID NO: 564), or a modified or unmodified variant thereof.

A RNAi agent comprising a first and a second strand, wherein the sequence of the first strand is the sequence of a first strand of: AD-38155.1 (SEQ ID NO: 565), or a modified or unmodified variant thereof.

A RNAi agent comprising a first and a second strand, wherein the sequence of the first strand is the sequence of a first strand of: AD-38161.1 (SEQ ID NO: 566), or a modified or unmodified variant thereof.

A RNAi agent comprising a first and a second strand, wherein the sequence of the first strand is the sequence of a first strand of: AD-38167.1 (SEQ ID NO: 567), or a modified or unmodified variant thereof.

A RNAi agent comprising a first and a second strand, wherein the sequence of the first strand is the sequence of a first strand of: AD-38173.1 (SEQ ID NO: 568), or a modified or unmodified variant thereof.

A RNAi agent comprising a first and a second strand, wherein the sequence of the first strand is the sequence of a first strand of: AD-38132.1 (SEQ ID NO: 569), or a modified or unmodified variant thereof.

A RNAi agent comprising a first and a second strand, wherein the sequence of the first strand is the sequence of a first strand of: AD-38138.1 (SEQ ID NO: 570), or a modified or unmodified variant thereof.

A RNAi agent comprising a first and a second strand, wherein the sequence of the first strand is the sequence of a first strand of: AD-38144.1 (SEQ ID NO: 571), or a modified or unmodified variant thereof.

A RNAi agent comprising a first and a second strand, wherein the sequence of the first strand is the sequence of a first strand of: AD-38150.1 (SEQ ID NO: 572), or a modified or unmodified variant thereof.

A RNAi agent comprising a first and a second strand, wherein the sequence of the first strand is the sequence of a first strand of: AD-38156.1 (SEQ ID NO: 573), or a modified or unmodified variant thereof.

A RNAi agent comprising a first and a second strand, wherein the sequence of the first strand is the sequence of a first strand of: AD-38162.1 (SEQ ID NO: 574), or a modified or unmodified variant thereof.

A RNAi agent comprising a first and a second strand, wherein the sequence of the first strand is the sequence of a first strand of: AD-38168.1 (SEQ ID NO: 575), or a modified or unmodified variant thereof.

A RNAi agent comprising a first and a second strand, wherein the sequence of the first strand is the sequence of a first strand of: AD-38174.1 (SEQ ID NO: 576), or a modified or unmodified variant thereof.

A RNAi agent comprising a first and a second strand, wherein the sequence of the first strand is the sequence of a first strand of: AD-39683.1 (SEQ ID NO: 577), or a modified or unmodified variant thereof.

A RNAi agent comprising a first and a second strand, wherein the sequence of the first strand is the sequence of a first strand of: AD-39689.1 (SEQ ID NO: 578), or a modified or unmodified variant thereof.

A RNAi agent comprising a first and a second strand, wherein the sequence of the first strand is the sequence of a first strand of: AD-39695.1 (SEQ ID NO: 579), or a modified or unmodified variant thereof.

A RNAi agent comprising a first and a second strand, wherein the sequence of the first strand is the sequence of a first strand of: AD-39701.1 (SEQ ID NO: 580), or a modified or unmodified variant thereof.

A RNAi agent comprising a first and a second strand, wherein the sequence of the first strand is the sequence of a first strand of: AD-39707.1 (SEQ ID NO: 581), or a modified or unmodified variant thereof.

A RNAi agent comprising a first and a second strand, wherein the sequence of the first strand is the sequence of a first strand of: AD-39713.1 (SEQ ID NO: 582), or a modified or unmodified variant thereof.

A RNAi agent comprising a first and a second strand, wherein the sequence of the first strand is the sequence of a first strand of: AD-39719.1 (SEQ ID NO: 583), or a modified or unmodified variant thereof.

A RNAi agent comprising a first and a second strand, wherein the sequence of the first strand is the sequence of a first strand of: AD-39725.1 (SEQ ID NO: 584), or a modified or unmodified variant thereof.

A RNAi agent comprising a first and a second strand, wherein the sequence of the first strand is the sequence of a first strand of: AD-39684.1 (SEQ ID NO: 585), or a modified or unmodified variant thereof.

A RNAi agent comprising a first and a second strand, wherein the sequence of the first strand is the sequence of a first strand of: AD-39690.1 (SEQ ID NO: 586), or a modified or unmodified variant thereof.

A RNAi agent comprising a first and a second strand, wherein the sequence of the first strand is the sequence of a first strand of: AD-39696.1 (SEQ ID NO: 587), or a modified or unmodified variant thereof.

A RNAi agent comprising a first and a second strand, wherein the sequence of the first strand is the sequence of a first strand of: AD-39702.1 (SEQ ID NO: 588), or a modified or unmodified variant thereof.

A RNAi agent comprising a first and a second strand, wherein the sequence of the first strand is the sequence of a first strand of: AD-39708.1 (SEQ ID NO: 589), or a modified or unmodified variant thereof.

A RNAi agent comprising a first and a second strand, wherein the sequence of the first strand is the sequence of a first strand of: AD-39714.1 (SEQ ID NO: 590), or a modified or unmodified variant thereof.

A RNAi agent comprising a first and a second strand, wherein the sequence of the first strand is the sequence of a first strand of: AD-39720.1 (SEQ ID NO: 591), or a modified or unmodified variant thereof.

A RNAi agent comprising a first and a second strand, wherein the sequence of the first strand is the sequence of a first strand of: AD-39726.1 (SEQ ID NO: 592), or a modified or unmodified variant thereof.

A RNAi agent comprising a first and a second strand, wherein the sequence of the first strand is the sequence of a first strand of: AD-39685.1 (SEQ ID NO: 593), or a modified or unmodified variant thereof.

A RNAi agent comprising a first and a second strand, wherein the sequence of the first strand is the sequence of a first strand of: AD-39691.1 (SEQ ID NO: 594), or a modified or unmodified variant thereof.

A RNAi agent comprising a first and a second strand, wherein the sequence of the first strand is the sequence of a first strand of: AD-39697.1 (SEQ ID NO: 595), or a modified or unmodified variant thereof.

A RNAi agent comprising a first and a second strand, wherein the sequence of the first strand is the sequence of a first strand of: AD-39703.1 (SEQ ID NO: 596), or a modified or unmodified variant thereof.

A RNAi agent comprising a first and a second strand, wherein the sequence of the first strand is the sequence of a first strand of: AD-39709.1 (SEQ ID NO: 597), or a modified or unmodified variant thereof.

A RNAi agent comprising a first and a second strand, wherein the sequence of the first strand is the sequence of a first strand of: AD-39715.1 (SEQ ID NO: 598), or a modified or unmodified variant thereof.

A RNAi agent comprising a first and a second strand, wherein the sequence of the first strand is the sequence of a first strand of: AD-39721.1 (SEQ ID NO: 599), or a modified or unmodified variant thereof.

A RNAi agent comprising a first and a second strand, wherein the sequence of the first strand is the sequence of a first strand of: AD-39727.1 (SEQ ID NO: 600), or a modified or unmodified variant thereof.
A RNAi agent comprising a first and a second strand, wherein the sequence of the first strand is the sequence of a first strand of: AD-39686.1 (SEQ ID NO: 601), or a modified or unmodified variant thereof.
A RNAi agent comprising a first and a second strand, wherein the sequence of the first strand is the sequence of a first strand of: AD-39692.1 (SEQ ID NO: 602), or a modified or unmodified variant thereof.
A RNAi agent comprising a first and a second strand, wherein the sequence of the first strand is the sequence of a first strand of: AD-39698.1 (SEQ ID NO: 603), or a modified or unmodified variant thereof.
A RNAi agent comprising a first and a second strand, wherein the sequence of the first strand is the sequence of a first strand of: AD-39704.1 (SEQ ID NO: 604), or a modified or unmodified variant thereof.
A RNAi agent comprising a first and a second strand, wherein the sequence of the first strand is the sequence of a first strand of: AD-39710.1 (SEQ ID NO: 605), or a modified or unmodified variant thereof.
A RNAi agent comprising a first and a second strand, wherein the sequence of the first strand is the sequence of a first strand of: AD-39716.1 (SEQ ID NO: 606), or a modified or unmodified variant thereof.
A RNAi agent comprising a first and a second strand, wherein the sequence of the first strand is the sequence of a first strand of: AD-39722.1 (SEQ ID NO: 607), or a modified or unmodified variant thereof.
A RNAi agent comprising a first and a second strand, wherein the sequence of the first strand is the sequence of a first strand of: AD-39728.1 (SEQ ID NO: 608), or a modified or unmodified variant thereof.
A RNAi agent comprising a first and a second strand, wherein the sequence of the first strand is the sequence of a first strand of: AD-39687.1 (SEQ ID NO: 609), or a modified or unmodified variant thereof.
A RNAi agent comprising a first and a second strand, wherein the sequence of the first strand is the sequence of a first strand of: AD-39693.1 (SEQ ID NO: 610), or a modified or unmodified variant thereof.
A RNAi agent comprising a first and a second strand, wherein the sequence of the first strand is the sequence of a first strand of: AD-39699.1 (SEQ ID NO: 611), or a modified or unmodified variant thereof.
A RNAi agent comprising a first and a second strand, wherein the sequence of the first strand is the sequence of a first strand of: AD-39705.1 (SEQ ID NO: 612), or a modified or unmodified variant thereof.
A RNAi agent comprising a first and a second strand, wherein the sequence of the first strand is the sequence of a first strand of: AD-39711.1 (SEQ ID NO: 613), or a modified or unmodified variant thereof.
A RNAi agent comprising a first and a second strand, wherein the sequence of the first strand is the sequence of a first strand of: AD-39717.1 (SEQ ID NO: 614), or a modified or unmodified variant thereof.
A RNAi agent comprising a first and a second strand, wherein the sequence of the first strand is the sequence of a first strand of: AD-39723.1 (SEQ ID NO: 615), or a modified or unmodified variant thereof.
A RNAi agent comprising a first and a second strand, wherein the sequence of the first strand is the sequence of a first strand of: AD-39729.1 (SEQ ID NO: 616), or a modified or unmodified variant thereof.
A RNAi agent comprising a first and a second strand, wherein the sequence of the first strand is the sequence of a first strand of: AD-39688.1 (SEQ ID NO: 617), or a modified or unmodified variant thereof.
A RNAi agent comprising a first and a second strand, wherein the sequence of the first strand is the sequence of a first strand of: AD-39694.1 (SEQ ID NO: 618), or a modified or unmodified variant thereof.
A RNAi agent comprising a first and a second strand, wherein the sequence of the first strand is the sequence of a first strand of: AD-39700.1 (SEQ ID NO: 619), or a modified or unmodified variant thereof.
A RNAi agent comprising a first and a second strand, wherein the sequence of the first strand is the sequence of a first strand of: AD-39706.1 (SEQ ID NO: 620), or a modified or unmodified variant thereof.
A RNAi agent comprising a first and a second strand, wherein the sequence of the first strand is the sequence of a first strand of: AD-39712.1 (SEQ ID NO: 621), or a modified or unmodified variant thereof.
A RNAi agent comprising a first and a second strand, wherein the sequence of the first strand is the sequence of a first strand of: AD-39718.1 (SEQ ID NO: 622), or a modified or unmodified variant thereof.
A RNAi agent comprising a first and a second strand, wherein the sequence of the first strand is the sequence of a first strand of: AD-39724.1 (SEQ ID NO: 623), or a modified or unmodified variant thereof.
A RNAi agent comprising a first and a second strand, wherein the sequence of the first strand is the sequence of a first strand of: AD-39730.1 (SEQ ID NO: 624), or a modified or unmodified variant thereof.
A RNAi agent comprising a first and a second strand, wherein the sequence of the first strand is the sequence of a first strand of: AD-39736.1 (SEQ ID NO: 625), or a modified or unmodified variant thereof.
A RNAi agent comprising a first and a second strand, wherein the sequence of the first strand is the sequence of a first strand of: AD-39742.1 (SEQ ID NO: 626), or a modified or unmodified variant thereof.
A RNAi agent comprising a first and a second strand, wherein the sequence of the first strand is the sequence of a first strand of: AD-39748.1 (SEQ ID NO: 627), or a modified or unmodified variant thereof.
A RNAi agent comprising a first and a second strand, wherein the sequence of the first strand is the sequence of a first strand of: AD-39754.1 (SEQ ID NO: 628), or a modified or unmodified variant thereof.

A RNAi agent comprising a first and a second strand, wherein the sequence of the first strand is the sequence of a first strand of: AD-39760.1 (SEQ ID NO: 629), or a modified or unmodified variant thereof.
A RNAi agent comprising a first and a second strand, wherein the sequence of the first strand is the sequence of a first strand of: AD-39766.1 (SEQ ID NO: 630), or a modified or unmodified variant thereof.
A RNAi agent comprising a first and a second strand, wherein the sequence of the first strand is the sequence of a first strand of: AD-39772.1 (SEQ ID NO: 631), or a modified or unmodified variant thereof.
A RNAi agent comprising a first and a second strand, wherein the sequence of the first strand is the sequence of a first strand of: AD-39731.1 (SEQ ID NO: 632), or a modified or unmodified variant thereof.
A RNAi agent comprising a first and a second strand, wherein the sequence of the first strand is the sequence of a first strand of: AD-39737.1 (SEQ ID NO: 633), or a modified or unmodified variant thereof.
A RNAi agent comprising a first and a second strand, wherein the sequence of the first strand is the sequence of a first strand of: AD-39743.1 (SEQ ID NO: 634), or a modified or unmodified variant thereof.
A RNAi agent comprising a first and a second strand, wherein the sequence of the first strand is the sequence of a first strand of: AD-39749.1 (SEQ ID NO: 635), or a modified or unmodified variant thereof.
A RNAi agent comprising a first and a second strand, wherein the sequence of the first strand is the sequence of a first strand of: AD-39755.1 (SEQ ID NO: 636), or a modified or unmodified variant thereof.
A RNAi agent comprising a first and a second strand, wherein the sequence of the first strand is the sequence of a first strand of: AD-39761.1 (SEQ ID NO: 637), or a modified or unmodified variant thereof.
A RNAi agent comprising a first and a second strand, wherein the sequence of the first strand is the sequence of a first strand of: AD-39767.1 (SEQ ID NO: 638), or a modified or unmodified variant thereof.
A RNAi agent comprising a first and a second strand, wherein the sequence of the first strand is the sequence of a first strand of: AD-39773.1 (SEQ ID NO: 639), or a modified or unmodified variant thereof.
A RNAi agent comprising a first and a second strand, wherein the sequence of the first strand is the sequence of a first strand of: AD-39732.1 (SEQ ID NO: 640), or a modified or unmodified variant thereof.
A RNAi agent comprising a first and a second strand, wherein the sequence of the first strand is the sequence of a first strand of: AD-39738.1 (SEQ ID NO: 641), or a modified or unmodified variant thereof.
A RNAi agent comprising a first and a second strand, wherein the sequence of the first strand is the sequence of a first strand of: AD-39744.1 (SEQ ID NO: 642), or a modified or unmodified variant thereof.
A RNAi agent comprising a first and a second strand, wherein the sequence of the first strand is the sequence of a first strand of: AD-39750.1 (SEQ ID NO: 643), or a modified or unmodified variant thereof.
A RNAi agent comprising a first and a second strand, wherein the sequence of the first strand is the sequence of a first strand of: AD-39756.1 (SEQ ID NO: 644), or a modified or unmodified variant thereof.
A RNAi agent comprising a first and a second strand, wherein the sequence of the first strand is the sequence of a first strand of: AD-39762.1 (SEQ ID NO: 645), or a modified or unmodified variant thereof.
A RNAi agent comprising a first and a second strand, wherein the sequence of the first strand is the sequence of a first strand of: AD-39768.1 (SEQ ID NO: 646), or a modified or unmodified variant thereof.
A RNAi agent comprising a first and a second strand, wherein the sequence of the first strand is the sequence of a first strand of: AD-39774.1 (SEQ ID NO: 647), or a modified or unmodified variant thereof.
A RNAi agent comprising a first and a second strand, wherein the sequence of the first strand is the sequence of a first strand of: AD-39733.1 (SEQ ID NO: 648), or a modified or unmodified variant thereof.
A RNAi agent comprising a first and a second strand, wherein the sequence of the first strand is the sequence of a first strand of: AD-39739.1 (SEQ ID NO: 649), or a modified or unmodified variant thereof.
A RNAi agent comprising a first and a second strand, wherein the sequence of the first strand is the sequence of a first strand of: AD-39745.1 (SEQ ID NO: 650), or a modified or unmodified variant thereof.
A RNAi agent comprising a first and a second strand, wherein the sequence of the first strand is the sequence of a first strand of: AD-39751.1 (SEQ ID NO: 651), or a modified or unmodified variant thereof.
A RNAi agent comprising a first and a second strand, wherein the sequence of the first strand is the sequence of a first strand of: AD-39757.1 (SEQ ID NO: 652), or a modified or unmodified variant thereof.
A RNAi agent comprising a first and a second strand, wherein the sequence of the first strand is the sequence of a first strand of: AD-39763.1 (SEQ ID NO: 653), or a modified or unmodified variant thereof.
A RNAi agent comprising a first and a second strand, wherein the sequence of the first strand is the sequence of a first strand of: AD-39769.1 (SEQ ID NO: 654), or a modified or unmodified variant thereof.
A RNAi agent comprising a first and a second strand, wherein the sequence of the first strand is the sequence of a first strand of: AD-39775.1 (SEQ ID NO: 655), or a modified or unmodified variant thereof.
A RNAi agent comprising a first and a second strand, wherein the sequence of the first strand is the sequence of a first strand of: AD-39734.1 (SEQ ID NO: 656), or a modified or unmodified variant thereof.
A RNAi agent comprising a first and a second strand, wherein the sequence of the first strand is the sequence of a first strand of: AD-39740.1 (SEQ ID NO: 657), or a modified or unmodified variant thereof.

A RNAi agent comprising a first and a second strand, wherein the sequence of the first strand is the sequence of a first strand of: AD-39746.1 (SEQ ID NO: 658), or a modified or unmodified variant thereof.

A RNAi agent comprising a first and a second strand, wherein the sequence of the first strand is the sequence of a first strand of: AD-39752.1 (SEQ ID NO: 659), or a modified or unmodified variant thereof.

A RNAi agent comprising a first and a second strand, wherein the sequence of the first strand is the sequence of a first strand of: AD-39758.1 (SEQ ID NO: 660), or a modified or unmodified variant thereof.

A RNAi agent comprising a first and a second strand, wherein the sequence of the first strand is the sequence of a first strand of: AD-39764.1 (SEQ ID NO: 661), or a modified or unmodified variant thereof.

A RNAi agent comprising a first and a second strand, wherein the sequence of the first strand is the sequence of a first strand of: AD-39770.1 (SEQ ID NO: 662), or a modified or unmodified variant thereof.

A RNAi agent comprising a first and a second strand, wherein the sequence of the first strand is the sequence of a first strand of: AD-39776.1 (SEQ ID NO: 663), or a modified or unmodified variant thereof.

A RNAi agent comprising a first and a second strand, wherein the sequence of the first strand is the sequence of a first strand of: AD-39735.1 (SEQ ID NO: 664), or a modified or unmodified variant thereof.

A RNAi agent comprising a first and a second strand, wherein the sequence of the first strand is the sequence of a first strand of: AD-39741.1 (SEQ ID NO: 665), or a modified or unmodified variant thereof.

A RNAi agent comprising a first and a second strand, wherein the sequence of the first strand is the sequence of a first strand of: AD-39747.1 (SEQ ID NO: 666), or a modified or unmodified variant thereof.

A RNAi agent comprising a first and a second strand, wherein the sequence of the first strand is the sequence of a first strand of: AD-39753.1 (SEQ ID NO: 667), or a modified or unmodified variant thereof.

A RNAi agent comprising a first and a second strand, wherein the sequence of the first strand is the sequence of a first strand of: AD-39759.1 (SEQ ID NO: 668), or a modified or unmodified variant thereof.

A RNAi agent comprising a first and a second strand, wherein the sequence of the first strand is the sequence of a first strand of: AD-39765.1 (SEQ ID NO: 669), or a modified or unmodified variant thereof.

A RNAi agent comprising a first and a second strand, wherein the sequence of the first strand is the sequence of a first strand of: AD-39771.1 (SEQ ID NO: 670), or a modified or unmodified variant thereof.

A RNAi agent comprising a first and a second strand, wherein the sequence of the first strand is the sequence of a first strand of: AD-39778.1 (SEQ ID NO: 671), or a modified or unmodified variant thereof.

A RNAi agent comprising a first and a second strand, wherein the sequence of the first strand is the sequence of a first strand of: AD-39784.1 (SEQ ID NO: 672), or a modified or unmodified variant thereof.

A RNAi agent comprising a first and a second strand, wherein the sequence of the first strand is the sequence of a first strand of: AD-39790.1 (SEQ ID NO: 673), or a modified or unmodified variant thereof.

A RNAi agent comprising a first and a second strand, wherein the sequence of the first strand is the sequence of a first strand of: AD-39796.1 (SEQ ID NO: 674), or a modified or unmodified variant thereof.

A RNAi agent comprising a first and a second strand, wherein the sequence of the first strand is the sequence of a first strand of: AD-39802.1 (SEQ ID NO: 675), or a modified or unmodified variant thereof.

A RNAi agent comprising a first and a second strand, wherein the sequence of the first strand is the sequence of a first strand of: AD-39808.1 (SEQ ID NO: 676), or a modified or unmodified variant thereof.

A RNAi agent comprising a first and a second strand, wherein the sequence of the first strand is the sequence of a first strand of: AD-39814.1 (SEQ ID NO: 677), or a modified or unmodified variant thereof.

A RNAi agent comprising a first and a second strand, wherein the sequence of the first strand is the sequence of a first strand of: AD-39820.1 (SEQ ID NO: 678), or a modified or unmodified variant thereof.

A RNAi agent comprising a first and a second strand, wherein the sequence of the first strand is the sequence of a first strand of: AD-39779.1 (SEQ ID NO: 679), or a modified or unmodified variant thereof.

A RNAi agent comprising a first and a second strand, wherein the sequence of the first strand is the sequence of a first strand of: AD-39785.1 (SEQ ID NO: 680), or a modified or unmodified variant thereof.

A RNAi agent comprising a first and a second strand, wherein the sequence of the first strand is the sequence of a first strand of: AD-39791.1 (SEQ ID NO: 681), or a modified or unmodified variant thereof.

A RNAi agent comprising a first and a second strand, wherein the sequence of the first strand is the sequence of a first strand of: AD-39797.1 (SEQ ID NO: 682), or a modified or unmodified variant thereof.

A RNAi agent comprising a first and a second strand, wherein the sequence of the first strand is the sequence of a first strand of: AD-39803.1 (SEQ ID NO: 683), or a modified or unmodified variant thereof.

A RNAi agent comprising a first and a second strand, wherein the sequence of the first strand is the sequence of a first strand of: AD-39809.1 (SEQ ID NO: 684), or a modified or unmodified variant thereof.

A RNAi agent comprising a first and a second strand, wherein the sequence of the first strand is the sequence of a first strand of: AD-39815.1 (SEQ ID NO: 685), or a modified or unmodified variant thereof.

A RNAi agent comprising a first and a second strand, wherein the sequence of the first strand is the sequence of a first strand of: AD-39821.1 (SEQ ID NO: 686), or a modified or unmodified variant thereof.

A RNAi agent comprising a first and a second strand, wherein the sequence of the first strand is the sequence of a first strand of: AD-39780.1 (SEQ ID NO: 687), or a modified or unmodified variant thereof.

A RNAi agent comprising a first and a second strand, wherein the sequence of the first strand is the sequence of a first strand of: AD-39786.1 (SEQ ID NO: 688), or a modified or unmodified variant thereof.

A RNAi agent comprising a first and a second strand, wherein the sequence of the first strand is the sequence of a first strand of: AD-39792.1 (SEQ ID NO: 689), or a modified or unmodified variant thereof.

A RNAi agent comprising a first and a second strand, wherein the sequence of the first strand is the sequence of a first strand of: AD-39798.1 (SEQ ID NO: 690), or a modified or unmodified variant thereof.

A RNAi agent comprising a first and a second strand, wherein the sequence of the first strand is the sequence of a first strand of: AD-39804.1 (SEQ ID NO: 691), or a modified or unmodified variant thereof.

A RNAi agent comprising a first and a second strand, wherein the sequence of the first strand is the sequence of a first strand of: AD-39810.1 (SEQ ID NO: 692), or a modified or unmodified variant thereof.

A RNAi agent comprising a first and a second strand, wherein the sequence of the first strand is the sequence of a first strand of: AD-39816.1 (SEQ ID NO: 693), or a modified or unmodified variant thereof.

A RNAi agent comprising a first and a second strand, wherein the sequence of the first strand is the sequence of a first strand of: AD-39822.1 (SEQ ID NO: 694), or a modified or unmodified variant thereof.

A RNAi agent comprising a first and a second strand, wherein the sequence of the first strand is the sequence of a first strand of: AD-39781.1 (SEQ ID NO: 695), or a modified or unmodified variant thereof.

A RNAi agent comprising a first and a second strand, wherein the sequence of the first strand is the sequence of a first strand of: AD-39787.1 (SEQ ID NO: 696), or a modified or unmodified variant thereof.

A RNAi agent comprising a first and a second strand, wherein the sequence of the first strand is the sequence of a first strand of: AD-39793.1 (SEQ ID NO: 697), or a modified or unmodified variant thereof.

A RNAi agent comprising a first and a second strand, wherein the sequence of the first strand is the sequence of a first strand of: AD-39799.1 (SEQ ID NO: 698), or a modified or unmodified variant thereof.

A RNAi agent comprising a first and a second strand, wherein the sequence of the first strand is the sequence of a first strand of: AD-39805.1 (SEQ ID NO: 699), or a modified or unmodified variant thereof.

A RNAi agent comprising a first and a second strand, wherein the sequence of the first strand is the sequence of a first strand of: AD-39811.1 (SEQ ID NO: 700), or a modified or unmodified variant thereof.

A RNAi agent comprising a first and a second strand, wherein the sequence of the first strand is the sequence of a first strand of: AD-39817.1 (SEQ ID NO: 701), or a modified or unmodified variant thereof.

A RNAi agent comprising a first and a second strand, wherein the sequence of the first strand is the sequence of a first strand of: AD-39823.1 (SEQ ID NO: 702), or a modified or unmodified variant thereof.

A RNAi agent comprising a first and a second strand, wherein the sequence of the first strand is the sequence of a first strand of: AD-39782.1 (SEQ ID NO: 703), or a modified or unmodified variant thereof.

A RNAi agent comprising a first and a second strand, wherein the sequence of the first strand is the sequence of a first strand of: AD-39788.1 (SEQ ID NO: 704), or a modified or unmodified variant thereof.

A RNAi agent comprising a first and a second strand, wherein the sequence of the first strand is the sequence of a first strand of: AD-39794.1 (SEQ ID NO: 705), or a modified or unmodified variant thereof.

A RNAi agent comprising a first and a second strand, wherein the sequence of the first strand is the sequence of a first strand of: AD-39800.1 (SEQ ID NO: 706), or a modified or unmodified variant thereof.

A RNAi agent comprising a first and a second strand, wherein the sequence of the first strand is the sequence of a first strand of: AD-39806.1 (SEQ ID NO: 707), or a modified or unmodified variant thereof.

A RNAi agent comprising a first and a second strand, wherein the sequence of the first strand is the sequence of a first strand of: AD-39812.1 (SEQ ID NO: 708), or a modified or unmodified variant thereof.

A RNAi agent comprising a first and a second strand, wherein the sequence of the first strand is the sequence of a first strand of: AD-39818.1 (SEQ ID NO: 709), or a modified or unmodified variant thereof.

A RNAi agent comprising a first and a second strand, wherein the sequence of the first strand is the sequence of a first strand of: AD-39824.1 (SEQ ID NO: 710), or a modified or unmodified variant thereof.

A RNAi agent comprising a first and a second strand, wherein the sequence of the first strand is the sequence of a first strand of: AD-39783.1 (SEQ ID NO: 711), or a modified or unmodified variant thereof.

A RNAi agent comprising a first and a second strand, wherein the sequence of the first strand is the sequence of a first strand of: AD-39789.1 (SEQ ID NO: 712), or a modified or unmodified variant thereof.

A RNAi agent comprising a first and a second strand, wherein the sequence of the first strand is the sequence of a first strand of: AD-39795.1 (SEQ ID NO: 713), or a modified or unmodified variant thereof.

A RNAi agent comprising a first and a second strand, wherein the sequence of the first strand is the sequence of a first strand of: AD-39801.1 (SEQ ID NO: 714), or a modified or unmodified variant thereof.

A RNAi agent comprising a first and a second strand, wherein the sequence of the first strand is the sequence of a first strand of: AD-39807.1 (SEQ ID NO: 715), or a modified or unmodified variant thereof.

A RNAi agent comprising a first and a second strand, wherein the sequence of the first strand is the sequence of a first strand of: AD-39813.1 (SEQ ID NO: 716), or a modified or unmodified variant thereof.

A RNAi agent comprising a first and a second strand, wherein the sequence of the first strand is the sequence of a first strand of: AD-39819.1 (SEQ ID NO: 717), or a modified or unmodified variant thereof.

A RNAi agent comprising a first and a second strand, wherein the sequence of the first strand is the sequence of a first strand of: AD-39825.1 (SEQ ID NO: 718), or a modified or unmodified variant thereof.

A RNAi agent comprising a first and a second strand, wherein the sequence of the first strand is the sequence of a first strand of: AD-39831.1 (SEQ ID NO: 719), or a modified or unmodified variant thereof.

A RNAi agent comprising a first and a second strand, wherein the sequence of the first strand is the sequence of a first strand of: AD-39837.1 (SEQ ID NO: 720), or a modified or unmodified variant thereof.

A RNAi agent comprising a first and a second strand, wherein the sequence of the first strand is the sequence of a first strand of: AD-39843.1 (SEQ ID NO: 721), or a modified or unmodified variant thereof.

A RNAi agent comprising a first and a second strand, wherein the sequence of the first strand is the sequence of a first strand of: AD-39849.1 (SEQ ID NO: 722), or a modified or unmodified variant thereof.

A RNAi agent comprising a first and a second strand, wherein the sequence of the first strand is the sequence of a first strand of: AD-39855.1 (SEQ ID NO: 723), or a modified or unmodified variant thereof.

A RNAi agent comprising a first and a second strand, wherein the sequence of the first strand is the sequence of a first strand of: AD-39861.1 (SEQ ID NO: 724), or a modified or unmodified variant thereof.

A RNAi agent comprising a first and a second strand, wherein the sequence of the first strand is the sequence of a first strand of: AD-39867.1 (SEQ ID NO: 725), or a modified or unmodified variant thereof.

A RNAi agent comprising a first and a second strand, wherein the sequence of the first strand is the sequence of a first strand of: AD-39826.1 (SEQ ID NO: 726), or a modified or unmodified variant thereof.

A RNAi agent comprising a first and a second strand, wherein the sequence of the first strand is the sequence of a first strand of: AD-39832.1 (SEQ ID NO: 727), or a modified or unmodified variant thereof.

A RNAi agent comprising a first and a second strand, wherein the sequence of the first strand is the sequence of a first strand of: AD-39838.1 (SEQ ID NO: 728), or a modified or unmodified variant thereof.

A RNAi agent comprising a first and a second strand, wherein the sequence of the first strand is the sequence of a first strand of: AD-39844.1 (SEQ ID NO: 729), or a modified or unmodified variant thereof.

A RNAi agent comprising a first and a second strand, wherein the sequence of the first strand is the sequence of a first strand of: AD-39850.1 (SEQ ID NO: 730), or a modified or unmodified variant thereof.

A RNAi agent comprising a first and a second strand, wherein the sequence of the first strand is the sequence of a first strand of: AD-39856.1 (SEQ ID NO: 731), or a modified or unmodified variant thereof.

A RNAi agent comprising a first and a second strand, wherein the sequence of the first strand is the sequence of a first strand of: AD-39862.1 (SEQ ID NO: 732), or a modified or unmodified variant thereof.

A RNAi agent comprising a first and a second strand, wherein the sequence of the first strand is the sequence of a first strand of: AD-39868.1 (SEQ ID NO: 733), or a modified or unmodified variant thereof.

A RNAi agent comprising a first and a second strand, wherein the sequence of the first strand is the sequence of a first strand of: AD-39827.1 (SEQ ID NO: 734), or a modified or unmodified variant thereof.

A RNAi agent comprising a first and a second strand, wherein the sequence of the first strand is the sequence of a first strand of: AD-39833.1 (SEQ ID NO: 735), or a modified or unmodified variant thereof.

A RNAi agent comprising a first and a second strand, wherein the sequence of the first strand is the sequence of a first strand of: AD-39839.1 (SEQ ID NO: 736), or a modified or unmodified variant thereof.

A RNAi agent comprising a first and a second strand, wherein the sequence of the first strand is the sequence of a first strand of: AD-39845.1 (SEQ ID NO: 737), or a modified or unmodified variant thereof.

A RNAi agent comprising a first and a second strand, wherein the sequence of the first strand is the sequence of a first strand of: AD-39851.1 (SEQ ID NO: 738), or a modified or unmodified variant thereof.

A RNAi agent comprising a first and a second strand, wherein the sequence of the first strand is the sequence of a first strand of: AD-39857.1 (SEQ ID NO: 739), or a modified or unmodified variant thereof.

A RNAi agent comprising a first and a second strand, wherein the sequence of the first strand is the sequence of a first strand of: AD-39863.1 (SEQ ID NO: 740), or a modified or unmodified variant thereof.

A RNAi agent comprising a first and a second strand, wherein the sequence of the first strand is the sequence of a first strand of: AD-39869.1 (SEQ ID NO: 741), or a modified or unmodified variant thereof.

A RNAi agent comprising a first and a second strand, wherein the sequence of the first strand is the sequence of a first strand of: AD-39828.1 (SEQ ID NO: 742), or a modified or unmodified variant thereof.

A RNAi agent comprising a first and a second strand, wherein the sequence of the first strand is the sequence of a first strand of: AD-39834.1 (SEQ ID NO: 743), or a modified or unmodified variant thereof.

A RNAi agent comprising a first and a second strand, wherein the sequence of the first strand is the sequence of a first strand of: AD-39840.1 (SEQ ID NO: 744), or a modified or unmodified variant thereof.

A RNAi agent comprising a first and a second strand, wherein the sequence of the first strand is the sequence of a first strand of: AD-39846.1 (SEQ ID NO: 745), or a modified or unmodified variant thereof.

A RNAi agent comprising a first and a second strand, wherein the sequence of the first strand is the sequence of a first strand of: AD-39852.1 (SEQ ID NO: 746), or a modified or unmodified variant thereof.

A RNAi agent comprising a first and a second strand, wherein the sequence of the first strand is the sequence of a first strand of: AD-39858.1 (SEQ ID NO: 747), or a modified or unmodified variant thereof.

A RNAi agent comprising a first and a second strand, wherein the sequence of the first strand is the sequence of a first strand of: AD-39864.1 (SEQ ID NO: 748), or a modified or unmodified variant thereof.

A RNAi agent comprising a first and a second strand, wherein the sequence of the first strand is the sequence of a first strand of: AD-39870.1 (SEQ ID NO: 749), or a modified or unmodified variant thereof.

A RNAi agent comprising a first and a second strand, wherein the sequence of the first strand is the sequence of a first strand of: AD-39829.1 (SEQ ID NO: 750), or a modified or unmodified variant thereof.

A RNAi agent comprising a first and a second strand, wherein the sequence of the first strand is the sequence of a first strand of: AD-39835.1 (SEQ ID NO: 751), or a modified or unmodified variant thereof.

A RNAi agent comprising a first and a second strand, wherein the sequence of the first strand is the sequence of a first strand of: AD-39841.1 (SEQ ID NO: 752), or a modified or unmodified variant thereof.

A RNAi agent comprising a first and a second strand, wherein the sequence of the first strand is the sequence of a first strand of: AD-39853.1 (SEQ ID NO: 753), or a modified or unmodified variant thereof.

A RNAi agent comprising a first and a second strand, wherein the sequence of the first strand is the sequence of a first strand of: AD-39859.1 (SEQ ID NO: 754), or a modified or unmodified variant thereof.

A RNAi agent comprising a first and a second strand, wherein the sequence of the first strand is the sequence of a first strand of: AD-39865.1 (SEQ ID NO: 755), or a modified or unmodified variant thereof.

A RNAi agent comprising a first and a second strand, wherein the sequence of the first strand is the sequence of a first strand of: AD-39871.1 (SEQ ID NO: 756), or a modified or unmodified variant thereof.

A RNAi agent comprising a first and a second strand, wherein the sequence of the first strand is the sequence of a first strand of: AD-39830.1 (SEQ ID NO: 757), or a modified or unmodified variant thereof.

A RNAi agent comprising a first and a second strand, wherein the sequence of the first strand is the sequence of a first strand of: AD-39836.1 (SEQ ID NO: 758), or a modified or unmodified variant thereof.

A RNAi agent comprising a first and a second strand, wherein the sequence of the first strand is the sequence of a first strand of: AD-39842.1 (SEQ ID NO: 759), or a modified or unmodified variant thereof.

A RNAi agent comprising a first and a second strand, wherein the sequence of the first strand is the sequence of a first strand of: AD-39848.1 (SEQ ID NO: 760), or a modified or unmodified variant thereof.

A RNAi agent comprising a first and a second strand, wherein the sequence of the first strand is the sequence of a first strand of: AD-39854.1 (SEQ ID NO: 761), or a modified or unmodified variant thereof.

A RNAi agent comprising a first and a second strand, wherein the sequence of the first strand is the sequence of a first strand of: AD-39860.1 (SEQ ID NO: 762), or a modified or unmodified variant thereof.

A RNAi agent comprising a first and a second strand, wherein the sequence of the first strand is the sequence of a first strand of: AD-39866.1 (SEQ ID NO: 763), or a modified or unmodified variant thereof.

A RNAi agent comprising a first and a second strand, wherein the sequence of the first strand is the sequence of a first strand of: AD-39992.1 (SEQ ID NO: 764), or a modified or unmodified variant thereof.

A RNAi agent comprising a first and a second strand, wherein the sequence of the first strand is the sequence of a first strand of: AD-39998.1 (SEQ ID NO: 765), or a modified or unmodified variant thereof.

A RNAi agent comprising a first and a second strand, wherein the sequence of the first strand is the sequence of a first strand of: AD-40004.1 (SEQ ID NO: 766), or a modified or unmodified variant thereof.

A RNAi agent comprising a first and a second strand, wherein the sequence of the first strand is the sequence of a first strand of: AD-40010.1 (SEQ ID NO: 767), or a modified or unmodified variant thereof.

A RNAi agent comprising a first and a second strand, wherein the sequence of the first strand is the sequence of a first strand of: AD-40016.1 (SEQ ID NO: 768), or a modified or unmodified variant thereof.

A RNAi agent comprising a first and a second strand, wherein the sequence of the first strand is the sequence of a first strand of: AD-40022.1 (SEQ ID NO: 769), or a modified or unmodified variant thereof.

A RNAi agent comprising a first and a second strand, wherein the sequence of the first strand is the sequence of a first strand of: AD-40028.1 (SEQ ID NO: 770), or a modified or unmodified variant thereof.

A RNAi agent comprising a first and a second strand, wherein the sequence of the first strand is the sequence of a first strand of: AD-40034.1 (SEQ ID NO: 771), or a modified or unmodified variant thereof.

A RNAi agent comprising a first and a second strand, wherein the sequence of the first strand is the sequence of a first strand of: AD-39999.1 (SEQ ID NO: 772), or a modified or unmodified variant thereof.

A RNAi agent comprising a first and a second strand, wherein the sequence of the first strand is the sequence of a first strand of: AD-40005.1 (SEQ ID NO: 773), or a modified or unmodified variant thereof.

A RNAi agent comprising a first and a second strand, wherein the sequence of the first strand is the sequence of a first strand of: AD-40011.1 (SEQ ID NO: 774), or a modified or unmodified variant thereof.

A RNAi agent comprising a first and a second strand, wherein the sequence of the first strand is the sequence of a first strand of: AD-40017.1 (SEQ ID NO: 775), or a modified or unmodified variant thereof.

A RNAi agent comprising a first and a second strand, wherein the sequence of the first strand is the sequence of a first strand of: AD-40029.1 (SEQ ID NO: 776), or a modified or unmodified variant thereof.

A RNAi agent comprising a first and a second strand, wherein the sequence of the first strand is the sequence of a first strand of: AD-40035.1 (SEQ ID NO: 777), or a modified or unmodified variant thereof.

A RNAi agent comprising a first and a second strand, wherein the sequence of the first strand is the sequence of a first strand of: AD-39994.1 (SEQ ID NO: 778), or a modified or unmodified variant thereof.

A RNAi agent comprising a first and a second strand, wherein the sequence of the first strand is the sequence of a first strand of: AD-40000.1 (SEQ ID NO: 779), or a modified or unmodified variant thereof.

A RNAi agent comprising a first and a second strand, wherein the sequence of the first strand is the sequence of a first strand of: AD-40006.1 (SEQ ID NO: 780), or a modified or unmodified variant thereof.

A RNAi agent comprising a first and a second strand, wherein the sequence of the first strand is the sequence of a first strand of: AD-40012.1 (SEQ ID NO: 781), or a modified or unmodified variant thereof.

A RNAi agent comprising a first and a second strand, wherein the sequence of the first strand is the sequence of a first strand of: AD-40018.1 (SEQ ID NO: 782), or a modified or unmodified variant thereof.

A RNAi agent comprising a first and a second strand, wherein the sequence of the first strand is the sequence of a first strand of: AD-40024.1 (SEQ ID NO: 783), or a modified or unmodified variant thereof.

A RNAi agent comprising a first and a second strand, wherein the sequence of the first strand is the sequence of a first strand of: AD-40030.1 (SEQ ID NO: 784), or a modified or unmodified variant thereof.

A RNAi agent comprising a first and a second strand, wherein the sequence of the first strand is the sequence of a first strand of: AD-40036.1 (SEQ ID NO: 785), or a modified or unmodified variant thereof.

A RNAi agent comprising a first and a second strand, wherein the sequence of the first strand is the sequence of a first strand of: AD-39995.1 (SEQ ID NO: 786), or a modified or unmodified variant thereof.

A RNAi agent comprising a first and a second strand, wherein the sequence of the first strand is the sequence of a first strand of: AD-40001.1 (SEQ ID NO: 787), or a modified or unmodified variant thereof.

A RNAi agent comprising a first and a second strand, wherein the sequence of the first strand is the sequence of a first strand of: AD-40007.1 (SEQ ID NO: 788), or a modified or unmodified variant thereof.

A RNAi agent comprising a first and a second strand, wherein the sequence of the first strand is the sequence of a first strand of: AD-40013.1 (SEQ ID NO: 789), or a modified or unmodified variant thereof.

A RNAi agent comprising a first and a second strand, wherein the sequence of the first strand is the sequence of a first strand of: AD-40019.1 (SEQ ID NO: 790), or a modified or unmodified variant thereof.

A RNAi agent comprising a first and a second strand, wherein the sequence of the first strand is the sequence of a first strand of: AD-40025.1 (SEQ ID NO: 791), or a modified or unmodified variant thereof.

A RNAi agent comprising a first and a second strand, wherein the sequence of the first strand is the sequence of a first strand of: AD-40031.1 (SEQ ID NO: 792), or a modified or unmodified variant thereof.

A RNAi agent comprising a first and a second strand, wherein the sequence of the first strand is the sequence of a first strand of: AD-40037.1 (SEQ ID NO: 793), or a modified or unmodified variant thereof.

A RNAi agent comprising a first and a second strand, wherein the sequence of the first strand is the sequence of a first strand of: AD-39996.1 (SEQ ID NO: 794), or a modified or unmodified variant thereof.

A RNAi agent comprising a first and a second strand, wherein the sequence of the first strand is the sequence of a first strand of: AD-40002.1 (SEQ ID NO: 795), or a modified or unmodified variant thereof.

A RNAi agent comprising a first and a second strand, wherein the sequence of the first strand is the sequence of a first strand of: AD-40008.1 (SEQ ID NO: 796), or a modified or unmodified variant thereof.

A RNAi agent comprising a first and a second strand, wherein the sequence of the first strand is the sequence of a first strand of: AD-40014.1 (SEQ ID NO: 797), or a modified or unmodified variant thereof.

A RNAi agent comprising a first and a second strand, wherein the sequence of the first strand is the sequence of a first strand of: AD-40020.1 (SEQ ID NO: 798), or a modified or unmodified variant thereof.

A RNAi agent comprising a first and a second strand, wherein the sequence of the first strand is the sequence of a first strand of: AD-40026.1 (SEQ ID NO: 799), or a modified or unmodified variant thereof.

A RNAi agent comprising a first and a second strand, wherein the sequence of the first strand is the sequence of a first strand of: AD-40032.1 (SEQ ID NO: 800), or a modified or unmodified variant thereof.

A RNAi agent comprising a first and a second strand, wherein the sequence of the first strand is the sequence of a first strand of: AD-40038.1 (SEQ ID NO: 801), or a modified or unmodified variant thereof.

A RNAi agent comprising a first and a second strand, wherein the sequence of the first strand is the sequence of a first strand of: AD-39997.1 (SEQ ID NO: 802), or a modified or unmodified variant thereof.

A RNAi agent comprising a first and a second strand, wherein the sequence of the first strand is the sequence of a first strand of: AD-40009.1 (SEQ ID NO: 803), or a modified or unmodified variant thereof.

A RNAi agent comprising a first and a second strand, wherein the sequence of the first strand is the sequence of a first strand of: AD-40015.1 (SEQ ID NO: 804), or a modified or unmodified variant thereof.

A RNAi agent comprising a first and a second strand, wherein the sequence of the first strand is the sequence of a first strand of: AD-40021.1 (SEQ ID NO: 805), or a modified or unmodified variant thereof.

A RNAi agent comprising a first and a second strand, wherein the sequence of the first strand is the sequence of a first strand of: AD-40027.1 (SEQ ID NO: 806), or a modified or unmodified variant thereof.

A RNAi agent comprising a first and a second strand, wherein the sequence of the first strand is the sequence of a first strand of: AD-40033.1 (SEQ ID NO: 807), or a modified or unmodified variant thereof.

A RNAi agent comprising a first and a second strand, wherein the sequence of the first strand is the sequence of a first strand of: AD-40039.1 (SEQ ID NO: 808), or a modified or unmodified variant thereof.

A RNAi agent comprising a first and a second strand, wherein the sequence of the first strand is the sequence of a first strand of: AD-40045.1 (SEQ ID NO: 809), or a modified or unmodified variant thereof.

A RNAi agent comprising a first and a second strand, wherein the sequence of the first strand is the sequence of a first strand of: AD-40051.1 (SEQ ID NO: 810), or a modified or unmodified variant thereof.

A RNAi agent comprising a first and a second strand, wherein the sequence of the first strand is the sequence of a first strand of: AD-40057.1 (SEQ ID NO: 811), or a modified or unmodified variant thereof.

A RNAi agent comprising a first and a second strand, wherein the sequence of the first strand is the sequence of a first strand of: AD-40063.1 (SEQ ID NO: 812), or a modified or unmodified variant thereof.

A RNAi agent comprising a first and a second strand, wherein the sequence of the first strand is the sequence of a first strand of: AD-40069.1 (SEQ ID NO: 813), or a modified or unmodified variant thereof.

A RNAi agent comprising a first and a second strand, wherein the sequence of the first strand is the sequence of a first strand of: AD-40075.1 (SEQ ID NO: 814), or a modified or unmodified variant thereof.

A RNAi agent comprising a first and a second strand, wherein the sequence of the first strand is the sequence of a first strand of: AD-40081.1 (SEQ ID NO: 815), or a modified or unmodified variant thereof.

A RNAi agent comprising a first and a second strand, wherein the sequence of the first strand is the sequence of a first strand of: AD-40040.1 (SEQ ID NO: 816), or a modified or unmodified variant thereof.

A RNAi agent comprising a first and a second strand, wherein the sequence of the first strand is the sequence of a first strand of: AD-40046.1 (SEQ ID NO: 817), or a modified or unmodified variant thereof.

A RNAi agent comprising a first and a second strand, wherein the sequence of the first strand is the sequence of a first strand of: AD-40052.1 (SEQ ID NO: 818), or a modified or unmodified variant thereof.

A RNAi agent comprising a first and a second strand, wherein the sequence of the first strand is the sequence of a first strand of: AD-40058.1 (SEQ ID NO: 819), or a modified or unmodified variant thereof.

A RNAi agent comprising a first and a second strand, wherein the sequence of the first strand is the sequence of a first strand of: AD-40064.1 (SEQ ID NO: 820), or a modified or unmodified variant thereof.

A RNAi agent comprising a first and a second strand, wherein the sequence of the first strand is the sequence of a first strand of: AD-40070.1 (SEQ ID NO: 821), or a modified or unmodified variant thereof.

A RNAi agent comprising a first and a second strand, wherein the sequence of the first strand is the sequence of a first strand of: AD-40076.1 (SEQ ID NO: 822), or a modified or unmodified variant thereof.

A RNAi agent comprising a first and a second strand, wherein the sequence of the first strand is the sequence of a first strand of: AD-40082.1 (SEQ ID NO: 823), or a modified or unmodified variant thereof.

A RNAi agent comprising a first and a second strand, wherein the sequence of the first strand is the sequence of a first strand of: AD-40041.1 (SEQ ID NO: 824), or a modified or unmodified variant thereof.

A RNAi agent comprising a first and a second strand, wherein the sequence of the first strand is the sequence of a first strand of: AD-40047.1 (SEQ ID NO: 825), or a modified or unmodified variant thereof.

A RNAi agent comprising a first and a second strand, wherein the sequence of the first strand is the sequence of a first strand of: AD-40053.1 (SEQ ID NO: 826), or a modified or unmodified variant thereof.

A RNAi agent comprising a first and a second strand, wherein the sequence of the first strand is the sequence of a first strand of: AD-40059.1 (SEQ ID NO: 827), or a modified or unmodified variant thereof.

A RNAi agent comprising a first and a second strand, wherein the sequence of the first strand is the sequence of a first strand of: AD-40065.1 (SEQ ID NO: 828), or a modified or unmodified variant thereof.

A RNAi agent comprising a first and a second strand, wherein the sequence of the first strand is the sequence of a first strand of: AD-40071.1 (SEQ ID NO: 829), or a modified or unmodified variant thereof.

A RNAi agent comprising a first and a second strand, wherein the sequence of the first strand is the sequence of a first strand of: AD-40077.1 (SEQ ID NO: 830), or a modified or unmodified variant thereof.

A RNAi agent comprising a first and a second strand, wherein the sequence of the first strand is the sequence of a first strand of: AD-40083.1 (SEQ ID NO: 831), or a modified or unmodified variant thereof.

A RNAi agent comprising a first and a second strand, wherein the sequence of the first strand is the sequence of a first strand of: AD-40042.1 (SEQ ID NO: 832), or a modified or unmodified variant thereof.
A RNAi agent comprising a first and a second strand, wherein the sequence of the first strand is the sequence of a first strand of: AD-40048.1 (SEQ ID NO: 833), or a modified or unmodified variant thereof.
A RNAi agent comprising a first and a second strand, wherein the sequence of the first strand is the sequence of a first strand of: AD-40054.1 (SEQ ID NO: 834), or a modified or unmodified variant thereof.
A RNAi agent comprising a first and a second strand, wherein the sequence of the first strand is the sequence of a first strand of: AD-40060.1 (SEQ ID NO: 835), or a modified or unmodified variant thereof.
A RNAi agent comprising a first and a second strand, wherein the sequence of the first strand is the sequence of a first strand of: AD-40066.1 (SEQ ID NO: 836), or a modified or unmodified variant thereof.
A RNAi agent comprising a first and a second strand, wherein the sequence of the first strand is the sequence of a first strand of: AD-40072.1 (SEQ ID NO: 837), or a modified or unmodified variant thereof.
A RNAi agent comprising a first and a second strand, wherein the sequence of the first strand is the sequence of a first strand of: AD-40078.1 (SEQ ID NO: 838), or a modified or unmodified variant thereof.
A RNAi agent comprising a first and a second strand, wherein the sequence of the first strand is the sequence of a first strand of: AD-40084.1 (SEQ ID NO: 839), or a modified or unmodified variant thereof.
A RNAi agent comprising a first and a second strand, wherein the sequence of the first strand is the sequence of a first strand of: AD-40043.1 (SEQ ID NO: 840), or a modified or unmodified variant thereof.
A RNAi agent comprising a first and a second strand, wherein the sequence of the first strand is the sequence of a first strand of: AD-40049.1 (SEQ ID NO: 841), or a modified or unmodified variant thereof.
A RNAi agent comprising a first and a second strand, wherein the sequence of the first strand is the sequence of a first strand of: AD-40055.1 (SEQ ID NO: 842), or a modified or unmodified variant thereof.
A RNAi agent comprising a first and a second strand, wherein the sequence of the first strand is the sequence of a first strand of: AD-40061.1 (SEQ ID NO: 843), or a modified or unmodified variant thereof.
A RNAi agent comprising a first and a second strand, wherein the sequence of the first strand is the sequence of a first strand of: AD-40067.1 (SEQ ID NO: 844), or a modified or unmodified variant thereof.
A RNAi agent comprising a first and a second strand, wherein the sequence of the first strand is the sequence of a first strand of: AD-40073.1 (SEQ ID NO: 845), or a modified or unmodified variant thereof.
A RNAi agent comprising a first and a second strand, wherein the sequence of the first strand is the sequence of a first strand of: AD-40079.1 (SEQ ID NO: 846), or a modified or unmodified variant thereof.
A RNAi agent comprising a first and a second strand, wherein the sequence of the first strand is the sequence of a first strand of: AD-40085.1 (SEQ ID NO: 847), or a modified or unmodified variant thereof.
A RNAi agent comprising a first and a second strand, wherein the sequence of the first strand is the sequence of a first strand of: AD-40044.1 (SEQ ID NO: 848), or a modified or unmodified variant thereof.
A RNAi agent comprising a first and a second strand, wherein the sequence of the first strand is the sequence of a first strand of: AD-40050.1 (SEQ ID NO: 849), or a modified or unmodified variant thereof.
A RNAi agent comprising a first and a second strand, wherein the sequence of the first strand is the sequence of a first strand of: AD-40056.1 (SEQ ID NO: 850), or a modified or unmodified variant thereof.
A RNAi agent comprising a first and a second strand, wherein the sequence of the first strand is the sequence of a first strand of: AD-40062.1 (SEQ ID NO: 851), or a modified or unmodified variant thereof.
A RNAi agent comprising a first and a second strand, wherein the sequence of the first strand is the sequence of a first strand of: AD-40068.1 (SEQ ID NO: 852), or a modified or unmodified variant thereof.
A RNAi agent comprising a first and a second strand, wherein the sequence of the first strand is the sequence of a first strand of: AD-40074.1 (SEQ ID NO: 853), or a modified or unmodified variant thereof.
A RNAi agent comprising a first and a second strand, wherein the sequence of the first strand is the sequence of a first strand of: AD-40080.1 (SEQ ID NO: 854), or a modified or unmodified variant thereof.
A RNAi agent comprising a first and a second strand, wherein the sequence of the first strand is the sequence of a first strand of: AD-39720.1 (SEQ ID NOs: 1716 and 1754), or a modified or unmodified variant thereof.
A RNAi agent comprising a first and a second strand, wherein the sequence of the first strand is the sequence of a first strand of: AD-39706.1 (SEQ ID NOs: 1717 and 1755), or a modified or unmodified variant thereof.
A RNAi agent comprising a first and a second strand, wherein the sequence of the first strand is the sequence of a first strand of: AD-39712.1 (SEQ ID NOs: 1718 and 1756), or a modified or unmodified variant thereof.
A RNAi agent comprising a first and a second strand, wherein the sequence of the first strand is the sequence of a first strand of: AD-39760.1 (SEQ ID NOs: 1719 and 1757), or a modified or unmodified variant thereof.
A RNAi agent comprising a first and a second strand, wherein the sequence of the first strand is the sequence of a first strand of: AD-39732.1 (SEQ ID NOs: 1720 and 1758), or a modified or unmodified variant thereof.
A RNAi agent comprising a first and a second strand, wherein the sequence of the first strand is the sequence of a first strand of: AD-35541.4 (SEQ ID NOs: 1721 and 1759), or a modified or unmodified variant thereof.

A RNAi agent comprising a first and a second strand, wherein the sequence of the first strand is the sequence of a first strand of: AD-39735.1 or hs_KRAS_321_A22S26 (SEQ ID NOs: 1722 and 1760), or a modified or unmodified variant thereof.

A RNAi agent comprising a first and a second strand, wherein the sequence of the first strand is the sequence of a first strand of: AD-39741.1, or modified or unmodified variants thereof.

A RNAi agent comprising a first and a second strand, wherein the sequence of the first strand is the sequence of a first strand of: hs_KRAS_322_A22S26 (SEQ ID NOs: 1723 and 1761), or a modified or unmodified variant thereof.

A RNAi agent comprising a first and a second strand, wherein the sequence of the first strand is the sequence of a first strand of: AD-39778.1 (SEQ ID NOs: 1724 and 1762), or a modified or unmodified variant thereof.

A RNAi agent comprising a first and a second strand, wherein the sequence of the first strand is the sequence of a first strand of: AD-39790.1 (SEQ ID NOs: 1725 and 1763), or a modified or unmodified variant thereof.

A RNAi agent comprising a first and a second strand, wherein the sequence of the first strand is the sequence of a first strand of: AD-39822.1 (SEQ ID NOs: 1726 and 1764), or a modified or unmodified variant thereof.

A RNAi agent comprising a first and a second strand, wherein the sequence of the first strand is the sequence of a first strand of: AD-35609.4 (SEQ ID NOs: 1727 and 1765), or a modified or unmodified variant thereof.

A RNAi agent comprising a first and a second strand, wherein the sequence of the first strand is the sequence of a first strand of: AD-35581.4 (SEQ ID NOs: 1728 and 1766), or a modified or unmodified variant thereof.

A RNAi agent comprising a first and a second strand, wherein the sequence of the first strand is the sequence of a first strand of: AD-39845.1 (SEQ ID NOs: 1729 and 1767), or a modified or unmodified variant thereof.

A RNAi agent comprising a first and a second strand, wherein the sequence of the first strand is the sequence of a first strand of: AD-39858.1 (SEQ ID NOs: 1730 and 1768), or a modified or unmodified variant thereof.

A RNAi agent comprising a first and a second strand, wherein the sequence of the first strand is the sequence of a first strand of: AD-39870.1 (SEQ ID NOs: 1731 and 1769), or a modified or unmodified variant thereof.

A RNAi agent comprising a first and a second strand, wherein the sequence of the first strand is the sequence of a first strand of: AD-35576.4 (SEQ ID NOs: 1732 and 1770), or a modified or unmodified variant thereof.

A RNAi agent comprising a first and a second strand, wherein the sequence of the first strand is the sequence of a first strand of: AD-35588.4 (SEQ ID NOs: 1733 and 1771), or a modified or unmodified variant thereof.

A RNAi agent comprising a first and a second strand, wherein the sequence of the first strand is the sequence of a first strand of: AD-35600.1 (SEQ ID NOs: 1734 and 1772), or a modified or unmodified variant thereof.

A RNAi agent comprising a first and a second strand, wherein the sequence of the first strand is the sequence of a first strand of: AD-35606.1 (SEQ ID NOs: 1735 and 1773), or a modified or unmodified variant thereof.

A RNAi agent comprising a first and a second strand, wherein the sequence of the first strand is the sequence of a first strand of: AD-38151.1 (SEQ ID NOs: 1736 and 1774), or a modified or unmodified variant thereof.

A RNAi agent comprising a first and a second strand, wherein the sequence of the first strand is the sequence of a first strand of: AD-38163.1 (SEQ ID NOs: 1737 and 1775), or a modified or unmodified variant thereof.

A RNAi agent comprising a first and a second strand, wherein the sequence of the first strand is the sequence of a first strand of: AD-39999.1 (SEQ ID NOs: 1738 and 1776), or a modified or unmodified variant thereof.

A RNAi agent comprising a first and a second strand, wherein the sequence of the first strand is the sequence of a first strand of: AD-38159.1 or hs_KRAS_528_A22S26 (SEQ ID NOs: 1739 and 1777), or a modified or unmodified variant thereof.

A RNAi agent comprising a first and a second strand, wherein the sequence of the first strand is the sequence of a first strand of: AD-38130.1 or hs_KRAS_531_A22S26 (SEQ ID NOs: 1740 and 1778), or a modified or unmodified variant thereof.

A RNAi agent comprising a first and a second strand, wherein the sequence of the first strand is the sequence of a first strand of: AD-38136.1 (SEQ ID NOs: 1741 and 1779), or a modified or unmodified variant thereof.

A RNAi agent comprising a first and a second strand, wherein the sequence of the first strand is the sequence of a first strand of: AD-40036.1 (SEQ ID NOs: 1742 and 1780), or a modified or unmodified variant thereof.

A RNAi agent comprising a first and a second strand, wherein the sequence of the first strand is the sequence of a first strand of: AD-40008.1 (SEQ ID NOs: 1743 and 1781), or a modified or unmodified variant thereof.

A RNAi agent comprising a first and a second strand, wherein the sequence of the first strand is the sequence of a first strand of: AD-40021.1 (SEQ ID NOs: 1744 and 1782), or a modified or unmodified variant thereof.

A RNAi agent comprising a first and a second strand, wherein the sequence of the first strand is the sequence of a first strand of: AD-40077.1 (SEQ ID NOs: 1745 and 1783), or a modified or unmodified variant thereof.

A RNAi agent comprising a first and a second strand, wherein the sequence of the first strand is the sequence of a first strand of: AD-40072.1 (SEQ ID NOs: 1746 and 1784), or a modified or unmodified variant thereof.

A RNAi agent comprising a first and a second strand, wherein the sequence of the first strand is the sequence of a first strand of: AD-40061.1 (SEQ ID NOs: 1747 and 1785), or a modified or unmodified variant thereof.

A RNAi agent comprising a first and a second strand, wherein the sequence of the first strand is the sequence of a first strand of: AD-40068.1 (SEQ ID NOs: 1748 and 1786), or a modified or unmodified variant thereof.

A RNAi agent comprising a first and a second strand, wherein the sequence of the first strand is the sequence of a first strand of: AD-38131.1 (SEQ ID NOs: 1749 and 1787), or a modified or unmodified variant thereof.

A RNAi agent comprising a first and a second strand, wherein the sequence of the first strand is the sequence of a first strand of: AD-38167.1 (SEQ ID NOs: 1750 and 1788), or a modified or unmodified variant thereof.

A RNAi agent comprising a first and a second strand, wherein the sequence of the first strand is the sequence of a first strand of: hs_KRAS 1273_A22S26 (SEQ ID NOs: 1751 and 1789), or a modified or unmodified variant thereof.

A RNAi agent comprising a first and a second strand, wherein the sequence of the first strand is the sequence of a first strand of: hs_KRAS 2892_A37S26 (SEQ ID NOs: 1752 and 1790), or a modified or unmodified variant thereof.

A RNAi agent comprising a first and a second strand, wherein the sequence of the first strand is the sequence of a first strand of: hs_KRAS 4731_A22S26 (SEQ ID NOs: 1753 and 1791), or a modified or unmodified variant thereof.

**Specific embodiments comprising the first and/or second strands of a RNAi agent disclosed herein**

In one embodiment, the present disclosure pertains to a composition comprising any one or more of: a RNAi agent comprising a first and a second strand, wherein the sequences of the first and/or second strands comprise the sequences of a first and/or second strand of any RNAi agent from any of Tables 1 to 6, or modified and unmodified variants thereof.

Thus, in various embodiments, the present disclosure pertains to a composition comprising any one or more of the following:

A RNAi agent comprising a first and a second strand, wherein the sequences of the first and/or second strands comprise the sequences of a first and/or second strand of: AD-35523.3 (SEQ ID NOs: 1 and 428), or a modified or unmodified variant thereof.

A RNAi agent comprising a first and a second strand, wherein the sequences of the first and/or second strands comprise the sequences of a first and/or second strand of: AD-35529.1 (SEQ ID NOs: 2 and 429), or a modified or unmodified variant thereof.

A RNAi agent comprising a first and a second strand, wherein the sequences of the first and/or second strands comprise the sequences of a first and/or second strand of: AD-35535.4 (SEQ ID NOs: 3 and 430), or a modified or unmodified variant thereof.

A RNAi agent comprising a first and a second strand, wherein the sequences of the first and/or second strands comprise the sequences of a first and/or second strand of: AD-35541.4 (SEQ ID NOs: 4 and 431), or a modified or unmodified variant thereof.

A RNAi agent comprising a first and a second strand, wherein the sequences of the first and/or second strands comprise the sequences of a first and/or second strand of: AD-35547.4 (SEQ ID NOs: 5 and 432), or a modified or unmodified variant thereof.

A RNAi agent comprising a first and a second strand, wherein the sequences of the first and/or second strands comprise the sequences of a first and/or second strand of: AD-35553.4 (SEQ ID NOs: 6 and 433), or a modified or unmodified variant thereof.

A RNAi agent comprising a first and a second strand, wherein the sequences of the first and/or second strands comprise the sequences of a first and/or second strand of: AD-35559.4 (SEQ ID NOs: 7 and 434), or a modified or unmodified variant thereof.

A RNAi agent comprising a first and a second strand, wherein the sequences of the first and/or second strands comprise the sequences of a first and/or second strand of: AD-35565.4 (SEQ ID NOs: 8 and 435), or a modified or unmodified variant thereof.

A RNAi agent comprising a first and a second strand, wherein the sequences of the first and/or second strands comprise the sequences of a first and/or second strand of: AD-35524.4 (SEQ ID NOs: 9 and 436), or a modified or unmodified variant thereof.

A RNAi agent comprising a first and a second strand, wherein the sequences of the first and/or second strands comprise the sequences of a first and/or second strand of: AD-35530.4 (SEQ ID NOs: 10 and 437), or a modified or unmodified variant thereof.

A RNAi agent comprising a first and a second strand, wherein the sequences of the first and/or second strands comprise the sequences of a first and/or second strand of: AD-35536.4 (SEQ ID NOs: 11 and 438), or a modified or unmodified variant thereof.

A RNAi agent comprising a first and a second strand, wherein the sequences of the first and/or second strands comprise the sequences of a first and/or second strand of: AD-35542.4 (SEQ ID NOs: 12 and 439), or a modified or unmodified variant thereof.

A RNAi agent comprising a first and a second strand, wherein the sequences of the first and/or second strands comprise the sequences of a first and/or second strand of: AD-35548.4 (SEQ ID NOs: 13 and 440), or a modified or unmodified variant thereof.

A RNAi agent comprising a first and a second strand, wherein the sequences of the first and/or second strands comprise the sequences of a first and/or second strand of: AD-35554.4 (SEQ ID NOs: 14 and 441), or a modified or unmodified variant thereof.

A RNAi agent comprising a first and a second strand, wherein the sequences of the first and/or second strands comprise the sequences of a first and/or second strand of: AD-35560.2 (SEQ ID NOs: 15 and 442), or a modified or unmodified variant thereof.

A RNAi agent comprising a first and a second strand, wherein the sequences of the first and/or second strands comprise the sequences of a first and/or second strand of: AD-35566.4 (SEQ ID NOs: 16 and 443), or a modified or unmodified variant thereof.

A RNAi agent comprising a first and a second strand, wherein the sequences of the first and/or second strands comprise the sequences of a first and/or second strand of: AD-35525.2 (SEQ ID NOs: 17 and 444), or a modified or unmodified variant thereof.

A RNAi agent comprising a first and a second strand, wherein the sequences of the first and/or second strands comprise the sequences of a first and/or second strand of: AD-35531.4 (SEQ ID NOs: 18 and 445), or a modified or unmodified variant thereof.

A RNAi agent comprising a first and a second strand, wherein the sequences of the first and/or second strands comprise the sequences of a first and/or second strand of: AD-35537.4 (SEQ ID NOs: 19 and 446), or a modified or unmodified variant thereof.

A RNAi agent comprising a first and a second strand, wherein the sequences of the first and/or second strands comprise the sequences of a first and/or second strand of: AD-35543.2 (SEQ ID NOs: 20 and 447), or a modified or unmodified variant thereof.

A RNAi agent comprising a first and a second strand, wherein the sequences of the first and/or second strands comprise the sequences of a first and/or second strand of: AD-35549.4 (SEQ ID NOs: 21 and 448), or a modified or unmodified variant thereof.

A RNAi agent comprising a first and a second strand, wherein the sequences of the first and/or second strands comprise the sequences of a first and/or second strand of: AD-35555.4 (SEQ ID NOs: 22 and 449), or a modified or unmodified variant thereof.

A RNAi agent comprising a first and a second strand, wherein the sequences of the first and/or second strands comprise the sequences of a first and/or second strand of: AD-35561.4 (SEQ ID NOs: 23 and 450), or a modified or unmodified variant thereof.

A RNAi agent comprising a first and a second strand, wherein the sequences of the first and/or second strands comprise the sequences of a first and/or second strand of: AD-35567.4 (SEQ ID NOs: 24 and 451), or a modified or unmodified variant thereof.

A RNAi agent comprising a first and a second strand, wherein the sequences of the first and/or second strands comprise the sequences of a first and/or second strand of: AD-35526.4 (SEQ ID NOs: 25 and 452), or a modified or unmodified variant thereof.

A RNAi agent comprising a first and a second strand, wherein the sequences of the first and/or second strands comprise the sequences of a first and/or second strand of: AD-35532.4 (SEQ ID NOs: 26 and 453), or a modified or unmodified variant thereof.

A RNAi agent comprising a first and a second strand, wherein the sequences of the first and/or second strands comprise the sequences of a first and/or second strand of: AD-35538.4 (SEQ ID NOs: 27 and 454), or a modified or unmodified variant thereof.

A RNAi agent comprising a first and a second strand, wherein the sequences of the first and/or second strands comprise the sequences of a first and/or second strand of: AD-35544.4 (SEQ ID NOs: 28 and 455), or a modified or unmodified variant thereof.

A RNAi agent comprising a first and a second strand, wherein the sequences of the first and/or second strands comprise the sequences of a first and/or second strand of: AD-35550.4 (SEQ ID NOs: 29 and 456), or a modified or unmodified variant thereof.

A RNAi agent comprising a first and a second strand, wherein the sequences of the first and/or second strands comprise the sequences of a first and/or second strand of: AD-35556.4 (SEQ ID NOs: 30 and 457), or a modified or unmodified variant thereof.

A RNAi agent comprising a first and a second strand, wherein the sequences of the first and/or second strands comprise the sequences of a first and/or second strand of: AD-35562.4 (SEQ ID NOs: 31 and 458), or a modified or unmodified variant thereof.

A RNAi agent comprising a first and a second strand, wherein the sequences of the first and/or second strands comprise the sequences of a first and/or second strand of: AD-35568.4 (SEQ ID NOs: 32 and 459), or a modified or unmodified variant thereof.

A RNAi agent comprising a first and a second strand, wherein the sequences of the first and/or second strands comprise the sequences of a first and/or second strand of: AD-35527.4 (SEQ ID NOs: 33 and 460), or a modified or unmodified variant thereof.

A RNAi agent comprising a first and a second strand, wherein the sequences of the first and/or second strands comprise the sequences of a first and/or second strand of: AD-35533.4 (SEQ ID NOs: 34 and 461), or a modified or unmodified variant thereof.

A RNAi agent comprising a first and a second strand, wherein the sequences of the first and/or second strands comprise the sequences of a first and/or second strand of: AD-35539.4 (SEQ ID NOs: 35 and 462), or a modified or unmodified variant thereof.

A RNAi agent comprising a first and a second strand, wherein the sequences of the first and/or second strands comprise the sequences of a first and/or second strand of: AD-35545.4 (SEQ ID NOs: 36 and 463), or a modified or unmodified variant thereof.

A RNAi agent comprising a first and a second strand, wherein the sequences of the first and/or second strands comprise the sequences of a first and/or second strand of: AD-35551.4 (SEQ ID NOs: 37 and 464), or a modified or unmodified variant thereof.

A RNAi agent comprising a first and a second strand, wherein the sequences of the first and/or second strands comprise the sequences of a first and/or second strand of: AD-35563.4 (SEQ ID NOs: 38 and 465), or a modified or unmodified variant thereof.

A RNAi agent comprising a first and a second strand, wherein the sequences of the first and/or second strands comprise the sequences of a first and/or second strand of: AD-35528.4 (SEQ ID NOs: 39 and 466), or a modified or unmodified variant thereof.

A RNAi agent comprising a first and a second strand, wherein the sequences of the first and/or second strands comprise the sequences of a first and/or second strand of: AD-35540.4 (SEQ ID NOs: 40 and 467), or a modified or unmodified variant thereof.

A RNAi agent comprising a first and a second strand, wherein the sequences of the first and/or second strands comprise the sequences of a first and/or second strand of: AD-35546.4 (SEQ ID NOs: 41 and 468), or a modified or unmodified variant thereof.

A RNAi agent comprising a first and a second strand, wherein the sequences of the first and/or second strands comprise the sequences of a first and/or second strand of: AD-35552.4 (SEQ ID NOs: 42 and 469), or a modified or unmodified variant thereof.

A RNAi agent comprising a first and a second strand, wherein the sequences of the first and/or second strands comprise the sequences of a first and/or second strand of: AD-35558.4 (SEQ ID NOs: 43 and 470), or a modified or unmodified variant thereof.

A RNAi agent comprising a first and a second strand, wherein the sequences of the first and/or second strands comprise the sequences of a first and/or second strand of: AD-35564.4 (SEQ ID NOs: 44 and 471), or a modified or unmodified variant thereof.

A RNAi agent comprising a first and a second strand, wherein the sequences of the first and/or second strands comprise the sequences of a first and/or second strand of: AD-35570.4 (SEQ ID NOs: 45 and 472), or a modified or unmodified variant thereof.

A RNAi agent comprising a first and a second strand, wherein the sequences of the first and/or second strands comprise the sequences of a first and/or second strand of: AD-35571.4 (SEQ ID NOs: 46 and 473), or a modified or unmodified variant thereof.

A RNAi agent comprising a first and a second strand, wherein the sequences of the first and/or second strands comprise the sequences of a first and/or second strand of: AD-35577.4 (SEQ ID NOs: 47 and 474), or a modified or unmodified variant thereof.

A RNAi agent comprising a first and a second strand, wherein the sequences of the first and/or second strands comprise the sequences of a first and/or second strand of: AD-35583.4 (SEQ ID NOs: 48 and 475), or a modified or unmodified variant thereof.

A RNAi agent comprising a first and a second strand, wherein the sequences of the first and/or second strands comprise the sequences of a first and/or second strand of: AD-35589.4 (SEQ ID NOs: 49 and 476), or a modified or unmodified variant thereof.

A RNAi agent comprising a first and a second strand, wherein the sequences of the first and/or second strands comprise the sequences of a first and/or second strand of: AD-35595.4 (SEQ ID NOs: 50 and 477), or a modified or unmodified variant thereof.

A RNAi agent comprising a first and a second strand, wherein the sequences of the first and/or second strands comprise the sequences of a first and/or second strand of: AD-35601.4 (SEQ ID NOs: 51 and 478), or a modified or unmodified variant thereof.

A RNAi agent comprising a first and a second strand, wherein the sequences of the first and/or second strands comprise the sequences of a first and/or second strand of: AD-35607.4 (SEQ ID NOs: 52 and 479), or a modified or unmodified variant thereof.

A RNAi agent comprising a first and a second strand, wherein the sequences of the first and/or second strands comprise the sequences of a first and/or second strand of: AD-35613.4 (SEQ ID NOs: 53 and 480), or a modified or unmodified variant thereof.

A RNAi agent comprising a first and a second strand, wherein the sequences of the first and/or second strands comprise the sequences of a first and/or second strand of: AD-35572.4 (SEQ ID NOs: 54 and 481), or a modified or unmodified variant thereof.

A RNAi agent comprising a first and a second strand, wherein the sequences of the first and/or second strands comprise the sequences of a first and/or second strand of: AD-35578.4 (SEQ ID NOs: 55 and 482), or a modified or unmodified variant thereof.

A RNAi agent comprising a first and a second strand, wherein the sequences of the first and/or second strands comprise the sequences of a first and/or second strand of: AD-35584.4 (SEQ ID NOs: 56 and 483), or a modified or unmodified variant thereof.

A RNAi agent comprising a first and a second strand, wherein the sequences of the first and/or second strands comprise the sequences of a first and/or second strand of: AD-35590.4 (SEQ ID NOs: 57 and 484), or a modified or unmodified variant thereof.

A RNAi agent comprising a first and a second strand, wherein the sequences of the first and/or second strands comprise the sequences of a first and/or second strand of: AD-35596.4 (SEQ ID NOs: 58 and 485), or a modified or unmodified variant thereof.

A RNAi agent comprising a first and a second strand, wherein the sequences of the first and/or second strands comprise the sequences of a first and/or second strand of: AD-35602.4 (SEQ ID NOs: 59 and 486), or a modified or unmodified variant thereof.

A RNAi agent comprising a first and a second strand, wherein the sequences of the first and/or second strands comprise the sequences of a first and/or second strand of: AD-35608.4 (SEQ ID NOs: 60 and 487), or a modified or unmodified variant thereof.

A RNAi agent comprising a first and a second strand, wherein the sequences of the first and/or second strands comprise the sequences of a first and/or second strand of: AD-35614.4 (SEQ ID NOs: 61 and 488), or a modified or unmodified variant thereof.

A RNAi agent comprising a first and a second strand, wherein the sequences of the first and/or second strands comprise the sequences of a first and/or second strand of: AD-35573.4 (SEQ ID NOs: 62 and 489), or a modified or unmodified variant thereof.

A RNAi agent comprising a first and a second strand, wherein the sequences of the first and/or second strands comprise the sequences of a first and/or second strand of: AD-35579.4 (SEQ ID NOs: 63 and 490), or a modified or unmodified variant thereof.

A RNAi agent comprising a first and a second strand, wherein the sequences of the first and/or second strands comprise the sequences of a first and/or second strand of: AD-35585.4 (SEQ ID NOs: 64 and 491), or a modified or unmodified variant thereof.

A RNAi agent comprising a first and a second strand, wherein the sequences of the first and/or second strands comprise the sequences of a first and/or second strand of: AD-35591.4 (SEQ ID NOs: 65 and 492), or a modified or unmodified variant thereof.

A RNAi agent comprising a first and a second strand, wherein the sequences of the first and/or second strands comprise the sequences of a first and/or second strand of: AD-35597.4 (SEQ ID NOs: 66 and 493), or a modified or unmodified variant thereof.

A RNAi agent comprising a first and a second strand, wherein the sequences of the first and/or second strands comprise the sequences of a first and/or second strand of: AD-35603.4 (SEQ ID NOs: 67 and 494), or a modified or unmodified variant thereof.

A RNAi agent comprising a first and a second strand, wherein the sequences of the first and/or second strands comprise the sequences of a first and/or second strand of: AD-35609.4 (SEQ ID NOs: 68 and 495), or a modified or unmodified variant thereof.

A RNAi agent comprising a first and a second strand, wherein the sequences of the first and/or second strands comprise the sequences of a first and/or second strand of: AD-35615.4 (SEQ ID NOs: 69 and 496), or a modified or unmodified variant thereof.

A RNAi agent comprising a first and a second strand, wherein the sequences of the first and/or second strands comprise the sequences of a first and/or second strand of: AD-35574.4 (SEQ ID NOs: 70 and 497), or a modified or unmodified variant thereof.

A RNAi agent comprising a first and a second strand, wherein the sequences of the first and/or second strands comprise the sequences of a first and/or second strand of: AD-35580.1 (SEQ ID NOs: 71 and 498), or a modified or unmodified variant thereof.

A RNAi agent comprising a first and a second strand, wherein the sequences of the first and/or second strands comprise the sequences of a first and/or second strand of: AD-35586.4 (SEQ ID NOs: 72 and 499), or a modified or unmodified variant thereof.

A RNAi agent comprising a first and a second strand, wherein the sequences of the first and/or second strands comprise the sequences of a first and/or second strand of: AD-35592.4 (SEQ ID NOs: 73 and 500), or a modified or unmodified variant thereof.

A RNAi agent comprising a first and a second strand, wherein the sequences of the first and/or second strands comprise the sequences of a first and/or second strand of: AD-35598.4 (SEQ ID NOs: 74 and 501), or a modified or unmodified variant thereof.

A RNAi agent comprising a first and a second strand, wherein the sequences of the first and/or second strands comprise the sequences of a first and/or second strand of: AD-35604.4 (SEQ ID NOs: 75 and 502), or a modified or unmodified variant thereof.

A RNAi agent comprising a first and a second strand, wherein the sequences of the first and/or second strands comprise the sequences of a first and/or second strand of: AD-35610.4 (SEQ ID NOs: 76 and 503), or a modified or unmodified variant thereof.

A RNAi agent comprising a first and a second strand, wherein the sequences of the first and/or second strands comprise the sequences of a first and/or second strand of: AD-35616.4 (SEQ ID NOs: 77 and 504), or a modified or unmodified variant thereof.

A RNAi agent comprising a first and a second strand, wherein the sequences of the first and/or second strands comprise the sequences of a first and/or second strand of: AD-35575.4 (SEQ ID NOs: 78 and 505), or a modified or unmodified variant thereof.

A RNAi agent comprising a first and a second strand, wherein the sequences of the first and/or second strands comprise the sequences of a first and/or second strand of: AD-35581.4 (SEQ ID NOs: 79 and 506), or a modified or unmodified variant thereof.

A RNAi agent comprising a first and a second strand, wherein the sequences of the first and/or second strands comprise the sequences of a first and/or second strand of: AD-35587.4 (SEQ ID NOs: 80 and 507), or a modified or unmodified variant thereof.

A RNAi agent comprising a first and a second strand, wherein the sequences of the first and/or second strands comprise the sequences of a first and/or second strand of: AD-35593.4 (SEQ ID NOs: 81 and 508), or a modified or unmodified variant thereof.

A RNAi agent comprising a first and a second strand, wherein the sequences of the first and/or second strands comprise the sequences of a first and/or second strand of: AD-35599.4 (SEQ ID NOs: 82 and 509), or a modified or unmodified variant thereof.

A RNAi agent comprising a first and a second strand, wherein the sequences of the first and/or second strands comprise the sequences of a first and/or second strand of: AD-35605.4 (SEQ ID NOs: 83 and 510), or a modified or unmodified variant thereof.

A RNAi agent comprising a first and a second strand, wherein the sequences of the first and/or second strands comprise the sequences of a first and/or second strand of: AD-35611.4 (SEQ ID NOs: 84 and 511), or a modified or unmodified variant thereof.

A RNAi agent comprising a first and a second strand, wherein the sequences of the first and/or second strands comprise the sequences of a first and/or second strand of: AD-35617.4 (SEQ ID NOs: 85 and 512), or a modified or unmodified variant thereof.

A RNAi agent comprising a first and a second strand, wherein the sequences of the first and/or second strands comprise the sequences of a first and/or second strand of: AD-35576.4 (SEQ ID NOs: 86 and 513), or a modified or unmodified variant thereof.

A RNAi agent comprising a first and a second strand, wherein the sequences of the first and/or second strands comprise the sequences of a first and/or second strand of: AD-35588.4 (SEQ ID NOs: 87 and 514), or a modified or unmodified variant thereof.

A RNAi agent comprising a first and a second strand, wherein the sequences of the first and/or second strands comprise the sequences of a first and/or second strand of: AD-35667.1 (SEQ ID NOs: 88 and 515), or a modified or unmodified variant thereof.

A RNAi agent comprising a first and a second strand, wherein the sequences of the first and/or second strands comprise the sequences of a first and/or second strand of: AD-35673.1 (SEQ ID NOs: 89 and 516), or a modified or unmodified variant thereof.

A RNAi agent comprising a first and a second strand, wherein the sequences of the first and/or second strands comprise the sequences of a first and/or second strand of: AD-35679.1 (SEQ ID NOs: 90 and 517), or a modified or unmodified variant thereof.

A RNAi agent comprising a first and a second strand, wherein the sequences of the first and/or second strands comprise the sequences of a first and/or second strand of: AD-35685.1 (SEQ ID NOs: 91 and 518), or a modified or unmodified variant thereof.

A RNAi agent comprising a first and a second strand, wherein the sequences of the first and/or second strands comprise the sequences of a first and/or second strand of: AD-35691.1 (SEQ ID NOs: 92 and 519), or a modified or unmodified variant thereof.

A RNAi agent comprising a first and a second strand, wherein the sequences of the first and/or second strands comprise the sequences of a first and/or second strand of: AD-35557.1 (SEQ ID NOs: 93 and 520), or a modified or unmodified variant thereof.

A RNAi agent comprising a first and a second strand, wherein the sequences of the first and/or second strands comprise the sequences of a first and/or second strand of: AD-35618.1 (SEQ ID NOs: 94 and 521), or a modified or unmodified variant thereof.

A RNAi agent comprising a first and a second strand, wherein the sequences of the first and/or second strands comprise the sequences of a first and/or second strand of: AD-35569.1 (SEQ ID NOs: 95 and 522), or a modified or unmodified variant thereof.

A RNAi agent comprising a first and a second strand, wherein the sequences of the first and/or second strands comprise the sequences of a first and/or second strand of: AD-35534.1 (SEQ ID NOs: 96 and 523), or a modified or unmodified variant thereof.

A RNAi agent comprising a first and a second strand, wherein the sequences of the first and/or second strands comprise the sequences of a first and/or second strand of: AD-35582.1 (SEQ ID NOs: 97 and 524), or a modified or unmodified variant thereof.

A RNAi agent comprising a first and a second strand, wherein the sequences of the first and/or second strands comprise the sequences of a first and/or second strand of: AD-35594.1 (SEQ ID NOs: 98 and 525), or a modified or unmodified variant thereof.

A RNAi agent comprising a first and a second strand, wherein the sequences of the first and/or second strands comprise the sequences of a first and/or second strand of: AD-35600.1 (SEQ ID NOs: 99 and 526), or a modified or unmodified variant thereof.

A RNAi agent comprising a first and a second strand, wherein the sequences of the first and/or second strands comprise the sequences of a first and/or second strand of: AD-35606.1 (SEQ ID NOs: 100 and 527), or a modified or unmodified variant thereof.

A RNAi agent comprising a first and a second strand, wherein the sequences of the first and/or second strands comprise the sequences of a first and/or second strand of: AD-35612.1 (SEQ ID NOs: 101 and 528), or a modified or unmodified variant thereof.

A RNAi agent comprising a first and a second strand, wherein the sequences of the first and/or second strands comprise the sequences of a first and/or second strand of: AD-38127.1 (SEQ ID NOs: 102 and 529), or a modified or unmodified variant thereof.

A RNAi agent comprising a first and a second strand, wherein the sequences of the first and/or second strands comprise the sequences of a first and/or second strand of: AD-38133.1 (SEQ ID NOs: 103 and 530), or a modified or unmodified variant thereof.

A RNAi agent comprising a first and a second strand, wherein the sequences of the first and/or second strands comprise the sequences of a first and/or second strand of: AD-38139.1 (SEQ ID NOs: 104 and 531), or a modified or unmodified variant thereof.

A RNAi agent comprising a first and a second strand, wherein the sequences of the first and/or second strands comprise the sequences of a first and/or second strand of: AD-38145.1 (SEQ ID NOs: 105 and 532), or a modified or unmodified variant thereof.

A RNAi agent comprising a first and a second strand, wherein the sequences of the first and/or second strands comprise the sequences of a first and/or second strand of: AD-38151.1 (SEQ ID NOs: 106 and 533), or a modified or unmodified variant thereof.

A RNAi agent comprising a first and a second strand, wherein the sequences of the first and/or second strands comprise the sequences of a first and/or second strand of: AD-38157.1 (SEQ ID NOs: 107 and 534), or a modified or unmodified variant thereof.

A RNAi agent comprising a first and a second strand, wherein the sequences of the first and/or second strands comprise the sequences of a first and/or second strand of: AD-38163.1 (SEQ ID NOs: 108 and 535), or a modified or unmodified variant thereof.

A RNAi agent comprising a first and a second strand, wherein the sequences of the first and/or second strands comprise the sequences of a first and/or second strand of: AD-38169.1 (SEQ ID NOs: 109 and 536), or a modified or unmodified variant thereof.

A RNAi agent comprising a first and a second strand, wherein the sequences of the first and/or second strands comprise the sequences of a first and/or second strand of: AD-38128.1 (SEQ ID NOs: 110 and 537), or a modified or unmodified variant thereof.

A RNAi agent comprising a first and a second strand, wherein the sequences of the first and/or second strands comprise the sequences of a first and/or second strand of: AD-38134.1 (SEQ ID NOs: 111 and 538), or a modified or unmodified variant thereof.

A RNAi agent comprising a first and a second strand, wherein the sequences of the first and/or second strands comprise the sequences of a first and/or second strand of: AD-38140.1 (SEQ ID NOs: 112 and 539), or a modified or unmodified variant thereof.

A RNAi agent comprising a first and a second strand, wherein the sequences of the first and/or second strands comprise the sequences of a first and/or second strand of: AD-38146.1 (SEQ ID NOs: 113 and 540), or a modified or unmodified variant thereof.

A RNAi agent comprising a first and a second strand, wherein the sequences of the first and/or second strands comprise the sequences of a first and/or second strand of: AD-38152.1 (SEQ ID NOs: 114 and 541), or a modified or unmodified variant thereof.

A RNAi agent comprising a first and a second strand, wherein the sequences of the first and/or second strands comprise the sequences of a first and/or second strand of: AD-38158.1 (SEQ ID NOs: 115 and 542), or a modified or unmodified variant thereof.

A RNAi agent comprising a first and a second strand, wherein the sequences of the first and/or second strands comprise the sequences of a first and/or second strand of: AD-38164.1 (SEQ ID NOs: 116 and 543), or a modified or unmodified variant thereof.

A RNAi agent comprising a first and a second strand, wherein the sequences of the first and/or second strands comprise the sequences of a first and/or second strand of: AD-38170.1 (SEQ ID NOs: 117 and 544), or a modified or unmodified variant thereof.

A RNAi agent comprising a first and a second strand, wherein the sequences of the first and/or second strands comprise the sequences of a first and/or second strand of: AD-38129.1 (SEQ ID NOs: 118 and 545), or a modified or unmodified variant thereof.

A RNAi agent comprising a first and a second strand, wherein the sequences of the first and/or second strands comprise the sequences of a first and/or second strand of: AD-38135.1 (SEQ ID NOs: 119 and 546), or a modified or unmodified variant thereof.

A RNAi agent comprising a first and a second strand, wherein the sequences of the first and/or second strands comprise the sequences of a first and/or second strand of: AD-38141.1 (SEQ ID NOs: 120 and 547), or a modified or unmodified variant thereof.

A RNAi agent comprising a first and a second strand, wherein the sequences of the first and/or second strands comprise the sequences of a first and/or second strand of: AD-38147.1 (SEQ ID NOs: 121 and 548), or a modified or unmodified variant thereof.

A RNAi agent comprising a first and a second strand, wherein the sequences of the first and/or second strands comprise the sequences of a first and/or second strand of: AD-38153.1 (SEQ ID NOs: 122 and 549), or a modified or unmodified variant thereof.

A RNAi agent comprising a first and a second strand, wherein the sequences of the first and/or second strands comprise the sequences of a first and/or second strand of: AD-38159.1 (SEQ ID NOs: 123 and 550), or a modified or unmodified variant thereof.

A RNAi agent comprising a first and a second strand, wherein the sequences of the first and/or second strands comprise the sequences of a first and/or second strand of: AD-38165.1 (SEQ ID NOs: 124 and 551), or a modified or unmodified variant thereof.

A RNAi agent comprising a first and a second strand, wherein the sequences of the first and/or second strands comprise the sequences of a first and/or second strand of: AD-38171.1 (SEQ ID NOs: 125 and 552), or a modified or unmodified variant thereof.

A RNAi agent comprising a first and a second strand, wherein the sequences of the first and/or second strands comprise the sequences of a first and/or second strand of: AD-38130.1 (SEQ ID NOs: 126 and 553), or a modified or unmodified variant thereof.

A RNAi agent comprising a first and a second strand, wherein the sequences of the first and/or second strands comprise the sequences of a first and/or second strand of: AD-38136.1 (SEQ ID NOs: 127 and 554), or a modified or unmodified variant thereof.

A RNAi agent comprising a first and a second strand, wherein the sequences of the first and/or second strands comprise the sequences of a first and/or second strand of: AD-38142.1 (SEQ ID NOs: 128 and 555), or a modified or unmodified variant thereof.

A RNAi agent comprising a first and a second strand, wherein the sequences of the first and/or second strands comprise the sequences of a first and/or second strand of: AD-38148.1 (SEQ ID NOs: 129 and 556), or a modified or unmodified variant thereof.

A RNAi agent comprising a first and a second strand, wherein the sequences of the first and/or second strands comprise the sequences of a first and/or second strand of: AD-38154.1 (SEQ ID NOs: 130 and 557), or a modified or unmodified variant thereof.

A RNAi agent comprising a first and a second strand, wherein the sequences of the first and/or second strands comprise the sequences of a first and/or second strand of: AD-38160.1 (SEQ ID NOs: 131 and 558), or a modified or unmodified variant thereof.

A RNAi agent comprising a first and a second strand, wherein the sequences of the first and/or second strands comprise the sequences of a first and/or second strand of: AD-38166.1 (SEQ ID NOs: 132 and 559), or a modified or unmodified variant thereof.

A RNAi agent comprising a first and a second strand, wherein the sequences of the first and/or second strands comprise the sequences of a first and/or second strand of: AD-38172.1 (SEQ ID NOs: 133 and 560), or a modified or unmodified variant thereof.

A RNAi agent comprising a first and a second strand, wherein the sequences of the first and/or second strands comprise the sequences of a first and/or second strand of: AD-38131.1 (SEQ ID NOs: 134 and 561), or a modified or unmodified variant thereof.

A RNAi agent comprising a first and a second strand, wherein the sequences of the first and/or second strands comprise the sequences of a first and/or second strand of: AD-38137.1 (SEQ ID NOs: 135 and 562), or a modified or unmodified variant thereof.

A RNAi agent comprising a first and a second strand, wherein the sequences of the first and/or second strands comprise the sequences of a first and/or second strand of: AD-38143.1 (SEQ ID NOs: 136 and 563), or a modified or unmodified variant thereof.

A RNAi agent comprising a first and a second strand, wherein the sequences of the first and/or second strands comprise the sequences of a first and/or second strand of: AD-38149.1 (SEQ ID NOs: 137 and 564), or a modified or unmodified variant thereof.

A RNAi agent comprising a first and a second strand, wherein the sequences of the first and/or second strands comprise the sequences of a first and/or second strand of: AD-38155.1 (SEQ ID NOs: 138 and 565), or a modified or unmodified variant thereof.

A RNAi agent comprising a first and a second strand, wherein the sequences of the first and/or second strands comprise the sequences of a first and/or second strand of: AD-38161.1 (SEQ ID NOs: 139 and 566), or a modified or unmodified variant thereof.

A RNAi agent comprising a first and a second strand, wherein the sequences of the first and/or second strands comprise the sequences of a first and/or second strand of: AD-38167.1 (SEQ ID NOs: 140 and 567), or a modified or unmodified variant thereof.

A RNAi agent comprising a first and a second strand, wherein the sequences of the first and/or second strands comprise the sequences of a first and/or second strand of: AD-38173.1 (SEQ ID NOs: 141 and 568), or a modified or unmodified variant thereof.

A RNAi agent comprising a first and a second strand, wherein the sequences of the first and/or second strands comprise the sequences of a first and/or second strand of: AD-38132.1 (SEQ ID NOs: 142 and 569), or a modified or unmodified variant thereof.

A RNAi agent comprising a first and a second strand, wherein the sequences of the first and/or second strands comprise the sequences of a first and/or second strand of: AD-38138.1 (SEQ ID NOs: 143 and 570), or a modified or unmodified variant thereof.

A RNAi agent comprising a first and a second strand, wherein the sequences of the first and/or second strands comprise the sequences of a first and/or second strand of: AD-38144.1 (SEQ ID NOs: 144 and 571), or a modified or unmodified variant thereof.

A RNAi agent comprising a first and a second strand, wherein the sequences of the first and/or second strands comprise the sequences of a first and/or second strand of: AD-38150.1 (SEQ ID NOs: 145 and 572), or a modified or unmodified variant thereof.

A RNAi agent comprising a first and a second strand, wherein the sequences of the first and/or second strands comprise the sequences of a first and/or second strand of: AD-38156.1 (SEQ ID NOs: 146 and 573), or a modified or unmodified variant thereof.

A RNAi agent comprising a first and a second strand, wherein the sequences of the first and/or second strands comprise the sequences of a first and/or second strand of: AD-38162.1 (SEQ ID NOs: 147 and 574), or a modified or unmodified variant thereof.

A RNAi agent comprising a first and a second strand, wherein the sequences of the first and/or second strands comprise the sequences of a first and/or second strand of: AD-38168.1 (SEQ ID NOs: 148 and 575), or a modified or unmodified variant thereof.

A RNAi agent comprising a first and a second strand, wherein the sequences of the first and/or second strands comprise the sequences of a first and/or second strand of: AD-38174.1 (SEQ ID NOs: 149 and 576), or a modified or unmodified variant thereof.

A RNAi agent comprising a first and a second strand, wherein the sequences of the first and/or second strands comprise the sequences of a first and/or second strand of: AD-39683.1 (SEQ ID NOs: 150 and 577), or a modified or unmodified variant thereof.

A RNAi agent comprising a first and a second strand, wherein the sequences of the first and/or second strands comprise the sequences of a first and/or second strand of: AD-39689.1 (SEQ ID NOs: 151 and 578), or a modified or unmodified variant thereof.

A RNAi agent comprising a first and a second strand, wherein the sequences of the first and/or second strands comprise the sequences of a first and/or second strand of: AD-39695.1 (SEQ ID NOs: 152 and 579), or a modified or unmodified variant thereof.

A RNAi agent comprising a first and a second strand, wherein the sequences of the first and/or second strands comprise the sequences of a first and/or second strand of: AD-39701.1 (SEQ ID NOs: 153 and 580), or a modified or unmodified variant thereof.

A RNAi agent comprising a first and a second strand, wherein the sequences of the first and/or second strands comprise the sequences of a first and/or second strand of: AD-39707.1 (SEQ ID NOs: 154 and 581), or a modified or unmodified variant thereof.

A RNAi agent comprising a first and a second strand, wherein the sequences of the first and/or second strands comprise the sequences of a first and/or second strand of: AD-39713.1 (SEQ ID NOs: 155 and 582), or a modified or unmodified variant thereof.

A RNAi agent comprising a first and a second strand, wherein the sequences of the first and/or second strands comprise the sequences of a first and/or second strand of: AD-39719.1 (SEQ ID NOs: 156 and 583), or a modified or unmodified variant thereof.

A RNAi agent comprising a first and a second strand, wherein the sequences of the first and/or second strands comprise the sequences of a first and/or second strand of: AD-39725.1 (SEQ ID NOs: 157 and 584), or a modified or unmodified variant thereof.

A RNAi agent comprising a first and a second strand, wherein the sequences of the first and/or second strands comprise the sequences of a first and/or second strand of: AD-39684.1 (SEQ ID NOs: 158 and 585), or a modified or unmodified variant thereof.

A RNAi agent comprising a first and a second strand, wherein the sequences of the first and/or second strands comprise the sequences of a first and/or second strand of: AD-39690.1 (SEQ ID NOs: 159 and 586), or a modified or unmodified variant thereof.

A RNAi agent comprising a first and a second strand, wherein the sequences of the first and/or second strands comprise the sequences of a first and/or second strand of: AD-39696.1 (SEQ ID NOs: 160 and 587), or a modified or unmodified variant thereof.

A RNAi agent comprising a first and a second strand, wherein the sequences of the first and/or second strands comprise the sequences of a first and/or second strand of: AD-39702.1 (SEQ ID NOs: 161 and 588), or a modified or unmodified variant thereof.

A RNAi agent comprising a first and a second strand, wherein the sequences of the first and/or second strands comprise the sequences of a first and/or second strand of: AD-39708.1 (SEQ ID NOs: 162 and 589), or a modified or unmodified variant thereof.

A RNAi agent comprising a first and a second strand, wherein the sequences of the first and/or second strands comprise the sequences of a first and/or second strand of: AD-39714.1 (SEQ ID NOs: 163 and 590), or a modified or unmodified variant thereof.

A RNAi agent comprising a first and a second strand, wherein the sequences of the first and/or second strands comprise the sequences of a first and/or second strand of: AD-39720.1 (SEQ ID NOs: 164 and 591), or a modified or unmodified variant thereof.

A RNAi agent comprising a first and a second strand, wherein the sequences of the first and/or second strands comprise the sequences of a first and/or second strand of: AD-39726.1 (SEQ ID NOs: 165 and 592), or a modified or unmodified variant thereof.

A RNAi agent comprising a first and a second strand, wherein the sequences of the first and/or second strands comprise the sequences of a first and/or second strand of: AD-39685.1 (SEQ ID NOs: 166 and 593), or a modified or unmodified variant thereof.

A RNAi agent comprising a first and a second strand, wherein the sequences of the first and/or second strands comprise the sequences of a first and/or second strand of: AD-39691.1 (SEQ ID NOs: 167 and 594), or a modified or unmodified variant thereof.

A RNAi agent comprising a first and a second strand, wherein the sequences of the first and/or second strands comprise the sequences of a first and/or second strand of: AD-39697.1 (SEQ ID NOs: 168 and 595), or a modified or unmodified variant thereof.

A RNAi agent comprising a first and a second strand, wherein the sequences of the first and/or second strands comprise the sequences of a first and/or second strand of: AD-39703.1 (SEQ ID NOs: 169 and 596), or a modified or unmodified variant thereof.

A RNAi agent comprising a first and a second strand, wherein the sequences of the first and/or second strands comprise the sequences of a first and/or second strand of: AD-39709.1 (SEQ ID NOs: 170 and 597), or a modified or unmodified variant thereof.

A RNAi agent comprising a first and a second strand, wherein the sequences of the first and/or second strands comprise the sequences of a first and/or second strand of: AD-39715.1 (SEQ ID NOs: 171 and 598), or a modified or unmodified variant thereof.

A RNAi agent comprising a first and a second strand, wherein the sequences of the first and/or second strands comprise the sequences of a first and/or second strand of: AD-39721.1 (SEQ ID NOs: 172 and 599), or a modified or unmodified variant thereof.

A RNAi agent comprising a first and a second strand, wherein the sequences of the first and/or second strands comprise the sequences of a first and/or second strand of: AD-39727.1 (SEQ ID NOs: 173 and 600), or a modified or unmodified variant thereof.

A RNAi agent comprising a first and a second strand, wherein the sequences of the first and/or second strands comprise the sequences of a first and/or second strand of: AD-39686.1 (SEQ ID NOs: 174 and 601), or a modified or unmodified variant thereof.

A RNAi agent comprising a first and a second strand, wherein the sequences of the first and/or second strands comprise the sequences of a first and/or second strand of: AD-39692.1 (SEQ ID NOs: 175 and 602), or a modified or unmodified variant thereof.

A RNAi agent comprising a first and a second strand, wherein the sequences of the first and/or second strands comprise the sequences of a first and/or second strand of: AD-39698.1 (SEQ ID NOs: 176 and 603), or a modified or unmodified variant thereof.

A RNAi agent comprising a first and a second strand, wherein the sequences of the first and/or second strands comprise the sequences of a first and/or second strand of: AD-39704.1 (SEQ ID NOs: 177 and 604), or a modified or unmodified variant thereof.

A RNAi agent comprising a first and a second strand, wherein the sequences of the first and/or second strands comprise the sequences of a first and/or second strand of: AD-39710.1 (SEQ ID NOs: 178 and 605), or a modified or unmodified variant thereof.

A RNAi agent comprising a first and a second strand, wherein the sequences of the first and/or second strands comprise the sequences of a first and/or second strand of: AD-39716.1 (SEQ ID NOs: 179 and 606), or a modified or unmodified variant thereof.

A RNAi agent comprising a first and a second strand, wherein the sequences of the first and/or second strands comprise the sequences of a first and/or second strand of: AD-39722.1 (SEQ ID NOs: 180 and 607), or a modified or unmodified variant thereof.

A RNAi agent comprising a first and a second strand, wherein the sequences of the first and/or second strands comprise the sequences of a first and/or second strand of: AD-39728.1 (SEQ ID NOs: 181 and 608), or a modified or unmodified variant thereof.

A RNAi agent comprising a first and a second strand, wherein the sequences of the first and/or second strands comprise the sequences of a first and/or second strand of: AD-39687.1 (SEQ ID NOs: 182 and 609), or a modified or unmodified variant thereof.

A RNAi agent comprising a first and a second strand, wherein the sequences of the first and/or second strands comprise the sequences of a first and/or second strand of: AD-39693.1 (SEQ ID NOs: 183 and 610), or a modified or unmodified variant thereof.

A RNAi agent comprising a first and a second strand, wherein the sequences of the first and/or second strands comprise the sequences of a first and/or second strand of: AD-39699.1 (SEQ ID NOs: 184 and 611), or a modified or unmodified variant thereof.

A RNAi agent comprising a first and a second strand, wherein the sequences of the first and/or second strands comprise the sequences of a first and/or second strand of: AD-39705.1 (SEQ ID NOs: 185 and 612), or a modified or unmodified variant thereof.

A RNAi agent comprising a first and a second strand, wherein the sequences of the first and/or second strands comprise the sequences of a first and/or second strand of: AD-39711.1 (SEQ ID NOs: 186 and 613), or a modified or unmodified variant thereof.

A RNAi agent comprising a first and a second strand, wherein the sequences of the first and/or second strands comprise the sequences of a first and/or second strand of: AD-39717.1 (SEQ ID NOs: 187 and 614), or a modified or unmodified variant thereof.

A RNAi agent comprising a first and a second strand, wherein the sequences of the first and/or second strands comprise the sequences of a first and/or second strand of: AD-39723.1 (SEQ ID NOs: 188 and 615), or a modified or unmodified variant thereof.

A RNAi agent comprising a first and a second strand, wherein the sequences of the first and/or second strands comprise the sequences of a first and/or second strand of: AD-39729.1 (SEQ ID NOs: 189 and 616), or a modified or unmodified variant thereof.

A RNAi agent comprising a first and a second strand, wherein the sequences of the first and/or second strands comprise the sequences of a first and/or second strand of: AD-39688.1 (SEQ ID NOs: 190 and 617), or a modified or unmodified variant thereof.

A RNAi agent comprising a first and a second strand, wherein the sequences of the first and/or second strands comprise the sequences of a first and/or second strand of: AD-39694.1 (SEQ ID NOs: 191 and 618), or a modified or unmodified variant thereof.

A RNAi agent comprising a first and a second strand, wherein the sequences of the first and/or second strands comprise the sequences of a first and/or second strand of: AD-39700.1 (SEQ ID NOs: 192 and 619), or a modified or unmodified variant thereof.

A RNAi agent comprising a first and a second strand, wherein the sequences of the first and/or second strands comprise the sequences of a first and/or second strand of: AD-39706.1 (SEQ ID NOs: 193 and 620), or a modified or unmodified variant thereof.

A RNAi agent comprising a first and a second strand, wherein the sequences of the first and/or second strands comprise the sequences of a first and/or second strand of: AD-39712.1 (SEQ ID NOs: 194 and 621), or a modified or unmodified variant thereof.

A RNAi agent comprising a first and a second strand, wherein the sequences of the first and/or second strands comprise the sequences of a first and/or second strand of: AD-39718.1 (SEQ ID NOs: 195 and 622), or a modified or unmodified variant thereof.

A RNAi agent comprising a first and a second strand, wherein the sequences of the first and/or second strands comprise the sequences of a first and/or second strand of: AD-39724.1 (SEQ ID NOs: 196 and 623), or a modified or unmodified variant thereof.

A RNAi agent comprising a first and a second strand, wherein the sequences of the first and/or second strands comprise the sequences of a first and/or second strand of: AD-39730.1 (SEQ ID NOs: 197 and 624), or a modified or unmodified variant thereof.

A RNAi agent comprising a first and a second strand, wherein the sequences of the first and/or second strands comprise the sequences of a first and/or second strand of: AD-39736.1 (SEQ ID NOs: 198 and 625), or a modified or unmodified variant thereof.

A RNAi agent comprising a first and a second strand, wherein the sequences of the first and/or second strands comprise the sequences of a first and/or second strand of: AD-39742.1 (SEQ ID NOs: 199 and 626), or a modified or unmodified variant thereof.

A RNAi agent comprising a first and a second strand, wherein the sequences of the first and/or second strands comprise the sequences of a first and/or second strand of: AD-39748.1 (SEQ ID NOs: 200 and 627), or a modified or unmodified variant thereof.

A RNAi agent comprising a first and a second strand, wherein the sequences of the first and/or second strands comprise the sequences of a first and/or second strand of: AD-39754.1 (SEQ ID NOs: 201 and 628), or a modified or unmodified variant thereof.

A RNAi agent comprising a first and a second strand, wherein the sequences of the first and/or second strands comprise the sequences of a first and/or second strand of: AD-39760.1 (SEQ ID NOs: 202 and 629), or a modified or unmodified variant thereof.

A RNAi agent comprising a first and a second strand, wherein the sequences of the first and/or second strands comprise the sequences of a first and/or second strand of: AD-39766.1 (SEQ ID NOs: 203 and 630), or a modified or unmodified variant thereof.

A RNAi agent comprising a first and a second strand, wherein the sequences of the first and/or second strands comprise the sequences of a first and/or second strand of: AD-39772.1 (SEQ ID NOs: 204 and 631), or a modified or unmodified variant thereof.

A RNAi agent comprising a first and a second strand, wherein the sequences of the first and/or second strands comprise the sequences of a first and/or second strand of: AD-39731.1 (SEQ ID NOs: 205 and 632), or a modified or unmodified variant thereof.

A RNAi agent comprising a first and a second strand, wherein the sequences of the first and/or second strands comprise the sequences of a first and/or second strand of: AD-39737.1 (SEQ ID NOs: 206 and 633), or a modified or unmodified variant thereof.

A RNAi agent comprising a first and a second strand, wherein the sequences of the first and/or second strands comprise the sequences of a first and/or second strand of: AD-39743.1 (SEQ ID NOs: 207 and 634), or a modified or unmodified variant thereof.

A RNAi agent comprising a first and a second strand, wherein the sequences of the first and/or second strands comprise the sequences of a first and/or second strand of: AD-39749.1 (SEQ ID NOs: 208 and 635), or a modified or unmodified variant thereof.

A RNAi agent comprising a first and a second strand, wherein the sequences of the first and/or second strands comprise the sequences of a first and/or second strand of: AD-39755.1 (SEQ ID NOs: 209 and 636), or a modified or unmodified variant thereof.

A RNAi agent comprising a first and a second strand, wherein the sequences of the first and/or second strands comprise the sequences of a first and/or second strand of: AD-39761.1 (SEQ ID NOs: 210 and 637), or a modified or unmodified variant thereof.

A RNAi agent comprising a first and a second strand, wherein the sequences of the first and/or second strands comprise the sequences of a first and/or second strand of: AD-39767.1 (SEQ ID NOs: 211 and 638), or a modified or unmodified variant thereof.

A RNAi agent comprising a first and a second strand, wherein the sequences of the first and/or second strands comprise the sequences of a first and/or second strand of: AD-39773.1 (SEQ ID NOs: 212 and 639), or a modified or unmodified variant thereof.

A RNAi agent comprising a first and a second strand, wherein the sequences of the first and/or second strands comprise the sequences of a first and/or second strand of: AD-39732.1 (SEQ ID NOs: 213 and 640), or a modified or unmodified variant thereof.

A RNAi agent comprising a first and a second strand, wherein the sequences of the first and/or second strands comprise the sequences of a first and/or second strand of: AD-39738.1 (SEQ ID NOs: 214 and 641), or a modified or unmodified variant thereof.

A RNAi agent comprising a first and a second strand, wherein the sequences of the first and/or second strands comprise the sequences of a first and/or second strand of: AD-39744.1 (SEQ ID NOs: 215 and 642), or a modified or unmodified variant thereof.

A RNAi agent comprising a first and a second strand, wherein the sequences of the first and/or second strands comprise the sequences of a first and/or second strand of: AD-39750.1 (SEQ ID NOs: 216 and 643), or a modified or unmodified variant thereof.

A RNAi agent comprising a first and a second strand, wherein the sequences of the first and/or second strands comprise the sequences of a first and/or second strand of: AD-39756.1 (SEQ ID NOs: 217 and 644), or a modified or unmodified variant thereof.

A RNAi agent comprising a first and a second strand, wherein the sequences of the first and/or second strands comprise the sequences of a first and/or second strand of: AD-39762.1 (SEQ ID NOs: 218 and 645), or a modified or unmodified variant thereof.

A RNAi agent comprising a first and a second strand, wherein the sequences of the first and/or second strands comprise the sequences of a first and/or second strand of: AD-39768.1 (SEQ ID NOs: 219 and 646), or a modified or unmodified variant thereof.

A RNAi agent comprising a first and a second strand, wherein the sequences of the first and/or second strands comprise the sequences of a first and/or second strand of: AD-39774.1 (SEQ ID NOs: 220 and 647), or a modified or unmodified variant thereof.

A RNAi agent comprising a first and a second strand, wherein the sequences of the first and/or second strands comprise the sequences of a first and/or second strand of: AD-39733.1 (SEQ ID NOs: 221 and 648), or a modified or unmodified variant thereof.

A RNAi agent comprising a first and a second strand, wherein the sequences of the first and/or second strands comprise the sequences of a first and/or second strand of: AD-39739.1 (SEQ ID NOs: 222 and 649), or a modified or unmodified variant thereof.

A RNAi agent comprising a first and a second strand, wherein the sequences of the first and/or second strands comprise the sequences of a first and/or second strand of: AD-39745.1 (SEQ ID NOs: 223 and 650), or a modified or unmodified variant thereof.

A RNAi agent comprising a first and a second strand, wherein the sequences of the first and/or second strands comprise the sequences of a first and/or second strand of: AD-39751.1 (SEQ ID NOs: 224 and 651), or a modified or unmodified variant thereof.

A RNAi agent comprising a first and a second strand, wherein the sequences of the first and/or second strands comprise the sequences of a first and/or second strand of: AD-39757.1 (SEQ ID NOs: 225 and 652), or a modified or unmodified variant thereof.

A RNAi agent comprising a first and a second strand, wherein the sequences of the first and/or second strands comprise the sequences of a first and/or second strand of: AD-39763.1 (SEQ ID NOs: 226 and 653), or a modified or unmodified variant thereof.

A RNAi agent comprising a first and a second strand, wherein the sequences of the first and/or second strands comprise the sequences of a first and/or second strand of: AD-39769.1 (SEQ ID NOs: 227 and 654), or a modified or unmodified variant thereof.

A RNAi agent comprising a first and a second strand, wherein the sequences of the first and/or second strands comprise the sequences of a first and/or second strand of: AD-39775.1 (SEQ ID NOs: 228 and 655), or a modified or unmodified variant thereof.

A RNAi agent comprising a first and a second strand, wherein the sequences of the first and/or second strands comprise the sequences of a first and/or second strand of: AD-39734.1 (SEQ ID NOs: 229 and 656), or a modified or unmodified variant thereof.

A RNAi agent comprising a first and a second strand, wherein the sequences of the first and/or second strands comprise the sequences of a first and/or second strand of: AD-39740.1 (SEQ ID NOs: 230 and 657), or a modified or unmodified variant thereof.

A RNAi agent comprising a first and a second strand, wherein the sequences of the first and/or second strands comprise the sequences of a first and/or second strand of: AD-39746.1 (SEQ ID NOs: 231 and 658), or a modified or unmodified variant thereof.

A RNAi agent comprising a first and a second strand, wherein the sequences of the first and/or second strands comprise the sequences of a first and/or second strand of: AD-39752.1 (SEQ ID NOs: 232 and 659), or a modified or unmodified variant thereof.

A RNAi agent comprising a first and a second strand, wherein the sequences of the first and/or second strands comprise the sequences of a first and/or second strand of: AD-39758.1 (SEQ ID NOs: 233 and 660), or a modified or unmodified variant thereof.

A RNAi agent comprising a first and a second strand, wherein the sequences of the first and/or second strands comprise the sequences of a first and/or second strand of: AD-39764.1 (SEQ ID NOs: 234 and 661), or a modified or unmodified variant thereof.

A RNAi agent comprising a first and a second strand, wherein the sequences of the first and/or second strands comprise the sequences of a first and/or second strand of: AD-39770.1 (SEQ ID NOs: 235 and 662), or a modified or unmodified variant thereof.

A RNAi agent comprising a first and a second strand, wherein the sequences of the first and/or second strands comprise the sequences of a first and/or second strand of: AD-39776.1 (SEQ ID NOs: 236 and 663), or a modified or unmodified variant thereof.

A RNAi agent comprising a first and a second strand, wherein the sequences of the first and/or second strands comprise the sequences of a first and/or second strand of: AD-39735.1 (SEQ ID NOs: 237 and 664), or a modified or unmodified variant thereof.

A RNAi agent comprising a first and a second strand, wherein the sequences of the first and/or second strands comprise the sequences of a first and/or second strand of: AD-39741.1 (SEQ ID NOs: 238 and 665), or a modified or unmodified variant thereof.

A RNAi agent comprising a first and a second strand, wherein the sequences of the first and/or second strands comprise the sequences of a first and/or second strand of: AD-39747.1 (SEQ ID NOs: 239 and 666), or a modified or unmodified variant thereof.

A RNAi agent comprising a first and a second strand, wherein the sequences of the first and/or second strands comprise the sequences of a first and/or second strand of: AD-39753.1 (SEQ ID NOs: 240 and 667), or a modified or unmodified variant thereof.

A RNAi agent comprising a first and a second strand, wherein the sequences of the first and/or second strands comprise the sequences of a first and/or second strand of: AD-39759.1 (SEQ ID NOs: 241 and 668), or a modified or unmodified variant thereof.

A RNAi agent comprising a first and a second strand, wherein the sequences of the first and/or second strands comprise the sequences of a first and/or second strand of: AD-39765.1 (SEQ ID NOs: 242 and 669), or a modified or unmodified variant thereof.

A RNAi agent comprising a first and a second strand, wherein the sequences of the first and/or second strands comprise the sequences of a first and/or second strand of: AD-39771.1 (SEQ ID NOs: 243 and 670), or a modified or unmodified variant thereof.

A RNAi agent comprising a first and a second strand, wherein the sequences of the first and/or second strands comprise the sequences of a first and/or second strand of: AD-39778.1 (SEQ ID NOs: 244 and 671), or a modified or unmodified variant thereof.

A RNAi agent comprising a first and a second strand, wherein the sequences of the first and/or second strands comprise the sequences of a first and/or second strand of: AD-39784.1 (SEQ ID NOs: 245 and 672), or a modified or unmodified variant thereof.

A RNAi agent comprising a first and a second strand, wherein the sequences of the first and/or second strands comprise the sequences of a first and/or second strand of: AD-39790.1 (SEQ ID NOs: 246 and 673), or a modified or unmodified variant thereof.

A RNAi agent comprising a first and a second strand, wherein the sequences of the first and/or second strands comprise the sequences of a first and/or second strand of: AD-39796.1 (SEQ ID NOs: 247 and 674), or a modified or unmodified variant thereof.

A RNAi agent comprising a first and a second strand, wherein the sequences of the first and/or second strands comprise the sequences of a first and/or second strand of: AD-39802.1 (SEQ ID NOs: 248 and 675), or a modified or unmodified variant thereof.

A RNAi agent comprising a first and a second strand, wherein the sequences of the first and/or second strands comprise the sequences of a first and/or second strand of: AD-39808.1 (SEQ ID NOs: 249 and 676), or a modified or unmodified variant thereof.

A RNAi agent comprising a first and a second strand, wherein the sequences of the first and/or second strands comprise the sequences of a first and/or second strand of: AD-39814.1 (SEQ ID NOs: 250 and 677), or a modified or unmodified variant thereof.

A RNAi agent comprising a first and a second strand, wherein the sequences of the first and/or second strands comprise the sequences of a first and/or second strand of: AD-39820.1 (SEQ ID NOs: 251 and 678), or a modified or unmodified variant thereof.

A RNAi agent comprising a first and a second strand, wherein the sequences of the first and/or second strands comprise the sequences of a first and/or second strand of: AD-39779.1 (SEQ ID NOs: 252 and 679), or a modified or unmodified variant thereof.

A RNAi agent comprising a first and a second strand, wherein the sequences of the first and/or second strands comprise the sequences of a first and/or second strand of: AD-39785.1 (SEQ ID NOs: 253 and 680), or a modified or unmodified variant thereof.

A RNAi agent comprising a first and a second strand, wherein the sequences of the first and/or second strands comprise the sequences of a first and/or second strand of: AD-39791.1 (SEQ ID NOs: 254 and 681), or a modified or unmodified variant thereof.

A RNAi agent comprising a first and a second strand, wherein the sequences of the first and/or second strands comprise the sequences of a first and/or second strand of: AD-39797.1 (SEQ ID NOs: 255 and 682), or a modified or unmodified variant thereof.

A RNAi agent comprising a first and a second strand, wherein the sequences of the first and/or second strands comprise the sequences of a first and/or second strand of: AD-39803.1 (SEQ ID NOs: 256 and 683), or a modified or unmodified variant thereof.

A RNAi agent comprising a first and a second strand, wherein the sequences of the first and/or second strands comprise the sequences of a first and/or second strand of: AD-39809.1 (SEQ ID NOs: 257 and 684), or a modified or unmodified variant thereof.

A RNAi agent comprising a first and a second strand, wherein the sequences of the first and/or second strands comprise the sequences of a first and/or second strand of: AD-39815.1 (SEQ ID NOs: 258 and 685), or a modified or unmodified variant thereof.

A RNAi agent comprising a first and a second strand, wherein the sequences of the first and/or second strands comprise the sequences of a first and/or second strand of: AD-39821.1 (SEQ ID NOs: 259 and 686), or a modified or unmodified variant thereof.

A RNAi agent comprising a first and a second strand, wherein the sequences of the first and/or second strands comprise the sequences of a first and/or second strand of: AD-39780.1 (SEQ ID NOs: 260 and 687), or a modified or unmodified variant thereof.

A RNAi agent comprising a first and a second strand, wherein the sequences of the first and/or second strands comprise the sequences of a first and/or second strand of: AD-39786.1 (SEQ ID NOs: 261 and 688), or a modified or unmodified variant thereof.

A RNAi agent comprising a first and a second strand, wherein the sequences of the first and/or second strands comprise the sequences of a first and/or second strand of: AD-39792.1 (SEQ ID NOs: 262 and 689), or a modified or unmodified variant thereof.

A RNAi agent comprising a first and a second strand, wherein the sequences of the first and/or second strands comprise the sequences of a first and/or second strand of: AD-39798.1 (SEQ ID NOs: 263 and 690), or a modified or unmodified variant thereof.

A RNAi agent comprising a first and a second strand, wherein the sequences of the first and/or second strands comprise the sequences of a first and/or second strand of: AD-39804.1 (SEQ ID NOs: 264 and 691), or a modified or unmodified variant thereof.

A RNAi agent comprising a first and a second strand, wherein the sequences of the first and/or second strands comprise the sequences of a first and/or second strand of: AD-39810.1 (SEQ ID NOs: 265 and 692), or a modified or unmodified variant thereof.

A RNAi agent comprising a first and a second strand, wherein the sequences of the first and/or second strands comprise the sequences of a first and/or second strand of: AD-39816.1 (SEQ ID NOs: 266 and 693), or a modified or unmodified variant thereof.

A RNAi agent comprising a first and a second strand, wherein the sequences of the first and/or second strands comprise the sequences of a first and/or second strand of: AD-39822.1 (SEQ ID NOs: 267 and 694), or a modified or unmodified variant thereof.

A RNAi agent comprising a first and a second strand, wherein the sequences of the first and/or second strands comprise the sequences of a first and/or second strand of: AD-39781.1 (SEQ ID NOs: 268 and 695), or a modified or unmodified variant thereof.

A RNAi agent comprising a first and a second strand, wherein the sequences of the first and/or second strands comprise the sequences of a first and/or second strand of: AD-39787.1 (SEQ ID NOs: 269 and 696), or a modified or unmodified variant thereof.

A RNAi agent comprising a first and a second strand, wherein the sequences of the first and/or second strands comprise the sequences of a first and/or second strand of: AD-39793.1 (SEQ ID NOs: 270 and 697), or a modified or unmodified variant thereof.

A RNAi agent comprising a first and a second strand, wherein the sequences of the first and/or second strands comprise the sequences of a first and/or second strand of: AD-39799.1 (SEQ ID NOs: 271 and 698), or a modified or unmodified variant thereof.

A RNAi agent comprising a first and a second strand, wherein the sequences of the first and/or second strands comprise the sequences of a first and/or second strand of: AD-39805.1 (SEQ ID NOs: 272 and 699), or a modified or unmodified variant thereof.

A RNAi agent comprising a first and a second strand, wherein the sequences of the first and/or second strands comprise the sequences of a first and/or second strand of: AD-39811.1 (SEQ ID NOs: 273 and 700), or a modified or unmodified variant thereof.

A RNAi agent comprising a first and a second strand, wherein the sequences of the first and/or second strands comprise the sequences of a first and/or second strand of: AD-39817.1 (SEQ ID NOs: 274 and 701), or a modified or unmodified variant thereof.

A RNAi agent comprising a first and a second strand, wherein the sequences of the first and/or second strands comprise the sequences of a first and/or second strand of: AD-39823.1 (SEQ ID NOs: 275 and 702), or a modified or unmodified variant thereof.

A RNAi agent comprising a first and a second strand, wherein the sequences of the first and/or second strands comprise the sequences of a first and/or second strand of: AD-39782.1 (SEQ ID NOs: 276 and 703), or a modified or unmodified variant thereof.

A RNAi agent comprising a first and a second strand, wherein the sequences of the first and/or second strands comprise the sequences of a first and/or second strand of: AD-39788.1 (SEQ ID NOs: 277 and 704), or a modified or unmodified variant thereof.

A RNAi agent comprising a first and a second strand, wherein the sequences of the first and/or second strands comprise the sequences of a first and/or second strand of: AD-39794.1 (SEQ ID NOs: 278 and 705), or a modified or unmodified variant thereof.

A RNAi agent comprising a first and a second strand, wherein the sequences of the first and/or second strands comprise the sequences of a first and/or second strand of: AD-39800.1 (SEQ ID NOs: 279 and 706), or a modified or unmodified variant thereof.

A RNAi agent comprising a first and a second strand, wherein the sequences of the first and/or second strands comprise the sequences of a first and/or second strand of: AD-39806.1 (SEQ ID NOs: 280 and 707), or a modified or unmodified variant thereof.

A RNAi agent comprising a first and a second strand, wherein the sequences of the first and/or second strands comprise the sequences of a first and/or second strand of: AD-39812.1 (SEQ ID NOs: 281 and 708), or a modified or unmodified variant thereof.

A RNAi agent comprising a first and a second strand, wherein the sequences of the first and/or second strands comprise the sequences of a first and/or second strand of: AD-39818.1 (SEQ ID NOs: 282 and 709), or a modified or unmodified variant thereof.

A RNAi agent comprising a first and a second strand, wherein the sequences of the first and/or second strands comprise the sequences of a first and/or second strand of: AD-39824.1 (SEQ ID NOs: 283 and 710), or a modified or unmodified variant thereof.

A RNAi agent comprising a first and a second strand, wherein the sequences of the first and/or second strands comprise the sequences of a first and/or second strand of: AD-39783.1 (SEQ ID NOs: 284 and 711), or a modified or unmodified variant thereof.

A RNAi agent comprising a first and a second strand, wherein the sequences of the first and/or second strands comprise the sequences of a first and/or second strand of: AD-39789.1 (SEQ ID NOs: 285 and 712), or a modified or unmodified variant thereof.

A RNAi agent comprising a first and a second strand, wherein the sequences of the first and/or second strands comprise the sequences of a first and/or second strand of: AD-39795.1 (SEQ ID NOs: 286 and 713), or a modified or unmodified variant thereof.

A RNAi agent comprising a first and a second strand, wherein the sequences of the first and/or second strands comprise the sequences of a first and/or second strand of: AD-39801.1 (SEQ ID NOs: 287 and 714), or a modified or unmodified variant thereof.

A RNAi agent comprising a first and a second strand, wherein the sequences of the first and/or second strands comprise the sequences of a first and/or second strand of: AD-39807.1 (SEQ ID NOs: 288 and 715), or a modified or unmodified variant thereof.

A RNAi agent comprising a first and a second strand, wherein the sequences of the first and/or second strands comprise the sequences of a first and/or second strand of: AD-39813.1 (SEQ ID NOs: 289 and 716), or a modified or unmodified variant thereof.

A RNAi agent comprising a first and a second strand, wherein the sequences of the first and/or second strands comprise the sequences of a first and/or second strand of: AD-39819.1 (SEQ ID NOs: 290 and 717), or a modified or unmodified variant thereof.

A RNAi agent comprising a first and a second strand, wherein the sequences of the first and/or second strands comprise the sequences of a first and/or second strand of: AD-39825.1 (SEQ ID NOs: 291 and 718), or a modified or unmodified variant thereof.

A RNAi agent comprising a first and a second strand, wherein the sequences of the first and/or second strands comprise the sequences of a first and/or second strand of: AD-39831.1 (SEQ ID NOs: 292 and 719), or a modified or unmodified variant thereof.

A RNAi agent comprising a first and a second strand, wherein the sequences of the first and/or second strands comprise the sequences of a first and/or second strand of: AD-39837.1 (SEQ ID NOs: 293 and 720), or a modified or unmodified variant thereof.

A RNAi agent comprising a first and a second strand, wherein the sequences of the first and/or second strands comprise the sequences of a first and/or second strand of: AD-39843.1 (SEQ ID NOs: 294 and 721), or a modified or unmodified variant thereof.

A RNAi agent comprising a first and a second strand, wherein the sequences of the first and/or second strands comprise the sequences of a first and/or second strand of: AD-39849.1 (SEQ ID NOs: 295 and 722), or a modified or unmodified variant thereof.

A RNAi agent comprising a first and a second strand, wherein the sequences of the first and/or second strands comprise the sequences of a first and/or second strand of: AD-39855.1 (SEQ ID NOs: 296 and 723), or a modified or unmodified variant thereof.

A RNAi agent comprising a first and a second strand, wherein the sequences of the first and/or second strands comprise the sequences of a first and/or second strand of: AD-39861.1 (SEQ ID NOs: 297 and 724), or a modified or unmodified variant thereof.

A RNAi agent comprising a first and a second strand, wherein the sequences of the first and/or second strands comprise the sequences of a first and/or second strand of: AD-39867.1 (SEQ ID NOs: 298 and 725), or a modified or unmodified variant thereof.

A RNAi agent comprising a first and a second strand, wherein the sequences of the first and/or second strands comprise the sequences of a first and/or second strand of: AD-39826.1 (SEQ ID NOs: 299 and 726), or a modified or unmodified variant thereof.

A RNAi agent comprising a first and a second strand, wherein the sequences of the first and/or second strands comprise the sequences of a first and/or second strand of: AD-39832.1 (SEQ ID NOs: 300 and 727), or a modified or unmodified variant thereof.

A RNAi agent comprising a first and a second strand, wherein the sequences of the first and/or second strands comprise the sequences of a first and/or second strand of: AD-39838.1 (SEQ ID NOs: 301 and 728), or a modified or unmodified variant thereof.

A RNAi agent comprising a first and a second strand, wherein the sequences of the first and/or second strands comprise the sequences of a first and/or second strand of: AD-39844.1 (SEQ ID NOs: 302 and 729), or a modified or unmodified variant thereof.

A RNAi agent comprising a first and a second strand, wherein the sequences of the first and/or second strands comprise the sequences of a first and/or second strand of: AD-39850.1 (SEQ ID NOs: 303 and 730), or a modified or unmodified variant thereof.

A RNAi agent comprising a first and a second strand, wherein the sequences of the first and/or second strands comprise the sequences of a first and/or second strand of: AD-39856.1 (SEQ ID NOs: 304 and 731), or a modified or unmodified variant thereof.

A RNAi agent comprising a first and a second strand, wherein the sequences of the first and/or second strands comprise the sequences of a first and/or second strand of: AD-39862.1 (SEQ ID NOs: 305 and 732), or a modified or unmodified variant thereof.

A RNAi agent comprising a first and a second strand, wherein the sequences of the first and/or second strands comprise the sequences of a first and/or second strand of: AD-39868.1 (SEQ ID NOs: 306 and 733), or a modified or unmodified variant thereof.

A RNAi agent comprising a first and a second strand, wherein the sequences of the first and/or second strands comprise the sequences of a first and/or second strand of: AD-39827.1 (SEQ ID NOs: 307 and 734), or a modified or unmodified variant thereof.

A RNAi agent comprising a first and a second strand, wherein the sequences of the first and/or second strands comprise the sequences of a first and/or second strand of: AD-39833.1 (SEQ ID NOs: 308 and 735), or a modified or unmodified variant thereof.

A RNAi agent comprising a first and a second strand, wherein the sequences of the first and/or second strands comprise the sequences of a first and/or second strand of: AD-39839.1 (SEQ ID NOs: 309 and 736), or a modified or unmodified variant thereof.

A RNAi agent comprising a first and a second strand, wherein the sequences of the first and/or second strands comprise the sequences of a first and/or second strand of: AD-39845.1 (SEQ ID NOs: 310 and 737), or a modified or unmodified variant thereof.

A RNAi agent comprising a first and a second strand, wherein the sequences of the first and/or second strands comprise the sequences of a first and/or second strand of: AD-39851.1 (SEQ ID NOs: 311 and 738), or a modified or unmodified variant thereof.

A RNAi agent comprising a first and a second strand, wherein the sequences of the first and/or second strands comprise the sequences of a first and/or second strand of: AD-39857.1 (SEQ ID NOs: 312 and 739), or a modified or unmodified variant thereof.

A RNAi agent comprising a first and a second strand, wherein the sequences of the first and/or second strands comprise the sequences of a first and/or second strand of: AD-39863.1 (SEQ ID NOs: 313 and 740), or a modified or unmodified variant thereof.

A RNAi agent comprising a first and a second strand, wherein the sequences of the first and/or second strands comprise the sequences of a first and/or second strand of: AD-39869.1 (SEQ ID NOs: 314 and 741), or a modified or unmodified variant thereof.

A RNAi agent comprising a first and a second strand, wherein the sequences of the first and/or second strands comprise the sequences of a first and/or second strand of: AD-39828.1 (SEQ ID NOs: 315 and 742), or a modified or unmodified variant thereof.

A RNAi agent comprising a first and a second strand, wherein the sequences of the first and/or second strands comprise the sequences of a first and/or second strand of: AD-39834.1 (SEQ ID NOs: 316 and 743), or a modified or unmodified variant thereof.

A RNAi agent comprising a first and a second strand, wherein the sequences of the first and/or second strands comprise the sequences of a first and/or second strand of: AD-39840.1 (SEQ ID NOs: 317 and 744), or a modified or unmodified variant thereof.

A RNAi agent comprising a first and a second strand, wherein the sequences of the first and/or second strands comprise the sequences of a first and/or second strand of: AD-39846.1 (SEQ ID NOs: 318 and 745), or a modified or unmodified variant thereof.

A RNAi agent comprising a first and a second strand, wherein the sequences of the first and/or second strands comprise the sequences of a first and/or second strand of: AD-39852.1 (SEQ ID NOs: 319 and 746), or a modified or unmodified variant thereof.

A RNAi agent comprising a first and a second strand, wherein the sequences of the first and/or second strands comprise the sequences of a first and/or second strand of: AD-39858.1 (SEQ ID NOs: 320 and 747), or a modified or unmodified variant thereof.

A RNAi agent comprising a first and a second strand, wherein the sequences of the first and/or second strands comprise the sequences of a first and/or second strand of: AD-39864.1 (SEQ ID NOs: 321 and 748), or a modified or unmodified variant thereof.

A RNAi agent comprising a first and a second strand, wherein the sequences of the first and/or second strands comprise the sequences of a first and/or second strand of: AD-39870.1 (SEQ ID NOs: 322 and 749), or a modified or unmodified variant thereof.

A RNAi agent comprising a first and a second strand, wherein the sequences of the first and/or second strands comprise the sequences of a first and/or second strand of: AD-39829.1 (SEQ ID NOs: 323 and 750), or a modified or unmodified variant thereof.

A RNAi agent comprising a first and a second strand, wherein the sequences of the first and/or second strands comprise the sequences of a first and/or second strand of: AD-39835.1 (SEQ ID NOs: 324 and 751), or a modified or unmodified variant thereof.

A RNAi agent comprising a first and a second strand, wherein the sequences of the first and/or second strands comprise the sequences of a first and/or second strand of: AD-39841.1 (SEQ ID NOs: 325 and 752), or a modified or unmodified variant thereof.

A RNAi agent comprising a first and a second strand, wherein the sequences of the first and/or second strands comprise the sequences of a first and/or second strand of: AD-39853.1 (SEQ ID NOs: 326 and 753), or a modified or unmodified variant thereof.

A RNAi agent comprising a first and a second strand, wherein the sequences of the first and/or second strands comprise the sequences of a first and/or second strand of: AD-39859.1 (SEQ ID NOs: 327 and 754), or a modified or unmodified variant thereof.

A RNAi agent comprising a first and a second strand, wherein the sequences of the first and/or second strands comprise the sequences of a first and/or second strand of: AD-39865.1 (SEQ ID NOs: 328 and 755), or a modified or unmodified variant thereof.

A RNAi agent comprising a first and a second strand, wherein the sequences of the first and/or second strands comprise the sequences of a first and/or second strand of: AD-39871.1 (SEQ ID NOs: 329 and 756), or a modified or unmodified variant thereof.

A RNAi agent comprising a first and a second strand, wherein the sequences of the first and/or second strands comprise the sequences of a first and/or second strand of: AD-39830.1 (SEQ ID NOs: 330 and 757), or a modified or unmodified variant thereof.

A RNAi agent comprising a first and a second strand, wherein the sequences of the first and/or second strands comprise the sequences of a first and/or second strand of: AD-39836.1 (SEQ ID NOs: 331 and 758), or a modified or unmodified variant thereof.

A RNAi agent comprising a first and a second strand, wherein the sequences of the first and/or second strands comprise the sequences of a first and/or second strand of: AD-39842.1 (SEQ ID NOs: 332 and 759), or a modified or unmodified variant thereof.

A RNAi agent comprising a first and a second strand, wherein the sequences of the first and/or second strands comprise the sequences of a first and/or second strand of: AD-39848.1 (SEQ ID NOs: 333 and 760), or a modified or unmodified variant thereof.

A RNAi agent comprising a first and a second strand, wherein the sequences of the first and/or second strands comprise the sequences of a first and/or second strand of: AD-39854.1 (SEQ ID NOs: 334 and 761), or a modified or unmodified variant thereof.

A RNAi agent comprising a first and a second strand, wherein the sequences of the first and/or second strands comprise the sequences of a first and/or second strand of: AD-39860.1 (SEQ ID NOs: 335 and 762), or a modified or unmodified variant thereof.

A RNAi agent comprising a first and a second strand, wherein the sequences of the first and/or second strands comprise the sequences of a first and/or second strand of: AD-39866.1 (SEQ ID NOs: 336 and 763), or a modified or unmodified variant thereof.

A RNAi agent comprising a first and a second strand, wherein the sequences of the first and/or second strands comprise the sequences of a first and/or second strand of: AD-39992.1 (SEQ ID NOs: 337 and 764), or a modified or unmodified variant thereof.

A RNAi agent comprising a first and a second strand, wherein the sequences of the first and/or second strands comprise the sequences of a first and/or second strand of: AD-39998.1 (SEQ ID NOs: 338 and 765), or a modified or unmodified variant thereof.

A RNAi agent comprising a first and a second strand, wherein the sequences of the first and/or second strands comprise the sequences of a first and/or second strand of: AD-40004.1 (SEQ ID NOs: 339 and 766), or a modified or unmodified variant thereof.

A RNAi agent comprising a first and a second strand, wherein the sequences of the first and/or second strands comprise the sequences of a first and/or second strand of: AD-40010.1 (SEQ ID NOs: 340 and 767), or a modified or unmodified variant thereof.

A RNAi agent comprising a first and a second strand, wherein the sequences of the first and/or second strands comprise the sequences of a first and/or second strand of: AD-40016.1 (SEQ ID NOs: 341 and 768), or a modified or unmodified variant thereof.

A RNAi agent comprising a first and a second strand, wherein the sequences of the first and/or second strands comprise the sequences of a first and/or second strand of: AD-40022.1 (SEQ ID NOs: 342 and 769), or a modified or unmodified variant thereof.

A RNAi agent comprising a first and a second strand, wherein the sequences of the first and/or second strands comprise the sequences of a first and/or second strand of: AD-40028.1 (SEQ ID NOs: 343 and 770), or a modified or unmodified variant thereof.

A RNAi agent comprising a first and a second strand, wherein the sequences of the first and/or second strands comprise the sequences of a first and/or second strand of: AD-40034.1 (SEQ ID NOs: 344 and 771), or a modified or unmodified variant thereof.

A RNAi agent comprising a first and a second strand, wherein the sequences of the first and/or second strands comprise the sequences of a first and/or second strand of: AD-39999.1 (SEQ ID NOs: 345 and 772), or a modified or unmodified variant thereof.

A RNAi agent comprising a first and a second strand, wherein the sequences of the first and/or second strands comprise the sequences of a first and/or second strand of: AD-40005.1 (SEQ ID NOs: 346 and 773), or a modified or unmodified variant thereof.

A RNAi agent comprising a first and a second strand, wherein the sequences of the first and/or second strands comprise the sequences of a first and/or second strand of: AD-40011.1 (SEQ ID NOs: 347 and 774), or a modified or unmodified variant thereof.

A RNAi agent comprising a first and a second strand, wherein the sequences of the first and/or second strands comprise the sequences of a first and/or second strand of: AD-40017.1 (SEQ ID NOs: 348 and 775), or a modified or unmodified variant thereof.

A RNAi agent comprising a first and a second strand, wherein the sequences of the first and/or second strands comprise the sequences of a first and/or second strand of: AD-40029.1 (SEQ ID NOs: 349 and 776), or a modified or unmodified variant thereof.

A RNAi agent comprising a first and a second strand, wherein the sequences of the first and/or second strands comprise the sequences of a first and/or second strand of: AD-40035.1 (SEQ ID NOs: 350 and 777), or a modified or unmodified variant thereof.

A RNAi agent comprising a first and a second strand, wherein the sequences of the first and/or second strands comprise the sequences of a first and/or second strand of: AD-39994.1 (SEQ ID NOs: 351 and 778), or a modified or unmodified variant thereof.

A RNAi agent comprising a first and a second strand, wherein the sequences of the first and/or second strands comprise the sequences of a first and/or second strand of: AD-40000.1 (SEQ ID NOs: 352 and 779), or a modified or unmodified variant thereof.

A RNAi agent comprising a first and a second strand, wherein the sequences of the first and/or second strands comprise the sequences of a first and/or second strand of: AD-40006.1 (SEQ ID NOs: 353 and 780), or a modified or unmodified variant thereof.

A RNAi agent comprising a first and a second strand, wherein the sequences of the first and/or second strands comprise the sequences of a first and/or second strand of: AD-40012.1 (SEQ ID NOs: 354 and 781), or a modified or unmodified variant thereof.

A RNAi agent comprising a first and a second strand, wherein the sequences of the first and/or second strands comprise the sequences of a first and/or second strand of: AD-40018.1 (SEQ ID NOs: 355 and 782), or a modified or unmodified variant thereof.

A RNAi agent comprising a first and a second strand, wherein the sequences of the first and/or second strands comprise the sequences of a first and/or second strand of: AD-40024.1 (SEQ ID NOs: 356 and 783), or a modified or unmodified variant thereof.

A RNAi agent comprising a first and a second strand, wherein the sequences of the first and/or second strands comprise the sequences of a first and/or second strand of: AD-40030.1 (SEQ ID NOs: 357 and 784), or a modified or unmodified variant thereof.

A RNAi agent comprising a first and a second strand, wherein the sequences of the first and/or second strands comprise the sequences of a first and/or second strand of: AD-40036.1 (SEQ ID NOs: 358 and 785), or a modified or unmodified variant thereof.

A RNAi agent comprising a first and a second strand, wherein the sequences of the first and/or second strands comprise the sequences of a first and/or second strand of: AD-39995.1 (SEQ ID NOs: 359 and 786), or a modified or unmodified variant thereof.

A RNAi agent comprising a first and a second strand, wherein the sequences of the first and/or second strands comprise the sequences of a first and/or second strand of: AD-40001.1 (SEQ ID NOs: 360 and 787), or a modified or unmodified variant thereof.

A RNAi agent comprising a first and a second strand, wherein the sequences of the first and/or second strands comprise the sequences of a first and/or second strand of: AD-40007.1 (SEQ ID NOs: 361 and 788), or a modified or unmodified variant thereof.

A RNAi agent comprising a first and a second strand, wherein the sequences of the first and/or second strands comprise the sequences of a first and/or second strand of: AD-40013.1 (SEQ ID NOs: 362 and 789), or a modified or unmodified variant thereof.

A RNAi agent comprising a first and a second strand, wherein the sequences of the first and/or second strands comprise the sequences of a first and/or second strand of: AD-40019.1 (SEQ ID NOs: 363 and 790), or a modified or unmodified variant thereof.

A RNAi agent comprising a first and a second strand, wherein the sequences of the first and/or second strands comprise the sequences of a first and/or second strand of: AD-40025.1 (SEQ ID NOs: 364 and 791), or a modified or unmodified variant thereof.

A RNAi agent comprising a first and a second strand, wherein the sequences of the first and/or second strands comprise the sequences of a first and/or second strand of: AD-40031.1 (SEQ ID NOs: 365 and 792), or a modified or unmodified variant thereof.

A RNAi agent comprising a first and a second strand, wherein the sequences of the first and/or second strands comprise the sequences of a first and/or second strand of: AD-40037.1 (SEQ ID NOs: 366 and 793), or a modified or unmodified variant thereof.

A RNAi agent comprising a first and a second strand, wherein the sequences of the first and/or second strands comprise the sequences of a first and/or second strand of: AD-39996.1 (SEQ ID NOs: 367 and 794), or a modified or unmodified variant thereof.

A RNAi agent comprising a first and a second strand, wherein the sequences of the first and/or second strands comprise the sequences of a first and/or second strand of: AD-40002.1 (SEQ ID NOs: 368 and 795), or a modified or unmodified variant thereof.

A RNAi agent comprising a first and a second strand, wherein the sequences of the first and/or second strands comprise the sequences of a first and/or second strand of: AD-40008.1 (SEQ ID NOs: 369 and 796), or a modified or unmodified variant thereof.

A RNAi agent comprising a first and a second strand, wherein the sequences of the first and/or second strands comprise the sequences of a first and/or second strand of: AD-40014.1 (SEQ ID NOs: 370 and 797), or a modified or unmodified variant thereof.

A RNAi agent comprising a first and a second strand, wherein the sequences of the first and/or second strands comprise the sequences of a first and/or second strand of: AD-40020.1 (SEQ ID NOs: 371 and 798), or a modified or unmodified variant thereof.

A RNAi agent comprising a first and a second strand, wherein the sequences of the first and/or second strands comprise the sequences of a first and/or second strand of: AD-40026.1 (SEQ ID NOs: 372 and 799), or a modified or unmodified variant thereof.

A RNAi agent comprising a first and a second strand, wherein the sequences of the first and/or second strands comprise the sequences of a first and/or second strand of: AD-40032.1 (SEQ ID NOs: 373 and 800), or a modified or unmodified variant thereof.

A RNAi agent comprising a first and a second strand, wherein the sequences of the first and/or second strands comprise the sequences of a first and/or second strand of: AD-40038.1 (SEQ ID NOs: 374 and 801), or a modified or unmodified variant thereof.

A RNAi agent comprising a first and a second strand, wherein the sequences of the first and/or second strands comprise the sequences of a first and/or second strand of: AD-39997.1 (SEQ ID NOs: 375 and 802), or a modified or unmodified variant thereof.

A RNAi agent comprising a first and a second strand, wherein the sequences of the first and/or second strands comprise the sequences of a first and/or second strand of: AD-40009.1 (SEQ ID NOs: 376 and 803), or a modified or unmodified variant thereof.

A RNAi agent comprising a first and a second strand, wherein the sequences of the first and/or second strands comprise the sequences of a first and/or second strand of: AD-40015.1 (SEQ ID NOs: 377 and 804), or a modified or unmodified variant thereof.

A RNAi agent comprising a first and a second strand, wherein the sequences of the first and/or second strands comprise the sequences of a first and/or second strand of: AD-40021.1 (SEQ ID NOs: 378 and 805), or a modified or unmodified variant thereof.

A RNAi agent comprising a first and a second strand, wherein the sequences of the first and/or second strands comprise the sequences of a first and/or second strand of: AD-40027.1 (SEQ ID NOs: 379 and 806), or a modified or unmodified variant thereof.

A RNAi agent comprising a first and a second strand, wherein the sequences of the first and/or second strands comprise the sequences of a first and/or second strand of: AD-40033.1 (SEQ ID NOs: 380 and 807), or a modified or unmodified variant thereof.

A RNAi agent comprising a first and a second strand, wherein the sequences of the first and/or second strands comprise the sequences of a first and/or second strand of: AD-40039.1 (SEQ ID NOs: 381 and 808), or a modified or unmodified variant thereof.

A RNAi agent comprising a first and a second strand, wherein the sequences of the first and/or second strands comprise the sequences of a first and/or second strand of: AD-40045.1 (SEQ ID NOs: 382 and 809), or a modified or unmodified variant thereof.

A RNAi agent comprising a first and a second strand, wherein the sequences of the first and/or second strands comprise the sequences of a first and/or second strand of: AD-40051.1 (SEQ ID NOs: 383 and 810), or a modified or unmodified variant thereof.

A RNAi agent comprising a first and a second strand, wherein the sequences of the first and/or second strands comprise the sequences of a first and/or second strand of: AD-40057.1 (SEQ ID NOs: 384 and 811), or a modified or unmodified variant thereof.

A RNAi agent comprising a first and a second strand, wherein the sequences of the first and/or second strands comprise the sequences of a first and/or second strand of: AD-40063.1 (SEQ ID NOs: 385 and 812), or a modified or unmodified variant thereof.

A RNAi agent comprising a first and a second strand, wherein the sequences of the first and/or second strands comprise the sequences of a first and/or second strand of: AD-40069.1 (SEQ ID NOs: 386 and 813), or a modified or unmodified variant thereof.

A RNAi agent comprising a first and a second strand, wherein the sequences of the first and/or second strands comprise the sequences of a first and/or second strand of: AD-40075.1 (SEQ ID NOs: 387 and 814), or a modified or unmodified variant thereof.

A RNAi agent comprising a first and a second strand, wherein the sequences of the first and/or second strands comprise the sequences of a first and/or second strand of: AD-40081.1 (SEQ ID NOs: 388 and 815), or a modified or unmodified variant thereof.

A RNAi agent comprising a first and a second strand, wherein the sequences of the first and/or second strands comprise the sequences of a first and/or second strand of: AD-40040.1 (SEQ ID NOs: 389 and 816), or a modified or unmodified variant thereof.

A RNAi agent comprising a first and a second strand, wherein the sequences of the first and/or second strands comprise the sequences of a first and/or second strand of: AD-40046.1 (SEQ ID NOs: 390 and 817), or a modified or unmodified variant thereof.

A RNAi agent comprising a first and a second strand, wherein the sequences of the first and/or second strands comprise the sequences of a first and/or second strand of: AD-40052.1 (SEQ ID NOs: 391 and 818), or a modified or unmodified variant thereof.

A RNAi agent comprising a first and a second strand, wherein the sequences of the first and/or second strands comprise the sequences of a first and/or second strand of: AD-40058.1 (SEQ ID NOs: 392 and 819), or a modified or unmodified variant thereof.

A RNAi agent comprising a first and a second strand, wherein the sequences of the first and/or second strands comprise the sequences of a first and/or second strand of: AD-40064.1 (SEQ ID NOs: 393 and 820), or a modified or unmodified variant thereof.

A RNAi agent comprising a first and a second strand, wherein the sequences of the first and/or second strands comprise the sequences of a first and/or second strand of: AD-40070.1 (SEQ ID NOs: 394 and 821), or a modified or unmodified variant thereof.

A RNAi agent comprising a first and a second strand, wherein the sequences of the first and/or second strands comprise the sequences of a first and/or second strand of: AD-40076.1 (SEQ ID NOs: 395 and 822), or a modified or unmodified variant thereof.

A RNAi agent comprising a first and a second strand, wherein the sequences of the first and/or second strands comprise the sequences of a first and/or second strand of: AD-40082.1 (SEQ ID NOs: 396 and 823), or a modified or unmodified variant thereof.

A RNAi agent comprising a first and a second strand, wherein the sequences of the first and/or second strands comprise the sequences of a first and/or second strand of: AD-40041.1 (SEQ ID NOs: 397 and 824), or a modified or unmodified variant thereof.

A RNAi agent comprising a first and a second strand, wherein the sequences of the first and/or second strands comprise the sequences of a first and/or second strand of: AD-40047.1 (SEQ ID NOs: 398 and 825), or a modified or unmodified variant thereof.

A RNAi agent comprising a first and a second strand, wherein the sequences of the first and/or second strands comprise the sequences of a first and/or second strand of: AD-40053.1 (SEQ ID NOs: 399 and 826), or a modified or unmodified variant thereof.

A RNAi agent comprising a first and a second strand, wherein the sequences of the first and/or second strands comprise the sequences of a first and/or second strand of: AD-40059.1 (SEQ ID NOs: 400 and 827), or a modified or unmodified variant thereof.

A RNAi agent comprising a first and a second strand, wherein the sequences of the first and/or second strands comprise the sequences of a first and/or second strand of: AD-40065.1 (SEQ ID NOs: 401 and 828), or a modified or unmodified variant thereof.

A RNAi agent comprising a first and a second strand, wherein the sequences of the first and/or second strands comprise the sequences of a first and/or second strand of: AD-40071.1 (SEQ ID NOs: 402 and 829), or a modified or unmodified variant thereof.

A RNAi agent comprising a first and a second strand, wherein the sequences of the first and/or second strands comprise the sequences of a first and/or second strand of: AD-40077.1 (SEQ ID NOs: 403 and 830), or a modified or unmodified variant thereof.

A RNAi agent comprising a first and a second strand, wherein the sequences of the first and/or second strands comprise the sequences of a first and/or second strand of: AD-40083.1 (SEQ ID NOs: 404 and 831), or a modified or unmodified variant thereof.

A RNAi agent comprising a first and a second strand, wherein the sequences of the first and/or second strands comprise the sequences of a first and/or second strand of: AD-40042.1 (SEQ ID NOs: 405 and 832), or a modified or unmodified variant thereof.

A RNAi agent comprising a first and a second strand, wherein the sequences of the first and/or second strands comprise the sequences of a first and/or second strand of: AD-40048.1 (SEQ ID NOs: 406 and 833), or a modified or unmodified variant thereof.

A RNAi agent comprising a first and a second strand, wherein the sequences of the first and/or second strands comprise the sequences of a first and/or second strand of: AD-40054.1 (SEQ ID NOs: 407 and 834), or a modified or unmodified variant thereof.

A RNAi agent comprising a first and a second strand, wherein the sequences of the first and/or second strands comprise the sequences of a first and/or second strand of: AD-40060.1 (SEQ ID NOs: 408 and 835), or a modified or unmodified variant thereof.

A RNAi agent comprising a first and a second strand, wherein the sequences of the first and/or second strands comprise the sequences of a first and/or second strand of: AD-40066.1 (SEQ ID NOs: 409 and 836), or a modified or unmodified variant thereof.

A RNAi agent comprising a first and a second strand, wherein the sequences of the first and/or second strands comprise the sequences of a first and/or second strand of: AD-40072.1 (SEQ ID NOs: 410 and 837), or a modified or unmodified variant thereof.

A RNAi agent comprising a first and a second strand, wherein the sequences of the first and/or second strands comprise the sequences of a first and/or second strand of: AD-40078.1 (SEQ ID NOs: 411 and 838), or a modified or unmodified variant thereof.

A RNAi agent comprising a first and a second strand, wherein the sequences of the first and/or second strands comprise the sequences of a first and/or second strand of: AD-40084.1 (SEQ ID NOs: 412 and 839), or a modified or unmodified variant thereof.

A RNAi agent comprising a first and a second strand, wherein the sequences of the first and/or second strands comprise the sequences of a first and/or second strand of: AD-40043.1 (SEQ ID NOs: 413 and 840), or a modified or unmodified variant thereof.

A RNAi agent comprising a first and a second strand, wherein the sequences of the first and/or second strands comprise the sequences of a first and/or second strand of: AD-40049.1 (SEQ ID NOs: 414 and 841), or a modified or unmodified variant thereof.

A RNAi agent comprising a first and a second strand, wherein the sequences of the first and/or second strands comprise the sequences of a first and/or second strand of: AD-40055.1 (SEQ ID NOs: 415 and 842), or a modified or unmodified variant thereof.

A RNAi agent comprising a first and a second strand, wherein the sequences of the first and/or second strands comprise the sequences of a first and/or second strand of: AD-40061.1 (SEQ ID NOs: 416 and 843), or a modified or unmodified variant thereof.

A RNAi agent comprising a first and a second strand, wherein the sequences of the first and/or second strands comprise the sequences of a first and/or second strand of: AD-40067.1 (SEQ ID NOs: 417 and 844), or a modified or unmodified variant thereof.

A RNAi agent comprising a first and a second strand, wherein the sequences of the first and/or second strands comprise the sequences of a first and/or second strand of: AD-40073.1 (SEQ ID NOs: 418 and 845), or a modified or unmodified variant thereof.

A RNAi agent comprising a first and a second strand, wherein the sequences of the first and/or second strands comprise the sequences of a first and/or second strand of: AD-40079.1 (SEQ ID NOs: 419 and 846), or a modified or unmodified variant thereof.

A RNAi agent comprising a first and a second strand, wherein the sequences of the first and/or second strands comprise the sequences of a first and/or second strand of: AD-40085.1 (SEQ ID NOs: 420 and 847), or a modified or unmodified variant thereof.

A RNAi agent comprising a first and a second strand, wherein the sequences of the first and/or second strands comprise the sequences of a first and/or second strand of: AD-40044.1 (SEQ ID NOs: 421 and 848), or a modified or unmodified variant thereof.

A RNAi agent comprising a first and a second strand, wherein the sequences of the first and/or second strands comprise the sequences of a first and/or second strand of: AD-40050.1 (SEQ ID NOs: 422 and 849), or a modified or unmodified variant thereof.

A RNAi agent comprising a first and a second strand, wherein the sequences of the first and/or second strands comprise the sequences of a first and/or second strand of: AD-40056.1 (SEQ ID NOs: 423 and 850), or a modified or unmodified variant thereof.

A RNAi agent comprising a first and a second strand, wherein the sequences of the first and/or second strands comprise the sequences of a first and/or second strand of: AD-40062.1 (SEQ ID NOs: 424 and 851), or a modified or unmodified variant thereof.

A RNAi agent comprising a first and a second strand, wherein the sequences of the first and/or second strands comprise the sequences of a first and/or second strand of: AD-40068.1 (SEQ ID NOs: 425 and 852), or a modified or unmodified variant thereof.

A RNAi agent comprising a first and a second strand, wherein the sequences of the first and/or second strands comprise the sequences of a first and/or second strand of: AD-40074.1 (SEQ ID NOs: 426 and 853), or a modified or unmodified variant thereof.

A RNAi agent comprising a first and a second strand, wherein the sequences of the first and/or second strands comprise the sequences of a first and/or second strand of: AD-40080.1 (SEQ ID NOs: 427 and 854), or a modified or unmodified variant thereof.

A RNAi agent comprising a first and a second strand, wherein the sequences of the first and/or second strands comprise the sequences of a first and/or second strand of: AD-39720.1 (SEQ ID NOs: 1716 and 1754), or a modified or unmodified variant thereof.

A RNAi agent comprising a first and a second strand, wherein the sequences of the first and/or second strands comprise the sequences of a first and/or second strand of: AD-39706.1 (SEQ ID NOs: 1717 and 1755), or a modified or unmodified variant thereof.

A RNAi agent comprising a first and a second strand, wherein the sequences of the first and/or second strands comprise the sequences of a first and/or second strand of: AD-39712.1 (SEQ ID NOs: 1718 and 1756), or a modified or unmodified variant thereof.

A RNAi agent comprising a first and a second strand, wherein the sequences of the first and/or second strands comprise the sequences of a first and/or second strand of: AD-39760.1 (SEQ ID NOs: 1719 and 1757), or a modified or unmodified variant thereof.

A RNAi agent comprising a first and a second strand, wherein the sequences of the first and/or second strands comprise the sequences of a first and/or second strand of: AD-39732.1 (SEQ ID NOs: 1720 and 1758), or a modified or unmodified variant thereof.

A RNAi agent comprising a first and a second strand, wherein the sequences of the first and/or second strands comprise the sequences of a first and/or second strand of: AD-35541.4 (SEQ ID NOs: 1721 and 1759), or a modified or unmodified variant thereof.

A RNAi agent comprising a first and a second strand, wherein the sequences of the first and/or second strands comprise the sequences of a first and/or second strand of: AD-39735.1 or hs_KRAS_321_A22S26 (SEQ ID NOs: 1722 and 1760), or a modified or unmodified variant thereof.

A RNAi agent comprising a first and a second strand, wherein the sequences of the first and/or second strands comprise the sequences of a first and/or second strand of: AD-39741.1, or modified or unmodified variants thereof.

A RNAi agent comprising a first and a second strand, wherein the sequences of the first and/or second strands comprise the sequences of a first and/or second strand of: hs_KRAS_322_A22S26 (SEQ ID NOs: 1723 and 1761), or a modified or unmodified variant thereof.

A RNAi agent comprising a first and a second strand, wherein the sequences of the first and/or second strands comprise the sequences of a first and/or second strand of: AD-39778.1 (SEQ ID NOs: 1724 and 1762), or a modified or unmodified variant thereof.

A RNAi agent comprising a first and a second strand, wherein the sequences of the first and/or second strands comprise the sequences of a first and/or second strand of: AD-39790.1 (SEQ ID NOs: 1725 and 1763), or a modified or unmodified variant thereof.

A RNAi agent comprising a first and a second strand, wherein the sequences of the first and/or second strands comprise the sequences of a first and/or second strand of: AD-39822.1 (SEQ ID NOs: 1726 and 1764), or a modified or unmodified variant thereof.

A RNAi agent comprising a first and a second strand, wherein the sequences of the first and/or second strands comprise the sequences of a first and/or second strand of: AD-35609.4 (SEQ ID NOs: 1727 and 1765), or a modified or unmodified variant thereof.

A RNAi agent comprising a first and a second strand, wherein the sequences of the first and/or second strands comprise the sequences of a first and/or second strand of: AD-35581.4 (SEQ ID NOs: 1728 and 1766), or a modified or unmodified variant thereof.

A RNAi agent comprising a first and a second strand, wherein the sequences of the first and/or second strands comprise the sequences of a first and/or second strand of: AD-39845.1 (SEQ ID NOs: 1729 and 1767), or a modified or unmodified variant thereof.

A RNAi agent comprising a first and a second strand, wherein the sequences of the first and/or second strands comprise the sequences of a first and/or second strand of: AD-39858.1 (SEQ ID NOs: 1730 and 1768), or a modified or unmodified variant thereof.

A RNAi agent comprising a first and a second strand, wherein the sequences of the first and/or second strands comprise the sequences of a first and/or second strand of: AD-39870.1 (SEQ ID NOs: 1731 and 1769), or a modified or unmodified variant thereof.

A RNAi agent comprising a first and a second strand, wherein the sequences of the first and/or second strands comprise the sequences of a first and/or second strand of: AD-35576.4 (SEQ ID NOs: 1732 and 1770), or a modified or unmodified variant thereof.

A RNAi agent comprising a first and a second strand, wherein the sequences of the first and/or second strands comprise the sequences of a first and/or second strand of: AD-35588.4 (SEQ ID NOs: 1733 and 1771), or a modified or unmodified variant thereof.

A RNAi agent comprising a first and a second strand, wherein the sequences of the first and/or second strands comprise the sequences of a first and/or second strand of: AD-35600.1 (SEQ ID NOs: 1734 and 1772), or a modified or unmodified variant thereof.

A RNAi agent comprising a first and a second strand, wherein the sequences of the first and/or second strands comprise the sequences of a first and/or second strand of: AD-35606.1 (SEQ ID NOs: 1735 and 1773), or a modified or unmodified variant thereof.

A RNAi agent comprising a first and a second strand, wherein the sequences of the first and/or second strands comprise the sequences of a first and/or second strand of: AD-38151.1 (SEQ ID NOs: 1736 and 1774), or a modified or unmodified variant thereof.

A RNAi agent comprising a first and a second strand, wherein the sequences of the first and/or second strands comprise the sequences of a first and/or second strand of: AD-38163.1 (SEQ ID NOs: 1737 and 1775), or a modified or unmodified variant thereof.

A RNAi agent comprising a first and a second strand, wherein the sequences of the first and/or second strands comprise the sequences of a first and/or second strand of: AD-39999.1 (SEQ ID NOs: 1738 and 1776), or a modified or unmodified variant thereof.

A RNAi agent comprising a first and a second strand, wherein the sequences of the first and/or second strands comprise the sequences of a first and/or second strand of: AD-38159.1 or hs_KRAS_528_A22S26 (SEQ ID NOs: 1739 and 1777), or a modified or unmodified variant thereof.

A RNAi agent comprising a first and a second strand, wherein the sequences of the first and/or second strands comprise the sequences of a first and/or second strand of: AD-38130.1 or hs_KRAS_531_A22S26 (SEQ ID NOs: 1740 and 1778), or a modified or unmodified variant thereof.

A RNAi agent comprising a first and a second strand, wherein the sequences of the first and/or second strands comprise the sequences of a first and/or second strand of: AD-38136.1 (SEQ ID NOs: 1741 and 1779), or a modified or unmodified variant thereof.

A RNAi agent comprising a first and a second strand, wherein the sequences of the first and/or second strands comprise the sequences of a first and/or second strand of: AD-40036.1 (SEQ ID NOs: 1742 and 1780), or a modified or unmodified variant thereof.

A RNAi agent comprising a first and a second strand, wherein the sequences of the first and/or second strands comprise the sequences of a first and/or second strand of: AD-40008.1 (SEQ ID NOs: 1743 and 1781), or a modified or unmodified variant thereof.

A RNAi agent comprising a first and a second strand, wherein the sequences of the first and/or second strands comprise the sequences of a first and/or second strand of: AD-40021.1 (SEQ ID NOs: 1744 and 1782), or a modified or unmodified variant thereof.

A RNAi agent comprising a first and a second strand, wherein the sequences of the first and/or second strands comprise the sequences of a first and/or second strand of: AD-40077.1 (SEQ ID NOs: 1745 and 1783), or a modified or unmodified variant thereof.

A RNAi agent comprising a first and a second strand, wherein the sequences of the first and/or second strands comprise the sequences of a first and/or second strand of: AD-40072.1 (SEQ ID NOs: 1746 and 1784), or a modified or unmodified variant thereof.

A RNAi agent comprising a first and a second strand, wherein the sequences of the first and/or second strands comprise the sequences of a first and/or second strand of: AD-40061.1 (SEQ ID NOs: 1747 and 1785), or a modified or unmodified variant thereof.

A RNAi agent comprising a first and a second strand, wherein the sequences of the first and/or second strands comprise the sequences of a first and/or second strand of: AD-40068.1 (SEQ ID NOs: 1748 and 1786), or a modified or unmodified variant thereof.

A RNAi agent comprising a first and a second strand, wherein the sequences of the first and/or second strands comprise the sequences of a first and/or second strand of: AD-38131.1 (SEQ ID NOs: 1749 and 1787), or a modified or unmodified variant thereof.

A RNAi agent comprising a first and a second strand, wherein the sequences of the first and/or second strands comprise the sequences of a first and/or second strand of: AD-38167.1 (SEQ ID NOs: 1750 and 1788), or a modified or unmodified variant thereof.

A RNAi agent comprising a first and a second strand, wherein the sequences of the first and/or second strands comprise the sequences of a first and/or second strand of: hs_KRAS 1273_A22S26 (SEQ ID NOs: 1751 and 1789), or a modified or unmodified variant thereof.

A RNAi agent comprising a first and a second strand, wherein the sequences of the first and/or second strands comprise the sequences of a first and/or second strand of: hs_KRAS 2892_A37S26 (SEQ ID NOs: 1752 and 1790), or a modified or unmodified variant thereof.

A RNAi agent comprising a first and a second strand, wherein the sequences of the first and/or second strands comprise the sequences of a first and/or second strand of: hs_KRAS 4731_A22S26 (SEQ ID NOs: 1753 and 1791), or a modified or unmodified variant thereof.

**Specific embodiments wherein the sequences of the first and/or second strands are the sequences of the first and/or second strands of a RNAi agent disclosed herein**

In one embodiment, the present disclosure pertains to a composition comprising any one or more of: a RNAi agent comprising a first and a second strand, wherein the sequences of the first and/or second strands are the sequences of the first and/or second strands of any RNAi agent from any of Tables 1 to 6, or modified and unmodified variants thereof.

Thus, in various embodiments, the present disclosure pertains to a composition comprising any one or more of the following:

A RNAi agent comprising a first and a second strand, wherein the sequences of the first and/or second strands are the sequences of the first and/or second strands of: AD-35523.3 (SEQ ID NOs: 1 and 428), or a modified or unmodified variant thereof.

A RNAi agent comprising a first and a second strand, wherein the sequences of the first and/or second strands are the sequences of the first and/or second strands of: AD-35529.1 (SEQ ID NOs: 2 and 429), or a modified or unmodified variant thereof.

A RNAi agent comprising a first and a second strand, wherein the sequences of the first and/or second strands are the sequences of the first and/or second strands of: AD-35535.4 (SEQ ID NOs: 3 and 430), or a modified or unmodified variant thereof.

A RNAi agent comprising a first and a second strand, wherein the sequences of the first and/or second strands are the sequences of the first and/or second strands of: AD-35541.4 (SEQ ID NOs: 4 and 431), or a modified or unmodified variant thereof.

A RNAi agent comprising a first and a second strand, wherein the sequences of the first and/or second strands are the sequences of the first and/or second strands of: AD-35547.4 (SEQ ID NOs: 5 and 432), or a modified or unmodified variant thereof.

A RNAi agent comprising a first and a second strand, wherein the sequences of the first and/or second strands are the sequences of the first and/or second strands of: AD-35553.4 (SEQ ID NOs: 6 and 433), or a modified or unmodified variant thereof.

A RNAi agent comprising a first and a second strand, wherein the sequences of the first and/or second strands are the sequences of the first and/or second strands of: AD-35559.4 (SEQ ID NOs: 7 and 434), or a modified or unmodified variant thereof.

A RNAi agent comprising a first and a second strand, wherein the sequences of the first and/or second strands are the sequences of the first and/or second strands of: AD-35565.4 (SEQ ID NOs: 8 and 435), or a modified or unmodified variant thereof.

A RNAi agent comprising a first and a second strand, wherein the sequences of the first and/or second strands are the sequences of the first and/or second strands of: AD-35524.4 (SEQ ID NOs: 9 and 436), or a modified or unmodified variant thereof.

A RNAi agent comprising a first and a second strand, wherein the sequences of the first and/or second strands are the sequences of the first and/or second strands of: AD-35530.4 (SEQ ID NOs: 10 and 437), or a modified or unmodified variant thereof.

A RNAi agent comprising a first and a second strand, wherein the sequences of the first and/or second strands are the sequences of the first and/or second strands of: AD-35536.4 (SEQ ID NOs: 11 and 438), or a modified or unmodified variant thereof.

A RNAi agent comprising a first and a second strand, wherein the sequences of the first and/or second strands are the sequences of the first and/or second strands of: AD-35542.4 (SEQ ID NOs: 12 and 439), or a modified or unmodified variant thereof.

A RNAi agent comprising a first and a second strand, wherein the sequences of the first and/or second strands are the sequences of the first and/or second strands of: AD-35548.4 (SEQ ID NOs: 13 and 440), or a modified or unmodified variant thereof.

A RNAi agent comprising a first and a second strand, wherein the sequences of the first and/or second strands are the sequences of the first and/or second strands of: AD-35554.4 (SEQ ID NOs: 14 and 441), or a modified or unmodified variant thereof.

A RNAi agent comprising a first and a second strand, wherein the sequences of the first and/or second strands are the sequences of the first and/or second strands of: AD-35560.2 (SEQ ID NOs: 15 and 442), or a modified or unmodified variant thereof.

A RNAi agent comprising a first and a second strand, wherein the sequences of the first and/or second strands are the sequences of the first and/or second strands of: AD-35566.4 (SEQ ID NOs: 16 and 443), or a modified or unmodified variant thereof.

A RNAi agent comprising a first and a second strand, wherein the sequences of the first and/or second strands are the sequences of the first and/or second strands of: AD-35525.2 (SEQ ID NOs: 17 and 444), or a modified or unmodified variant thereof.

A RNAi agent comprising a first and a second strand, wherein the sequences of the first and/or second strands are the sequences of the first and/or second strands of: AD-35531.4 (SEQ ID NOs: 18 and 445), or a modified or unmodified variant thereof.

A RNAi agent comprising a first and a second strand, wherein the sequences of the first and/or second strands are the sequences of the first and/or second strands of: AD-35537.4 (SEQ ID NOs: 19 and 446), or a modified or unmodified variant thereof.

A RNAi agent comprising a first and a second strand, wherein the sequences of the first and/or second strands are the sequences of the first and/or second strands of: AD-35543.2 (SEQ ID NOs: 20 and 447), or a modified or unmodified variant thereof.

A RNAi agent comprising a first and a second strand, wherein the sequences of the first and/or second strands are the sequences of the first and/or second strands of: AD-35549.4 (SEQ ID NOs: 21 and 448), or a modified or unmodified variant thereof.

A RNAi agent comprising a first and a second strand, wherein the sequences of the first and/or second strands are the sequences of the first and/or second strands of: AD-35555.4 (SEQ ID NOs: 22 and 449), or a modified or unmodified variant thereof.

A RNAi agent comprising a first and a second strand, wherein the sequences of the first and/or second strands are the sequences of the first and/or second strands of: AD-35561.4 (SEQ ID NOs: 23 and 450), or a modified or unmodified variant thereof.

A RNAi agent comprising a first and a second strand, wherein the sequences of the first and/or second strands are the sequences of the first and/or second strands of: AD-35567.4 (SEQ ID NOs: 24 and 451), or a modified or unmodified variant thereof.

A RNAi agent comprising a first and a second strand, wherein the sequences of the first and/or second strands are the sequences of the first and/or second strands of: AD-35526.4 (SEQ ID NOs: 25 and 452), or a modified or unmodified variant thereof.

A RNAi agent comprising a first and a second strand, wherein the sequences of the first and/or second strands are the sequences of the first and/or second strands of: AD-35532.4 (SEQ ID NOs: 26 and 453), or a modified or unmodified variant thereof.

A RNAi agent comprising a first and a second strand, wherein the sequences of the first and/or second strands are the sequences of the first and/or second strands of: AD-35538.4 (SEQ ID NOs: 27 and 454), or a modified or unmodified variant thereof.

A RNAi agent comprising a first and a second strand, wherein the sequences of the first and/or second strands are the sequences of the first and/or second strands of: AD-35544.4 (SEQ ID NOs: 28 and 455), or a modified or unmodified variant thereof.

A RNAi agent comprising a first and a second strand, wherein the sequences of the first and/or second strands are the sequences of the first and/or second strands of: AD-35550.4 (SEQ ID NOs: 29 and 456), or a modified or unmodified variant thereof.

A RNAi agent comprising a first and a second strand, wherein the sequences of the first and/or second strands are the sequences of the first and/or second strands of: AD-35556.4 (SEQ ID NOs: 30 and 457), or a modified or unmodified variant thereof.

A RNAi agent comprising a first and a second strand, wherein the sequences of the first and/or second strands are the sequences of the first and/or second strands of: AD-35562.4 (SEQ ID NOs: 31 and 458), or a modified or unmodified variant thereof.

A RNAi agent comprising a first and a second strand, wherein the sequences of the first and/or second strands are the sequences of the first and/or second strands of: AD-35568.4 (SEQ ID NOs: 32 and 459), or a modified or unmodified variant thereof.

A RNAi agent comprising a first and a second strand, wherein the sequences of the first and/or second strands are the sequences of the first and/or second strands of: AD-35527.4 (SEQ ID NOs: 33 and 460), or a modified or unmodified variant thereof.

A RNAi agent comprising a first and a second strand, wherein the sequences of the first and/or second strands are the sequences of the first and/or second strands of: AD-35533.4 (SEQ ID NOs: 34 and 461), or a modified or unmodified variant thereof.

A RNAi agent comprising a first and a second strand, wherein the sequences of the first and/or second strands are the sequences of the first and/or second strands of: AD-35539.4 (SEQ ID NOs: 35 and 462), or a modified or unmodified variant thereof.

A RNAi agent comprising a first and a second strand, wherein the sequences of the first and/or second strands are the sequences of the first and/or second strands of: AD-35545.4 (SEQ ID NOs: 36 and 463), or a modified or unmodified variant thereof.

A RNAi agent comprising a first and a second strand, wherein the sequences of the first and/or second strands are the sequences of the first and/or second strands of: AD-35551.4 (SEQ ID NOs: 37 and 464), or a modified or unmodified variant thereof.

A RNAi agent comprising a first and a second strand, wherein the sequences of the first and/or second strands are the sequences of the first and/or second strands of: AD-35563.4 (SEQ ID NOs: 38 and 465), or a modified or unmodified variant thereof.

A RNAi agent comprising a first and a second strand, wherein the sequences of the first and/or second strands are the sequences of the first and/or second strands of: AD-35528.4 (SEQ ID NOs: 39 and 466), or a modified or unmodified variant thereof.

A RNAi agent comprising a first and a second strand, wherein the sequences of the first and/or second strands are the sequences of the first and/or second strands of: AD-35540.4 (SEQ ID NOs: 40 and 467), or a modified or unmodified variant thereof.

A RNAi agent comprising a first and a second strand, wherein the sequences of the first and/or second strands are the sequences of the first and/or second strands of: AD-35546.4 (SEQ ID NOs: 41 and 468), or a modified or unmodified variant thereof.

A RNAi agent comprising a first and a second strand, wherein the sequences of the first and/or second strands are the sequences of the first and/or second strands of: AD-35552.4 (SEQ ID NOs: 42 and 469), or a modified or unmodified variant thereof.

A RNAi agent comprising a first and a second strand, wherein the sequences of the first and/or second strands are the sequences of the first and/or second strands of: AD-35558.4 (SEQ ID NOs: 43 and 470), or a modified or unmodified variant thereof.

A RNAi agent comprising a first and a second strand, wherein the sequences of the first and/or second strands are the sequences of the first and/or second strands of: AD-35564.4 (SEQ ID NOs: 44 and 471), or a modified or unmodified variant thereof.

A RNAi agent comprising a first and a second strand, wherein the sequences of the first and/or second strands are the sequences of the first and/or second strands of: AD-35570.4 (SEQ ID NOs: 45 and 472), or a modified or unmodified variant thereof.

A RNAi agent comprising a first and a second strand, wherein the sequences of the first and/or second strands are the sequences of the first and/or second strands of: AD-35571.4 (SEQ ID NOs: 46 and 473), or a modified or unmodified variant thereof.

A RNAi agent comprising a first and a second strand, wherein the sequences of the first and/or second strands are the sequences of the first and/or second strands of: AD-35577.4 (SEQ ID NOs: 47 and 474), or a modified or unmodified variant thereof.

A RNAi agent comprising a first and a second strand, wherein the sequences of the first and/or second strands are the sequences of the first and/or second strands of: AD-35583.4 (SEQ ID NOs: 48 and 475), or a modified or unmodified variant thereof.

A RNAi agent comprising a first and a second strand, wherein the sequences of the first and/or second strands are the sequences of the first and/or second strands of: AD-35589.4 (SEQ ID NOs: 49 and 476), or a modified or unmodified variant thereof.

A RNAi agent comprising a first and a second strand, wherein the sequences of the first and/or second strands are the sequences of the first and/or second strands of: AD-35595.4 (SEQ ID NOs: 50 and 477), or a modified or unmodified variant thereof.

A RNAi agent comprising a first and a second strand, wherein the sequences of the first and/or second strands are the sequences of the first and/or second strands of: AD-35601.4 (SEQ ID NOs: 51 and 478), or a modified or unmodified variant thereof.

A RNAi agent comprising a first and a second strand, wherein the sequences of the first and/or second strands are the sequences of the first and/or second strands of: AD-35607.4 (SEQ ID NOs: 52 and 479), or a modified or unmodified variant thereof.

A RNAi agent comprising a first and a second strand, wherein the sequences of the first and/or second strands are the sequences of the first and/or second strands of: AD-35613.4 (SEQ ID NOs: 53 and 480), or a modified or unmodified variant thereof.

A RNAi agent comprising a first and a second strand, wherein the sequences of the first and/or second strands are the sequences of the first and/or second strands of: AD-35572.4 (SEQ ID NOs: 54 and 481), or a modified or unmodified variant thereof.

A RNAi agent comprising a first and a second strand, wherein the sequences of the first and/or second strands are the sequences of the first and/or second strands of: AD-35578.4 (SEQ ID NOs: 55 and 482), or a modified or unmodified variant thereof.

A RNAi agent comprising a first and a second strand, wherein the sequences of the first and/or second strands are the sequences of the first and/or second strands of: AD-35584.4 (SEQ ID NOs: 56 and 483), or a modified or unmodified variant thereof.

A RNAi agent comprising a first and a second strand, wherein the sequences of the first and/or second strands are the sequences of the first and/or second strands of: AD-35590.4 (SEQ ID NOs: 57 and 484), or a modified or unmodified variant thereof.

A RNAi agent comprising a first and a second strand, wherein the sequences of the first and/or second strands are the sequences of the first and/or second strands of: AD-35596.4 (SEQ ID NOs: 58 and 485), or a modified or unmodified variant thereof.

A RNAi agent comprising a first and a second strand, wherein the sequences of the first and/or second strands are the sequences of the first and/or second strands of: AD-35602.4 (SEQ ID NOs: 59 and 486), or a modified or unmodified variant thereof.

A RNAi agent comprising a first and a second strand, wherein the sequences of the first and/or second strands are the sequences of the first and/or second strands of: AD-35608.4 (SEQ ID NOs: 60 and 487), or a modified or unmodified variant thereof.

A RNAi agent comprising a first and a second strand, wherein the sequences of the first and/or second strands are the sequences of the first and/or second strands of: AD-35614.4 (SEQ ID NOs: 61 and 488), or a modified or unmodified variant thereof.

A RNAi agent comprising a first and a second strand, wherein the sequences of the first and/or second strands are the sequences of the first and/or second strands of: AD-35573.4 (SEQ ID NOs: 62 and 489), or a modified or unmodified variant thereof.

A RNAi agent comprising a first and a second strand, wherein the sequences of the first and/or second strands are the sequences of the first and/or second strands of: AD-35579.4 (SEQ ID NOs: 63 and 490), or a modified or unmodified variant thereof.

A RNAi agent comprising a first and a second strand, wherein the sequences of the first and/or second strands are the sequences of the first and/or second strands of: AD-35585.4 (SEQ ID NOs: 64 and 491), or a modified or unmodified variant thereof.

A RNAi agent comprising a first and a second strand, wherein the sequences of the first and/or second strands are the sequences of the first and/or second strands of: AD-35591.4 (SEQ ID NOs: 65 and 492), or a modified or unmodified variant thereof.

A RNAi agent comprising a first and a second strand, wherein the sequences of the first and/or second strands are the sequences of the first and/or second strands of: AD-35597.4 (SEQ ID NOs: 66 and 493), or a modified or unmodified variant thereof.

A RNAi agent comprising a first and a second strand, wherein the sequences of the first and/or second strands are the sequences of the first and/or second strands of: AD-35603.4 (SEQ ID NOs: 67 and 494), or a modified or unmodified variant thereof.

A RNAi agent comprising a first and a second strand, wherein the sequences of the first and/or second strands are the sequences of the first and/or second strands of: AD-35609.4 (SEQ ID NOs: 68 and 495), or a modified or unmodified variant thereof.

A RNAi agent comprising a first and a second strand, wherein the sequences of the first and/or second strands are the sequences of the first and/or second strands of: AD-35615.4 (SEQ ID NOs: 69 and 496), or a modified or unmodified variant thereof.

A RNAi agent comprising a first and a second strand, wherein the sequences of the first and/or second strands are the sequences of the first and/or second strands of: AD-35574.4 (SEQ ID NOs: 70 and 497), or a modified or unmodified variant thereof.

A RNAi agent comprising a first and a second strand, wherein the sequences of the first and/or second strands are the sequences of the first and/or second strands of: AD-35580.1 (SEQ ID NOs: 71 and 498), or a modified or unmodified variant thereof.

A RNAi agent comprising a first and a second strand, wherein the sequences of the first and/or second strands are the sequences of the first and/or second strands of: AD-35586.4 (SEQ ID NOs: 72 and 499), or a modified or unmodified variant thereof.

A RNAi agent comprising a first and a second strand, wherein the sequences of the first and/or second strands are the sequences of the first and/or second strands of: AD-35592.4 (SEQ ID NOs: 73 and 500), or a modified or unmodified variant thereof.

A RNAi agent comprising a first and a second strand, wherein the sequences of the first and/or second strands are the sequences of the first and/or second strands of: AD-35598.4 (SEQ ID NOs: 74 and 501), or a modified or unmodified variant thereof.

A RNAi agent comprising a first and a second strand, wherein the sequences of the first and/or second strands are the sequences of the first and/or second strands of: AD-35604.4 (SEQ ID NOs: 75 and 502), or a modified or unmodified variant thereof.

A RNAi agent comprising a first and a second strand, wherein the sequences of the first and/or second strands are the sequences of the first and/or second strands of: AD-35610.4 (SEQ ID NOs: 76 and 503), or a modified or unmodified variant thereof.

A RNAi agent comprising a first and a second strand, wherein the sequences of the first and/or second strands are the sequences of the first and/or second strands of: AD-35616.4 (SEQ ID NOs: 77 and 504), or a modified or unmodified variant thereof.

A RNAi agent comprising a first and a second strand, wherein the sequences of the first and/or second strands are the sequences of the first and/or second strands of: AD-35575.4 (SEQ ID NOs: 78 and 505), or a modified or unmodified variant thereof.

A RNAi agent comprising a first and a second strand, wherein the sequences of the first and/or second strands are the sequences of the first and/or second strands of: AD-35581.4 (SEQ ID NOs: 79 and 506), or a modified or unmodified variant thereof.

A RNAi agent comprising a first and a second strand, wherein the sequences of the first and/or second strands are the sequences of the first and/or second strands of: AD-35587.4 (SEQ ID NOs: 80 and 507), or a modified or unmodified variant thereof.

A RNAi agent comprising a first and a second strand, wherein the sequences of the first and/or second strands are the sequences of the first and/or second strands of: AD-35593.4 (SEQ ID NOs: 81 and 508), or a modified or unmodified variant thereof.

A RNAi agent comprising a first and a second strand, wherein the sequences of the first and/or second strands are the sequences of the first and/or second strands of: AD-35599.4 (SEQ ID NOs: 82 and 509), or a modified or unmodified variant thereof.

A RNAi agent comprising a first and a second strand, wherein the sequences of the first and/or second strands are the sequences of the first and/or second strands of: AD-35605.4 (SEQ ID NOs: 83 and 510), or a modified or unmodified variant thereof.

A RNAi agent comprising a first and a second strand, wherein the sequences of the first and/or second strands are the sequences of the first and/or second strands of: AD-35611.4 (SEQ ID NOs: 84 and 511), or a modified or unmodified variant thereof.

A RNAi agent comprising a first and a second strand, wherein the sequences of the first and/or second strands are the sequences of the first and/or second strands of: AD-35617.4 (SEQ ID NOs: 85 and 512), or a modified or unmodified variant thereof.

A RNAi agent comprising a first and a second strand, wherein the sequences of the first and/or second strands are the sequences of the first and/or second strands of: AD-35576.4 (SEQ ID NOs: 86 and 513), or a modified or unmodified variant thereof.

A RNAi agent comprising a first and a second strand, wherein the sequences of the first and/or second strands are the sequences of the first and/or second strands of: AD-35588.4 (SEQ ID NOs: 87 and 514), or a modified or unmodified variant thereof.

A RNAi agent comprising a first and a second strand, wherein the sequences of the first and/or second strands are the sequences of the first and/or second strands of: AD-35667.1 (SEQ ID NOs: 88 and 515), or a modified or unmodified variant thereof.

A RNAi agent comprising a first and a second strand, wherein the sequences of the first and/or second strands are the sequences of the first and/or second strands of: AD-35673.1 (SEQ ID NOs: 89 and 516), or a modified or unmodified variant thereof.

A RNAi agent comprising a first and a second strand, wherein the sequences of the first and/or second strands are the sequences of the first and/or second strands of: AD-35679.1 (SEQ ID NOs: 90 and 517), or a modified or unmodified variant thereof.

A RNAi agent comprising a first and a second strand, wherein the sequences of the first and/or second strands are the sequences of the first and/or second strands of: AD-35685.1 (SEQ ID NOs: 91 and 518), or a modified or unmodified variant thereof.

A RNAi agent comprising a first and a second strand, wherein the sequences of the first and/or second strands are the sequences of the first and/or second strands of: AD-35691.1 (SEQ ID NOs: 92 and 519), or a modified or unmodified variant thereof.

A RNAi agent comprising a first and a second strand, wherein the sequences of the first and/or second strands are the sequences of the first and/or second strands of: AD-35557.1 (SEQ ID NOs: 93 and 520), or a modified or unmodified variant thereof.

A RNAi agent comprising a first and a second strand, wherein the sequences of the first and/or second strands are the sequences of the first and/or second strands of: AD-35618.1 (SEQ ID NOs: 94 and 521), or a modified or unmodified variant thereof.

A RNAi agent comprising a first and a second strand, wherein the sequences of the first and/or second strands are the sequences of the first and/or second strands of: AD-35569.1 (SEQ ID NOs: 95 and 522), or a modified or unmodified variant thereof.

A RNAi agent comprising a first and a second strand, wherein the sequences of the first and/or second strands are the sequences of the first and/or second strands of: AD-35534.1 (SEQ ID NOs: 96 and 523), or a modified or unmodified variant thereof.

A RNAi agent comprising a first and a second strand, wherein the sequences of the first and/or second strands are the sequences of the first and/or second strands of: AD-35582.1 (SEQ ID NOs: 97 and 524), or a modified or unmodified variant thereof.

A RNAi agent comprising a first and a second strand, wherein the sequences of the first and/or second strands are the sequences of the first and/or second strands of: AD-35594.1 (SEQ ID NOs: 98 and 525), or a modified or unmodified variant thereof.

A RNAi agent comprising a first and a second strand, wherein the sequences of the first and/or second strands are the sequences of the first and/or second strands of: AD-35600.1 (SEQ ID NOs: 99 and 526), or a modified or unmodified variant thereof.

A RNAi agent comprising a first and a second strand, wherein the sequences of the first and/or second strands are the sequences of the first and/or second strands of: AD-35606.1 (SEQ ID NOs: 100 and 527), or a modified or unmodified variant thereof.

A RNAi agent comprising a first and a second strand, wherein the sequences of the first and/or second strands are the sequences of the first and/or second strands of: AD-35612.1 (SEQ ID NOs: 101 and 528), or a modified or unmodified variant thereof.

A RNAi agent comprising a first and a second strand, wherein the sequences of the first and/or second strands are the sequences of the first and/or second strands of: AD-38127.1 (SEQ ID NOs: 102 and 529), or a modified or unmodified variant thereof.

A RNAi agent comprising a first and a second strand, wherein the sequences of the first and/or second strands are the sequences of the first and/or second strands of: AD-38133.1 (SEQ ID NOs: 103 and 530), or a modified or unmodified variant thereof.

A RNAi agent comprising a first and a second strand, wherein the sequences of the first and/or second strands are the sequences of the first and/or second strands of: AD-38139.1 (SEQ ID NOs: 104 and 531), or a modified or unmodified variant thereof.

A RNAi agent comprising a first and a second strand, wherein the sequences of the first and/or second strands are the sequences of the first and/or second strands of: AD-38145.1 (SEQ ID NOs: 105 and 532), or a modified or unmodified variant thereof.

A RNAi agent comprising a first and a second strand, wherein the sequences of the first and/or second strands are the sequences of the first and/or second strands of: AD-38151.1 (SEQ ID NOs: 106 and 533), or a modified or unmodified variant thereof.

A RNAi agent comprising a first and a second strand, wherein the sequences of the first and/or second strands are the sequences of the first and/or second strands of: AD-38157.1 (SEQ ID NOs: 107 and 534), or a modified or unmodified variant thereof.

A RNAi agent comprising a first and a second strand, wherein the sequences of the first and/or second strands are the sequences of the first and/or second strands of: AD-38163.1 (SEQ ID NOs: 108 and 535), or a modified or unmodified variant thereof.

A RNAi agent comprising a first and a second strand, wherein the sequences of the first and/or second strands are the sequences of the first and/or second strands of: AD-38169.1 (SEQ ID NOs: 109 and 536), or a modified or unmodified variant thereof.

A RNAi agent comprising a first and a second strand, wherein the sequences of the first and/or second strands are the sequences of the first and/or second strands of: AD-38128.1 (SEQ ID NOs: 110 and 537), or a modified or unmodified variant thereof.

A RNAi agent comprising a first and a second strand, wherein the sequences of the first and/or second strands are the sequences of the first and/or second strands of: AD-38134.1 (SEQ ID NOs: 111 and 538), or a modified or unmodified variant thereof.

A RNAi agent comprising a first and a second strand, wherein the sequences of the first and/or second strands are the sequences of the first and/or second strands of: AD-38140.1 (SEQ ID NOs: 112 and 539), or a modified or unmodified variant thereof.

A RNAi agent comprising a first and a second strand, wherein the sequences of the first and/or second strands are the sequences of the first and/or second strands of: AD-38146.1 (SEQ ID NOs: 113 and 540), or a modified or unmodified variant thereof.

A RNAi agent comprising a first and a second strand, wherein the sequences of the first and/or second strands are the sequences of the first and/or second strands of: AD-38152.1 (SEQ ID NOs: 114 and 541), or a modified or unmodified variant thereof.

A RNAi agent comprising a first and a second strand, wherein the sequences of the first and/or second strands are the sequences of the first and/or second strands of: AD-38158.1 (SEQ ID NOs: 115 and 542), or a modified or unmodified variant thereof.

A RNAi agent comprising a first and a second strand, wherein the sequences of the first and/or second strands are the sequences of the first and/or second strands of: AD-38164.1 (SEQ ID NOs: 116 and 543), or a modified or unmodified variant thereof.

A RNAi agent comprising a first and a second strand, wherein the sequences of the first and/or second strands are the sequences of the first and/or second strands of: AD-38170.1 (SEQ ID NOs: 117 and 544), or a modified or unmodified variant thereof.

A RNAi agent comprising a first and a second strand, wherein the sequences of the first and/or second strands are the sequences of the first and/or second strands of: AD-38129.1 (SEQ ID NOs: 118 and 545), or a modified or unmodified variant thereof.

A RNAi agent comprising a first and a second strand, wherein the sequences of the first and/or second strands are the sequences of the first and/or second strands of: AD-38135.1 (SEQ ID NOs: 119 and 546), or a modified or unmodified variant thereof.

A RNAi agent comprising a first and a second strand, wherein the sequences of the first and/or second strands are the sequences of the first and/or second strands of: AD-38141.1 (SEQ ID NOs: 120 and 547), or a modified or unmodified variant thereof.

A RNAi agent comprising a first and a second strand, wherein the sequences of the first and/or second strands are the sequences of the first and/or second strands of: AD-38147.1 (SEQ ID NOs: 121 and 548), or a modified or unmodified variant thereof.

A RNAi agent comprising a first and a second strand, wherein the sequences of the first and/or second strands are the sequences of the first and/or second strands of: AD-38153.1 (SEQ ID NOs: 122 and 549), or a modified or unmodified variant thereof.

A RNAi agent comprising a first and a second strand, wherein the sequences of the first and/or second strands are the sequences of the first and/or second strands of: AD-38159.1 (SEQ ID NOs: 123 and 550), or a modified or unmodified variant thereof.

A RNAi agent comprising a first and a second strand, wherein the sequences of the first and/or second strands are the sequences of the first and/or second strands of: AD-38165.1 (SEQ ID NOs: 124 and 551), or a modified or unmodified variant thereof.

A RNAi agent comprising a first and a second strand, wherein the sequences of the first and/or second strands are the sequences of the first and/or second strands of: AD-38171.1 (SEQ ID NOs: 125 and 552), or a modified or unmodified variant thereof.

A RNAi agent comprising a first and a second strand, wherein the sequences of the first and/or second strands are the sequences of the first and/or second strands of: AD-38130.1 (SEQ ID NOs: 126 and 553), or a modified or unmodified variant thereof.

A RNAi agent comprising a first and a second strand, wherein the sequences of the first and/or second strands are the sequences of the first and/or second strands of: AD-38136.1 (SEQ ID NOs: 127 and 554), or a modified or unmodified variant thereof.

A RNAi agent comprising a first and a second strand, wherein the sequences of the first and/or second strands are the sequences of the first and/or second strands of: AD-38142.1 (SEQ ID NOs: 128 and 555), or a modified or unmodified variant thereof.

A RNAi agent comprising a first and a second strand, wherein the sequences of the first and/or second strands are the sequences of the first and/or second strands of: AD-38148.1 (SEQ ID NOs: 129 and 556), or a modified or unmodified variant thereof.

A RNAi agent comprising a first and a second strand, wherein the sequences of the first and/or second strands are the sequences of the first and/or second strands of: AD-38154.1 (SEQ ID NOs: 130 and 557), or a modified or unmodified variant thereof.

A RNAi agent comprising a first and a second strand, wherein the sequences of the first and/or second strands are the sequences of the first and/or second strands of: AD-38160.1 (SEQ ID NOs: 131 and 558), or a modified or unmodified variant thereof.

A RNAi agent comprising a first and a second strand, wherein the sequences of the first and/or second strands are the sequences of the first and/or second strands of: AD-38166.1 (SEQ ID NOs: 132 and 559), or a modified or unmodified variant thereof.

A RNAi agent comprising a first and a second strand, wherein the sequences of the first and/or second strands are the sequences of the first and/or second strands of: AD-38172.1 (SEQ ID NOs: 133 and 560), or a modified or unmodified variant thereof.

A RNAi agent comprising a first and a second strand, wherein the sequences of the first and/or second strands are the sequences of the first and/or second strands of: AD-38131.1 (SEQ ID NOs: 134 and 561), or a modified or unmodified variant thereof.

A RNAi agent comprising a first and a second strand, wherein the sequences of the first and/or second strands are the sequences of the first and/or second strands of: AD-38137.1 (SEQ ID NOs: 135 and 562), or a modified or unmodified variant thereof.

A RNAi agent comprising a first and a second strand, wherein the sequences of the first and/or second strands are the sequences of the first and/or second strands of: AD-38143.1 (SEQ ID NOs: 136 and 563), or a modified or unmodified variant thereof.

A RNAi agent comprising a first and a second strand, wherein the sequences of the first and/or second strands are the sequences of the first and/or second strands of: AD-38149.1 (SEQ ID NOs: 137 and 564), or a modified or unmodified variant thereof.

A RNAi agent comprising a first and a second strand, wherein the sequences of the first and/or second strands are the sequences of the first and/or second strands of: AD-38155.1 (SEQ ID NOs: 138 and 565), or a modified or unmodified variant thereof.

A RNAi agent comprising a first and a second strand, wherein the sequences of the first and/or second strands are the sequences of the first and/or second strands of: AD-38161.1 (SEQ ID NOs: 139 and 566), or a modified or unmodified variant thereof.

A RNAi agent comprising a first and a second strand, wherein the sequences of the first and/or second strands are the sequences of the first and/or second strands of: AD-38167.1 (SEQ ID NOs: 140 and 567), or a modified or unmodified variant thereof.

A RNAi agent comprising a first and a second strand, wherein the sequences of the first and/or second strands are the sequences of the first and/or second strands of: AD-38173.1 (SEQ ID NOs: 141 and 568), or a modified or unmodified variant thereof.

A RNAi agent comprising a first and a second strand, wherein the sequences of the first and/or second strands are the sequences of the first and/or second strands of: AD-38132.1 (SEQ ID NOs: 142 and 569), or a modified or unmodified variant thereof.

A RNAi agent comprising a first and a second strand, wherein the sequences of the first and/or second strands are the sequences of the first and/or second strands of: AD-38138.1 (SEQ ID NOs: 143 and 570), or a modified or unmodified variant thereof.

A RNAi agent comprising a first and a second strand, wherein the sequences of the first and/or second strands are the sequences of the first and/or second strands of: AD-38144.1 (SEQ ID NOs: 144 and 571), or a modified or unmodified variant thereof.

A RNAi agent comprising a first and a second strand, wherein the sequences of the first and/or second strands are the sequences of the first and/or second strands of: AD-38150.1 (SEQ ID NOs: 145 and 572), or a modified or unmodified variant thereof.

A RNAi agent comprising a first and a second strand, wherein the sequences of the first and/or second strands are the sequences of the first and/or second strands of: AD-38156.1 (SEQ ID NOs: 146 and 573), or a modified or unmodified variant thereof.

A RNAi agent comprising a first and a second strand, wherein the sequences of the first and/or second strands are the sequences of the first and/or second strands of: AD-38162.1 (SEQ ID NOs: 147 and 574), or a modified or unmodified variant thereof.

A RNAi agent comprising a first and a second strand, wherein the sequences of the first and/or second strands are the sequences of the first and/or second strands of: AD-38168.1 (SEQ ID NOs: 148 and 575), or a modified or unmodified variant thereof.

A RNAi agent comprising a first and a second strand, wherein the sequences of the first and/or second strands are the sequences of the first and/or second strands of: AD-38174.1 (SEQ ID NOs: 149 and 576), or a modified or unmodified variant thereof.

A RNAi agent comprising a first and a second strand, wherein the sequences of the first and/or second strands are the sequences of the first and/or second strands of: AD-39683.1 (SEQ ID NOs: 150 and 577), or a modified or unmodified variant thereof.

A RNAi agent comprising a first and a second strand, wherein the sequences of the first and/or second strands are the sequences of the first and/or second strands of: AD-39689.1 (SEQ ID NOs: 151 and 578), or a modified or unmodified variant thereof.

A RNAi agent comprising a first and a second strand, wherein the sequences of the first and/or second strands are the sequences of the first and/or second strands of: AD-39695.1 (SEQ ID NOs: 152 and 579), or a modified or unmodified variant thereof.

A RNAi agent comprising a first and a second strand, wherein the sequences of the first and/or second strands are the sequences of the first and/or second strands of: AD-39701.1 (SEQ ID NOs: 153 and 580), or a modified or unmodified variant thereof.

A RNAi agent comprising a first and a second strand, wherein the sequences of the first and/or second strands are the sequences of the first and/or second strands of: AD-39707.1 (SEQ ID NOs: 154 and 581), or a modified or unmodified variant thereof.

A RNAi agent comprising a first and a second strand, wherein the sequences of the first and/or second strands are the sequences of the first and/or second strands of: AD-39713.1 (SEQ ID NOs: 155 and 582), or a modified or unmodified variant thereof.

A RNAi agent comprising a first and a second strand, wherein the sequences of the first and/or second strands are the sequences of the first and/or second strands of: AD-39719.1 (SEQ ID NOs: 156 and 583), or a modified or unmodified variant thereof.

A RNAi agent comprising a first and a second strand, wherein the sequences of the first and/or second strands are the sequences of the first and/or second strands of: AD-39725.1 (SEQ ID NOs: 157 and 584), or a modified or unmodified variant thereof.

A RNAi agent comprising a first and a second strand, wherein the sequences of the first and/or second strands are the sequences of the first and/or second strands of: AD-39684.1 (SEQ ID NOs: 158 and 585), or a modified or unmodified variant thereof.

A RNAi agent comprising a first and a second strand, wherein the sequences of the first and/or second strands are the sequences of the first and/or second strands of: AD-39690.1 (SEQ ID NOs: 159 and 586), or a modified or unmodified variant thereof.

A RNAi agent comprising a first and a second strand, wherein the sequences of the first and/or second strands are the sequences of the first and/or second strands of: AD-39696.1 (SEQ ID NOs: 160 and 587), or a modified or unmodified variant thereof.

A RNAi agent comprising a first and a second strand, wherein the sequences of the first and/or second strands are the sequences of the first and/or second strands of: AD-39702.1 (SEQ ID NOs: 161 and 588), or a modified or unmodified variant thereof.

A RNAi agent comprising a first and a second strand, wherein the sequences of the first and/or second strands are the sequences of the first and/or second strands of: AD-39708.1 (SEQ ID NOs: 162 and 589), or a modified or unmodified variant thereof.

A RNAi agent comprising a first and a second strand, wherein the sequences of the first and/or second strands are the sequences of the first and/or second strands of: AD-39714.1 (SEQ ID NOs: 163 and 590), or a modified or unmodified variant thereof.

A RNAi agent comprising a first and a second strand, wherein the sequences of the first and/or second strands are the sequences of the first and/or second strands of: AD-39720.1 (SEQ ID NOs: 164 and 591), or a modified or unmodified variant thereof.

A RNAi agent comprising a first and a second strand, wherein the sequences of the first and/or second strands are the sequences of the first and/or second strands of: AD-39726.1 (SEQ ID NOs: 165 and 592), or a modified or unmodified variant thereof.

A RNAi agent comprising a first and a second strand, wherein the sequences of the first and/or second strands are the sequences of the first and/or second strands of: AD-39685.1 (SEQ ID NOs: 166 and 593), or a modified or unmodified variant thereof.

A RNAi agent comprising a first and a second strand, wherein the sequences of the first and/or second strands are the sequences of the first and/or second strands of: AD-39691.1 (SEQ ID NOs: 167 and 594), or a modified or unmodified variant thereof.

A RNAi agent comprising a first and a second strand, wherein the sequences of the first and/or second strands are the sequences of the first and/or second strands of: AD-39697.1 (SEQ ID NOs: 168 and 595), or a modified or unmodified variant thereof.

A RNAi agent comprising a first and a second strand, wherein the sequences of the first and/or second strands are the sequences of the first and/or second strands of: AD-39703.1 (SEQ ID NOs: 169 and 596), or a modified or unmodified variant thereof.

A RNAi agent comprising a first and a second strand, wherein the sequences of the first and/or second strands are the sequences of the first and/or second strands of: AD-39709.1 (SEQ ID NOs: 170 and 597), or a modified or unmodified variant thereof.

A RNAi agent comprising a first and a second strand, wherein the sequences of the first and/or second strands are the sequences of the first and/or second strands of: AD-39715.1 (SEQ ID NOs: 171 and 598), or a modified or unmodified variant thereof.

A RNAi agent comprising a first and a second strand, wherein the sequences of the first and/or second strands are the sequences of the first and/or second strands of: AD-39721.1 (SEQ ID NOs: 172 and 599), or a modified or unmodified variant thereof.

A RNAi agent comprising a first and a second strand, wherein the sequences of the first and/or second strands are the sequences of the first and/or second strands of: AD-39727.1 (SEQ ID NOs: 173 and 600), or a modified or unmodified variant thereof.

A RNAi agent comprising a first and a second strand, wherein the sequences of the first and/or second strands are the sequences of the first and/or second strands of: AD-39686.1 (SEQ ID NOs: 174 and 601), or a modified or unmodified variant thereof.

A RNAi agent comprising a first and a second strand, wherein the sequences of the first and/or second strands are the sequences of the first and/or second strands of: AD-39692.1 (SEQ ID NOs: 175 and 602), or a modified or unmodified variant thereof.

A RNAi agent comprising a first and a second strand, wherein the sequences of the first and/or second strands are the sequences of the first and/or second strands of: AD-39698.1 (SEQ ID NOs: 176 and 603), or a modified or unmodified variant thereof.

A RNAi agent comprising a first and a second strand, wherein the sequences of the first and/or second strands are the sequences of the first and/or second strands of: AD-39704.1 (SEQ ID NOs: 177 and 604), or a modified or unmodified variant thereof.

A RNAi agent comprising a first and a second strand, wherein the sequences of the first and/or second strands are the sequences of the first and/or second strands of: AD-39710.1 (SEQ ID NOs: 178 and 605), or a modified or unmodified variant thereof.

A RNAi agent comprising a first and a second strand, wherein the sequences of the first and/or second strands are the sequences of the first and/or second strands of: AD-39716.1 (SEQ ID NOs: 179 and 606), or a modified or unmodified variant thereof.

A RNAi agent comprising a first and a second strand, wherein the sequences of the first and/or second strands are the sequences of the first and/or second strands of: AD-39722.1 (SEQ ID NOs: 180 and 607), or a modified or unmodified variant thereof.

A RNAi agent comprising a first and a second strand, wherein the sequences of the first and/or second strands are the sequences of the first and/or second strands of: AD-39728.1 (SEQ ID NOs: 181 and 608), or a modified or unmodified variant thereof.

A RNAi agent comprising a first and a second strand, wherein the sequences of the first and/or second strands are the sequences of the first and/or second strands of: AD-39687.1 (SEQ ID NOs: 182 and 609), or a modified or unmodified variant thereof.

A RNAi agent comprising a first and a second strand, wherein the sequences of the first and/or second strands are the sequences of the first and/or second strands of: AD-39693.1 (SEQ ID NOs: 183 and 610), or a modified or unmodified variant thereof.

A RNAi agent comprising a first and a second strand, wherein the sequences of the first and/or second strands are the sequences of the first and/or second strands of: AD-39699.1 (SEQ ID NOs: 184 and 611), or a modified or unmodified variant thereof.

A RNAi agent comprising a first and a second strand, wherein the sequences of the first and/or second strands are the sequences of the first and/or second strands of: AD-39705.1 (SEQ ID NOs: 185 and 612), or a modified or unmodified variant thereof.

A RNAi agent comprising a first and a second strand, wherein the sequences of the first and/or second strands are the sequences of the first and/or second strands of: AD-39711.1 (SEQ ID NOs: 186 and 613), or a modified or unmodified variant thereof.

A RNAi agent comprising a first and a second strand, wherein the sequences of the first and/or second strands are the sequences of the first and/or second strands of: AD-39717.1 (SEQ ID NOs: 187 and 614), or a modified or unmodified variant thereof.

A RNAi agent comprising a first and a second strand, wherein the sequences of the first and/or second strands are the sequences of the first and/or second strands of: AD-39723.1 (SEQ ID NOs: 188 and 615), or a modified or unmodified variant thereof.

A RNAi agent comprising a first and a second strand, wherein the sequences of the first and/or second strands are the sequences of the first and/or second strands of: AD-39729.1 (SEQ ID NOs: 189 and 616), or a modified or unmodified variant thereof.

A RNAi agent comprising a first and a second strand, wherein the sequences of the first and/or second strands are the sequences of the first and/or second strands of: AD-39688.1 (SEQ ID NOs: 190 and 617), or a modified or unmodified variant thereof.

A RNAi agent comprising a first and a second strand, wherein the sequences of the first and/or second strands are the sequences of the first and/or second strands of: AD-39694.1 (SEQ ID NOs: 191 and 618), or a modified or unmodified variant thereof.

A RNAi agent comprising a first and a second strand, wherein the sequences of the first and/or second strands are the sequences of the first and/or second strands of: AD-39700.1 (SEQ ID NOs: 192 and 619), or a modified or unmodified variant thereof.

A RNAi agent comprising a first and a second strand, wherein the sequences of the first and/or second strands are the sequences of the first and/or second strands of: AD-39706.1 (SEQ ID NOs: 193 and 620), or a modified or unmodified variant thereof.

A RNAi agent comprising a first and a second strand, wherein the sequences of the first and/or second strands are the sequences of the first and/or second strands of: AD-39712.1 (SEQ ID NOs: 194 and 621), or a modified or unmodified variant thereof.

A RNAi agent comprising a first and a second strand, wherein the sequences of the first and/or second strands are the sequences of the first and/or second strands of: AD-39718.1 (SEQ ID NOs: 195 and 622), or a modified or unmodified variant thereof.

A RNAi agent comprising a first and a second strand, wherein the sequences of the first and/or second strands are the sequences of the first and/or second strands of: AD-39724.1 (SEQ ID NOs: 196 and 623), or a modified or unmodified variant thereof.

A RNAi agent comprising a first and a second strand, wherein the sequences of the first and/or second strands are the sequences of the first and/or second strands of: AD-39730.1 (SEQ ID NOs: 197 and 624), or a modified or unmodified variant thereof.

A RNAi agent comprising a first and a second strand, wherein the sequences of the first and/or second strands are the sequences of the first and/or second strands of: AD-39736.1 (SEQ ID NOs: 198 and 625), or a modified or unmodified variant thereof.

A RNAi agent comprising a first and a second strand, wherein the sequences of the first and/or second strands are the sequences of the first and/or second strands of: AD-39742.1 (SEQ ID NOs: 199 and 626), or a modified or unmodified variant thereof.

A RNAi agent comprising a first and a second strand, wherein the sequences of the first and/or second strands are the sequences of the first and/or second strands of: AD-39748.1 (SEQ ID NOs: 200 and 627), or a modified or unmodified variant thereof.

A RNAi agent comprising a first and a second strand, wherein the sequences of the first and/or second strands are the sequences of the first and/or second strands of: AD-39754.1 (SEQ ID NOs: 201 and 628), or a modified or unmodified variant thereof.

A RNAi agent comprising a first and a second strand, wherein the sequences of the first and/or second strands are the sequences of the first and/or second strands of: AD-39760.1 (SEQ ID NOs: 202 and 629), or a modified or unmodified variant thereof.

A RNAi agent comprising a first and a second strand, wherein the sequences of the first and/or second strands are the sequences of the first and/or second strands of: AD-39766.1 (SEQ ID NOs: 203 and 630), or a modified or unmodified variant thereof.

A RNAi agent comprising a first and a second strand, wherein the sequences of the first and/or second strands are the sequences of the first and/or second strands of: AD-39772.1 (SEQ ID NOs: 204 and 631), or a modified or unmodified variant thereof.

A RNAi agent comprising a first and a second strand, wherein the sequences of the first and/or second strands are the sequences of the first and/or second strands of: AD-39731.1 (SEQ ID NOs: 205 and 632), or a modified or unmodified variant thereof.

A RNAi agent comprising a first and a second strand, wherein the sequences of the first and/or second strands are the sequences of the first and/or second strands of: AD-39737.1 (SEQ ID NOs: 206 and 633), or a modified or unmodified variant thereof.

A RNAi agent comprising a first and a second strand, wherein the sequences of the first and/or second strands are the sequences of the first and/or second strands of: AD-39743.1 (SEQ ID NOs: 207 and 634), or a modified or unmodified variant thereof.

A RNAi agent comprising a first and a second strand, wherein the sequences of the first and/or second strands are the sequences of the first and/or second strands of: AD-39749.1 (SEQ ID NOs: 208 and 635), or a modified or unmodified variant thereof.

A RNAi agent comprising a first and a second strand, wherein the sequences of the first and/or second strands are the sequences of the first and/or second strands of: AD-39755.1 (SEQ ID NOs: 209 and 636), or a modified or unmodified variant thereof.

A RNAi agent comprising a first and a second strand, wherein the sequences of the first and/or second strands are the sequences of the first and/or second strands of: AD-39761.1 (SEQ ID NOs: 210 and 637), or a modified or unmodified variant thereof.

A RNAi agent comprising a first and a second strand, wherein the sequences of the first and/or second strands are the sequences of the first and/or second strands of: AD-39767.1 (SEQ ID NOs: 211 and 638), or a modified or unmodified variant thereof.

A RNAi agent comprising a first and a second strand, wherein the sequences of the first and/or second strands are the sequences of the first and/or second strands of: AD-39773.1 (SEQ ID NOs: 212 and 639), or a modified or unmodified variant thereof.

A RNAi agent comprising a first and a second strand, wherein the sequences of the first and/or second strands are the sequences of the first and/or second strands of: AD-39732.1 (SEQ ID NOs: 213 and 640), or a modified or unmodified variant thereof.

A RNAi agent comprising a first and a second strand, wherein the sequences of the first and/or second strands are the sequences of the first and/or second strands of: AD-39738.1 (SEQ ID NOs: 214 and 641), or a modified or unmodified variant thereof.

A RNAi agent comprising a first and a second strand, wherein the sequences of the first and/or second strands are the sequences of the first and/or second strands of: AD-39744.1 (SEQ ID NOs: 215 and 642), or a modified or unmodified variant thereof.

A RNAi agent comprising a first and a second strand, wherein the sequences of the first and/or second strands are the sequences of the first and/or second strands of: AD-39750.1 (SEQ ID NOs: 216 and 643), or a modified or unmodified variant thereof.

A RNAi agent comprising a first and a second strand, wherein the sequences of the first and/or second strands are the sequences of the first and/or second strands of: AD-39756.1 (SEQ ID NOs: 217 and 644), or a modified or unmodified variant thereof.

A RNAi agent comprising a first and a second strand, wherein the sequences of the first and/or second strands are the sequences of the first and/or second strands of: AD-39762.1 (SEQ ID NOs: 218 and 645), or a modified or unmodified variant thereof.

A RNAi agent comprising a first and a second strand, wherein the sequences of the first and/or second strands are the sequences of the first and/or second strands of: AD-39768.1 (SEQ ID NOs: 219 and 646), or a modified or unmodified variant thereof.

A RNAi agent comprising a first and a second strand, wherein the sequences of the first and/or second strands are the sequences of the first and/or second strands of: AD-39774.1 (SEQ ID NOs: 220 and 647), or a modified or unmodified variant thereof.

A RNAi agent comprising a first and a second strand, wherein the sequences of the first and/or second strands are the sequences of the first and/or second strands of: AD-39733.1 (SEQ ID NOs: 221 and 648), or a modified or unmodified variant thereof.

A RNAi agent comprising a first and a second strand, wherein the sequences of the first and/or second strands are the sequences of the first and/or second strands of: AD-39739.1 (SEQ ID NOs: 222 and 649), or a modified or unmodified variant thereof.

A RNAi agent comprising a first and a second strand, wherein the sequences of the first and/or second strands are the sequences of the first and/or second strands of: AD-39745.1 (SEQ ID NOs: 223 and 650), or a modified or unmodified variant thereof.

A RNAi agent comprising a first and a second strand, wherein the sequences of the first and/or second strands are the sequences of the first and/or second strands of: AD-39751.1 (SEQ ID NOs: 224 and 651), or a modified or unmodified variant thereof.

A RNAi agent comprising a first and a second strand, wherein the sequences of the first and/or second strands are the sequences of the first and/or second strands of: AD-39757.1 (SEQ ID NOs: 225 and 652), or a modified or unmodified variant thereof.

A RNAi agent comprising a first and a second strand, wherein the sequences of the first and/or second strands are the sequences of the first and/or second strands of: AD-39763.1 (SEQ ID NOs: 226 and 653), or a modified or unmodified variant thereof.

A RNAi agent comprising a first and a second strand, wherein the sequences of the first and/or second strands are the sequences of the first and/or second strands of: AD-39769.1 (SEQ ID NOs: 227 and 654), or a modified or unmodified variant thereof.

A RNAi agent comprising a first and a second strand, wherein the sequences of the first and/or second strands are the sequences of the first and/or second strands of: AD-39775.1 (SEQ ID NOs: 228 and 655), or a modified or unmodified variant thereof.

A RNAi agent comprising a first and a second strand, wherein the sequences of the first and/or second strands are the sequences of the first and/or second strands of: AD-39734.1 (SEQ ID NOs: 229 and 656), or a modified or unmodified variant thereof.

A RNAi agent comprising a first and a second strand, wherein the sequences of the first and/or second strands are the sequences of the first and/or second strands of: AD-39740.1 (SEQ ID NOs: 230 and 657), or a modified or unmodified variant thereof.

A RNAi agent comprising a first and a second strand, wherein the sequences of the first and/or second strands are the sequences of the first and/or second strands of: AD-39746.1 (SEQ ID NOs: 231 and 658), or a modified or unmodified variant thereof.

A RNAi agent comprising a first and a second strand, wherein the sequences of the first and/or second strands are the sequences of the first and/or second strands of: AD-39752.1 (SEQ ID NOs: 232 and 659), or a modified or unmodified variant thereof.

A RNAi agent comprising a first and a second strand, wherein the sequences of the first and/or second strands are the sequences of the first and/or second strands of: AD-39758.1 (SEQ ID NOs: 233 and 660), or a modified or unmodified variant thereof.

A RNAi agent comprising a first and a second strand, wherein the sequences of the first and/or second strands are the sequences of the first and/or second strands of: AD-39764.1 (SEQ ID NOs: 234 and 661), or a modified or unmodified variant thereof.

A RNAi agent comprising a first and a second strand, wherein the sequences of the first and/or second strands are the sequences of the first and/or second strands of: AD-39770.1 (SEQ ID NOs: 235 and 662), or a modified or unmodified variant thereof.

A RNAi agent comprising a first and a second strand, wherein the sequences of the first and/or second strands are the sequences of the first and/or second strands of: AD-39776.1 (SEQ ID NOs: 236 and 663), or a modified or unmodified variant thereof.

A RNAi agent comprising a first and a second strand, wherein the sequences of the first and/or second strands are the sequences of the first and/or second strands of: AD-39735.1 (SEQ ID NOs: 237 and 664), or a modified or unmodified variant thereof.

A RNAi agent comprising a first and a second strand, wherein the sequences of the first and/or second strands are the sequences of the first and/or second strands of: AD-39741.1 (SEQ ID NOs: 238 and 665), or a modified or unmodified variant thereof.

A RNAi agent comprising a first and a second strand, wherein the sequences of the first and/or second strands are the sequences of the first and/or second strands of: AD-39747.1 (SEQ ID NOs: 239 and 666), or a modified or unmodified variant thereof.

A RNAi agent comprising a first and a second strand, wherein the sequences of the first and/or second strands are the sequences of the first and/or second strands of: AD-39753.1 (SEQ ID NOs: 240 and 667), or a modified or unmodified variant thereof.

A RNAi agent comprising a first and a second strand, wherein the sequences of the first and/or second strands are the sequences of the first and/or second strands of: AD-39759.1 (SEQ ID NOs: 241 and 668), or a modified or unmodified variant thereof.

A RNAi agent comprising a first and a second strand, wherein the sequences of the first and/or second strands are the sequences of the first and/or second strands of: AD-39765.1 (SEQ ID NOs: 242 and 669), or a modified or unmodified variant thereof.

A RNAi agent comprising a first and a second strand, wherein the sequences of the first and/or second strands are the sequences of the first and/or second strands of: AD-39771.1 (SEQ ID NOs: 243 and 670), or a modified or unmodified variant thereof.

A RNAi agent comprising a first and a second strand, wherein the sequences of the first and/or second strands are the sequences of the first and/or second strands of: AD-39778.1 (SEQ ID NOs: 244 and 671), or a modified or unmodified variant thereof.

A RNAi agent comprising a first and a second strand, wherein the sequences of the first and/or second strands are the sequences of the first and/or second strands of: AD-39784.1 (SEQ ID NOs: 245 and 672), or a modified or unmodified variant thereof.

A RNAi agent comprising a first and a second strand, wherein the sequences of the first and/or second strands are the sequences of the first and/or second strands of: AD-39790.1 (SEQ ID NOs: 246 and 673), or a modified or unmodified variant thereof.

A RNAi agent comprising a first and a second strand, wherein the sequences of the first and/or second strands are the sequences of the first and/or second strands of: AD-39796.1 (SEQ ID NOs: 247 and 674), or a modified or unmodified variant thereof.

A RNAi agent comprising a first and a second strand, wherein the sequences of the first and/or second strands are the sequences of the first and/or second strands of: AD-39802.1 (SEQ ID NOs: 248 and 675), or a modified or unmodified variant thereof.

A RNAi agent comprising a first and a second strand, wherein the sequences of the first and/or second strands are the sequences of the first and/or second strands of: AD-39808.1 (SEQ ID NOs: 249 and 676), or a modified or unmodified variant thereof.

A RNAi agent comprising a first and a second strand, wherein the sequences of the first and/or second strands are the sequences of the first and/or second strands of: AD-39814.1 (SEQ ID NOs: 250 and 677), or a modified or unmodified variant thereof.

A RNAi agent comprising a first and a second strand, wherein the sequences of the first and/or second strands are the sequences of the first and/or second strands of: AD-39820.1 (SEQ ID NOs: 251 and 678), or a modified or unmodified variant thereof.

A RNAi agent comprising a first and a second strand, wherein the sequences of the first and/or second strands are the sequences of the first and/or second strands of: AD-39779.1 (SEQ ID NOs: 252 and 679), or a modified or unmodified variant thereof.

A RNAi agent comprising a first and a second strand, wherein the sequences of the first and/or second strands are the sequences of the first and/or second strands of: AD-39785.1 (SEQ ID NOs: 253 and 680), or a modified or unmodified variant thereof.

A RNAi agent comprising a first and a second strand, wherein the sequences of the first and/or second strands are the sequences of the first and/or second strands of: AD-39791.1 (SEQ ID NOs: 254 and 681), or a modified or unmodified variant thereof.

A RNAi agent comprising a first and a second strand, wherein the sequences of the first and/or second strands are the sequences of the first and/or second strands of: AD-39797.1 (SEQ ID NOs: 255 and 682), or a modified or unmodified variant thereof.

A RNAi agent comprising a first and a second strand, wherein the sequences of the first and/or second strands are the sequences of the first and/or second strands of: AD-39803.1 (SEQ ID NOs: 256 and 683), or a modified or unmodified variant thereof.

A RNAi agent comprising a first and a second strand, wherein the sequences of the first and/or second strands are the sequences of the first and/or second strands of: AD-39809.1 (SEQ ID NOs: 257 and 684), or a modified or unmodified variant thereof.

A RNAi agent comprising a first and a second strand, wherein the sequences of the first and/or second strands are the sequences of the first and/or second strands of: AD-39815.1 (SEQ ID NOs: 258 and 685), or a modified or unmodified variant thereof.

A RNAi agent comprising a first and a second strand, wherein the sequences of the first and/or second strands are the sequences of the first and/or second strands of: AD-39821.1 (SEQ ID NOs: 259 and 686), or a modified or unmodified variant thereof.

A RNAi agent comprising a first and a second strand, wherein the sequences of the first and/or second strands are the sequences of the first and/or second strands of: AD-39780.1 (SEQ ID NOs: 260 and 687), or a modified or unmodified variant thereof.

A RNAi agent comprising a first and a second strand, wherein the sequences of the first and/or second strands are the sequences of the first and/or second strands of: AD-39786.1 (SEQ ID NOs: 261 and 688), or a modified or unmodified variant thereof.

A RNAi agent comprising a first and a second strand, wherein the sequences of the first and/or second strands are the sequences of the first and/or second strands of: AD-39792.1 (SEQ ID NOs: 262 and 689), or a modified or unmodified variant thereof.

A RNAi agent comprising a first and a second strand, wherein the sequences of the first and/or second strands are the sequences of the first and/or second strands of: AD-39798.1 (SEQ ID NOs: 263 and 690), or a modified or unmodified variant thereof.

A RNAi agent comprising a first and a second strand, wherein the sequences of the first and/or second strands are the sequences of the first and/or second strands of: AD-39804.1 (SEQ ID NOs: 264 and 691), or a modified or unmodified variant thereof.

A RNAi agent comprising a first and a second strand, wherein the sequences of the first and/or second strands are the sequences of the first and/or second strands of: AD-39810.1 (SEQ ID NOs: 265 and 692), or a modified or unmodified variant thereof.

A RNAi agent comprising a first and a second strand, wherein the sequences of the first and/or second strands are the sequences of the first and/or second strands of: AD-39816.1 (SEQ ID NOs: 266 and 693), or a modified or unmodified variant thereof.

A RNAi agent comprising a first and a second strand, wherein the sequences of the first and/or second strands are the sequences of the first and/or second strands of: AD-39822.1 (SEQ ID NOs: 267 and 694), or a modified or unmodified variant thereof.

A RNAi agent comprising a first and a second strand, wherein the sequences of the first and/or second strands are the sequences of the first and/or second strands of: AD-39781.1 (SEQ ID NOs: 268 and 695), or a modified or unmodified variant thereof.

A RNAi agent comprising a first and a second strand, wherein the sequences of the first and/or second strands are the sequences of the first and/or second strands of: AD-39787.1 (SEQ ID NOs: 269 and 696), or a modified or unmodified variant thereof.

A RNAi agent comprising a first and a second strand, wherein the sequences of the first and/or second strands are the sequences of the first and/or second strands of: AD-39793.1 (SEQ ID NOs: 270 and 697), or a modified or unmodified variant thereof.

A RNAi agent comprising a first and a second strand, wherein the sequences of the first and/or second strands are the sequences of the first and/or second strands of: AD-39799.1 (SEQ ID NOs: 271 and 698), or a modified or unmodified variant thereof.

A RNAi agent comprising a first and a second strand, wherein the sequences of the first and/or second strands are the sequences of the first and/or second strands of: AD-39805.1 (SEQ ID NOs: 272 and 699), or a modified or unmodified variant thereof.

A RNAi agent comprising a first and a second strand, wherein the sequences of the first and/or second strands are the sequences of the first and/or second strands of: AD-39811.1 (SEQ ID NOs: 273 and 700), or a modified or unmodified variant thereof.

A RNAi agent comprising a first and a second strand, wherein the sequences of the first and/or second strands are the sequences of the first and/or second strands of: AD-39817.1 (SEQ ID NOs: 274 and 701), or a modified or unmodified variant thereof.

A RNAi agent comprising a first and a second strand, wherein the sequences of the first and/or second strands are the sequences of the first and/or second strands of: AD-39823.1 (SEQ ID NOs: 275 and 702), or a modified or unmodified variant thereof.

A RNAi agent comprising a first and a second strand, wherein the sequences of the first and/or second strands are the sequences of the first and/or second strands of: AD-39782.1 (SEQ ID NOs: 276 and 703), or a modified or unmodified variant thereof.

A RNAi agent comprising a first and a second strand, wherein the sequences of the first and/or second strands are the sequences of the first and/or second strands of: AD-39788.1 (SEQ ID NOs: 277 and 704), or a modified or unmodified variant thereof.

A RNAi agent comprising a first and a second strand, wherein the sequences of the first and/or second strands are the sequences of the first and/or second strands of: AD-39794.1 (SEQ ID NOs: 278 and 705), or a modified or unmodified variant thereof.

A RNAi agent comprising a first and a second strand, wherein the sequences of the first and/or second strands are the sequences of the first and/or second strands of: AD-39800.1 (SEQ ID NOs: 279 and 706), or a modified or unmodified variant thereof.

A RNAi agent comprising a first and a second strand, wherein the sequences of the first and/or second strands are the sequences of the first and/or second strands of: AD-39806.1 (SEQ ID NOs: 280 and 707), or a modified or unmodified variant thereof.

A RNAi agent comprising a first and a second strand, wherein the sequences of the first and/or second strands are the sequences of the first and/or second strands of: AD-39812.1 (SEQ ID NOs: 281 and 708), or a modified or unmodified variant thereof.

A RNAi agent comprising a first and a second strand, wherein the sequences of the first and/or second strands are the sequences of the first and/or second strands of: AD-39818.1 (SEQ ID NOs: 282 and 709), or a modified or unmodified variant thereof.

A RNAi agent comprising a first and a second strand, wherein the sequences of the first and/or second strands are the sequences of the first and/or second strands of: AD-39824.1 (SEQ ID NOs: 283 and 710), or a modified or unmodified variant thereof.

A RNAi agent comprising a first and a second strand, wherein the sequences of the first and/or second strands are the sequences of the first and/or second strands of: AD-39783.1 (SEQ ID NOs: 284 and 711), or a modified or unmodified variant thereof.

A RNAi agent comprising a first and a second strand, wherein the sequences of the first and/or second strands are the sequences of the first and/or second strands of: AD-39789.1 (SEQ ID NOs: 285 and 712), or a modified or unmodified variant thereof.

A RNAi agent comprising a first and a second strand, wherein the sequences of the first and/or second strands are the sequences of the first and/or second strands of: AD-39795.1 (SEQ ID NOs: 286 and 713), or a modified or unmodified variant thereof.

A RNAi agent comprising a first and a second strand, wherein the sequences of the first and/or second strands are the sequences of the first and/or second strands of: AD-39801.1 (SEQ ID NOs: 287 and 714), or a modified or unmodified variant thereof.

A RNAi agent comprising a first and a second strand, wherein the sequences of the first and/or second strands are the sequences of the first and/or second strands of: AD-39807.1 (SEQ ID NOs: 288 and 715), or a modified or unmodified variant thereof.

A RNAi agent comprising a first and a second strand, wherein the sequences of the first and/or second strands are the sequences of the first and/or second strands of: AD-39813.1 (SEQ ID NOs: 289 and 716), or a modified or unmodified variant thereof.

A RNAi agent comprising a first and a second strand, wherein the sequences of the first and/or second strands are the sequences of the first and/or second strands of: AD-39819.1 (SEQ ID NOs: 290 and 717), or a modified or unmodified variant thereof.

A RNAi agent comprising a first and a second strand, wherein the sequences of the first and/or second strands are the sequences of the first and/or second strands of: AD-39825.1 (SEQ ID NOs: 291 and 718), or a modified or unmodified variant thereof.

A RNAi agent comprising a first and a second strand, wherein the sequences of the first and/or second strands are the sequences of the first and/or second strands of: AD-39831.1 (SEQ ID NOs: 292 and 719), or a modified or unmodified variant thereof.

A RNAi agent comprising a first and a second strand, wherein the sequences of the first and/or second strands are the sequences of the first and/or second strands of: AD-39837.1 (SEQ ID NOs: 293 and 720), or a modified or unmodified variant thereof.

A RNAi agent comprising a first and a second strand, wherein the sequences of the first and/or second strands are the sequences of the first and/or second strands of: AD-39843.1 (SEQ ID NOs: 294 and 721), or a modified or unmodified variant thereof.

A RNAi agent comprising a first and a second strand, wherein the sequences of the first and/or second strands are the sequences of the first and/or second strands of: AD-39849.1 (SEQ ID NOs: 295 and 722), or a modified or unmodified variant thereof.

A RNAi agent comprising a first and a second strand, wherein the sequences of the first and/or second strands are the sequences of the first and/or second strands of: AD-39855.1 (SEQ ID NOs: 296 and 723), or a modified or unmodified variant thereof.

A RNAi agent comprising a first and a second strand, wherein the sequences of the first and/or second strands are the sequences of the first and/or second strands of: AD-39861.1 (SEQ ID NOs: 297 and 724), or a modified or unmodified variant thereof.

A RNAi agent comprising a first and a second strand, wherein the sequences of the first and/or second strands are the sequences of the first and/or second strands of: AD-39867.1 (SEQ ID NOs: 298 and 725), or a modified or unmodified variant thereof.

A RNAi agent comprising a first and a second strand, wherein the sequences of the first and/or second strands are the sequences of the first and/or second strands of: AD-39826.1 (SEQ ID NOs: 299 and 726), or a modified or unmodified variant thereof.

A RNAi agent comprising a first and a second strand, wherein the sequences of the first and/or second strands are the sequences of the first and/or second strands of: AD-39832.1 (SEQ ID NOs: 300 and 727), or a modified or unmodified variant thereof.

A RNAi agent comprising a first and a second strand, wherein the sequences of the first and/or second strands are the sequences of the first and/or second strands of: AD-39838.1 (SEQ ID NOs: 301 and 728), or a modified or unmodified variant thereof.

A RNAi agent comprising a first and a second strand, wherein the sequences of the first and/or second strands are the sequences of the first and/or second strands of: AD-39844.1 (SEQ ID NOs: 302 and 729), or a modified or unmodified variant thereof.

A RNAi agent comprising a first and a second strand, wherein the sequences of the first and/or second strands are the sequences of the first and/or second strands of: AD-39850.1 (SEQ ID NOs: 303 and 730), or a modified or unmodified variant thereof.

A RNAi agent comprising a first and a second strand, wherein the sequences of the first and/or second strands are the sequences of the first and/or second strands of: AD-39856.1 (SEQ ID NOs: 304 and 731), or a modified or unmodified variant thereof.

A RNAi agent comprising a first and a second strand, wherein the sequences of the first and/or second strands are the sequences of the first and/or second strands of: AD-39862.1 (SEQ ID NOs: 305 and 732), or a modified or unmodified variant thereof.

A RNAi agent comprising a first and a second strand, wherein the sequences of the first and/or second strands are the sequences of the first and/or second strands of: AD-39868.1 (SEQ ID NOs: 306 and 733), or a modified or unmodified variant thereof.

A RNAi agent comprising a first and a second strand, wherein the sequences of the first and/or second strands are the sequences of the first and/or second strands of: AD-39827.1 (SEQ ID NOs: 307 and 734), or a modified or unmodified variant thereof.

A RNAi agent comprising a first and a second strand, wherein the sequences of the first and/or second strands are the sequences of the first and/or second strands of: AD-39833.1 (SEQ ID NOs: 308 and 735), or a modified or unmodified variant thereof.

A RNAi agent comprising a first and a second strand, wherein the sequences of the first and/or second strands are the sequences of the first and/or second strands of: AD-39839.1 (SEQ ID NOs: 309 and 736), or a modified or unmodified variant thereof.

A RNAi agent comprising a first and a second strand, wherein the sequences of the first and/or second strands are the sequences of the first and/or second strands of: AD-39845.1 (SEQ ID NOs: 310 and 737), or a modified or unmodified variant thereof.

A RNAi agent comprising a first and a second strand, wherein the sequences of the first and/or second strands are the sequences of the first and/or second strands of: AD-39851.1 (SEQ ID NOs: 311 and 738), or a modified or unmodified variant thereof.

A RNAi agent comprising a first and a second strand, wherein the sequences of the first and/or second strands are the sequences of the first and/or second strands of: AD-39857.1 (SEQ ID NOs: 312 and 739), or a modified or unmodified variant thereof.

A RNAi agent comprising a first and a second strand, wherein the sequences of the first and/or second strands are the sequences of the first and/or second strands of: AD-39863.1 (SEQ ID NOs: 313 and 740), or a modified or unmodified variant thereof.

A RNAi agent comprising a first and a second strand, wherein the sequences of the first and/or second strands are the sequences of the first and/or second strands of: AD-39869.1 (SEQ ID NOs: 314 and 741), or a modified or unmodified variant thereof.

A RNAi agent comprising a first and a second strand, wherein the sequences of the first and/or second strands are the sequences of the first and/or second strands of: AD-39828.1 (SEQ ID NOs: 315 and 742), or a modified or unmodified variant thereof.

A RNAi agent comprising a first and a second strand, wherein the sequences of the first and/or second strands are the sequences of the first and/or second strands of: AD-39834.1 (SEQ ID NOs: 316 and 743), or a modified or unmodified variant thereof.

A RNAi agent comprising a first and a second strand, wherein the sequences of the first and/or second strands are the sequences of the first and/or second strands of: AD-39840.1 (SEQ ID NOs: 317 and 744), or a modified or unmodified variant thereof.

A RNAi agent comprising a first and a second strand, wherein the sequences of the first and/or second strands are the sequences of the first and/or second strands of: AD-39846.1 (SEQ ID NOs: 318 and 745), or a modified or unmodified variant thereof.

A RNAi agent comprising a first and a second strand, wherein the sequences of the first and/or second strands are the sequences of the first and/or second strands of: AD-39852.1 (SEQ ID NOs: 319 and 746), or a modified or unmodified variant thereof.

A RNAi agent comprising a first and a second strand, wherein the sequences of the first and/or second strands are the sequences of the first and/or second strands of: AD-39858.1 (SEQ ID NOs: 320 and 747), or a modified or unmodified variant thereof.

A RNAi agent comprising a first and a second strand, wherein the sequences of the first and/or second strands are the sequences of the first and/or second strands of: AD-39864.1 (SEQ ID NOs: 321 and 748), or a modified or unmodified variant thereof.

A RNAi agent comprising a first and a second strand, wherein the sequences of the first and/or second strands are the sequences of the first and/or second strands of: AD-39870.1 (SEQ ID NOs: 322 and 749), or a modified or unmodified variant thereof.

A RNAi agent comprising a first and a second strand, wherein the sequences of the first and/or second strands are the sequences of the first and/or second strands of: AD-39829.1 (SEQ ID NOs: 323 and 750), or a modified or unmodified variant thereof.

A RNAi agent comprising a first and a second strand, wherein the sequences of the first and/or second strands are the sequences of the first and/or second strands of: AD-39835.1 (SEQ ID NOs: 324 and 751), or a modified or unmodified variant thereof.

A RNAi agent comprising a first and a second strand, wherein the sequences of the first and/or second strands are the sequences of the first and/or second strands of: AD-39841.1 (SEQ ID NOs: 325 and 752), or a modified or unmodified variant thereof.

A RNAi agent comprising a first and a second strand, wherein the sequences of the first and/or second strands are the sequences of the first and/or second strands of: AD-39853.1 (SEQ ID NOs: 326 and 753), or a modified or unmodified variant thereof.

A RNAi agent comprising a first and a second strand, wherein the sequences of the first and/or second strands are the sequences of the first and/or second strands of: AD-39859.1 (SEQ ID NOs: 327 and 754), or a modified or unmodified variant thereof.

A RNAi agent comprising a first and a second strand, wherein the sequences of the first and/or second strands are the sequences of the first and/or second strands of: AD-39865.1 (SEQ ID NOs: 328 and 755), or a modified or unmodified variant thereof.

A RNAi agent comprising a first and a second strand, wherein the sequences of the first and/or second strands are the sequences of the first and/or second strands of: AD-39871.1 (SEQ ID NOs: 329 and 756), or a modified or unmodified variant thereof.

A RNAi agent comprising a first and a second strand, wherein the sequences of the first and/or second strands are the sequences of the first and/or second strands of: AD-39830.1 (SEQ ID NOs: 330 and 757), or a modified or unmodified variant thereof.

A RNAi agent comprising a first and a second strand, wherein the sequences of the first and/or second strands are the sequences of the first and/or second strands of: AD-39836.1 (SEQ ID NOs: 331 and 758), or a modified or unmodified variant thereof.

A RNAi agent comprising a first and a second strand, wherein the sequences of the first and/or second strands are the sequences of the first and/or second strands of: AD-39842.1 (SEQ ID NOs: 332 and 759), or a modified or unmodified variant thereof.

A RNAi agent comprising a first and a second strand, wherein the sequences of the first and/or second strands are the sequences of the first and/or second strands of: AD-39848.1 (SEQ ID NOs: 333 and 760), or a modified or unmodified variant thereof.

A RNAi agent comprising a first and a second strand, wherein the sequences of the first and/or second strands are the sequences of the first and/or second strands of: AD-39854.1 (SEQ ID NOs: 334 and 761), or a modified or unmodified variant thereof.

A RNAi agent comprising a first and a second strand, wherein the sequences of the first and/or second strands are the sequences of the first and/or second strands of: AD-39860.1 (SEQ ID NOs: 335 and 762), or a modified or unmodified variant thereof.

A RNAi agent comprising a first and a second strand, wherein the sequences of the first and/or second strands are the sequences of the first and/or second strands of: AD-39866.1 (SEQ ID NOs: 336 and 763), or a modified or unmodified variant thereof.

A RNAi agent comprising a first and a second strand, wherein the sequences of the first and/or second strands are the sequences of the first and/or second strands of: AD-39992.1 (SEQ ID NOs: 337 and 764), or a modified or unmodified variant thereof.

A RNAi agent comprising a first and a second strand, wherein the sequences of the first and/or second strands are the sequences of the first and/or second strands of: AD-39998.1 (SEQ ID NOs: 338 and 765), or a modified or unmodified variant thereof.

A RNAi agent comprising a first and a second strand, wherein the sequences of the first and/or second strands are the sequences of the first and/or second strands of: AD-40004.1 (SEQ ID NOs: 339 and 766), or a modified or unmodified variant thereof.

A RNAi agent comprising a first and a second strand, wherein the sequences of the first and/or second strands are the sequences of the first and/or second strands of: AD-40010.1 (SEQ ID NOs: 340 and 767), or a modified or unmodified variant thereof.

A RNAi agent comprising a first and a second strand, wherein the sequences of the first and/or second strands are the sequences of the first and/or second strands of: AD-40016.1 (SEQ ID NOs: 341 and 768), or a modified or unmodified variant thereof.

A RNAi agent comprising a first and a second strand, wherein the sequences of the first and/or second strands are the sequences of the first and/or second strands of: AD-40022.1 (SEQ ID NOs: 342 and 769), or a modified or unmodified variant thereof.

A RNAi agent comprising a first and a second strand, wherein the sequences of the first and/or second strands are the sequences of the first and/or second strands of: AD-40028.1 (SEQ ID NOs: 343 and 770), or a modified or unmodified variant thereof.

A RNAi agent comprising a first and a second strand, wherein the sequences of the first and/or second strands are the sequences of the first and/or second strands of: AD-40034.1 (SEQ ID NOs: 344 and 771), or a modified or unmodified variant thereof.

A RNAi agent comprising a first and a second strand, wherein the sequences of the first and/or second strands are the sequences of the first and/or second strands of: AD-39999.1 (SEQ ID NOs: 345 and 772), or a modified or unmodified variant thereof.

A RNAi agent comprising a first and a second strand, wherein the sequences of the first and/or second strands are the sequences of the first and/or second strands of: AD-40005.1 (SEQ ID NOs: 346 and 773), or a modified or unmodified variant thereof.

A RNAi agent comprising a first and a second strand, wherein the sequences of the first and/or second strands are the sequences of the first and/or second strands of: AD-40011.1 (SEQ ID NOs: 347 and 774), or a modified or unmodified variant thereof.

A RNAi agent comprising a first and a second strand, wherein the sequences of the first and/or second strands are the sequences of the first and/or second strands of: AD-40017.1 (SEQ ID NOs: 348 and 775), or a modified or unmodified variant thereof.

A RNAi agent comprising a first and a second strand, wherein the sequences of the first and/or second strands are the sequences of the first and/or second strands of: AD-40029.1 (SEQ ID NOs: 349 and 776), or a modified or unmodified variant thereof.

A RNAi agent comprising a first and a second strand, wherein the sequences of the first and/or second strands are the sequences of the first and/or second strands of: AD-40035.1 (SEQ ID NOs: 350 and 777), or a modified or unmodified variant thereof.

A RNAi agent comprising a first and a second strand, wherein the sequences of the first and/or second strands are the sequences of the first and/or second strands of: AD-39994.1 (SEQ ID NOs: 351 and 778), or a modified or unmodified variant thereof.

A RNAi agent comprising a first and a second strand, wherein the sequences of the first and/or second strands are the sequences of the first and/or second strands of: AD-40000.1 (SEQ ID NOs: 352 and 779), or a modified or unmodified variant thereof.

A RNAi agent comprising a first and a second strand, wherein the sequences of the first and/or second strands are the sequences of the first and/or second strands of: AD-40006.1 (SEQ ID NOs: 353 and 780), or a modified or unmodified variant thereof.

A RNAi agent comprising a first and a second strand, wherein the sequences of the first and/or second strands are the sequences of the first and/or second strands of: AD-40012.1 (SEQ ID NOs: 354 and 781), or a modified or unmodified variant thereof.

A RNAi agent comprising a first and a second strand, wherein the sequences of the first and/or second strands are the sequences of the first and/or second strands of: AD-40018.1 (SEQ ID NOs: 355 and 782), or a modified or unmodified variant thereof.

A RNAi agent comprising a first and a second strand, wherein the sequences of the first and/or second strands are the sequences of the first and/or second strands of: AD-40024.1 (SEQ ID NOs: 356 and 783), or a modified or unmodified variant thereof.

A RNAi agent comprising a first and a second strand, wherein the sequences of the first and/or second strands are the sequences of the first and/or second strands of: AD-40030.1 (SEQ ID NOs: 357 and 784), or a modified or unmodified variant thereof.

A RNAi agent comprising a first and a second strand, wherein the sequences of the first and/or second strands are the sequences of the first and/or second strands of: AD-40036.1 (SEQ ID NOs: 358 and 785), or a modified or unmodified variant thereof.

A RNAi agent comprising a first and a second strand, wherein the sequences of the first and/or second strands are the sequences of the first and/or second strands of: AD-39995.1 (SEQ ID NOs: 359 and 786), or a modified or unmodified variant thereof.

A RNAi agent comprising a first and a second strand, wherein the sequences of the first and/or second strands are the sequences of the first and/or second strands of: AD-40001.1 (SEQ ID NOs: 360 and 787), or a modified or unmodified variant thereof.

A RNAi agent comprising a first and a second strand, wherein the sequences of the first and/or second strands are the sequences of the first and/or second strands of: AD-40007.1 (SEQ ID NOs: 361 and 788), or a modified or unmodified variant thereof.

A RNAi agent comprising a first and a second strand, wherein the sequences of the first and/or second strands are the sequences of the first and/or second strands of: AD-40013.1 (SEQ ID NOs: 362 and 789), or a modified or unmodified variant thereof.

A RNAi agent comprising a first and a second strand, wherein the sequences of the first and/or second strands are the sequences of the first and/or second strands of: AD-40019.1 (SEQ ID NOs: 363 and 790), or a modified or unmodified variant thereof.

A RNAi agent comprising a first and a second strand, wherein the sequences of the first and/or second strands are the sequences of the first and/or second strands of: AD-40025.1 (SEQ ID NOs: 364 and 791), or a modified or unmodified variant thereof.

A RNAi agent comprising a first and a second strand, wherein the sequences of the first and/or second strands are the sequences of the first and/or second strands of: AD-40031.1 (SEQ ID NOs: 365 and 792), or a modified or unmodified variant thereof.

A RNAi agent comprising a first and a second strand, wherein the sequences of the first and/or second strands are the sequences of the first and/or second strands of: AD-40037.1 (SEQ ID NOs: 366 and 793), or a modified or unmodified variant thereof.

A RNAi agent comprising a first and a second strand, wherein the sequences of the first and/or second strands are the sequences of the first and/or second strands of: AD-39996.1 (SEQ ID NOs: 367 and 794), or a modified or unmodified variant thereof.

A RNAi agent comprising a first and a second strand, wherein the sequences of the first and/or second strands are the sequences of the first and/or second strands of: AD-40002.1 (SEQ ID NOs: 368 and 795), or a modified or unmodified variant thereof.

A RNAi agent comprising a first and a second strand, wherein the sequences of the first and/or second strands are the sequences of the first and/or second strands of: AD-40008.1 (SEQ ID NOs: 369 and 796), or a modified or unmodified variant thereof.

A RNAi agent comprising a first and a second strand, wherein the sequences of the first and/or second strands are the sequences of the first and/or second strands of: AD-40014.1 (SEQ ID NOs: 370 and 797), or a modified or unmodified variant thereof.

A RNAi agent comprising a first and a second strand, wherein the sequences of the first and/or second strands are the sequences of the first and/or second strands of: AD-40020.1 (SEQ ID NOs: 371 and 798), or a modified or unmodified variant thereof.

A RNAi agent comprising a first and a second strand, wherein the sequences of the first and/or second strands are the sequences of the first and/or second strands of: AD-40026.1 (SEQ ID NOs: 372 and 799), or a modified or unmodified variant thereof.

A RNAi agent comprising a first and a second strand, wherein the sequences of the first and/or second strands are the sequences of the first and/or second strands of: AD-40032.1 (SEQ ID NOs: 373 and 800), or a modified or unmodified variant thereof.

A RNAi agent comprising a first and a second strand, wherein the sequences of the first and/or second strands are the sequences of the first and/or second strands of: AD-40038.1 (SEQ ID NOs: 374 and 801), or a modified or unmodified variant thereof.

A RNAi agent comprising a first and a second strand, wherein the sequences of the first and/or second strands are the sequences of the first and/or second strands of: AD-39997.1 (SEQ ID NOs: 375 and 802), or a modified or unmodified variant thereof.

A RNAi agent comprising a first and a second strand, wherein the sequences of the first and/or second strands are the sequences of the first and/or second strands of: AD-40009.1 (SEQ ID NOs: 376 and 803), or a modified or unmodified variant thereof.

A RNAi agent comprising a first and a second strand, wherein the sequences of the first and/or second strands are the sequences of the first and/or second strands of: AD-40015.1 (SEQ ID NOs: 377 and 804), or a modified or unmodified variant thereof.

A RNAi agent comprising a first and a second strand, wherein the sequences of the first and/or second strands are the sequences of the first and/or second strands of: AD-40021.1 (SEQ ID NOs: 378 and 805), or a modified or unmodified variant thereof.

A RNAi agent comprising a first and a second strand, wherein the sequences of the first and/or second strands are the sequences of the first and/or second strands of: AD-40027.1 (SEQ ID NOs: 379 and 806), or a modified or unmodified variant thereof.

A RNAi agent comprising a first and a second strand, wherein the sequences of the first and/or second strands are the sequences of the first and/or second strands of: AD-40033.1 (SEQ ID NOs: 380 and 807), or a modified or unmodified variant thereof.

A RNAi agent comprising a first and a second strand, wherein the sequences of the first and/or second strands are the sequences of the first and/or second strands of: AD-40039.1 (SEQ ID NOs: 381 and 808), or a modified or unmodified variant thereof.

A RNAi agent comprising a first and a second strand, wherein the sequences of the first and/or second strands are the sequences of the first and/or second strands of: AD-40045.1 (SEQ ID NOs: 382 and 809), or a modified or unmodified variant thereof.

A RNAi agent comprising a first and a second strand, wherein the sequences of the first and/or second strands are the sequences of the first and/or second strands of: AD-40051.1 (SEQ ID NOs: 383 and 810), or a modified or unmodified variant thereof.

A RNAi agent comprising a first and a second strand, wherein the sequences of the first and/or second strands are the sequences of the first and/or second strands of: AD-40057.1 (SEQ ID NOs: 384 and 811), or a modified or unmodified variant thereof.

A RNAi agent comprising a first and a second strand, wherein the sequences of the first and/or second strands are the sequences of the first and/or second strands of: AD-40063.1 (SEQ ID NOs: 385 and 812), or a modified or unmodified variant thereof.

A RNAi agent comprising a first and a second strand, wherein the sequences of the first and/or second strands are the sequences of the first and/or second strands of: AD-40069.1 (SEQ ID NOs: 386 and 813), or a modified or unmodified variant thereof.

A RNAi agent comprising a first and a second strand, wherein the sequences of the first and/or second strands are the sequences of the first and/or second strands of: AD-40075.1 (SEQ ID NOs: 387 and 814), or a modified or unmodified variant thereof.

A RNAi agent comprising a first and a second strand, wherein the sequences of the first and/or second strands are the sequences of the first and/or second strands of: AD-40081.1 (SEQ ID NOs: 388 and 815), or a modified or unmodified variant thereof.

A RNAi agent comprising a first and a second strand, wherein the sequences of the first and/or second strands are the sequences of the first and/or second strands of: AD-40040.1 (SEQ ID NOs: 389 and 816), or a modified or unmodified variant thereof.

A RNAi agent comprising a first and a second strand, wherein the sequences of the first and/or second strands are the sequences of the first and/or second strands of: AD-40046.1 (SEQ ID NOs: 390 and 817), or a modified or unmodified variant thereof.

A RNAi agent comprising a first and a second strand, wherein the sequences of the first and/or second strands are the sequences of the first and/or second strands of: AD-40052.1 (SEQ ID NOs: 391 and 818), or a modified or unmodified variant thereof.

A RNAi agent comprising a first and a second strand, wherein the sequences of the first and/or second strands are the sequences of the first and/or second strands of: AD-40058.1 (SEQ ID NOs: 392 and 819), or a modified or unmodified variant thereof.

A RNAi agent comprising a first and a second strand, wherein the sequences of the first and/or second strands are the sequences of the first and/or second strands of: AD-40064.1 (SEQ ID NOs: 393 and 820), or a modified or unmodified variant thereof.

A RNAi agent comprising a first and a second strand, wherein the sequences of the first and/or second strands are the sequences of the first and/or second strands of: AD-40070.1 (SEQ ID NOs: 394 and 821), or a modified or unmodified variant thereof.

A RNAi agent comprising a first and a second strand, wherein the sequences of the first and/or second strands are the sequences of the first and/or second strands of: AD-40076.1 (SEQ ID NOs: 395 and 822), or a modified or unmodified variant thereof.

A RNAi agent comprising a first and a second strand, wherein the sequences of the first and/or second strands are the sequences of the first and/or second strands of: AD-40082.1 (SEQ ID NOs: 396 and 823), or a modified or unmodified variant thereof.

A RNAi agent comprising a first and a second strand, wherein the sequences of the first and/or second strands are the sequences of the first and/or second strands of: AD-40041.1 (SEQ ID NOs: 397 and 824), or a modified or unmodified variant thereof.

A RNAi agent comprising a first and a second strand, wherein the sequences of the first and/or second strands are the sequences of the first and/or second strands of: AD-40047.1 (SEQ ID NOs: 398 and 825), or a modified or unmodified variant thereof.

A RNAi agent comprising a first and a second strand, wherein the sequences of the first and/or second strands are the sequences of the first and/or second strands of: AD-40053.1 (SEQ ID NOs: 399 and 826), or a modified or unmodified variant thereof.

A RNAi agent comprising a first and a second strand, wherein the sequences of the first and/or second strands are the sequences of the first and/or second strands of: AD-40059.1 (SEQ ID NOs: 400 and 827), or a modified or unmodified variant thereof.

A RNAi agent comprising a first and a second strand, wherein the sequences of the first and/or second strands are the sequences of the first and/or second strands of: AD-40065.1 (SEQ ID NOs: 401 and 828), or a modified or unmodified variant thereof.

A RNAi agent comprising a first and a second strand, wherein the sequences of the first and/or second strands are the sequences of the first and/or second strands of: AD-40071.1 (SEQ ID NOs: 402 and 829), or a modified or unmodified variant thereof.

A RNAi agent comprising a first and a second strand, wherein the sequences of the first and/or second strands are the sequences of the first and/or second strands of: AD-40077.1 (SEQ ID NOs: 403 and 830), or a modified or unmodified variant thereof.

A RNAi agent comprising a first and a second strand, wherein the sequences of the first and/or second strands are the sequences of the first and/or second strands of: AD-40083.1 (SEQ ID NOs: 404 and 831), or a modified or unmodified variant thereof.

A RNAi agent comprising a first and a second strand, wherein the sequences of the first and/or second strands are the sequences of the first and/or second strands of: AD-40042.1 (SEQ ID NOs: 405 and 832), or a modified or unmodified variant thereof.

A RNAi agent comprising a first and a second strand, wherein the sequences of the first and/or second strands are the sequences of the first and/or second strands of: AD-40048.1 (SEQ ID NOs: 406 and 833), or a modified or unmodified variant thereof.

A RNAi agent comprising a first and a second strand, wherein the sequences of the first and/or second strands are the sequences of the first and/or second strands of: AD-40054.1 (SEQ ID NOs: 407 and 834), or a modified or unmodified variant thereof.

A RNAi agent comprising a first and a second strand, wherein the sequences of the first and/or second strands are the sequences of the first and/or second strands of: AD-40060.1 (SEQ ID NOs: 408 and 835), or a modified or unmodified variant thereof.

A RNAi agent comprising a first and a second strand, wherein the sequences of the first and/or second strands are the sequences of the first and/or second strands of: AD-40066.1 (SEQ ID NOs: 409 and 836), or a modified or unmodified variant thereof.

A RNAi agent comprising a first and a second strand, wherein the sequences of the first and/or second strands are the sequences of the first and/or second strands of: AD-40072.1 (SEQ ID NOs: 410 and 837), or a modified or unmodified variant thereof.

A RNAi agent comprising a first and a second strand, wherein the sequences of the first and/or second strands are the sequences of the first and/or second strands of: AD-40078.1 (SEQ ID NOs: 411 and 838), or a modified or unmodified variant thereof.

A RNAi agent comprising a first and a second strand, wherein the sequences of the first and/or second strands are the sequences of the first and/or second strands of: AD-40084.1 (SEQ ID NOs: 412 and 839), or a modified or unmodified variant thereof.

A RNAi agent comprising a first and a second strand, wherein the sequences of the first and/or second strands are the sequences of the first and/or second strands of: AD-40043.1 (SEQ ID NOs: 413 and 840), or a modified or unmodified variant thereof.

A RNAi agent comprising a first and a second strand, wherein the sequences of the first and/or second strands are the sequences of the first and/or second strands of: AD-40049.1 (SEQ ID NOs: 414 and 841), or a modified or unmodified variant thereof.

A RNAi agent comprising a first and a second strand, wherein the sequences of the first and/or second strands are the sequences of the first and/or second strands of: AD-40055.1 (SEQ ID NOs: 415 and 842), or a modified or unmodified variant thereof.

A RNAi agent comprising a first and a second strand, wherein the sequences of the first and/or second strands are the sequences of the first and/or second strands of: AD-40061.1 (SEQ ID NOs: 416 and 843), or a modified or unmodified variant thereof.

A RNAi agent comprising a first and a second strand, wherein the sequences of the first and/or second strands are the sequences of the first and/or second strands of: AD-40067.1 (SEQ ID NOs: 417 and 844), or a modified or unmodified variant thereof.

A RNAi agent comprising a first and a second strand, wherein the sequences of the first and/or second strands are the sequences of the first and/or second strands of: AD-40073.1 (SEQ ID NOs: 418 and 845), or a modified or unmodified variant thereof.

A RNAi agent comprising a first and a second strand, wherein the sequences of the first and/or second strands are the sequences of the first and/or second strands of: AD-40079.1 (SEQ ID NOs: 419 and 846), or a modified or unmodified variant thereof.

A RNAi agent comprising a first and a second strand, wherein the sequences of the first and/or second strands are the sequences of the first and/or second strands of: AD-40085.1 (SEQ ID NOs: 420 and 847), or a modified or unmodified variant thereof.

A RNAi agent comprising a first and a second strand, wherein the sequences of the first and/or second strands are the sequences of the first and/or second strands of: AD-40044.1 (SEQ ID NOs: 421 and 848), or a modified or unmodified variant thereof.

A RNAi agent comprising a first and a second strand, wherein the sequences of the first and/or second strands are the sequences of the first and/or second strands of: AD-40050.1 (SEQ ID NOs: 422 and 849), or a modified or unmodified variant thereof.

A RNAi agent comprising a first and a second strand, wherein the sequences of the first and/or second strands are the sequences of the first and/or second strands of: AD-40056.1 (SEQ ID NOs: 423 and 850), or a modified or unmodified variant thereof.

A RNAi agent comprising a first and a second strand, wherein the sequences of the first and/or second strands are the sequences of the first and/or second strands of: AD-40062.1 (SEQ ID NOs: 424 and 851), or a modified or unmodified variant thereof.

A RNAi agent comprising a first and a second strand, wherein the sequences of the first and/or second strands are the sequences of the first and/or second strands of: AD-40068.1 (SEQ ID NOs: 425 and 852), or a modified or unmodified variant thereof.

A RNAi agent comprising a first and a second strand, wherein the sequences of the first and/or second strands are the sequences of the first and/or second strands of: AD-40074.1 (SEQ ID NOs: 426 and 853), or a modified or unmodified variant thereof.

A RNAi agent comprising a first and a second strand, wherein the sequences of the first and/or second strands are the sequences of the first and/or second strands of: AD-40080.1 (SEQ ID NOs: 427 and 854), or a modified or unmodified variant thereof.

A RNAi agent comprising a first and a second strand, wherein the sequences of the first and/or second strands are the sequences of the first and/or second strands of: AD-39720.1 (SEQ ID NOs: 1716 and 1754), or a modified or unmodified variant thereof.

A RNAi agent comprising a first and a second strand, wherein the sequences of the first and/or second strands are the sequences of the first and/or second strands of: AD-39706.1 (SEQ ID NOs: 1717 and 1755), or a modified or unmodified variant thereof.

A RNAi agent comprising a first and a second strand, wherein the sequences of the first and/or second strands are the sequences of the first and/or second strands of: AD-39712.1 (SEQ ID NOs: 1718 and 1756), or a modified or unmodified variant thereof.

A RNAi agent comprising a first and a second strand, wherein the sequences of the first and/or second strands are the sequences of the first and/or second strands of: AD-39760.1 (SEQ ID NOs: 1719 and 1757), or a modified or unmodified variant thereof.

A RNAi agent comprising a first and a second strand, wherein the sequences of the first and/or second strands are the sequences of the first and/or second strands of: AD-39732.1 (SEQ ID NOs: 1720 and 1758), or a modified or unmodified variant thereof.

A RNAi agent comprising a first and a second strand, wherein the sequences of the first and/or second strands are the sequences of the first and/or second strands of: AD-35541.4 (SEQ ID NOs: 1721 and 1759), or a modified or unmodified variant thereof.

A RNAi agent comprising a first and a second strand, wherein the sequences of the first and/or second strands are the sequences of the first and/or second strands of: AD-39735.1 or hs_KRAS_321_A22S26 (SEQ ID NOs: 1722 and 1760), or a modified or unmodified variant thereof.

A RNAi agent comprising a first and a second strand, wherein the sequences of the first and/or second strands are the sequences of the first and/or second strands of: AD-39741.1, or modified or unmodified variants thereof.

A RNAi agent comprising a first and a second strand, wherein the sequences of the first and/or second strands are the sequences of the first and/or second strands of: hs_KRAS_322_A22S26 (SEQ ID NOs: 1723 and 1761), or a modified or unmodified variant thereof.

A RNAi agent comprising a first and a second strand, wherein the sequences of the first and/or second strands are the sequences of the first and/or second strands of: AD-39778.1 (SEQ ID NOs: 1724 and 1762), or a modified or unmodified variant thereof.

A RNAi agent comprising a first and a second strand, wherein the sequences of the first and/or second strands are the sequences of the first and/or second strands of: AD-39790.1 (SEQ ID NOs: 1725 and 1763), or a modified or unmodified variant thereof.

A RNAi agent comprising a first and a second strand, wherein the sequences of the first and/or second strands are the sequences of the first and/or second strands of: AD-39822.1 (SEQ ID NOs: 1726 and 1764), or a modified or unmodified variant thereof.

A RNAi agent comprising a first and a second strand, wherein the sequences of the first and/or second strands are the sequences of the first and/or second strands of: AD-35609.4 (SEQ ID NOs: 1727 and 1765), or a modified or unmodified variant thereof.

A RNAi agent comprising a first and a second strand, wherein the sequences of the first and/or second strands are the sequences of the first and/or second strands of: AD-35581.4 (SEQ ID NOs: 1728 and 1766), or a modified or unmodified variant thereof.

A RNAi agent comprising a first and a second strand, wherein the sequences of the first and/or second strands are the sequences of the first and/or second strands of: AD-39845.1 (SEQ ID NOs: 1729 and 1767), or a modified or unmodified variant thereof.

A RNAi agent comprising a first and a second strand, wherein the sequences of the first and/or second strands are the sequences of the first and/or second strands of: AD-39858.1 (SEQ ID NOs: 1730 and 1768), or a modified or unmodified variant thereof.

A RNAi agent comprising a first and a second strand, wherein the sequences of the first and/or second strands are the sequences of the first and/or second strands of: AD-39870.1 (SEQ ID NOs: 1731 and 1769), or a modified or unmodified variant thereof.

A RNAi agent comprising a first and a second strand, wherein the sequences of the first and/or second strands are the sequences of the first and/or second strands of: AD-35576.4 (SEQ ID NOs: 1732 and 1770), or a modified or unmodified variant thereof.

A RNAi agent comprising a first and a second strand, wherein the sequences of the first and/or second strands are the sequences of the first and/or second strands of: AD-35588.4 (SEQ ID NOs: 1733 and 1771), or a modified or unmodified variant thereof.

A RNAi agent comprising a first and a second strand, wherein the sequences of the first and/or second strands are the sequences of the first and/or second strands of: AD-35600.1 (SEQ ID NOs: 1734 and 1772), or a modified or unmodified variant thereof.

A RNAi agent comprising a first and a second strand, wherein the sequences of the first and/or second strands are the sequences of the first and/or second strands of: AD-35606.1 (SEQ ID NOs: 1735 and 1773), or a modified or unmodified variant thereof.

A RNAi agent comprising a first and a second strand, wherein the sequences of the first and/or second strands are the sequences of the first and/or second strands of: AD-38151.1 (SEQ ID NOs: 1736 and 1774), or a modified or unmodified variant thereof.

A RNAi agent comprising a first and a second strand, wherein the sequences of the first and/or second strands are the sequences of the first and/or second strands of: AD-38163.1 (SEQ ID NOs: 1737 and 1775), or a modified or unmodified variant thereof.

A RNAi agent comprising a first and a second strand, wherein the sequences of the first and/or second strands are the sequences of the first and/or second strands of: AD-39999.1 (SEQ ID NOs: 1738 and 1776), or a modified or unmodified variant thereof.

A RNAi agent comprising a first and a second strand, wherein the sequences of the first and/or second strands are the sequences of the first and/or second strands of: AD-38159.1 or hs_KRAS_528_A22S26 (SEQ ID NOs: 1739 and 1777), or a modified or unmodified variant thereof.

A RNAi agent comprising a first and a second strand, wherein the sequences of the first and/or second strands are the sequences of the first and/or second strands of: AD-38130.1 or hs_KRAS_531_A22S26 (SEQ ID NOs: 1740 and 1778), or a modified or unmodified variant thereof.

A RNAi agent comprising a first and a second strand, wherein the sequences of the first and/or second strands are the sequences of the first and/or second strands of: AD-38136.1 (SEQ ID NOs: 1741 and 1779), or a modified or unmodified variant thereof.

A RNAi agent comprising a first and a second strand, wherein the sequences of the first and/or second strands are the sequences of the first and/or second strands of: AD-40036.1 (SEQ ID NOs: 1742 and 1780), or a modified or unmodified variant thereof.

A RNAi agent comprising a first and a second strand, wherein the sequences of the first and/or second strands are the sequences of the first and/or second strands of: AD-40008.1 (SEQ ID NOs: 1743 and 1781), or a modified or unmodified variant thereof.

A RNAi agent comprising a first and a second strand, wherein the sequences of the first and/or second strands are the sequences of the first and/or second strands of: AD-40021.1 (SEQ ID NOs: 1744 and 1782), or a modified or unmodified variant thereof.

A RNAi agent comprising a first and a second strand, wherein the sequences of the first and/or second strands are the sequences of the first and/or second strands of: AD-40077.1 (SEQ ID NOs: 1745 and 1783), or a modified or unmodified variant thereof.

A RNAi agent comprising a first and a second strand, wherein the sequences of the first and/or second strands are the sequences of the first and/or second strands of: AD-40072.1 (SEQ ID NOs: 1746 and 1784), or a modified or unmodified variant thereof.

A RNAi agent comprising a first and a second strand, wherein the sequences of the first and/or second strands are the sequences of the first and/or second strands of: AD-40061.1 (SEQ ID NOs: 1747 and 1785), or a modified or unmodified variant thereof.

A RNAi agent comprising a first and a second strand, wherein the sequences of the first and/or second strands are the sequences of the first and/or second strands of: AD-40068.1 (SEQ ID NOs: 1748 and 1786), or a modified or unmodified variant thereof.

A RNAi agent comprising a first and a second strand, wherein the sequences of the first and/or second strands are the sequences of the first and/or second strands of: AD-38131.1 (SEQ ID NOs: 1749 and 1787), or a modified or unmodified variant thereof.

A RNAi agent comprising a first and a second strand, wherein the sequences of the first and/or second strands are the sequences of the first and/or second strands of: AD-38167.1 (SEQ ID NOs: 1750 and 1788), or a modified or unmodified variant thereof.

A RNAi agent comprising a first and a second strand, wherein the sequences of the first and/or second strands are the sequences of the first and/or second strands of: hs_KRAS 1273_A22S26 (SEQ ID NOs: 1751 and 1789), or a modified or unmodified variant thereof.

A RNAi agent comprising a first and a second strand, wherein the sequences of the first and/or second strands are the sequences of the first and/or second strands of: hs_KRAS 2892_A37S26 (SEQ ID NOs: 1752 and 1790), or a modified or unmodified variant thereof.

A RNAi agent comprising a first and a second strand, wherein the sequences of the first and/or second strands are the sequences of the first and/or second strands of: hs_KRAS 4731_A22S26 (SEQ ID NOs: 1753 and 1791), or a modified or unmodified variant thereof.

**Specific embodiments wherein the sequences of the first and/or second strands are the sequences of the first and/or second strand of a RNAi agent disclosed herein, further comprising up to about 20 additional nucleotides**

In one embodiment, the present disclosure pertains to a composition comprising any one or more of: a RNAi agent comprising a first and a second strand, wherein the sequence of the first and/or second strand is the sequence of a first and/or second strand of any RNAi agent from any of Tables 1 to 6, or modified and unmodified variants thereof, wherein the first and/or second strand further comprise up to about 20 additional nucleotides, wherein the first and/or second strand further comprise up to about 20 additional nucleotides.

Thus, in various embodiments, the present disclosure pertains to a composition comprising any one or more of the following:

A RNAi agent comprising a first and a second strand, wherein the sequence of the first and/or second strand is the sequence of a first and/or second strand of: AD-35523.3 (SEQ ID NOs: 1 and 428), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-35529.1 (SEQ ID NOs: 2 and 429), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-35535.4 (SEQ ID NOs: 3 and 430), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-35541.4 (SEQ ID NOs: 4 and 431), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-35547.4 (SEQ ID NOs: 5 and 432), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-35553.4 (SEQ ID NOs: 6 and 433), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-35559.4 (SEQ ID NOs: 7 and 434), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-35565.4 (SEQ ID NOs: 8 and 435), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-35524.4 (SEQ ID NOs: 9 and 436), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-35530.4 (SEQ ID NOs: 10 and 437), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-35536.4 (SEQ ID NOs: 11 and 438), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-35542.4 (SEQ ID NOs: 12 and 439), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-35548.4 (SEQ ID NOs: 13 and 440), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-35554.4 (SEQ ID NOs: 14 and 441), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-35560.2 (SEQ ID NOs: 15 and 442), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-35566.4 (SEQ ID NOs: 16 and 443), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-35525.2 (SEQ ID NOs: 17 and 444), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-35531.4 (SEQ ID NOs: 18 and 445), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-35537.4 (SEQ ID NOs: 19 and 446), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-35543.2 (SEQ ID NOs: 20 and 447), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-35549.4 (SEQ ID NOs: 21 and 448), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-35555.4 (SEQ ID NOs: 22 and 449), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-35561.4 (SEQ ID NOs: 23 and 450), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-35567.4 (SEQ ID NOs: 24 and 451), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-35526.4 (SEQ ID NOs: 25 and 452), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-35532.4 (SEQ ID NOs: 26 and 453), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-35538.4 (SEQ ID NOs: 27 and 454), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-35544.4 (SEQ ID NOs: 28 and 455), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-35550.4 (SEQ ID NOs: 29 and 456), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-35556.4 (SEQ ID NOs: 30 and 457), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-35562.4 (SEQ ID NOs: 31 and 458), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-35568.4 (SEQ ID NOs: 32 and 459), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-35527.4 (SEQ ID NOs: 33 and 460), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-35533.4 (SEQ ID NOs: 34 and 461), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-35539.4 (SEQ ID NOs: 35 and 462), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-35545.4 (SEQ ID NOs: 36 and 463), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-35551.4 (SEQ ID NOs: 37 and 464), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-35563.4 (SEQ ID NOs: 38 and 465), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-35528.4 (SEQ ID NOs: 39 and 466), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-35540.4 (SEQ ID NOs: 40 and 467), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-35546.4 (SEQ ID NOs: 41 and 468), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-35552.4 (SEQ ID NOs: 42 and 469), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-35558.4 (SEQ ID NOs: 43 and 470), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-35564.4 (SEQ ID NOs: 44 and 471), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-35570.4 (SEQ ID NOs: 45 and 472), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-35571.4 (SEQ ID NOs: 46 and 473), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-35577.4 (SEQ ID NOs: 47 and 474), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-35583.4 (SEQ ID NOs: 48 and 475), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-35589.4 (SEQ ID NOs: 49 and 476), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-35595.4 (SEQ ID NOs: 50 and 477), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-35601.4 (SEQ ID NOs: 51 and 478), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-35607.4 (SEQ ID NOs: 52 and 479), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-35613.4 (SEQ ID NOs: 53 and 480), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-35572.4 (SEQ ID NOs: 54 and 481), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-35578.4 (SEQ ID NOs: 55 and 482), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-35584.4 (SEQ ID NOs: 56 and 483), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-35590.4 (SEQ ID NOs: 57 and 484), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-35596.4 (SEQ ID NOs: 58 and 485), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-35602.4 (SEQ ID NOs: 59 and 486), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-35608.4 (SEQ ID NOs: 60 and 487), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-35614.4 (SEQ ID NOs: 61 and 488), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-35573.4 (SEQ ID NOs: 62 and 489), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-35579.4 (SEQ ID NOs: 63 and 490), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-35585.4 (SEQ ID NOs: 64 and 491), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-35591.4 (SEQ ID NOs: 65 and 492), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-35597.4 (SEQ ID NOs: 66 and 493), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-35603.4 (SEQ ID NOs: 67 and 494), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-35609.4 (SEQ ID NOs: 68 and 495), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-35615.4 (SEQ ID NOs: 69 and 496), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-35574.4 (SEQ ID NOs: 70 and 497), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-35580.1 (SEQ ID NOs: 71 and 498), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-35586.4 (SEQ ID NOs: 72 and 499), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-35592.4 (SEQ ID NOs: 73 and 500), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-35598.4 (SEQ ID NOs: 74 and 501), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-35604.4 (SEQ ID NOs: 75 and 502), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-35610.4 (SEQ ID NOs: 76 and 503), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-35616.4 (SEQ ID NOs: 77 and 504), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-35575.4 (SEQ ID NOs: 78 and 505), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-35581.4 (SEQ ID NOs: 79 and 506), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-35587.4 (SEQ ID NOs: 80 and 507), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-35593.4 (SEQ ID NOs: 81 and 508), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-35599.4 (SEQ ID NOs: 82 and 509), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-35605.4 (SEQ ID NOs: 83 and 510), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-35611.4 (SEQ ID NOs: 84 and 511), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-35617.4 (SEQ ID NOs: 85 and 512), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-35576.4 (SEQ ID NOs: 86 and 513), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-35588.4 (SEQ ID NOs: 87 and 514), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-35667.1 (SEQ ID NOs: 88 and 515), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-35673.1 (SEQ ID NOs: 89 and 516), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-35679.1 (SEQ ID NOs: 90 and 517), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-35685.1 (SEQ ID NOs: 91 and 518), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-35691.1 (SEQ ID NOs: 92 and 519), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-35557.1 (SEQ ID NOs: 93 and 520), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-35618.1 (SEQ ID NOs: 94 and 521), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-35569.1 (SEQ ID NOs: 95 and 522), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-35534.1 (SEQ ID NOs: 96 and 523), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-35582.1 (SEQ ID NOs: 97 and 524), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-35594.1 (SEQ ID NOs: 98 and 525), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-35600.1 (SEQ ID NOs: 99 and 526), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-35606.1 (SEQ ID NOs: 100 and 527), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-35612.1 (SEQ ID NOs: 101 and 528), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-38127.1 (SEQ ID NOs: 102 and 529), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-38133.1 (SEQ ID NOs: 103 and 530), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-38139.1 (SEQ ID NOs: 104 and 531), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-38145.1 (SEQ ID NOs: 105 and 532), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-38151.1 (SEQ ID NOs: 106 and 533), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-38157.1 (SEQ ID NOs: 107 and 534), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-38163.1 (SEQ ID NOs: 108 and 535), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-38169.1 (SEQ ID NOs: 109 and 536), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-38128.1 (SEQ ID NOs: 110 and 537), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-38134.1 (SEQ ID NOs: 111 and 538), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-38140.1 (SEQ ID NOs: 112 and 539), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-38146.1 (SEQ ID NOs: 113 and 540), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-38152.1 (SEQ ID NOs: 114 and 541), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-38158.1 (SEQ ID NOs: 115 and 542), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-38164.1 (SEQ ID NOs: 116 and 543), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-38170.1 (SEQ ID NOs: 117 and 544), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-38129.1 (SEQ ID NOs: 118 and 545), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-38135.1 (SEQ ID NOs: 119 and 546), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-38141.1 (SEQ ID NOs: 120 and 547), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-38147.1 (SEQ ID NOs: 121 and 548), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-38153.1 (SEQ ID NOs: 122 and 549), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-38159.1 (SEQ ID NOs: 123 and 550), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-38165.1 (SEQ ID NOs: 124 and 551), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-38171.1 (SEQ ID NOs: 125 and 552), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-38130.1 (SEQ ID NOs: 126 and 553), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-38136.1 (SEQ ID NOs: 127 and 554), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-38142.1 (SEQ ID NOs: 128 and 555), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-38148.1 (SEQ ID NOs: 129 and 556), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-38154.1 (SEQ ID NOs: 130 and 557), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-38160.1 (SEQ ID NOs: 131 and 558), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-38166.1 (SEQ ID NOs: 132 and 559), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-38172.1 (SEQ ID NOs: 133 and 560), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-38131.1 (SEQ ID NOs: 134 and 561), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-38137.1 (SEQ ID NOs: 135 and 562), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-38143.1 (SEQ ID NOs: 136 and 563), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-38149.1 (SEQ ID NOs: 137 and 564), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-38155.1 (SEQ ID NOs: 138 and 565), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-38161.1 (SEQ ID NOs: 139 and 566), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-38167.1 (SEQ ID NOs: 140 and 567), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-38173.1 (SEQ ID NOs: 141 and 568), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-38132.1 (SEQ ID NOs: 142 and 569), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-38138.1 (SEQ ID NOs: 143 and 570), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-38144.1 (SEQ ID NOs: 144 and 571), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-38150.1 (SEQ ID NOs: 145 and 572), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-38156.1 (SEQ ID NOs: 146 and 573), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-38162.1 (SEQ ID NOs: 147 and 574), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-38168.1 (SEQ ID NOs: 148 and 575), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-38174.1 (SEQ ID NOs: 149 and 576), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39683.1 (SEQ ID NOs: 150 and 577), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39689.1 (SEQ ID NOs: 151 and 578), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39695.1 (SEQ ID NOs: 152 and 579), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39701.1 (SEQ ID NOs: 153 and 580), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39707.1 (SEQ ID NOs: 154 and 581), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39713.1 (SEQ ID NOs: 155 and 582), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39719.1 (SEQ ID NOs: 156 and 583), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39725.1 (SEQ ID NOs: 157 and 584), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39684.1 (SEQ ID NOs: 158 and 585), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39690.1 (SEQ ID NOs: 159 and 586), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39696.1 (SEQ ID NOs: 160 and 587), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39702.1 (SEQ ID NOs: 161 and 588), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39708.1 (SEQ ID NOs: 162 and 589), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39714.1 (SEQ ID NOs: 163 and 590), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39720.1 (SEQ ID NOs: 164 and 591), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39726.1 (SEQ ID NOs: 165 and 592), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39685.1 (SEQ ID NOs: 166 and 593), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39691.1 (SEQ ID NOs: 167 and 594), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39697.1 (SEQ ID NOs: 168 and 595), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39703.1 (SEQ ID NOs: 169 and 596), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39709.1 (SEQ ID NOs: 170 and 597), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39715.1 (SEQ ID NOs: 171 and 598), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39721.1 (SEQ ID NOs: 172 and 599), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39727.1 (SEQ ID NOs: 173 and 600), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39686.1 (SEQ ID NOs: 174 and 601), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39692.1 (SEQ ID NOs: 175 and 602), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39698.1 (SEQ ID NOs: 176 and 603), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39704.1 (SEQ ID NOs: 177 and 604), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39710.1 (SEQ ID NOs: 178 and 605), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39716.1 (SEQ ID NOs: 179 and 606), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39722.1 (SEQ ID NOs: 180 and 607), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39728.1 (SEQ ID NOs: 181 and 608), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39687.1 (SEQ ID NOs: 182 and 609), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39693.1 (SEQ ID NOs: 183 and 610), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39699.1 (SEQ ID NOs: 184 and 611), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39705.1 (SEQ ID NOs: 185 and 612), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39711.1 (SEQ ID NOs: 186 and 613), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39717.1 (SEQ ID NOs: 187 and 614), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39723.1 (SEQ ID NOs: 188 and 615), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39729.1 (SEQ ID NOs: 189 and 616), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39688.1 (SEQ ID NOs: 190 and 617), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39694.1 (SEQ ID NOs: 191 and 618), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39700.1 (SEQ ID NOs: 192 and 619), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39706.1 (SEQ ID NOs: 193 and 620), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39712.1 (SEQ ID NOs: 194 and 621), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39718.1 (SEQ ID NOs: 195 and 622), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39724.1 (SEQ ID NOs: 196 and 623), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39730.1 (SEQ ID NOs: 197 and 624), or modified or unmodified variants thereof, wherein the first and/or second strand further comprise up to about 20 additional nucleotides.
A RNAi agent comprising a first and a second strand, wherein the sequence of the first and/or second strand is the sequence of a first and/or second strand of: AD-39736.1 (SEQ ID NOs: 198 and 625), or modified or unmodified variants thereof, wherein the first and/or second strand further comprise up to about 20 additional nucleotides.
A RNAi agent comprising a first and a second strand, wherein the sequence of the first and/or second strand is the sequence of a first and/or second strand of: AD-39742.1 (SEQ ID NOs: 199 and 626), or modified or unmodified variants thereof, wherein the first and/or second strand further comprise up to about 20 additional nucleotides.
A RNAi agent comprising a first and a second strand, wherein the sequence of the first and/or second strand is the sequence of a first and/or second strand of: AD-39748.1 (SEQ ID NOs: 200 and 627), or modified or unmodified variants thereof, wherein the first and/or second strand further comprise up to about 20 additional nucleotides.
A RNAi agent comprising a first and a second strand, wherein the sequence of the first and/or second strand is the sequence of a first and/or second strand of: AD-39754.1 (SEQ ID NOs: 201 and 628), or modified or unmodified variants thereof, wherein the first and/or second strand further comprise up to about 20 additional nucleotides.
A RNAi agent comprising a first and a second strand, wherein the sequence of the first and/or second strand is the sequence of a first and/or second strand of: AD-39760.1 (SEQ ID NOs: 202 and 629), or modified or unmodified variants thereof, wherein the first and/or second strand further comprise up to about 20 additional nucleotides.
A RNAi agent comprising a first and a second strand, wherein the sequence of the first and/or second strand is the sequence of a first and/or second strand of: AD-39766.1 (SEQ ID NOs: 203 and 630), or modified or unmodified variants thereof, wherein the first and/or second strand further comprise up to about 20 additional nucleotides.
A RNAi agent comprising a first and a second strand, wherein the sequence of the first and/or second strand is the sequence of a first and/or second strand of: AD-39772.1 (SEQ ID NOs: 204 and 631), or modified or unmodified variants thereof, wherein the first and/or second strand further comprise up to about 20 additional nucleotides.
A RNAi agent comprising a first and a second strand, wherein the sequence of the first and/or second strand is the sequence of a first and/or second strand of: AD-39731.1 (SEQ ID NOs: 205 and 632), or modified or unmodified variants thereof, wherein the first and/or second strand further comprise up to about 20 additional nucleotides.
A RNAi agent comprising a first and a second strand, wherein the sequence of the first and/or second strand is the sequence of a first and/or second strand of: AD-39737.1 (SEQ ID NOs: 206 and 633), or modified or unmodified variants thereof, wherein the first and/or second strand further comprise up to about 20 additional nucleotides.
A RNAi agent comprising a first and a second strand, wherein the sequence of the first and/or second strand is the sequence of a first and/or second strand of: AD-39743.1 (SEQ ID NOs: 207 and 634), or modified or unmodified variants thereof, wherein the first and/or second strand further comprise up to about 20 additional nucleotides.
A RNAi agent comprising a first and a second strand, wherein the sequence of the first and/or second strand is the sequence of a first and/or second strand of: AD-39749.1 (SEQ ID NOs: 208 and 635), or modified or unmodified variants thereof, wherein the first and/or second strand further comprise up to about 20 additional nucleotides.
A RNAi agent comprising a first and a second strand, wherein the sequence of the first and/or second strand is the sequence of a first and/or second strand of: AD-39755.1 (SEQ ID NOs: 209 and 636), or modified or unmodified variants thereof, wherein the first and/or second strand further comprise up to about 20 additional nucleotides.
A RNAi agent comprising a first and a second strand, wherein the sequence of the first and/or second strand is the sequence of a first and/or second strand of: AD-39761.1 (SEQ ID NOs: 210 and 637), or modified or unmodified variants thereof, wherein the first and/or second strand further comprise up to about 20 additional nucleotides.
A RNAi agent comprising a first and a second strand, wherein the sequence of the first and/or second strand is the sequence of a first and/or second strand of: AD-39767.1 (SEQ ID NOs: 211 and 638), or modified or unmodified variants thereof, wherein the first and/or second strand further comprise up to about 20 additional nucleotides.
A RNAi agent comprising a first and a second strand, wherein the sequence of the first and/or second strand is the sequence of a first and/or second strand of: AD-39773.1 (SEQ ID NOs: 212 and 639), or modified or unmodified variants thereof, wherein the first and/or second strand further comprise up to about 20 additional nucleotides.
A RNAi agent comprising a first and a second strand, wherein the sequence of the first and/or second strand is the sequence of a first and/or second strand of: AD-39732.1 (SEQ ID NOs: 213 and 640), or modified or unmodified variants thereof, wherein the first and/or second strand further comprise up to about 20 additional nucleotides.
A RNAi agent comprising a first and a second strand, wherein the sequence of the first and/or second strand is the sequence of a first and/or second strand of: AD-39738.1 (SEQ ID NOs: 214 and 641), or modified or unmodified variants thereof, wherein the first and/or second strand further comprise up to about 20 additional nucleotides.
A RNAi agent comprising a first and a second strand, wherein the sequence of the first and/or second strand is the sequence of a first and/or second strand of: AD-39744.1 (SEQ ID NOs: 215 and 642), or modified or unmodified variants thereof, wherein the first and/or second strand further comprise up to about 20 additional nucleotides.
A RNAi agent comprising a first and a second strand, wherein the sequence of the first and/or second strand is the sequence of a first and/or second strand of: AD-39750.1 (SEQ ID NOs: 216 and 643), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39756.1 (SEQ ID NOs: 217 and 644), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39762.1 (SEQ ID NOs: 218 and 645), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39768.1 (SEQ ID NOs: 219 and 646), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39774.1 (SEQ ID NOs: 220 and 647), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39733.1 (SEQ ID NOs: 221 and 648), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39739.1 (SEQ ID NOs: 222 and 649), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39745.1 (SEQ ID NOs: 223 and 650), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39751.1 (SEQ ID NOs: 224 and 651), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39757.1 (SEQ ID NOs: 225 and 652), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39763.1 (SEQ ID NOs: 226 and 653), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39769.1 (SEQ ID NOs: 227 and 654), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39775.1 (SEQ ID NOs: 228 and 655), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39734.1 (SEQ ID NOs: 229 and 656), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39740.1 (SEQ ID NOs: 230 and 657), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39746.1 (SEQ ID NOs: 231 and 658), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39752.1 (SEQ ID NOs: 232 and 659), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39758.1 (SEQ ID NOs: 233 and 660), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39764.1 (SEQ ID NOs: 234 and 661), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39770.1 (SEQ ID NOs: 235 and 662), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39776.1 (SEQ ID NOs: 236 and 663), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39735.1 (SEQ ID NOs: 237 and 664), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39741.1 (SEQ ID NOs: 238 and 665), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39747.1 (SEQ ID NOs: 239 and 666), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39753.1 (SEQ ID NOs: 240 and 667), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39759.1 (SEQ ID NOs: 241 and 668), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39765.1 (SEQ ID NOs: 242 and 669), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39771.1 (SEQ ID NOs: 243 and 670), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39778.1 (SEQ ID NOs: 244 and 671), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39784.1 (SEQ ID NOs: 245 and 672), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39790.1 (SEQ ID NOs: 246 and 673), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39796.1 (SEQ ID NOs: 247 and 674), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39802.1 (SEQ ID NOs: 248 and 675), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39808.1 (SEQ ID NOs: 249 and 676), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39814.1 (SEQ ID NOs: 250 and 677), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39820.1 (SEQ ID NOs: 251 and 678), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39779.1 (SEQ ID NOs: 252 and 679), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39785.1 (SEQ ID NOs: 253 and 680), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39791.1 (SEQ ID NOs: 254 and 681), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39797.1 (SEQ ID NOs: 255 and 682), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39803.1 (SEQ ID NOs: 256 and 683), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39809.1 (SEQ ID NOs: 257 and 684), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39815.1 (SEQ ID NOs: 258 and 685), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39821.1 (SEQ ID NOs: 259 and 686), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39780.1 (SEQ ID NOs: 260 and 687), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39786.1 (SEQ ID NOs: 261 and 688), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39792.1 (SEQ ID NOs: 262 and 689), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39798.1 (SEQ ID NOs: 263 and 690), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39804.1 (SEQ ID NOs: 264 and 691), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39810.1 (SEQ ID NOs: 265 and 692), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39816.1 (SEQ ID NOs: 266 and 693), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39822.1 (SEQ ID NOs: 267 and 694), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39781.1 (SEQ ID NOs: 268 and 695), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39787.1 (SEQ ID NOs: 269 and 696), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39793.1 (SEQ ID NOs: 270 and 697), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39799.1 (SEQ ID NOs: 271 and 698), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39805.1 (SEQ ID NOs: 272 and 699), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39811.1 (SEQ ID NOs: 273 and 700), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39817.1 (SEQ ID NOs: 274 and 701), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39823.1 (SEQ ID NOs: 275 and 702), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39782.1 (SEQ ID NOs: 276 and 703), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39788.1 (SEQ ID NOs: 277 and 704), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39794.1 (SEQ ID NOs: 278 and 705), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39800.1 (SEQ ID NOs: 279 and 706), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39806.1 (SEQ ID NOs: 280 and 707), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39812.1 (SEQ ID NOs: 281 and 708), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39818.1 (SEQ ID NOs: 282 and 709), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39824.1 (SEQ ID NOs: 283 and 710), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39783.1 (SEQ ID NOs: 284 and 711), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39789.1 (SEQ ID NOs: 285 and 712), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39795.1 (SEQ ID NOs: 286 and 713), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39801.1 (SEQ ID NOs: 287 and 714), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39807.1 (SEQ ID NOs: 288 and 715), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39813.1 (SEQ ID NOs: 289 and 716), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39819.1 (SEQ ID NOs: 290 and 717), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39825.1 (SEQ ID NOs: 291 and 718), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39831.1 (SEQ ID NOs: 292 and 719), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39837.1 (SEQ ID NOs: 293 and 720), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39843.1 (SEQ ID NOs: 294 and 721), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39849.1 (SEQ ID NOs: 295 and 722), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39855.1 (SEQ ID NOs: 296 and 723), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39861.1 (SEQ ID NOs: 297 and 724), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39867.1 (SEQ ID NOs: 298 and 725), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39826.1 (SEQ ID NOs: 299 and 726), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39832.1 (SEQ ID NOs: 300 and 727), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39838.1 (SEQ ID NOs: 301 and 728), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39844.1 (SEQ ID NOs: 302 and 729), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39850.1 (SEQ ID NOs: 303 and 730), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39856.1 (SEQ ID NOs: 304 and 731), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39862.1 (SEQ ID NOs: 305 and 732), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39868.1 (SEQ ID NOs: 306 and 733), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39827.1 (SEQ ID NOs: 307 and 734), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39833.1 (SEQ ID NOs: 308 and 735), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39839.1 (SEQ ID NOs: 309 and 736), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39845.1 (SEQ ID NOs: 310 and 737), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39851.1 (SEQ ID NOs: 311 and 738), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39857.1 (SEQ ID NOs: 312 and 739), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39863.1 (SEQ ID NOs: 313 and 740), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39869.1 (SEQ ID NOs: 314 and 741), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39828.1 (SEQ ID NOs: 315 and 742), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39834.1 (SEQ ID NOs: 316 and 743), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39840.1 (SEQ ID NOs: 317 and 744), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39846.1 (SEQ ID NOs: 318 and 745), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39852.1 (SEQ ID NOs: 319 and 746), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39858.1 (SEQ ID NOs: 320 and 747), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39864.1 (SEQ ID NOs: 321 and 748), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39870.1 (SEQ ID NOs: 322 and 749), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39829.1 (SEQ ID NOs: 323 and 750), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39835.1 (SEQ ID NOs: 324 and 751), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39841.1 (SEQ ID NOs: 325 and 752), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39853.1 (SEQ ID NOs: 326 and 753), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39859.1 (SEQ ID NOs: 327 and 754), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39865.1 (SEQ ID NOs: 328 and 755), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39871.1 (SEQ ID NOs: 329 and 756), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39830.1 (SEQ ID NOs: 330 and 757), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39836.1 (SEQ ID NOs: 331 and 758), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39842.1 (SEQ ID NOs: 332 and 759), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39848.1 (SEQ ID NOs: 333 and 760), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39854.1 (SEQ ID NOs: 334 and 761), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39860.1 (SEQ ID NOs: 335 and 762), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39866.1 (SEQ ID NOs: 336 and 763), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39992.1 (SEQ ID NOs: 337 and 764), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39998.1 (SEQ ID NOs: 338 and 765), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-40004.1 (SEQ ID NOs: 339 and 766), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-40010.1 (SEQ ID NOs: 340 and 767), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-40016.1 (SEQ ID NOs: 341 and 768), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-40022.1 (SEQ ID NOs: 342 and 769), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-40028.1 (SEQ ID NOs: 343 and 770), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-40034.1 (SEQ ID NOs: 344 and 771), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39999.1 (SEQ ID NOs: 345 and 772), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-40005.1 (SEQ ID NOs: 346 and 773), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-40011.1 (SEQ ID NOs: 347 and 774), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-40017.1 (SEQ ID NOs: 348 and 775), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-40029.1 (SEQ ID NOs: 349 and 776), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-40035.1 (SEQ ID NOs: 350 and 777), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39994.1 (SEQ ID NOs: 351 and 778), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-40000.1 (SEQ ID NOs: 352 and 779), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-40006.1 (SEQ ID NOs: 353 and 780), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-40012.1 (SEQ ID NOs: 354 and 781), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-40018.1 (SEQ ID NOs: 355 and 782), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-40024.1 (SEQ ID NOs: 356 and 783), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-40030.1 (SEQ ID NOs: 357 and 784), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-40036.1 (SEQ ID NOs: 358 and 785), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39995.1 (SEQ ID NOs: 359 and 786), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-40001.1 (SEQ ID NOs: 360 and 787), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-40007.1 (SEQ ID NOs: 361 and 788), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-40013.1 (SEQ ID NOs: 362 and 789), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-40019.1 (SEQ ID NOs: 363 and 790), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-40025.1 (SEQ ID NOs: 364 and 791), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-40031.1 (SEQ ID NOs: 365 and 792), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-40037.1 (SEQ ID NOs: 366 and 793), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39996.1 (SEQ ID NOs: 367 and 794), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-40002.1 (SEQ ID NOs: 368 and 795), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-40008.1 (SEQ ID NOs: 369 and 796), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-40014.1 (SEQ ID NOs: 370 and 797), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-40020.1 (SEQ ID NOs: 371 and 798), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-40026.1 (SEQ ID NOs: 372 and 799), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-40032.1 (SEQ ID NOs: 373 and 800), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-40038.1 (SEQ ID NOs: 374 and 801), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39997.1 (SEQ ID NOs: 375 and 802), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-40009.1 (SEQ ID NOs: 376 and 803), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-40015.1 (SEQ ID NOs: 377 and 804), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-40021.1 (SEQ ID NOs: 378 and 805), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-40027.1 (SEQ ID NOs: 379 and 806), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-40033.1 (SEQ ID NOs: 380 and 807), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-40039.1 (SEQ ID NOs: 381 and 808), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-40045.1 (SEQ ID NOs: 382 and 809), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-40051.1 (SEQ ID NOs: 383 and 810), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-40057.1 (SEQ ID NOs: 384 and 811), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-40063.1 (SEQ ID NOs: 385 and 812), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-40069.1 (SEQ ID NOs: 386 and 813), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-40075.1 (SEQ ID NOs: 387 and 814), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-40081.1 (SEQ ID NOs: 388 and 815), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-40040.1 (SEQ ID NOs: 389 and 816), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-40046.1 (SEQ ID NOs: 390 and 817), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-40052.1 (SEQ ID NOs: 391 and 818), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-40058.1 (SEQ ID NOs: 392 and 819), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-40064.1 (SEQ ID NOs: 393 and 820), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-40070.1 (SEQ ID NOs: 394 and 821), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-40076.1 (SEQ ID NOs: 395 and 822), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-40082.1 (SEQ ID NOs: 396 and 823), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-40041.1 (SEQ ID NOs: 397 and 824), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-40047.1 (SEQ ID NOs: 398 and 825), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-40053.1 (SEQ ID NOs: 399 and 826), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-40059.1 (SEQ ID NOs: 400 and 827), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-40065.1 (SEQ ID NOs: 401 and 828), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-40071.1 (SEQ ID NOs: 402 and 829), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-40077.1 (SEQ ID NOs: 403 and 830), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-40083.1 (SEQ ID NOs: 404 and 831), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-40042.1 (SEQ ID NOs: 405 and 832), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-40048.1 (SEQ ID NOs: 406 and 833), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-40054.1 (SEQ ID NOs: 407 and 834), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-40060.1 (SEQ ID NOs: 408 and 835), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-40066.1 (SEQ ID NOs: 409 and 836), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-40072.1 (SEQ ID NOs: 410 and 837), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-40078.1 (SEQ ID NOs: 411 and 838), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-40084.1 (SEQ ID NOs: 412 and 839), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-40043.1 (SEQ ID NOs: 413 and 840), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-40049.1 (SEQ ID NOs: 414 and 841), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-40055.1 (SEQ ID NOs: 415 and 842), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-40061.1 (SEQ ID NOs: 416 and 843), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-40067.1 (SEQ ID NOs: 417 and 844), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-40073.1 (SEQ ID NOs: 418 and 845), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-40079.1 (SEQ ID NOs: 419 and 846), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-40085.1 (SEQ ID NOs: 420 and 847), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-40044.1 (SEQ ID NOs: 421 and 848), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-40050.1 (SEQ ID NOs: 422 and 849), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-40056.1 (SEQ ID NOs: 423 and 850), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-40062.1 (SEQ ID NOs: 424 and 851), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-40068.1 (SEQ ID NOs: 425 and 852), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-40074.1 (SEQ ID NOs: 426 and 853), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-40080.1 (SEQ ID NOs: 427 and 854), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39720.1 (SEQ ID NOs: 1716 and 1754), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39706.1 (SEQ ID NOs: 1717 and 1755), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39712.1 (SEQ ID NOs: 1718 and 1756), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39760.1 (SEQ ID NOs: 1719 and 1757), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39732.1 (SEQ ID NOs: 1720 and 1758), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-35541.4 (SEQ ID NOs: 1721 and 1759), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39735.1 or hs_KRAS_ 321_A22S26 (SEQ ID NOs: 1722 and 1760), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39741.1, or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: hs_KRAS_ 322_A22S26 (SEQ ID NOs: 1723 and 1761), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39778.1 (SEQ ID NOs: 1724 and 1762), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39790.1 (SEQ ID NOs: 1725 and 1763), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39822.1 (SEQ ID NOs: 1726 and 1764), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-35609.4 (SEQ ID NOs: 1727 and 1765), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-35581.4 (SEQ ID NOs: 1728 and 1766), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39845.1 (SEQ ID NOs: 1729 and 1767), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39858.1 (SEQ ID NOs: 1730 and 1768), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39870.1 (SEQ ID NOs: 1731 and 1769), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-35576.4 (SEQ ID NOs: 1732 and 1770), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-35588.4 (SEQ ID NOs: 1733 and 1771), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-35600.1 (SEQ ID NOs: 1734 and 1772), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-35606.1 (SEQ ID NOs: 1735 and 1773), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-38151.1 (SEQ ID NOs: 1736 and 1774), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-38163.1 (SEQ ID NOs: 1737 and 1775), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-39999.1 (SEQ ID NOs: 1738 and 1776), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-38159.1 or hs_KRAS_ 528_A22S26 (SEQ ID NOs: 1739 and 1777), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-38130.1 or hs_KRAS_ 531_A22S26 (SEQ ID NOs: 1740 and 1778), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-38136.1 (SEQ ID NOs: 1741 and 1779), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-40036.1 (SEQ ID NOs: 1742 and 1780), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-40008.1 (SEQ ID NOs: 1743 and 1781), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-40021.1 (SEQ ID NOs: 1744 and 1782), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-40077.1 (SEQ ID NOs: 1745 and 1783), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-40072.1 (SEQ ID NOs: 1746 and 1784), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-40061.1 (SEQ ID NOs: 1747 and 1785), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-40068.1 (SEQ ID NOs: 1748 and 1786), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-38131.1 (SEQ ID NOs: 1749 and 1787), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: AD-38167.1 (SEQ ID NOs: 1750 and 1788), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: hs_KRAS 1273_A22S26 (SEQ ID NOs: 1751 and 1789), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: hs_KRAS 2892_A37S26 (SEQ ID NOs: 1752 and 1790), or modified or unmodified variants thereof, wherein the first and/or second strand further comprise up to about 20 additional nucleotides.

A RNAi agent comprising a first and a second strand, wherein the sequence of the first and/or second strand is the sequence of a first and/or second strand of: hs_KRAS 4731_A22S26 (SEQ ID NOs: 1753 and 1791), or modified or unmodified variants thereof, wherein the first and/or second strand further comprise up to about 20 additional nucleotides.

In various embodiments, the disclosure comprises a RNAi agent comprising a sense and an antisense strand, wherein the antisense strand comprises at least 15 contiguous nucleotides differing by 0, 1, 2, or 3 nt from the antisense strand of any RNAi agent disclosed herein, or modified or unmodified variants thereof, wherein the antisense strand optionally further comprises 0, 1, 2, 3, 4, 5, 6, 7, 8, 9, or 10 or more nt (or any range thereof, e.g., 0-1, 1-2, 1-3, 1-4 nt, etc.).

In one embodiment, the disclosure comprises any one or more RNAi agent listed herein.

**Specific embodiments wherein the sequences of the first and/or second strands are the sequences of the first and/or second strand of a RNAi agent disclosed herein, further comprising up to about 20 additional nucleotides**

In one embodiment, the present disclosure pertains to a composition comprising any one or more of: a RNAi agent comprising a first and a second strand, wherein the sequence of the first and/or second strand comprise a sequence of 15 contiguous nt from the sequence of a first and/or second strand of any RNAi agent from any of Tables 1 to 6, or modified and unmodified variants thereof.

Thus, in various embodiments, the present disclosure pertains to a composition comprising any one or more of the following:

A RNAi agent comprising a first and a second strand, wherein the sequence of the first and/or second strand comprise a sequence of 15 contiguous nt from the sequence of a first and/or second strand of: AD-35523.3 (SEQ ID NOs: 1 and 428), or a modified or unmodified variant thereof.

A RNAi agent comprising a first and a second strand, wherein the sequence of the first and/or second strand comprise a sequence of 15 contiguous nt from the sequence of a first and/or second strand of: AD-35529.1 (SEQ ID NOs: 2 and 429), or a modified or unmodified variant thereof.

A RNAi agent comprising a first and a second strand, wherein the sequence of the first and/or second strand comprise a sequence of 15 contiguous nt from the sequence of a first and/or second strand of: AD-35535.4 (SEQ ID NOs: 3 and 430), or a modified or unmodified variant thereof.

A RNAi agent comprising a first and a second strand, wherein the sequence of the first and/or second strand comprise a sequence of 15 contiguous nt from the sequence of a first and/or second strand of: AD-35541.4 (SEQ ID NOs: 4 and 431), or a modified or unmodified variant thereof.

A RNAi agent comprising a first and a second strand, wherein the sequence of the first and/or second strand comprise a sequence of 15 contiguous nt from the sequence of a first and/or second strand of: AD-35547.4 (SEQ ID NOs: 5 and 432), or a modified or unmodified variant thereof.

A RNAi agent comprising a first and a second strand, wherein the sequence of the first and/or second strand comprise a sequence of 15 contiguous nt from the sequence of a first and/or second strand of: AD-35553.4 (SEQ ID NOs: 6 and 433), or a modified or unmodified variant thereof.

A RNAi agent comprising a first and a second strand, wherein the sequence of the first and/or second strand comprise a sequence of 15 contiguous nt from the sequence of a first and/or second strand of: AD-35559.4 (SEQ ID NOs: 7 and 434), or a modified or unmodified variant thereof.

A RNAi agent comprising a first and a second strand, wherein the sequence of the first and/or second strand comprise a sequence of 15 contiguous nt from the sequence of a first and/or second strand of: AD-35565.4 (SEQ ID NOs: 8 and 435), or a modified or unmodified variant thereof.

A RNAi agent comprising a first and a second strand, wherein the sequence of the first and/or second strand comprise a sequence of 15 contiguous nt from the sequence of a first and/or second strand of: AD-35524.4 (SEQ ID NOs: 9 and 436), or a modified or unmodified variant thereof.

A RNAi agent comprising a first and a second strand, wherein the sequence of the first and/or second strand comprise a sequence of 15 contiguous nt from the sequence of a first and/or second strand of: AD-35530.4 (SEQ ID NOs: 10 and 437), or a modified or unmodified variant thereof.

A RNAi agent comprising a first and a second strand, wherein the sequence of the first and/or second strand comprise a sequence of 15 contiguous nt from the sequence of a first and/or second strand of: AD-35536.4 (SEQ ID NOs: 11 and 438), or a modified or unmodified variant thereof.

A RNAi agent comprising a first and a second strand, wherein the sequence of the first and/or second strand comprise a sequence of 15 contiguous nt from the sequence of a first and/or second strand of: AD-35542.4 (SEQ ID NOs: 12 and 439), or a modified or unmodified variant thereof.

A RNAi agent comprising a first and a second strand, wherein the sequence of the first and/or second strand comprise a sequence of 15 contiguous nt from the sequence of a first and/or second strand of: AD-35548.4 (SEQ ID NOs: 13 and 440), or a modified or unmodified variant thereof.

A RNAi agent comprising a first and a second strand, wherein the sequence of the first and/or second strand comprise a sequence of 15 contiguous nt from the sequence of a first and/or second strand of: AD-35554.4 (SEQ ID NOs: 14 and 441), or a modified or unmodified variant thereof.

A RNAi agent comprising a first and a second strand, wherein the sequence of the first and/or second strand comprise a sequence of 15 contiguous nt from the sequence of a first and/or second strand of: AD-35560.2 (SEQ ID NOs: 15 and 442), or a modified or unmodified variant thereof.

A RNAi agent comprising a first and a second strand, wherein the sequence of the first and/or second strand comprise a sequence of 15 contiguous nt from the sequence of a first and/or second strand of: AD-35566.4 (SEQ ID NOs: 16 and 443), or a modified or unmodified variant thereof.

A RNAi agent comprising a first and a second strand, wherein the sequence of the first and/or second strand comprise a sequence of 15 contiguous nt from the sequence of a first and/or second strand of: AD-35525.2 (SEQ ID NOs: 17 and 444), or a modified or unmodified variant thereof.

A RNAi agent comprising a first and a second strand, wherein the sequence of the first and/or second strand comprise a sequence of 15 contiguous nt from the sequence of a first and/or second strand of: AD-35531.4 (SEQ ID NOs: 18 and 445), or a modified or unmodified variant thereof.

A RNAi agent comprising a first and a second strand, wherein the sequence of the first and/or second strand comprise a sequence of 15 contiguous nt from the sequence of a first and/or second strand of: AD-35537.4 (SEQ ID NOs: 19 and 446), or a modified or unmodified variant thereof.

A RNAi agent comprising a first and a second strand, wherein the sequence of the first and/or second strand comprise a sequence of 15 contiguous nt from the sequence of a first and/or second strand of: AD-35543.2 (SEQ ID NOs: 20 and 447), or a modified or unmodified variant thereof.

A RNAi agent comprising a first and a second strand, wherein the sequence of the first and/or second strand comprise a sequence of 15 contiguous nt from the sequence of a first and/or second strand of: AD-35549.4 (SEQ ID NOs: 21 and 448), or a modified or unmodified variant thereof.

A RNAi agent comprising a first and a second strand, wherein the sequence of the first and/or second strand comprise a sequence of 15 contiguous nt from the sequence of a first and/or second strand of: AD-35555.4 (SEQ ID NOs: 22 and 449), or a modified or unmodified variant thereof.

A RNAi agent comprising a first and a second strand, wherein the sequence of the first and/or second strand comprise a sequence of 15 contiguous nt from the sequence of a first and/or second strand of: AD-35561.4 (SEQ ID NOs: 23 and 450), or a modified or unmodified variant thereof.

A RNAi agent comprising a first and a second strand, wherein the sequence of the first and/or second strand comprise a sequence of 15 contiguous nt from the sequence of a first and/or second strand of: AD-35567.4 (SEQ ID NOs: 24 and 451), or a modified or unmodified variant thereof.

A RNAi agent comprising a first and a second strand, wherein the sequence of the first and/or second strand comprise a sequence of 15 contiguous nt from the sequence of a first and/or second strand of: AD-35526.4 (SEQ ID NOs: 25 and 452), or a modified or unmodified variant thereof.

A RNAi agent comprising a first and a second strand, wherein the sequence of the first and/or second strand comprise a sequence of 15 contiguous nt from the sequence of a first and/or second strand of: AD-35532.4 (SEQ ID NOs: 26 and 453), or a modified or unmodified variant thereof.

A RNAi agent comprising a first and a second strand, wherein the sequence of the first and/or second strand comprise a sequence of 15 contiguous nt from the sequence of a first and/or second strand of: AD-35538.4 (SEQ ID NOs: 27 and 454), or a modified or unmodified variant thereof.

A RNAi agent comprising a first and a second strand, wherein the sequence of the first and/or second strand comprise a sequence of 15 contiguous nt from the sequence of a first and/or second strand of: AD-35544.4 (SEQ ID NOs: 28 and 455), or a modified or unmodified variant thereof.

A RNAi agent comprising a first and a second strand, wherein the sequence of the first and/or second strand comprise a sequence of 15 contiguous nt from the sequence of a first and/or second strand of: AD-35550.4 (SEQ ID NOs: 29 and 456), or a modified or unmodified variant thereof.

A RNAi agent comprising a first and a second strand, wherein the sequence of the first and/or second strand comprise a sequence of 15 contiguous nt from the sequence of a first and/or second strand of: AD-35556.4 (SEQ ID NOs: 30 and 457), or a modified or unmodified variant thereof.

A RNAi agent comprising a first and a second strand, wherein the sequence of the first and/or second strand comprise a sequence of 15 contiguous nt from the sequence of a first and/or second strand of: AD-35562.4 (SEQ ID NOs: 31 and 458), or a modified or unmodified variant thereof.

A RNAi agent comprising a first and a second strand, wherein the sequence of the first and/or second strand comprise a sequence of 15 contiguous nt from the sequence of a first and/or second strand of: AD-35568.4 (SEQ ID NOs: 32 and 459), or a modified or unmodified variant thereof.

A RNAi agent comprising a first and a second strand, wherein the sequence of the first and/or second strand comprise a sequence of 15 contiguous nt from the sequence of a first and/or second strand of: AD-35527.4 (SEQ ID NOs: 33 and 460), or a modified or unmodified variant thereof.

A RNAi agent comprising a first and a second strand, wherein the sequence of the first and/or second strand comprise a sequence of 15 contiguous nt from the sequence of a first and/or second strand of: AD-35533.4 (SEQ ID NOs: 34 and 461), or a modified or unmodified variant thereof.

A RNAi agent comprising a first and a second strand, wherein the sequence of the first and/or second strand comprise a sequence of 15 contiguous nt from the sequence of a first and/or second strand of: AD-35539.4 (SEQ ID NOs: 35 and 462), or a modified or unmodified variant thereof.

A RNAi agent comprising a first and a second strand, wherein the sequence of the first and/or second strand comprise a sequence of 15 contiguous nt from the sequence of a first and/or second strand of: AD-35545.4 (SEQ ID NOs: 36 and 463), or a modified or unmodified variant thereof.

A RNAi agent comprising a first and a second strand, wherein the sequence of the first and/or second strand comprise a sequence of 15 contiguous nt from the sequence of a first and/or second strand of: AD-35551.4 (SEQ ID NOs: 37 and 464), or a modified or unmodified variant thereof.

A RNAi agent comprising a first and a second strand, wherein the sequence of the first and/or second strand comprise a sequence of 15 contiguous nt from the sequence of a first and/or second strand of: AD-35563.4 (SEQ ID NOs: 38 and 465), or a modified or unmodified variant thereof.

A RNAi agent comprising a first and a second strand, wherein the sequence of the first and/or second strand comprise a sequence of 15 contiguous nt from the sequence of a first and/or second strand of: AD-35528.4 (SEQ ID NOs: 39 and 466), or a modified or unmodified variant thereof.

A RNAi agent comprising a first and a second strand, wherein the sequence of the first and/or second strand comprise a sequence of 15 contiguous nt from the sequence of a first and/or second strand of: AD-35540.4 (SEQ ID NOs: 40 and 467), or a modified or unmodified variant thereof.

A RNAi agent comprising a first and a second strand, wherein the sequence of the first and/or second strand comprise a sequence of 15 contiguous nt from the sequence of a first and/or second strand of: AD-35546.4 (SEQ ID NOs: 41 and 468), or a modified or unmodified variant thereof.

A RNAi agent comprising a first and a second strand, wherein the sequence of the first and/or second strand comprise a sequence of 15 contiguous nt from the sequence of a first and/or second strand of: AD-35552.4 (SEQ ID NOs: 42 and 469), or a modified or unmodified variant thereof.

A RNAi agent comprising a first and a second strand, wherein the sequence of the first and/or second strand comprise a sequence of 15 contiguous nt from the sequence of a first and/or second strand of: AD-35558.4 (SEQ ID NOs: 43 and 470), or a modified or unmodified variant thereof.

A RNAi agent comprising a first and a second strand, wherein the sequence of the first and/or second strand comprise a sequence of 15 contiguous nt from the sequence of a first and/or second strand of: AD-35564.4 (SEQ ID NOs: 44 and 471), or a modified or unmodified variant thereof.

A RNAi agent comprising a first and a second strand, wherein the sequence of the first and/or second strand comprise a sequence of 15 contiguous nt from the sequence of a first and/or second strand of: AD-35570.4 (SEQ ID NOs: 45 and 472), or a modified or unmodified variant thereof.

A RNAi agent comprising a first and a second strand, wherein the sequence of the first and/or second strand comprise a sequence of 15 contiguous nt from the sequence of a first and/or second strand of: AD-35571.4 (SEQ ID NOs: 46 and 473), or a modified or unmodified variant thereof.

A RNAi agent comprising a first and a second strand, wherein the sequence of the first and/or second strand comprise a sequence of 15 contiguous nt from the sequence of a first and/or second strand of: AD-35577.4 (SEQ ID NOs: 47 and 474), or a modified or unmodified variant thereof.

A RNAi agent comprising a first and a second strand, wherein the sequence of the first and/or second strand comprise a sequence of 15 contiguous nt from the sequence of a first and/or second strand of: AD-35583.4 (SEQ ID NOs: 48 and 475), or a modified or unmodified variant thereof.

A RNAi agent comprising a first and a second strand, wherein the sequence of the first and/or second strand comprise a sequence of 15 contiguous nt from the sequence of a first and/or second strand of: AD-35589.4 (SEQ ID NOs: 49 and 476), or a modified or unmodified variant thereof.

A RNAi agent comprising a first and a second strand, wherein the sequence of the first and/or second strand comprise a sequence of 15 contiguous nt from the sequence of a first and/or second strand of: AD-35595.4 (SEQ ID NOs: 50 and 477), or a modified or unmodified variant thereof.

A RNAi agent comprising a first and a second strand, wherein the sequence of the first and/or second strand comprise a sequence of 15 contiguous nt from the sequence of a first and/or second strand of: AD-35601.4 (SEQ ID NOs: 51 and 478), or a modified or unmodified variant thereof.

A RNAi agent comprising a first and a second strand, wherein the sequence of the first and/or second strand comprise a sequence of 15 contiguous nt from the sequence of a first and/or second strand of: AD-35607.4 (SEQ ID NOs: 52 and 479), or a modified or unmodified variant thereof.

A RNAi agent comprising a first and a second strand, wherein the sequence of the first and/or second strand comprise a sequence of 15 contiguous nt from the sequence of a first and/or second strand of: AD-35613.4 (SEQ ID NOs: 53 and 480), or a modified or unmodified variant thereof.

A RNAi agent comprising a first and a second strand, wherein the sequence of the first and/or second strand comprise a sequence of 15 contiguous nt from the sequence of a first and/or second strand of: AD-35572.4 (SEQ ID NOs: 54 and 481), or a modified or unmodified variant thereof.

A RNAi agent comprising a first and a second strand, wherein the sequence of the first and/or second strand comprise a sequence of 15 contiguous nt from the sequence of a first and/or second strand of: AD-35578.4 (SEQ ID NOs: 55 and 482), or a modified or unmodified variant thereof.

A RNAi agent comprising a first and a second strand, wherein the sequence of the first and/or second strand comprise a sequence of 15 contiguous nt from the sequence of a first and/or second strand of: AD-35584.4 (SEQ ID NOs: 56 and 483), or a modified or unmodified variant thereof.

A RNAi agent comprising a first and a second strand, wherein the sequence of the first and/or second strand comprise a sequence of 15 contiguous nt from the sequence of a first and/or second strand of: AD-35590.4 (SEQ ID NOs: 57 and 484), or a modified or unmodified variant thereof.

A RNAi agent comprising a first and a second strand, wherein the sequence of the first and/or second strand comprise a sequence of 15 contiguous nt from the sequence of a first and/or second strand of: AD-35596.4 (SEQ ID NOs: 58 and 485), or a modified or unmodified variant thereof.

A RNAi agent comprising a first and a second strand, wherein the sequence of the first and/or second strand comprise a sequence of 15 contiguous nt from the sequence of a first and/or second strand of: AD-35602.4 (SEQ ID NOs: 59 and 486), or a modified or unmodified variant thereof.

A RNAi agent comprising a first and a second strand, wherein the sequence of the first and/or second strand comprise a sequence of 15 contiguous nt from the sequence of a first and/or second strand of: AD-35608.4 (SEQ ID NOs: 60 and 487), or a modified or unmodified variant thereof.

A RNAi agent comprising a first and a second strand, wherein the sequence of the first and/or second strand comprise a sequence of 15 contiguous nt from the sequence of a first and/or second strand of: AD-35614.4 (SEQ ID NOs: 61 and 488), or a modified or unmodified variant thereof.

A RNAi agent comprising a first and a second strand, wherein the sequence of the first and/or second strand comprise a sequence of 15 contiguous nt from the sequence of a first and/or second strand of: AD-35573.4 (SEQ ID NOs: 62 and 489), or a modified or unmodified variant thereof.

A RNAi agent comprising a first and a second strand, wherein the sequence of the first and/or second strand comprise a sequence of 15 contiguous nt from the sequence of a first and/or second strand of: AD-35579.4 (SEQ ID NOs: 63 and 490), or a modified or unmodified variant thereof.

A RNAi agent comprising a first and a second strand, wherein the sequence of the first and/or second strand comprise a sequence of 15 contiguous nt from the sequence of a first and/or second strand of: AD-35585.4 (SEQ ID NOs: 64 and 491), or a modified or unmodified variant thereof.

A RNAi agent comprising a first and a second strand, wherein the sequence of the first and/or second strand comprise a sequence of 15 contiguous nt from the sequence of a first and/or second strand of: AD-35591.4 (SEQ ID NOs: 65 and 492), or a modified or unmodified variant thereof.

A RNAi agent comprising a first and a second strand, wherein the sequence of the first and/or second strand comprise a sequence of 15 contiguous nt from the sequence of a first and/or second strand of: AD-35597.4 (SEQ ID NOs: 66 and 493), or a modified or unmodified variant thereof.

A RNAi agent comprising a first and a second strand, wherein the sequence of the first and/or second strand comprise a sequence of 15 contiguous nt from the sequence of a first and/or second strand of: AD-35603.4 (SEQ ID NOs: 67 and 494), or a modified or unmodified variant thereof.

A RNAi agent comprising a first and a second strand, wherein the sequence of the first and/or second strand comprise a sequence of 15 contiguous nt from the sequence of a first and/or second strand of: AD-35609.4 (SEQ ID NOs: 68 and 495), or a modified or unmodified variant thereof.

A RNAi agent comprising a first and a second strand, wherein the sequence of the first and/or second strand comprise a sequence of 15 contiguous nt from the sequence of a first and/or second strand of: AD-35615.4 (SEQ ID NOs: 69 and 496), or a modified or unmodified variant thereof.

A RNAi agent comprising a first and a second strand, wherein the sequence of the first and/or second strand comprise a sequence of 15 contiguous nt from the sequence of a first and/or second strand of: AD-35574.4 (SEQ ID NOs: 70 and 497), or a modified or unmodified variant thereof.

A RNAi agent comprising a first and a second strand, wherein the sequence of the first and/or second strand comprise a sequence of 15 contiguous nt from the sequence of a first and/or second strand of: AD-35580.1 (SEQ ID NOs: 71 and 498), or a modified or unmodified variant thereof.

A RNAi agent comprising a first and a second strand, wherein the sequence of the first and/or second strand comprise a sequence of 15 contiguous nt from the sequence of a first and/or second strand of: AD-35586.4 (SEQ ID NOs: 72 and 499), or a modified or unmodified variant thereof.

A RNAi agent comprising a first and a second strand, wherein the sequence of the first and/or second strand comprise a sequence of 15 contiguous nt from the sequence of a first and/or second strand of: AD-35592.4 (SEQ ID NOs: 73 and 500), or a modified or unmodified variant thereof.

A RNAi agent comprising a first and a second strand, wherein the sequence of the first and/or second strand comprise a sequence of 15 contiguous nt from the sequence of a first and/or second strand of: AD-35598.4 (SEQ ID NOs: 74 and 501), or a modified or unmodified variant thereof.

A RNAi agent comprising a first and a second strand, wherein the sequence of the first and/or second strand comprise a sequence of 15 contiguous nt from the sequence of a first and/or second strand of: AD-35604.4 (SEQ ID NOs: 75 and 502), or a modified or unmodified variant thereof.

A RNAi agent comprising a first and a second strand, wherein the sequence of the first and/or second strand comprise a sequence of 15 contiguous nt from the sequence of a first and/or second strand of: AD-35610.4 (SEQ ID NOs: 76 and 503), or a modified or unmodified variant thereof.

A RNAi agent comprising a first and a second strand, wherein the sequence of the first and/or second strand comprise a sequence of 15 contiguous nt from the sequence of a first and/or second strand of: AD-35616.4 (SEQ ID NOs: 77 and 504), or a modified or unmodified variant thereof.

A RNAi agent comprising a first and a second strand, wherein the sequence of the first and/or second strand comprise a sequence of 15 contiguous nt from the sequence of a first and/or second strand of: AD-35575.4 (SEQ ID NOs: 78 and 505), or a modified or unmodified variant thereof.

A RNAi agent comprising a first and a second strand, wherein the sequence of the first and/or second strand comprise a sequence of 15 contiguous nt from the sequence of a first and/or second strand of: AD-35581.4 (SEQ ID NOs: 79 and 506), or a modified or unmodified variant thereof.

A RNAi agent comprising a first and a second strand, wherein the sequence of the first and/or second strand comprise a sequence of 15 contiguous nt from the sequence of a first and/or second strand of: AD-35587.4 (SEQ ID NOs: 80 and 507), or a modified or unmodified variant thereof.

A RNAi agent comprising a first and a second strand, wherein the sequence of the first and/or second strand comprise a sequence of 15 contiguous nt from the sequence of a first and/or second strand of: AD-35593.4 (SEQ ID NOs: 81 and 508), or a modified or unmodified variant thereof.

A RNAi agent comprising a first and a second strand, wherein the sequence of the first and/or second strand comprise a sequence of 15 contiguous nt from the sequence of a first and/or second strand of: AD-35599.4 (SEQ ID NOs: 82 and 509), or a modified or unmodified variant thereof.

A RNAi agent comprising a first and a second strand, wherein the sequence of the first and/or second strand comprise a sequence of 15 contiguous nt from the sequence of a first and/or second strand of: AD-35605.4 (SEQ ID NOs: 83 and 510), or a modified or unmodified variant thereof.

A RNAi agent comprising a first and a second strand, wherein the sequence of the first and/or second strand comprise a sequence of 15 contiguous nt from the sequence of a first and/or second strand of: AD-35611.4 (SEQ ID NOs: 84 and 511), or a modified or unmodified variant thereof.

A RNAi agent comprising a first and a second strand, wherein the sequence of the first and/or second strand comprise a sequence of 15 contiguous nt from the sequence of a first and/or second strand of: AD-35617.4 (SEQ ID NOs: 85 and 512), or a modified or unmodified variant thereof.

A RNAi agent comprising a first and a second strand, wherein the sequence of the first and/or second strand comprise a sequence of 15 contiguous nt from the sequence of a first and/or second strand of: AD-35576.4 (SEQ ID NOs: 86 and 513), or a modified or unmodified variant thereof.

A RNAi agent comprising a first and a second strand, wherein the sequence of the first and/or second strand comprise a sequence of 15 contiguous nt from the sequence of a first and/or second strand of: AD-35588.4 (SEQ ID NOs: 87 and 514), or a modified or unmodified variant thereof.

A RNAi agent comprising a first and a second strand, wherein the sequence of the first and/or second strand comprise a sequence of 15 contiguous nt from the sequence of a first and/or second strand of: AD-35667.1 (SEQ ID NOs: 88 and 515), or a modified or unmodified variant thereof.

A RNAi agent comprising a first and a second strand, wherein the sequence of the first and/or second strand comprise a sequence of 15 contiguous nt from the sequence of a first and/or second strand of: AD-35673.1 (SEQ ID NOs: 89 and 516), or a modified or unmodified variant thereof.

A RNAi agent comprising a first and a second strand, wherein the sequence of the first and/or second strand comprise a sequence of 15 contiguous nt from the sequence of a first and/or second strand of: AD-35679.1 (SEQ ID NOs: 90 and 517), or a modified or unmodified variant thereof.

A RNAi agent comprising a first and a second strand, wherein the sequence of the first and/or second strand comprise a sequence of 15 contiguous nt from the sequence of a first and/or second strand of: AD-35685.1 (SEQ ID NOs: 91 and 518), or a modified or unmodified variant thereof.

A RNAi agent comprising a first and a second strand, wherein the sequence of the first and/or second strand comprise a sequence of 15 contiguous nt from the sequence of a first and/or second strand of: AD-35691.1 (SEQ ID NOs: 92 and 519), or a modified or unmodified variant thereof.

A RNAi agent comprising a first and a second strand, wherein the sequence of the first and/or second strand comprise a sequence of 15 contiguous nt from the sequence of a first and/or second strand of: AD-35557.1 (SEQ ID NOs: 93 and 520), or a modified or unmodified variant thereof.

A RNAi agent comprising a first and a second strand, wherein the sequence of the first and/or second strand comprise a sequence of 15 contiguous nt from the sequence of a first and/or second strand of: AD-35618.1 (SEQ ID NOs: 94 and 521), or a modified or unmodified variant thereof.

A RNAi agent comprising a first and a second strand, wherein the sequence of the first and/or second strand comprise a sequence of 15 contiguous nt from the sequence of a first and/or second strand of: AD-35569.1 (SEQ ID NOs: 95 and 522), or a modified or unmodified variant thereof.

A RNAi agent comprising a first and a second strand, wherein the sequence of the first and/or second strand comprise a sequence of 15 contiguous nt from the sequence of a first and/or second strand of: AD-35534.1 (SEQ ID NOs: 96 and 523), or a modified or unmodified variant thereof.

A RNAi agent comprising a first and a second strand, wherein the sequence of the first and/or second strand comprise a sequence of 15 contiguous nt from the sequence of a first and/or second strand of: AD-35582.1 (SEQ ID NOs: 97 and 524), or a modified or unmodified variant thereof.

A RNAi agent comprising a first and a second strand, wherein the sequence of the first and/or second strand comprise a sequence of 15 contiguous nt from the sequence of a first and/or second strand of: AD-35594.1 (SEQ ID NOs: 98 and 525), or a modified or unmodified variant thereof.

A RNAi agent comprising a first and a second strand, wherein the sequence of the first and/or second strand comprise a sequence of 15 contiguous nt from the sequence of a first and/or second strand of: AD-35600.1 (SEQ ID NOs: 99 and 526), or a modified or unmodified variant thereof.

A RNAi agent comprising a first and a second strand, wherein the sequence of the first and/or second strand comprise a sequence of 15 contiguous nt from the sequence of a first and/or second strand of: AD-35606.1 (SEQ ID NOs: 100 and 527), or a modified or unmodified variant thereof.

A RNAi agent comprising a first and a second strand, wherein the sequence of the first and/or second strand comprise a sequence of 15 contiguous nt from the sequence of a first and/or second strand of: AD-35612.1 (SEQ ID NOs: 101 and 528), or a modified or unmodified variant thereof.

A RNAi agent comprising a first and a second strand, wherein the sequence of the first and/or second strand comprise a sequence of 15 contiguous nt from the sequence of a first and/or second strand of: AD-38127.1 (SEQ ID NOs: 102 and 529), or a modified or unmodified variant thereof.

A RNAi agent comprising a first and a second strand, wherein the sequence of the first and/or second strand comprise a sequence of 15 contiguous nt from the sequence of a first and/or second strand of: AD-38133.1 (SEQ ID NOs: 103 and 530), or a modified or unmodified variant thereof.

A RNAi agent comprising a first and a second strand, wherein the sequence of the first and/or second strand comprise a sequence of 15 contiguous nt from the sequence of a first and/or second strand of: AD-38139.1 (SEQ ID NOs: 104 and 531), or a modified or unmodified variant thereof.

A RNAi agent comprising a first and a second strand, wherein the sequence of the first and/or second strand comprise a sequence of 15 contiguous nt from the sequence of a first and/or second strand of: AD-38145.1 (SEQ ID NOs: 105 and 532), or a modified or unmodified variant thereof.

A RNAi agent comprising a first and a second strand, wherein the sequence of the first and/or second strand comprise a sequence of 15 contiguous nt from the sequence of a first and/or second strand of: AD-38151.1 (SEQ ID NOs: 106 and 533), or a modified or unmodified variant thereof.

A RNAi agent comprising a first and a second strand, wherein the sequence of the first and/or second strand comprise a sequence of 15 contiguous nt from the sequence of a first and/or second strand of: AD-38157.1 (SEQ ID NOs: 107 and 534), or a modified or unmodified variant thereof.

A RNAi agent comprising a first and a second strand, wherein the sequence of the first and/or second strand comprise a sequence of 15 contiguous nt from the sequence of a first and/or second strand of: AD-38163.1 (SEQ ID NOs: 108 and 535), or a modified or unmodified variant thereof.

A RNAi agent comprising a first and a second strand, wherein the sequence of the first and/or second strand comprise a sequence of 15 contiguous nt from the sequence of a first and/or second strand of: AD-38169.1 (SEQ ID NOs: 109 and 536), or a modified or unmodified variant thereof.

A RNAi agent comprising a first and a second strand, wherein the sequence of the first and/or second strand comprise a sequence of 15 contiguous nt from the sequence of a first and/or second strand of: AD-38128.1 (SEQ ID NOs: 110 and 537), or a modified or unmodified variant thereof.

A RNAi agent comprising a first and a second strand, wherein the sequence of the first and/or second strand comprise a sequence of 15 contiguous nt from the sequence of a first and/or second strand of: AD-38134.1 (SEQ ID NOs: 111 and 538), or a modified or unmodified variant thereof.

A RNAi agent comprising a first and a second strand, wherein the sequence of the first and/or second strand comprise a sequence of 15 contiguous nt from the sequence of a first and/or second strand of: AD-38140.1 (SEQ ID NOs: 112 and 539), or a modified or unmodified variant thereof.

A RNAi agent comprising a first and a second strand, wherein the sequence of the first and/or second strand comprise a sequence of 15 contiguous nt from the sequence of a first and/or second strand of: AD-38146.1 (SEQ ID NOs: 113 and 540), or a modified or unmodified variant thereof.

A RNAi agent comprising a first and a second strand, wherein the sequence of the first and/or second strand comprise a sequence of 15 contiguous nt from the sequence of a first and/or second strand of: AD-38152.1 (SEQ ID NOs: 114 and 541), or a modified or unmodified variant thereof.

A RNAi agent comprising a first and a second strand, wherein the sequence of the first and/or second strand comprise a sequence of 15 contiguous nt from the sequence of a first and/or second strand of: AD-38158.1 (SEQ ID NOs: 115 and 542), or a modified or unmodified variant thereof.

A RNAi agent comprising a first and a second strand, wherein the sequence of the first and/or second strand comprise a sequence of 15 contiguous nt from the sequence of a first and/or second strand of: AD-38164.1 (SEQ ID NOs: 116 and 543), or a modified or unmodified variant thereof.

A RNAi agent comprising a first and a second strand, wherein the sequence of the first and/or second strand comprise a sequence of 15 contiguous nt from the sequence of a first and/or second strand of: AD-38170.1 (SEQ ID NOs: 117 and 544), or a modified or unmodified variant thereof.

A RNAi agent comprising a first and a second strand, wherein the sequence of the first and/or second strand comprise a sequence of 15 contiguous nt from the sequence of a first and/or second strand of: AD-38129.1 (SEQ ID NOs: 118 and 545), or a modified or unmodified variant thereof.

A RNAi agent comprising a first and a second strand, wherein the sequence of the first and/or second strand comprise a sequence of 15 contiguous nt from the sequence of a first and/or second strand of: AD-38135.1 (SEQ ID NOs: 119 and 546), or a modified or unmodified variant thereof.

A RNAi agent comprising a first and a second strand, wherein the sequence of the first and/or second strand comprise a sequence of 15 contiguous nt from the sequence of a first and/or second strand of: AD-38141.1 (SEQ ID NOs: 120 and 547), or a modified or unmodified variant thereof.

A RNAi agent comprising a first and a second strand, wherein the sequence of the first and/or second strand comprise a sequence of 15 contiguous nt from the sequence of a first and/or second strand of: AD-38147.1 (SEQ ID NOs: 121 and 548), or a modified or unmodified variant thereof.

A RNAi agent comprising a first and a second strand, wherein the sequence of the first and/or second strand comprise a sequence of 15 contiguous nt from the sequence of a first and/or second strand of: AD-38153.1 (SEQ ID NOs: 122 and 549), or a modified or unmodified variant thereof.

A RNAi agent comprising a first and a second strand, wherein the sequence of the first and/or second strand comprise a sequence of 15 contiguous nt from the sequence of a first and/or second strand of: AD-38159.1 (SEQ ID NOs: 123 and 550), or a modified or unmodified variant thereof.

A RNAi agent comprising a first and a second strand, wherein the sequence of the first and/or second strand comprise a sequence of 15 contiguous nt from the sequence of a first and/or second strand of: AD-38165.1 (SEQ ID NOs: 124 and 551), or a modified or unmodified variant thereof.

A RNAi agent comprising a first and a second strand, wherein the sequence of the first and/or second strand comprise a sequence of 15 contiguous nt from the sequence of a first and/or second strand of: AD-38171.1 (SEQ ID NOs: 125 and 552), or a modified or unmodified variant thereof.

A RNAi agent comprising a first and a second strand, wherein the sequence of the first and/or second strand comprise a sequence of 15 contiguous nt from the sequence of a first and/or second strand of: AD-38130.1 (SEQ ID NOs: 126 and 553), or a modified or unmodified variant thereof.

A RNAi agent comprising a first and a second strand, wherein the sequence of the first and/or second strand comprise a sequence of 15 contiguous nt from the sequence of a first and/or second strand of: AD-38136.1 (SEQ ID NOs: 127 and 554), or a modified or unmodified variant thereof.

A RNAi agent comprising a first and a second strand, wherein the sequence of the first and/or second strand comprise a sequence of 15 contiguous nt from the sequence of a first and/or second strand of: AD-38142.1 (SEQ ID NOs: 128 and 555), or a modified or unmodified variant thereof.

A RNAi agent comprising a first and a second strand, wherein the sequence of the first and/or second strand comprise a sequence of 15 contiguous nt from the sequence of a first and/or second strand of: AD-38148.1 (SEQ ID NOs: 129 and 556), or a modified or unmodified variant thereof.

A RNAi agent comprising a first and a second strand, wherein the sequence of the first and/or second strand comprise a sequence of 15 contiguous nt from the sequence of a first and/or second strand of: AD-38154.1 (SEQ ID NOs: 130 and 557), or a modified or unmodified variant thereof.

A RNAi agent comprising a first and a second strand, wherein the sequence of the first and/or second strand comprise a sequence of 15 contiguous nt from the sequence of a first and/or second strand of: AD-38160.1 (SEQ ID NOs: 131 and 558), or a modified or unmodified variant thereof.

A RNAi agent comprising a first and a second strand, wherein the sequence of the first and/or second strand comprise a sequence of 15 contiguous nt from the sequence of a first and/or second strand of: AD-38166.1 (SEQ ID NOs: 132 and 559), or a modified or unmodified variant thereof.

A RNAi agent comprising a first and a second strand, wherein the sequence of the first and/or second strand comprise a sequence of 15 contiguous nt from the sequence of a first and/or second strand of: AD-38172.1 (SEQ ID NOs: 133 and 560), or a modified or unmodified variant thereof.

A RNAi agent comprising a first and a second strand, wherein the sequence of the first and/or second strand comprise a sequence of 15 contiguous nt from the sequence of a first and/or second strand of: AD-38131.1 (SEQ ID NOs: 134 and 561), or a modified or unmodified variant thereof.

A RNAi agent comprising a first and a second strand, wherein the sequence of the first and/or second strand comprise a sequence of 15 contiguous nt from the sequence of a first and/or second strand of: AD-38137.1 (SEQ ID NOs: 135 and 562), or a modified or unmodified variant thereof.

A RNAi agent comprising a first and a second strand, wherein the sequence of the first and/or second strand comprise a sequence of 15 contiguous nt from the sequence of a first and/or second strand of: AD-38143.1 (SEQ ID NOs: 136 and 563), or a modified or unmodified variant thereof.

A RNAi agent comprising a first and a second strand, wherein the sequence of the first and/or second strand comprise a sequence of 15 contiguous nt from the sequence of a first and/or second strand of: AD-38149.1 (SEQ ID NOs: 137 and 564), or a modified or unmodified variant thereof.

A RNAi agent comprising a first and a second strand, wherein the sequence of the first and/or second strand comprise a sequence of 15 contiguous nt from the sequence of a first and/or second strand of: AD-38155.1 (SEQ ID NOs: 138 and 565), or a modified or unmodified variant thereof.

A RNAi agent comprising a first and a second strand, wherein the sequence of the first and/or second strand comprise a sequence of 15 contiguous nt from the sequence of a first and/or second strand of: AD-38161.1 (SEQ ID NOs: 139 and 566), or a modified or unmodified variant thereof.

A RNAi agent comprising a first and a second strand, wherein the sequence of the first and/or second strand comprise a sequence of 15 contiguous nt from the sequence of a first and/or second strand of: AD-38167.1 (SEQ ID NOs: 140 and 567), or a modified or unmodified variant thereof.

A RNAi agent comprising a first and a second strand, wherein the sequence of the first and/or second strand comprise a sequence of 15 contiguous nt from the sequence of a first and/or second strand of: AD-38173.1 (SEQ ID NOs: 141 and 568), or a modified or unmodified variant thereof.

A RNAi agent comprising a first and a second strand, wherein the sequence of the first and/or second strand comprise a sequence of 15 contiguous nt from the sequence of a first and/or second strand of: AD-38132.1 (SEQ ID NOs: 142 and 569), or a modified or unmodified variant thereof.

A RNAi agent comprising a first and a second strand, wherein the sequence of the first and/or second strand comprise a sequence of 15 contiguous nt from the sequence of a first and/or second strand of: AD-38138.1 (SEQ ID NOs: 143 and 570), or a modified or unmodified variant thereof.

A RNAi agent comprising a first and a second strand, wherein the sequence of the first and/or second strand comprise a sequence of 15 contiguous nt from the sequence of a first and/or second strand of: AD-38144.1 (SEQ ID NOs: 144 and 571), or a modified or unmodified variant thereof.

A RNAi agent comprising a first and a second strand, wherein the sequence of the first and/or second strand comprise a sequence of 15 contiguous nt from the sequence of a first and/or second strand of: AD-38150.1 (SEQ ID NOs: 145 and 572), or a modified or unmodified variant thereof.

A RNAi agent comprising a first and a second strand, wherein the sequence of the first and/or second strand comprise a sequence of 15 contiguous nt from the sequence of a first and/or second strand of: AD-38156.1 (SEQ ID NOs: 146 and 573), or a modified or unmodified variant thereof.

A RNAi agent comprising a first and a second strand, wherein the sequence of the first and/or second strand comprise a sequence of 15 contiguous nt from the sequence of a first and/or second strand of: AD-38162.1 (SEQ ID NOs: 147 and 574), or a modified or unmodified variant thereof.

A RNAi agent comprising a first and a second strand, wherein the sequence of the first and/or second strand comprise a sequence of 15 contiguous nt from the sequence of a first and/or second strand of: AD-38168.1 (SEQ ID NOs: 148 and 575), or a modified or unmodified variant thereof.

A RNAi agent comprising a first and a second strand, wherein the sequence of the first and/or second strand comprise a sequence of 15 contiguous nt from the sequence of a first and/or second strand of: AD-38174.1 (SEQ ID NOs: 149 and 576), or a modified or unmodified variant thereof.

A RNAi agent comprising a first and a second strand, wherein the sequence of the first and/or second strand comprise a sequence of 15 contiguous nt from the sequence of a first and/or second strand of: AD-39683.1 (SEQ ID NOs: 150 and 577), or a modified or unmodified variant thereof.

A RNAi agent comprising a first and a second strand, wherein the sequence of the first and/or second strand comprise a sequence of 15 contiguous nt from the sequence of a first and/or second strand of: AD-39689.1 (SEQ ID NOs: 151 and 578), or a modified or unmodified variant thereof.

A RNAi agent comprising a first and a second strand, wherein the sequence of the first and/or second strand comprise a sequence of 15 contiguous nt from the sequence of a first and/or second strand of: AD-39695.1 (SEQ ID NOs: 152 and 579), or a modified or unmodified variant thereof.

A RNAi agent comprising a first and a second strand, wherein the sequence of the first and/or second strand comprise a sequence of 15 contiguous nt from the sequence of a first and/or second strand of: AD-39701.1 (SEQ ID NOs: 153 and 580), or a modified or unmodified variant thereof.

A RNAi agent comprising a first and a second strand, wherein the sequence of the first and/or second strand comprise a sequence of 15 contiguous nt from the sequence of a first and/or second strand of: AD-39707.1 (SEQ ID NOs: 154 and 581), or a modified or unmodified variant thereof.

A RNAi agent comprising a first and a second strand, wherein the sequence of the first and/or second strand comprise a sequence of 15 contiguous nt from the sequence of a first and/or second strand of: AD-39713.1 (SEQ ID NOs: 155 and 582), or a modified or unmodified variant thereof.

A RNAi agent comprising a first and a second strand, wherein the sequence of the first and/or second strand comprise a sequence of 15 contiguous nt from the sequence of a first and/or second strand of: AD-39719.1 (SEQ ID NOs: 156 and 583), or a modified or unmodified variant thereof.

A RNAi agent comprising a first and a second strand, wherein the sequence of the first and/or second strand comprise a sequence of 15 contiguous nt from the sequence of a first and/or second strand of: AD-39725.1 (SEQ ID NOs: 157 and 584), or a modified or unmodified variant thereof.

A RNAi agent comprising a first and a second strand, wherein the sequence of the first and/or second strand comprise a sequence of 15 contiguous nt from the sequence of a first and/or second strand of: AD-39684.1 (SEQ ID NOs: 158 and 585), or a modified or unmodified variant thereof.

A RNAi agent comprising a first and a second strand, wherein the sequence of the first and/or second strand comprise a sequence of 15 contiguous nt from the sequence of a first and/or second strand of: AD-39690.1 (SEQ ID NOs: 159 and 586), or a modified or unmodified variant thereof.

A RNAi agent comprising a first and a second strand, wherein the sequence of the first and/or second strand comprise a sequence of 15 contiguous nt from the sequence of a first and/or second strand of: AD-39696.1 (SEQ ID NOs: 160 and 587), or a modified or unmodified variant thereof.

A RNAi agent comprising a first and a second strand, wherein the sequence of the first and/or second strand comprise a sequence of 15 contiguous nt from the sequence of a first and/or second strand of: AD-39702.1 (SEQ ID NOs: 161 and 588), or a modified or unmodified variant thereof.

A RNAi agent comprising a first and a second strand, wherein the sequence of the first and/or second strand comprise a sequence of 15 contiguous nt from the sequence of a first and/or second strand of: AD-39708.1 (SEQ ID NOs: 162 and 589), or a modified or unmodified variant thereof.

A RNAi agent comprising a first and a second strand, wherein the sequence of the first and/or second strand comprise a sequence of 15 contiguous nt from the sequence of a first and/or second strand of: AD-39714.1 (SEQ ID NOs: 163 and 590), or a modified or unmodified variant thereof.

A RNAi agent comprising a first and a second strand, wherein the sequence of the first and/or second strand comprise a sequence of 15 contiguous nt from the sequence of a first and/or second strand of: AD-39720.1 (SEQ ID NOs: 164 and 591), or a modified or unmodified variant thereof.

A RNAi agent comprising a first and a second strand, wherein the sequence of the first and/or second strand comprise a sequence of 15 contiguous nt from the sequence of a first and/or second strand of: AD-39726.1 (SEQ ID NOs: 165 and 592), or a modified or unmodified variant thereof.

A RNAi agent comprising a first and a second strand, wherein the sequence of the first and/or second strand comprise a sequence of 15 contiguous nt from the sequence of a first and/or second strand of: AD-39685.1 (SEQ ID NOs: 166 and 593), or a modified or unmodified variant thereof.

A RNAi agent comprising a first and a second strand, wherein the sequence of the first and/or second strand comprise a sequence of 15 contiguous nt from the sequence of a first and/or second strand of: AD-39691.1 (SEQ ID NOs: 167 and 594), or a modified or unmodified variant thereof.

A RNAi agent comprising a first and a second strand, wherein the sequence of the first and/or second strand comprise a sequence of 15 contiguous nt from the sequence of a first and/or second strand of: AD-39697.1 (SEQ ID NOs: 168 and 595), or a modified or unmodified variant thereof.

A RNAi agent comprising a first and a second strand, wherein the sequence of the first and/or second strand comprise a sequence of 15 contiguous nt from the sequence of a first and/or second strand of: AD-39703.1 (SEQ ID NOs: 169 and 596), or a modified or unmodified variant thereof.

A RNAi agent comprising a first and a second strand, wherein the sequence of the first and/or second strand comprise a sequence of 15 contiguous nt from the sequence of a first and/or second strand of: AD-39709.1 (SEQ ID NOs: 170 and 597), or a modified or unmodified variant thereof.

A RNAi agent comprising a first and a second strand, wherein the sequence of the first and/or second strand comprise a sequence of 15 contiguous nt from the sequence of a first and/or second strand of: AD-39715.1 (SEQ ID NOs: 171 and 598), or a modified or unmodified variant thereof.

A RNAi agent comprising a first and a second strand, wherein the sequence of the first and/or second strand comprise a sequence of 15 contiguous nt from the sequence of a first and/or second strand of: AD-39721.1 (SEQ ID NOs: 172 and 599), or a modified or unmodified variant thereof.

A RNAi agent comprising a first and a second strand, wherein the sequence of the first and/or second strand comprise a sequence of 15 contiguous nt from the sequence of a first and/or second strand of: AD-39727.1 (SEQ ID NOs: 173 and 600), or a modified or unmodified variant thereof.

A RNAi agent comprising a first and a second strand, wherein the sequence of the first and/or second strand comprise a sequence of 15 contiguous nt from the sequence of a first and/or second strand of: AD-39686.1 (SEQ ID NOs: 174 and 601), or a modified or unmodified variant thereof.

A RNAi agent comprising a first and a second strand, wherein the sequence of the first and/or second strand comprise a sequence of 15 contiguous nt from the sequence of a first and/or second strand of: AD-39692.1 (SEQ ID NOs: 175 and 602), or a modified or unmodified variant thereof.

A RNAi agent comprising a first and a second strand, wherein the sequence of the first and/or second strand comprise a sequence of 15 contiguous nt from the sequence of a first and/or second strand of: AD-39698.1 (SEQ ID NOs: 176 and 603), or a modified or unmodified variant thereof.

A RNAi agent comprising a first and a second strand, wherein the sequence of the first and/or second strand comprise a sequence of 15 contiguous nt from the sequence of a first and/or second strand of: AD-39704.1 (SEQ ID NOs: 177 and 604), or a modified or unmodified variant thereof.

A RNAi agent comprising a first and a second strand, wherein the sequence of the first and/or second strand comprise a sequence of 15 contiguous nt from the sequence of a first and/or second strand of: AD-39710.1 (SEQ ID NOs: 178 and 605), or a modified or unmodified variant thereof.

A RNAi agent comprising a first and a second strand, wherein the sequence of the first and/or second strand comprise a sequence of 15 contiguous nt from the sequence of a first and/or second strand of: AD-39716.1 (SEQ ID NOs: 179 and 606), or a modified or unmodified variant thereof.

A RNAi agent comprising a first and a second strand, wherein the sequence of the first and/or second strand comprise a sequence of 15 contiguous nt from the sequence of a first and/or second strand of: AD-39722.1 (SEQ ID NOs: 180 and 607), or a modified or unmodified variant thereof.

A RNAi agent comprising a first and a second strand, wherein the sequence of the first and/or second strand comprise a sequence of 15 contiguous nt from the sequence of a first and/or second strand of: AD-39728.1 (SEQ ID NOs: 181 and 608), or a modified or unmodified variant thereof.

A RNAi agent comprising a first and a second strand, wherein the sequence of the first and/or second strand comprise a sequence of 15 contiguous nt from the sequence of a first and/or second strand of: AD-39687.1 (SEQ ID NOs: 182 and 609), or a modified or unmodified variant thereof.

A RNAi agent comprising a first and a second strand, wherein the sequence of the first and/or second strand comprise a sequence of 15 contiguous nt from the sequence of a first and/or second strand of: AD-39693.1 (SEQ ID NOs: 183 and 610), or a modified or unmodified variant thereof.

A RNAi agent comprising a first and a second strand, wherein the sequence of the first and/or second strand comprise a sequence of 15 contiguous nt from the sequence of a first and/or second strand of: AD-39699.1 (SEQ ID NOs: 184 and 611), or a modified or unmodified variant thereof.

A RNAi agent comprising a first and a second strand, wherein the sequence of the first and/or second strand comprise a sequence of 15 contiguous nt from the sequence of a first and/or second strand of: AD-39705.1 (SEQ ID NOs: 185 and 612), or a modified or unmodified variant thereof.

A RNAi agent comprising a first and a second strand, wherein the sequence of the first and/or second strand comprise a sequence of 15 contiguous nt from the sequence of a first and/or second strand of: AD-39711.1 (SEQ ID NOs: 186 and 613), or a modified or unmodified variant thereof.

A RNAi agent comprising a first and a second strand, wherein the sequence of the first and/or second strand comprise a sequence of 15 contiguous nt from the sequence of a first and/or second strand of: AD-39717.1 (SEQ ID NOs: 187 and 614), or a modified or unmodified variant thereof.

A RNAi agent comprising a first and a second strand, wherein the sequence of the first and/or second strand comprise a sequence of 15 contiguous nt from the sequence of a first and/or second strand of: AD-39723.1 (SEQ ID NOs: 188 and 615), or a modified or unmodified variant thereof.

A RNAi agent comprising a first and a second strand, wherein the sequence of the first and/or second strand comprise a sequence of 15 contiguous nt from the sequence of a first and/or second strand of: AD-39729.1 (SEQ ID NOs: 189 and 616), or a modified or unmodified variant thereof.

A RNAi agent comprising a first and a second strand, wherein the sequence of the first and/or second strand comprise a sequence of 15 contiguous nt from the sequence of a first and/or second strand of: AD-39688.1 (SEQ ID NOs: 190 and 617), or a modified or unmodified variant thereof.

A RNAi agent comprising a first and a second strand, wherein the sequence of the first and/or second strand comprise a sequence of 15 contiguous nt from the sequence of a first and/or second strand of: AD-39694.1 (SEQ ID NOs: 191 and 618), or a modified or unmodified variant thereof.

A RNAi agent comprising a first and a second strand, wherein the sequence of the first and/or second strand comprise a sequence of 15 contiguous nt from the sequence of a first and/or second strand of: AD-39700.1 (SEQ ID NOs: 192 and 619), or a modified or unmodified variant thereof.

A RNAi agent comprising a first and a second strand, wherein the sequence of the first and/or second strand comprise a sequence of 15 contiguous nt from the sequence of a first and/or second strand of: AD-39706.1 (SEQ ID NOs: 193 and 620), or a modified or unmodified variant thereof.

A RNAi agent comprising a first and a second strand, wherein the sequence of the first and/or second strand comprise a sequence of 15 contiguous nt from the sequence of a first and/or second strand of: AD-39712.1 (SEQ ID NOs: 194 and 621), or a modified or unmodified variant thereof.

A RNAi agent comprising a first and a second strand, wherein the sequence of the first and/or second strand comprise a sequence of 15 contiguous nt from the sequence of a first and/or second strand of: AD-39718.1 (SEQ ID NOs: 195 and 622), or a modified or unmodified variant thereof.

A RNAi agent comprising a first and a second strand, wherein the sequence of the first and/or second strand comprise a sequence of 15 contiguous nt from the sequence of a first and/or second strand of: AD-39724.1 (SEQ ID NOs: 196 and 623), or a modified or unmodified variant thereof.

A RNAi agent comprising a first and a second strand, wherein the sequence of the first and/or second strand comprise a sequence of 15 contiguous nt from the sequence of a first and/or second strand of: AD-39730.1 (SEQ ID NOs: 197 and 624), or a modified or unmodified variant thereof.

A RNAi agent comprising a first and a second strand, wherein the sequence of the first and/or second strand comprise a sequence of 15 contiguous nt from the sequence of a first and/or second strand of: AD-39736.1 (SEQ ID NOs: 198 and 625), or a modified or unmodified variant thereof.

A RNAi agent comprising a first and a second strand, wherein the sequence of the first and/or second strand comprise a sequence of 15 contiguous nt from the sequence of a first and/or second strand of: AD-39742.1 (SEQ ID NOs: 199 and 626), or a modified or unmodified variant thereof.

A RNAi agent comprising a first and a second strand, wherein the sequence of the first and/or second strand comprise a sequence of 15 contiguous nt from the sequence of a first and/or second strand of: AD-39748.1 (SEQ ID NOs: 200 and 627), or a modified or unmodified variant thereof.

A RNAi agent comprising a first and a second strand, wherein the sequence of the first and/or second strand comprise a sequence of 15 contiguous nt from the sequence of a first and/or second strand of: AD-39754.1 (SEQ ID NOs: 201 and 628), or a modified or unmodified variant thereof.

A RNAi agent comprising a first and a second strand, wherein the sequence of the first and/or second strand comprise a sequence of 15 contiguous nt from the sequence of a first and/or second strand of: AD-39760.1 (SEQ ID NOs: 202 and 629), or a modified or unmodified variant thereof.

A RNAi agent comprising a first and a second strand, wherein the sequence of the first and/or second strand comprise a sequence of 15 contiguous nt from the sequence of a first and/or second strand of: AD-39766.1 (SEQ ID NOs: 203 and 630), or a modified or unmodified variant thereof.

A RNAi agent comprising a first and a second strand, wherein the sequence of the first and/or second strand comprise a sequence of 15 contiguous nt from the sequence of a first and/or second strand of: AD-39772.1 (SEQ ID NOs: 204 and 631), or a modified or unmodified variant thereof.

A RNAi agent comprising a first and a second strand, wherein the sequence of the first and/or second strand comprise a sequence of 15 contiguous nt from the sequence of a first and/or second strand of: AD-39731.1 (SEQ ID NOs: 205 and 632), or a modified or unmodified variant thereof.

A RNAi agent comprising a first and a second strand, wherein the sequence of the first and/or second strand comprise a sequence of 15 contiguous nt from the sequence of a first and/or second strand of: AD-39737.1 (SEQ ID NOs: 206 and 633), or a modified or unmodified variant thereof.

A RNAi agent comprising a first and a second strand, wherein the sequence of the first and/or second strand comprise a sequence of 15 contiguous nt from the sequence of a first and/or second strand of: AD-39743.1 (SEQ ID NOs: 207 and 634), or a modified or unmodified variant thereof.

A RNAi agent comprising a first and a second strand, wherein the sequence of the first and/or second strand comprise a sequence of 15 contiguous nt from the sequence of a first and/or second strand of: AD-39749.1 (SEQ ID NOs: 208 and 635), or a modified or unmodified variant thereof.

A RNAi agent comprising a first and a second strand, wherein the sequence of the first and/or second strand comprise a sequence of 15 contiguous nt from the sequence of a first and/or second strand of: AD-39755.1 (SEQ ID NOs: 209 and 636), or a modified or unmodified variant thereof.

A RNAi agent comprising a first and a second strand, wherein the sequence of the first and/or second strand comprise a sequence of 15 contiguous nt from the sequence of a first and/or second strand of: AD-39761.1 (SEQ ID NOs: 210 and 637), or a modified or unmodified variant thereof.

A RNAi agent comprising a first and a second strand, wherein the sequence of the first and/or second strand comprise a sequence of 15 contiguous nt from the sequence of a first and/or second strand of: AD-39767.1 (SEQ ID NOs: 211 and 638), or a modified or unmodified variant thereof.

A RNAi agent comprising a first and a second strand, wherein the sequence of the first and/or second strand comprise a sequence of 15 contiguous nt from the sequence of a first and/or second strand of: AD-39773.1 (SEQ ID NOs: 212 and 639), or a modified or unmodified variant thereof.

A RNAi agent comprising a first and a second strand, wherein the sequence of the first and/or second strand comprise a sequence of 15 contiguous nt from the sequence of a first and/or second strand of: AD-39732.1 (SEQ ID NOs: 213 and 640), or a modified or unmodified variant thereof.

A RNAi agent comprising a first and a second strand, wherein the sequence of the first and/or second strand comprise a sequence of 15 contiguous nt from the sequence of a first and/or second strand of: AD-39738.1 (SEQ ID NOs: 214 and 641), or a modified or unmodified variant thereof.

A RNAi agent comprising a first and a second strand, wherein the sequence of the first and/or second strand comprise a sequence of 15 contiguous nt from the sequence of a first and/or second strand of: AD-39744.1 (SEQ ID NOs: 215 and 642), or a modified or unmodified variant thereof.

A RNAi agent comprising a first and a second strand, wherein the sequence of the first and/or second strand comprise a sequence of 15 contiguous nt from the sequence of a first and/or second strand of: AD-39750.1 (SEQ ID NOs: 216 and 643), or a modified or unmodified variant thereof.

A RNAi agent comprising a first and a second strand, wherein the sequence of the first and/or second strand comprise a sequence of 15 contiguous nt from the sequence of a first and/or second strand of: AD-39756.1 (SEQ ID NOs: 217 and 644), or a modified or unmodified variant thereof.

A RNAi agent comprising a first and a second strand, wherein the sequence of the first and/or second strand comprise a sequence of 15 contiguous nt from the sequence of a first and/or second strand of: AD-39762.1 (SEQ ID NOs: 218 and 645), or a modified or unmodified variant thereof.

A RNAi agent comprising a first and a second strand, wherein the sequence of the first and/or second strand comprise a sequence of 15 contiguous nt from the sequence of a first and/or second strand of: AD-39768.1 (SEQ ID NOs: 219 and 646), or a modified or unmodified variant thereof.

A RNAi agent comprising a first and a second strand, wherein the sequence of the first and/or second strand comprise a sequence of 15 contiguous nt from the sequence of a first and/or second strand of: AD-39774.1 (SEQ ID NOs: 220 and 647), or a modified or unmodified variant thereof.

A RNAi agent comprising a first and a second strand, wherein the sequence of the first and/or second strand comprise a sequence of 15 contiguous nt from the sequence of a first and/or second strand of: AD-39733.1 (SEQ ID NOs: 221 and 648), or a modified or unmodified variant thereof.

A RNAi agent comprising a first and a second strand, wherein the sequence of the first and/or second strand comprise a sequence of 15 contiguous nt from the sequence of a first and/or second strand of: AD-39739.1 (SEQ ID NOs: 222 and 649), or a modified or unmodified variant thereof.

A RNAi agent comprising a first and a second strand, wherein the sequence of the first and/or second strand comprise a sequence of 15 contiguous nt from the sequence of a first and/or second strand of: AD-39745.1 (SEQ ID NOs: 223 and 650), or a modified or unmodified variant thereof.

A RNAi agent comprising a first and a second strand, wherein the sequence of the first and/or second strand comprise a sequence of 15 contiguous nt from the sequence of a first and/or second strand of: AD-39751.1 (SEQ ID NOs: 224 and 651), or a modified or unmodified variant thereof.

A RNAi agent comprising a first and a second strand, wherein the sequence of the first and/or second strand comprise a sequence of 15 contiguous nt from the sequence of a first and/or second strand of: AD-39757.1 (SEQ ID NOs: 225 and 652), or a modified or unmodified variant thereof.

A RNAi agent comprising a first and a second strand, wherein the sequence of the first and/or second strand comprise a sequence of 15 contiguous nt from the sequence of a first and/or second strand of: AD-39763.1 (SEQ ID NOs: 226 and 653), or a modified or unmodified variant thereof.

A RNAi agent comprising a first and a second strand, wherein the sequence of the first and/or second strand comprise a sequence of 15 contiguous nt from the sequence of a first and/or second strand of: AD-39769.1 (SEQ ID NOs: 227 and 654), or a modified or unmodified variant thereof.

A RNAi agent comprising a first and a second strand, wherein the sequence of the first and/or second strand comprise a sequence of 15 contiguous nt from the sequence of a first and/or second strand of: AD-39775.1 (SEQ ID NOs: 228 and 655), or a modified or unmodified variant thereof.

A RNAi agent comprising a first and a second strand, wherein the sequence of the first and/or second strand comprise a sequence of 15 contiguous nt from the sequence of a first and/or second strand of: AD-39734.1 (SEQ ID NOs: 229 and 656), or a modified or unmodified variant thereof.

A RNAi agent comprising a first and a second strand, wherein the sequence of the first and/or second strand comprise a sequence of 15 contiguous nt from the sequence of a first and/or second strand of: AD-39740.1 (SEQ ID NOs: 230 and 657), or a modified or unmodified variant thereof.

A RNAi agent comprising a first and a second strand, wherein the sequence of the first and/or second strand comprise a sequence of 15 contiguous nt from the sequence of a first and/or second strand of: AD-39746.1 (SEQ ID NOs: 231 and 658), or a modified or unmodified variant thereof.

A RNAi agent comprising a first and a second strand, wherein the sequence of the first and/or second strand comprise a sequence of 15 contiguous nt from the sequence of a first and/or second strand of: AD-39752.1 (SEQ ID NOs: 232 and 659), or a modified or unmodified variant thereof.

A RNAi agent comprising a first and a second strand, wherein the sequence of the first and/or second strand comprise a sequence of 15 contiguous nt from the sequence of a first and/or second strand of: AD-39758.1 (SEQ ID NOs: 233 and 660), or a modified or unmodified variant thereof.

A RNAi agent comprising a first and a second strand, wherein the sequence of the first and/or second strand comprise a sequence of 15 contiguous nt from the sequence of a first and/or second strand of: AD-39764.1 (SEQ ID NOs: 234 and 661), or a modified or unmodified variant thereof.

A RNAi agent comprising a first and a second strand, wherein the sequence of the first and/or second strand comprise a sequence of 15 contiguous nt from the sequence of a first and/or second strand of: AD-39770.1 (SEQ ID NOs: 235 and 662), or a modified or unmodified variant thereof.

A RNAi agent comprising a first and a second strand, wherein the sequence of the first and/or second strand comprise a sequence of 15 contiguous nt from the sequence of a first and/or second strand of: AD-39776.1 (SEQ ID NOs: 236 and 663), or a modified or unmodified variant thereof.

A RNAi agent comprising a first and a second strand, wherein the sequence of the first and/or second strand comprise a sequence of 15 contiguous nt from the sequence of a first and/or second strand of: AD-39735.1 (SEQ ID NOs: 237 and 664), or a modified or unmodified variant thereof.

A RNAi agent comprising a first and a second strand, wherein the sequence of the first and/or second strand comprise a sequence of 15 contiguous nt from the sequence of a first and/or second strand of: AD-39741.1 (SEQ ID NOs: 238 and 665), or a modified or unmodified variant thereof.

A RNAi agent comprising a first and a second strand, wherein the sequence of the first and/or second strand comprise a sequence of 15 contiguous nt from the sequence of a first and/or second strand of: AD-39747.1 (SEQ ID NOs: 239 and 666), or a modified or unmodified variant thereof.

A RNAi agent comprising a first and a second strand, wherein the sequence of the first and/or second strand comprise a sequence of 15 contiguous nt from the sequence of a first and/or second strand of: AD-39753.1 (SEQ ID NOs: 240 and 667), or a modified or unmodified variant thereof.

A RNAi agent comprising a first and a second strand, wherein the sequence of the first and/or second strand comprise a sequence of 15 contiguous nt from the sequence of a first and/or second strand of: AD-39759.1 (SEQ ID NOs: 241 and 668), or a modified or unmodified variant thereof.

A RNAi agent comprising a first and a second strand, wherein the sequence of the first and/or second strand comprise a sequence of 15 contiguous nt from the sequence of a first and/or second strand of: AD-39765.1 (SEQ ID NOs: 242 and 669), or a modified or unmodified variant thereof.

A RNAi agent comprising a first and a second strand, wherein the sequence of the first and/or second strand comprise a sequence of 15 contiguous nt from the sequence of a first and/or second strand of: AD-39771.1 (SEQ ID NOs: 243 and 670), or a modified or unmodified variant thereof.

A RNAi agent comprising a first and a second strand, wherein the sequence of the first and/or second strand comprise a sequence of 15 contiguous nt from the sequence of a first and/or second strand of: AD-39778.1 (SEQ ID NOs: 244 and 671), or a modified or unmodified variant thereof.

A RNAi agent comprising a first and a second strand, wherein the sequence of the first and/or second strand comprise a sequence of 15 contiguous nt from the sequence of a first and/or second strand of: AD-39784.1 (SEQ ID NOs: 245 and 672), or a modified or unmodified variant thereof.

A RNAi agent comprising a first and a second strand, wherein the sequence of the first and/or second strand comprise a sequence of 15 contiguous nt from the sequence of a first and/or second strand of: AD-39790.1 (SEQ ID NOs: 246 and 673), or a modified or unmodified variant thereof.

A RNAi agent comprising a first and a second strand, wherein the sequence of the first and/or second strand comprise a sequence of 15 contiguous nt from the sequence of a first and/or second strand of: AD-39796.1 (SEQ ID NOs: 247 and 674), or a modified or unmodified variant thereof.

A RNAi agent comprising a first and a second strand, wherein the sequence of the first and/or second strand comprise a sequence of 15 contiguous nt from the sequence of a first and/or second strand of: AD-39802.1 (SEQ ID NOs: 248 and 675), or a modified or unmodified variant thereof.

A RNAi agent comprising a first and a second strand, wherein the sequence of the first and/or second strand comprise a sequence of 15 contiguous nt from the sequence of a first and/or second strand of: AD-39808.1 (SEQ ID NOs: 249 and 676), or a modified or unmodified variant thereof.

A RNAi agent comprising a first and a second strand, wherein the sequence of the first and/or second strand comprise a sequence of 15 contiguous nt from the sequence of a first and/or second strand of: AD-39814.1 (SEQ ID NOs: 250 and 677), or a modified or unmodified variant thereof.

A RNAi agent comprising a first and a second strand, wherein the sequence of the first and/or second strand comprise a sequence of 15 contiguous nt from the sequence of a first and/or second strand of: AD-39820.1 (SEQ ID NOs: 251 and 678), or a modified or unmodified variant thereof.

A RNAi agent comprising a first and a second strand, wherein the sequence of the first and/or second strand comprise a sequence of 15 contiguous nt from the sequence of a first and/or second strand of: AD-39779.1 (SEQ ID NOs: 252 and 679), or a modified or unmodified variant thereof.

A RNAi agent comprising a first and a second strand, wherein the sequence of the first and/or second strand comprise a sequence of 15 contiguous nt from the sequence of a first and/or second strand of: AD-39785.1 (SEQ ID NOs: 253 and 680), or a modified or unmodified variant thereof.

A RNAi agent comprising a first and a second strand, wherein the sequence of the first and/or second strand comprise a sequence of 15 contiguous nt from the sequence of a first and/or second strand of: AD-39791.1 (SEQ ID NOs: 254 and 681), or a modified or unmodified variant thereof.

A RNAi agent comprising a first and a second strand, wherein the sequence of the first and/or second strand comprise a sequence of 15 contiguous nt from the sequence of a first and/or second strand of: AD-39797.1 (SEQ ID NOs: 255 and 682), or a modified or unmodified variant thereof.

A RNAi agent comprising a first and a second strand, wherein the sequence of the first and/or second strand comprise a sequence of 15 contiguous nt from the sequence of a first and/or second strand of: AD-39803.1 (SEQ ID NOs: 256 and 683), or a modified or unmodified variant thereof.

A RNAi agent comprising a first and a second strand, wherein the sequence of the first and/or second strand comprise a sequence of 15 contiguous nt from the sequence of a first and/or second strand of: AD-39809.1 (SEQ ID NOs: 257 and 684), or a modified or unmodified variant thereof.

A RNAi agent comprising a first and a second strand, wherein the sequence of the first and/or second strand comprise a sequence of 15 contiguous nt from the sequence of a first and/or second strand of: AD-39815.1 (SEQ ID NOs: 258 and 685), or a modified or unmodified variant thereof.

A RNAi agent comprising a first and a second strand, wherein the sequence of the first and/or second strand comprise a sequence of 15 contiguous nt from the sequence of a first and/or second strand of: AD-39821.1 (SEQ ID NOs: 259 and 686), or a modified or unmodified variant thereof.

A RNAi agent comprising a first and a second strand, wherein the sequence of the first and/or second strand comprise a sequence of 15 contiguous nt from the sequence of a first and/or second strand of: AD-39780.1 (SEQ ID NOs: 260 and 687), or a modified or unmodified variant thereof.

A RNAi agent comprising a first and a second strand, wherein the sequence of the first and/or second strand comprise a sequence of 15 contiguous nt from the sequence of a first and/or second strand of: AD-39786.1 (SEQ ID NOs: 261 and 688), or a modified or unmodified variant thereof.

A RNAi agent comprising a first and a second strand, wherein the sequence of the first and/or second strand comprise a sequence of 15 contiguous nt from the sequence of a first and/or second strand of: AD-39792.1 (SEQ ID NOs: 262 and 689), or a modified or unmodified variant thereof.

A RNAi agent comprising a first and a second strand, wherein the sequence of the first and/or second strand comprise a sequence of 15 contiguous nt from the sequence of a first and/or second strand of: AD-39798.1 (SEQ ID NOs: 263 and 690), or a modified or unmodified variant thereof.

A RNAi agent comprising a first and a second strand, wherein the sequence of the first and/or second strand comprise a sequence of 15 contiguous nt from the sequence of a first and/or second strand of: AD-39804.1 (SEQ ID NOs: 264 and 691), or a modified or unmodified variant thereof.

A RNAi agent comprising a first and a second strand, wherein the sequence of the first and/or second strand comprise a sequence of 15 contiguous nt from the sequence of a first and/or second strand of: AD-39810.1 (SEQ ID NOs: 265 and 692), or a modified or unmodified variant thereof.

A RNAi agent comprising a first and a second strand, wherein the sequence of the first and/or second strand comprise a sequence of 15 contiguous nt from the sequence of a first and/or second strand of: AD-39816.1 (SEQ ID NOs: 266 and 693), or a modified or unmodified variant thereof.

A RNAi agent comprising a first and a second strand, wherein the sequence of the first and/or second strand comprise a sequence of 15 contiguous nt from the sequence of a first and/or second strand of: AD-39822.1 (SEQ ID NOs: 267 and 694), or a modified or unmodified variant thereof.

A RNAi agent comprising a first and a second strand, wherein the sequence of the first and/or second strand comprise a sequence of 15 contiguous nt from the sequence of a first and/or second strand of: AD-39781.1 (SEQ ID NOs: 268 and 695), or a modified or unmodified variant thereof.

A RNAi agent comprising a first and a second strand, wherein the sequence of the first and/or second strand comprise a sequence of 15 contiguous nt from the sequence of a first and/or second strand of: AD-39787.1 (SEQ ID NOs: 269 and 696), or a modified or unmodified variant thereof.

A RNAi agent comprising a first and a second strand, wherein the sequence of the first and/or second strand comprise a sequence of 15 contiguous nt from the sequence of a first and/or second strand of: AD-39793.1 (SEQ ID NOs: 270 and 697), or a modified or unmodified variant thereof.

A RNAi agent comprising a first and a second strand, wherein the sequence of the first and/or second strand comprise a sequence of 15 contiguous nt from the sequence of a first and/or second strand of: AD-39799.1 (SEQ ID NOs: 271 and 698), or a modified or unmodified variant thereof.

A RNAi agent comprising a first and a second strand, wherein the sequence of the first and/or second strand comprise a sequence of 15 contiguous nt from the sequence of a first and/or second strand of: AD-39805.1 (SEQ ID NOs: 272 and 699), or a modified or unmodified variant thereof.

A RNAi agent comprising a first and a second strand, wherein the sequence of the first and/or second strand comprise a sequence of 15 contiguous nt from the sequence of a first and/or second strand of: AD-39811.1 (SEQ ID NOs: 273 and 700), or a modified or unmodified variant thereof.

A RNAi agent comprising a first and a second strand, wherein the sequence of the first and/or second strand comprise a sequence of 15 contiguous nt from the sequence of a first and/or second strand of: AD-39817.1 (SEQ ID NOs: 274 and 701), or a modified or unmodified variant thereof.

A RNAi agent comprising a first and a second strand, wherein the sequence of the first and/or second strand comprise a sequence of 15 contiguous nt from the sequence of a first and/or second strand of: AD-39823.1 (SEQ ID NOs: 275 and 702), or a modified or unmodified variant thereof.

A RNAi agent comprising a first and a second strand, wherein the sequence of the first and/or second strand comprise a sequence of 15 contiguous nt from the sequence of a first and/or second strand of: AD-39782.1 (SEQ ID NOs: 276 and 703), or a modified or unmodified variant thereof.

A RNAi agent comprising a first and a second strand, wherein the sequence of the first and/or second strand comprise a sequence of 15 contiguous nt from the sequence of a first and/or second strand of: AD-39788.1 (SEQ ID NOs: 277 and 704), or a modified or unmodified variant thereof.

A RNAi agent comprising a first and a second strand, wherein the sequence of the first and/or second strand comprise a sequence of 15 contiguous nt from the sequence of a first and/or second strand of: AD-39794.1 (SEQ ID NOs: 278 and 705), or a modified or unmodified variant thereof.

A RNAi agent comprising a first and a second strand, wherein the sequence of the first and/or second strand comprise a sequence of 15 contiguous nt from the sequence of a first and/or second strand of: AD-39800.1 (SEQ ID NOs: 279 and 706), or a modified or unmodified variant thereof.

A RNAi agent comprising a first and a second strand, wherein the sequence of the first and/or second strand comprise a sequence of 15 contiguous nt from the sequence of a first and/or second strand of: AD-39806.1 (SEQ ID NOs: 280 and 707), or a modified or unmodified variant thereof.

A RNAi agent comprising a first and a second strand, wherein the sequence of the first and/or second strand comprise a sequence of 15 contiguous nt from the sequence of a first and/or second strand of: AD-39812.1 (SEQ ID NOs: 281 and 708), or a modified or unmodified variant thereof.

A RNAi agent comprising a first and a second strand, wherein the sequence of the first and/or second strand comprise a sequence of 15 contiguous nt from the sequence of a first and/or second strand of: AD-39818.1 (SEQ ID NOs: 282 and 709), or a modified or unmodified variant thereof.

A RNAi agent comprising a first and a second strand, wherein the sequence of the first and/or second strand comprise a sequence of 15 contiguous nt from the sequence of a first and/or second strand of: AD-39824.1 (SEQ ID NOs: 283 and 710), or a modified or unmodified variant thereof.

A RNAi agent comprising a first and a second strand, wherein the sequence of the first and/or second strand comprise a sequence of 15 contiguous nt from the sequence of a first and/or second strand of: AD-39783.1 (SEQ ID NOs: 284 and 711), or a modified or unmodified variant thereof.

A RNAi agent comprising a first and a second strand, wherein the sequence of the first and/or second strand comprise a sequence of 15 contiguous nt from the sequence of a first and/or second strand of: AD-39789.1 (SEQ ID NOs: 285 and 712), or a modified or unmodified variant thereof.

A RNAi agent comprising a first and a second strand, wherein the sequence of the first and/or second strand comprise a sequence of 15 contiguous nt from the sequence of a first and/or second strand of: AD-39795.1 (SEQ ID NOs: 286 and 713), or a modified or unmodified variant thereof.

A RNAi agent comprising a first and a second strand, wherein the sequence of the first and/or second strand comprise a sequence of 15 contiguous nt from the sequence of a first and/or second strand of: AD-39801.1 (SEQ ID NOs: 287 and 714), or a modified or unmodified variant thereof.

A RNAi agent comprising a first and a second strand, wherein the sequence of the first and/or second strand comprise a sequence of 15 contiguous nt from the sequence of a first and/or second strand of: AD-39807.1 (SEQ ID NOs: 288 and 715), or a modified or unmodified variant thereof.

A RNAi agent comprising a first and a second strand, wherein the sequence of the first and/or second strand comprise a sequence of 15 contiguous nt from the sequence of a first and/or second strand of: AD-39813.1 (SEQ ID NOs: 289 and 716), or a modified or unmodified variant thereof.

A RNAi agent comprising a first and a second strand, wherein the sequence of the first and/or second strand comprise a sequence of 15 contiguous nt from the sequence of a first and/or second strand of: AD-39819.1 (SEQ ID NOs: 290 and 717), or a modified or unmodified variant thereof.

A RNAi agent comprising a first and a second strand, wherein the sequence of the first and/or second strand comprise a sequence of 15 contiguous nt from the sequence of a first and/or second strand of: AD-39825.1 (SEQ ID NOs: 291 and 718), or a modified or unmodified variant thereof.

A RNAi agent comprising a first and a second strand, wherein the sequence of the first and/or second strand comprise a sequence of 15 contiguous nt from the sequence of a first and/or second strand of: AD-39831.1 (SEQ ID NOs: 292 and 719), or a modified or unmodified variant thereof.

A RNAi agent comprising a first and a second strand, wherein the sequence of the first and/or second strand comprise a sequence of 15 contiguous nt from the sequence of a first and/or second strand of: AD-39837.1 (SEQ ID NOs: 293 and 720), or a modified or unmodified variant thereof.

A RNAi agent comprising a first and a second strand, wherein the sequence of the first and/or second strand comprise a sequence of 15 contiguous nt from the sequence of a first and/or second strand of: AD-39843.1 (SEQ ID NOs: 294 and 721), or a modified or unmodified variant thereof.

A RNAi agent comprising a first and a second strand, wherein the sequence of the first and/or second strand comprise a sequence of 15 contiguous nt from the sequence of a first and/or second strand of: AD-39849.1 (SEQ ID NOs: 295 and 722), or a modified or unmodified variant thereof.

A RNAi agent comprising a first and a second strand, wherein the sequence of the first and/or second strand comprise a sequence of 15 contiguous nt from the sequence of a first and/or second strand of: AD-39855.1 (SEQ ID NOs: 296 and 723), or a modified or unmodified variant thereof.

A RNAi agent comprising a first and a second strand, wherein the sequence of the first and/or second strand comprise a sequence of 15 contiguous nt from the sequence of a first and/or second strand of: AD-39861.1 (SEQ ID NOs: 297 and 724), or a modified or unmodified variant thereof.

A RNAi agent comprising a first and a second strand, wherein the sequence of the first and/or second strand comprise a sequence of 15 contiguous nt from the sequence of a first and/or second strand of: AD-39867.1 (SEQ ID NOs: 298 and 725), or a modified or unmodified variant thereof.

A RNAi agent comprising a first and a second strand, wherein the sequence of the first and/or second strand comprise a sequence of 15 contiguous nt from the sequence of a first and/or second strand of: AD-39826.1 (SEQ ID NOs: 299 and 726), or a modified or unmodified variant thereof.

A RNAi agent comprising a first and a second strand, wherein the sequence of the first and/or second strand comprise a sequence of 15 contiguous nt from the sequence of a first and/or second strand of: AD-39832.1 (SEQ ID NOs: 300 and 727), or a modified or unmodified variant thereof.

A RNAi agent comprising a first and a second strand, wherein the sequence of the first and/or second strand comprise a sequence of 15 contiguous nt from the sequence of a first and/or second strand of: AD-39838.1 (SEQ ID NOs: 301 and 728), or a modified or unmodified variant thereof.

A RNAi agent comprising a first and a second strand, wherein the sequence of the first and/or second strand comprise a sequence of 15 contiguous nt from the sequence of a first and/or second strand of: AD-39844.1 (SEQ ID NOs: 302 and 729), or a modified or unmodified variant thereof.

A RNAi agent comprising a first and a second strand, wherein the sequence of the first and/or second strand comprise a sequence of 15 contiguous nt from the sequence of a first and/or second strand of: AD-39850.1 (SEQ ID NOs: 303 and 730), or a modified or unmodified variant thereof.

A RNAi agent comprising a first and a second strand, wherein the sequence of the first and/or second strand comprise a sequence of 15 contiguous nt from the sequence of a first and/or second strand of: AD-39856.1 (SEQ ID NOs: 304 and 731), or a modified or unmodified variant thereof.

A RNAi agent comprising a first and a second strand, wherein the sequence of the first and/or second strand comprise a sequence of 15 contiguous nt from the sequence of a first and/or second strand of: AD-39862.1 (SEQ ID NOs: 305 and 732), or a modified or unmodified variant thereof.

A RNAi agent comprising a first and a second strand, wherein the sequence of the first and/or second strand comprise a sequence of 15 contiguous nt from the sequence of a first and/or second strand of: AD-39868.1 (SEQ ID NOs: 306 and 733), or a modified or unmodified variant thereof.

A RNAi agent comprising a first and a second strand, wherein the sequence of the first and/or second strand comprise a sequence of 15 contiguous nt from the sequence of a first and/or second strand of: AD-39827.1 (SEQ ID NOs: 307 and 734), or a modified or unmodified variant thereof.

A RNAi agent comprising a first and a second strand, wherein the sequence of the first and/or second strand comprise a sequence of 15 contiguous nt from the sequence of a first and/or second strand of: AD-39833.1 (SEQ ID NOs: 308 and 735), or a modified or unmodified variant thereof.

A RNAi agent comprising a first and a second strand, wherein the sequence of the first and/or second strand comprise a sequence of 15 contiguous nt from the sequence of a first and/or second strand of: AD-39839.1 (SEQ ID NOs: 309 and 736), or a modified or unmodified variant thereof.

A RNAi agent comprising a first and a second strand, wherein the sequence of the first and/or second strand comprise a sequence of 15 contiguous nt from the sequence of a first and/or second strand of: AD-39845.1 (SEQ ID NOs: 310 and 737), or a modified or unmodified variant thereof.

A RNAi agent comprising a first and a second strand, wherein the sequence of the first and/or second strand comprise a sequence of 15 contiguous nt from the sequence of a first and/or second strand of: AD-39851.1 (SEQ ID NOs: 311 and 738), or a modified or unmodified variant thereof.

A RNAi agent comprising a first and a second strand, wherein the sequence of the first and/or second strand comprise a sequence of 15 contiguous nt from the sequence of a first and/or second strand of: AD-39857.1 (SEQ ID NOs: 312 and 739), or a modified or unmodified variant thereof.

A RNAi agent comprising a first and a second strand, wherein the sequence of the first and/or second strand comprise a sequence of 15 contiguous nt from the sequence of a first and/or second strand of: AD-39863.1 (SEQ ID NOs: 313 and 740), or a modified or unmodified variant thereof.

A RNAi agent comprising a first and a second strand, wherein the sequence of the first and/or second strand comprise a sequence of 15 contiguous nt from the sequence of a first and/or second strand of: AD-39869.1 (SEQ ID NOs: 314 and 741), or a modified or unmodified variant thereof.

A RNAi agent comprising a first and a second strand, wherein the sequence of the first and/or second strand comprise a sequence of 15 contiguous nt from the sequence of a first and/or second strand of: AD-39828.1 (SEQ ID NOs: 315 and 742), or a modified or unmodified variant thereof.

A RNAi agent comprising a first and a second strand, wherein the sequence of the first and/or second strand comprise a sequence of 15 contiguous nt from the sequence of a first and/or second strand of: AD-39834.1 (SEQ ID NOs: 316 and 743), or a modified or unmodified variant thereof.

A RNAi agent comprising a first and a second strand, wherein the sequence of the first and/or second strand comprise a sequence of 15 contiguous nt from the sequence of a first and/or second strand of: AD-39840.1 (SEQ ID NOs: 317 and 744), or a modified or unmodified variant thereof.

A RNAi agent comprising a first and a second strand, wherein the sequence of the first and/or second strand comprise a sequence of 15 contiguous nt from the sequence of a first and/or second strand of: AD-39846.1 (SEQ ID NOs: 318 and 745), or a modified or unmodified variant thereof.

A RNAi agent comprising a first and a second strand, wherein the sequence of the first and/or second strand comprise a sequence of 15 contiguous nt from the sequence of a first and/or second strand of: AD-39852.1 (SEQ ID NOs: 319 and 746), or a modified or unmodified variant thereof.

A RNAi agent comprising a first and a second strand, wherein the sequence of the first and/or second strand comprise a sequence of 15 contiguous nt from the sequence of a first and/or second strand of: AD-39858.1 (SEQ ID NOs: 320 and 747), or a modified or unmodified variant thereof.

A RNAi agent comprising a first and a second strand, wherein the sequence of the first and/or second strand comprise a sequence of 15 contiguous nt from the sequence of a first and/or second strand of: AD-39864.1 (SEQ ID NOs: 321 and 748), or a modified or unmodified variant thereof.

A RNAi agent comprising a first and a second strand, wherein the sequence of the first and/or second strand comprise a sequence of 15 contiguous nt from the sequence of a first and/or second strand of: AD-39870.1 (SEQ ID NOs: 322 and 749), or a modified or unmodified variant thereof.

A RNAi agent comprising a first and a second strand, wherein the sequence of the first and/or second strand comprise a sequence of 15 contiguous nt from the sequence of a first and/or second strand of: AD-39829.1 (SEQ ID NOs: 323 and 750), or a modified or unmodified variant thereof.

A RNAi agent comprising a first and a second strand, wherein the sequence of the first and/or second strand comprise a sequence of 15 contiguous nt from the sequence of a first and/or second strand of: AD-39835.1 (SEQ ID NOs: 324 and 751), or a modified or unmodified variant thereof.

A RNAi agent comprising a first and a second strand, wherein the sequence of the first and/or second strand comprise a sequence of 15 contiguous nt from the sequence of a first and/or second strand of: AD-39841.1 (SEQ ID NOs: 325 and 752), or a modified or unmodified variant thereof.

A RNAi agent comprising a first and a second strand, wherein the sequence of the first and/or second strand comprise a sequence of 15 contiguous nt from the sequence of a first and/or second strand of: AD-39853.1 (SEQ ID NOs: 326 and 753), or a modified or unmodified variant thereof.

A RNAi agent comprising a first and a second strand, wherein the sequence of the first and/or second strand comprise a sequence of 15 contiguous nt from the sequence of a first and/or second strand of: AD-39859.1 (SEQ ID NOs: 327 and 754), or a modified or unmodified variant thereof.

A RNAi agent comprising a first and a second strand, wherein the sequence of the first and/or second strand comprise a sequence of 15 contiguous nt from the sequence of a first and/or second strand of: AD-39865.1 (SEQ ID NOs: 328 and 755), or a modified or unmodified variant thereof.

A RNAi agent comprising a first and a second strand, wherein the sequence of the first and/or second strand comprise a sequence of 15 contiguous nt from the sequence of a first and/or second strand of: AD-39871.1 (SEQ ID NOs: 329 and 756), or a modified or unmodified variant thereof.

A RNAi agent comprising a first and a second strand, wherein the sequence of the first and/or second strand comprise a sequence of 15 contiguous nt from the sequence of a first and/or second strand of: AD-39830.1 (SEQ ID NOs: 330 and 757), or a modified or unmodified variant thereof.

A RNAi agent comprising a first and a second strand, wherein the sequence of the first and/or second strand comprise a sequence of 15 contiguous nt from the sequence of a first and/or second strand of: AD-39836.1 (SEQ ID NOs: 331 and 758), or a modified or unmodified variant thereof.

A RNAi agent comprising a first and a second strand, wherein the sequence of the first and/or second strand comprise a sequence of 15 contiguous nt from the sequence of a first and/or second strand of: AD-39842.1 (SEQ ID NOs: 332 and 759), or a modified or unmodified variant thereof.

A RNAi agent comprising a first and a second strand, wherein the sequence of the first and/or second strand comprise a sequence of 15 contiguous nt from the sequence of a first and/or second strand of: AD-39848.1 (SEQ ID NOs: 333 and 760), or a modified or unmodified variant thereof.

A RNAi agent comprising a first and a second strand, wherein the sequence of the first and/or second strand comprise a sequence of 15 contiguous nt from the sequence of a first and/or second strand of: AD-39854.1 (SEQ ID NOs: 334 and 761), or a modified or unmodified variant thereof.

A RNAi agent comprising a first and a second strand, wherein the sequence of the first and/or second strand comprise a sequence of 15 contiguous nt from the sequence of a first and/or second strand of: AD-39860.1 (SEQ ID NOs: 335 and 762), or a modified or unmodified variant thereof.

A RNAi agent comprising a first and a second strand, wherein the sequence of the first and/or second strand comprise a sequence of 15 contiguous nt from the sequence of a first and/or second strand of: AD-39866.1 (SEQ ID NOs: 336 and 763), or a modified or unmodified variant thereof.

A RNAi agent comprising a first and a second strand, wherein the sequence of the first and/or second strand comprise a sequence of 15 contiguous nt from the sequence of a first and/or second strand of: AD-39992.1 (SEQ ID NOs: 337 and 764), or a modified or unmodified variant thereof.

A RNAi agent comprising a first and a second strand, wherein the sequence of the first and/or second strand comprise a sequence of 15 contiguous nt from the sequence of a first and/or second strand of: AD-39998.1 (SEQ ID NOs: 338 and 765), or a modified or unmodified variant thereof.

A RNAi agent comprising a first and a second strand, wherein the sequence of the first and/or second strand comprise a sequence of 15 contiguous nt from the sequence of a first and/or second strand of: AD-40004.1 (SEQ ID NOs: 339 and 766), or a modified or unmodified variant thereof.

A RNAi agent comprising a first and a second strand, wherein the sequence of the first and/or second strand comprise a sequence of 15 contiguous nt from the sequence of a first and/or second strand of: AD-40010.1 (SEQ ID NOs: 340 and 767), or a modified or unmodified variant thereof.

A RNAi agent comprising a first and a second strand, wherein the sequence of the first and/or second strand comprise a sequence of 15 contiguous nt from the sequence of a first and/or second strand of: AD-40016.1 (SEQ ID NOs: 341 and 768), or a modified or unmodified variant thereof.

A RNAi agent comprising a first and a second strand, wherein the sequence of the first and/or second strand comprise a sequence of 15 contiguous nt from the sequence of a first and/or second strand of: AD-40022.1 (SEQ ID NOs: 342 and 769), or a modified or unmodified variant thereof.

A RNAi agent comprising a first and a second strand, wherein the sequence of the first and/or second strand comprise a sequence of 15 contiguous nt from the sequence of a first and/or second strand of: AD-40028.1 (SEQ ID NOs: 343 and 770), or a modified or unmodified variant thereof.

A RNAi agent comprising a first and a second strand, wherein the sequence of the first and/or second strand comprise a sequence of 15 contiguous nt from the sequence of a first and/or second strand of: AD-40034.1 (SEQ ID NOs: 344 and 771), or a modified or unmodified variant thereof.

A RNAi agent comprising a first and a second strand, wherein the sequence of the first and/or second strand comprise a sequence of 15 contiguous nt from the sequence of a first and/or second strand of: AD-39999.1 (SEQ ID NOs: 345 and 772), or a modified or unmodified variant thereof.

A RNAi agent comprising a first and a second strand, wherein the sequence of the first and/or second strand comprise a sequence of 15 contiguous nt from the sequence of a first and/or second strand of: AD-40005.1 (SEQ ID NOs: 346 and 773), or a modified or unmodified variant thereof.

A RNAi agent comprising a first and a second strand, wherein the sequence of the first and/or second strand comprise a sequence of 15 contiguous nt from the sequence of a first and/or second strand of: AD-40011.1 (SEQ ID NOs: 347 and 774), or a modified or unmodified variant thereof.

A RNAi agent comprising a first and a second strand, wherein the sequence of the first and/or second strand comprise a sequence of 15 contiguous nt from the sequence of a first and/or second strand of: AD-40017.1 (SEQ ID NOs: 348 and 775), or a modified or unmodified variant thereof.

A RNAi agent comprising a first and a second strand, wherein the sequence of the first and/or second strand comprise a sequence of 15 contiguous nt from the sequence of a first and/or second strand of: AD-40029.1 (SEQ ID NOs: 349 and 776), or a modified or unmodified variant thereof.

A RNAi agent comprising a first and a second strand, wherein the sequence of the first and/or second strand comprise a sequence of 15 contiguous nt from the sequence of a first and/or second strand of: AD-40035.1 (SEQ ID NOs: 350 and 777), or a modified or unmodified variant thereof.

A RNAi agent comprising a first and a second strand, wherein the sequence of the first and/or second strand comprise a sequence of 15 contiguous nt from the sequence of a first and/or second strand of: AD-39994.1 (SEQ ID NOs: 351 and 778), or a modified or unmodified variant thereof.

A RNAi agent comprising a first and a second strand, wherein the sequence of the first and/or second strand comprise a sequence of 15 contiguous nt from the sequence of a first and/or second strand of: AD-40000.1 (SEQ ID NOs: 352 and 779), or a modified or unmodified variant thereof.

A RNAi agent comprising a first and a second strand, wherein the sequence of the first and/or second strand comprise a sequence of 15 contiguous nt from the sequence of a first and/or second strand of: AD-40006.1 (SEQ ID NOs: 353 and 780), or a modified or unmodified variant thereof.

A RNAi agent comprising a first and a second strand, wherein the sequence of the first and/or second strand comprise a sequence of 15 contiguous nt from the sequence of a first and/or second strand of: AD-40012.1 (SEQ ID NOs: 354 and 781), or a modified or unmodified variant thereof.

A RNAi agent comprising a first and a second strand, wherein the sequence of the first and/or second strand comprise a sequence of 15 contiguous nt from the sequence of a first and/or second strand of: AD-40018.1 (SEQ ID NOs: 355 and 782), or a modified or unmodified variant thereof.

A RNAi agent comprising a first and a second strand, wherein the sequence of the first and/or second strand comprise a sequence of 15 contiguous nt from the sequence of a first and/or second strand of: AD-40024.1 (SEQ ID NOs: 356 and 783), or a modified or unmodified variant thereof.

A RNAi agent comprising a first and a second strand, wherein the sequence of the first and/or second strand comprise a sequence of 15 contiguous nt from the sequence of a first and/or second strand of: AD-40030.1 (SEQ ID NOs: 357 and 784), or a modified or unmodified variant thereof.

A RNAi agent comprising a first and a second strand, wherein the sequence of the first and/or second strand comprise a sequence of 15 contiguous nt from the sequence of a first and/or second strand of: AD-40036.1 (SEQ ID NOs: 358 and 785), or a modified or unmodified variant thereof.

A RNAi agent comprising a first and a second strand, wherein the sequence of the first and/or second strand comprise a sequence of 15 contiguous nt from the sequence of a first and/or second strand of: AD-39995.1 (SEQ ID NOs: 359 and 786), or a modified or unmodified variant thereof.

A RNAi agent comprising a first and a second strand, wherein the sequence of the first and/or second strand comprise a sequence of 15 contiguous nt from the sequence of a first and/or second strand of: AD-40001.1 (SEQ ID NOs: 360 and 787), or a modified or unmodified variant thereof.

A RNAi agent comprising a first and a second strand, wherein the sequence of the first and/or second strand comprise a sequence of 15 contiguous nt from the sequence of a first and/or second strand of: AD-40007.1 (SEQ ID NOs: 361 and 788), or a modified or unmodified variant thereof.

A RNAi agent comprising a first and a second strand, wherein the sequence of the first and/or second strand comprise a sequence of 15 contiguous nt from the sequence of a first and/or second strand of: AD-40013.1 (SEQ ID NOs: 362 and 789), or a modified or unmodified variant thereof.

A RNAi agent comprising a first and a second strand, wherein the sequence of the first and/or second strand comprise a sequence of 15 contiguous nt from the sequence of a first and/or second strand of: AD-40019.1 (SEQ ID NOs: 363 and 790), or a modified or unmodified variant thereof.

A RNAi agent comprising a first and a second strand, wherein the sequence of the first and/or second strand comprise a sequence of 15 contiguous nt from the sequence of a first and/or second strand of: AD-40025.1 (SEQ ID NOs: 364 and 791), or a modified or unmodified variant thereof.

A RNAi agent comprising a first and a second strand, wherein the sequence of the first and/or second strand comprise a sequence of 15 contiguous nt from the sequence of a first and/or second strand of: AD-40031.1 (SEQ ID NOs: 365 and 792), or a modified or unmodified variant thereof.

A RNAi agent comprising a first and a second strand, wherein the sequence of the first and/or second strand comprise a sequence of 15 contiguous nt from the sequence of a first and/or second strand of: AD-40037.1 (SEQ ID NOs: 366 and 793), or a modified or unmodified variant thereof.

A RNAi agent comprising a first and a second strand, wherein the sequence of the first and/or second strand comprise a sequence of 15 contiguous nt from the sequence of a first and/or second strand of: AD-39996.1 (SEQ ID NOs: 367 and 794), or a modified or unmodified variant thereof.

A RNAi agent comprising a first and a second strand, wherein the sequence of the first and/or second strand comprise a sequence of 15 contiguous nt from the sequence of a first and/or second strand of: AD-40002.1 (SEQ ID NOs: 368 and 795), or a modified or unmodified variant thereof.

A RNAi agent comprising a first and a second strand, wherein the sequence of the first and/or second strand comprise a sequence of 15 contiguous nt from the sequence of a first and/or second strand of: AD-40008.1 (SEQ ID NOs: 369 and 796), or a modified or unmodified variant thereof.

A RNAi agent comprising a first and a second strand, wherein the sequence of the first and/or second strand comprise a sequence of 15 contiguous nt from the sequence of a first and/or second strand of: AD-40014.1 (SEQ ID NOs: 370 and 797), or a modified or unmodified variant thereof.

A RNAi agent comprising a first and a second strand, wherein the sequence of the first and/or second strand comprise a sequence of 15 contiguous nt from the sequence of a first and/or second strand of: AD-40020.1 (SEQ ID NOs: 371 and 798), or a modified or unmodified variant thereof.

A RNAi agent comprising a first and a second strand, wherein the sequence of the first and/or second strand comprise a sequence of 15 contiguous nt from the sequence of a first and/or second strand of: AD-40026.1 (SEQ ID NOs: 372 and 799), or a modified or unmodified variant thereof.

A RNAi agent comprising a first and a second strand, wherein the sequence of the first and/or second strand comprise a sequence of 15 contiguous nt from the sequence of a first and/or second strand of: AD-40032.1 (SEQ ID NOs: 373 and 800), or a modified or unmodified variant thereof.

A RNAi agent comprising a first and a second strand, wherein the sequence of the first and/or second strand comprise a sequence of 15 contiguous nt from the sequence of a first and/or second strand of: AD-40038.1 (SEQ ID NOs: 374 and 801), or a modified or unmodified variant thereof.

A RNAi agent comprising a first and a second strand, wherein the sequence of the first and/or second strand comprise a sequence of 15 contiguous nt from the sequence of a first and/or second strand of: AD-39997.1 (SEQ ID NOs: 375 and 802), or a modified or unmodified variant thereof.

A RNAi agent comprising a first and a second strand, wherein the sequence of the first and/or second strand comprise a sequence of 15 contiguous nt from the sequence of a first and/or second strand of: AD-40009.1 (SEQ ID NOs: 376 and 803), or a modified or unmodified variant thereof.

A RNAi agent comprising a first and a second strand, wherein the sequence of the first and/or second strand comprise a sequence of 15 contiguous nt from the sequence of a first and/or second strand of: AD-40015.1 (SEQ ID NOs: 377 and 804), or a modified or unmodified variant thereof.

A RNAi agent comprising a first and a second strand, wherein the sequence of the first and/or second strand comprise a sequence of 15 contiguous nt from the sequence of a first and/or second strand of: AD-40021.1 (SEQ ID NOs: 378 and 805), or a modified or unmodified variant thereof.

A RNAi agent comprising a first and a second strand, wherein the sequence of the first and/or second strand comprise a sequence of 15 contiguous nt from the sequence of a first and/or second strand of: AD-40027.1 (SEQ ID NOs: 379 and 806), or a modified or unmodified variant thereof.

A RNAi agent comprising a first and a second strand, wherein the sequence of the first and/or second strand comprise a sequence of 15 contiguous nt from the sequence of a first and/or second strand of: AD-40033.1 (SEQ ID NOs: 380 and 807), or a modified or unmodified variant thereof.

A RNAi agent comprising a first and a second strand, wherein the sequence of the first and/or second strand comprise a sequence of 15 contiguous nt from the sequence of a first and/or second strand of: AD-40039.1 (SEQ ID NOs: 381 and 808), or a modified or unmodified variant thereof.

A RNAi agent comprising a first and a second strand, wherein the sequence of the first and/or second strand comprise a sequence of 15 contiguous nt from the sequence of a first and/or second strand of: AD-40045.1 (SEQ ID NOs: 382 and 809), or a modified or unmodified variant thereof.

A RNAi agent comprising a first and a second strand, wherein the sequence of the first and/or second strand comprise a sequence of 15 contiguous nt from the sequence of a first and/or second strand of: AD-40051.1 (SEQ ID NOs: 383 and 810), or a modified or unmodified variant thereof.

A RNAi agent comprising a first and a second strand, wherein the sequence of the first and/or second strand comprise a sequence of 15 contiguous nt from the sequence of a first and/or second strand of: AD-40057.1 (SEQ ID NOs: 384 and 811), or a modified or unmodified variant thereof.

A RNAi agent comprising a first and a second strand, wherein the sequence of the first and/or second strand comprise a sequence of 15 contiguous nt from the sequence of a first and/or second strand of: AD-40063.1 (SEQ ID NOs: 385 and 812), or a modified or unmodified variant thereof.

A RNAi agent comprising a first and a second strand, wherein the sequence of the first and/or second strand comprise a sequence of 15 contiguous nt from the sequence of a first and/or second strand of: AD-40069.1 (SEQ ID NOs: 386 and 813), or a modified or unmodified variant thereof.

A RNAi agent comprising a first and a second strand, wherein the sequence of the first and/or second strand comprise a sequence of 15 contiguous nt from the sequence of a first and/or second strand of: AD-40075.1 (SEQ ID NOs: 387 and 814), or a modified or unmodified variant thereof.

A RNAi agent comprising a first and a second strand, wherein the sequence of the first and/or second strand comprise a sequence of 15 contiguous nt from the sequence of a first and/or second strand of: AD-40081.1 (SEQ ID NOs: 388 and 815), or a modified or unmodified variant thereof.

A RNAi agent comprising a first and a second strand, wherein the sequence of the first and/or second strand comprise a sequence of 15 contiguous nt from the sequence of a first and/or second strand of: AD-40040.1 (SEQ ID NOs: 389 and 816), or a modified or unmodified variant thereof.

A RNAi agent comprising a first and a second strand, wherein the sequence of the first and/or second strand comprise a sequence of 15 contiguous nt from the sequence of a first and/or second strand of: AD-40046.1 (SEQ ID NOs: 390 and 817), or a modified or unmodified variant thereof.

A RNAi agent comprising a first and a second strand, wherein the sequence of the first and/or second strand comprise a sequence of 15 contiguous nt from the sequence of a first and/or second strand of: AD-40052.1 (SEQ ID NOs: 391 and 818), or a modified or unmodified variant thereof.

A RNAi agent comprising a first and a second strand, wherein the sequence of the first and/or second strand comprise a sequence of 15 contiguous nt from the sequence of a first and/or second strand of: AD-40058.1 (SEQ ID NOs: 392 and 819), or a modified or unmodified variant thereof.

A RNAi agent comprising a first and a second strand, wherein the sequence of the first and/or second strand comprise a sequence of 15 contiguous nt from the sequence of a first and/or second strand of: AD-40064.1 (SEQ ID NOs: 393 and 820), or a modified or unmodified variant thereof.

A RNAi agent comprising a first and a second strand, wherein the sequence of the first and/or second strand comprise a sequence of 15 contiguous nt from the sequence of a first and/or second strand of: AD-40070.1 (SEQ ID NOs: 394 and 821), or a modified or unmodified variant thereof.

A RNAi agent comprising a first and a second strand, wherein the sequence of the first and/or second strand comprise a sequence of 15 contiguous nt from the sequence of a first and/or second strand of: AD-40076.1 (SEQ ID NOs: 395 and 822), or a modified or unmodified variant thereof.

A RNAi agent comprising a first and a second strand, wherein the sequence of the first and/or second strand comprise a sequence of 15 contiguous nt from the sequence of a first and/or second strand of: AD-40082.1 (SEQ ID NOs: 396 and 823), or a modified or unmodified variant thereof.

A RNAi agent comprising a first and a second strand, wherein the sequence of the first and/or second strand comprise a sequence of 15 contiguous nt from the sequence of a first and/or second strand of: AD-40041.1 (SEQ ID NOs: 397 and 824), or a modified or unmodified variant thereof.

A RNAi agent comprising a first and a second strand, wherein the sequence of the first and/or second strand comprise a sequence of 15 contiguous nt from the sequence of a first and/or second strand of: AD-40047.1 (SEQ ID NOs: 398 and 825), or a modified or unmodified variant thereof.

A RNAi agent comprising a first and a second strand, wherein the sequence of the first and/or second strand comprise a sequence of 15 contiguous nt from the sequence of a first and/or second strand of: AD-40053.1 (SEQ ID NOs: 399 and 826), or a modified or unmodified variant thereof.

A RNAi agent comprising a first and a second strand, wherein the sequence of the first and/or second strand comprise a sequence of 15 contiguous nt from the sequence of a first and/or second strand of: AD-40059.1 (SEQ ID NOs: 400 and 827), or a modified or unmodified variant thereof.

A RNAi agent comprising a first and a second strand, wherein the sequence of the first and/or second strand comprise a sequence of 15 contiguous nt from the sequence of a first and/or second strand of: AD-40065.1 (SEQ ID NOs: 401 and 828), or a modified or unmodified variant thereof.

A RNAi agent comprising a first and a second strand, wherein the sequence of the first and/or second strand comprise a sequence of 15 contiguous nt from the sequence of a first and/or second strand of: AD-40071.1 (SEQ ID NOs: 402 and 829), or a modified or unmodified variant thereof.

A RNAi agent comprising a first and a second strand, wherein the sequence of the first and/or second strand comprise a sequence of 15 contiguous nt from the sequence of a first and/or second strand of: AD-40077.1 (SEQ ID NOs: 403 and 830), or a modified or unmodified variant thereof.

A RNAi agent comprising a first and a second strand, wherein the sequence of the first and/or second strand comprise a sequence of 15 contiguous nt from the sequence of a first and/or second strand of: AD-40083.1 (SEQ ID NOs: 404 and 831), or a modified or unmodified variant thereof.

A RNAi agent comprising a first and a second strand, wherein the sequence of the first and/or second strand comprise a sequence of 15 contiguous nt from the sequence of a first and/or second strand of: AD-40042.1 (SEQ ID NOs: 405 and 832), or a modified or unmodified variant thereof.

A RNAi agent comprising a first and a second strand, wherein the sequence of the first and/or second strand comprise a sequence of 15 contiguous nt from the sequence of a first and/or second strand of: AD-40048.1 (SEQ ID NOs: 406 and 833), or a modified or unmodified variant thereof.

A RNAi agent comprising a first and a second strand, wherein the sequence of the first and/or second strand comprise a sequence of 15 contiguous nt from the sequence of a first and/or second strand of: AD-40054.1 (SEQ ID NOs: 407 and 834), or a modified or unmodified variant thereof.

A RNAi agent comprising a first and a second strand, wherein the sequence of the first and/or second strand comprise a sequence of 15 contiguous nt from the sequence of a first and/or second strand of: AD-40060.1 (SEQ ID NOs: 408 and 835), or a modified or unmodified variant thereof.

A RNAi agent comprising a first and a second strand, wherein the sequence of the first and/or second strand comprise a sequence of 15 contiguous nt from the sequence of a first and/or second strand of: AD-40066.1 (SEQ ID NOs: 409 and 836), or a modified or unmodified variant thereof.

A RNAi agent comprising a first and a second strand, wherein the sequence of the first and/or second strand comprise a sequence of 15 contiguous nt from the sequence of a first and/or second strand of: AD-40072.1 (SEQ ID NOs: 410 and 837), or a modified or unmodified variant thereof.

A RNAi agent comprising a first and a second strand, wherein the sequence of the first and/or second strand comprise a sequence of 15 contiguous nt from the sequence of a first and/or second strand of: AD-40078.1 (SEQ ID NOs: 411 and 838), or a modified or unmodified variant thereof.

A RNAi agent comprising a first and a second strand, wherein the sequence of the first and/or second strand comprise a sequence of 15 contiguous nt from the sequence of a first and/or second strand of: AD-40084.1 (SEQ ID NOs: 412 and 839), or a modified or unmodified variant thereof.

A RNAi agent comprising a first and a second strand, wherein the sequence of the first and/or second strand comprise a sequence of 15 contiguous nt from the sequence of a first and/or second strand of: AD-40043.1 (SEQ ID NOs: 413 and 840), or a modified or unmodified variant thereof.

A RNAi agent comprising a first and a second strand, wherein the sequence of the first and/or second strand comprise a sequence of 15 contiguous nt from the sequence of a first and/or second strand of: AD-40049.1 (SEQ ID NOs: 414 and 841), or a modified or unmodified variant thereof.

A RNAi agent comprising a first and a second strand, wherein the sequence of the first and/or second strand comprise a sequence of 15 contiguous nt from the sequence of a first and/or second strand of: AD-40055.1 (SEQ ID NOs: 415 and 842), or a modified or unmodified variant thereof.

A RNAi agent comprising a first and a second strand, wherein the sequence of the first and/or second strand comprise a sequence of 15 contiguous nt from the sequence of a first and/or second strand of: AD-40061.1 (SEQ ID NOs: 416 and 843), or a modified or unmodified variant thereof.

A RNAi agent comprising a first and a second strand, wherein the sequence of the first and/or second strand comprise a sequence of 15 contiguous nt from the sequence of a first and/or second strand of: AD-40067.1 (SEQ ID NOs: 417 and 844), or a modified or unmodified variant thereof.

A RNAi agent comprising a first and a second strand, wherein the sequence of the first and/or second strand comprise a sequence of 15 contiguous nt from the sequence of a first and/or second strand of: AD-40073.1 (SEQ ID NOs: 418 and 845), or a modified or unmodified variant thereof.

A RNAi agent comprising a first and a second strand, wherein the sequence of the first and/or second strand comprise a sequence of 15 contiguous nt from the sequence of a first and/or second strand of: AD-40079.1 (SEQ ID NOs: 419 and 846), or a modified or unmodified variant thereof.

A RNAi agent comprising a first and a second strand, wherein the sequence of the first and/or second strand comprise a sequence of 15 contiguous nt from the sequence of a first and/or second strand of: AD-40085.1 (SEQ ID NOs: 420 and 847), or a modified or unmodified variant thereof.

A RNAi agent comprising a first and a second strand, wherein the sequence of the first and/or second strand comprise a sequence of 15 contiguous nt from the sequence of a first and/or second strand of: AD-40044.1 (SEQ ID NOs: 421 and 848), or a modified or unmodified variant thereof.

A RNAi agent comprising a first and a second strand, wherein the sequence of the first and/or second strand comprise a sequence of 15 contiguous nt from the sequence of a first and/or second strand of: AD-40050.1 (SEQ ID NOs: 422 and 849), or a modified or unmodified variant thereof.

A RNAi agent comprising a first and a second strand, wherein the sequence of the first and/or second strand comprise a sequence of 15 contiguous nt from the sequence of a first and/or second strand of: AD-40056.1 (SEQ ID NOs: 423 and 850), or a modified or unmodified variant thereof.

A RNAi agent comprising a first and a second strand, wherein the sequence of the first and/or second strand comprise a sequence of 15 contiguous nt from the sequence of a first and/or second strand of: AD-40062.1 (SEQ ID NOs: 424 and 851), or a modified or unmodified variant thereof.

A RNAi agent comprising a first and a second strand, wherein the sequence of the first and/or second strand comprise a sequence of 15 contiguous nt from the sequence of a first and/or second strand of: AD-40068.1 (SEQ ID NOs: 425 and 852), or a modified or unmodified variant thereof.

A RNAi agent comprising a first and a second strand, wherein the sequence of the first and/or second strand comprise a sequence of 15 contiguous nt from the sequence of a first and/or second strand of: AD-40074.1 (SEQ ID NOs: 426 and 853), or a modified or unmodified variant thereof.

A RNAi agent comprising a first and a second strand, wherein the sequence of the first and/or second strand comprise a sequence of 15 contiguous nt from the sequence of a first and/or second strand of: AD-40080.1 (SEQ ID NOs: 427 and 854), or a modified or unmodified variant thereof.

A RNAi agent comprising a first and a second strand, wherein the sequence of the first and/or second strand comprise a sequence of 15 contiguous nt from the sequence of a first and/or second strand of: AD-39720.1 (SEQ ID NOs: 1716 and 1754), or a modified or unmodified variant thereof.

A RNAi agent comprising a first and a second strand, wherein the sequence of the first and/or second strand comprise a sequence of 15 contiguous nt from the sequence of a first and/or second strand of: AD-39706.1 (SEQ ID NOs: 1717 and 1755), or a modified or unmodified variant thereof.

A RNAi agent comprising a first and a second strand, wherein the sequence of the first and/or second strand comprise a sequence of 15 contiguous nt from the sequence of a first and/or second strand of: AD-39712.1 (SEQ ID NOs: 1718 and 1756), or a modified or unmodified variant thereof.

A RNAi agent comprising a first and a second strand, wherein the sequence of the first and/or second strand comprise a sequence of 15 contiguous nt from the sequence of a first and/or second strand of: AD-39760.1 (SEQ ID NOs: 1719 and 1757), or a modified or unmodified variant thereof.

A RNAi agent comprising a first and a second strand, wherein the sequence of the first and/or second strand comprise a sequence of 15 contiguous nt from the sequence of a first and/or second strand of: AD-39732.1 (SEQ ID NOs: 1720 and 1758), or a modified or unmodified variant thereof.

A RNAi agent comprising a first and a second strand, wherein the sequence of the first and/or second strand comprise a sequence of 15 contiguous nt from the sequence of a first and/or second strand of: AD-35541.4 (SEQ ID NOs: 1721 and 1759), or a modified or unmodified variant thereof.

A RNAi agent comprising a first and a second strand, wherein the sequence of the first and/or second strand comprise a sequence of 15 contiguous nt from the sequence of a first and/or second strand of: AD-39735.1 or hs_KRAS_321_A22S26 (SEQ ID NOs: 1722 and 1760), or a modified or unmodified variant thereof.

A RNAi agent comprising a first and a second strand, wherein the sequence of the first and/or second strand comprise a sequence of 15 contiguous nt from the sequence of a first and/or second strand of: AD-39741.1, or a modified or unmodified variant thereof.

A RNAi agent comprising a first and a second strand, wherein the sequence of the first and/or second strand comprise a sequence of 15 contiguous nt from the sequence of a first and/or second strand of: hs_KRAS_ 322_A22S26 (SEQ ID NOs: 1723 and 1761), or a modified or unmodified variant thereof.

A RNAi agent comprising a first and a second strand, wherein the sequence of the first and/or second strand comprise a sequence of 15 contiguous nt from the sequence of a first and/or second strand of: AD-39778.1 (SEQ ID NOs: 1724 and 1762), or a modified or unmodified variant thereof.

A RNAi agent comprising a first and a second strand, wherein the sequence of the first and/or second strand comprise a sequence of 15 contiguous nt from the sequence of a first and/or second strand of: AD-39790.1 (SEQ ID NOs: 1725 and 1763), or a modified or unmodified variant thereof.

A RNAi agent comprising a first and a second strand, wherein the sequence of the first and/or second strand comprise a sequence of 15 contiguous nt from the sequence of a first and/or second strand of: AD-39822.1 (SEQ ID NOs: 1726 and 1764), or a modified or unmodified variant thereof.

A RNAi agent comprising a first and a second strand, wherein the sequence of the first and/or second strand comprise a sequence of 15 contiguous nt from the sequence of a first and/or second strand of: AD-35609.4 (SEQ ID NOs: 1727 and 1765), or a modified or unmodified variant thereof.

A RNAi agent comprising a first and a second strand, wherein the sequence of the first and/or second strand comprise a sequence of 15 contiguous nt from the sequence of a first and/or second strand of: AD-35581.4 (SEQ ID NOs: 1728 and 1766), or a modified or unmodified variant thereof.

A RNAi agent comprising a first and a second strand, wherein the sequence of the first and/or second strand comprise a sequence of 15 contiguous nt from the sequence of a first and/or second strand of: AD-39845.1 (SEQ ID NOs: 1729 and 1767), or a modified or unmodified variant thereof.

A RNAi agent comprising a first and a second strand, wherein the sequence of the first and/or second strand comprise a sequence of 15 contiguous nt from the sequence of a first and/or second strand of: AD-39858.1 (SEQ ID NOs: 1730 and 1768), or a modified or unmodified variant thereof.

A RNAi agent comprising a first and a second strand, wherein the sequence of the first and/or second strand comprise a sequence of 15 contiguous nt from the sequence of a first and/or second strand of: AD-39870.1 (SEQ ID NOs: 1731 and 1769), or a modified or unmodified variant thereof.

A RNAi agent comprising a first and a second strand, wherein the sequence of the first and/or second strand comprise a sequence of 15 contiguous nt from the sequence of a first and/or second strand of: AD-35576.4 (SEQ ID NOs: 1732 and 1770), or a modified or unmodified variant thereof.

A RNAi agent comprising a first and a second strand, wherein the sequence of the first and/or second strand comprise a sequence of 15 contiguous nt from the sequence of a first and/or second strand of: AD-35588.4 (SEQ ID NOs: 1733 and 1771), or a modified or unmodified variant thereof.

A RNAi agent comprising a first and a second strand, wherein the sequence of the first and/or second strand comprise a sequence of 15 contiguous nt from the sequence of a first and/or second strand of: AD-35600.1 (SEQ ID NOs: 1734 and 1772), or a modified or unmodified variant thereof.

A RNAi agent comprising a first and a second strand, wherein the sequence of the first and/or second strand comprise a sequence of 15 contiguous nt from the sequence of a first and/or second strand of: AD-35606.1 (SEQ ID NOs: 1735 and 1773), or a modified or unmodified variant thereof.

A RNAi agent comprising a first and a second strand, wherein the sequence of the first and/or second strand comprise a sequence of 15 contiguous nt from the sequence of a first and/or second strand of: AD-38151.1 (SEQ ID NOs: 1736 and 1774), or a modified or unmodified variant thereof.

A RNAi agent comprising a first and a second strand, wherein the sequence of the first and/or second strand comprise a sequence of 15 contiguous nt from the sequence of a first and/or second strand of: AD-38163.1 (SEQ ID NOs: 1737 and 1775), or a modified or unmodified variant thereof.

A RNAi agent comprising a first and a second strand, wherein the sequence of the first and/or second strand comprise a sequence of 15 contiguous nt from the sequence of a first and/or second strand of: AD-39999.1 (SEQ ID NOs: 1738 and 1776), or a modified or unmodified variant thereof.

A RNAi agent comprising a first and a second strand, wherein the sequence of the first and/or second strand comprise a sequence of 15 contiguous nt from the sequence of a first and/or second strand of: AD-38159.1 or hs_KRAS_528_A22S26 (SEQ ID NOs: 1739 and 1777), or a modified or unmodified variant thereof.

A RNAi agent comprising a first and a second strand, wherein the sequence of the first and/or second strand comprise a sequence of 15 contiguous nt from the sequence of a first and/or second strand of: AD-38130.1 or hs_KRAS_531_A22S26 (SEQ ID NOs: 1740 and 1778), or a modified or unmodified variant thereof.

A RNAi agent comprising a first and a second strand, wherein the sequence of the first and/or second strand comprise a sequence of 15 contiguous nt from the sequence of a first and/or second strand of: AD-38136.1 (SEQ ID NOs: 1741 and 1779), or a modified or unmodified variant thereof.

A RNAi agent comprising a first and a second strand, wherein the sequence of the first and/or second strand comprise a sequence of 15 contiguous nt from the sequence of a first and/or second strand of: AD-40036.1 (SEQ ID NOs: 1742 and 1780), or a modified or unmodified variant thereof.

A RNAi agent comprising a first and a second strand, wherein the sequence of the first and/or second strand comprise a sequence of 15 contiguous nt from the sequence of a first and/or second strand of: AD-40008.1 (SEQ ID NOs: 1743 and 1781), or a modified or unmodified variant thereof.

A RNAi agent comprising a first and a second strand, wherein the sequence of the first and/or second strand comprise a sequence of 15 contiguous nt from the sequence of a first and/or second strand of: AD-40021.1 (SEQ ID NOs: 1744 and 1782), or a modified or unmodified variant thereof.

A RNAi agent comprising a first and a second strand, wherein the sequence of the first and/or second strand comprise a sequence of 15 contiguous nt from the sequence of a first and/or second strand of: AD-40077.1 (SEQ ID NOs: 1745 and 1783), or a modified or unmodified variant thereof.

A RNAi agent comprising a first and a second strand, wherein the sequence of the first and/or second strand comprise a sequence of 15 contiguous nt from the sequence of a first and/or second strand of: AD-40072.1 (SEQ ID NOs: 1746 and 1784), or a modified or unmodified variant thereof.

A RNAi agent comprising a first and a second strand, wherein the sequence of the first and/or second strand comprise a sequence of 15 contiguous nt from the sequence of a first and/or second strand of: AD-40061.1 (SEQ ID NOs: 1747 and 1785), or a modified or unmodified variant thereof.

A RNAi agent comprising a first and a second strand, wherein the sequence of the first and/or second strand comprise a sequence of 15 contiguous nt from the sequence of a first and/or second strand of: AD-40068.1 (SEQ ID NOs: 1748 and 1786), or a modified or unmodified variant thereof.

A RNAi agent comprising a first and a second strand, wherein the sequence of the first and/or second strand comprise a sequence of 15 contiguous nt from the sequence of a first and/or second strand of: AD-38131.1 (SEQ ID NOs: 1749 and 1787), or a modified or unmodified variant thereof.

A RNAi agent comprising a first and a second strand, wherein the sequence of the first and/or second strand comprise a sequence of 15 contiguous nt from the sequence of a first and/or second strand of: AD-38167.1 (SEQ ID NOs: 1750 and 1788), or a modified or unmodified variant thereof.

A RNAi agent comprising a first and a second strand, wherein the sequence of the first and/or second strand comprise a sequence of 15 contiguous nt from the sequence of a first and/or second strand of: hs_KRAS 1273_A22S26 (SEQ ID NOs: 1751 and 1789), or a modified or unmodified variant thereof.

A RNAi agent comprising a first and a second strand, wherein the sequence of the first and/or second strand comprise a sequence of 15 contiguous nt from the sequence of a first and/or second strand of: hs_KRAS 2892_A37S26 (SEQ ID NOs: 1752 and 1790), or a modified or unmodified variant thereof.

A RNAi agent comprising a first and a second strand, wherein the sequence of the first and/or second strand comprise a sequence of 15 contiguous nt from the sequence of a first and/or second strand of: hs_KRAS 4731_A22S26 (SEQ ID NOs: 1753 and 1791), or a modified or unmodified variant thereof.

In various embodiments, the disclosure comprises a RNAi agent comprising a sense and an antisense strand, wherein the antisense strand comprises at least 15 contiguous nucleotides differing by 0, 1, 2, or 3 nt from the antisense strand of any RNAi agent disclosed herein, or a modified or unmodified variant thereof, wherein the antisense strand optionally further comprises 0, 1, 2, 3, 4, 5, 6, 7, 8, 9, or 10 or more nt (or any range thereof, e.g., 0-1, 1-2, 1-3, 1-4 nt, etc.).

In one embodiment, the disclosure comprises any one or more RNAi agent listed herein.

**Overlapping sets of RNAi agents to KRAS**

In various embodiments, the present disclosure relates to groups of RNAi agents to KRAS with overlapping sequences. Thus, the present disclosure encompasses groups of RNAi agents wherein each RNAi agent in the group overlaps with each other RNAi agent in the same group by at least 5, 6, 7, 8, 9, 10, 11, 12, 13, 14, 15, 16, 17, 18, 19 or more nucleotides. Particularly, in one embodiment, the overlap is at least 12 nt.

Some of the RNAi agents listed herein overlap each other in sequence. Table 2 presents a compilation of some of these groups of overlapping RNAi agents, wherein each member of a group overlaps with each other member of the same group by at least 12 nt. A 12-nt portion of the overlap of the sense and anti-sense strand are presented.

Thus, for example, as shown in Table 4, the sequences of duplexes AD-39695.1, AD-39689.1,AD-39707.1, AD-39683.1, and AD-39701.1 all overlap, wherein the overlap in sense strand comprises the sequence CUGAAUAUAAAC (SEQ ID NO: 1874) and the overlap in the anti-sense strand comprises the sequence GUUUAUAUUCAG (SEQ ID NO: 1875).

However, in the present disclosure encompasses any set or subset or group or subgroup of KRAS RNAi agents which share the common technical feature of a sequence overlap (e.g., by at least 12 nt). Thus the present disclosure also encompasses the example groups of AD-39695.1, AD-39689.1, AD-39707.1, AD-39683.1, and AD-39701.1, but also these subgroups: AD-39695.1 and AD-39689.1; AD-39695.1 and AD-39707.1; AD-39695.1, AD-39689.1, and AD-39701.1; AD-39689.1, AD-39707.1, and AD-39701.1; AD-39707.1 and AD-39683.1; AD-39707.1, AD-39683.1, and AD-39701.1; etc, and any other group of overlapping KRAS RNAi agents.

Thus, these and other various sets of overlapping RNAi agents presented in Table 4 share common technical features, for example, the overlap in the sense and anti-sense strand.

Particular sets of overlapping RNAi agents to KRAS are provided below in Table 4.

The present disclosure thus encompasses any group or subgroup of RNAi agents comprising a common technical feature, wherein the common technical feature is an overlap (e.g., of at least 12 nt) of a sequence in the sense or anti-sense strand.

Thus:

The present disclosure encompasses a RNAi agent comprising a first and a second strand, wherein the first strand comprises at least 15 contiguous nucleotides differing by 0, 1, 2, or 3 nt from the first strand of, and/or the second strand comprises at least 15 contiguous nucleotides differing by 0, 1, 2, or 3 nt from the second strand, of any of the group or subgroup or set or subset of overlapping RNAi agents presented in Table 4.

The present disclosure similarly encompasses various embodiments encompassing groups of overlapping RNAi agents presented in Table 4.

**Additional Definitions**

Unless defined otherwise, the technical and scientific terms used herein have the same meaning as that usually understood by a specialist familiar with the field to which the present disclosure belongs.

Unless indicated otherwise, all methods, steps, techniques and manipulations that are not specifically described in detail can be performed and have been performed in a manner known per se, as will be clear to the skilled person. Reference is for example again made to the standard handbooks and the general background art mentioned herein and to the further references cited therein.

Claims to the present disclosure are non-limiting and are provided below.

Although particular embodiments and claims have been disclosed herein in detail, this has been done by way of example for purposes of illustration only, and is not intended to be limiting with respect to the scope of the appended claims, or the scope of subject matter of claims of any corresponding future application. In particular, it is contemplated by the inventors that various substitutions, alterations, and modifications may be made to the present disclosure without departing from the spirit and scope of the present disclosure as defined by the claims. The choice of nucleic acid starting material, clone of interest, or library type is believed to be a matter of routine for a person of ordinary skill in the art with knowledge of the embodiments described herein. Other aspects, advantages, and modifications considered to be within the scope of the following claims. Redrafting of claim scope in later-filed corresponding applications may be due to limitations by the patent laws of various countries and should not be interpreted as giving up subject matter of the claims.

Various additional formulations and obvious variants of the described RNAi agents to KRAS can be devised by those of ordinary skill in the art. Non-limiting example RNAi agents to KRAS are described in the Examples below, which do not limit the scope of the present disclosure as described in the claims.

## EXAMPLES

### EXAMPLE 1

**Bioinformatics and KRAS RNAi Agent (siRNA) sequences**

The sequences of unmodifed and example modified KRAS RNAi agent sequences are provided in Tables 2 and 3, below.

KRAS oligonucleotide design is carried out to identify siRNAs targeting mRNAs encoding the KRAS gene.

**Experimental Methods**

**Bioinformatics**

Transcripts

siRNA design is carried out to identify RNAi agents (e.g., siRNAs) targeting the human Kirsten rat sarcoma viral oncogene (KRAS). Design uses the following transcripts from the NCBI RefSeq collection: KRAS - NM_033360.2, NM_004985.3. For KRAS, the requirement of perfect matching to the non-human primate *Macaca fascicularis is* waived.

siRNA Design and Specificity Prediction

The predicted specificity of all possible 19mers is predicted from each sequence. Candidate 19mers are then selected as matching Coding Sequence ("CDS") or Untranslated Regions ("UTRs"). The candidate siRNAs for each gene (KRAS, 432 from CDS) are used in a comprehensive search against the human transcriptome (defined as the set of NM_ and XM_ records within the human NCBI Refseq set) using the FASTA algorithm. The perl script 'parseFasta.pl' is then used to parse the alignments and generate a score based on the position and number of mismatches between the siRNA and any potential 'off-target' transcript. The off-target score is weighted to emphasize differences in the 'seed' region of siRNAs, in positions 2-9 from the 5' end of the molecule. Each oligo-transcript pair from the FASTA search is given a mismatch score by summing the individual mismatch scores; mismatches in the position 2-9 are counted as 2.8, mismatches in the cleavage site positions 10-11 are counted as 1.2, and mismatches in region 12-19 counted as 1.0. An additional off-target prediction is carried out by comparing the frequency of heptamers and octomers derived from 3 distinct, seed-derived hexamers of each oligo. The hexamers from positions 2-7 relative to the 5' start is used to create 2 heptamers and one octomer. We create heptamer1' by adding a 3' A to the hexamer; we create heptamer2 by adding a 5' A to the hexamer; we create the octomer by adding an A to both 5' and 3' ends of the hexamer. The frequency of octomers and heptamers in the human 3'UTRome (defined as the subsequence of the transcriptome from NCBI's Refseq database where the end of the coding region, the 'CDS', is clearly defined) is pre-calculated. The octomer frequency is normalized to the heptamer frequency using the median value from the range of octomer frequencies. A 'mirSeedScore' is then calculated by calculating the sum of [(3 X normalized octomer count) + (2 X heptamer2 count) + (1 X heptamer1 count)].

Both siRNAs strands are assigned to a category of specificity according to the calculated scores: a score above 3 qualifies as highly specific, equal to 3 as specific and between 2.2 and 2.8 as moderately specific. We sorted by the specificity of the antisense strand.

siRNA sequence selection

Sense and antisense derived siRNA oligos are synthesized and formed into duplexes. The total numbers of duplexes per gene were: KRAS, 432.

**Synthesis of KRAS sequences**

Synthesis:

KRAS sequences are synthesized on MerMade 192 synthesizer at 1μmol scale.
A total of 864 single strands (432 duplexes) for KRAS are synthesized for the human KRAS gene. For all the sequences, 'endolight' chemistry is applied as detailed below.

- All pyrimidines (cytosine and uridine) in the sense strand are replaced with corresponding 2'-O-Methyl bases (2' O-Methyl C and 2'-O-Methyl U)
- In the antisense strand, pyrimidines (C and U) adjacent to (towards 5' position) ribo A nucleoside are replaced with their corresponding 2-O-Methyl nucleosides
- A two base dTdT extension at 3' end of both sense and anti sense sequences is introduced. This two base overhang has a phosphodiester linkage
- The sequence file is converted to a text file to make it compatible for loading in the MerMade 192 synthesis software
- A unique ID is generated for each single strand and these are shown in Tables 1 to 3.

The synthesis of KRAS sequences used solid supported oligonucleotide synthesis using phosphoramidite chemistry

The synthesis of the above sequences is performed at 1 μm scale in 96 well plates and 192 sequences are made per run. The amidite solutions are prepared at 0.1M concentration and ethyl thio tetrazole (0.6M in Acetonitrile) is used as activator.

Cleavage and Deprotection:

The synthesized sequences are cleaved and deprotected in 96 well plates, using a mixture of aqueous and ethanolic methylamine (3:1 ratio) in the first step and triethylamine $^{3}$HF in the second step. The crude sequences thus obtained are precipitated using acetone: ethanol mix and the pellet are re-suspended in 0.02M sodium acetate buffer. Samples from each sequence are analyzed by LC-MS and the resulting mass data confirmed the identity of the sequences. A selected set of samples are also analyzed by IEX chromatography.

Purification and Desalting:

All single strands are isolated as desalted solutions in water prior to annealing step. Sequences are purified on AKTA explorer purification system using Source 15Q column. Purification is performed using a column and inline buffer heater set at 60°C. A single peak corresponding to the full length sequence is collected in the eluant and is subsequently analyzed for purity by ion exchange chromatography.

The purified sequences are desalted on a Sephadex G25 column using AKTA purifier. The desalted KRAS sequences are analyzed for concentration and purity. The single strands are annealed on an automated platform to form siRNA duplexes. Each duplex is made at a concentration of 10μM in 1x PBS and are analyzed for purity by capillary gel electrophoresis. Details on individual duplexes are provided in Tables 1 to 3. Some sequences in Table 2, and other tables herein, represent the DNA target of the recited RNAi agent (these DNA targets comprise DNA sequences, comprising T, rather than RNA sequences, which comprise U).

**Table 2. KRAS duplexes with unmodified sequences**

| Duplex | Sense Seq | SEQ ID NO: | Antisense Seq | SEQ ID NO: |
|---|---|---|---|---|
| AD-35523.3 | GGACGAAUAUGAUCCAACA | 1 | UGUUGGAUCAUAUUCGUCC | 428 |
| AD-35529.1 | GACGAAUAUGAUCCAACAA | 2 | UUGUUGGAUCAUAUUCGUC | 429 |
| AD-35535.4 | ACGAAUAUGAUCCAACAAU | 3 | AUUGUUGGAUCAUAUUCGU | 430 |
| AD-35541.4 | CGAAUAUGAUCCAACAAUA | 4 | UAUUGUUGGAUCAUAUUCG | 431 |
| AD-35547.4 | GAAUAUGAUCCAACAAUAG | 5 | CUAUUGUUGGAUCAUAUUC | 432 |
| AD-35553.4 | AAUAUGAUCCAACAAUAGA | 6 | UCUAUUGUUGGAUCAUAUU | 433 |
| AD-35559.4 | AUAUGAUCCAACAAUAGAG | 7 | CUCUAUUGUUGGAUCAUAU | 434 |
| AD-35565.4 | UAUGAUCCAACAAUAGAGG | 8 | CCUCUAUUGUUGGAUCAUA | 435 |
| AD-35524.4 | AUGAUCCAACAAUAGAGGA | 9 | UCCUCUAUUGUUGGAUCAU | 436 |
| AD-35530.4 | UGAUCCAACAAUAGAGGAU | 10 | AUCCUCUAUUGUUGGAUCA | 437 |
| AD-35536.4 | GAUCCAACAAUAGAGGAUU | 11 | AAUCCUCUAUUGUUGGAUC | 438 |
| AD-35542.4 | AUCCAACAAUAGAGGAUUC | 12 | GAAUCCUCUAUUGUUGGAU | 439 |
| AD-35548.4 | UCCAACAAUAGAGGAUUCC | 13 | GGAAUCCUCUAUUGUUGGA | 440 |
| AD-35554.4 | CCAACAAUAGAGGAUUCCU | 14 | AGGAAUCCUCUAUUGUUGG | 441 |
| AD-35560.2 | CAACAAUAGAGGAUUCCUA | 15 | UAGGAAUCCUCUAUUGUUG | 442 |
| AD-35566.4 | AACAAUAGAGGAUUCCUAC | 16 | GUAGGAAUCCUCUAUUGUU | 443 |
| AD-35525.2 | ACAAUAGAGGAUUCCUACA | 17 | UGUAGGAAUCCUCUAUUGU | 444 |
| AD-35531.4 | CAAUAGAGGAUUCCUACAG | 18 | CUGUAGGAAUCCUCUAUUG | 445 |
| AD-35537.4 | AAUAGAGGAUUCCUACAGG | 19 | CCUGUAGGAAUCCUCUAUU | 446 |
| AD-35543.2 | AUAGAGGAUUCCUACAGGA | 20 | UCCUGUAGGAAUCCUCUAU | 447 |
| AD-35549.4 | UAGAGGAUUCCUACAGGAA | 21 | UUCCUGUAGGAAUCCUCUA | 448 |
| AD-35555.4 | AGAGGAUUCCUACAGGAAG | 22 | CUUCCUGUAGGAAUCCUCU | 449 |
| AD-35561.4 | GAGGAUUCCUACAGGAAGC | 23 | GCUUCCUGUAGGAAUCCUC | 450 |
| AD-35567.4 | AGGAUUCCUACAGGAAGCA | 24 | UGCUUCCUGUAGGAAUCCU | 451 |
| AD-35526.4 | GGAUUCCUACAGGAAGCAA | 25 | UUGCUUCCUGUAGGAAUCC | 452 |
| AD-35532.4 | GAUUCCUACAGGAAGCAAG | 26 | CUUGCUUCCUGUAGGAAUC | 453 |
| AD-35538.4 | AUUCCUACAGGAAGCAAGU | 27 | ACUUGCUUCCUGUAGGAAU | 454 |
| AD-35544.4 | UUCCUACAGGAAGCAAGUA | 28 | UACUUGCUUCCUGUAGGAA | 455 |
| AD-35550.4 | UCCUACAGGAAGCAAGUAG | 29 | CUACUUGCUUCCUGUAGGA | 456 |
| AD-35556.4 | CCUACAGGAAGCAAGUAGU | 30 | ACUACUUGCUUCCUGUAGG | 457 |
| AD-35562.4 | CUACAGGAAGCAAGUAGUA | 31 | UACUACUUGCUUCCUGUAG | 458 |
| AD-35568.4 | UACAGGAAGCAAGUAGUAA | 32 | UUACUACUUGCUUCCUGUA | 459 |

(continued)

| Duplex | Sense Seq | SEQ ID NO: | Antisense Seq | SEQ ID NO: |
|---|---|---|---|---|
| AD-35527.4 | ACAGGAAGCAAGUAGUAAU | 33 | AUUACUACUUGCUUCCUGU | 460 |
| AD-35533.4 | CAGGAAGCAAGUAGUAAUU | 34 | AAUUACUACUUGCUUCCUG | 461 |
| AD-35539.4 | UUGGAUAUUCUCGACACAG | 35 | CUGUGUCGAGAAUAUCCAA | 462 |
| AD-35545.4 | UGGAUAUUCUCGACACAGC | 36 | GCUGUGUCGAGAAUAUCCA | 463 |
| AD-35551.4 | GGAUAUUCUCGACACAGCA | 37 | UGCUGUGUCGAGAAUAUCC | 464 |
| AD-35563.4 | AUAUUCUCGACACAGCAGG | 38 | CCUGCUGUGUCGAGAAUAU | 465 |
| AD-35528.4 | AUUCUCGACACAGCAGGUC | 39 | GACCUGCUGUGUCGAGAAU | 466 |
| AD-35540.4 | UCUCGACACAGCAGGUCAA | 40 | UUGACCUGCUGUGUCGAGA | 467 |
| AD-35546.4 | CUCGACACAGCAGGUCAAG | 41 | CUUGACCUGCUGUGUCGAG | 468 |
| AD-35552.4 | UCGACACAGCAGGUCAAGA | 42 | UCUUGACCUGCUGUGUCGA | 469 |
| AD-35558.4 | CGACACAGCAGGUCAAGAG | 43 | CUCUUGACCUGCUGUGUCG | 470 |
| AD-35564.4 | GACACAGCAGGUCAAGAGG | 44 | CCUCUUGACCUGCUGUGUC | 471 |
| AD-35570.4 | ACACAGCAGGUCAAGAGGA | 45 | UCCUCUUGACCUGCUGUGU | 472 |
| AD-35571.4 | CACAGCAGGUCAAGAGGAG | 46 | CUCCUCUUGACCUGCUGUG | 473 |
| AD-35577.4 | ACAGCAGGUCAAGAGGAGU | 47 | ACUCCUCUUGACCUGCUGU | 474 |
| AD-35583.4 | CAGCAGGUCAAGAGGAGUA | 48 | UACUCCUCUUGACCUGCUG | 475 |
| AD-35589.4 | AGCAGGUCAAGAGGAGUAC | 49 | GUACUCCUCUUGACCUGCU | 476 |
| AD-35595.4 | GCAGGUCAAGAGGAGUACA | 50 | UGUACUCCUCUUGACCUGC | 477 |
| AD-35601.4 | CAGGUCAAGAGGAGUACAG | 51 | CUGUACUCCUCUUGACCUG | 478 |
| AD-35607.4 | AGGUCAAGAGGAGUACAGU | 52 | ACUGUACUCCUCUUGACCU | 479 |
| AD-35613.4 | GGUCAAGAGGAGUACAGUG | 53 | CACUGUACUCCUCUUGACC | 480 |
| AD-35572.4 | GUCAAGAGGAGUACAGUGC | 54 | GCACUGUACUCCUCUUGAC | 481 |
| AD-35578.4 | UCAAGAGGAGUACAGUGCA | 55 | UGCACUGUACUCCUCUUGA | 482 |
| AD-35584.4 | CAAGAGGAGUACAGUGCAA | 56 | UUGCACUGUACUCCUCUUG | 483 |
| AD-35590.4 | AAGAGGAGUACAGUGCAAU | 57 | AUUGCACUGUACUCCUCUU | 484 |
| AD-35596.4 | AGAGGAGUACAGUGCAAUG | 58 | CAUUGCACUGUACUCCUCU | 485 |
| AD-35602.4 | GAGGAGUACAGUGCAAUGA | 59 | UCAUUGCACUGUACUCCUC | 486 |
| AD-35608.4 | AGGAGUACAGUGCAAUGAG | 60 | CUCAUUGCACUGUACUCCU | 487 |
| AD-35614.4 | GGAGUACAGUGCAAUGAGG | 61 | CCUCAUUGCACUGUACUCC | 488 |
| AD-35573.4 | GAGUACAGUGCAAUGAGGG | 62 | CCCUCAUUGCACUGUACUC | 489 |
| AD-35579.4 | GGGGAGGGCUUUCUUUGUG | 63 | CACAAAGAAAGCCCUCCCC | 490 |
| AD-35585.4 | GGGAGGGCUUUCUUUGUGU | 64 | ACACAAAGAAAGCCCUCCC | 491 |
| AD-35591.4 | GGAGGGCUUUCUUUGUGUA | 65 | UACACAAAGAAAGCCCUCC | 492 |
| AD-35597.4 | GAGGGCUUUCUUUGUGUAU | 66 | AUACACAAAGAAAGCCCUC | 493 |
| AD-35603.4 | AGGGCUUUCUUUGUGUAUU | 67 | AAUACACAAAGAAAGCCCU | 494 |
| AD-35609.4 | GGGCUUUCUUUGUGUAUUU | 68 | AAAUACACAAAGAAAGCCC | 495 |

(continued)

| Duplex | Sense Seq | SEQ ID NO: | Antisense Seq | SEQ ID NO: |
|---|---|---|---|---|
| AD-35615.4 | GGCUUUCUUUGUGUAUUUG | 69 | CAAAUACACAAAGAAAGCC | 496 |
| AD-35574.4 | GCUUUCUUUGUGUAUUUGC | 70 | GCAAAUACACAAAGAAAGC | 497 |
| AD-35580.1 | CUUUCUUUGUGUAUUUGCC | 71 | GGCAAAUACACAAAGAAAG | 498 |
| AD-35586.4 | UUUCUUUGUGUAUUUGCCA | 72 | UGGCAAAUACACAAAGAAA | 499 |
| AD-35592.4 | UUCUUUGUGUAUUUGCCAU | 73 | AUGGCAAAUACACAAAGAA | 500 |
| AD-35598.4 | UCUUUGUGUAUUUGCCAUA | 74 | UAUGGCAAAUACACAAAGA | 501 |
| AD-35604.4 | CUUUGUGUAUUUGCCAUAA | 75 | UUAUGGCAAAUACACAAAG | 502 |
| AD-35610.4 | UUUGUGUAUUUGCCAUAAA | 76 | UUUAUGGCAAAUACACAAA | 503 |
| AD-35616.4 | UUGUGUAUUUGCCAUAAAU | 77 | AUUUAUGGCAAAUACACAA | 504 |
| AD-35575.4 | UGUGUAUUUGCCAUAAAUA | 78 | UAUUUAUGGCAAAUACACA | 505 |
| AD-35581.4 | GUGUAUUUGCCAUAAAUAA | 79 | UUAUUUAUGGCAAAUACAC | 506 |
| AD-35587.4 | UGUAUUUGCCAUAAAUAAU | 80 | AUUAUUUAUGGCAAAUACA | 507 |
| AD-35593.4 | GUAUUUGCCAUAAAUAAUA | 81 | UAUUAUUUAUGGCAAAUAC | 508 |
| AD-35599.4 | UAUUUGCCAUAAAUAAUAC | 82 | GUAUUAUUUAUGGCAAAUA | 509 |
| AD-35605.4 | AUUUGCCAUAAAUAAUACU | 83 | AGUAUUAUUUAUGGCAAAU | 510 |
| AD-35611.4 | UUUGCCAUAAAUAAUACUA | 84 | UAGUAUUAUUUAUGGCAAA | 511 |
| AD-35617.4 | UGAAGAUAUUCACCAUUAU | 85 | AUAAUGGUGAAUAUCUUCA | 512 |
| AD-35576.4 | GAAGAUAUUCACCAUUAUA | 86 | UAUAAUGGUGAAUAUCUUC | 513 |
| AD-35588.4 | AGAUAUUCACCAUUAUAGA | 87 | UCUAUAAUGGUGAAUAUCU | 514 |
| AD-35667.1 | AAACAUCAGCAAAGACAAG | 88 | CUUGUCUUUGCUGAUGUUU | 515 |
| AD-35673.1 | AACAUCAGCAAAGACAAGA | 89 | UCUUGUCUUUGCUGAUGUU | 516 |
| AD-35679.1 | ACAUCAGCAAAGACAAGAC | 90 | GUCUUGUCUUUGCUGAUGU | 517 |
| AD-35685.1 | CAUCAGCAAAGACAAGACA | 91 | UGUCUUGUCUUUGCUGAUG | 518 |
| AD-35691.1 | AUCAGCAAAGACAAGACAG | 92 | CUGUCUUGUCUUUGCUGAU | 519 |
| AD-35557.1 | GAUAUUCUCGACACAGCAG | 93 | CUGCUGUGUCGAGAAUAUC | 520 |
| AD-35618.1 | UUCACCAUUAUAGAGAACA | 94 | UGUUCUCUAUAAUGGUGAA | 521 |
| AD-35569.1 | UAUUCUCGACACAGCAGGU | 95 | ACCUGCUGUGUCGAGAAUA | 522 |
| AD-35534.1 | UUCUCGACACAGCAGGUCA | 96 | UGACCUGCUGUGUCGAGAA | 523 |
| AD-35582.1 | AAGAUAUUCACCAUUAUAG | 97 | CUAUAAUGGUGAAUAUCUU | 524 |
| AD-35594.1 | GAUAUUCACCAUUAUAGAG | 98 | CUCUAUAAUGGUGAAUAUC | 525 |
| AD-35600.1 | AUAUUCACCAUUAUAGAGA | 99 | UCUCUAUAAUGGUGAAUAU | 526 |
| AD-35606.1 | UAUUCACCAUUAUAGAGAA | 100 | UUCUCUAUAAUGGUGAAUA | 527 |
| AD-35612.1 | AUUCACCAUUAUAGAGAAC | 101 | GUUCUCUAUAAUGGUGAAU | 528 |
| AD-38127.1 | UCACCAUUAUAGAGAACAA | 102 | UUGUUCUCUAUAAUGGUGA | 529 |
| AD-38133.1 | CACCAUUAUAGAGAACAAA | 103 | UUUGUUCUCUAUAAUGGUG | 530 |
| AD-38139.1 | ACCAUUAUAGAGAACAAAU | 104 | AUUUGUUCUCUAUAAUGGU | 531 |

(continued)

| Duplex | Sense Seq | SEQ ID NO: | Antisense Seq | SEQ ID NO: |
|---|---|---|---|---|
| AD-38145.1 | CCAUUAUAGAGAACAAAUU | 105 | AAUUUGUUCUCUAUAAUGG | 532 |
| AD-38151.1 | CAUUAUAGAGAACAAAUUA | 106 | UAAUUUGUUCUCUAUAAUG | 533 |
| AD-38157.1 | AUUAUAGAGAACAAAUUAA | 107 | UUAAUUUGUUCUCUAUAAU | 534 |
| AD-38163.1 | UUAUAGAGAACAAAUUAAA | 108 | UUUAAUUUGUUCUCUAUAA | 535 |
| AD-38169.1 | UAUAGAGAACAAAUUAAAA | 109 | UUUUAAUUUGUUCUCUAUA | 536 |
| AD-38128.1 | AUAGAGAACAAAUUAAAAG | 110 | CUUUUAAUUUGUUCUCUAU | 537 |
| AD-38134.1 | UAGAGAACAAAUUAAAAGA | 111 | UCUUUUAAUUUGUUCUCUA | 538 |
| AD-38140.1 | AGAGAACAAAUUAAAAGAG | 112 | CUCUUUUAAUUUGUUCUCU | 539 |
| AD-38146.1 | GAAGAUGUACCUAUGGUCC | 113 | GGACCAUAGGUACAUCUUC | 540 |
| AD-38152.1 | AAGAUGUACCUAUGGUCCU | 114 | AGGACCAUAGGUACAUCUU | 541 |
| AD-38158.1 | AGAUGUACCUAUGGUCCUA | 115 | UAGGACCAUAGGUACAUCU | 542 |
| AD-38164.1 | GAUGUACCUAUGGUCCUAG | 116 | CUAGGACCAUAGGUACAUC | 543 |
| AD-38170.1 | AUGUACCUAUGGUCCUAGU | 117 | ACUAGGACCAUAGGUACAU | 544 |
| AD-38129.1 | UGUACCUAUGGUCCUAGUA | 118 | UACUAGGACCAUAGGUACA | 545 |
| AD-38135.1 | GUACCUAUGGUCCUAGUAG | 119 | CUACUAGGACCAUAGGUAC | 546 |
| AD-38141.1 | UACCUAUGGUCCUAGUAGG | 120 | CCUACUAGGACCAUAGGUA | 547 |
| AD-38147.1 | ACCUAUGGUCCUAGUAGGA | 121 | UCCUACUAGGACCAUAGGU | 548 |
| AD-38153.1 | CCUAUGGUCCUAGUAGGAA | 122 | UUCCUACUAGGACCAUAGG | 549 |
| AD-38159.1 | CUAUGGUCCUAGUAGGAAA | 123 | UUUCCUACUAGGACCAUAG | 550 |
| AD-38165.1 | UAUGGUCCUAGUAGGAAAU | 124 | AUUUCCUACUAGGACCAUA | 551 |
| AD-38171.1 | AUGGUCCUAGUAGGAAAUA | 125 | UAUUUCCUACUAGGACCAU | 552 |
| AD-38130.1 | UGGUCCUAGUAGGAAAUAA | 126 | UUAUUUCCUACUAGGACCA | 553 |
| AD-38136.1 | GGUCCUAGUAGGAAAUAAA | 127 | UUUAUUUCCUACUAGGACC | 554 |
| AD-38142.1 | GUCCUAGUAGGAAAUAAAU | 128 | AUUUAUUUCCUACUAGGAC | 555 |
| AD-38148.1 | UCCUAGUAGGAAAUAAAUG | 129 | CAUUUAUUUCCUACUAGGA | 556 |
| AD-38154.1 | CCUAGUAGGAAAUAAAUGU | 130 | ACAUUUAUUUCCUACUAGG | 557 |
| AD-38160.1 | CUAGUAGGAAAUAAAUGUG | 131 | CACAUUUAUUUCCUACUAG | 558 |
| AD-38166.1 | UAGUAGGAAAUAAAUGUGA | 132 | UCACAUUUAUUUCCUACUA | 559 |
| AD-38172.1 | AGUAGGAAAUAAAUGUGAU | 133 | AUCACAUUUAUUUCCUACU | 560 |
| AD-38131.1 | GGAAUUCCUUUUAUUGAAA | 134 | UUUCAAUAAAAGGAAUUCC | 561 |
| AD-38137.1 | GAAUUCCUUUUAUUGAAAC | 135 | GUUUCAAUAAAAGGAAUUC | 562 |
| AD-38143.1 | AAUUCCUUUUAUUGAAACA | 136 | UGUUUCAAUAAAAGGAAUU | 563 |
| AD-38149.1 | AUUCCUUUUAUUGAAACAU | 137 | AUGUUUCAAUAAAAGGAAU | 564 |
| AD-38155.1 | UUCCUUUUAUUGAAACAUC | 138 | GAUGUUUCAAUAAAAGGAA | 565 |
| AD-38161.1 | UCCUUUUAUUGAAACAUCA | 139 | UGAUGUUUCAAUAAAAGGA | 566 |
| AD-38167.1 | CCUUUUAUUGAAACAUCAG | 140 | CUGAUGUUUCAAUAAAAGG | 567 |

(continued)

| Duplex | Sense Seq | SEQ ID NO: | Antisense Seq | SEQ ID NO: |
|---|---|---|---|---|
| AD-38173.1 | CUUUUAUUGAAACAUCAGC | 141 | GCUGAUGUUUCAAUAAAAG | 568 |
| AD-38132.1 | UUUUAUUGAAACAUCAGCA | 142 | UGCUGAUGUUUCAAUAAAA | 569 |
| AD-38138.1 | UUUAUUGAAACAUCAGCAA | 143 | UUGCUGAUGUUUCAAUAAA | 570 |
| AD-38144.1 | UUAUUGAAACAUCAGCAAA | 144 | UUUGCUGAUGUUUCAAUAA | 571 |
| AD-38150.1 | UAUUGAAACAUCAGCAAAG | 145 | CUUUGCUGAUGUUUCAAUA | 572 |
| AD-38156.1 | AUUGAAACAUCAGCAAAGA | 146 | UCUUUGCUGAUGUUUCAAU | 573 |
| AD-38162.1 | UUGAAACAUCAGCAAAGAC | 147 | GUCUUUGCUGAUGUUUCAA | 574 |
| AD-38168.1 | UGAAACAUCAGCAAAGACA | 148 | UGUCUUUGCUGAUGUUUCA | 575 |
| AD-38174.1 | GAAACAUCAGCAAAGACAA | 149 | UUGUCUUUGCUGAUGUUUC | 576 |
| AD-39683.1 | AUGACUGAAUAUAAACUUG | 150 | CAAGUUUAUAUUCAGUCAU | 577 |
| AD-39689.1 | UGACUGAAUAUAAACUUGU | 151 | ACAAGUUUAUAUUCAGUCA | 578 |
| AD-39695.1 | GACUGAAUAUAAACUUGUG | 152 | CACAAGUUUAUAUUCAGUC | 579 |
| AD-39701.1 | ACUGAAUAUAAACUUGUGG | 153 | CCACAAGUUUAUAUUCAGU | 580 |
| AD-39707.1 | CUGAAUAUAAACUUGUGGU | 154 | ACCACAAGUUUAUAUUCAG | 581 |
| AD-39713.1 | UGAAUAUAAACUUGUGGUA | 155 | UACCACAAGUUUAUAUUCA | 582 |
| AD-39719.1 | GAAUAUAAACUUGUGGUAG | 156 | CUACCACAAGUUUAUAUUC | 583 |
| AD-39725.1 | AAUAUAAACUUGUGGUAGU | 157 | ACUACCACAAGUUUAUAUU | 584 |
| AD-39684.1 | AUAUAAACUUGUGGUAGUU | 158 | AACUACCACAAGUUUAUAU | 585 |
| AD-39690.1 | UAUAAACUUGUGGUAGUUG | 159 | CAACUACCACAAGUUUAUA | 586 |
| AD-39696.1 | AUAAACUUGUGGUAGUUGG | 160 | CCAACUACCACAAGUUUAU | 587 |
| AD-39702.1 | UAAACUUGUGGUAGUUGGA | 161 | UCCAACUACCACAAGUUUA | 588 |
| AD-39708.1 | AAACUUGUGGUAGUUGGAG | 162 | CUCCAACUACCACAAGUUU | 589 |
| AD-39714.1 | AACUUGUGGUAGUUGGAGC | 163 | GCUCCAACUACCACAAGUU | 590 |
| AD-39720.1 | ACUUGUGGUAGUUGGAGCU | 164 | AGCUCCAACUACCACAAGU | 591 |
| AD-39726.1 | CUUGUGGUAGUUGGAGCUG | 165 | CAGCUCCAACUACCACAAG | 592 |
| AD-39685.1 | UUGUGGUAGUUGGAGCUGG | 166 | CCAGCUCCAACUACCACAA | 593 |
| AD-39691.1 | UGUGGUAGUUGGAGCUGGU | 167 | ACCAGCUCCAACUACCACA | 594 |
| AD-39697.1 | GUGGUAGUUGGAGCUGGUG | 168 | CACCAGCUCCAACUACCAC | 595 |
| AD-39703.1 | UGGUAGUUGGAGCUGGUGG | 169 | CCACCAGCUCCAACUACCA | 596 |
| AD-39709.1 | GGUAGUUGGAGCUGGUGGC | 170 | GCCACCAGCUCCAACUACC | 597 |
| AD-39715.1 | GUAGUUGGAGCUGGUGGCG | 171 | CGCCACCAGCUCCAACUAC | 598 |
| AD-39721.1 | UAGUUGGAGCUGGUGGCGU | 172 | ACGCCACCAGCUCCAACUA | 599 |
| AD-39727.1 | AGUUGGAGCUGGUGGCGUA | 173 | UACGCCACCAGCUCCAACU | 600 |
| AD-39686.1 | GUUGGAGCUGGUGGCGUAG | 174 | CUACGCCACCAGCUCCAAC | 601 |
| AD-39692.1 | UUGGAGCUGGUGGCGUAGG | 175 | CCUACGCCACCAGCUCCAA | 602 |
| AD-39698.1 | UGGAGCUGGUGGCGUAGGC | 176 | GCCUACGCCACCAGCUCCA | 603 |

(continued)

| Duplex | Sense Seq | SEQ ID NO: | Antisense Seq | SEQ ID NO: |
|---|---|---|---|---|
| AD-39704.1 | GGAGCUGGUGGCGUAGGCA | 177 | UGCCUACGCCACCAGCUCC | 604 |
| AD-39710.1 | GAGCUGGUGGCGUAGGCAA | 178 | UUGCCUACGCCACCAGCUC | 605 |
| AD-39716.1 | AGCUGGUGGCGUAGGCAAG | 179 | CUUGCCUACGCCACCAGCU | 606 |
| AD-39722.1 | GCUGGUGGCGUAGGCAAGA | 180 | UCUUGCCUACGCCACCAGC | 607 |
| AD-39728.1 | CUGGUGGCGUAGGCAAGAG | 181 | CUCUUGCCUACGCCACCAG | 608 |
| AD-39687.1 | UGGUGGCGUAGGCAAGAGU | 182 | ACUCUUGCCUACGCCACCA | 609 |
| AD-39693.1 | GGUGGCGUAGGCAAGAGUG | 183 | CACUCUUGCCUACGCCACC | 610 |
| AD-39699.1 | GUGGCGUAGGCAAGAGUGC | 184 | GCACUCUUGCCUACGCCAC | 611 |
| AD-39705.1 | UGGCGUAGGCAAGAGUGCC | 185 | GGCACUCUUGCCUACGCCA | 612 |
| AD-39711.1 | GGCGUAGGCAAGAGUGCCU | 186 | AGGCACUCUUGCCUACGCC | 613 |
| AD-39717.1 | GCGUAGGCAAGAGUGCCUU | 187 | AAGGCACUCUUGCCUACGC | 614 |
| AD-39723.1 | CGUAGGCAAGAGUGCCUUG | 188 | CAAGGCACUCUUGCCUACG | 615 |
| AD-39729.1 | GUAGGCAAGAGUGCCUUGA | 189 | UCAAGGCACUCUUGCCUAC | 616 |
| AD-39688.1 | UAGGCAAGAGUGCCUUGAC | 190 | GUCAAGGCACUCUUGCCUA | 617 |
| AD-39694.1 | AGGCAAGAGUGCCUUGACG | 191 | CGUCAAGGCACUCUUGCCU | 618 |
| AD-39700.1 | GGCAAGAGUGCCUUGACGA | 192 | UCGUCAAGGCACUCUUGCC | 619 |
| AD-39706.1 | GCAAGAGUGCCUUGACGAU | 193 | AUCGUCAAGGCACUCUUGC | 620 |
| AD-39712.1 | CAAGAGUGCCUUGACGAUA | 194 | UAUCGUCAAGGCACUCUUG | 621 |
| AD-39718.1 | AAGAGUGCCUUGACGAUAC | 195 | GUAUCGUCAAGGCACUCUU | 622 |
| AD-39724.1 | AGAGUGCCUUGACGAUACA | 196 | UGUAUCGUCAAGGCACUCU | 623 |
| AD-39730.1 | GAGUGCCUUGACGAUACAG | 197 | CUGUAUCGUCAAGGCACUC | 624 |
| AD-39736.1 | AGUGCCUUGACGAUACAGC | 198 | GCUGUAUCGUCAAGGCACU | 625 |
| AD-39742.1 | GUGCCUUGACGAUACAGCU | 199 | AGCUGUAUCGUCAAGGCAC | 626 |
| AD-39748.1 | UGCCUUGACGAUACAGCUA | 200 | UAGCUGUAUCGUCAAGGCA | 627 |
| AD-39754.1 | GCCUUGACGAUACAGCUAA | 201 | UUAGCUGUAUCGUCAAGGC | 628 |
| AD-39760.1 | CCUUGACGAUACAGCUAAU | 202 | AUUAGCUGUAUCGUCAAGG | 629 |
| AD-39766.1 | CUUGACGAUACAGCUAAUU | 203 | AAUUAGCUGUAUCGUCAAG | 630 |
| AD-39772.1 | UUGACGAUACAGCUAAUUC | 204 | GAAUUAGCUGUAUCGUCAA | 631 |
| AD-39731.1 | UGACGAUACAGCUAAUUCA | 205 | UGAAUUAGCUGUAUCGUCA | 632 |
| AD-39737.1 | GACGAUACAGCUAAUUCAG | 206 | CUGAAUUAGCUGUAUCGUC | 633 |
| AD-39743.1 | ACGAUACAGCUAAUUCAGA | 207 | UCUGAAUUAGCUGUAUCGU | 634 |
| AD-39749.1 | CGAUACAGCUAAUUCAGAA | 208 | UUCUGAAUUAGCUGUAUCG | 635 |
| AD-39755.1 | GAUACAGCUAAUUCAGAAU | 209 | AUUCUGAAUUAGCUGUAUC | 636 |
| AD-39761.1 | AUACAGCUAAUUCAGAAUC | 210 | GAUUCUGAAUUAGCUGUAU | 637 |
| AD-39767.1 | UACAGCUAAUUCAGAAUCA | 211 | UGAUUCUGAAUUAGCUGUA | 638 |
| AD-39773.1 | ACAGCUAAUUCAGAAUCAU | 212 | AUGAUUCUGAAUUAGCUGU | 639 |

(continued)

| Duplex | Sense Seq | SEQ ID NO: | Antisense Seq | SEQ ID NO: |
|---|---|---|---|---|
| AD-39732.1 | CAGCUAAUUCAGAAUCAUU | 213 | AAUGAUUCUGAAUUAGCUG | 640 |
| AD-39738.1 | AGCUAAUUCAGAAUCAUUU | 214 | AAAUGAUUCUGAAUUAGCU | 641 |
| AD-39744.1 | GCUAAUUCAGAAUCAUUUU | 215 | AAAAUGAUUCUGAAUUAGC | 642 |
| AD-39750.1 | CUAAUUCAGAAUCAUUUUG | 216 | CAAAAUGAUUCUGAAUUAG | 643 |
| AD-39756.1 | UAAUUCAGAAUCAUUUUGU | 217 | ACAAAAUGAUUCUGAAUUA | 644 |
| AD-39762.1 | AAUUCAGAAUCAUUUUGUG | 218 | CACAAAAUGAUUCUGAAUU | 645 |
| AD-39768.1 | AUUCAGAAUCAUUUUGUGG | 219 | CCACAAAAUGAUUCUGAAU | 646 |
| AD-39774.1 | UUCAGAAUCAUUUUGUGGA | 220 | UCCACAAAAUGAUUCUGAA | 647 |
| AD-39733.1 | UCAGAAUCAUUUUGUGGAC | 221 | GUCCACAAAAUGAUUCUGA | 648 |
| AD-39739.1 | CAGAAUCAUUUUGUGGACG | 222 | CGUCCACAAAAUGAUUCUG | 649 |
| AD-39745.1 | AGAAUCAUUUUGUGGACGA | 223 | UCGUCCACAAAAUGAUUCU | 650 |
| AD-39751.1 | GAAUCAUUUUGUGGACGAA | 224 | UUCGUCCACAAAAUGAUUC | 651 |
| AD-39757.1 | AAUCAUUUUGUGGACGAAU | 225 | AU UCGUCCACAAAAUGAUU | 652 |
| AD-39763.1 | AUCAUUUUGUGGACGAAUA | 226 | UAUUCGUCCACAAAAUGAU | 653 |
| AD-39769.1 | UCAUUUUGUGGACGAAUAU | 227 | AUAUUCGUCCACAAAAUGA | 654 |
| AD-39775.1 | CAUUUUGUGGACGAAUAUG | 228 | CAUAUUCGUCCACAAAAUG | 655 |
| AD-39734.1 | AUUUUGUGGACGAAUAUGA | 229 | UCAUAUUCGUCCACAAAAU | 656 |
| AD-39740.1 | UUUUGUGGACGAAUAUGAU | 230 | AUCAUAUUCGUCCACAAAA | 657 |
| AD-39746.1 | UUUGUGGACGAAUAUGAUC | 231 | GAUCAUAUUCGUCCACAAA | 658 |
| AD-39752.1 | UUGUGGACGAAUAUGAUCC | 232 | GGAUCAUAUUCGUCCACAA | 659 |
| AD-39758.1 | UGUGGACGAAUAUGAUCCA | 233 | UGGAUCAUAUUCGUCCACA | 660 |
| AD-39764.1 | GUGGACGAAUAUGAUCCAA | 234 | UUGGAUCAUAUUCGUCCAC | 661 |
| AD-39770.1 | UGGACGAAUAUGAUCCAAC | 235 | GUUGGAUCAUAUUCGUCCA | 662 |
| AD-39776.1 | AGGAAGCAAGUAGUAAUUG | 236 | CAAUUACUACUUGCUUCCU | 663 |
| AD-39735.1 | GGAAGCAAGUAGUAAUUGA | 237 | UCAAUUACUACUUGCUUCC | 664 |
| AD-39741.1 | GAAGCAAGUAGUAAUUGAU | 238 | AUCAAUUACUACUUGCUUC | 665 |
| AD-39747.1 | AAGCAAGUAGUAAUUGAUG | 239 | CAUCAAUUACUACUUGCUU | 666 |
| AD-39753.1 | AGCAAGUAGUAAUUGAUGG | 240 | CCAUCAAUUACUACUUGCU | 667 |
| AD-39759.1 | GCAAGUAGUAAUUGAUGGA | 241 | UCCAUCAAUUACUACUUGC | 668 |
| AD-39765.1 | CAAGUAGUAAUUGAUGGAG | 242 | CUCCAUCAAUUACUACUUG | 669 |
| AD-39771.1 | AAGUAGUAAUUGAUGGAGA | 243 | UCUCCAUCAAUUACUACUU | 670 |
| AD-39778.1 | AGUAGUAAUUGAUGGAGAA | 244 | UUCUCCAUCAAUUACUACU | 671 |
| AD-39784.1 | GUAGUAAUUGAUGGAGAAA | 245 | UUUCUCCAUCAAUUACUAC | 672 |
| AD-39790.1 | UAGUAAUUGAUGGAGAAAC | 246 | GUUUCUCCAUCAAUUACUA | 673 |
| AD-39796.1 | AGUAAUUGAUGGAGAAACC | 247 | GGUUUCUCCAUCAAUUACU | 674 |
| AD-39802.1 | GUAAUUGAUGGAGAAACCU | 248 | AGGUUUCUCCAUCAAUUAC | 675 |

(continued)

| Duplex | Sense Seq | SEQ ID NO: | Antisense Seq | SEQ ID NO: |
|---|---|---|---|---|
| AD-39808.1 | UAAUUGAUGGAGAAACCUG | 249 | CAGGUUUCUCCAUCAAUUA | 676 |
| AD-39814.1 | AAUUGAUGGAGAAACCUGU | 250 | ACAGGUUUCUCCAUCAAUU | 677 |
| AD-39820.1 | AUUGAUGGAGAAACCUGUC | 251 | GACAGGUUUCUCCAUCAAU | 678 |
| AD-39779.1 | UUGAUGGAGAAACCUGUCU | 252 | AGACAGGUUUCUCCAUCAA | 679 |
| AD-39785.1 | UGAUGGAGAAACCUGUCUC | 253 | GAGACAGGUUUCUCCAUCA | 680 |
| AD-39791.1 | GAUGGAGAAACCUGUCUCU | 254 | AGAGACAGGUUUCUCCAUC | 681 |
| AD-39797.1 | AUGGAGAAACCUGUCUCUU | 255 | AAGAGACAGGUUUCUCCAU | 682 |
| AD-39803.1 | UGGAGAAACCUGUCUCUUG | 256 | CAAGAGACAGGUUUCUCCA | 683 |
| AD-39809.1 | GGAGAAACCUGUCUCUUGG | 257 | CCAAGAGACAGGUUUCUCC | 684 |
| AD-39815.1 | GAGAAACCUGUCUCUUGGA | 258 | UCCAAGAGACAGGUUUCUC | 685 |
| AD-39821.1 | AGAAACCUGUCUCUUGGAU | 259 | AUCCAAGAGACAGGUUUCU | 686 |
| AD-39780.1 | GAAACCUGUCUCUUGGAUA | 260 | UAUCCAAGAGACAGGUUUC | 687 |
| AD-39786.1 | AAACCUGUCUCUUGGAUAU | 261 | AUAUCCAAGAGACAGGUUU | 688 |
| AD-39792.1 | AACCUGUCUCUUGGAUAUU | 262 | AAUAUCCAAGAGACAGGUU | 689 |
| AD-39798.1 | ACCUGUCUCUUGGAUAUUC | 263 | GAAUAUCCAAGAGACAGGU | 690 |
| AD-39804.1 | CCUGUCUCUUGGAUAUUCU | 264 | AGAAUAUCCAAGAGACAGG | 691 |
| AD-39810.1 | CUGUCUCUUGGAUAUUCUC | 265 | GAGAAUAUCCAAGAGACAG | 692 |
| AD-39816.1 | UGUCUCUUGGAUAUUCUCG | 266 | CGAGAAUAUCCAAGAGACA | 693 |
| AD-39822.1 | GUCUCUUGGAUAUUCUCGA | 267 | UCGAGAAUAUCCAAGAGAC | 694 |
| AD-39781.1 | UCUCUUGGAUAUUCUCGAC | 268 | GUCGAGAAUAUCCAAGAGA | 695 |
| AD-39787.1 | CUCUUGGAUAUUCUCGACA | 269 | UGUCGAGAAUAUCCAAGAG | 696 |
| AD-39793.1 | UCUUGGAUAUUCUCGACAC | 270 | GUGUCGAGAAUAUCCAAGA | 697 |
| AD-39799.1 | CUUGGAUAUUCUCGACACA | 271 | UGUGUCGAGAAUAUCCAAG | 698 |
| AD-39805.1 | AGUACAGUGCAAUGAGGGA | 272 | UCCCUCAUUGCACUGUACU | 699 |
| AD-39811.1 | GUACAGUGCAAUGAGGGAC | 273 | GUCCCUCAUUGCACUGUAC | 700 |
| AD-39817.1 | UACAGUGCAAUGAGGGACC | 274 | GGUCCCUCAUUGCACUGUA | 701 |
| AD-39823.1 | ACAGUGCAAUGAGGGACCA | 275 | UGGUCCCUCAUUGCACUGU | 702 |
| AD-39782.1 | CAGUGCAAUGAGGGACCAG | 276 | CUGGUCCCUCAUUGCACUG | 703 |
| AD-39788.1 | AGUGCAAUGAGGGACCAGU | 277 | ACUGGUCCCUCAUUGCACU | 704 |
| AD-39794.1 | GUGCAAUGAGGGACCAGUA | 278 | UACUGGUCCCUCAUUGCAC | 705 |
| AD-39800.1 | UGCAAUGAGGGACCAGUAC | 279 | GUACUGGUCCCUCAUUGCA | 706 |
| AD-39806.1 | GCAAUGAGGGACCAGUACA | 280 | UGUACUGGUCCCUCAUUGC | 707 |
| AD-39812.1 | CAAUGAGGGACCAGUACAU | 281 | AUGUACUGGUCCCUCAUUG | 708 |
| AD-39818.1 | AAUGAGGGACCAGUACAUG | 282 | CAUGUACUGGUCCCUCAUU | 709 |
| AD-39824.1 | AUGAGGGACCAGUACAUGA | 283 | UCAUGUACUGGUCCCUCAU | 710 |
| AD-39783.1 | UGAGGGACCAGUACAUGAG | 284 | CUCAUGUACUGGUCCCUCA | 711 |

(continued)

| Duplex | Sense Seq | SEQ ID NO: | Antisense Seq | SEQ ID NO: |
|---|---|---|---|---|
| AD-39789.1 | GAGGGACCAGUACAUGAGG | 285 | CCUCAUGUACUGGUCCCUC | 712 |
| AD-39795.1 | AGGGACCAGUACAUGAGGA | 286 | UCCUCAUGUACUGGUCCCU | 713 |
| AD-39801.1 | GGGACCAGUACAUGAGGAC | 287 | GUCCUCAUGUACUGGUCCC | 714 |
| AD-39807.1 | GGACCAGUACAUGAGGACU | 288 | AGUCCUCAUGUACUGGUCC | 715 |
| AD-39813.1 | GACCAGUACAUGAGGACUG | 289 | CAGUCCUCAUGUACUGGUC | 716 |
| AD-39819.1 | ACCAGUACAUGAGGACUGG | 290 | CCAGUCCUCAUGUACUGGU | 717 |
| AD-39825.1 | CCAGUACAUGAGGACUGGG | 291 | CCCAGUCCUCAUGUACUGG | 718 |
| AD-39831.1 | CAGUACAUGAGGACUGGGG | 292 | CCCCAGUCCUCAUGUACUG | 719 |
| AD-39837.1 | AGUACAUGAGGACUGGGGA | 293 | UCCCCAGUCCUCAUGUACU | 720 |
| AD-39843.1 | GUACAUGAGGACUGGGGAG | 294 | CUCCCCAGUCCUCAUGUAC | 721 |
| AD-39849.1 | UACAUGAGGACUGGGGAGG | 295 | CCUCCCCAGUCCUCAUGUA | 722 |
| AD-39855.1 | ACAUGAGGACUGGGGAGGG | 296 | CCCUCCCCAGUCCUCAUGU | 723 |
| AD-39861.1 | CAUGAGGACUGGGGAGGGC | 297 | GCCCUCCCCAGUCCUCAUG | 724 |
| AD-39867.1 | AUGAGGACUGGGGAGGGCU | 298 | AGCCCUCCCCAGUCCUCAU | 725 |
| AD-39826.1 | UGAGGACUGGGGAGGGCUU | 299 | AAGCCCUCCCCAGUCCUCA | 726 |
| AD-39832.1 | GAGGACUGGGGAGGGCUUU | 300 | AAAGCCCUCCCCAGUCCUC | 727 |
| AD-39838.1 | AGGACUGGGGAGGGCUUUC | 301 | GAAAGCCCUCCCCAGUCCU | 728 |
| AD-39844.1 | GGACUGGGGAGGGCUUUCU | 302 | AGAAAGCCCUCCCCAGUCC | 729 |
| AD-39850.1 | GACUGGGGAGGGCUUUCUU | 303 | AAGAAAGCCCUCCCCAGUC | 730 |
| AD-39856.1 | ACUGGGGAGGGCUUUCUUU | 304 | AAAGAAAGCCCUCCCCAGU | 731 |
| AD-39862.1 | CUGGGGAGGGCUUUCUUUG | 305 | CAAAGAAAGCCCUCCCCAG | 732 |
| AD-39868.1 | UGGGGAGGGCUUUCUUUGU | 306 | ACAAAGAAAGCCCUCCCCA | 733 |
| AD-39827.1 | UUGCCAUAAAUAAUACUAA | 307 | UUAGUAUUAUUUAUGGCAA | 734 |
| AD-39833.1 | UGCCAUAAAUAAUACUAAA | 308 | UUUAGUAUUAUUUAUGGCA | 735 |
| AD-39839.1 | GCCAUAAAUAAUACUAAAU | 309 | AUUUAGUAUUAUUUAUGGC | 736 |
| AD-39845.1 | CCAUAAAUAAUACUAAAUC | 310 | GAUUUAGUAUUAUUUAUGG | 737 |
| AD-39851.1 | CAUAAAUAAUACUAAAUCA | 311 | UGAUUUAGUAUUAUUUAUG | 738 |
| AD-39857.1 | AUAAAUAAUACUAAAUCAU | 312 | AUGAUUUAGUAUUAUUUAU | 739 |
| AD-39863.1 | UAAAUAAUACUAAAUCAUU | 313 | AAUGAUUUAGUAUUAUUUA | 740 |
| AD-39869.1 | AAAUAAUACUAAAUCAUUU | 314 | AAAUGAUUUAGUAUUAUUU | 741 |
| AD-39828.1 | AAUAAUACUAAAUCAUUUG | 315 | CAAAUGAUUUAGUAUUAUU | 742 |
| AD-39834.1 | AUAAUACUAAAUCAUUUGA | 316 | UCAAAUGAUUUAGUAUUAU | 743 |
| AD-39840.1 | UAAUACUAAAUCAUUUGAA | 317 | UUCAAAUGAUUUAGUAUUA | 744 |
| AD-39846.1 | AAUACUAAAUCAUUUGAAG | 318 | CUUCAAAUGAUUUAGUAUU | 745 |
| AD-39852.1 | AUACUAAAUCAUUUGAAGA | 319 | UCUUCAAAUGAUUUAGUAU | 746 |
| AD-39858.1 | UACUAAAUCAUUUGAAGAU | 320 | AUCUUCAAAUGAUUUAGUA | 747 |

(continued)

| Duplex | Sense Seq | SEQ ID NO: | Antisense Seq | SEQ ID NO: |
|---|---|---|---|---|
| AD-39864.1 | ACUAAAUCAUUUGAAGAUA | 321 | UAUCUUCAAAUGAUUUAGU | 748 |
| AD-39870.1 | CUAAAUCAUUUGAAGAUAU | 322 | AUAUCUUCAAAUGAUUUAG | 749 |
| AD-39829.1 | UAAAUCAUUUGAAGAUAUU | 323 | AAUAUCUUCAAAUGAUUUA | 750 |
| AD-39835.1 | AAAUCAUUUGAAGAUAUUC | 324 | GAAUAUCUUCAAAUGAUUU | 751 |
| AD-39841.1 | AAUCAUUUGAAGAUAUUCA | 325 | UGAAUAUCUUCAAAUGAUU | 752 |
| AD-39853.1 | UCAUUUGAAGAUAUUCACC | 326 | GGUGAAUAUCUUCAAAUGA | 753 |
| AD-39859.1 | CAUUUGAAGAUAUUCACCA | 327 | UGGUGAAUAUCUUCAAAUG | 754 |
| AD-39865.1 | AUUUGAAGAUAUUCACCAU | 328 | AUGGUGAAUAUCUUCAAAU | 755 |
| AD-39871.1 | UUUGAAGAUAUUCACCAUU | 329 | AAUGGUGAAUAUCUUCAAA | 756 |
| AD-39830.1 | UUGAAGAUAUUCACCAUUA | 330 | UAAUGGUGAAUAUCUUCAA | 757 |
| AD-39836.1 | GAGAACAAAUUAAAAGAGU | 331 | ACUCUUUUAAUUUGUUCUC | 758 |
| AD-39842.1 | AGAACAAAUUAAAAGAGUU | 332 | AACUCUUUUAAUUUGUUCU | 759 |
| AD-39848.1 | GAACAAAUUAAAAGAGUUA | 333 | UAACUCUUUUAAUUUGUUC | 760 |
| AD-39854.1 | AACAAAUUAAAAGAGUUAA | 334 | UUAACUCUUUUAAUUUGUU | 761 |
| AD-39860.1 | ACAAAUUAAAAGAGUUAAG | 335 | CUUAACUCUUUUAAUUUGU | 762 |
| AD-39866.1 | CAAAUUAAAAGAGUUAAGG | 336 | CCUUAACUCUUUUAAUUUG | 763 |
| AD-39992.1 | AAAUUAAAAGAGUUAAGGA | 337 | UCCUUAACUCUUUUAAUUU | 764 |
| AD-39998.1 | AAUUAAAAGAGUUAAGGAC | 338 | GUCCUUAACUCUUUUAAUU | 765 |
| AD-40004.1 | AUUAAAAGAGUUAAGGACU | 339 | AGUCCUUAACUCUUUUAAU | 766 |
| AD-40010.1 | UUAAAAGAGUUAAGGACUC | 340 | GAGUCCUUAACUCUUUUAA | 767 |
| AD-40016.1 | UAAAAGAGUUAAGGACUCU | 341 | AGAGUCCUUAACUCUUUUA | 768 |
| AD-40022.1 | AAAAGAGUUAAGGACUCUG | 342 | CAGAGUCCUUAACUCUUUU | 769 |
| AD-40028.1 | AAAGAGUUAAGGACUCUGA | 343 | UCAGAGUCCUUAACUCUUU | 770 |
| AD-40034.1 | AAGAGUUAAGGACUCUGAA | 344 | UUCAGAGUCCUUAACUCUU | 771 |
| AD-39999.1 | GAGUUAAGGACUCUGAAGA | 345 | UCUUCAGAGUCCUUAACUC | 772 |
| AD-40005.1 | AGUUAAGGACUCUGAAGAU | 346 | AUCUUCAGAGUCCUUAACU | 773 |
| AD-40011.1 | GUUAAGGACUCUGAAGAUG | 347 | CAUCUUCAGAGUCCUUAAC | 774 |
| AD-40017.1 | UUAAGGACUCUGAAGAUGU | 348 | ACAUCUUCAGAGUCCUUAA | 775 |
| AD-40029.1 | AAGGACUCUGAAGAUGUAC | 349 | GUACAUCUUCAGAGUCCUU | 776 |
| AD-40035.1 | AGGACUCUGAAGAUGUACC | 350 | GGUACAUCUUCAGAGUCCU | 777 |
| AD-39994.1 | GGACUCUGAAGAUGUACCU | 351 | AGGUACAUCUUCAGAGUCC | 778 |
| AD-40000.1 | GACUCUGAAGAUGUACCUA | 352 | UAGGUACAUCUUCAGAGUC | 779 |
| AD-40006.1 | ACUCUGAAGAUGUACCUAU | 353 | AUAGGUACAUCUUCAGAGU | 780 |
| AD-40012.1 | CUCUGAAGAUGUACCUAUG | 354 | CAUAGGUACAUCUUCAGAG | 781 |
| AD-40018.1 | UCUGAAGAUGUACCUAUGG | 355 | CCAUAGGUACAUCUUCAGA | 782 |
| AD-40024.1 | CUGAAGAUGUACCUAUGGU | 356 | ACCAUAGGUACAUCUUCAG | 783 |

(continued)

| Duplex | Sense Seq | SEQ ID NO: | Antisense Seq | SEQ ID NO: |
|---|---|---|---|---|
| AD-40030.1 | UGAAGAUGUACCUAUGGUC | 357 | GACCAUAGGUACAUCUUCA | 784 |
| AD-40036.1 | GUAGGAAAUAAAUGUGAUU | 358 | AAUCACAUUUAUUUCCUAC | 785 |
| AD-39995.1 | UAGGAAAUAAAUGUGAUUU | 359 | AAAUCACAUUUAUUUCCUA | 786 |
| AD-40001.1 | AGGAAAUAAAUGUGAUUUG | 360 | CAAAUCACAUUUAUUUCCU | 787 |
| AD-40007.1 | GGAAAUAAAUGUGAUUUGC | 361 | GCAAAUCACAUUUAUUUCC | 788 |
| AD-40013.1 | GAAAUAAAUGUGAUUUGCC | 362 | GGCAAAUCACAUUUAUUUC | 789 |
| AD-40019.1 | AAAUAAAUGUGAUUUGCCU | 363 | AGGCAAAUCACAUUUAUUU | 790 |
| AD-40025.1 | AAUAAAUGUGAUUUGCCUU | 364 | AAGGCAAAUCACAUUUAUU | 791 |
| AD-40031.1 | AUAAAUGUGAUUUGCCUUC | 365 | GAAGGCAAAUCACAUUUAU | 792 |
| AD-40037.1 | UAAAUGUGAUUUGCCUUCU | 366 | AGAAGGCAAAUCACAUUUA | 793 |
| AD-39996.1 | AAAUGUGAUUUGCCUUCUA | 367 | UAGAAGGCAAAUCACAUUU | 794 |
| AD-40002.1 | AAUGUGAUUUGCCUUCUAG | 368 | CUAGAAGGCAAAUCACAUU | 795 |
| AD-40008.1 | AUGUGAUUUGCCUUCUAGA | 369 | UCUAGAAGGCAAAUCACAU | 796 |
| AD-40014.1 | UGUGAUUUGCCUUCUAGAA | 370 | UUCUAGAAGGCAAAUCACA | 797 |
| AD-40020.1 | GUGAUUUGCCUUCUAGAAC | 371 | GUUCUAGAAGGCAAAUCAC | 798 |
| AD-40026.1 | UGAUUUGCCUUCUAGAACA | 372 | UGUUCUAGAAGGCAAAUCA | 799 |
| AD-40032.1 | GAUUUGCCUUCUAGAACAG | 373 | CUGUUCUAGAAGGCAAAUC | 800 |
| AD-40038.1 | AUUUGCCUUCUAGAACAGU | 374 | ACUGUUCUAGAAGGCAAAU | 801 |
| AD-39997.1 | UUUGCCUUCUAGAACAGUA | 375 | UACUGUUCUAGAAGGCAAA | 802 |
| AD-40009.1 | UGCCUUCUAGAACAGUAGA | 376 | UCUACUGUUCUAGAAGGCA | 803 |
| AD-40015.1 | GCCUUCUAGAACAGUAGAC | 377 | GUCUACUGUUCUAGAAGGC | 804 |
| AD-40021.1 | CCUUCUAGAACAGUAGACA | 378 | UGUCUACUGUUCUAGAAGG | 805 |
| AD-40027.1 | CUUCUAGAACAGUAGACAC | 379 | GUGUCUACUGUUCUAGAAG | 806 |
| AD-40033.1 | UUCUAGAACAGUAGACACA | 380 | UGUGUCUACUGUUCUAGAA | 807 |
| AD-40039.1 | UCUAGAACAGUAGACACAA | 381 | UUGUGUCUACUGUUCUAGA | 808 |
| AD-40045.1 | CUAGAACAGUAGACACAAA | 382 | UUUGUGUCUACUGUUCUAG | 809 |
| AD-40051.1 | UAGAACAGUAGACACAAAA | 383 | UUUUGUGUCUACUGUUCUA | 810 |
| AD-40057.1 | AGAACAGUAGACACAAAAC | 384 | GUUUUGUGUCUACUGUUCU | 811 |
| AD-40063.1 | GAACAGUAGACACAAAACA | 385 | UGUUUUGUGUCUACUGUUC | 812 |
| AD-40069.1 | AACAGUAGACACAAAACAG | 386 | CUGUUUUGUGUCUACUGUU | 813 |
| AD-40075.1 | ACAGUAGACACAAAACAGG | 387 | CCUGUUUUGUGUCUACUGU | 814 |
| AD-40081.1 | CAGUAGACACAAAACAGGC | 388 | GCCUGUUUUGUGUCUACUG | 815 |
| AD-40040.1 | AGUAGACACAAAACAGGCU | 389 | AGCCUGUUUUGUGUCUACU | 816 |
| AD-40046.1 | GUAGACACAAAACAGGCUC | 390 | GAGCCUGUUUUGUGUCUAC | 817 |
| AD-40052.1 | UAGACACAAAACAGGCUCA | 391 | UGAGCCUGUUUUGUGUCUA | 818 |
| AD-40058.1 | AGACACAAAACAGGCUCAG | 392 | CUGAGCCUGUUUUGUGUCU | 819 |

(continued)

| Duplex | Sense Seq | SEQ ID NO: | Antisense Seq | SEQ ID NO: |
|---|---|---|---|---|
| AD-40064.1 | GACACAAAACAGGCUCAGG | 393 | CCUGAGCCUGUUUUGUGUC | 820 |
| AD-40070.1 | ACACAAAACAGGCUCAGGA | 394 | UCCUGAGCCUGUUUUGUGU | 821 |
| AD-40076.1 | CACAAAACAGGCUCAGGAC | 395 | GUCCUGAGCCUGUUUUGUG | 822 |
| AD-40082.1 | ACAAAACAGGCUCAGGACU | 396 | AGUCCUGAGCCUGUUUUGU | 823 |
| AD-40041.1 | CAAAACAGGCUCAGGACUU | 397 | AAGUCCUGAGCCUGUUUUG | 824 |
| AD-40047.1 | AAAACAGGCUCAGGACUUA | 398 | UAAGUCCUGAGCCUGUUUU | 825 |
| AD-40053.1 | AAACAGGCUCAGGACUUAG | 399 | CUAAGUCCUGAGCCUGUUU | 826 |
| AD-40059.1 | AACAGGCUCAGGACUUAGC | 400 | GCUAAGUCCUGAGCCUGUU | 827 |
| AD-40065.1 | ACAGGCUCAGGACUUAGCA | 401 | UGCUAAGUCCUGAGCCUGU | 828 |
| AD-40071.1 | CAGGCUCAGGACUUAGCAA | 402 | UUGCUAAGUCCUGAGCCUG | 829 |
| AD-40077.1 | AGGCUCAGGACUUAGCAAG | 403 | CUUGCUAAGUCCUGAGCCU | 830 |
| AD-40083.1 | GGCUCAGGACUUAGCAAGA | 404 | UCUUGCUAAGUCCUGAGCC | 831 |
| AD-40042.1 | GCUCAGGACUUAGCAAGAA | 405 | UUCUUGCUAAGUCCUGAGC | 832 |
| AD-40048.1 | CUCAGGACUUAGCAAGAAG | 406 | CUUCUUGCUAAGUCCUGAG | 833 |
| AD-40054.1 | UCAGGACUUAGCAAGAAGU | 407 | ACUUCUUGCUAAGUCCUGA | 834 |
| AD-40060.1 | CAGGACUUAGCAAGAAGUU | 408 | AACUUCUUGCUAAGUCCUG | 835 |
| AD-40066.1 | AGGACUUAGCAAGAAGUUA | 409 | UAACUUCUUGCUAAGUCCU | 836 |
| AD-40072.1 | GGACUUAGCAAGAAGUUAU | 410 | AUAACUUCUUGCUAAGUCC | 837 |
| AD-40078.1 | GACUUAGCAAGAAGUUAUG | 411 | CAUAACUUCUUGCUAAGUC | 838 |
| AD-40084.1 | ACUUAGCAAGAAGUUAUGG | 412 | CCAUAACUUCUUGCUAAGU | 839 |
| AD-40043.1 | CUUAGCAAGAAGUUAUGGA | 413 | UCCAUAACUUCUUGCUAAG | 840 |
| AD-40049.1 | UUAGCAAGAAGUUAUGGAA | 414 | UUCCAUAACUUCUUGCUAA | 841 |
| AD-40055.1 | UAGCAAGAAGUUAUGGAAU | 415 | AUUCCAUAACUUCUUGCUA | 842 |
| AD-40061.1 | AGCAAGAAGUUAUGGAAUU | 416 | AAUUCCAUAACUUCUUGCU | 843 |
| AD-40067.1 | GCAAGAAGUUAUGGAAUUC | 417 | GAAUUCCAUAACUUCUUGC | 844 |
| AD-40073.1 | CAAGAAGUUAUGGAAUUCC | 418 | GGAAUUCCAUAACUUCUUG | 845 |
| AD-40079.1 | AAGAAGUUAUGGAAUUCCU | 419 | AGGAAUUCCAUAACUUCUU | 846 |
| AD-40085.1 | AGAAGUUAUGGAAUUCCUU | 420 | AAGGAAUUCCAUAACUUCU | 847 |
| AD-40044.1 | GAAGUUAUGGAAUUCCUUU | 421 | AAAGGAAUUCCAUAACUUC | 848 |
| AD-40050.1 | AAGUUAUGGAAUUCCUUUU | 422 | AAAAGGAAUUCCAUAACUU | 849 |
| AD-40056.1 | AGUUAUGGAAUUCCUUUUA | 423 | UAAAAGGAAUUCCAUAACU | 850 |
| AD-40062.1 | GUUAUGGAAUUCCUUUUAU | 424 | AUAAAAGGAAUUCCAUAAC | 851 |
| AD-40068.1 | UUAUGGAAUUCCUUUUAUU | 425 | AAUAAAAGGAAUUCCAUAA | 852 |
| AD-40074.1 | UAUGGAAUUCCUUUUAUUG | 426 | CAAUAAAAGGAAUUCCAUA | 853 |
| AD-40080.1 | AUGGAAUUCCUUUUAUUGA | 427 | UCAAUAAAAGGAAUUCCAU | 854 |
| AD-39720.1 | ACTTGTGGTAGTTGGAGCT | 1716 | AGCTCCAACTACCACAAGT | 1754 |

(continued)

| Duplex | Sense Seq | SEQ ID NO: | Antisense Seq | SEQ ID NO: |
|---|---|---|---|---|
| AD-39706.1 | GCAAGAGTGCCTTGACGAT | 1717 | ATCGTCAAGGCACTCTTGC | 1755 |
| AD-39712.1 | CAAGAGTGCCTTGACGATA | 1718 | TATCGTCAAGGCACTCTTG | 1756 |
| AD-39760.1 | CCTTGACGATACAGCTAAT | 1719 | ATTAGCTGTATCGTCAAGG | 1757 |
| AD-39732.1 | CAGCTAATTCAGAATCATT | 1720 | AATGATTCTGAATTAGCTG | 1758 |
| AD-35541.4 | CGAATATGATCCAACAATA | 1721 | TATTGTTGGATCATATTCG | 1759 |
| AD-39735.1 or hs_ KRAS_ 321_A22S26 | GGAAGCAAGTAGTAATTGA | 1722 | TCAATTACTACTTGCTTCC | 1760 |
| AD-39741.1 or hs_ KRAS_ 322_A22S26 | GAAGCAAGTAGTAATTGAT | 1723 | ATCAATTACTACTTGCTTC | 1761 |
| AD-39778.1 | AGTAGTAATTGATGGAGAA | 1724 | TTCTCCATCAATTACTACT | 1762 |
| AD-39790.1 | TAGTAATTGATGGAGAAAC | 1725 | GTTTCTCCATCAATTACTA | 1763 |
| AD-39822.1 | GTCTCTTGGATATTCTCGA | 1726 | TCGAGAATATCCAAGAGAC | 1764 |
| AD-35609.4 | GGGCTTTCTTTGTGTATTT | 1727 | AAATACACAAAGAAAGCCC | 1765 |
| AD-35581.4 | GTGTATTTGCCATAAATAA | 1728 | TTATTTATGGCAAATACAC | 1766 |
| AD-39845.1 | CCATAAATAATACTAAATC | 1729 | GATTTAGTATTATTTATGG | 1767 |
| AD-39858.1 | TACTAAATCATTTGAAGAT | 1730 | ATCTTCAAATGATTTAGTA | 1768 |
| AD-39870.1 | CTAAATCATTTGAAGATAT | 1731 | ATATCTTCAAATGATTTAG | 1769 |
| AD-35576.4 | GAAGATATTCACCATTATA | 1732 | TATAATGGTGAATATCTTC | 1770 |
| AD-35588.4 | AGATATTCACCATTATAGA | 1733 | TCTATAATGGTGAATATCT | 1771 |
| AD-35600.1 | ATATTCACCATTATAGAGA | 1734 | TCTCTATAATGGTGAATAT | 1772 |
| AD-35606.1 | TATTCACCATTATAGAGAA | 1735 | TTCTCTATAATGGTGAATA | 1773 |
| AD-38151.1 | CATTATAGAGAACAAATTA | 1736 | TAATTTGTTCTCTATAATG | 1774 |
| AD-38163.1 | TTATAGAGAACAAATTAAA | 1737 | TTTAATTTGTTCTCTATAA | 1775 |
| AD-39999.1 | GAGTTAAGGACTCTGAAGA | 1738 | TCTTCAGAGTCCTTAACTC | 1776 |
| AD-38159.1 or hs_ KRAS_ 528_A22S26 | CTATGGTCCTAGTAGGAAA | 1739 | TTTCCTACTAGGACCATAG | 1777 |
| AD-38130.1 or hs_ KRAS_ 531_A22S26 | TGGTCCTAGTAGGAAATAA | 1740 | TTATTTCCTACTAGGACCA | 1778 |
| AD-38136.1 | GGTCCTAGTAGGAAATAAA | 1741 | TTTATTTCCTACTAGGACC | 1779 |
| AD-40036.1 | GTAGGAAATAAATGTGATT | 1742 | AATCACATTTATTTCCTAC | 1780 |
| AD-40008.1 | ATGTGATTTGCCTTCTAGA | 1743 | TCTAGAAGGCAAATCACAT | 1781 |
| AD-40021.1 | CCTTCTAGAACAGTAGACA | 1744 | TGTCTACTGTTCTAGAAGG | 1782 |
| AD-40077.1 | AGGCTCAGGACTTAGCAAG | 1745 | CTTGCTAAGTCCTGAGCCT | 1783 |
| AD-40072.1 | GGACTTAGCAAGAAGTTAT | 1746 | ATAACTTCTTGCTAAGTCC | 1784 |
| AD-40061.1 | AGCAAGAAGTTATGGAATT | 1747 | AATTCCATAACTTCTTGCT | 1785 |
| AD-40068.1 | TTATGGAATTCCTTTTATT | 1748 | AATAAAAGGAATTCCATAA | 1786 |
| AD-38131.1 | GGAATTCCTTTTATTGAAA | 1749 | TTTCAATAAAAGGAATTCC | 1787 |

(continued)

| Duplex | Sense Seq | SEQ ID NO: | Antisense Seq | SEQ ID NO: |
|---|---|---|---|---|
| AD-38167.1 | CCTTTTATTGAAACATCAG | 1750 | CTGATGTTTCAATAAAAGG | 1788 |
| hs_KRAS 1273_A22S26 | CCTGATGAATGTAAAGTTA | 1751 | TAACTTTACATTCATCAGG | 1789 |
| hs_KRAS 2892_A37S26 | GGAGAATTTAATAAAGATA | 1752 | TATCTTTATTAAATTCTCC | 1790 |
| hs_KRAS 4731_A22S26 | GAATAGTCATAACTAGATT | 1753 | AATCTAGTTATGACTATTC | 1791 |

**Table 3. KRAS duplexes with modified sequences**

| Duplex | Sense Sequence | Seq SEQ ID NO: | Anti sense Sequence | Seq SEQ ID NO: |
|---|---|---|---|---|
| AD-35523.3 | GGAcGAAuAuGAuccAAcAdTdT | 855 | UGUUGGAUcAuAUUCGUCCdTdT | 1282 |
| AD-35529.1 | GAcGAAuAuGAuccAAcAAdTdT | 856 | UUGUUGGAUcAuAUUCGUCdTdT | 1283 |
| AD-35535.4 | AcGAAuAuGAuccAAcAAudTdT | 857 | AUUGUUGGAUcAuAUUCGUdTdT | 1284 |
| AD-35541.4 | cGAAuAuGAuccAAcAAuAdTdT | 858 | uAUUGUUGGAUcAuAUUCGdTdT | 1285 |
| AD-35547.4 | GAAuAuGAuccAAcAAuAGdTdT | 859 | CuAUUGUUGGAUcAuAUUCdTdT | 1286 |
| AD-35553.4 | AAuAuGAuccAAcAAuAGAdTdT | 860 | UCuAUUGUUGGAUcAuAUUdTdT | 1287 |
| AD-35559.4 | AuAuGAuccAAcAAuAGAGdTdT | 861 | CUCuAUUGUUGGAUcAuAUdTdT | 1288 |
| AD-35565.4 | uAuGAuccAAcAAuAGAGGdTdT | 862 | CCUCuAUUGUUGGAUcAuAdTdT | 1289 |
| AD-35524.4 | AuGAuccAAcAAuAGAGGAdTdT | 863 | UCCUCuAUUGUUGGAUcAUdTdT | 1290 |
| AD-35530.4 | uGAuccAAcAAuAGAGGAudTdT | 864 | AUCCUCuAUUGUUGGAUcAdTdT | 1291 |
| AD-35536.4 | GAuccAAcAAuAGAGGAuudTdT | 865 | AAUCCUCuAUUGUUGGAUCdTdT | 1292 |
| AD-35542.4 | AuccAAcAAuAGAGGAuucdTdT | 866 | GAAUCCUCuAUUGUUGGAUdTdT | 1293 |
| AD-35548.4 | uccAAcAAuAGAGGAuuccdTdT | 867 | GGAAUCCUCuAUUGUUGGAdTdT | 1294 |
| AD-35554.4 | ccAAcAAuAGAGGAuuccudTdT | 868 | AGGAAUCCUCuAUUGUUGGdTdT | 1295 |
| AD-35560.2 | cAAcAAuAGAGGAuuccuAdTdT | 869 | uAGGAAUCCUCuAUUGUUGdTdT | 1296 |
| AD-35566.4 | AAcAAuAGAGGAuuccuAcdTdT | 870 | GuAGGAAUCCUCuAUUGUUdTdT | 1297 |
| AD-35525.2 | AcAAuAGAGGAuuccuAcAdTdT | 871 | UGuAGGAAUCCUCuAUUGUdTdT | 1298 |
| AD-35531.4 | cAAuAGAGGAuuccuAcAGdTdT | 872 | CUGuAGGAAUCCUCuAUUGdTdT | 1299 |
| AD-35537.4 | AAuAGAGGAuuccuAcAGGdTdT | 873 | CCUGuAGGAAUCCUCuAUUdTdT | 1300 |
| AD-35543.2 | AuAGAGGAuuccuAcAGGAdTdT | 874 | UCCUGuAGGAAUCCUCuAUdTdT | 1301 |
| AD-35549.4 | uAGAGGAuuccuAcAGGAAdTdT | 875 | UUCCUGuAGGAAUCCUCuAdTdT | 1302 |
| AD-35555.4 | AGAGGAuuccuAcAGGAAGdTdT | 876 | CUUCCUGuAGGAAUCCUCUdTdT | 1303 |
| AD-35561.4 | GAGGAuuccuAcAGGAAGcdTdT | 877 | GCUUCCUGuAGGAAUCCUCdTdT | 1304 |
| AD-35567.4 | AGGAuuccuAcAGGAAGcAdTdT | 878 | UGCUUCCUGuAGGAAUCCUdTdT | 1305 |
| AD-35526.4 | GGAuuccuAcAGGAAGcAAdTdT | 879 | UUGCUUCCUGuAGGAAUCCdTdT | 1306 |
| AD-35532.4 | GAuuccuAcAGGAAGcAAGdTdT | 880 | CUUGCUUCCUGuAGGAAUCdTdT | 1307 |
| AD-35538.4 | AuuccuAcAGGAAGcAAGudTdT | 881 | ACUUGCUUCCUGuAGGAAUdTdT | 1308 |

(continued)

| Duplex | Sense Sequence | Seq SEQ ID NO: | Anti sense Sequence | Seq SEQ ID NO: |
|---|---|---|---|---|
| AD-35544.4 | uuccuAcAGGAAGcAAGuAdTdT | 882 | uACUUGCUUCCUGuAGGAAdTdT | 1309 |
| AD-35550.4 | uccuAcAGGAAGcAAGuAGdTdT | 883 | CuACUUGCUUCCUGuAGGAdTdT | 1310 |
| AD-35556.4 | ccuAcAGGAAGcAAGuAGudTdT | 884 | ACuACUUGCUUCCUGuAGGdTdT | 1311 |
| AD-35562.4 | cuAcAGGAAGcAAGuAGuAdTdT | 885 | uACuACUUGCUUCCUGuAGdTdT | 1312 |
| AD-35568.4 | uAcAGGAAGcAAGuAGuAAdTdT | 886 | UuACuACUUGCUUCCUGuAdTdT | 1313 |
| AD-35527.4 | AcAGGAAGcAAGuAGuAAudTdT | 887 | AUuACuACUUGCUUCCUGUdTdT | 1314 |
| AD-35533.4 | cAGGAAGcAAGuAGuAAuudTdT | 888 | AAUuACuACUUGCUUCCUGdTdT | 1315 |
| AD-35539.4 | uuGGAuAuucucGAcAcAGdTdT | 889 | CUGUGUCGAGAAuAUCcAAdTdT | 1316 |
| AD-35545.4 | uGGAuAuucucGAcAcAGcdTdT | 890 | GCUGUGUCGAGAAuAUCcAdTdT | 1317 |
| AD-35551.4 | GGAuAuucucGAcAcAGcAdTdT | 891 | UGCUGUGUCGAGAAuAUCCdTdT | 1318 |
| AD-35563.4 | AuAuucucGAcAcAGcAGGdTdT | 892 | CCUGCUGUGUCGAGAAuAUdTdT | 1319 |
| AD-35528.4 | AuucucGAcAcAGcAGGucdTdT | 893 | GACCUGCUGUGUCGAGAAUdTdT | 1320 |
| AD-35540.4 | ucucGAcAcAGcAGGucAAdTdT | 894 | UUGACCUGCUGUGUCGAGAdTdT | 1321 |
| AD-35546.4 | cucGAcAcAGcAGGucAAGdTdT | 895 | CUUGACCUGCUGUGUCGAGdTdT | 1322 |
| AD-35552.4 | ucGAcAcAGcAGGucAAGAdTdT | 896 | UCUUGACCUGCUGUGUCGAdTdT | 1323 |
| AD-35558.4 | cGAcAcAGcAGGucAAGAGdTdT | 897 | CUCUUGACCUGCUGUGUCGdTdT | 1324 |
| AD-35564.4 | GAcAcAGcAGGucAAGAGGdTdT | 898 | CCUCUUGACCUGCUGUGUCdTdT | 1325 |
| AD-35570.4 | AcAcAGcAGGucAAGAGGAdTdT | 899 | UCCUCUUGACCUGCUGUGUdTdT | 1326 |
| AD-35571.4 | cAcAGcAGGucAAGAGGAGdTdT | 900 | CUCCUCUUGACCUGCUGUGdTdT | 1327 |
| AD-35577.4 | AcAGcAGGucAAGAGGAGudTdT | 901 | ACUCCUCUUGACCUGCUGUdTdT | 1328 |
| AD-35583.4 | cAGcAGGucAAGAGGAGuAdTdT | 902 | uACUCCUCUUGACCUGCUGdTdT | 1329 |
| AD-35589.4 | AGcAGGucAAGAGGAGuAcdTdT | 903 | GuACUCCUCUUGACCUGCUdTdT | 1330 |
| AD-35595.4 | GcAGGucAAGAGGAGuAcAdTdT | 904 | UGuACUCCUCUUGACCUGCdTdT | 1331 |
| AD-35601.4 | cAGGucAAGAGGAGuAcAGdTdT | 905 | CUGuACUCCUCUUGACCUGdTdT | 1332 |
| AD-35607.4 | AGGucAAGAGGAGuAcAGudTdT | 906 | ACUGuACUCCUCUUGACCUdTdT | 1333 |
| AD-35613.4 | GGucAAGAGGAGuAcAGuGdTdT | 907 | cACUGuACUCCUCUUGACCdTdT | 1334 |
| AD-35572.4 | GucAAGAGGAGuAcAGuGcdTdT | 908 | GcACUGuACUCCUCUUGACdTdT | 1335 |
| AD-35578.4 | ucAAGAGGAGuAcAGuGcAdTdT | 909 | UGcACUGuACUCCUCUUGAdTdT | 1336 |
| AD-35584.4 | cAAGAGGAGuAcAGuGcAAdTdT | 910 | UUGcACUGuACUCCUCUUGdTdT | 1337 |
| AD-35590.4 | AAGAGGAGuAcAGuGcAAudTdT | 911 | AUUGcACUGuACUCCUCUUdTdT | 1338 |
| AD-35596.4 | AGAGGAGuAcAGuGcAAuGdTdT | 912 | cAUUGcACUGuACUCCUCUdTdT | 1339 |
| AD-35602.4 | GAGGAGuAcAGuGcAAuGAdTdT | 913 | UcAUUGcACUGuACUCCUCdTdT | 1340 |
| AD-35608.4 | AGGAGuAcAGuGcAAuGAGdTdT | 914 | CUcAUUGcACUGuACUCCUdTdT | 1341 |
| AD-35614.4 | GGAGuAcAGuGcAAuGAGGdTdT | 915 | CCUcAUUGcACUGuACUCCdTdT | 1342 |
| AD-35573.4 | GAGuAcAGuGcAAuGAGGGdTdT | 916 | CCCUcAUUGcACUGuACUCdTdT | 1343 |
| AD-35579.4 | GGGGAGGGcuuucuuuGuGdTdT | 917 | cAcAAAGAAAGCCCUCCCCdTdT | 1344 |

(continued)

| Duplex | Sense Sequence | Seq SEQ ID NO: | Anti sense Sequence | Seq SEQ ID NO: |
|---|---|---|---|---|
| AD-35585.4 | GGGAGGGcuuucuuuGuGudTdT | 918 | AcAcAAAGAAAGCCCUCCCdTdT | 1345 |
| AD-35591.4 | GGAGGGcuuucuuuGuGuAdTdT | 919 | uAcAcAAAGAAAGCCCUCCdTdT | 1346 |
| AD-35597.4 | GAGGGcuuucuuuGuGuAudTdT | 920 | AuAcAcAAAGAAAGCCCUCdTdT | 1347 |
| AD-35603.4 | AGGGcuuucuuuGuGuAuudTdT | 921 | AAuAcAcAAAGAAAGCCCUdTdT | 1348 |
| AD-35609.4 | GGGcuuucuuuGuGuAuuudTdT | 922 | AAAuAcAcAAAGAAAGCCCdTdT | 1349 |
| AD-35615.4 | GGcuuucuuuGuGuAuuuGdTdT | 923 | cAAAuAcAcAAAGAAAGCCdTdT | 1350 |
| AD-35574.4 | GcuuucuuuGuGuAuuuGcdTdT | 924 | GcAAAuAcAcAAAGAAAGCdTdT | 1351 |
| AD-35580.1 | cuuucuuuGuGuAuuuGccdTdT | 925 | GGcAAAuAcAcAAAGAAAGdTdT | 1352 |
| AD-35586.4 | uuucuuuGuGuAuuuGccAdTdT | 926 | UGGcAAAuAcAcAAAGAAAdTdT | 1353 |
| AD-35592.4 | uucuuuGuGuAuuuGccAudTdT | 927 | AUGGcAAAuAcAcAAAGAAdTdT | 1354 |
| AD-35598.4 | ucuuuGuGuAuuuGccAuAdTdT | 928 | uAUGGcAAAuAcAcAAAGAdTdT | 1355 |
| AD-35604.4 | cuuuGuGuAuuuGccAuAAdTdT | 929 | UuAUGGcAAAuAcAcAAAGdTdT | 1356 |
| AD-35610.4 | uuuGuGuAuuuGccAuAAAdTdT | 930 | UUuAUGGcAAAuAcAcAAAdTdT | 1357 |
| AD-35616.4 | uuGuGuAuuuGccAuAAAudTdT | 931 | AUUuAUGGcAAAuAcAcAAdTdT | 1358 |
| AD-35575.4 | uGuGuAuuuGccAuAAAuAdTdT | 932 | uAUUuAUGGcAAAuAcAcAdTdT | 1359 |
| AD-35581.4 | GuGuAuuuGccAuAAAuAAdTdT | 933 | UuAUUuAUGGcAAAuAcACdTdT | 1360 |
| AD-35587.4 | uGuAuuuGccAuAAAuAAudTdT | 934 | AUuAUUuAUGGcAAAuAcAdTdT | 1361 |
| AD-35593.4 | GuAuuuGccAuAAAuAAuAdTdT | 935 | uAUuAUUuAUGGcAAAuACdTdT | 1362 |
| AD-35599.4 | uAuuuGccAuAAAuAAuAcdTdT | 936 | GuAUuAUUuAUGGcAAAuAdTdT | 1363 |
| AD-35605.4 | AuuuGccAuAAAuAAuAcudTdT | 937 | AGuAUuAUUuAUGGcAAAUdTdT | 1364 |
| AD-35611.4 | uuuGccAuAAAuAAuAcuAdTdT | 938 | uAGuAUuAUUuAUGGcAAAdTdT | 1365 |
| AD-35617.4 | uGAAGAuAuucAccAuuAudTdT | 939 | AuAAUGGUGAAuAUCUUcAdTdT | 1366 |
| AD-35576.4 | GAAGAuAuucAccAuuAuAdTdT | 940 | uAuAAUGGUGAAuAUCUUCdTdT | 1367 |
| AD-35588.4 | AGAuAuucAccAuuAuAGAdTdT | 941 | UCuAuAAUGGUGAAuAUCUdTdT | 1368 |
| AD-35667.1 | AAAcAucAGcAAAGAcAAGdTdT | 942 | CUUGUCUUUGCUGAUGUUUdTdT | 1369 |
| AD-35673.1 | AAcAucAGcAAAGAcAAGAdTdT | 943 | UCUUGUCUUUGCUGAUGUUdTdT | 1370 |
| AD-35679.1 | AcAucAGcAAAGAcAAGAcdTdT | 944 | GUCUUGUCUUUGCUGAUGUdTdT | 1371 |
| AD-35685.1 | cAucAGcAAAGAcAAGAcAdTdT | 945 | UGUCUUGUCUUUGCUGAUGdTdT | 1372 |
| AD-35691.1 | AucAGcAAAGAcAAGAcAGdTdT | 946 | CUGUCUUGUCUUUGCUGAUdTdT | 1373 |
| AD-35557.1 | GAuAuucucGAcAcAGcAGdTdT | 947 | CUGCUGUGUCGAGAAuAUCdTdT | 1374 |
| AD-35618.1 | uucAccAuuAuAGAGAAcAdTdT | 948 | UGUUCUCuAuAAUGGUGAAdTdT | 1375 |
| AD-35569.1 | uAuucucGAcAcAGcAGGudTdT | 949 | ACCUGCUGUGUCGAGAAuAdTdT | 1376 |
| AD-35534.1 | uucucGAcAcAGcAGGucAdTdT | 950 | UGACCUGCUGUGUCGAGAAdTdT | 1377 |
| AD-35582.1 | AAGAuAuucAccAuuAuAGdTdT | 951 | CuAuAAUGGUGAAuAUCUUdTdT | 1378 |
| AD-35594.1 | GAuAuucAccAuuAuAGAGdTdT | 952 | CUCuAuAAUGGUGAAuAUCdTdT | 1379 |
| AD-35600.1 | AuAuucAccAuuAuAGAGAdTdT | 953 | UCUCuAuAAUGGUGAAuAUdTdT | 1380 |

(continued)

| Duplex | Sense Sequence | Seq SEQ ID NO: | Anti sense Sequence | Seq SEQ ID NO: |
|---|---|---|---|---|
| AD-35606.1 | uAuucAccAuuAuAGAGAAdTdT | 954 | UUCUCuAuAAUGGUGAAuAdTdT | 1381 |
| AD-35612.1 | AuucAccAuuAuAGAGAAcdTdT | 955 | GUUCUCuAuAAUGGUGAAUdTdT | 1382 |
| AD-38127.1 | ucAccAuuAuAGAGAAcAAdTdT | 956 | UUGUUCUCuAuAAUGGUGAdTdT | 1383 |
| AD-38133.1 | cAccAuuAuAGAGAAcAAAdTdT | 957 | UUUGUUCUCuAuAAUGGUGdTdT | 1384 |
| AD-38139.1 | AccAuuAuAGAGAAcAAAudTdT | 958 | AUUUGUUCUCuAuAAUGGUdTdT | 1385 |
| AD-38145.1 | ccAuuAuAGAGAAcAAAuudTdT | 959 | AAUUUGUUCUCuAuAAUGGdTdT | 1386 |
| AD-38151.1 | cAuuAuAGAGAAcAAAuuAdTdT | 960 | uAAUUUGUUCUCuAuAAUGdTdT | 1387 |
| AD-38157.1 | AuuAuAGAGAAcAAAuuAAdTdT | 961 | UuAAUUUGUUCUCuAuAAUdTdT | 1388 |
| AD-38163.1 | uuAuAGAGAAcAAAuuAAAdTdT | 962 | UUuAAUUUGUUCUCuAuAAdTdT | 1389 |
| AD-38169.1 | uAuAGAGAAcAAAuuAAAAdTdT | 963 | UUUuAAUUUGUUCUCuAuAdTdT | 1390 |
| AD-38128.1 | AuAGAGAAcAAAuuAAAAGdTdT | 964 | CUUUuAAUUUGUUCUCuAUdTdT | 1391 |
| AD-38134.1 | uAGAGAAcAAAuuAAAAGAdTdT | 965 | UCUUUuAAUUUGUUCUCuAdTdT | 1392 |
| AD-38140.1 | AGAGAAcAAAuuAAAAGAGdTdT | 966 | CUCUUUuAAUUUGUUCUCUdTdT | 1393 |
| AD-38146.1 | GAAGAuGuAccuAuGGuccdTdT | 967 | GGACcAuAGGuAcAUCUUCdTdT | 1394 |
| AD-38152.1 | AAGAuGuAccuAuGGuccudTdT | 968 | AGGACcAuAGGuAcAUCUUdTdT | 1395 |
| AD-38158.1 | AGAuGuAccuAuGGuccuAdTdT | 969 | uAGGACcAuAGGuAcAUCUdTdT | 1396 |
| AD-38164.1 | GAuGuAccuAuGGuccuAGdTdT | 970 | CuAGGACcAuAGGuAcAUCdTdT | 1397 |
| AD-38170.1 | AuGuAccuAuGGuccuAGudTdT | 971 | ACuAGGACcAuAGGuAcAUdTdT | 1398 |
| AD-38129.1 | uGuAccuAuGGuccuAGuAdTdT | 972 | uACuAGGACcAuAGGuAcAdTdT | 1399 |
| AD-38135.1 | GuAccuAuGGuccuAGuAGdTdT | 973 | CuACuAGGACcAuAGGuACdTdT | 1400 |
| AD-38141.1 | uAccuAuGGuccuAGuAGGdT dT | 974 | CCuACuAGGACcAuAGGuAdTdT | 1401 |
| AD-38147.1 | AccuAuGGuccuAGuAGGAdTdT | 975 | UCCuACuAGGACcAuAGGUdTdT | 1402 |
| AD-38153.1 | ccuAuGGuccuAGuAGGAAdTdT | 976 | UUCCuACuAGGACcAuAGGdTdT | 1403 |
| AD-38159.1 | cuAuGGuccuAGuAGGAAAdTdT | 977 | UUUCCuACuAGGACcAuAGdTdT | 1404 |
| AD-38165.1 | uAuGGuccuAGuAGGAAAudTdT | 978 | AUUUCCuACuAGGACcAuAdTdT | 1405 |
| AD-38171.1 | AuGGuccuAGuAGGAAAuAdTdT | 979 | uAUUUCCuACuAGGACcAUdTdT | 1406 |
| AD-38130.1 | uGGuccuAGuAGGAAAuAAdTdT | 980 | UuAUUUCCuACuAGGACcAdTdT | 1407 |
| AD-38136.1 | GGuccuAGuAGGAAAuAAAdTdT | 981 | UUuAUUUCCuACuAGGACCdTdT | 1408 |
| AD-38142.1 | GuccuAGuAGGAAAuAAAudTdT | 982 | AUUuAUUUCCuACuAGGACdTdT | 1409 |
| AD-38148.1 | uccuAGuAGGAAAuAAAuGdTdT | 983 | cAUUuAUUUCCuACuAGGAdTdT | 1410 |
| AD-38154.1 | ccuAGuAGGAAAuAAAuGudTdT | 984 | AcAUUuAUUUCCuACuAGGdTdT | 1411 |
| AD-38160.1 | cuAGuAGGAAAuAAAuGuGdTdT | 985 | cAcAUUuAUUUCCuACuAGdTdT | 1412 |
| AD-38166.1 | uAGuAGGAAAuAAAuGuGAdTdT | 986 | UcAcAUUuAUUUCCuACuAdTdT | 1413 |
| AD-38172.1 | AGuAGGAAAuAAAuGuGAudTdT | 987 | AUcAcAUUuAUUUCCuACUdTdT | 1414 |
| AD-38131.1 | GGAAuuccuuuuAuuGAAAdTdT | 988 | UUUcAAuAAAAGGAAUUCCdTdT | 1415 |
| AD-38137.1 | GAAuuccuuuuAuuGAAAcdTdT | 989 | GUUUcAAuAAAAGGAAUUCdTdT | 1416 |

(continued)

| Duplex | Sense Sequence | Seq SEQ ID NO: | Anti sense Sequence | Seq SEQ ID NO: |
|---|---|---|---|---|
| AD-38143.1 | AAuuccuuuuAuuGAAAcAdTdT | 990 | UGUUUcAAuAAAAGGAAUUdTdT | 1417 |
| AD-38149.1 | AuuccuuuuAuuGAAAcAudTdT | 991 | AUGUUUcAAuAAAAGGAAUdTdT | 1418 |
| AD-38155.1 | uuccuuuuAuuGAAAcAucdTdT | 992 | GAUGUUUcAAuAAAAGGAAdTdT | 1419 |
| AD-38161.1 | uccuuuuAuuGAAAcAucAdTdT | 993 | UGAUGUUUcAAuAAAAGGAdTdT | 1420 |
| AD-38167.1 | ccuuuuAuuGAAAcAucAGdTdT | 994 | CUGAUGUUUcAAuAAAAGGdTdT | 1421 |
| AD-38173.1 | cuuuuAuuGAAAcAucAGcdTdT | 995 | GCUGAUGUUUcAAuAAAAGdTdT | 1422 |
| AD-38132.1 | uuuuAuuGAAAcAucAGcAdTdT | 996 | UGCUGAUGUUUcAAuAAAAdTdT | 1423 |
| AD-38138.1 | uuuAuuGAAAcAucAGcAAdTdT | 997 | UUGCUGAUGUUUcAAuAAAdTdT | 1424 |
| AD-38144.1 | uuAuuGAAAcAucAGcAAAdTdT | 998 | UUUGCUGAUGUUUcAAuAAdTdT | 1425 |
| AD-38150.1 | uAuuGAAAcAucAGcAAAGdTdT | 999 | CUUUGCUGAUGUUUcAAuAdTdT | 1426 |
| AD-38156.1 | AuuGAAAcAucAGcAAAGAdTdT | 1000 | UCUUUGCUGAUGUUUcAAUdTdT | 1427 |
| AD-38162.1 | uuGAAAcAucAGcAAAGAcdTdT | 1001 | GUCUUUGCUGAUGUUUcAAdTdT | 1428 |
| AD-38168.1 | uGAAAcAucAGcAAAGAcAdTdT | 1002 | UGUCUUUGCUGAUGUUUcAdTdT | 1429 |
| AD-38174.1 | GAAAcAucAGcAAAGAcAAdTdT | 1003 | UUGUCUUUGCUGAUGUUUCdTdT | 1430 |
| AD-39683.1 | AuGAcuGAAuAuAAAcuuGdTdT | 1004 | cAAGUUuAuAUUcAGUcAUdTdT | 1431 |
| AD-39689.1 | uGAcuGAAuAuAAAcuuGudTdT | 1005 | AcAAGUUuAuAUUcAGUcAdTdT | 1432 |
| AD-39695.1 | GAcuGAAuAuAAAcuuGuGdTdT | 1006 | cAcAAGUUuAuAUUcAGUCdTdT | 1433 |
| AD-39701.1 | AcuGAAuAuAAAcuuGuGGdTdT | 1007 | CcAcAAGUUuAuAUUcAGUdTdT | 1434 |
| AD-39707.1 | cuGAAuAuAAAcuuGuGGudTdT | 1008 | ACcAcAAGUUuAuAUUcAGdTdT | 1435 |
| AD-39713.1 | uGAAuAuAAAcuuGuGGuAdTdT | 1009 | uACcAcAAGUUuAuAUUcAdTdT | 1436 |
| AD-39719.1 | GAAuAuAAAcuuGuGGuAGdTdT | 1010 | CuACcAcAAGUUuAuAUUCdTdT | 1437 |
| AD-39725.1 | AAuAuAAAcuuGuGGuAGudTdT | 1011 | ACuACcAcAAGUUuAuAUUdTdT | 1438 |
| AD-39684.1 | AuAuAAAcuuGuGGuAGuudTdT | 1012 | AACuACcAcAAGUUuAuAUdTdT | 1439 |
| AD-39690.1 | uAuAAAcuuGuGGuAGuuGdTdT | 1013 | cAACuACcAcAAGUUuAuAdTdT | 1440 |
| AD-39696.1 | AuAAAcuuGuGGuAGuuGGdTdT | 1014 | CcAACuACcAcAAGUUuAUdTdT | 1441 |
| AD-39702.1 | uAAAcuuGuGGuAGuuGGAdTdT | 1015 | UCcAACuACcAcAAGUUuAdTdT | 1442 |
| AD-39708.1 | AAAcuuGuGGuAGuuGGAGdTdT | 1016 | CUCcAACuACcAcAAGUUUdTdT | 1443 |
| AD-39714.1 | AAcuuGuGGuAGuuGGAGcdTdT | 1017 | GCUCcAACuACcAcAAGUUdTdT | 1444 |
| AD-39720.1 | AcuuGuGGuAGuuGGAGcudTdT | 1018 | AGCUCcAACuACcAcAAGUdTdT | 1445 |
| AD-39726.1 | cuuGuGGuAGuuGGAGcuGdTdT | 1019 | cAGCUCcAACuACcAcAAGdTdT | 1446 |
| AD-39685.1 | uuGuGGuAGuuGGAGcuGGdTdT | 1020 | CcAGCUCcAACuACcAcAAdTdT | 1447 |
| AD-39691.1 | uGuGGuAGuuGGAGcuGGudTdT | 1021 | ACcAGCUCcAACuACcAcAdTdT | 1448 |
| AD-39697.1 | GuGGuAGuuGGAGcuGGuGdTdT | 1022 | cACcAGCUCcAACuACcACdTdT | 1449 |
| AD-39703.1 | uGGuAGuuGGAGcuGGuGGdTdT | 1023 | CcACcAGCUCcAACuACcAdTdT | 1450 |
| AD-39709.1 | GGuAGuuGGAGcuGGuGGcdTdT | 1024 | GCcACcAGCUCcAACuACCdTdT | 1451 |
| AD-39715.1 | GuAGuuGGAGcuGGuGGcGdTdT | 1025 | CGCcACcAGCUCcAACuACdTdT | 1452 |

(continued)

| Duplex | Sense Sequence | Seq SEQ ID NO: | Anti sense Sequence | Seq SEQ ID NO: |
|---|---|---|---|---|
| AD-39721.1 | uAGuuGGAGcuGGuGGcGudTdT | 1026 | ACGCcACcAGCUCcAACuAdTdT | 1453 |
| AD-39727.1 | AGuuGGAGcuGGuGGcGuAdTdT | 1027 | uACGCcACcAGCUCcAACUdTdT | 1454 |
| AD-39686.1 | GuuGGAGcuGGuGGcGuAGdTdT | 1028 | CuACGCcACcAGCUCcAACdTdT | 1455 |
| AD-39692.1 | uuGGAGcuGGuGGcGuAGGdTdT | 1029 | CCuACGCcACcAGCUCcAAdTdT | 1456 |
| AD-39698.1 | uGGAGcuGGuGGcGuAGGcdTdT | 1030 | GCCuACGCcACcAGCUCcAdTdT | 1457 |
| AD-39704.1 | GGAGcuGGuGGcGuAGGcAdTdT | 1031 | UGCCuACGCcACcAGCUCCdTdT | 1458 |
| AD-39710.1 | GAGcuGGuGGcGuAGGcAAdTdT | 1032 | UUGCCuACGCcACcAGCUCdTdT | 1459 |
| AD-39716.1 | AGcuGGuGGcGuAGGcAAGdTdT | 1033 | CUUGCCuACGCcACcAGCUdTdT | 1460 |
| AD-39722.1 | GcuGGuGGcGuAGGcAAGAdTdT | 1034 | UCUUGCCuACGCcACcAGCdTdT | 1461 |
| AD-39728.1 | cuGGuGGcGuAGGcAAGAGdTdT | 1035 | CUCUUGCCuACGCcACcAGdTdT | 1462 |
| AD-39687.1 | uGGuGGcGuAGGcAAGAGudTdT | 1036 | ACUCUUGCCuACGCcACcAdTdT | 1463 |
| AD-39693.1 | GGuGGcGuAGGcAAGAGuGdTdT | 1037 | cACUCUUGCCuACGCcACCdTdT | 1464 |
| AD-39699.1 | GuGGcGuAGGcAAGAGuGcdTdT | 1038 | GcACUCUUGCCuACGCcACdTdT | 1465 |
| AD-39705.1 | uGGcGuAGGcAAGAGuGccdTdT | 1039 | GGcACUCUUGCCuACGCcAdTdT | 1466 |
| AD-39711.1 | GGcGuAGGcAAGAGuGccudTdT | 1040 | AGGcACUCUUGCCuACGCCdTdT | 1467 |
| AD-39717.1 | GcGuAGGcAAGAGuGccuudTdT | 1041 | AAGGcACUCUUGCCuACGCdTdT | 1468 |
| AD-39723.1 | cGuAGGcAAGAGuGccuuGdTdT | 1042 | cAAGGcACUCUUGCCuACGdTdT | 1469 |
| AD-39729.1 | GuAGGcAAGAGuGccuuGAdTdT | 1043 | UcAAGGcACUCUUGCCuACdTdT | 1470 |
| AD-39688.1 | uAGGcAAGAGuGccuuGAcdTdT | 1044 | GUcAAGGcACUCUUGCCuAdTdT | 1471 |
| AD-39694.1 | AGGcAAGAGuGccuuGAcGdTdT | 1045 | CGUcAAGGcACUCUUGCCUdTdT | 1472 |
| AD-39700.1 | GGcAAGAGuGccuuGAcGAdTdT | 1046 | UCGUcAAGGcACUCUUGCCdTdT | 1473 |
| AD-39706.1 | GcAAGAGuGccuuGAcGAudTdT | 1047 | AUCGUcAAGGcACUCUUGCdTdT | 1474 |
| AD-39712.1 | cAAGAGuGccuuGAcGAuAdTdT | 1048 | uAUCGUcAAGGcACUCUUGdTdT | 1475 |
| AD-39718.1 | AAGAGuGccuuGAcGAuAcdTdT | 1049 | GuAUCGUcAAGGcACUCUUdTdT | 1476 |
| AD-39724.1 | AGAGuGccuuGAcGAuAcAdTdT | 1050 | UGuAUCGUcAAGGcACUCUdTdT | 1477 |
| AD-39730.1 | GAGuGccuuGAcGAuAcAGdTdT | 1051 | CUGuAUCGUcAAGGcACUCdTdT | 1478 |
| AD-39736.1 | AGuGccuuGAcGAuAcAGcdTdT | 1052 | GCUGuAUCGUcAAGGcACUdTdT | 1479 |
| AD-39742.1 | GuGccuuGAcGAuAcAGcudTdT | 1053 | AGCUGuAUCGUcAAGGcACdTdT | 1480 |
| AD-39748.1 | uGccuuGAcGAuAcAGcuAdTdT | 1054 | uAGCUGuAUCGUcAAGGcAdTdT | 1481 |
| AD-39754.1 | GccuuGAcGAuAcAGcuAAdTdT | 1055 | UuAGCUGuAUCGUcAAGGCdTdT | 1482 |
| AD-39760.1 | ccuuGAcGAuAcAGcuAAudTdT | 1056 | AUuAGCUGuAUCGUcAAGGdTdT | 1483 |
| AD-39766.1 | cuuGAcGAuAcAGcuAAuudTdT | 1057 | AAUuAGCUGuAUCGUcAAGdTdT | 1484 |
| AD-39772.1 | uuGAcGAuAcAGcuAAuucdTdT | 1058 | GAAUuAGCUGuAUCGUcAAdTdT | 1485 |
| AD-39731.1 | uGAcGAuAcAGcuAAuucAdTdT | 1059 | UGAAUuAGCUGuAUCGUcAdTdT | 1486 |
| AD-39737.1 | GAcGAuAcAGcuAAuucAGdTdT | 1060 | CUGAAUuAGCUGuAUCGUCdTdT | 1487 |
| AD-39743.1 | AcGAuAcAGcuAAuucAGAdTdT | 1061 | UCUGAAUuAGCUGuAUCGUdTdT | 1488 |

(continued)

| Duplex | Sense Sequence | Seq SEQ ID NO: | Anti sense Sequence | Seq SEQ ID NO: |
|---|---|---|---|---|
| AD-39749.1 | cGAuAcAGcuAAuucAGAAdTdT | 1062 | UUCUGAAUuAGCUGuAUCGdTdT | 1489 |
| AD-39755.1 | GAuAcAGcuAAuucAGAAudTdT | 1063 | AUUCUGAAUuAGCUGuAUCdTdT | 1490 |
| AD-39761.1 | AuAcAGcuAAuucAGAAucdTdT | 1064 | GAUUCUGAAUuAGCUGuAUdTdT | 1491 |
| AD-39767.1 | uAcAGcuAAuucAGAAucAdTdT | 1065 | UGAUUCUGAAUuAGCUGuAdTdT | 1492 |
| AD-39773.1 | AcAGcuAAuucAGAAucAudTdT | 1066 | AUGAUUCUGAAUuAGCUGUdTdT | 1493 |
| AD-39732.1 | cAGcuAAuucAGAAucAuudTdT | 1067 | AAUGAUUCUGAAUuAGCUGdTdT | 1494 |
| AD-39738.1 | AGcuAAuucAGAAucAuuudTdT | 1068 | AAAUGAUUCUGAAUuAGCUdTdT | 1495 |
| AD-39744.1 | GcuAAuucAGAAucAuuuudTdT | 1069 | AAAAUGAUUCUGAAUuAGCdTdT | 1496 |
| AD-39750.1 | cuAAuucAGAAucAuuuuGdTdT | 1070 | cAAAAUGAUUCUGAAUuAGdTdT | 1497 |
| AD-39756.1 | uAAuucAGAAucAuuuuGudTdT | 1071 | AcAAAAUGAUUCUGAAUuAdTdT | 1498 |
| AD-39762.1 | AAuucAGAAucAuuuuGuGdTdT | 1072 | cAcAAAAUGAUUCUGAAUUdTdT | 1499 |
| AD-39768.1 | AuucAGAAucAuuuuGuGGdTdT | 1073 | CcAcAAAAUGAUUCUGAAUdTdT | 1500 |
| AD-39774.1 | uucAGAAucAuuuuGuGGAdTdT | 1074 | UCcAcAAAAUGAUUCUGAAdTdT | 1501 |
| AD-39733.1 | ucAGAAucAuuuuGuGGAcdTdT | 1075 | GUCcAcAAAAUGAUUCUGAdTdT | 1502 |
| AD-39739.1 | cAGAAucAuuuuGuGGAcGdTdT | 1076 | CGUCcAcAAAAUGAUUCUGdTdT | 1503 |
| AD-39745.1 | AGAAucAuuuuGuGGAcGAdTdT | 1077 | UCGUCcAcAAAAUGAUUCUdTdT | 1504 |
| AD-39751.1 | GAAucAuuuuGuGGAcGAAdTdT | 1078 | UUCGUCcAcAAAAUGAUUCdTdT | 1505 |
| AD-39757.1 | AAucAuuuuGuGGAcGAAudTdT | 1079 | AUUCGUCcAcAAAAUGAUUdTdT | 1506 |
| AD-39763.1 | AucAuuuuGuGGAcGAAuAdTdT | 1080 | uAUUCGUCcAcAAAAUGAUdTdT | 1507 |
| AD-39769.1 | ucAuuuuGuGGAcGAAuAudTdT | 1081 | AuAUUCGUCcAcAAAAUGAdTdT | 1508 |
| AD-39775.1 | cAuuuuGuGGAcGAAuAuGdTdT | 1082 | cAuAUUCGUCcAcAAAAUGdTdT | 1509 |
| AD-39734.1 | AuuuuGuGGAcGAAuAuGAdTdT | 1083 | UcAuAUUCGUCcAcAAAAUdTdT | 1510 |
| AD-39740.1 | uuuuGuGGAcGAAuAuGAudTdT | 1084 | AUcAuAUUCGUCcAcAAAAdTdT | 1511 |
| AD-39746.1 | uuuGuGGAcGAAuAuGAucdTdT | 1085 | GAUcAuAUUCGUCcAcAAAdTdT | 1512 |
| AD-39752.1 | uuGuGGAcGAAuAuGAuccdTdT | 1086 | GGAUcAuAUUCGUCcAcAAdTdT | 1513 |
| AD-39758.1 | uGuGGAcGAAuAuGAuccAdTdT | 1087 | UGGAUcAuAUUCGUCcAcAdTdT | 1514 |
| AD-39764.1 | GuGGAcGAAuAuGAuccAAdTdT | 1088 | UUGGAUcAuAUUCGUCcACdTdT | 1515 |
| AD-39770.1 | uGGAcGAAuAuGAuccAAcdTdT | 1089 | GUUGGAUcAuAUUCGUCcAdTdT | 1516 |
| AD-39776.1 | AGGAAGcAAGuAGuAAuuGdTdT | 1090 | cAAUuACuACUUGCUUCCUdTdT | 1517 |
| AD-39735.1 | GGAAGcAAGuAGuAAuuGAdTdT | 1091 | UcAAUuACuACUUGCUUCCdTdT | 1518 |
| AD-39741.1 | GAAGcAAGuAGuAAuuGAudTdT | 1092 | AUcAAUuACuACUUGCUUCdTdT | 1519 |
| AD-39747.1 | AAGcAAGuAGuAAuuGAuGdTdT | 1093 | cAUcAAUuACuACUUGCUUdTdT | 1520 |
| AD-39753.1 | AGcAAGuAGuAAuuGAuGGdTdT | 1094 | CcAUcAAUuACuACUUGCUdTdT | 1521 |
| AD-39759.1 | GcAAGuAGuAAuuGAuGGAdTdT | 1095 | UCcAUcAAUuACuACUUGCdTdT | 1522 |
| AD-39765.1 | cAAGuAGuAAuuGAuGGAGdTdT | 1096 | CUCcAUcAAUuACuACUUGdTdT | 1523 |
| AD-39771.1 | AAGuAGuAAuuGAuGGAGAdTdT | 1097 | UCUCcAUcAAUuACuACUUdTdT | 1524 |

(continued)

| Duplex | Sense Sequence | Seq SEQ ID NO: | Anti sense Sequence | Seq SEQ ID NO: |
|---|---|---|---|---|
| AD-39778.1 | AGuAGuAAuuGAuGGAGAAdTdT | 1098 | UUCUCcAUcAAUuACuACUdTdT | 1525 |
| AD-39784.1 | GuAGuAAuuGAuGGAGAAAdTdT | 1099 | UUUCUCcAUcAAUuACuACdTdT | 1526 |
| AD-39790.1 | uAGuAAuuGAuGGAGAAAcdTdT | 1100 | GUUUCUCcAUcAAUuACuAdTdT | 1527 |
| AD-39796.1 | AGuAAuuGAuGGAGAAAccdTdT | 1101 | GGUUUCUCcAUcAAUuACUdTdT | 1528 |
| AD-39802.1 | GuAAuuGAuGGAGAAAccudTdT | 1102 | AGGUUUCUCcAUcAAUuACdTdT | 1529 |
| AD-39808.1 | uAAuuGAuGGAGAAAccuGdTdT | 1103 | cAGGUUUCUCcAUcAAUuAdTdT | 1530 |
| AD-39814.1 | AAuuGAuGGAGAAAccuGudTdT | 1104 | AcAGGUUUCUCcAUcAAUUdTdT | 1531 |
| AD-39820.1 | AuuGAuGGAGAAAccuGucdTdT | 1105 | GAcAGGUUUCUCcAUcAAUdTdT | 1532 |
| AD-39779.1 | uuGAuGGAGAAAccuGucudTdT | 1106 | AGAcAGGUUUCUCcAUcAAdTdT | 1533 |
| AD-39785.1 | uGAuGGAGAAAccuGucucdTdT | 1107 | GAGAcAGGUUUCUCcAUcAdTdT | 1534 |
| AD-39791.1 | GAuGGAGAAAccuGucucudTdT | 1108 | AGAGAcAGGUUUCUCcAUCdTdT | 1535 |
| AD-39797.1 | AuGGAGAAAccuGucucuudTdT | 1109 | AAGAGAcAGGUUUCUCcAUdTdT | 1536 |
| AD-39803.1 | uGGAGAAAccuGucucuuGdTdT | 1110 | cAAGAGAcAGGUUUCUCcAdTdT | 1537 |
| AD-39809.1 | GGAGAAAccuGucucuuGGdTdT | 1111 | CcAAGAGAcAGGUUUCUCCdTdT | 1538 |
| AD-39815.1 | GAGAAAccuGucucuuGGAdTdT | 1112 | UCcAAGAGAcAGGUUUCUCdTdT | 1539 |
| AD-39821.1 | AGAAAccuGucucuuGGAudTdT | 1113 | AUCcAAGAGAcAGGUUUCUdTdT | 1540 |
| AD-39780.1 | GAAAccuGucucuuGGAuAdTdT | 1114 | uAUCcAAGAGAcAGGUUUCdTdT | 1541 |
| AD-39786.1 | AAAccuGucucuuGGAuAudTdT | 1115 | AuAUCcAAGAGAcAGGUUUdTdT | 1542 |
| AD-39792.1 | AAccuGucucuuGGAuAuudTdT | 1116 | AAuAUCcAAGAGAcAGGUUdTdT | 1543 |
| AD-39798.1 | AccuGucucuuGGAuAuucdTdT | 1117 | GAAuAUCcAAGAGAcAGGUdTdT | 1544 |
| AD-39804.1 | ccuGucucuuGGAuAuucudTdT | 1118 | AGAAuAUCcAAGAGAcAGGdTdT | 1545 |
| AD-39810.1 | cuGucucuuGGAuAuucucdTdT | 1119 | GAGAAuAUCcAAGAGAcAGdTdT | 1546 |
| AD-39816.1 | uGucucuuGGAuAuucucGdTdT | 1120 | CGAGAAuAUCcAAGAGAcAdTdT | 1547 |
| AD-39822.1 | GucucuuGGAuAuucucGAdTdT | 1121 | UCGAGAAuAUCcAAGAGACdTdT | 1548 |
| AD-39781.1 | ucucuuGGAuAuucucGAcdTdT | 1122 | GUCGAGAAuAUCcAAGAGAdTdT | 1549 |
| AD-39787.1 | cucuuGGAuAuucucGAcAdTdT | 1123 | UGUCGAGAAuAUCcAAGAGdTdT | 1550 |
| AD-39793.1 | ucuuGGAuAuucucGAcAcdTdT | 1124 | GUGUCGAGAAuAUCcAAGAdTdT | 1551 |
| AD-39799.1 | cuuGGAuAuucucGAcAcAdTdT | 1125 | UGUGUCGAGAAuAUCcAAGdTdT | 1552 |
| AD-39805.1 | AGuAcAGuGcAAuGAGGGAdTdT | 1126 | UCCCUcAUUGcACUGuACUdTdT | 1553 |
| AD-39811.1 | GuAcAGuGcAAuGAGGGAcdTdT | 1127 | GUCCCUcAUUGcACUGuACdTdT | 1554 |
| AD-39817.1 | uAcAGuGcAAuGAGGGAccdTdT | 1128 | GGUCCCUcAUUGcACUGuAdTdT | 1555 |
| AD-39823.1 | AcAGuGcAAuGAGGGAccAdTdT | 1129 | UGGUCCCUcAUUGcACUGUdTdT | 1556 |
| AD-39782.1 | cAGuGcAAuGAGGGAccAGdTdT | 1130 | CUGGUCCCUcAUUGcACUGdTdT | 1557 |
| AD-39788.1 | AGuGcAAuGAGGGAccAGudTdT | 1131 | ACUGGUCCCUcAUUGcACUdTdT | 1558 |
| AD-39794.1 | GuGcAAuGAGGGAccAGuAdTdT | 1132 | uACUGGUCCCUcAUUGcACdTdT | 1559 |
| AD-39800.1 | uGcAAuGAGGGAccAGuAcdTdT | 1133 | GuACUGGUCCCUcAUUGcAdTdT | 1560 |

(continued)

| Duplex | Sense Sequence | Seq SEQ ID NO: | Anti sense Sequence | Seq SEQ ID NO: |
|---|---|---|---|---|
| AD-39806.1 | GcAAuGAGGGAccAGuAcAdTdT | 1134 | UGuACUGGUCCCUcAUUGCdTdT | 1561 |
| AD-39812.1 | cAAuGAGGGAccAGuAcAudTdT | 1135 | AUGuACUGGUCCCUcAUUGdTdT | 1562 |
| AD-39818.1 | AAuGAGGGAccAGuAcAuGdTdT | 1136 | cAUGuACUGGUCCCUcAUUdTdT | 1563 |
| AD-39824.1 | AuGAGGGAccAGuAcAuGAdTdT | 1137 | UcAUGuACUGGUCCCUcAUdTdT | 1564 |
| AD-39783.1 | uGAGGGAccAGuAcAuGAGdTdT | 1138 | CUcAUGuACUGGUCCCUcAdTdT | 1565 |
| AD-39789.1 | GAGGGAccAGuAcAuGAGGdTdT | 1139 | CCUcAUGuACUGGUCCCUCdTdT | 1566 |
| AD-39795.1 | AGGGAccAGuAcAuGAGGAdTdT | 1140 | UCCUcAUGuACUGGUCCCUdTdT | 1567 |
| AD-39801.1 | GGGAccAGuAcAuGAGGAcdTdT | 1141 | GUCCUcAUGuACUGGUCCCdTdT | 1568 |
| AD-39807.1 | GGAccAGuAcAuGAGGAcudTdT | 1142 | AGUCCUcAUGuACUGGUCCdTdT | 1569 |
| AD-39813.1 | GAccAGuAcAuGAGGAcuGdTdT | 1143 | cAGUCCUcAUGuACUGGUCdTdT | 1570 |
| AD-39819.1 | AccAGuAcAuGAGGAcuGGdTdT | 1144 | CcAGUCCUcAUGuACUGGUdTdT | 1571 |
| AD-39825.1 | ccAGuAcAuGAGGAcuGGGdTdT | 1145 | CCcAGUCCUcAUGuACUGGdTdT | 1572 |
| AD-39831.1 | cAGuAcAuGAGGAcuGGGGdTdT | 1146 | CCCcAGUCCUcAUGuACUGdTdT | 1573 |
| AD-39837.1 | AGuAcAuGAGGAcuGGGGAdTdT | 1147 | UCCCcAGUCCUcAUGuACUdTdT | 1574 |
| AD-39843.1 | GuAcAuGAGGAcuGGGGAGdTdT | 1148 | CUCCCcAGUCCUcAUGuACdTdT | 1575 |
| AD-39849.1 | uAcAuGAGGAcuGGGGAGGdTdT | 1149 | CCUCCCcAGUCCUcAUGuAdTdT | 1576 |
| AD-39855.1 | AcAuGAGGAcuGGGGAGGGdTdT | 1150 | CCCUCCCcAGUCCUcAUGUdTdT | 1577 |
| AD-39861.1 | cAuGAGGAcuGGGGAGGGcdTdT | 1151 | GCCCUCCCcAGUCCUcAUGdTdT | 1578 |
| AD-39867.1 | AuGAGGAcuGGGGAGGGcudTdT | 1152 | AGCCCUCCCcAGUCCUcAUdTdT | 1579 |
| AD-39826.1 | uGAGGAcuGGGGAGGGcuudTdT | 1153 | AAGCCCUCCCcAGUCCUcAdTdT | 1580 |
| AD-39832.1 | GAGGAcuGGGGAGGGcuuudTdT | 1154 | AAAGCCCUCCCcAGUCCUCdTdT | 1581 |
| AD-39838.1 | AGGAcuGGGGAGGGcuuucdTdT | 1155 | GAAAGCCCUCCCcAGUCCUdTdT | 1582 |
| AD-39844.1 | GGAcuGGGGAGGGcuuucudTdT | 1156 | AGAAAGCCCUCCCcAGUCCdTdT | 1583 |
| AD-39850.1 | GAcuGGGGAGGGcuuucuudTdT | 1157 | AAGAAAGCCCUCCCcAGUCdTdT | 1584 |
| AD-39856.1 | AcuGGGGAGGGcuuucuuudTdT | 1158 | AAAGAAAGCCCUCCCcAGUdTdT | 1585 |
| AD-39862.1 | cuGGGGAGGGcuuucuuuGdTdT | 1159 | cAAAGAAAGCCCUCCCcAGdTdT | 1586 |
| AD-39868.1 | uGGGGAGGGcuuucuuuGudTdT | 1160 | AcAAAGAAAGCCCUCCCcAdTdT | 1587 |
| AD-39827.1 | uuGccAuAAAuAAuAcuAAdTdT | 1161 | UuAGuAUuAUUuAUGGcAAdTdT | 1588 |
| AD-39833.1 | uGccAuAAAuAAuAcuAAAdTdT | 1162 | UUuAGuAUuAUUuAUGGcAdTdT | 1589 |
| AD-39839.1 | GccAuAAAuAAuAcuAAAudTdT | 1163 | AUUuAGuAUuAUUuAUGGCdTdT | 1590 |
| AD-39845.1 | ccAuAAAuAAuAcuAAAucdTdT | 1164 | GAUUuAGuAUuAUUuAUGGdTdT | 1591 |
| AD-39851.1 | cAuAAAuAAuAcuAAAucAdTdT | 1165 | UGAUUuAGuAUuAUUuAUGdTdT | 1592 |
| AD-39857.1 | AuAAAuAAuAcuAAAucAudTdT | 1166 | AUGAUUuAGuAUuAUUuAUdTdT | 1593 |
| AD-39863.1 | uAAAuAAuAcuAAAucAuudTdT | 1167 | AAUGAUUuAGuAUuAUUuAdTdT | 1594 |
| AD-39869.1 | AAAuAAuAcuAAAucAuuudTdT | 1168 | AAAUGAUUuAGuAUuAUUUdTdT | 1595 |
| AD-39828.1 | AAuAAuAcuAAAucAuuuGdTdT | 1169 | cAAAUGAUUuAGuAUuAUUdTdT | 1596 |

(continued)

| Duplex | Sense Sequence | Seq SEQ ID NO: | Anti sense Sequence | Seq SEQ ID NO: |
|---|---|---|---|---|
| AD-39834.1 | AuAAuAcuAAAucAuuuGAdTdT | 1170 | UcAAAUGAUUuAGuAUuAUdTdT | 1597 |
| AD-39840.1 | uAAuAcuAAAucAuuuGAAdTdT | 1171 | UUcAAAUGAUUuAGuAUuAdTdT | 1598 |
| AD-39846.1 | AAuAcuAAAucAuuuGAAGdTdT | 1172 | CUUcAAAUGAUUuAGuAUUdTdT | 1599 |
| AD-39852.1 | AuAcuAAAucAuuuGAAGAdTdT | 1173 | UCUUcAAAUGAUUuAGuAUdTdT | 1600 |
| AD-39858.1 | uAcuAAAucAuuuGAAGAudTdT | 1174 | AUCUUcAAAUGAUUuAGuAdTdT | 1601 |
| AD-39864.1 | AcuAAAucAuuuGAAGAuAdTdT | 1175 | uAUCUUcAAAUGAUUuAGUdTdT | 1602 |
| AD-39870.1 | cuAAAucAuuuGAAGAuAudTdT | 1176 | AuAUCUUcAAAUGAUUuAGdTdT | 1603 |
| AD-39829.1 | uAAAucAuuuGAAGAuAuudTdT | 1177 | AAuAUCUUcAAAUGAUUuAdTdT | 1604 |
| AD-39835.1 | AAAucAuuuGAAGAuAuucdTdT | 1178 | GAAuAUCUUcAAAUGAUUUdTdT | 1605 |
| AD-39841.1 | AAucAuuuGAAGAuAuucAdTdT | 1179 | UGAAuAUCUUcAAAUGAUUdTdT | 1606 |
| AD-39853.1 | ucAuuuGAAGAuAuucAccdTdT | 1180 | GGUGAAuAUCUUcAAAUGAdTdT | 1607 |
| AD-39859.1 | cAuuuGAAGAuAuucAccAdTdT | 1181 | UGGUGAAuAUCUUcAAAUGdTdT | 1608 |
| AD-39865.1 | AuuuGAAGAuAuucAccAudTdT | 1182 | AUGGUGAAuAUCUUcAAAUdTdT | 1609 |
| AD-39871.1 | uuuGAAGAuAuucAccAuudTdT | 1183 | AAUGGUGAAuAUCUUcAAAdTdT | 1610 |
| AD-39830.1 | uuGAAGAuAuucAccAuuAdTdT | 1184 | uAAUGGUGAAuAUCUUcAAdTdT | 1611 |
| AD-39836.1 | GAGAAcAAAuuAAAAGAGudTdT | 1185 | ACUCUUUuAAUUUGUUCUCdTdT | 1612 |
| AD-39842.1 | AGAAcAAAuuAAAAGAGuudTdT | 1186 | AACUCUUUuAAUUUGUUCUdTdT | 1613 |
| AD-39848.1 | GAAcAAAuuAAAAGAGuuAdTdT | 1187 | uAACUCUUUuAAUUUGUUCdTdT | 1614 |
| AD-39854.1 | AAcAAAuuAAAAGAGuuAAdTdT | 1188 | UuAACUCUUUuAAUUUGUUdTdT | 1615 |
| AD-39860.1 | AcAAAuuAAAAGAGuuAAGdTdT | 1189 | CUuAACUCUUUuAAUUUGUdTdT | 1616 |
| AD-39866.1 | cAAAuuAAAAGAGuuAAGGdTdT | 1190 | CCUuAACUCUUUuAAUUUGdTdT | 1617 |
| AD-39992.1 | AAAuuAAAAGAGuuAAGGAdTdT | 1191 | UCCUuAACUCUUUuAAUUUdTdT | 1618 |
| AD-39998.1 | AAuuAAAAGAGuuAAGGAcdTdT | 1192 | GUCCUuAACUCUUUuAAUUdTdT | 1619 |
| AD-40004.1 | AuuAAAAGAGuuAAGGAcudTdT | 1193 | AGUCCUuAACUCUUUuAAUdTdT | 1620 |
| AD-40010.1 | uuAAAAGAGuuAAGGAcucdTdT | 1194 | GAGUCCUuAACUCUUUuAAdTdT | 1621 |
| AD-40016.1 | uAAAAGAGuuAAGGAcucudTdT | 1195 | AGAGUCCUuAACUCUUUuAdTdT | 1622 |
| AD-40022.1 | AAAAGAGuuAAGGAcucuGdTdT | 1196 | cAGAGUCCUuAACUCUUUUdTdT | 1623 |
| AD-40028.1 | AAAGAGuuAAGGAcucuGAdTdT | 1197 | UcAGAGUCCUuAACUCUUUdTdT | 1624 |
| AD-40034.1 | AAGAGuuAAGGAcucuGAAdTdT | 1198 | UUcAGAGUCCUuAACUCUUdTdT | 1625 |
| AD-39999.1 | GAGuuAAGGAcucuGAAGAdTdT | 1199 | UCUUcAGAGUCCUuAACUCdTdT | 1626 |
| AD-40005.1 | AGuuAAGGAcucuGAAGAudTdT | 1200 | AUCUUcAGAGUCCUuAACUdTdT | 1627 |
| AD-40011.1 | GuuAAGGAcucuGAAGAuGdTdT | 1201 | cAUCUUcAGAGUCCUuAACdTdT | 1628 |
| AD-40017.1 | uuAAGGAcucuGAAGAuGudTdT | 1202 | AcAUCUUcAGAGUCCUuAAdTdT | 1629 |
| AD-40029.1 | AAGGAcucuGAAGAuGuAcdTdT | 1203 | GuAcAUCUUcAGAGUCCUUdTdT | 1630 |
| AD-40035.1 | AGGAcucuGAAGAuGuAccdTdT | 1204 | GGuAcAUCUUcAGAGUCCUdTdT | 1631 |
| AD-39994.1 | GGAcucuGAAGAuGuAccudTdT | 1205 | AGGuAcAUCUUcAGAGUCCdTdT | 1632 |

(continued)

| Duplex | Sense Sequence | Seq SEQ ID NO: | Anti sense Sequence | Seq SEQ ID NO: |
|---|---|---|---|---|
| AD-40000.1 | GAcucuGAAGAuGuAccuAdTdT | 1206 | uAGGuAcAUCUUcAGAGUCdTdT | 1633 |
| AD-40006.1 | AcucuGAAGAuGuAccuAudTdT | 1207 | AuAGGuAcAUCUUcAGAGUdTdT | 1634 |
| AD-40012.1 | cucuGAAGAuGuAccuAuGdTdT | 1208 | cAuAGGuAcAUCUUcAGAGdTdT | 1635 |
| AD-40018.1 | ucuGAAGAuGuAccuAuGGdTdT | 1209 | CcAuAGGuAcAUCUUcAGAdTdT | 1636 |
| AD-40024.1 | cuGAAGAuGuAccuAuGGudTdT | 1210 | ACcAuAGGuAcAUCUUcAGdTdT | 1637 |
| AD-40030.1 | uGAAGAuGuAccuAuGGucdTdT | 1211 | GACcAuAGGuAcAUCUUcAdTdT | 1638 |
| AD-40036.1 | GuAGGAAAuAAAuGuGAuudTdT | 1212 | AAUcAcAUUuAUUUCCuACdTdT | 1639 |
| AD-39995.1 | uAGGAAAuAAAuGuGAuuudTdT | 1213 | AAAUcAcAUUuAUUUCCuAdTdT | 1640 |
| AD-40001.1 | AGGAAAuAAAuGuGAuuuGdTdT | 1214 | cAAAUcAcAUUuAUUUCCUdTdT | 1641 |
| AD-40007.1 | GGAAAuAAAuGuGAuuuGcdTdT | 1215 | GcAAAUcAcAUUuAUUUCCdTdT | 1642 |
| AD-40013.1 | GAAAuAAAuGuGAuuuGccdTdT | 1216 | GGcAAAUcAcAUUuAUUUCdTdT | 1643 |
| AD-40019.1 | AAAuAAAuGuGAuuuGccudTdT | 1217 | AGGcAAAUcAcAUUuAUUUdTdT | 1644 |
| AD-40025.1 | AAuAAAuGuGAuuuGccuudTdT | 1218 | AAGGcAAAUcAcAUUuAUUdTdT | 1645 |
| AD-40031.1 | AuAAAuGuGAuuuGccuucdTdT | 1219 | GAAGGcAAAUcAcAUUuAUdTdT | 1646 |
| AD-40037.1 | uAAAuGuGAuuuGccuucudTdT | 1220 | AGAAGGcAAAUcAcAUUuAdTdT | 1647 |
| AD-39996.1 | AAAuGuGAuuuGccuucuAdTdT | 1221 | uAGAAGGcAAAUcAcAUUUdTdT | 1648 |
| AD-40002.1 | AAuGuGAuuuGccuucuAGdTdT | 1222 | CuAGAAGGcAAAUcAcAUUdTdT | 1649 |
| AD-40008.1 | AuGuGAuuuGccuucuAGAdTdT | 1223 | UCuAGAAGGcAAAUcAcAUdTdT | 1650 |
| AD-40014.1 | uGuGAuuuGccuucuAGAAdTdT | 1224 | UUCuAGAAGGcAAAUcAcAdTdT | 1651 |
| AD-40020.1 | GuGAuuuGccuucuAGAAcdTdT | 1225 | GUUCuAGAAGGcAAAUcACdTdT | 1652 |
| AD-40026.1 | uGAuuuGccuucuAGAAcAdTdT | 1226 | UGUUCuAGAAGGcAAAUcAdTdT | 1653 |
| AD-40032.1 | GAuuuGccuucuAGAAcAGdTdT | 1227 | CUGUUCuAGAAGGcAAAUCdTdT | 1654 |
| AD-40038.1 | AuuuGccuucuAGAAcAGudTdT | 1228 | ACUGUUCuAGAAGGcAAAUdTdT | 1655 |
| AD-39997.1 | uuuGccuucuAGAAcAGuAdTdT | 1229 | uACUGUUCuAGAAGGcAAAdTdT | 1656 |
| AD-40009.1 | uGccuucuAGAAcAGuAGAdTdT | 1230 | UCuACUGUUCuAGAAGGcAdTdT | 1657 |
| AD-40015.1 | GccuucuAGAAcAGuAGAcdTdT | 1231 | GUCuACUGUUCuAGAAGGCdTdT | 1658 |
| AD-40021.1 | ccuucuAGAAcAGuAGAcAdTdT | 1232 | UGUCuACUGUUCuAGAAGGdTdT | 1659 |
| AD-40027.1 | cuucuAGAAcAGuAGAcAcdTdT | 1233 | GUGUCuACUGUUCuAGAAGdTdT | 1660 |
| AD-40033.1 | uucuAGAAcAGuAGAcAcAdTdT | 1234 | UGUGUCuACUGUUCuAGAAdTdT | 1661 |
| AD-40039.1 | ucuAGAAcAGuAGAcAcAAdTdT | 1235 | UUGUGUCuACUGUUCuAGAdTdT | 1662 |
| AD-40045.1 | cuAGAAcAGuAGAcAcAAAdTdT | 1236 | UUUGUGUCuACUGUUCuAGdTdT | 1663 |
| AD-40051.1 | uAGAAcAGuAGAcAcAAAAdTdT | 1237 | UUUUGUGUCuACUGUUCuAdTdT | 1664 |
| AD-40057.1 | AGAAcAGuAGAcAcAAAAcdTdT | 1238 | GUUUUGUGUCuACUGUUCUdTdT | 1665 |
| AD-40063.1 | GAAcAGuAGAcAcAAAAcAdTdT | 1239 | UGUUUUGUGUCuACUGUUCdTdT | 1666 |
| AD-40069.1 | AAcAGuAGAcAcAAAAcAGdTdT | 1240 | CUGUUUUGUGUCuACUGUUdTdT | 1667 |
| AD-40075.1 | AcAGuAGAcAcAAAAcAGGdTdT | 1241 | CCUGUUUUGUGUCuACUGUdTdT | 1668 |

(continued)

| Duplex | Sense Sequence | Seq SEQ ID NO: | Anti sense Sequence | Seq SEQ ID NO: |
|---|---|---|---|---|
| AD-40081.1 | cAGuAGAcAcAAAAcAGGcdTdT | 1242 | GCCUGUUUUGUGUCuACUGdTdT | 1669 |
| AD-40040.1 | AGuAGAcAcAAAAcAGGcudTdT | 1243 | AGCCUGUUUUGUGUCuACUdTdT | 1670 |
| AD-40046.1 | GuAGAcAcAAAAcAGGcucdTdT | 1244 | GAGCCUGUUUUGUGUCuACdTdT | 1671 |
| AD-40052.1 | uAGAcAcAAAAcAGGcucAdTdT | 1245 | UGAGCCUGUUUUGUGUCuAdTdT | 1672 |
| AD-40058.1 | AGAcAcAAAAcAGGcucAGdTdT | 1246 | CUGAGCCUGUUUUGUGUCUdTdT | 1673 |
| AD-40064.1 | GAcAcAAAAcAGGcucAGGdTdT | 1247 | CCUGAGCCUGUUUUGUGUCdTdT | 1674 |
| AD-40070.1 | AcAcAAAAcAGGcucAGGAdTdT | 1248 | UCCUGAGCCUGUUUUGUGUdTdT | 1675 |
| AD-40076.1 | cAcAAAAcAGGcucAGGAcdTdT | 1249 | GUCCUGAGCCUGUUUUGUGdTdT | 1676 |
| AD-40082.1 | AcAAAAcAGGcucAGGAcudTdT | 1250 | AGUCCUGAGCCUGUUUUGUdTdT | 1677 |
| AD-40041.1 | cAAAAcAGGcucAGGAcuudTdT | 1251 | AAGUCCUGAGCCUGUUUUGdTdT | 1678 |
| AD-40047.1 | AAAAcAGGcucAGGAcuuAdTdT | 1252 | uAAGUCCUGAGCCUGUUUUdTdT | 1679 |
| AD-40053.1 | AAAcAGGcucAGGAcuuAGdTdT | 1253 | CuAAGUCCUGAGCCUGUUUdTdT | 1680 |
| AD-40059.1 | AAcAGGcucAGGAcuuAGcdTdT | 1254 | GCuAAGUCCUGAGCCUGUUdTdT | 1681 |
| AD-40065.1 | AcAGGcucAGGAcuuAGcAdTdT | 1255 | UGCuAAGUCCUGAGCCUGUdTdT | 1682 |
| AD-40071.1 | cAGGcucAGGAcuuAGcAAdTdT | 1256 | UUGCuAAGUCCUGAGCCUGdTdT | 1683 |
| AD-40077.1 | AGGcucAGGAcuuAGcAAGdTdT | 1257 | CUUGCuAAGUCCUGAGCCUdTdT | 1684 |
| AD-40083.1 | GGcucAGGAcuuAGcAAGAdTdT | 1258 | UCUUGCuAAGUCCUGAGCCdTdT | 1685 |
| AD-40042.1 | GcucAGGAcuuAGcAAGAAdTdT | 1259 | UUCUUGCuAAGUCCUGAGCdTdT | 1686 |
| AD-40048.1 | cucAGGAcuuAGcAAGAAGdTdT | 1260 | CUUCUUGCuAAGUCCUGAGdTdT | 1687 |
| AD-40054.1 | ucAGGAcuuAGcAAGAAGudTdT | 1261 | ACUUCUUGCuAAGUCCUGAdTdT | 1688 |
| AD-40060.1 | cAGGAcuuAGcAAGAAGuudTdT | 1262 | AACUUCUUGCuAAGUCCUGdTdT | 1689 |
| AD-40066.1 | AGGAcuuAGcAAGAAGuuAdTdT | 1263 | uAACUUCUUGCuAAGUCCUdTdT | 1690 |
| AD-40072.1 | GGAcuuAGcAAGAAGuuAudTdT | 1264 | AuAACUUCUUGCuAAGUCCdTdT | 1691 |
| AD-40078.1 | GAcuuAGcAAGAAGuuAuGdTdT | 1265 | cAuAACUUCUUGCuAAGUCdTdT | 1692 |
| AD-40084.1 | AcuuAGcAAGAAGuuAuGGdTdT | 1266 | CcAuAACUUCUUGCuAAGUdTdT | 1693 |
| AD-40043.1 | cuuAGcAAGAAGuuAuGGAdTdT | 1267 | UCcAuAACUUCUUGCuAAGdTdT | 1694 |
| AD-40049.1 | uuAGcAAGAAGuuAuGGAAdTdT | 1268 | UUCcAuAACUUCUUGCuAAdTdT | 1695 |
| AD-40055.1 | uAGcAAGAAGuuAuGGAAudTdT | 1269 | AUUCcAuAACUUCUUGCuAdTdT | 1696 |
| AD-40061.1 | AGcAAGAAGuuAuGGAAuudTdT | 1270 | AAUUCcAuAACUUCUUGCUdTdT | 1697 |
| AD-40067.1 | GcAAGAAGuuAuGGAAuucdTdT | 1271 | GAAUUCcAuAACUUCUUGCdTdT | 1698 |
| AD-40073.1 | cAAGAAGuuAuGGAAuuccdTdT | 1272 | GGAAUUCcAuAACUUCUUGdTdT | 1699 |
| AD-40079.1 | AAGAAGuuAuGGAAuuccudTdT | 1273 | AGGAAUUCcAuAACUUCUUdTdT | 1700 |
| AD-40085.1 | AGAAGuuAuGGAAuuccuudTdT | 1274 | AAGGAAUUCcAuAACUUCUdTdT | 1701 |
| AD-40044.1 | GAAGuuAuGGAAuuccuuudTdT | 1275 | AAAGGAAUUCcAuAACUUCdTdT | 1702 |
| AD-40050.1 | AAGuuAuGGAAuuccuuuudTdT | 1276 | AAAAGGAAUUCcAuAACUUdTdT | 1703 |
| AD-40056.1 | AGuuAuGGAAuuccuuuuAdTdT | 1277 | uAAAAGGAAUUCcAuAACUdTdT | 1704 |

(continued)

| Duplex | Sense Sequence | Seq SEQ ID NO: | Anti sense Sequence | Seq SEQ ID NO: |
|---|---|---|---|---|
| AD-40062.1 | GuuAuGGAAuuccuuuuAudTdT | 1278 | AuAAAAGGAAUUCcAuAACdTdT | 1705 |
| AD-40068.1 | uuAuGGAAuuccuuuuAuudTdT | 1279 | AAuAAAAGGAAUUCcAuAAdTdT | 1706 |
| AD-40074.1 | uAuGGAAuuccuuuuAuuGdTdT | 1280 | cAAuAAAAGGAAUUCcAuAdTdT | 1707 |
| AD-40080.1 | AuGGAAuuccuuuuAuuGAdTdT | 1281 | UcAAuAAAAGGAAUUCcAUdTdT | 1708 |
| AD-39720.1 | ActtGtGGtAGttGGAGcttt | 1792 | AGCTCcAACtACcAcAAGTtt | 1830 |
| AD-39706.1 | GcAAGAGtGccttGAcGAttt | 1793 | ATCGTcAAGGcACTCTTGCtt | 1831 |
| AD-39712.1 | cAAGAGtGccttGAcGAtAtt | 1794 | tATCGTcAAGGcACTCTTGtt | 1832 |
| AD-39760.1 | ccttGAcGAtAcAGctAAttt | 1795 | ATtAGCTGtATCGTcAAGGtt | 1833 |
| AD-39732.1 | cAGctAAttcAGAAtcAtttt | 1796 | AATGATTCTGAATtAGCTGtt | 1834 |
| AD-35541.4 | cGAAtAtGAtccAAcAAtAtt | 1797 | tATTGTTGGATcAtATTCGtt | 1835 |
| AD-39735.1 or hs_KRAS 321_A22S26 | GGAAGcAAGtAGtAAttGAuu | 1798 | TcAATtACtACTTGCTTCCuu | 1836 |
| AD-39741.1 or hs_KRAS 322_A22S26 | GAAGcAAGtAGtAAttGAtuu | 1799 | ATcAATtACtACTTGCTTCuu | 1837 |
| AD-39778.1 | AGtAGtAAttGAtGGAGAAtt | 1800 | TTCTCcATcAATtACtACTtt | 1838 |
| AD-39790.1 | tAGtAAttGAtGGAGAAActt | 1801 | GTTTCTCcATcAATtACtAtt | 1839 |
| AD-39822.1 | GtctcttGGAtAttctcGAtt | 1802 | TCGAGAAtATCcAAGAGACtt | 1840 |
| AD-35609.4 | GGGctttctttGtGtAttttt | 1803 | AAAtAcAcAAAGAAAGCCCtt | 1841 |
| AD-35581.4 | GtGtAtttGccAtAAAtAAtt | 1804 | TtATTtATGGcAAAtAcACtt | 1842 |
| AD-39845.1 | ccAtAAAtAAtActAAAtctt | 1805 | GATTtAGtATtATTtATGGtt | 1843 |
| AD-39858.1 | tActAAAtcAtttGAAGAttt | 1806 | ATCTTcAAATGATTtAGtAtt | 1844 |
| AD-39870.1 | ctAAAtcAtttGAAGAtAtuu | 1807 | AtATCTTcAAATGATTtAGuu | 1845 |
| AD-35576.4 | GAAGAtAttcAccAttAtAtt | 1808 | tAtAATGGTGAAtATCTTCtt | 1846 |
| AD-35588.4 | AGAtAttcAccAttAtAGAtt | 1809 | TCtAtAATGGTGAAtATCTtt | 1847 |
| AD-35600.1 | AtAttcAccAttAtAGAGAtt | 1810 | TCTCtAtAATGGTGAAtATtt | 1848 |
| AD-35606.1 | tAttcAccAttAtAGAGAAtt | 1811 | TTCTCtAtAATGGTGAAtAtt | 1849 |
| AD-38151.1 | cAttAtAGAGAAcAAAttAtt | 1812 | tAATTTGTTCTCtAtAATGtt | 1850 |
| AD-38163.1 | ttAtAGAGAAcAAAttAAAtt | 1813 | TTtAATTTGTTCTCtAtAAtt | 1851 |
| AD-39999.1 | GAGttAAGGActctGAAGAtt | 1814 | TCTTcAGAGTCCTtAACTCtt | 1852 |
| AD-38159.1 or hs_KRAS 528_A22S26 | ctAtGGtcctAGtAGGAAAuu | 1815 | TTTCCtACtAGGACcAtAGuu | 1853 |
| AD-38130.1 or hs_KRAS | tGGtcctAGtAGGAAAtAAuu | 1816 | TtATTTCCtACtAGGACcAuu | 1854 |
| 531_A22S26 | | | | |
| AD-38136.1 | GGtcctAGtAGGAAAtAAAtt | 1817 | TTtATTTCCtACtAGGACCtt | 1855 |

(continued)

| Duplex | Sense Sequence | Seq SEQ ID NO: | Anti sense Sequence | Seq SEQ ID NO: |
|---|---|---|---|---|
| AD-40036.1 | GtAGGAAAtAAAtGtGAtttt | 1818 | AATcAcATTtATTTCCtACtt | 1856 |
| AD-40008.1 | AtGtGAtttGccttctAGAtt | 1819 | TCtAGAAGGcAAATcAcATtt | 1857 |
| AD-40021.1 | ccttctAGAAcAGtAGAcAtt | 1820 | TGTCtACTGTTCtAGAAGGtt | 1858 |
| AD-40077.1 | AGGctcAGGActtAGcAAGtt | 1821 | CTTGCtAAGTCCTGAGCCTtt | 1859 |
| AD-40072.1 | GGActtAGcAAGAAGttAttt | 1822 | AtAACTTCTTGCtAAGTCCtt | 1860 |
| AD-40061.1 | AGcAAGAAGttAtGGAAtttt | 1823 | AATTCcAtAACTTCTTGCTtt | 1861 |
| AD-40068.1 | ttAtGGAAttccttttAttt | 1824 | AAtAAAAGGAATTCcAtAAtt | 1862 |
| AD-38131.1 | GGAAttccttttAttGAAAtt | 1825 | TTTcAAtAAAAGGAATTCCtt | 1863 |
| AD-38167.1 | ccttttAttGAAAcAtcAGtt | 1826 | CTGATGTTTcAAtAAAAGGtt | 1864 |
| hs_KRAS 1273_A22S26 | cctGAtGAAtGtAAAGttAuu | 1827 | tAACTTtAcATTcATcAGGuu | 1865 |
| hs_KRAS 2892_A37S26 | GGAGAAtttAAtAAAGAtAuu | 1828 | TATCTTtATtAAATTCTCCuu | 1866 |
| hs_KRAS 4731_A22S26 | GAAtAGtcAtAActAGAttuu | 1829 | AATCtAGTtATGACtATTCuu | 1867 |

Modifications of the sequences of RNAi agents are easily conceived by one of skill in the art. Examples and non-limiting modifications of these sequences are conceived and are also listed in Table 3. Note for example, that many sequences end with a terminal dTdT. This is a variant of the TT terminal overhang known to protect siRNAs from nuclease degradation (e.g., in blood serum or intestinal fluid). The dTdT or TT terminal overhangs are optional; they can be replaced by various types of 3' end caps, provided that the 3' end caps are capable of both mediating nuclease resistance and allowing siRNA activity.

Some modifications are placed at sites predicted to be sensitive to endonucleases. Some modifications are designed to eliminate an immune response to the siRNA while preserving activity. In general, the sense strand is heavily modified, and the antisense strand lightly modified. Some modifications serve more than one purpose.

The sequences in Table s 1 to 3 are represented by these abbreviations:

**TABLE 1A. ABBREVIATIONS**

| Abbreviation | Nucleotide(s) |
|---|---|
| A | adenosine-5'-phosphate |
| C | cytidine-5'-phosphate |
| G | guanosine-5'-phosphate |
| dT | 2'-deoxy-thymidine-5'-phosphate |
| U | uridine-5'-phosphate |
| c | 2'-O-methylcytidine-5'-phosphate |
| u | 2'-O-methyluridine-5'-phosphate |
| sdT | 2'-deoxy Thymidine 5'-phosphorothioate |

**siRNA sequence selection**

Sense and antisense human SCNNB1-derived siRNA oligos (RNAi agents to KRAS) are synthesized, as described herein. The sense and their respective antisense oligos are annealed into duplexes.

**EXAMPLE 2**

Overlapping sets of KRAS RNAi Agents

The present disclosure also relates to groups of RNAi agents to KRAS with overlapping sequences. Thus, the present disclosure encompasses groups of RNAi agents wherein each RNAi agent in the group overlaps with each other RNAi agent in the same group by at least 5, 6, 7, 8, 9, 10, 11, 12, 13, 14, 15, 16, 17, 18, 19 or more nucleotides. Particularly, in one embodiment, the overlap is at least 12 nt.

Some of the RNAi agents listed herein overlap each other in sequence. Table 4 presents a compilation of some of these groups of overlapping RNAi agents, wherein each member of a group overlaps with each other member of the same group by at least 12 nt. A 12-nt portion of the overlap of the sense and anti-sense strand are presented.

Thus, for example, as shown in Table 4, the sequences of duplexes AD-39695.1, AD-39689.1,AD-39707.1, AD-39683.1, and AD-39701.1 all overlap, wherein the overlap in sense strand comprises the sequence CUGAAUAUAAAC (SEQ ID NO: 1874) and the overlap in the anti-sense strand comprises the sequence GUUUAUAUUCAG (SEQ ID NO: 1875).

However, in the present disclosure encompasses any set or subset or group or subgroup of KRAS RNAi agents which share the common technical feature of a sequence overlap (e.g., by at least 12 nt). Thus the present disclosure also encompasses the example groups of AD-39695.1, AD-39689.1, AD-39707.1, AD-39683.1, and AD-39701.1, but also these subgroups: AD-39695.1 and AD-39689.1; AD-39695.1 and AD-39707.1; AD-39695.1, AD-39689.1, and AD-39701.1; AD-39689.1, AD-39707.1, and AD-39701.1; AD-39707.1 and AD-39683.1; AD-39707.1, AD-39683.1, and AD-39701.1; etc, and any other group of overlapping KRAS RNAi agents.

**Table 4. Overlapping sets of KRAS RNAi agents**

| Sense overlap | SEQ ID NO: | Anti-sense overlap | SEQ ID NO: | Overlapping RNAi agents to KRAS |
|---|---|---|---|---|
| UGACUGAAUAUA | **1868** | UAUAUUCAGUCA | **1869** | AD-39689.1,AD-39683.1 |
| GACUGAAUAUAA | **1870** | UUAUAUUCAGUC | **1871** | AD-39695.1,AD-39689.1,AD-39683.1 |
| ACUGAAUAUAAA | **1872** | UUUAUAUUCAGU | **1873** | AD-39695.1,AD-39689.1,AD-39683.1,AD-39701.1 |
| CUGAAUAUAAAC | **1874** | GUUUAUAUUCAG | **1875** | AD-39695.1,AD-39689.1,AD-39707.1,AD-39683.1,AD-39701.1 |
| UGAAUAUAAACU | **1876** | AGUUUAUAUUCA | **1877** | AD-39695.1,AD-39689.1,AD-39713.1,AD-39707.1,AD-39683.1,AD-39701.1 |
| GAAUAUAAACUU | **1878** | AAGUUUAUAUUC | **1879** | AD-39695.1,AD-39689.1,AD-39713.1,AD-39707.1,AD-39683.1,AD-39701.1,AD-39719.1 |
| AAUAUAAACUUG | **1880** | CAAGUUUAUAUU | **1881** | AD-39695.1,AD-39689.1,AD-39713.1,AD-39707.1,AD-39683.1,AD-39701.1,AD-39725.1 , AD-39719.1 |
| AUAUAAACUUGU | **1882** | ACAAGUUUAUAU | **1883** | AD-39695.1,AD-39689.1,AD-39713.1,AD-39707.1,AD-39701.1,AD-39725.1 ,AD-39719.1 , AD-39684.1 |
| UAUAAACUUGUG | **1884** | CACAAGUUUAUA | **1885** | AD-39690.1,AD-39695.1,AD-39713.1,AD-39707.1,AD-39701.1,AD-39725.1,AD-39719.1,AD-39684.1 |
| AUAAACUUGUGG | **1886** | CCACAAGUUUAU | **1887** | AD-39690.1,AD-39713.1,AD-39707.1,AD-39701.1,AD-39725.1,AD-39719.1,AD-39684.1,AD-39696.1 |
| UAAACUUGUGGU | **1888** | ACCACAAGUUUA | **1889** | AD-39690.1,AD-39713.1,AD-39707.1,AD-39725.1,AD-39702.1,AD-39719.1,AD-39684.1,AD-39696.1 |

(continued)

| Sense overlap | SEQ ID NO: | Anti-sense overlap | SEQ ID NO: | Overlapping RNAi agents to KRAS |
|---|---|---|---|---|
| AAACUUGUGGUA | **1890** | UACCACAAGUUU | **1891** | AD-39690.1,AD-39713.1,AD-39725.1 ,AD-39702.1,AD-39719.1,AD-39684.1,AD-39696.1,AD-39708.1 |
| AACUUGUGGUAG | **1892** | CUACCACAAGUU | **1893** | AD-39690.1,AD-39725.1,AD-39702.1,AD-39719.1,AD-39684.1,AD-39714.1,AD-39696.1,AD-39708.1 |
| ACUUGUGGUAGU | **1894** | ACUACCACAAGU | **1895** | AD-39690.1,AD-39725.1,AD-39702.1,AD-39684.1,AD-39714.1,AD-39696.1,AD-39708.1,AD-39720.1 |
| CUUGUGGUAGUU | **1896** | AACUACCACAAG | **1897** | AD-39690.1,AD-39702.1,AD-39684.1,AD-39714.1,AD-39696.1,AD-39708.1,AD-39720.1,AD-39726.1 |
| UUGUGGUAGUUG | **1898** | CAACUACCACAA | **1899** | AD-39690.1,AD-39702.1,AD-39714.1,AD-39696.1,AD-39708.1,AD-39720.1,AD-39726.1,AD-39685.1 |
| UGUGGUAGUUGG | **1900** | CCAACUACCACA | **1901** | AD-39691.1,AD-39702.1,AD-39714.1,AD-39696.1,AD-39708.1,AD-39720.1,AD-39726.1,AD-39685.1 |
| GUGGUAGUUGGA | **1902** | UCCAACUACCAC | **1903** | AD-39691.1,AD-39697.1,AD-39702.1,AD-39714.1,AD-39708.1,AD-39720.1,AD-39726.1,AD-39685.1 |
| UGGUAGUUGGAG | **1904** | CUCCAACUACCA | **1905** | AD-39703.1,AD-39691.1,AD-39697.1,AD-39714.1,AD-39708.1,AD-39720.1,AD-39726.1,AD-39685.1 |
| GGUAGUUGGAGC | **1906** | GCUCCAACUACC | **1907** | AD-39703.1,AD-39691.1,AD-39697.1,AD-39714.1,AD-39709.1,AD-39720.1,AD-39726.1,AD-39685.1 |
| GUAGUUGGAGCU | **1908** | AGCUCCAACUAC | **1909** | AD-39703.1,AD-39691.1,AD-39697.1,AD-39715.1,AD-39709.1,AD-39720.1,AD-39726.1,AD-39685.1 |
| UAGUUGGAGCUG | **1910** | CAGCUCCAACUA | **1911** | AD-39703.1,AD-39691.1,AD-39697.1,AD-39715.1,AD-39709.1,AD-39721.1,AD-39726.1,AD-39685.1 |
| AGUUGGAGCUGG | **1912** | CCAGCUCCAACU | **1913** | AD-39703.1,AD-39691.1,AD-39697.1,AD-39715.1,AD-39709.1,AD-39721.1,AD-39727.1,AD-39685.1 |
| GUUGGAGCUGGU | **1914** | ACCAGCUCCAAC | **1915** | AD-39703.1,AD-39691.1,AD-39686.1,AD-39697.1,AD-39715.1,AD-39709.1,AD-39721.1,AD-39727.1 |
| UUGGAGCUGGUG | **1916** | CACCAGCUCCAA | **1917** | AD-39703.1,AD-39686.1,AD-39697.1,AD-39692.1,AD-39715.1,AD-39709.1,AD-39721.1,AD-39727.1 |
| UGGAGCUGGUGG | **1918** | CCACCAGCUCCA | **1919** | AD-39703.1,AD-39686.1,AD-39692.1,AD-39715.1,AD-39709.1,AD-39698.1,AD-39721.1,AD-39727.1 |

(continued)

| Sense overlap | SEQ ID NO: | Anti-sense overlap | SEQ ID NO: | Overlapping RNAi agents to KRAS |
|---|---|---|---|---|
| GGAGCUGGUGGC | **1920** | GCCACCAGCUCC | **1921** | AD-39686.1,AD-39692.1,AD-39715.1,AD-39709.1,AD-39698.1,AD-39721.1,AD-39704.1,AD-39727.1 |
| GAGCUGGUGGCG | **1922** | CGCCACCAGCUC | **1923** | AD-39710.1,AD-39686.1,AD-39692.1,AD-39715.1,AD-39698.1,AD-39721.1,AD-39704.1,AD-39727.1 |
| AGCUGGUGGCGU | **1924** | ACGCCACCAGCU | **1925** | AD-39710.1,AD-39686.1,AD-39692.1,AD-39716.1,AD-39698.1,AD-39721.1,AD-39704.1,AD-39727.1 |
| GCUGGUGGCGUA | **1926** | UACGCCACCAGC | **1927** | AD-39710.1,AD-39686.1,AD-39692.1,AD-39722.1,AD-39716.1,AD-39698.1,AD-39704.1,AD-39727.1 |
| CUGGUGGCGUAG | **1928** | CUACGCCACCAG | **1929** | AD-39710.1,AD-39686.1,AD-39692.1,AD-39722.1,AD-39716.1,AD-39728.1,AD-39698.1,AD-39704.1 |
| UGGUGGCGUAGG | **1930** | CCUACGCCACCA | **1931** | AD-39710.1,AD-39687.1,AD-39692.1,AD-39722.1,AD-39716.1,AD-39728.1,AD-39698.1,AD-39704.1 |
| GGUGGCGUAGGC | **1932** | GCCUACGCCACC | **1933** | AD-39693.1,AD-39710.1,AD-39687.1,AD-39722.1,AD-39716.1,AD-39728.1,AD-39698.1,AD-39704.1 |
| GUGGCGUAGGCA | **1934** | UGCCUACGCCAC | **1935** | AD-39693.1,AD-39710.1,AD-39699.1,AD-39687.1,AD-39722.1,AD-39716.1,AD-39728.1,AD-39704.1 |
| UGGCGUAGGCAA | **1936** | UUGCCUACGCCA | **1937** | AD-39693.1,AD-39710.1,AD-39699.1,AD-39687.1,AD-39722.1,AD-39716.1,AD-39705.1,AD-39728.1 |
| GGCGUAGGCAAG | **1938** | CUUGCCUACGCC | **1939** | AD-39693.1,AD-39699.1,AD-39687.1,AD-39711.1,AD-39722.1,AD-39716.1,AD-39705.1,AD-39728.1 |
| GCGUAGGCAAGA | **1940** | UCUUGCCUACGC | **1941** | AD-39693.1,AD-39699.1,AD-39687.1,AD-39717.1,AD-39711.1,AD-39722.1,AD-39705.1,AD-39728.1 |
| CGUAGGCAAGAG | **1942** | CUCUUGCCUACG | **1943** | AD-39693.1,AD-39699.1,AD-39687.1,AD-39717.1,AD-39711.1,AD-39705.1,AD-39728.1,AD-39723.1 |
| GUAGGCAAGAGU | **1944** | ACUCUUGCCUAC | **1945** | AD-39693.1,AD-39699.1,AD-39687.1,AD-39717.1,AD-39711.1,AD-39729.1,AD-39705.1,AD-39723.1 |
| UAGGCAAGAGUG | **1946** | CACUCUUGCCUA | **1947** | AD-39693.1,AD-39699.1,AD-39717.1,AD-39711.1,AD-39688.1,AD-39729.1,AD-39705.1,AD-39723.1 |
| AGGCAAGAGUGC | **1948** | GCACUCUUGCCU | **1949** | AD-39699.1,AD-39717.1,AD-39711.1,AD-39688.1,AD-39729.1,AD-39705.1,AD-39694.1,AD-39723.1 |

(continued)

| Sense overlap | SEQ ID NO: | Anti-sense overlap | SEQ ID NO: | Overlapping RNAi agents to KRAS |
|---|---|---|---|---|
| GGCAAGAGUGCC | **1950** | GGCACUCUUGCC | **1951** | AD-39717.1,AD-39711.1,AD-39688.1,AD-39729.1,AD-39705.1,AD-39694.1,AD-39700.1,AD-39723.1 |
| GCAAGAGUGCCU | **1952** | AGGCACUCUUGC | **1953** | AD-39717.1,AD-39711.1,AD-39688.1,AD-39729.1,AD-39706.1,AD-39694.1,AD-39700.1,AD-39723.1 |
| CAAGAGUGCCUU | **1954** | AAGGCACUCUUG | **1955** | AD-39712.1,AD-39717.1,AD-39688.1,AD-39729.1,AD-39706.1,AD-39694.1,AD-39700.1,AD-39723.1 |
| AAGAGUGCCUUG | **1956** | CAAGGCACUCUU | **1957** | AD-39718.1,AD-39712.1,AD-39688.1,AD-39729.1,AD-39706.1,AD-39694.1,AD-39700.1,AD-39723.1 |
| AGAGUGCCUUGA | **1958** | UCAAGGCACUCU | **1959** | AD-39718.1,AD-39712.1,AD-39724.1,AD-39688.1,AD-39729.1,AD-39706.1,AD-39694.1,AD-39700.1 |
| GAGUGCCUUGAC | **1960** | GUCAAGGCACUC | **1961** | AD-39718.1 ,AD-39712.1 ,AD-39724.1,AD-39688.1,AD-39706.1,AD-39694.1,AD-39700.1,AD-39730.1 |
| AGUGCCUUGACG | **1962** | CGUCAAGGCACU | **1963** | AD-39718.1,AD-39712.1,AD-39724.1,AD-39706.1,AD-39694.1,AD-39736.1,AD-39700.1,AD-39730.1 |
| GUGCCUUGACGA | **1964** | UCGUCAAGGCAC | **1965** | AD-39718.1,AD-39712.1,AD-39724.1,AD-39742.1,AD-39706.1,AD-39736.1,AD-39700.1,AD-39730.1 |
| UGCCUUGACGAU | **1966** | AUCGUCAAGGCA | **1967** | AD-39718.1,AD-39712.1,AD-39724.1,AD-39742.1,AD-39706.1,AD-39736.1.AD-39748.1,AD-39730.1 |
| GCCUUGACGAUA | **1968** | UAUCGUCAAGGC | **1969** | AD-39718.1,AD-39712.1,AD-39724.1,AD-39742.1,AD-39754.1,AD-39736.1.AD-39748.1,AD-39730.1 |
| CCUUGACGAUAC | **1970** | GUAUCGUCAAGG | **1971** | AD-39718.1,AD-39724.1,AD-39742.1,AD-39754.1,AD-39736.1,AD-39748.1,AD-39760.1,AD-39730.1 |
| CUUGACGAUACA | **1972** | UGUAUCGUCAAG | **1973** | AD-39724.1,AD-39742.1,AD-39754.1,AD-39736.1,AD-39748.1,AD-39760.1,AD-39730.1,AD-39766.1 |
| UUGACGAUACAG | **1974** | CUGUAUCGUCAA | **1975** | AD-39742.1,AD-39772.1,AD-39754.1,AD-39736.1,AD-39748.1,AD-39760.1,AD-39730.1,AD-39766.1 |
| UGACGAUACAGC | **1976** | GCUGUAUCGUCA | **1977** | AD-39742.1,AD-39772.1,AD-39754.1,AD-39736.1,AD-39731.1,AD-39748.1,AD-39760.1,AD-39766.1 |
| GACGAUACAGCU | **1978** | AGCUGUAUCGUC | **1979** | AD-39737.1,AD-39742.1,AD-39772.1,AD-39754.1,AD-39731.1,AD-39748.1,AD-39760.1,AD-39766.1 |

(continued)

| Sense overlap | SEQ ID NO: | Anti-sense overlap | SEQ ID NO: | Overlapping RNAi agents to KRAS |
|---|---|---|---|---|
| ACGAUACAGCUA | **1980** | UAGCUGUAUCGU | **1981** | AD-39737.1,AD-39772.1,AD-39754.1,AD-39743.1,AD-39731.1,AD-39748.1,AD-39760.1,AD-39766.1 |
| CGAUACAGCUAA | **1982** | UUAGCUGUAUCG | **1983** | AD-39737.1,AD-39772.1,AD-39754.1,AD-39743.1,AD-39731.1,AD-39760.1.AD-39749.1,AD-39766.1 |
| GAUACAGCUAAU | **1984** | AUUAGCUGUAUC | **1985** | AD-39737.1,AD-39772.1,AD-39743.1,AD-39731.1,AD-39760.1,AD-39749.1,AD-39755.1,AD-39766.1 |
| AUACAGCUAAUU | **1986** | AAUUAGCUGUAU | **1987** | AD-39761.1,AD-39737.1,AD-39772.1,AD-39743.1,AD-39731.1,AD-39749.1,AD-39755.1,AD-39766.1 |
| UACAGCUAAUUC | **1988** | GAAUUAGCUGUA | **1989** | AD-39767.1,AD-39761.1,AD-39737.1,AD-39772.1,AD-39743.1,AD-39731.1.AD-39749.1,AD-39755.1 |
| ACAGCUAAUUCA | **1990** | UGAAUUAGCUGU | **1991** | AD-39767.1,AD-39761.1,AD-39737.1,AD-39743.1,AD-39731.1,AD-39773.1,AD-39749.1,AD-39755.1 |
| CAGCUAAUUCAG | **1992** | CUGAAUUAGCUG | **1993** | AD-39767.1,AD-39761.1,AD-39737.1,AD-39732.1,AD-39743.1,AD-39773.1.AD-39749.1,AD-39755.1 |
| AGCUAAUUCAGA | **1994** | UCUGAAUUAGCU | **1995** | AD-39767.1,AD-39761.1,AD-39732.1,AD-39743.1,AD-39738.1,AD-39773.1,AD-39749.1,AD-39755.1 |
| GCUAAUUCAGAA | **1996** | UUCUGAAUUAGC | **1997** | AD-39767.1,AD-39761.1,AD-39744.1,AD-39732.1,AD-39738.1,AD-39773.1.AD-39749.1,AD-39755.1 |
| CUAAUUCAGAAU | **1998** | AUUCUGAAUUAG | **1999** | AD-39767.1,AD-39761.1,AD-39744.1,AD-39732.1,AD-39750.1,AD-39738.1,AD-39773.1,AD-39755.1 |
| UAAUUCAGAAUC | **2000** | GAUUCUGAAUUA | **2001** | AD-39756.1,AD-39767.1,AD-39761.1,AD-39744.1,AD-39732.1,AD-39750.1,AD-39738.1,AD-39773.1 |
| AAUUCAGAAUCA | **2002** | UGAUUCUGAAUU | **2003** | AD-39756.1,AD-39767.1,AD-39744.1,AD-39762.1,AD-39732.1,AD-39750.1,AD-39738.1,AD-39773.1 |
| AUUCAGAAUCAU | **2004** | AUGAUUCUGAAU | **2005** | AD-39756.1,AD-39744.1,AD-39762.1,AD-39732.1,AD-39768.1,AD-39750.1,AD-39738.1,AD-39773.1 |
| UUCAGAAUCAUU | **2006** | AAUGAUUCUGAA | **2007** | AD-39756.1,AD-39774.1,AD-39744.1,AD-39762.1,AD-39732.1,AD-39768.1,AD-39750.1,AD-39738.1 |
| UCAGAAUCAUUU | **2008** | AAAUGAUUCUGA | **2009** | AD-39756.1,AD-39774.1,AD-39744.1,AD-39762.1,AD-39733.1,AD-39768.1,AD-39750.1,AD-39738.1 |

(continued)

| Sense overlap | SEQ ID NO: | Anti-sense overlap | SEQ ID NO: | Overlapping RNAi agents to KRAS |
|---|---|---|---|---|
| CAGAAUCAUUUU | **2010** | AAAAUGAUUCUG | **2011** | AD-39756.1,AD-39739.1,AD-39774.1,AD-39744.1,AD-39762.1,AD-39733.1,AD-39768.1,AD-39750.1 |
| AGAAUCAUUUUG | **2012** | CAAAAUGAUUCU | **2013** | AD-39745.1,AD-39756.1,AD-39739.1,AD-39774.1,AD-39762.1,AD-39733.1,AD-39768.1,AD-39750.1 |
| GAAUCAUUUUGU | **2014** | ACAAAAUGAUUC | **2015** | AD-39745.1,AD-39756.1,AD-39739.1,AD-39774.1,AD-39762.1,AD-39733.1,AD-39768.1,AD-39751.1 |
| AAUCAUUUUGUG | **2016** | CACAAAAUGAUU | **2017** | AD-39745.1,AD-39739.1,AD-39774.1,AD-39757.1,AD-39762.1,AD-39733.1,AD-39768.1,AD-39751.1 |
| AUCAUUUUGUGG | **2018** | CCACAAAAUGAU | **2019** | AD-39745.1,AD-39739.1,AD-39774.1,AD-39757.1,AD-39733.1,AD-39768.1,AD-39751.1,AD-39763.1 |
| UCAUUUUGUGGA | **2020** | UCCACAAAAUGA | **2021** | AD-39745.1,AD-39739.1,AD-39774.1,AD-39757.1,AD-39733.1,AD-39751.1,AD-39769.1,AD-39763.1 |
| CAUUUUGUGGAC | **2022** | GUCCACAAAAUG | **2023** | AD-39745.1,AD-39739.1,AD-39775.1,AD-39757.1,AD-39733.1,AD-39751.1,AD-39769.1,AD-39763.1 |
| AUUUUGUGGACG | **2024** | CGUCCACAAAAU | **2025** | AD-39745.1,AD-39739.1,AD-39775.1,AD-39757.1,AD-39751.1,AD-39769.1,AD-39763.1 , AD-39734.1 |
| UUUUGUGGACGA | **2026** | UCGUCCACAAAA | **2027** | AD-39745.1,AD-39775.1,AD-39757.1,AD-39751.1,AD-39769.1,AD-39740.1,AD-39763.1,AD-39734.1 |
| UUUGUGGACGAA | **2028** | UUCGUCCACAAA | **2029** | AD-39775.1,AD-39746.1,AD-39757.1,AD-39751.1,AD-39769.1,AD-39740.1,AD-39763.1 , AD-39734.1 |
| UUGUGGACGAAU | **2030** | AUUCGUCCACAA | **2031** | AD-39752.1,AD-39775.1,AD-39746.1,AD-39757.1,AD-39769.1,AD-39740.1,AD-39763.1 , AD-39734.1 |
| UGUGGACGAAUA | **2032** | UAUUCGUCCACA | **2033** | AD-39752.1,AD-39775.1,AD-39746.1,AD-39758.1,AD-39769.1,AD-39740.1,AD-39763.1 , AD-39734.1 |
| GUGGACGAAUAU | **2034** | AUAUUCGUCCAC | **2035** | AD-39752.1,AD-39775.1,AD-39764.1,AD-39746.1,AD-39758.1,AD-39769.1,AD-39740.1,AD-39734.1 |
| UGGACGAAUAUG | **2036** | CAUAUUCGUCCA | **2037** | AD-39752.1,AD-39775.1,AD-39764.1,AD-39746.1,AD-39770.1,AD-39758.1,AD-39740.1,AD-39734.1 |
| GGACGAAUAUGA | **2038** | UCAUAUUCGUCC | **2039** | AD-39752.1,AD-35523.3,AD-39764.1,AD-39746.1,AD-39770.1,AD-39758.1,AD-39740.1,AD-39734.1 |

(continued)

| Sense overlap | SEQ ID NO: | Anti-sense overlap | SEQ ID NO: | Overlapping RNAi agents to KRAS |
|---|---|---|---|---|
| GACGAAUAUGAU | **2040** | AUCAUAUUCGUC | **2041** | AD-39752.1,AD-35529.1,AD-35523.3,AD-39764.1,AD-39746.1,AD-39770.1,AD-39758.1,AD-39740.1 |
| ACGAAUAUGAUC | **2042** | GAUCAUAUUCGU | **2043** | AD-39752.1,AD-35529.1,AD-35523.3,AD-39764.1,AD-39746.1,AD-35535.4,AD-39770.1,AD-39758.1 |
| CGAAUAUGAUCC | **2044** | GGAUCAUAUUCG | **2045** | AD-39752.1,AD-35529.1,AD-35523.3,AD-35541.4,AD-39764.1,AD-35535.4,AD-39770.1,AD-39758.1 |
| GAAUAUGAUCCA | **2046** | UGGAUCAUAUUC | **2047** | AD-35529.1,AD-35523.3,AD-35541.4,AD-39764.1,AD-35535.4,AD-39770.1,AD-39758.1,AD-35547.4 |
| AAUAUGAUCCAA | **2048** | UUGGAUCAUAUU | **2049** | AD-35529.1,AD-35523.3,AD-35541.4,AD-39764.1,AD-35535.4,AD-39770.1,AD-35553.4,AD-35547.4 |
| AUAUGAUCCAAC | **2050** | GUUGGAUCAUAU | **2051** | AD-35529.1,AD-35523.3,AD-35541.4,AD-35559.4,AD-35535.4,AD-39770.1,AD-35553.4,AD-35547.4 |
| UAUGAUCCAACA | **2052** | UGUUGGAUCAUA | **2053** | AD-35529.1,AD-35523.3,AD-35541.4,AD-35565.4,AD-35559.4,AD-35535.4,AD-35553.4,AD-35547.4 |
| AUGAUCCAACAA | **2054** | UUGUUGGAUCAU | **2055** | AD-35529.1,AD-35541.4,AD-35565.4,AD-35559.4,AD-35535.4,AD-35553.4,AD-35547.4,AD-35524.4 |
| UGAUCCAACAAU | **2056** | AUUGUUGGAUCA | **2057** | AD-35530.4,AD-35541.4,AD-35565.4,AD-35559.4,AD-35535.4,AD-35553.4,AD-35547.4,AD-35524.4 |
| GAUCCAACAAUA | **2058** | UAUUGUUGGAUC | **2059** | AD-35530.4,AD-35541.4,AD-35565.4,AD-35559.4,AD-35553.4,AD-35536.4,AD-35547.4,AD-35524.4 |
| AUCCAACAAUAG | **2060** | CUAUUGUUGGAU | **2061** | AD-35530.4,AD-35565.4,AD-35559.4,AD-35553.4,AD-35542.4,AD-35536.4,AD-35547.4,AD-35524.4 |
| UCCAACAAUAGA | **2062** | UCUAUUGUUGGA | **2063** | AD-35530.4,AD-35565.4,AD-35559.4,AD-35553.4,AD-35548.4,AD-35542.4,AD-35536.4,AD-35524.4 |
| CCAACAAUAGAG | **2064** | CUCUAUUGUUGG | **2065** | AD-35554.4,AD-35530.4,AD-35565.4,AD-35559.4,AD-35548.4,AD-35542.4,AD-35536.4,AD-35524.4 |
| CAACAAUAGAGG | **2066** | CCUCUAUUGUUG | **2067** | AD-35554.4,AD-35530.4,AD-35565.4,AD-35548.4,AD-35542.4,AD-35536.4,AD-35560.2,AD-35524.4 |
| AACAAUAGAGGA | **2068** | UCCUCUAUUGUU | **2069** | AD-35554.4,AD-35530.4,AD-35566.4,AD-35548.4,AD-35542.4,AD-35536.4,AD-35560.2,AD-35524.4 |

(continued)

| Sense overlap | SEQ ID NO: | Anti-sense overlap | SEQ ID NO: | Overlapping RNAi agents to KRAS |
|---|---|---|---|---|
| ACAAUAGAGGAU | **2070** | AUCCUCUAUUGU | **2071** | AD-35554.4,AD-35530.4,AD-35525.2,AD-35566.4,AD-35548.4,AD-35542.4,AD-35536.4,AD-35560.2 |
| CAAUAGAGGAUU | **2072** | AAUCCUCUAUUG | **2073** | AD-35554.4,AD-35525.2,AD-35566.4,AD-35548.4,AD-35542.4,AD-35536.4,AD-35531.4,AD-35560.2 |
| AAUAGAGGAUUC | **2074** | GAAUCCUCUAUU | **2075** | AD-35554.4,AD-35525.2,AD-35537.4,AD-35566.4,AD-35548.4,AD-35542.4,AD-35531.4,AD-35560.2 |
| AUAGAGGAUUCC | **2076** | GGAAUCCUCUAU | **2077** | AD-35554.4,AD-35525.2,AD-35537.4,AD-35566.4,AD-35548.4,AD-35531.4,AD-35560.2,AD-35543.2 |
| UAGAGGAUUCCU | **2078** | AGGAAUCCUCUA | **2079** | AD-35549.4,AD-35554.4,AD-35525.2,AD-35537.4,AD-35566.4,AD-35531.4,AD-35560.2,AD-35543.2 |
| AGAGGAUUCCUA | **2080** | UAGGAAUCCUCU | **2081** | AD-35549.4,AD-35555.4,AD-35525.2,AD-35537.4,AD-35566.4,AD-35531.4,AD-35560.2,AD-35543.2 |
| GAGGAUUCCUAC | **2082** | GUAGGAAUCCUC | **2083** | AD-35549.4,AD-35555.4,AD-35525.2,AD-35537.4,AD-35566.4,AD-35531.4,AD-35561.4,AD-35543.2 |
| AGGAUUCCUACA | **2084** | UGUAGGAAUCCU | **2085** | AD-35549.4,AD-35555.4,AD-35525.2,AD-35537.4,AD-35531.4,AD-35561.4,AD-35543.2,AD-35567.4 |
| GGAUUCCUACAG | **2086** | CUGUAGGAAUCC | **2087** | AD-35549.4,AD-35555.4,AD-35526.4,AD-35537.4,AD-35531.4,AD-35561.4,AD-35543.2,AD-35567.4 |
| GAUUCCUACAGG | **2088** | CCUGUAGGAAUC | **2089** | AD-35549.4,AD-35555.4,AD-35532.4,AD-35526.4,AD-35537.4,AD-35561.4,AD-35543.2,AD-35567.4 |
| AUUCCUACAGGA | **2090** | UCCUGUAGGAAU | **2091** | AD-35549.4,AD-35555.4,AD-35532.4,AD-35526.4,AD-35538.4,AD-35561.4,AD-35543.2,AD-35567.4 |
| UUCCUACAGGAA | **2092** | UUCCUGUAGGAA | **2093** | AD-35549.4,AD-35555.4,AD-35532.4,AD-35526.4,AD-35538.4,AD-35544.4,AD-35561.4,AD-35567.4 |
| UCCUACAGGAAG | **2094** | CUUCCUGUAGGA | **2095** | AD-35555.4,AD-35532.4,AD-35526.4,AD-35550.4,AD-35538.4,AD-35544.4,AD-35561.4,AD-35567.4 |
| CCUACAGGAAGC | **2096** | GCUUCCUGUAGG | **2097** | AD-35556.4,AD-35532.4,AD-35526.4,AD-35550.4,AD-35538.4,AD-35544.4,AD-35561.4,AD-35567.4 |
| CUACAGGAAGCA | **2098** | UGCUUCCUGUAG | **2099** | AD-35562.4,AD-35556.4,AD-35532.4,AD-35526.4,AD-35550.4,AD-35538.4,AD-35544.4,AD-35567.4 |

(continued)

| Sense overlap | SEQ ID NO: | Anti-sense overlap | SEQ ID NO: | Overlapping RNAi agents to KRAS |
|---|---|---|---|---|
| UACAGGAAGCAA | **2100** | UUGCUUCCUGUA | **2101** | AD-35562.4,AD-35556.4,AD-35532.4,AD-35526.4,AD-35550.4,AD-35568.4,AD-35538.4,AD-35544.4 |
| ACAGGAAGCAAG | **2102** | CUUGCUUCCUGU | **2103** | AD-35562.4,AD-35556.4,AD-35532.4,AD-35550.4,AD-35568.4,AD-35538.4,AD-35544.4,AD-35527.4 |
| CAGGAAGCAAGU | **2104** | ACUUGCUUCCUG | **2105** | AD-35562.4,AD-35556.4,AD-35550.4,AD-35568.4,AD-35538.4,AD-35544.4,AD-35533.4,AD-35527.4 |
| AGGAAGCAAGUA | **2106** | UACUUGCUUCCU | **2107** | AD-35562.4,AD-35556.4,AD-35550.4,AD-39776.1,AD-35568.4,AD-35544.4,AD-35533.4,AD-35527.4 |
| GGAAGCAAGUAG | **2108** | CUACUUGCUUCC | **2109** | AD-35562.4,AD-39735.1,AD-35556.4,AD-35550.4,AD-39776.1,AD-35568.4,AD-35533.4,AD-35527.4 |
| GAAGCAAGUAGU | **2110** | ACUACUUGCUUC | **2111** | AD-35562.4,AD-39735.1,AD-35556.4,AD-39776.1,AD-35568.4,AD-35533.4,AD-35527.4,AD-39741.1 |
| AAGCAAGUAGUA | **2112** | UACUACUUGCUU | **2113** | AD-35562.4,AD-39735.1,AD-39776.1,AD-35568.4,AD-39747.1,AD-35533.4,AD-35527.4,AD-39741.1 |
| AGCAAGUAGUAA | **2114** | UUACUACUUGCU | **2115** | AD-39735.1,AD-39753.1,AD-39776.1,AD-35568.4,AD-39747.1,AD-35533.4,AD-35527.4,AD-39741.1 |
| GCAAGUAGUAAU | **2116** | AUUACUACUUGC | **2117** | AD-39735.1,AD-39759.1,AD-39753.1,AD-39776.1,AD-39747.1,AD-35533.4,AD-35527.4,AD-39741.1 |
| CAAGUAGUAAUU | **2118** | AAUUACUACUUG | **2119** | AD-39735.1,AD-39759.1,AD-39753.1,AD-39776.1,AD-39747.1,AD-35533.4,AD-39765.1,AD-39741.1 |
| AAGUAGUAAUUG | **2120** | CAAUUACUACUU | **2121** | AD-39735.1,AD-39771.1,AD-39759.1,AD-39753.1,AD-39776.1,AD-39747.1,AD-39765.1,AD-39741.1 |
| AGUAGUAAUUGA | **2122** | UCAAUUACUACU | **2123** | AD-39735.1,AD-39778.1,AD-39771.1,AD-39759.1,AD-39753.1,AD-39747.1,AD-39765.1,AD-39741.1 |
| GUAGUAAUUGAU | **2124** | AUCAAUUACUAC | **2125** | AD-39784.1,AD-39778.1,AD-39771.1,AD-39759.1,AD-39753.1,AD-39747.1,AD-39765.1,AD-39741.1 |
| UAGUAAUUGAUG | **2126** | CAUCAAUUACUA | **2127** | AD-39784.1,AD-39778.1,AD-39771.1,AD-39759.1,AD-39753.1,AD-39747.1,AD-39765.1,AD-39790.1 |
| AGUAAUUGAUGG | **2128** | CCAUCAAUUACU | **2129** | AD-39784.1,AD-39778.1,AD-39771.1,AD-39759.1,AD-39753.1,AD-39796.1,AD-39765.1,AD-39790.1 |

(continued)

| Sense overlap | SEQ ID NO: | Anti-sense overlap | SEQ ID NO: | Overlapping RNAi agents to KRAS |
|---|---|---|---|---|
| GUAAUUGAUGGA | **2130** | UCCAUCAAUUAC | **2131** | AD-39784.1,AD-39778.1,AD-39771.1,AD-39759.1,AD-39796.1,AD-39765.1,AD-39802.1,AD-39790.1 |
| UAAUUGAUGGAG | **2132** | CUCCAUCAAUUA | **2133** | AD-39784.1,AD-39778.1,AD-39808.1,AD-39771.1,AD-39796.1,AD-39765.1,AD-39802.1,AD-39790.1 |
| AAUUGAUGGAGA | **2134** | UCUCCAUCAAUU | **2135** | AD-39784.1,AD-39814.1,AD-39778.1,AD-39808.1,AD-39771.1,AD-39796.1,AD-39802.1,AD-39790.1 |
| AUUGAUGGAGAA | **2136** | UUCUCCAUCAAU | **2137** | AD-39784.1,AD-39814.1,AD-39778.1,AD-39808.1,AD-39820.1,AD-39796.1,AD-39802.1,AD-39790.1 |
| UUGAUGGAGAAA | **2138** | UUUCUCCAUCAA | **2139** | AD-39784.1,AD-39814.1,AD-39808.1,AD-39820.1,AD-39779.1,AD-39796.1,AD-39802.1,AD-39790.1 |
| UGAUGGAGAAAC | **2140** | GUUUCUCCAUCA | **2141** | AD-39814.1,AD-39808.1,AD-39785.1,AD-39820.1,AD-39779.1,AD-39796.1,AD-39802.1,AD-39790.1 |
| GAUGGAGAAACC | **2142** | GGUUUCUCCAUC | **2143** | AD-39791.1,AD-39814.1,AD-39808.1,AD-39785.1,AD-39820.1,AD-39779.1,AD-39796.1,AD-39802.1 |
| AUGGAGAAACCU | **2144** | AGGUUUCUCCAU | **2145** | AD-39791.1,AD-39814.1,AD-39808.1,AD-39785.1,AD-39820.1,AD-39779.1,AD-39797.1,AD-39802.1 |
| UGGAGAAACCUG | **2146** | CAGGUUUCUCCA | **2147** | AD-39791.1,AD-39814.1,AD-39808.1,AD-39803.1,AD-39785.1,AD-39820.1,AD-39779.1,AD-39797.1 |
| GGAGAAACCUGU | **2148** | ACAGGUUUCUCC | **2149** | AD-39791.1,AD-39814.1,AD-39803.1,AD-39785.1,AD-39820.1,AD-39809.1,AD-39779.1,AD-39797.1 |
| GAGAAACCUGUC | **2150** | GACAGGUUUCUC | **2151** | AD-39815.1,AD-39791.1,AD-39803.1,AD-39785.1,AD-39820.1,AD-39809.1,AD-39779.1,AD-39797.1 |
| AGAAACCUGUCU | **2152** | AGACAGGUUUCU | **2153** | AD-39815.1,AD-39791.1,AD-39803.1,AD-39821.1,AD-39785.1,AD-39809.1,AD-39779.1,AD-39797.1 |
| GAAACCUGUCUC | **2154** | GAGACAGGUUUC | **2155** | AD-39815.1 ,AD-39791.1,AD-39803.1,AD-39821.1,AD-39785.1,AD-39809.1 ,AD-39780.1 , AD-39797.1 |
| AAACCUGUCUCU | **2156** | AGAGACAGGUUU | **2157** | AD-39815.1,AD-39791.1,AD-39803.1,AD-39821.1,AD-39809.1,AD-39780.1,AD-39797.1,AD-39786.1 |
| AACCUGUCUCUU | **2158** | AAGAGACAGGUU | **2159** | AD-39815.1,AD-39803.1,AD-39821.1,AD-39809.1,AD-39792.1,AD-39780.1,AD-39797.1,AD-39786.1 |

(continued)

| Sense overlap | SEQ ID NO: | Anti-sense overlap | SEQ ID NO: | Overlapping RNAi agents to KRAS |
|---|---|---|---|---|
| ACCUGUCUCUUG | **2160** | CAAGAGACAGGU | **2161** | AD-39815.1,AD-39803.1,AD-39821.1,AD-39809.1,AD-39792.1,AD-39780.1,AD-39798.1,AD-39786.1 |
| CCUGUCUCUUGG | **2162** | CCAAGAGACAGG | **2163** | AD-39815.1,AD-39821.1,AD-39809.1,AD-39792.1,AD-39804.1,AD-39780.1,AD-39798.1,AD-39786.1 |
| CUGUCUCUUGGA | **2164** | UCCAAGAGACAG | **2165** | AD-39815.1,AD-39821.1,AD-39792.1,AD-39804.1,AD-39810.1,AD-39780.1,AD-39798.1,AD-39786.1 |
| UGUCUCUUGGAU | **2166** | AUCCAAGAGACA | **2167** | AD-39816.1,AD-39821.1,AD-39792.1,AD-39804.1,AD-39810.1,AD-39780.1,AD-39798.1,AD-39786.1 |
| GUCUCUUGGAUA | **2168** | UAUCCAAGAGAC | **2169** | AD-39816.1,AD-39792.1,AD-39804.1,AD-39810.1,AD-39780.1,AD-39822.1,AD-39798.1,AD-39786.1 |
| UCUCUUGGAUAU | **2170** | AUAUCCAAGAGA | **2171** | AD-39816.1,AD-39792.1,AD-39804.1,AD-39810.1,AD-39781.1,AD-39822.1,AD-39798.1,AD-39786.1 |
| CUCUUGGAUAUU | **2172** | AAUAUCCAAGAG | **2173** | AD-39816.1,AD-39792.1,AD-39804.1,AD-39787.1,AD-39810.1,AD-39781.1,AD-39822.1,AD-39798.1 |
| UCUUGGAUAUUC | **2174** | GAAUAUCCAAGA | **2175** | AD-39816.1,AD-39793.1,AD-39804.1,AD-39787.1,AD-39810.1,AD-39781.1,AD-39822.1,AD-39798.1 |
| CUUGGAUAUUCU | **2176** | AGAAUAUCCAAG | **2177** | AD-39799.1,AD-39816.1,AD-39793.1,AD-39804.1,AD-39787.1,AD-39810.1,AD-39781.1,AD-39822.1 |
| UUGGAUAUUCUC | **2178** | GAGAAUAUCCAA | **2179** | AD-39799.1,AD-39816.1,AD-39793.1,AD-39787.1,AD-35539.4,AD-39810.1,AD-39781.1,AD-39822.1 |
| UGGAUAUUCUCG | **2180** | CGAGAAUAUCCA | **2181** | AD-39799.1,AD-39816.1,AD-39793.1,AD-39787.1,AD-35539.4,AD-35545.4,AD-39781.1,AD-39822.1 |
| GGAUAUUCUCGA | **2182** | UCGAGAAUAUCC | **2183** | AD-39799.1,AD-35551.4,AD-39793.1,AD-39787.1,AD-35539.4,AD-35545.4,AD-39781.1,AD-39822.1 |
| GAUAUUCUCGAC | **2184** | GUCGAGAAUAUC | **2185** | AD-39799.1,AD-35551.4,AD-39793.1,AD-35557.1,AD-39787.1,AD-35539.4,AD-35545.4,AD-39781.1 |
| AUAUUCUCGACA | **2186** | UGUCGAGAAUAU | **2187** | AD-39799.1,AD-35551.4,AD-39793.1,AD-35557.1,AD-39787.1,AD-35539.4,AD-35545.4,AD-35563.4 |
| UAUUCUCGACAC | **2188** | GUGUCGAGAAUA | **2189** | AD-39799.1,AD-35551.4,AD-39793.1,AD-35557.1,AD-35539.4,AD-35545.4,AD-35569.1 , AD-35563.4 |

(continued)

| Sense overlap | SEQ ID NO: | Anti-sense overlap | SEQ ID NO: | Overlapping RNAi agents to KRAS |
|---|---|---|---|---|
| AUUCUCGACACA | **2190** | UGUGUCGAGAAU | **2191** | AD-39799.1,AD-35528.4,AD-35551.4,AD-35557.1,AD-35539.4,AD-35545.4,AD-35569.1,AD-35563.4 |
| UUCUCGACACAG | **2192** | CUGUGUCGAGAA | **2193** | AD-35528.4,AD-35551.4,AD-35557.1,AD-35539.4,AD-35534.1,AD-35545.4,AD-35569.1,AD-35563.4 |
| UCUCGACACAGC | **2194** | GCUGUGUCGAGA | **2195** | AD-35528.4,AD-35551.4,AD-35557.1,AD-35540.4,AD-35534.1,AD-35545.4,AD-35569.1,AD-35563.4 |
| CUCGACACAGCA | **2196** | UGCUGUGUCGAG | **2197** | AD-35528.4,AD-35551.4,AD-35546.4,AD-35557.1,AD-35540.4,AD-35534.1,AD-35569.1,AD-35563.4 |
| UCGACACAGCAG | **2198** | CUGCUGUGUCGA | **2199** | AD-35552.4,AD-35528.4,AD-35546.4,AD-35557.1,AD-35540.4,AD-35534.1,AD-35569.1,AD-35563.4 |
| CGACACAGCAGG | **2200** | CCUGCUGUGUCG | **2201** | AD-35552.4,AD-35528.4,AD-35558.4,AD-35546.4,AD-35540.4,AD-35534.1,AD-35569.1,AD-35563.4 |
| GACACAGCAGGU | **2202** | ACCUGCUGUGUC | **2203** | AD-35552.4,AD-35528.4,AD-35558.4,AD-35546.4,AD-35540.4,AD-35534.1,AD-35564.4,AD-35569.1 |
| ACACAGCAGGUC | **2204** | GACCUGCUGUGU | **2205** | AD-35552.4,AD-35528.4,AD-35558.4,AD-35546.4,AD-35540.4,AD-35570.4,AD-35534.1,AD-35564.4 |
| CACAGCAGGUCA | **2206** | UGACCUGCUGUG | **2207** | AD-35552.4,AD-35558.4,AD-35546.4,AD-35540.4,AD-35570.4,AD-35534.1,AD-35564.4,AD-35571.4 |
| ACAGCAGGUCAA | **2208** | UUGACCUGCUGU | **2209** | AD-35577.4,AD-35552.4,AD-35558.4,AD-35546.4,AD-35540.4,AD-35570.4,AD-35564.4,AD-35571.4 |
| CAGCAGGUCAAG | **2210** | CUUGACCUGCUG | **2211** | AD-35577.4,AD-35552.4,AD-35558.4,AD-35546.4,AD-35583.4,AD-35570.4,AD-35564.4,AD-35571.4 |
| AGCAGGUCAAGA | **2212** | UCUUGACCUGCU | **2213** | AD-35577.4,AD-35552.4,AD-35558.4,AD-35583.4,AD-35570.4,AD-35564.4,AD-35589.4,AD-35571.4 |
| GCAGGUCAAGAG | **2214** | CUCUUGACCUGC | **2215** | AD-35577.4,AD-35595.4,AD-35558.4,AD-35583.4,AD-35570.4,AD-35564.4,AD-35589.4,AD-35571.4 |
| CAGGUCAAGAGG | **2216** | CCUCUUGACCUG | **2217** | AD-35577.4,AD-35595.4,AD-35583.4,AD-35570.4,AD-35564.4,AD-35589.4,AD-35571.4,AD-35601.4 |
| AGGUCAAGAGGA | **2218** | UCCUCUUGACCU | **2219** | AD-35577.4,AD-35595.4,AD-35583.4,AD-35570.4,AD-35589.4,AD-35607.4,AD-35571.4,AD-35601.4 |

(continued)

| Sense overlap | SEQ ID NO: | Anti-sense overlap | SEQ ID NO: | Overlapping RNAi agents to KRAS |
|---|---|---|---|---|
| GGUCAAGAGGAG | **2220** | CUCCUCUUGACC | **2221** | AD-35577.4,AD-35595.4,AD-35583.4,AD-35589.4,AD-35607.4,AD-35571.4,AD-35613.4,AD-35601.4 |
| GUCAAGAGGAGU | **2222** | ACUCCUCUUGAC | **2223** | AD-35577.4,AD-35595.4,AD-35583.4,AD-35572.4,AD-35589.4,AD-35607.4,AD-35613.4,AD-35601.4 |
| UCAAGAGGAGUA | **2224** | UACUCCUCUUGA | **2225** | AD-35595.4,AD-35583.4,AD-35578.4,AD-35572.4,AD-35589.4,AD-35607.4,AD-35613.4,AD-35601.4 |
| CAAGAGGAGUAC | **2226** | GUACUCCUCUUG | **2227** | AD-35595.4,AD-35578.4,AD-35572.4,AD-35584.4,AD-35589.4,AD-35607.4,AD-35613.4,AD-35601.4 |
| AAGAGGAGUACA | **2228** | UGUACUCCUCUU | **2229** | AD-35590.4,AD-35595.4,AD-35578.4,AD-35572.4,AD-35584.4,AD-35607.4,AD-35613.4,AD-35601.4 |
| AGAGGAGUACAG | **2230** | CUGUACUCCUCU | **2231** | AD-35596.4,AD-35590.4,AD-35578.4,AD-35572.4,AD-35584.4,AD-35607.4,AD-35613.4,AD-35601.4 |
| GAGGAGUACAGU | **2232** | ACUGUACUCCUC | **2233** | AD-35596.4,AD-35590.4,AD-35578.4,AD-35572.4,AD-35602.4,AD-35584.4,AD-35607.4,AD-35613.4 |
| AGGAGUACAGUG | **2234** | CACUGUACUCCU | **2235** | AD-35608.4,AD-35596.4,AD-35590.4,AD-35578.4,AD-35572.4,AD-35602.4,AD-35584.4,AD-35613.4 |
| GGAGUACAGUGC | **2236** | GCACUGUACUCC | **2237** | AD-35608.4,AD-35596.4,AD-35590.4,AD-35578.4,AD-35572.4,AD-35614.4,AD-35602.4,AD-35584.4 |
| GAGUACAGUGCA | **2238** | UGCACUGUACUC | **2239** | AD-35608.4,AD-35596.4,AD-35590.4,AD-35578.4,AD-35614.4,AD-35602.4,AD-35584.4,AD-35573.4 |
| AGUACAGUGCAA | **2240** | UUGCACUGUACU | **2241** | AD-39805.1,AD-35608.4,AD-35596.4,AD-35590.4,AD-35614.4,AD-35602.4,AD-35584.4,AD-35573.4 |
| GUACAGUGCAAU | **2242** | AUUGCACUGUAC | **2243** | AD-39805.1,AD-35608.4,AD-35596.4,AD-35590.4,AD-39811.1,AD-35614.4,AD-35602.4,AD-35573.4 |
| UACAGUGCAAUG | **2244** | CAUUGCACUGUA | **2245** | AD-39805.1,AD-35608.4,AD-35596.4,AD-39817.1,AD-39811.1,AD-35614.4,AD-35602.4,AD-35573.4 |
| ACAGUGCAAUGA | **2246** | UCAUUGCACUGU | **2247** | AD-39805.1,AD-35608.4,AD-39823.1,AD-39817.1,AD-39811.1,AD-35614.4,AD-35602.4,AD-35573.4 |
| CAGUGCAAUGAG | **2248** | CUCAUUGCACUG | **2249** | AD-39805.1,AD-35608.4,AD-39823.1,AD-39817.1,AD-39811.1,AD-35614.4,AD-35573.4,AD-39782.1 |

(continued)

| Sense overlap | SEQ ID NO: | Anti-sense overlap | SEQ ID NO: | Overlapping RNAi agents to KRAS |
|---|---|---|---|---|
| AGUGCAAUGAGG | **2250** | CCUCAUUGCACU | **2251** | AD-39805.1,AD-39823.1,AD-39817.1,AD-39788.1,AD-39811.1,AD-35614.4,AD-35573.4,AD-39782.1 |
| GUGCAAUGAGGG | **2252** | CCCUCAUUGCAC | **2253** | AD-39805.1,AD-39823.1,AD-39817.1,AD-39788.1,AD-39811.1,AD-35573.4,AD-39782.1 , AD-39794.1 |
| UGCAAUGAGGGA | **2254** | UCCCUCAUUGCA | **2255** | AD-39805.1,AD-39823.1,AD-39817.1,AD-39788.1,AD-39811.1,AD-39782.1,AD-39794.1,AD-39800.1 |
| GCAAUGAGGGAC | **2256** | GUCCCUCAUUGC | **2257** | AD-39806.1,AD-39823.1,AD-39817.1,AD-39788.1,AD-39811.1,AD-39782.1,AD-39794.1,AD-39800.1 |
| CAAUGAGGGACC | **2258** | GGUCCCUCAUUG | **2259** | AD-39806.1,AD-39823.1,AD-39812.1,AD-39817.1,AD-39788.1,AD-39782.1,AD-39794.1,AD-39800.1 |
| AAUGAGGGACCA | **2260** | UGGUCCCUCAUU | **2261** | AD-39806.1,AD-39823.1,AD-39812.1,AD-39788.1,AD-39782.1,AD-39794.1,AD-39818.1,AD-39800.1 |
| AUGAGGGACCAG | **2262** | CUGGUCCCUCAU | **2263** | AD-39806.1,AD-39812.1,AD-39788.1,AD-39824.1,AD-39782.1,AD-39794.1,AD-39818.1,AD-39800.1 |
| UGAGGGACCAGU | **2264** | ACUGGUCCCUCA | **2265** | AD-39806.1,AD-39812.1,AD-39788.1,AD-39824.1,AD-39783.1,AD-39794.1,AD-39818.1,AD-39800.1 |
| GAGGGACCAGUA | **2266** | UACUGGUCCCUC | **2267** | AD-39789.1,AD-39806.1,AD-39812.1,AD-39824.1,AD-39783.1,AD-39794.1,AD-39818.1,AD-39800.1 |
| AGGGACCAGUAC | **2268** | GUACUGGUCCCU | **2269** | AD-39789.1,AD-39806.1,AD-39812.1,AD-39824.1,AD-39795.1,AD-39783.1,AD-39818.1,AD-39800.1 |
| GGGACCAGUACA | **2270** | UGUACUGGUCCC | **2271** | AD-39789.1,AD-39806.1,AD-39812.1,AD-39824.1,AD-39795.1,AD-39783.1,AD-39801.1,AD-39818.1 |
| GGACCAGUACAU | **2272** | AUGUACUGGUCC | **2273** | AD-39789.1,AD-39807.1,AD-39812.1,AD-39824.1,AD-39795.1,AD-39783.1,AD-39801.1,AD-39818.1 |
| GACCAGUACAUG | **2274** | CAUGUACUGGUC | **2275** | AD-39789.1,AD-39807.1,AD-39824.1,AD-39795.1,AD-39783.1,AD-39801.1,AD-39813.1,AD-39818.1 |
| ACCAGUACAUGA | **2276** | UCAUGUACUGGU | **2277** | AD-39789.1,AD-39807.1,AD-39824.1,AD-39795.1,AD-39783.1,AD-39801.1,AD-39813.1,AD-39819.1 |
| CCAGUACAUGAG | **2278** | CUCAUGUACUGG | **2279** | AD-39789.1,AD-39807.1,AD-39795.1,AD-39783.1,AD-39801.1,AD-39813.1,AD-39825.1,AD-39819.1 |

(continued)

| Sense overlap | SEQ ID NO: | Anti-sense overlap | SEQ ID NO: | Overlapping RNAi agents to KRAS |
|---|---|---|---|---|
| CAGUACAUGAGG | **2280** | CCUCAUGUACUG | **2281** | AD-39789.1,AD-39807.1,AD-39795.1,AD-39801.1,AD-39813.1,AD-39825.1,AD-39831.1,AD-39819.1 |
| AGUACAUGAGGA | **2282** | UCCUCAUGUACU | **2283** | AD-39807.1,AD-39837.1,AD-39795.1,AD-39801.1,AD-39813.1,AD-39825.1,AD-39831.1,AD-39819.1 |
| GUACAUGAGGAC | **2284** | GUCCUCAUGUAC | **2285** | AD-39807.1,AD-39837.1,AD-39843.1,AD-39801.1,AD-39813.1,AD-39825.1,AD-39831.1,AD-39819.1 |
| UACAUGAGGACU | **2286** | AGUCCUCAUGUA | **2287** | AD-39807.1,AD-39837.1,AD-39843.1,AD-39813.1,AD-39825.1,AD-39831.1,AD-39849.1,AD-39819.1 |
| ACAUGAGGACUG | **2288** | CAGUCCUCAUGU | **2289** | AD-39837.1,AD-39843.1,AD-39813.1,AD-39825.1,AD-39855.1,AD-39831.1,AD-39849.1,AD-39819.1 |
| CAUGAGGACUGG | **2290** | CCAGUCCUCAUG | **2291** | AD-39861.1,AD-39837.1,AD-39843.1,AD-39825.1,AD-39855.1,AD-39831.1,AD-39849.1,AD-39819.1 |
| AUGAGGACUGGG | **2292** | CCCAGUCCUCAU | **2293** | AD-39861.1,AD-39837.1,AD-39843.1,AD-39825.1,AD-39855.1,AD-39831.1,AD-39867.1,AD-39849.1 |
| UGAGGACUGGGG | **2294** | CCCCAGUCCUCA | **2295** | AD-39861.1,AD-39837.1,AD-39843.1,AD-39826.1,AD-39855.1,AD-39831.1,AD-39867.1,AD-39849.1 |
| GAGGACUGGGGA | **2296** | UCCCCAGUCCUC | **2297** | AD-39861.1,AD-39832.1,AD-39837.1,AD-39843.1,AD-39826.1,AD-39855.1,AD-39867.1,AD-39849.1 |
| AGGACUGGGGAG | **2298** | CUCCCCAGUCCU | **2299** | AD-39861.1,AD-39832.1,AD-39843.1,AD-39826.1,AD-39855.1,AD-39867.1,AD-39849.1,AD-39838.1 |
| GGACUGGGGAGG | **2300** | CCUCCCCAGUCC | **2301** | AD-39844.1,AD-39861.1,AD-39832.1,AD-39826.1,AD-39855.1,AD-39867.1,AD-39849.1,AD-39838.1 |
| GACUGGGGAGGG | **2302** | CCCUCCCCAGUC | **2303** | AD-39850.1,AD-39844.1,AD-39861.1,AD-39832.1,AD-39826.1,AD-39855.1,AD-39867.1,AD-39838.1 |
| ACUGGGGAGGGC | **2304** | GCCCUCCCCAGU | **2305** | AD-39850.1,AD-39844.1,AD-39861.1,AD-39832.1,AD-39856.1,AD-39826.1,AD-39867.1,AD-39838.1 |
| CUGGGGAGGGCU | **2306** | AGCCCUCCCCAG | **2307** | AD-39850.1,AD-39844.1,AD-39862.1,AD-39832.1,AD-39856.1,AD-39826.1,AD-39867.1,AD-39838.1 |
| UGGGGAGGGCUU | **2308** | AAGCCCUCCCCA | **2309** | AD-39850.1,AD-39844.1,AD-39868.1,AD-39862.1,AD-39832.1,AD-39856.1,AD-39826.1,AD-39838.1 |

(continued)

| Sense overlap | SEQ ID NO: | Anti-sense overlap | SEQ ID NO: | Overlapping RNAi agents to KRAS |
|---|---|---|---|---|
| GGGGAGGGCUUU | **2310** | AAAGCCCUCCCC | **2311** | AD-39850.1,AD-39844.1,AD-39868.1,AD-35579.4,AD-39862.1,AD-39832.1,AD-39856.1,AD-39838.1 |
| GGGAGGGCUUUC | **2312** | GAAAGCCCUCCC | **2313** | AD-35585.4,AD-39850.1,AD-39844.1,AD-39868.1,AD-35579.4,AD-39862.1,AD-39856.1,AD-39838.1 |
| GGAGGGCUUUCU | **2314** | AGAAAGCCCUCC | **2315** | AD-35585.4,AD-39850.1,AD-39844.1,AD-39868.1,AD-35579.4,AD-39862.1,AD-39856.1,AD-35591.4 |
| GAGGGCUUUCUU | **2316** | AAGAAAGCCCUC | **2317** | AD-35585.4,AD-39850.1,AD-39868.1,AD-35579.4,AD-39862.1,AD-35597.4,AD-39856.1,AD-35591.4 |
| AGGGCUUUCUUU | **2318** | AAAGAAAGCCCU | **2319** | AD-35585.4,AD-39868.1,AD-35579.4,AD-39862.1,AD-35597.4,AD-39856.1,AD-35591.4,AD-35603.4 |
| GGGCUUUCUUUG | **2320** | CAAAGAAAGCCC | **2321** | AD-35585.4,AD-39868.1,AD-35579.4,AD-39862.1,AD-35597.4,AD-35609.4,AD-35591.4,AD-35603.4 |
| GGCUUUCUUUGU | **2322** | ACAAAGAAAGCC | **2323** | AD-35585.4,AD-39868.1,AD-35579.4,AD-35615.4,AD-35597.4,AD-35609.4,AD-35591.4,AD-35603.4 |
| GCUUUCUUUGUG | **2324** | CACAAAGAAAGC | **2325** | AD-35585.4,AD-35579.4,AD-35615.4,AD-35597.4,AD-35609.4,AD-35591.4,AD-35603.4,AD-35574.4 |
| CUUUCUUUGUGU | **2326** | ACACAAAGAAAG | **2327** | AD-35585.4,AD-35615.4,AD-35597.4,AD-35609.4,AD-35591.4,AD-35603.4,AD-35580.1,AD-35574.4 |
| UUUCUUUGUGUA | **2328** | UACACAAAGAAA | **2329** | AD-35615.4,AD-35597.4,AD-35609.4,AD-35591.4,AD-35603.4,AD-35586.4,AD-35580.1,AD-35574.4 |
| UUCUUUGUGUAU | **2330** | AUACACAAAGAA | **2331** | AD-35615.4,AD-35597.4,AD-35609.4,AD-35592.4,AD-35603.4,AD-35586.4,AD-35580.1,AD-35574.4 |
| UCUUUGUGUAUU | **2332** | AAUACACAAAGA | **2333** | AD-35615.4,AD-35609.4,AD-35592.4,AD-35603.4,AD-35586.4,AD-35598.4,AD-35580.1,AD-35574.4 |
| CUUUGUGUAUUU | **2334** | AAAUACACAAAG | **2335** | AD-35604.4,AD-35615.4,AD-35609.4,AD-35592.4,AD-35586.4,AD-35598.4,AD-35580.1,AD-35574.4 |
| UUUGUGUAUUUG | **2336** | CAAAUACACAAA | **2337** | AD-35604.4,AD-35615.4,AD-35592.4,AD-35586.4,AD-35610.4,AD-35598.4,AD-35580.1,AD-35574.4 |
| UUGUGUAUUUGC | **2338** | GCAAAUACACAA | **2339** | AD-35604.4,AD-35592.4,AD-35586.4,AD-35610.4,AD-35598.4,AD-35616.4,AD-35580.1,AD-35574.4 |

(continued)

| Sense overlap | SEQ ID NO: | Anti-sense overlap | SEQ ID NO: | Overlapping RNAi agents to KRAS |
|---|---|---|---|---|
| UGUGUAUUUGCC | **2340** | GGCAAAUACACA | **2341** | AD-35604.4,AD-35575.4,AD-35592.4,AD-35586.4,AD-35610.4,AD-35598.4,AD-35616.4,AD-35580.1 |
| GUGUAUUUGCCA | **2342** | UGGCAAAUACAC | **2343** | AD-35604.4,AD-35575.4,AD-35581.4,AD-35592.4,AD-35586.4,AD-35610.4,AD-35598.4,AD-35616.4 |
| UGUAUUUGCCAU | **2344** | AUGGCAAAUACA | **2345** | AD-35604.4,AD-35575.4,AD-35581.4,AD-35592.4,AD-35587.4,AD-35610.4,AD-35598.4,AD-35616.4 |
| GUAUUUGCCAUA | **2346** | UAUGGCAAAUAC | **2347** | AD-35604.4,AD-35575.4,AD-35581.4,AD-35587.4,AD-35610.4,AD-35598.4,AD-35616.4,AD-35593.4 |
| UAUUUGCCAUAA | **2348** | UUAUGGCAAAUA | **2349** | AD-35604.4,AD-35575.4,AD-35581.4,AD-35587.4,AD-35610.4,AD-35616.4,AD-35593.4,AD-35599.4 |
| AUUUGCCAUAAA | **2350** | UUUAUGGCAAAU | **2351** | AD-35575.4,AD-35581.4,AD-35587.4,AD-35610.4,AD-35605.4,AD-35616.4,AD-35593.4,AD-35599.4 |
| UUUGCCAUAAAU | **2352** | AUUUAUGGCAAA | **2353** | AD-35611.4,AD-35575.4,AD-35581.4,AD-35587.4,AD-35605.4,AD-35616.4,AD-35593.4,AD-35599.4 |
| UUGCCAUAAAUA | **2354** | UAUUUAUGGCAA | **2355** | AD-35611.4,AD-35575.4,AD-35581.4,AD-35587.4,AD-35605.4,AD-35593.4,AD-39827.1,AD-35599.4 |
| UGCCAUAAAUAA | **2356** | UUAUUUAUGGCA | **2357** | AD-35611.4,AD-35581.4,AD-35587.4,AD-39833.1,AD-35605.4,AD-35593.4,AD-39827.1,AD-35599.4 |
| GCCAUAAAUAAU | **2358** | AUUAUUUAUGGC | **2359** | AD-39839.1,AD-35611.4,AD-35587.4,AD-39833.1,AD-35605.4,AD-35593.4,AD-39827.1,AD-35599.4 |
| CCAUAAAUAAUA | **2360** | UAUUAUUUAUGG | **2361** | AD-39839.1,AD-35611.4,AD-39833.1,AD-35605.4,AD-35593.4,AD-39827.1,AD-35599.4,AD-39845.1 |
| CAUAAAUAAUAC | **2362** | GUAUUAUUUAUG | **2363** | AD-39839.1,AD-35611.4,AD-39833.1,AD-35605.4,AD-39851.1,AD-39827.1,AD-35599.4,AD-39845.1 |
| AUAAAUAAUACU | **2364** | AGUAUUAUUUAU | **2365** | AD-39839.1,AD-35611.4,AD-39857.1,AD-39833.1,AD-35605.4,AD-39851.1,AD-39827.1,AD-39845.1 |
| UAAAUAAUACUA | **2366** | UAGUAUUAUUUA | **2367** | AD-39839.1,AD-35611.4,AD-39857.1,AD-39833.1,AD-39851.1,AD-39863.1,AD-39827.1,AD-39845.1 |
| AAAUAAUACUAA | **2368** | UUAGUAUUAUUU | **2369** | AD-39839.1,AD-39857.1,AD-39869.1,AD-39833.1,AD-39851.1,AD-39863.1,AD-39827.1,AD-39845.1 |

(continued)

| Sense overlap | SEQ ID NO: | Anti-sense overlap | SEQ ID NO: | Overlapping RNAi agents to KRAS |
|---|---|---|---|---|
| AAUAAUACUAAA | **2370** | UUUAGUAUUAUU | **2371** | AD-39828.1,AD-39839.1,AD-39857.1,AD-39869.1,AD-39833.1,AD-39851.1,AD-39863.1,AD-39845.1 |
| AUAAUACUAAAU | **2372** | AUUUAGUAUUAU | **2373** | AD-39828.1,AD-39839.1,AD-39857.1,AD-39834.1,AD-39869.1,AD-39851.1,AD-39863.1,AD-39845.1 |
| UAAUACUAAAUC | **2374** | GAUUUAGUAUUA | **2375** | AD-39828.1,AD-39857.1,AD-39834.1,AD-39869.1,AD-39840.1,AD-39851.1,AD-39863.1,AD-39845.1 |
| AAUACUAAAUCA | **2376** | UGAUUUAGUAUU | **2377** | AD-39828.1,AD-39857.1,AD-39834.1,AD-39869.1,AD-39840.1,AD-39851.1,AD-39863.1,AD-39846.1 |
| AUACUAAAUCAU | **2378** | AUGAUUUAGUAU | **2379** | AD-39828.1,AD-39852.1,AD-39857.1,AD-39834.1,AD-39869.1,AD-39840.1,AD-39863.1,AD-39846.1 |
| UACUAAAUCAUU | **2380** | AAUGAUUUAGUA | **2381** | AD-39828.1,AD-39852.1,AD-39834.1,AD-39858.1,AD-39869.1,AD-39840.1,AD-39863.1,AD-39846.1 |
| ACUAAAUCAUUU | **2382** | AAAUGAUUUAGU | **2383** | AD-39828.1,AD-39852.1,AD-39834.1,AD-39858.1,AD-39869.1,AD-39840.1,AD-39864.1,AD-39846.1 |
| CUAAAUCAUUUG | **2384** | CAAAUGAUUUAG | **2385** | AD-39828.1,AD-39852.1,AD-39870.1,AD-39834.1,AD-39858.1,AD-39840.1,AD-39864.1,AD-39846.1 |
| UAAAUCAUUUGA | **2386** | UCAAAUGAUUUA | **2387** | AD-39852.1,AD-39870.1,AD-39829.1,AD-39834.1,AD-39858.1,AD-39840.1,AD-39864.1,AD-39846.1 |
| AAAUCAUUUGAA | **2388** | UUCAAAUGAUUU | **2389** | AD-39852.1,AD-39870.1,AD-39829.1,AD-39858.1,AD-39840.1,AD-39835.1,AD-39864.1,AD-39846.1 |
| AAUCAUUUGAAG | **2390** | CUUCAAAUGAUU | **2391** | AD-39852.1,AD-39870.1,AD-39841.1,AD-39829.1,AD-39858.1,AD-39835.1,AD-39864.1,AD-39846.1 |
| AUCAUUUGAAGA | **2392** | UCUUCAAAUGAU | **2393** | AD-39852.1,AD-39870.1,AD-39841.1,AD-39829.1,AD-39858.1,AD-39835.1,AD-39864.1 |
| UCAUUUGAAGAU | **2394** | AUCUUCAAAUGA | **2395** | AD-39870.1,AD-39841.1,AD-39829.1,AD-39858.1,AD-39835.1,AD-39864.1,AD-39853.1 |
| CAUUUGAAGAUA | **2396** | UAUCUUCAAAUG | **2397** | AD-39870.1,AD-39841.1,AD-39829.1,AD-39835.1,AD-39859.1,AD-39864.1,AD-39853.1 |
| AUUUGAAGAUAU | **2398** | AUAUCUUCAAAU | **2399** | AD-39865.1,AD-39870.1,AD-39841.1,AD-39829.1,AD-39835.1,AD-39859.1,AD-39853.1 |
| UUUGAAGAUAUU | **2400** | AAUAUCUUCAAA | **2401** | AD-39871.1,AD-39865.1,AD-39841.1,AD-39829.1,AD-39835.1,AD-39859.1,AD-39853.1 |
| UUGAAGAUAUUC | **2402** | GAAUAUCUUCAA | **2403** | AD-39871.1,AD-39865.1,AD-39841.1,AD-39830.1,AD-39835.1,AD-39859.1,AD-39853.1 |

(continued)

| Sense overlap | SEQ ID NO: | Anti-sense overlap | SEQ ID NO: | Overlapping RNAi agents to KRAS |
|---|---|---|---|---|
| UGAAGAUAUUCA | **2404** | UGAAUAUCUUCA | **2405** | AD-39871.1,AD-39865.1,AD-39841.1,AD-39830.1,AD-35617.4,AD-39859.1,AD-39853.1 |
| GAAGAUAUUCAC | **2406** | GUGAAUAUCUUC | **2407** | AD-39871.1,AD-39865.1,AD-39830.1,AD-35617.4,AD-35576.4,AD-39859.1,AD-39853.1 |
| AAGAUAUUCACC | **2408** | GGUGAAUAUCUU | **2409** | AD-35582.1,AD-39871.1,AD-39865.1,AD-39830.1,AD-35617.4,AD-35576.4,AD-39859.1,AD-39853.1 |
| AGAUAUUCACCA | **2410** | UGGUGAAUAUCU | **2411** | AD-35582.1,AD-39871.1,AD-39865.1,AD-39830.1,AD-35617.4,AD-35576.4,AD-39859.1,AD-35588.4 |
| GAUAUUCACCAU | **2412** | AUGGUGAAUAUC | **2413** | AD-35582.1,AD-39871.1,AD-39865.1,AD-35594.1,AD-39830.1,AD-35617.4,AD-35576.4,AD-35588.4 |
| AUAUUCACCAUU | **2414** | AAUGGUGAAUAU | **2415** | AD-35582.1,AD-39871.1,AD-35594.1,AD-35600.1,AD-39830.1,AD-35617.4,AD-35576.4,AD-35588.4 |
| UAUUCACCAUUA | **2416** | UAAUGGUGAAUA | **2417** | AD-35582.1,AD-35606.1,AD-35594.1,AD-35600.1,AD-39830.1,AD-35617.4,AD-35576.4,AD-35588.4 |
| AUUCACCAUUAU | **2418** | AUAAUGGUGAAU | **2419** | AD-35582.1,AD-35606.1,AD-35594.1,AD-35612.1,AD-35600.1,AD-35617.4,AD-35576.4,AD-35588.4 |
| UUCACCAUUAUA | **2420** | UAUAAUGGUGAA | **2421** | AD-35582.1,AD-35606.1,AD-35594.1,AD-35612.1,AD-35600.1,AD-35576.4,AD-35618.1,AD-35588.4 |
| UCACCAUUAUAG | **2422** | CUAUAAUGGUGA | **2423** | AD-35582.1,AD-35606.1,AD-35594.1,AD-35612.1,AD-35600.1,AD-35618.1,AD-35588.4,AD-38127.1 |
| CACCAUUAUAGA | **2424** | UCUAUAAUGGUG | **2425** | AD-38133.1,AD-35606.1,AD-35594.1,AD-35612.1,AD-35600.1,AD-35618.1,AD-35588.4,AD-38127.1 |
| ACCAUUAUAGAG | **2426** | CUCUAUAAUGGU | **2427** | AD-38133.1,AD-35606.1,AD-35594.1,AD-35612.1,AD-35600.1,AD-38139.1,AD-35618.1,AD-38127.1 |
| CCAUUAUAGAGA | **2428** | UCUCUAUAAUGG | **2429** | AD-38133.1,AD-35606.1,AD-35612.1,AD-35600.1,AD-38145.1,AD-38139.1,AD-35618.1,AD-38127.1 |
| CAUUAUAGAGAA | **2430** | UUCUCUAUAAUG | **2431** | AD-38133.1,AD-35606.1,AD-35612.1,AD-38145.1,AD-38139.1,AD-38151.1,AD-35618.1,AD-38127.1 |
| AUUAUAGAGAAC | **2432** | GUUCUCUAUAAU | **2433** | AD-38133.1,AD-35612.1,AD-38145.1,AD-38139.1,AD-38151.1,AD-38157.1,AD-35618.1,AD-38127.1 |

(continued)

| Sense overlap | SEQ ID NO: | Anti-sense overlap | SEQ ID NO: | Overlapping RNAi agents to KRAS |
|---|---|---|---|---|
| UUAUAGAGAACA | **2434** | UGUUCUCUAUAA | **2435** | AD-38133.1,AD-38163.1,AD-38145.1,AD-38139.1,AD-38151.1,AD-38157.1,AD-35618.1,AD-38127.1 |
| UAUAGAGAACAA | **2436** | UUGUUCUCUAUA | **2437** | AD-38133.1,AD-38163.1,AD-38145.1,AD-38169.1,AD-38139.1,AD-38151.1,AD-38157.1,AD-38127.1 |
| AUAGAGAACAAA | **2438** | UUUGUUCUCUAU | **2439** | AD-38133.1,AD-38163.1,AD-38145.1,AD-38169.1,AD-38139.1,AD-38151.1,AD-38157.1,AD-38128.1 |
| UAGAGAACAAAU | **2440** | AUUUGUUCUCUA | **2441** | AD-38163.1,AD-38145.1,AD-38169.1,AD-38139.1,AD-38151.1,AD-38157.1,AD-38128.1,AD-38134.1 |
| AGAGAACAAAUU | **2442** | AAUUUGUUCUCU | **2443** | AD-38163.1,AD-38145.1,AD-38140.1,AD-38169.1,AD-38151.1,AD-38157.1,AD-38128.1,AD-38134.1 |
| GAGAACAAAUUA | **2444** | UAAUUUGUUCUC | **2445** | AD-38163.1,AD-38140.1,AD-38169.1,AD-38151.1,AD-38157.1,AD-38128.1,AD-39836.1,AD-38134.1 |
| AGAACAAAUUAA | **2446** | UUAAUUUGUUCU | **2447** | AD-38163.1,AD-38140.1,AD-38169.1,AD-38157.1,AD-38128.1,AD-39842.1,AD-39836.1,AD-38134.1 |
| GAACAAAUUAAA | **2448** | UUUAAUUUGUUC | **2449** | AD-38163.1,AD-38140.1,AD-38169.1,AD-38128.1,AD-39842.1,AD-39848.1,AD-39836.1,AD-38134.1 |
| AACAAAUUAAAA | **2450** | UUUUAAUUUGUU | **2451** | AD-38140.1,AD-38169.1,AD-39854.1,AD-38128.1,AD-39842.1,AD-39848.1,AD-39836.1,AD-38134.1 |
| ACAAAUUAAAAG | **2452** | CUUUUAAUUUGU | **2453** | AD-39860.1,AD-38140.1,AD-39854.1,AD-38128.1,AD-39842.1,AD-39848.1,AD-39836.1,AD-38134.1 |
| CAAAUUAAAAGA | **2454** | UCUUUUAAUUUG | **2455** | AD-39860.1,AD-38140.1,AD-39854.1,AD-39842.1,AD-39848.1,AD-39866.1,AD-39836.1,AD-38134.1 |
| AAAUUAAAAGAG | **2456** | CUCUUUUAAUUU | **2457** | AD-39992.1,AD-39860.1,AD-38140.1,AD-39854.1,AD-39842.1,AD-39848.1,AD-39866.1,AD-39836.1 |
| AAUUAAAAGAGU | **2458** | ACUCUUUUAAUU | **2459** | AD-39992.1,AD-39860.1,AD-39998.1,AD-39854.1,AD-39842.1,AD-39848.1,AD-39866.1,AD-39836.1 |
| AUUAAAAGAGUU | **2460** | AACUCUUUUAAU | **2461** | AD-39992.1,AD-39860.1,AD-40004.1,AD-39998.1,AD-39854.1,AD-39842.1,AD-39848.1,AD-39866.1 |
| UUAAAAGAGUUA | **2462** | UAACUCUUUUAA | **2463** | AD-39992.1,AD-39860.1,AD-40004.1,AD-39998.1,AD-39854.1,AD-40010.1,AD-39848.1,AD-39866.1 |

(continued)

| Sense overlap | SEQ ID NO: | Anti-sense overlap | SEQ ID NO: | Overlapping RNAi agents to KRAS |
|---|---|---|---|---|
| UAAAAGAGUUAA | **2464** | UUAACUCUUUUA | **2465** | AD-40016.1,AD-39992.1,AD-39860.1,AD-40004.1,AD-39998.1,AD-39854.1,AD-40010.1,AD-39866.1 |
| AAAAGAGUUAAG | **2466** | CUUAACUCUUUU | **2467** | AD-40016.1,AD-39992.1,AD-39860.1,AD-40022.1,AD-40004.1,AD-39998.1,AD-40010.1,AD-39866.1 |
| AAAGAGUUAAGG | **2468** | CCUUAACUCUUU | **2469** | AD-40016.1,AD-40028.1,AD-39992.1,AD-40022.1,AD-40004.1,AD-39998.1,AD-40010.1,AD-39866.1 |
| AAGAGUUAAGGA | **2470** | UCCUUAACUCUU | **2471** | AD-40016.1,AD-40028.1,AD-40034.1,AD-39992.1,AD-40022.1,AD-40004.1,AD-39998.1,AD-40010.1 |
| AGAGUUAAGGAC | **2472** | GUCCUUAACUCU | **2473** | AD-40016.1,AD-40028.1,AD-40034.1,AD-40022.1,AD-40004.1,AD-39998.1,AD-40010.1 |
| GAGUUAAGGACU | **2474** | AGUCCUUAACUC | **2475** | AD-40016.1,AD-40028.1,AD-40034.1,AD-39999.1,AD-40022.1,AD-40004.1,AD-40010.1 |
| AGUUAAGGACUC | **2476** | GAGUCCUUAACU | **2477** | AD-40016.1,AD-40028.1,AD-40034.1,AD-39999.1,AD-40022.1,AD-40005.1,AD-40010.1 |
| GUUAAGGACUCU | **2478** | AGAGUCCUUAAC | **2479** | AD-40016.1,AD-40011.1,AD-40028.1,AD-40034.1,AD-39999.1,AD-40022.1,AD-40005.1 |
| UUAAGGACUCUG | **2480** | CAGAGUCCUUAA | **2481** | AD-40017.1 ,AD-40011.1,AD-40028.1,AD-40034.1,AD-39999.1,AD-40022.1,AD-40005.1 |
| UAAGGACUCUGA | **2482** | UCAGAGUCCUUA | **2483** | AD-40017.1,AD-40011.1,AD-40028.1,AD-40034.1,AD-39999.1,AD-40005.1 |
| AAGGACUCUGAA | **2484** | UUCAGAGUCCUU | **2485** | AD-40017.1,AD-40029.1,AD-40011.1,AD-40034.1,AD-39999.1,AD-40005.1 |
| AGGACUCUGAAG | **2486** | CUUCAGAGUCCU | **2487** | AD-40017.1,AD-40029.1,AD-40011.1,AD-39999.1,AD-40005.1,AD-40035.1 |
| GGACUCUGAAGA | **2488** | UCUUCAGAGUCC | **2489** | AD-40017.1,AD-40029.1,AD-40011.1,AD-39994.1,AD-39999.1,AD-40005.1,AD-40035.1 |
| GACUCUGAAGAU | **2490** | AUCUUCAGAGUC | **2491** | AD-40017.1,AD-40029.1,AD-40011.1,AD-40000.1,AD-39994.1,AD-40005.1,AD-40035.1 |
| ACUCUGAAGAUG | **2492** | CAUCUUCAGAGU | **2493** | AD-40017.1,AD-40029.1,AD-40011.1,AD-40000.1,AD-39994.1,AD-40006.1,AD-40035.1 |
| CUCUGAAGAUGU | **2494** | ACAUCUUCAGAG | **2495** | AD-40017.1,AD-40029.1,AD-40000.1,AD-39994.1,AD-40012.1,AD-40006.1,AD-40035.1 |
| UCUGAAGAUGUA | **2496** | UACAUCUUCAGA | **2497** | AD-40029.1,AD-40000.1,AD-39994.1,AD-40012.1,AD-40006.1,AD-40035.1,AD-40018.1 |
| CUGAAGAUGUAC | **2498** | GUACAUCUUCAG | **2499** | AD-40029.1,AD-40000.1,AD-39994.1,AD-40024.1,AD-40012.1,AD-40006.1,AD-40035.1,AD-40018.1 |
| UGAAGAUGUACC | **2500** | GGUACAUCUUCA | **2501** | AD-40030.1,AD-40000.1,AD-39994.1,AD-40024.1,AD-40012.1,AD-40006.1,AD-40035.1,AD-40018.1 |

(continued)

| Sense overlap | SEQ ID NO: | Anti-sense overlap | SEQ ID NO: | Overlapping RNAi agents to KRAS |
|---|---|---|---|---|
| GAAGAUGUACCU | **2502** | AGGUACAUCUUC | **2503** | AD-40030.1,AD-40000.1,AD-39994.1,AD-40024.1,AD-40012.1,AD-40006.1,AD-38146.1,AD-40018.1 |
| AAGAUGUACCUA | **2504** | UAGGUACAUCUU | **2505** | AD-40030.1,AD-40000.1,AD-40024.1,AD-40012.1,AD-40006.1,AD-38146.1,AD-40018.1,AD-38152.1 |
| AGAUGUACCUAU | **2506** | AUAGGUACAUCU | **2507** | AD-40030.1,AD-40024.1,AD-40012.1,AD-40006.1,AD-38158.1,AD-38146.1,AD-40018.1,AD-38152.1 |
| GAUGUACCUAUG | **2508** | CAUAGGUACAUC | **2509** | AD-40030.1,AD-40024.1,AD-40012.1,AD-38164.1,AD-38158.1,AD-38146.1,AD-40018.1,AD-38152.1 |
| AUGUACCUAUGG | **2510** | CCAUAGGUACAU | **2511** | AD-40030.1,AD-40024.1,AD-38164.1,AD-38170.1,AD-38158.1,AD-38146.1,AD-40018.1,AD-38152.1 |
| UGUACCUAUGGU | **2512** | ACCAUAGGUACA | **2513** | AD-40030.1,AD-40024.1,AD-38164.1,AD-38129.1,AD-38170.1,AD-38158.1,AD-38146.1,AD-38152.1 |
| GUACCUAUGGUC | **2514** | GACCAUAGGUAC | **2515** | AD-40030.1,AD-38164.1,AD-38129.1,AD-38170.1,AD-38158.1,AD-38146.1,AD-38152.1,AD-38135.1 |
| UACCUAUGGUCC | **2516** | GGACCAUAGGUA | **2517** | AD-38164.1,AD-38129.1,AD-38170.1,AD-38158.1,AD-38146.1,AD-38141.1,AD-38152.1,AD-38135.1 |
| ACCUAUGGUCCU | **2518** | AGGACCAUAGGU | **2519** | AD-38164.1,AD-38129.1,AD-38170.1,AD-38147.1,AD-38158.1,AD-38141.1,AD-38152.1,AD-38135.1 |
| CCUAUGGUCCUA | **2520** | UAGGACCAUAGG | **2521** | AD-38164.1,AD-38129.1,AD-38153.1,AD-38170.1,AD-38147.1,AD-38158.1,AD-38141.1,AD-38135.1 |
| CUAUGGUCCUAG | **2522** | CUAGGACCAUAG | **2523** | AD-38164.1,AD-38159.1,AD-38129.1,AD-38153.1,AD-38170.1,AD-38147.1,AD-38141.1,AD-38135.1 |
| UAUGGUCCUAGU | **2524** | ACUAGGACCAUA | **2525** | AD-38165.1,AD-38159.1,AD-38129.1,AD-38153.1,AD-38170.1,AD-38147.1,AD-38141.1,AD-38135.1 |
| AUGGUCCUAGUA | **2526** | UACUAGGACCAU | **2527** | AD-38165.1,AD-38171.1,AD-38159.1,AD-38129.1,AD-38153.1,AD-38147.1,AD-38141.1,AD-38135.1 |
| UGGUCCUAGUAG | **2528** | CUACUAGGACCA | **2529** | AD-38165.1,AD-38171.1,AD-38159.1,AD-38153.1,AD-38147.1,AD-38141.1,AD-38130.1,AD-38135.1 |
| GGUCCUAGUAGG | **2530** | CCUACUAGGACC | **2531** | AD-38165.1,AD-38171.1,AD-38159.1,AD-38136.1,AD-38153.1,AD-38147.1,AD-38141.1,AD-38130.1 |

(continued)

| Sense overlap | SEQ ID NO: | Anti-sense overlap | SEQ ID NO: | Overlapping RNAi agents to KRAS |
|---|---|---|---|---|
| GUCCUAGUAGGA | **2532** | UCCUACUAGGAC | **2533** | AD-38165.1,AD-38171.1,AD-38159.1,AD-38136.1,AD-38153.1,AD-38147.1,AD-38142.1,AD-38130.1 |
| UCCUAGUAGGAA | **2534** | UUCCUACUAGGA | **2535** | AD-38165.1,AD-38171.1,AD-38159.1,AD-38136.1,AD-38148.1,AD-38153.1,AD-38142.1,AD-38130.1 |
| CCUAGUAGGAAA | **2536** | UUUCCUACUAGG | **2537** | AD-38165.1,AD-38171.1,AD-38154.1,AD-38159.1,AD-38136.1,AD-38148.1,AD-38142.1,AD-38130.1 |
| CUAGUAGGAAAU | **2538** | AUUUCCUACUAG | **2539** | AD-38165.1,AD-38171.1,AD-38154.1,AD-38136.1,AD-38148.1,AD-38160.1,AD-38142.1,AD-38130.1 |
| UAGUAGGAAAUA | **2540** | UAUUUCCUACUA | **2541** | AD-38171.1,AD-38154.1,AD-38136.1,AD-38148.1,AD-38166.1,AD-38160.1,AD-38142.1,AD-38130.1 |
| AGUAGGAAAUAA | **2542** | UUAUUUCCUACU | **2543** | AD-38154.1,AD-38136.1,AD-38148.1,AD-38172.1,AD-38166.1,AD-38160.1,AD-38142.1,AD-38130.1 |
| GUAGGAAAUAAA | **2544** | UUUAUUUCCUAC | **2545** | AD-40036.1,AD-38154.1,AD-38136.1,AD-38148.1,AD-38172.1,AD-38166.1,AD-38160.1,AD-38142.1 |
| UAGGAAAUAAAU | **2546** | AUUUAUUUCCUA | **2547** | AD-39995.1,AD-40036.1,AD-38154.1,AD-38148.1,AD-38172.1,AD-38166.1,AD-38160.1,AD-38142.1 |
| AGGAAAUAAAUG | **2548** | CAUUUAUUUCCU | **2549** | AD-39995.1,AD-40036.1,AD-38154.1,AD-40001.1,AD-38148.1,AD-38172.1,AD-38166.1,AD-38160.1 |
| GGAAAUAAAUGU | **2550** | ACAUUUAUUUCC | **2551** | AD-39995.1,AD-40036.1,AD-38154.1,AD-40001.1,AD-38172.1,AD-38166.1,AD-38160.1,AD-40007.1 |
| GAAAUAAAUGUG | **2552** | CACAUUUAUUUC | **2553** | AD-39995.1,AD-40036.1,AD-40013.1,AD-40001.1,AD-38172.1,AD-38166.1,AD-38160.1,AD-40007.1 |
| AAAUAAAUGUGA | **2554** | UCACAUUUAUUU | **2555** | AD-39995.1,AD-40036.1,AD-40013.1,AD-40001.1,AD-38172.1,AD-38166.1,AD-40007.1,AD-40019.1 |
| AAUAAAUGUGAU | **2556** | AUCACAUUUAUU | **2557** | AD-39995.1,AD-40036.1,AD-40013.1,AD-40001.1,AD-40025.1,AD-38172.1,AD-40007.1,AD-40019.1 |
| AUAAAUGUGAUU | **2558** | AAUCACAUUUAU | **2559** | AD-39995.1,AD-40036.1,AD-40013.1,AD-40001.1,AD-40025.1,AD-40031.1,AD-40007.1,AD-40019.1 |
| UAAAUGUGAUUU | **2560** | AAAUCACAUUUA | **2561** | AD-39995.1,AD-40013.1,AD-40037.1,AD-40001.1,AD-40025.1,AD-40031.1,AD-40007.1,AD-40019.1 |

(continued)

| Sense overlap | SEQ ID NO: | Anti-sense overlap | SEQ ID NO: | Overlapping RNAi agents to KRAS |
|---|---|---|---|---|
| AAAUGUGAUUUG | **2562** | CAAAUCACAUUU | **2563** | AD-40013.1,AD-40037.1,AD-40001.1,AD-40025.1,AD-40031.1,AD-40007.1,AD-39996.1,AD-40019.1 |
| AAUGUGAUUUGC | **2564** | GCAAAUCACAUU | **2565** | AD-40013.1,AD-40037.1,AD-40025.1,AD-40031.1,AD-40002.1,AD-40007.1,AD-39996.1,AD-40019.1 |
| AUGUGAUUUGCC | **2566** | GGCAAAUCACAU | **2567** | AD-40008.1,AD-40013.1,AD-40037.1,AD-40025.1,AD-40031.1,AD-40002.1,AD-39996.1,AD-40019.1 |
| UGUGAUUUGCCU | **2568** | AGGCAAAUCACA | **2569** | AD-40008.1,AD-40037.1,AD-40025.1,AD-40031.1,AD-40002.1,AD-40014.1,AD-39996.1,AD-40019.1 |
| GUGAUUUGCCUU | **2570** | AAGGCAAAUCAC | **2571** | AD-40008.1,AD-40037.1,AD-40020.1,AD-40025.1,AD-40031.1,AD-40002.1,AD-40014.1,AD-39996.1 |
| UGAUUUGCCUUC | **2572** | GAAGGCAAAUCA | **2573** | AD-40026.1,AD-40008.1,AD-40037.1,AD-40020.1,AD-40031.1,AD-40002.1,AD-40014.1,AD-39996.1 |
| GAUUUGCCUUCU | **2574** | AGAAGGCAAAUC | **2575** | AD-40026.1,AD-40008.1,AD-40032.1,AD-40037.1,AD-40020.1,AD-40002.1,AD-40014.1,AD-39996.1 |
| AUUUGCCUUCUA | **2576** | UAGAAGGCAAAU | **2577** | AD-40038.1,AD-40026.1,AD-40008.1,AD-40032.1,AD-40020.1,AD-40002.1,AD-40014.1,AD-39996.1 |
| UUUGCCUUCUAG | **2578** | CUAGAAGGCAAA | **2579** | AD-40038.1,AD-40026.1,AD-40008.1,AD-40032.1,AD-40020.1,AD-40002.1,AD-39997.1,AD-40014.1 |
| UUGCCUUCUAGA | **2580** | UCUAGAAGGCAA | **2581** | AD-40038.1,AD-40026.1,AD-40008.1,AD-40032.1,AD-40020.1,AD-39997.1,AD-40014.1 |
| UGCCUUCUAGAA | **2582** | UUCUAGAAGGCA | **2583** | AD-40038.1,AD-40026.1,AD-40032.1,AD-40020.1,AD-40009.1,AD-39997.1,AD-40014.1 |
| GCCUUCUAGAAC | **2584** | GUUCUAGAAGGC | **2585** | AD-40038.1,AD-40026.1,AD-40015.1,AD-40032.1,AD-40020.1,AD-40009.1,AD-39997.1 |
| CCUUCUAGAACA | **2586** | UGUUCUAGAAGG | **2587** | AD-40038.1,AD-40026.1,AD-40015.1,AD-40021.1,AD-40032.1,AD-40009.1,AD-39997.1 |
| CUUCUAGAACAG | **2588** | CUGUUCUAGAAG | **2589** | AD-40038.1,AD-40027.1,AD-40015.1,AD-40021.1,AD-40032.1,AD-40009.1,AD-39997.1 |
| UUCUAGAACAGU | **2590** | ACUGUUCUAGAA | **2591** | AD-40038.1,AD-40027.1,AD-40015.1,AD-40021.1,AD-40009.1,AD-40033.1,AD-39997.1 |
| UCUAGAACAGUA | **2592** | UACUGUUCUAGA | **2593** | AD-40027.1,AD-40015.1,AD-40021.1,AD-40039.1,AD-40009.1,AD-40033.1,AD-39997.1 |
| CUAGAACAGUAG | **2594** | CUACUGUUCUAG | **2595** | AD-40027.1,AD-40015.1,AD-40021.1,AD-40039.1,AD-40009.1,AD-40033.1,AD-40045.1 |

(continued)

| Sense overlap | SEQ ID NO: | Anti-sense overlap | SEQ ID NO: | Overlapping RNAi agents to KRAS |
|---|---|---|---|---|
| UAGAACAGUAGA | **2596** | UCUACUGUUCUA | **2597** | AD-40027.1,AD-40015.1,AD-40021.1,AD-40051.1,AD-40039.1,AD-40009.1,AD-40033.1,AD-40045.1 |
| AGAACAGUAGAC | **2598** | GUCUACUGUUCU | **2599** | AD-40027.1,AD-40057.1,AD-40015.1,AD-40021.1,AD-40051.1,AD-40039.1,AD-40033.1,AD-40045.1 |
| GAACAGUAGACA | **2600** | UGUCUACUGUUC | **2601** | AD-40027.1,AD-40057.1,AD-40021.1,AD-40051.1,AD-40039.1,AD-40063.1,AD-40033.1,AD-40045.1 |
| AACAGUAGACAC | **2602** | GUGUCUACUGUU | **2603** | AD-40027.1,AD-40057.1,AD-40051.1,AD-40039.1,AD-40063.1,AD-40033.1,AD-40045.1 , AD-40069.1 |
| ACAGUAGACACA | **2604** | UGUGUCUACUGU | **2605** | AD-40057.1,AD-40051.1,AD-40075.1,AD-40039.1,AD-40063.1,AD-40033.1,AD-40045.1 , AD-40069.1 |
| CAGUAGACACAA | **2606** | UUGUGUCUACUG | **2607** | AD-40057.1,AD-40051.1,AD-40075.1,AD-40039.1,AD-40081.1,AD-40063.1,AD-40045.1 , AD-40069.1 |
| AGUAGACACAAA | **2608** | UUUGUGUCUACU | **2609** | AD-40057.1,AD-40051.1,AD-40075.1,AD-40040.1,AD-40081.1,AD-40063.1,AD-40045.1,AD-40069.1 |
| GUAGACACAAAA | **2610** | UUUUGUGUCUAC | **2611** | AD-40057.1,AD-40051.1,AD-40075.1,AD-40046.1,AD-40040.1,AD-40081.1,AD-40063.1,AD-40069.1 |
| UAGACACAAAAC | **2612** | GUUUUGUGUCUA | **2613** | AD-40057.1,AD-40075.1,AD-40052.1,AD-40046.1,AD-40040.1,AD-40081.1,AD-40063.1,AD-40069.1 |
| AGACACAAAACA | **2614** | UGUUUUGUGUCU | **2615** | AD-40058.1,AD-40075.1,AD-40052.1,AD-40046.1,AD-40040.1,AD-40081.1,AD-40063.1,AD-40069.1 |
| GACACAAAACAG | **2616** | CUGUUUUGUGUC | **2617** | AD-40058.1,AD-40075.1,AD-40052.1,AD-40046.1,AD-40040.1,AD-40081.1,AD-40064.1,AD-40069.1 |
| ACACAAAACAGG | **2618** | CCUGUUUUGUGU | **2619** | AD-40070.1,AD-40058.1,AD-40075.1,AD-40052.1,AD-40046.1,AD-40040.1,AD-40081.1,AD-40064.1 |
| CACAAAACAGGC | **2620** | GCCUGUUUUGUG | **2621** | AD-40070.1,AD-40058.1,AD-40052.1,AD-40046.1,AD-40076.1,AD-40040.1,AD-40081.1,AD-40064.1 |
| ACAAAACAGGCU | **2622** | AGCCUGUUUUGU | **2623** | AD-40070.1,AD-40058.1,AD-40052.1,AD-40046.1,AD-40076.1,AD-40040.1,AD-40082.1,AD-40064.1 |
| CAAAACAGGCUC | **2624** | GAGCCUGUUUUG | **2625** | AD-40070.1,AD-40058.1,AD-40052.1,AD-40046.1,AD-40076.1,AD-40041.1,AD-40082.1,AD-40064.1 |

(continued)

| Sense overlap | SEQ ID NO: | Anti-sense overlap | SEQ ID NO: | Overlapping RNAi agents to KRAS |
|---|---|---|---|---|
| AAAACAGGCUCA | **2626** | UGAGCCUGUUUU | **2627** | AD-40070.1,AD-40058.1,AD-40052.1,AD-40076.1,AD-40041.1,AD-40082.1,AD-40064.1,AD-40047.1 |
| AAACAGGCUCAG | **2628** | CUGAGCCUGUUU | **2629** | AD-40070.1,AD-40058.1,AD-40076.1,AD-40041.1,AD-40082.1,AD-40053.1,AD-40064.1,AD-40047.1 |
| AACAGGCUCAGG | **2630** | CCUGAGCCUGUU | **2631** | AD-40059.1,AD-40070.1,AD-40076.1,AD-40041.1,AD-40082.1,AD-40053.1,AD-40064.1,AD-40047.1 |
| ACAGGCUCAGGA | **2632** | UCCUGAGCCUGU | **2633** | AD-40059.1,AD-40070.1,AD-40065.1,AD-40076.1,AD-40041.1,AD-40082.1,AD-40053.1,AD-40047.1 |
| CAGGCUCAGGAC | **2634** | GUCCUGAGCCUG | **2635** | AD-40059.1,AD-40065.1,AD-40076.1,AD-40041.1,AD-40082.1,AD-40071.1,AD-40053.1,AD-40047.1 |
| AGGCUCAGGACU | **2636** | AGUCCUGAGCCU | **2637** | AD-40059.1,AD-40065.1,AD-40041.1,AD-40082.1,AD-40077.1,AD-40071.1,AD-40053.1,AD-40047.1 |
| GGCUCAGGACUU | **2638** | AAGUCCUGAGCC | **2639** | AD-40059.1,AD-40065.1,AD-40041.1,AD-40077.1,AD-40071.1,AD-40053.1,AD-40083.1,AD-40047.1 |
| GCUCAGGACUUA | **2640** | UAAGUCCUGAGC | **2641** | AD-40059.1,AD-40065.1,AD-40042.1,AD-40077.1,AD-40071.1,AD-40053.1,AD-40083.1,AD-40047.1 |
| CUCAGGACUUAG | **2642** | CUAAGUCCUGAG | **2643** | AD-40059.1,AD-40065.1,AD-40042.1,AD-40048.1,AD-40077.1,AD-40071.1,AD-40053.1,AD-40083.1 |
| UCAGGACUUAGC | **2644** | GCUAAGUCCUGA | **2645** | AD-40059.1,AD-40065.1,AD-40054.1,AD-40042.1,AD-40048.1,AD-40077.1,AD-40071.1,AD-40083.1 |
| CAGGACUUAGCA | **2646** | UGCUAAGUCCUG | **2647** | AD-40065.1,AD-40054.1,AD-40060.1,AD-40042.1,AD-40048.1,AD-40077.1,AD-40071.1,AD-40083.1 |
| AGGACUUAGCAA | **2648** | UUGCUAAGUCCU | **2649** | AD-40054.1,AD-40060.1,AD-40042.1,AD-40066.1,AD-40048.1,AD-40077.1,AD-40071.1,AD-40083.1 |
| GGACUUAGCAAG | **2650** | CUUGCUAAGUCC | **2651** | AD-40072.1,AD-40054.1,AD-40060.1,AD-40042.1,AD-40066.1,AD-40048.1,AD-40077.1,AD-40083.1 |
| GACUUAGCAAGA | **2652** | UCUUGCUAAGUC | **2653** | AD-40072.1,AD-40054.1,AD-40060.1,AD-40042.1,AD-40066.1,AD-40048.1,AD-40078.1,AD-40083.1 |
| ACUUAGCAAGAA | **2654** | UUCUUGCUAAGU | **2655** | AD-40072.1,AD-40054.1,AD-40060.1,AD-40042.1,AD-40066.1,AD-40048.1,AD-40084.1,AD-40078.1 |

(continued)

| Sense overlap | SEQ ID NO: | Anti-sense overlap | SEQ ID NO: | Overlapping RNAi agents to KRAS |
|---|---|---|---|---|
| CUUAGCAAGAAG | **2656** | CUUCUUGCUAAG | **2657** | AD-40072.1,AD-40054.1,AD-40060.1,AD-40066.1,AD-40043.1,AD-40048.1,AD-40084.1,AD-40078.1 |
| UUAGCAAGAAGU | **2658** | ACUUCUUGCUAA | **2659** | AD-40072.1,AD-40054.1,AD-40060.1,AD-40066.1,AD-40043.1,AD-40084.1,AD-40049.1,AD-40078.1 |
| UAGCAAGAAGUU | **2660** | AACUUCUUGCUA | **2661** | AD-40072.1,AD-40060.1,AD-40066.1,AD-40055.1,AD-40043.1,AD-40084.1,AD-40049.1,AD-40078.1 |
| AGCAAGAAGUUA | **2662** | UAACUUCUUGCU | **2663** | AD-40072.1,AD-40066.1,AD-40055.1,AD-40043.1,AD-40061.1,AD-40084.1,AD-40049.1,AD-40078.1 |
| GCAAGAAGUUAU | **2664** | AUAACUUCUUGC | **2665** | AD-40072.1,AD-40055.1,AD-40067.1,AD-40043.1,AD-40061.1,AD-40084.1,AD-40049.1,AD-40078.1 |
| CAAGAAGUUAUG | **2666** | CAUAACUUCUUG | **2667** | AD-40055.1,AD-40067.1,AD-40043.1,AD-40061.1,AD-40084.1,AD-40073.1,AD-40049.1,AD-40078.1 |
| AAGAAGUUAUGG | **2668** | CCAUAACUUCUU | **2669** | AD-40055.1,AD-40067.1,AD-40043.1,AD-40061.1,AD-40084.1,AD-40073.1,AD-40079.1,AD-40049.1 |
| AGAAGUUAUGGA | **2670** | UCCAUAACUUCU | **2671** | AD-40085.1,AD-40055.1,AD-40067.1,AD-40043.1,AD-40061.1,AD-40073.1,AD-40079.1,AD-40049.1 |
| GAAGUUAUGGAA | **2672** | UUCCAUAACUUC | **2673** | AD-40085.1,AD-40044.1,AD-40055.1,AD-40067.1,AD-40061.1,AD-40073.1,AD-40079.1,AD-40049.1 |
| AAGUUAUGGAAU | **2674** | AUUCCAUAACUU | **2675** | AD-40085.1,AD-40044.1,AD-40055.1,AD-40067.1,AD-40061.1,AD-40050.1,AD-40073.1,AD-40079.1 |
| AGUUAUGGAAUU | **2676** | AAUUCCAUAACU | **2677** | AD-40085.1,AD-40044.1,AD-40067.1,AD-40056.1,AD-40061.1,AD-40050.1,AD-40073.1,AD-40079.1 |
| GUUAUGGAAUUC | **2678** | GAAUUCCAUAAC | **2679** | AD-40062.1,AD-40085.1,AD-40044.1,AD-40067.1,AD-40056.1,AD-40050.1,AD-40073.1,AD-40079.1 |
| UUAUGGAAUUCC | **2680** | GGAAUUCCAUAA | **2681** | AD-40068.1,AD-40062.1,AD-40085.1,AD-40044.1,AD-40056.1,AD-40050.1,AD-40073.1,AD-40079.1 |
| UAUGGAAUUCCU | **2682** | AGGAAUUCCAUA | **2683** | AD-40074.1,AD-40068.1,AD-40062.1,AD-40085.1,AD-40044.1,AD-40056.1,AD-40050.1,AD-40079.1 |
| AUGGAAUUCCUU | **2684** | AAGGAAUUCCAU | **2685** | AD-40080.1,AD-40074.1,AD-40068.1,AD-40062.1,AD-40085.1,AD-40044.1,AD-40056.1,AD-40050.1 |

(continued)

| Sense overlap | SEQ ID NO: | Anti-sense overlap | SEQ ID NO: | Overlapping RNAi agents to KRAS |
|---|---|---|---|---|
| UGGAAUUCCUUU | **2686** | AAAGGAAUUCCA | **2687** | AD-40080.1,AD-40074.1,AD-40068.1,AD-40062.1,AD-40044.1,AD-40056.1,AD-40050.1 |
| GGAAUUCCUUUU | **2688** | AAAAGGAAUUCC | **2689** | AD-40080.1,AD-40074.1,AD-40068.1,AD-40062.1,AD-40056.1,AD-40050.1,AD-38131.1 |
| GAAUUCCUUUUA | **2690** | UAAAAGGAAUUC | **2691** | AD-40080.1,AD-40074.1,AD-40068.1,AD-40062.1,AD-38137.1,AD-40056.1,AD-38131.1 |
| AAUUCCUUUUAU | **2692** | AUAAAAGGAAUU | **2693** | AD-40080.1,AD-38143.1,AD-40074.1,AD-40068.1,AD-40062.1,AD-38137.1,AD-38131.1 |
| AUUCCUUUUAUU | **2694** | AAUAAAAGGAAU | **2695** | AD-40080.1,AD-38149.1,AD-38143.1,AD-40074.1,AD-40068.1,AD-38137.1,AD-38131.1 |
| UUCCUUUUAUUG | **2696** | CAAUAAAAGGAA | **2697** | AD-40080.1,AD-38149.1,AD-38143.1,AD-40074.1,AD-38137.1,AD-38155.1,AD-38131.1 |
| UCCUUUUAUUGA | **2698** | UCAAUAAAAGGA | **2699** | AD-40080.1,AD-38149.1,AD-38143.1,AD-38137.1,AD-38161.1,AD-38155.1,AD-38131.1 |
| CCUUUUAUUGAA | **2700** | UUCAAUAAAAGG | **2701** | AD-38149.1,AD-38143.1,AD-38137.1,AD-38161.1,AD-38167.1,AD-38155.1,AD-38131.1 |
| CUUUUAUUGAAA | **2702** | UUUCAAUAAAAG | **2703** | AD-38149.1,AD-38143.1,AD-38137.1,AD-38161.1,AD-38173.1,AD-38167.1,AD-38155.1,AD-38131.1 |
| UUUUAUUGAAAC | **2704** | GUUUCAAUAAAA | **2705** | AD-38149.1,AD-38143.1,AD-38132.1,AD-38137.1,AD-38161.1,AD-38173.1,AD-38167.1,AD-38155.1 |
| UUUAUUGAAACA | **2706** | UGUUUCAAUAAA | **2707** | AD-38149.1,AD-38143.1,AD-38132.1,AD-38161.1,AD-38173.1,AD-38138.1,AD-38167.1,AD-38155.1 |
| UUAUUGAAACAU | **2708** | AUGUUUCAAUAA | **2709** | AD-38149.1,AD-38132.1,AD-38161.1,AD-38173.1,AD-38138.1,AD-38167.1,AD-38155.1,AD-38144.1 |
| UAUUGAAACAUC | **2710** | GAUGUUUCAAUA | **2711** | AD-38132.1,AD-38161.1,AD-38173.1,AD-38138.1,AD-38167.1,AD-38155.1,AD-38150.1,AD-38144.1 |
| AUUGAAACAUCA | **2712** | UGAUGUUUCAAU | **2713** | AD-38132.1,AD-38156.1,AD-38161.1,AD-38173.1,AD-38138.1,AD-38167.1,AD-38150.1,AD-38144.1 |
| UUGAAACAUCAG | **2714** | CUGAUGUUUCAA | **2715** | AD-38132.1,AD-38162.1,AD-38156.1,AD-38173.1,AD-38138.1,AD-38167.1,AD-38150.1,AD-38144.1 |
| UGAAACAUCAGC | **2716** | GCUGAUGUUUCA | **2717** | AD-38132.1,AD-38162.1,AD-38156.1,AD-38168.1,AD-38173.1,AD-38138.1,AD-38150.1,AD-38144.1 |
| GAAACAUCAGCA | **2718** | UGCUGAUGUUUC | **2719** | AD-38174.1,AD-38132.1,AD-38162.1,AD-38156.1,AD-38168.1,AD-38138.1,AD-38150.1,AD-38144.1 |

(continued)

| Sense overlap | SEQ ID NO: | Anti-sense overlap | SEQ ID NO: | Overlapping RNAi agents to KRAS |
|---|---|---|---|---|
| AAACAUCAGCAA | **2720** | UUGCUGAUGUUU | **2721** | AD-38174.1,AD-38162.1,AD-35667.1,AD-38156.1,AD-38168.1,AD-38138.1,AD-38150.1,AD-38144.1 |
| AACAUCAGCAAA | **2722** | UUUGCUGAUGUU | **2723** | AD-38174.1,AD-35673.1,AD-38162.1,AD-35667.1,AD-38156.1,AD-38168.1,AD-38150.1,AD-38144.1 |
| ACAUCAGCAAAG | **2724** | CUUUGCUGAUGU | **2725** | AD-38174.1,AD-35673.1,AD-38162.1,AD-35667.1,AD-35679.1,AD-38156.1,AD-38168.1,AD-38150.1 |
| CAUCAGCAAAGA | **2726** | UCUUUGCUGAUG | **2727** | AD-35685.1,AD-38174.1,AD-35673.1,AD-38162.1,AD-35667.1,AD-35679.1,AD-38156.1,AD-38168.1 |
| AUCAGCAAAGAC | **2728** | GUCUUUGCUGAU | **2729** | AD-35685.1,AD-38174.1,AD-35673.1,AD-38162.1,AD-35667.1,AD-35679.1,AD-38168.1,AD-35691.1 |
| UCAGCAAAGACA | **2730** | UGUCUUUGCUGA | **2731** | AD-35685.1,AD-38174.1,AD-35673.1,AD-35667.1,AD-35679.1,AD-38168.1,AD-35691.1 |
| CAGCAAAGACAA | **2732** | UUGUCUUUGCUG | **2733** | AD-35685.1,AD-38174.1,AD-35673.1,AD-35667.1,AD-35679.1,AD-35691.1 |
| AGCAAAGACAAG | **2734** | CUUGUCUUUGCU | **2735** | AD-35685.1,AD-35673.1,AD-35667.1,AD-35679.1,AD-35691.1 |
| GCAAAGACAAGA | **2736** | UCUUGUCUUUGC | **2737** | AD-35685.1,AD-35673.1,AD-35679.1,AD-35691.1 |
| CAAAGACAAGAC | **2738** | GUCUUGUCUUUG | **2739** | AD-35685.1,AD-35679.1,AD-35691.1 |
| AAAGACAAGACA | **2740** | UGUCUUGUCUUU | **2741** | AD-35685.1,AD-35691.1 |

The position ("Pos") in NM_000336.2 is indicated. 12 examplary nt of the overlap in the sense and anti-sense strand are presented; in many cases, the overlap is actually longer.

**EXAMPLE 3**

**EFFICACY OF KRAS RNAi AGENTS**

KRAS RNAi agents were tested for efficacy, and the results are shown in Tables 5 and 6, below.

**In vitro screening:**

Cell culture and transfections:

RKO cells (ATCC, Manassas, VA) are grown to near confluence at 37°C in an atmosphere of 5% $CO_2$ in McCoy's Medium (ATCC) supplemented with 10% FBS, streptomycin, and glutamine (ATCC) before being released from the plate by trypsinization. Transfection is carried out by adding 14.8μl of Opti-MEM plus 0.2μl of Lipofectamine RNAiMax per well (Invitrogen, Carlsbad, CA, cat # 13778-150) to 5μl of siRNA duplexes per well into a 96-well plate and incubated at room temperature for 15 minutes. 80μl of complete growth media without antibiotic containing ~2 x$10^4$ RKO cells are then added to the siRNA mixture. Cells are incubated for either 24 hours prior to RNA purification. Single dose experiments are performed at 10nM and in some cases 0.1nM final duplex concentration and dose response experiments are done at 10, 1, 0.5, 0.1,0.05, 0.01, 0.005, 0.001, 0.0005, 0.0001, 0.00005, 0.00001 nM final duplex concentration.

Total RNA isolation using DYNABEADS mRNA Isolation Kit (Invitrogen, part #: 610-12):

Cells are harvested and lysed in 150μl of Lysis/Binding Buffer then mixed for 5 minute at 850rpm using an Eppendorf Thermomixer (the mixing speed is the same throughout the process). Ten microliters of magnetic beads and 80μl Lysis/Binding Buffer mixture are added to a round bottom plate and mixed for 1 minute. Magnetic beads are captured using magnetic stand and the supernatant is removed without disturbing the beads. After removing supernatant, the lysed cells are added to the remaining beads and mixed for 5 minutes. After removing supernatant, magnetic beads are washed 2 times with 150μl Wash Buffer A and mixed for 1 minute. Beads are capture again and supernatant removed. Beads are then washed with 150μl Wash Buffer B, captured and supernatant is removed. Beads are next washed with 150μl Elution Buffer, captured and supernatant removed. Beads are allowed to dry for 2 minutes. After drying, 50μl of Elution Buffer is added and mixed for 5 minutes at 70°C. Beads are captured on magnet for 5 minutes. 40μl of supernatant is removed and added to another 96 well plate.

cDNA synthesis using ABI High capacity cDNA reverse transcription kit (Applied Biosystems, Foster City, CA, Cat #4368813):

A master mix of 2μl 10X Buffer, 0.8μl 25X dNTPs, 2μl Random primers, 1μl Reverse Transcriptase, 1μl RNase inhibitor and 3.2μl of H2O per reaction are added into 10μl total RNA. cDNA is generated using a Bio-Rad C-1000 or S-1000 thermal cycler (Hercules, CA) through the following steps: 25°C 10 min, 37°C 120 min, 85°C 5 sec, 4°C hold.

Real time PCR:

2μl of cDNA are added to a master mix containing 1μl 18S TaqMan Probe (Applied Biosystems Cat # 4319413E), 1μl KRAS TaqMan probe (Applied Biosystems cat # HS00180035_m1, HS00932330_m1, or HS00610483) and 10μl TaqMan Universal PCR Master Mix (Applied Biosystems Cat #4324018) per well in a MicroAmp Optical 96 well plate (Applied Biosystems cat # 4326659). Real time PCR is done in an ABI 7900HT Real Time PCR system (Applied Biosystems) using the ΔΔCt(RQ) assay. Each duplex is tested in two independent transfections and each transfection is assayed in duplicate, unless otherwise noted in the summary tables.

Real time data are analyzed using the ΔΔCt method and normalized to assays performed with cells transfected with 10nM AD-1955 to calculate relative fold change.

**Table 5. KRAS RNAi agents, 0.1 and 10nM KD at 24 hrs**

| SD = standard deviation, AVG = average | | | | | | | | |
|---|---|---|---|---|---|---|---|---|
| | | | | | | | | |
| | **10nM KRAS** | | **.1nM KRAS** | | **10nM** | **.1nM** | **10nM** | **.1nM** |
| **Duplex ID** | **bio1** | **bio2** | **bio1** | **bio2** | **SD** | **SD** | **AVG** | **AVG** |
| AD-35613.4 | 0.332 | 0.542 | 1.814 | 1.365 | 0.1484 | 0.3177 | 0.437 | 1.589 |
| AD-35572.4 | 0.403 | 0.666 | 1.850 | 1.571 | 0.1859 | 0.1975 | 0.535 | 1.711 |
| AD-35578.4 | 0.194 | 0.905 | 0.622 | 0.485 | 0.5030 | 0.0965 | 0.550 | 0.554 |
| AD-35584.4 | 0.171 | 0.239 | 0.953 | 0.654 | 0.0480 | 0.2115 | 0.205 | 0.803 |
| AD-35590.4 | 0.167 | 0.296 | 0.988 | 0.832 | 0.0919 | 0.1105 | 0.231 | 0.910 |
| AD-35596.4 | 0.782 | 0.837 | 1.540 | 1.263 | 0.0392 | 0.1962 | 0.809 | 1.402 |
| AD-35602.4 | 1.013 | 0.915 | 1.264 | 0.934 | 0.0694 | 0.2334 | 0.964 | 1.099 |
| AD-35608.4 | 0.818 | 0.631 | 1.120 | 1.093 | 0.1321 | 0.0191 | 0.725 | 1.106 |
| AD-35614.4 | 0.898 | 1.025 | 1.929 | 1.195 | 0.0898 | 0.5194 | 0.961 | 1.562 |
| AD-35573.4 | 0.920 | 0.809 | 1.412 | 0.957 | 0.0785 | 0.3219 | 0.865 | 1.185 |
| AD-35579.4 | 0.794 | 0.946 | 1.694 | 1.244 | 0.1072 | 0.3189 | 0.870 | 1.469 |
| AD-35585.4 | 0.861 | 0.738 | 3.016 | 2.552 | 0.0870 | 0.3281 | 0.800 | 2.784 |

(continued)

| | 10nM KRAS | | .1nM KRAS | | 10nM | .1nM | 10nM | .1nM |
|---|---|---|---|---|---|---|---|---|
| Duplex ID | bio1 | bio2 | bio1 | bio2 | SD | SD | AVG | AVG |
| AD-35591.4 | 0.458 | 0.325 | 1.238 | 1.073 | 0.0938 | 0.1162 | 0.392 | 1.156 |
| AD-35597.4 | 0.948 | 0.897 | 1.523 | 0.864 | 0.0358 | 0.4657 | 0.922 | 1.194 |
| AD-35603.4 | 0.401 | 0.405 | 0.817 | 0.618 | 0.0025 | 0.1402 | 0.403 | 0.718 |
| AD-35609.4 | 0.168 | 0.165 | 0.503 | 0.320 | 0.0022 | 0.1295 | 0.167 | 0.411 |
| AD-35615.4 | 0.555 | 0.620 | 1.397 | 0.818 | 0.0460 | 0.4099 | 0.587 | 1.108 |
| AD-35574.4 | 0.762 | 0.650 | 1.009 | 0.643 | 0.0790 | 0.2590 | 0.706 | 0.826 |
| AD-35580.1 | 0.436 | 0.398 | 1.334 | 0.768 | 0.0275 | 0.4003 | 0.417 | 1.051 |
| AD-35586.4 | 0.242 | 0.266 | 0.908 | 0.577 | 0.0175 | 0.2337 | 0.254 | 0.743 |
| AD-35592.4 | 0.212 | 0.166 | 0.600 | 0.504 | 0.0324 | 0.0685 | 0.189 | 0.552 |
| AD-35598.4 | 0.828 | 0.893 | 1.318 | 1.147 | 0.0461 | 0.1205 | 0.861 | 1.232 |
| AD-35604.4 | 0.070 | 0.179 | 0.594 | 0.496 | 0.0770 | 0.0692 | 0.124 | 0.545 |
| AD-35610.4 | 0.415 | 0.633 | 1.068 | 0.887 | 0.1537 | 0.1275 | 0.524 | 0.977 |
| AD-35616.4 | 0.159 | 0.342 | 0.300 | 0.213 | 0.1288 | 0.0610 | 0.251 | 0.256 |
| AD-35575.4 | 0.470 | 0.689 | 0.789 | 0.749 | 0.1547 | 0.0284 | 0.579 | 0.769 |
| AD-35581.4 | 0.137 | 0.288 | 0.220 | 0.196 | 0.1064 | 0.0170 | 0.212 | 0.208 |
| AD-35587.4 | 0.637 | 0.670 | 1.059 | 0.975 | 0.0231 | 0.0591 | 0.653 | 1.017 |
| AD-35593.4 | 0.200 | 0.463 | 0.724 | 0.489 | 0.1859 | 0.1661 | 0.332 | 0.607 |
| AD-35599.4 | 0.685 | 1.281 | 1.273 | 0.952 | 0.4217 | 0.2266 | 0.983 | 1.113 |
| AD-35605.4 | 0.564 | 0.798 | 1.423 | 0.897 | 0.1649 | 0.3718 | 0.681 | 1.160 |
| AD-35611.4 | 0.644 | 0.844 | 1.189 | 0.922 | 0.1418 | 0.1887 | 0.744 | 1.055 |
| AD-35617.4 | 0.219 | 0.213 | 0.594 | 0.625 | 0.0047 | 0.0220 | 0.216 | 0.609 |
| AD-35576.4 | 0.451 | 0.365 | 1.167 | 0.928 | 0.0604 | 0.1693 | 0.408 | 1.047 |
| AD-35588.4 | 0.185 | 0.290 | 0.442 | 0.323 | 0.0736 | 0.0841 | 0.237 | 0.383 |
| AD-35667.1 | 0.497 | 0.623 | 1.030 | 0.857 | 0.0897 | 0.1228 | 0.560 | 0.943 |
| AD-35673.1 | 0.367 | 0.405 | 0.771 | 0.693 | 0.0268 | 0.0547 | 0.386 | 0.732 |
| AD-35679.1 | 0.217 | 0.330 | 0.329 | 0.293 | 0.0798 | 0.0258 | 0.274 | 0.311 |
| AD-35685.1 | 0.310 | 0.255 | 0.345 | 0.339 | 0.0390 | 0.0045 | 0.283 | 0.342 |
| AD-35691.1 | 0.258 | 0.257 | 0.955 | 0.535 | 0.0005 | 0.2968 | 0.258 | 0.745 |
| AD-35557.1 | 0.843 | 0.624 | 1.056 | 0.947 | 0.1547 | 0.0766 | 0.734 | 1.002 |
| AD-35618.1 | 0.447 | 0.425 | 0.611 | 0.439 | 0.0157 | 0.1218 | 0.436 | 0.525 |
| AD-35569.1 | 0.319 | 0.404 | 0.805 | 0.715 | 0.0605 | 0.0636 | 0.362 | 0.760 |
| AD-35534.1 | 0.578 | 0.582 | 0.877 | 0.944 | 0.0028 | 0.0475 | 0.580 | 0.910 |
| AD-35582.1 | 0.518 | 0.974 | 0.982 | 0.977 | 0.3223 | 0.0035 | 0.746 | 0.980 |
| AD-35594.1 | 0.441 | 0.317 | 0.884 | 0.679 | 0.0877 | 0.1451 | 0.379 | 0.782 |
| AD-35600.1 | 0.207 | 0.394 | 0.309 | 0.233 | 0.1322 | 0.0539 | 0.301 | 0.271 |
| AD-35606.1 | 0.285 | 0.177 | 0.384 | 0.346 | 0.0770 | 0.0272 | 0.231 | 0.365 |
| AD-35612.1 | 1.070 | 0.947 | 1.027 | 0.827 | 0.0870 | 0.1415 | 1.009 | 0.927 |

(continued)

| | 10nM KRAS | | .1nM KRAS | | 10nM | .1nM | 10nM | .1nM |
|---|---|---|---|---|---|---|---|---|
| Duplex ID | bio1 | bio2 | bio1 | bio2 | SD | SD | AVG | AVG |
| AD-38127.1 | 0.217 | 0.162 | 0.594 | 0.491 | 0.0388 | 0.0728 | 0.189 | 0.542 |
| AD-38128.1 | 0.157 | 0.146 | 0.521 | 0.366 | 0.0072 | 0.1095 | 0.151 | 0.443 |
| AD-38164.1 | 1.112 | 1.993 | 1.213 | 0.982 | 0.6224 | 0.1633 | 1.553 | 1.097 |
| AD-38165.1 | 0.619 | 0.731 | 1.002 | 0.837 | 0.0790 | 0.1173 | 0.675 | 0.920 |
| AD-38160.1 | 1.159 | 1.129 | 1.300 | 0.920 | 0.0216 | 0.2685 | 1.144 | 1.110 |
| AD-38161.1 | 0.144 | 0.125 | 0.355 | 0.319 | 0.0130 | 0.0256 | 0.134 | 0.337 |
| AD-38133.1 | 0.164 | 0.142 | 0.574 | 0.590 | 0.0154 | 0.0113 | 0.153 | 0.582 |
| AD-38170.1 | 0.147 | 0.191 | 0.734 | 0.487 | 0.0311 | 0.1744 | 0.169 | 0.611 |
| AD-38171.1 | 0.858 | 1.067 | 1.408 | 1.101 | 0.1480 | 0.2168 | 0.962 | 1.254 |
| AD-38166.1 | 0.319 | 0.486 | 0.846 | 0.848 | 0.1184 | 0.0016 | 0.402 | 0.847 |
| AD-38167.1 | 0.119 | 0.182 | 0.740 | 0.546 | 0.0440 | 0.1368 | 0.150 | 0.643 |
| AD-38139.1 | 0.148 | 0.085 | 0.259 | 0.198 | 0.0445 | 0.0433 | 0.116 | 0.229 |
| AD-38134.1 | 0.117 | 0.190 | 0.248 | 0.380 | 0.0514 | 0.0936 | 0.154 | 0.314 |
| AD-38129.1 | 0.776 | 0.860 | 1.038 | 1.089 | 0.0596 | 0.0356 | 0.818 | 1.064 |
| AD-38130.1 | 0.528 | 0.820 | 0.900 | 0.846 | 0.2064 | 0.0386 | 0.674 | 0.873 |
| AD-38172.1 | 0.076 | 0.094 | 0.289 | 0.247 | 0.0127 | 0.0298 | 0.085 | 0.268 |
| AD-38145.1 | 0.150 | 0.128 | 0.440 | 0.371 | 0.0154 | 0.0488 | 0.139 | 0.406 |
| AD-38135.1 | 0.959 | 1.429 | 0.965 | 0.900 | 0.3321 | 0.0459 | 1.194 | 0.932 |
| AD-38131.1 | 0.135 | 0.173 | 0.414 | 0.279 | 0.0267 | 0.0955 | 0.154 | 0.347 |
| AD-38173.1 | 0.609 | 0.988 | 1.132 | 1.373 | 0.2681 | 0.1707 | 0.798 | 1.253 |
| AD-38151.1 | 0.211 | 0.181 | 0.378 | 0.554 | 0.0218 | 0.1246 | 0.196 | 0.466 |
| AD-38140.1 | 0.652 | 0.794 | 1.087 | 1.045 | 0.1004 | 0.0298 | 0.723 | 1.066 |
| AD-38141.1 | 0.450 | 0.512 | 1.002 | 0.840 | 0.0433 | 0.1147 | 0.481 | 0.921 |
| AD-38136.1 | 0.053 | 0.096 | 0.150 | 0.121 | 0.0301 | 0.0199 | 0.074 | 0.136 |
| AD-38137.1 | 0.283 | 0.259 | 0.677 | 0.580 | 0.0169 | 0.0687 | 0.271 | 0.628 |
| AD-38132.1 | 0.256 | 0.333 | 0.669 | 0.726 | 0.0543 | 0.0404 | 0.294 | 0.697 |
| AD-38157.1 | 0.760 | 0.543 | 0.768 | 0.900 | 0.1536 | 0.0937 | 0.651 | 0.834 |
| AD-38146.1 | 0.249 | 0.240 | 0.617 | 0.584 | 0.0065 | 0.0237 | 0.245 | 0.600 |
| AD-38147.1 | 0.271 | 0.361 | 0.858 | 0.909 | 0.0638 | 0.0365 | 0.316 | 0.883 |
| AD-38142.1 | 0.389 | 0.634 | 0.971 | 0.712 | 0.1732 | 0.1831 | 0.512 | 0.841 |
| AD-38143.1 | 0.378 | 0.657 | 0.920 | 0.808 | 0.1966 | 0.0791 | 0.518 | 0.864 |
| AD-38163.1 | 0.088 | 0.083 | 0.143 | 0.172 | 0.0036 | 0.0206 | 0.085 | 0.158 |
| AD-38152.1 | 0.755 | 0.580 | 1.057 | 0.897 | 0.1238 | 0.1134 | 0.667 | 0.977 |
| AD-38153.1 | 0.727 | 0.781 | 0.855 | 1.006 | 0.0380 | 0.1067 | 0.754 | 0.930 |
| AD-38148.1 | 0.553 | 0.522 | 0.784 | 0.831 | 0.0215 | 0.0333 | 0.537 | 0.807 |
| AD-38149.1 | 0.269 | 0.295 | 0.607 | 0.641 | 0.0186 | 0.0242 | 0.282 | 0.624 |
| AD-38138.1 | 0.330 | 0.417 | 0.402 | 0.448 | 0.0615 | 0.0321 | 0.373 | 0.425 |

(continued)

| | 10nM KRAS | | .1nM KRAS | | 10nM | .1nM | 10nM | .1nM |
|---|---|---|---|---|---|---|---|---|
| Duplex ID | bio1 | bio2 | bio1 | bio2 | SD | SD | AVG | AVG |
| AD-38169.1 | 0.380 | 0.248 | 0.626 | 0.540 | 0.0929 | 0.0607 | 0.314 | 0.583 |
| AD-38158.1 | 0.699 | 0.611 | 0.800 | 1.250 | 0.0619 | 0.3184 | 0.655 | 1.025 |
| AD-38159.1 | 0.161 | 0.122 | 0.210 | 0.227 | 0.0274 | 0.0123 | 0.142 | 0.219 |
| AD-38154.1 | 0.212 | 0.172 | 0.628 | 0.507 | 0.0286 | 0.0858 | 0.192 | 0.567 |
| AD-38155.1 | 0.198 | 0.215 | 0.331 | 0.309 | 0.0122 | 0.0153 | 0.207 | 0.320 |
| AD-39683.1 | 0.471 | 0.536 | 1.135 | 0.812 | 0.0461 | 0.2283 | 0.504 | 0.973 |
| AD-39689.1 | 0.630 | 0.627 | 1.148 | 0.979 | 0.0025 | 0.1194 | 0.629 | 1.064 |
| AD-39695.1 | 0.659 | 0.557 | 1.222 | 1.022 | 0.0723 | 0.1414 | 0.608 | 1.122 |
| AD-39701.1 | 1.218 | 1.231 | 0.704 | 0.724 | 0.0092 | 0.0141 | 1.225 | 0.714 |
| AD-39707.1 | 0.384 | 0.160 | 1.387 | 1.087 | 0.1584 | 0.2121 | 0.272 | 1.237 |
| AD-39713.1 | 1.157 | 0.481 | 1.128 | 1.228 | 0.4778 | 0.0707 | 0.819 | 1.178 |
| AD-39719.1 | 0.885 | 0.631 | 1.178 | 1.189 | 0.1791 | 0.0073 | 0.758 | 1.183 |
| AD-39725.1 | 1.105 | 1.074 | 1.203 | 1.751 | 0.0222 | 0.3878 | 1.089 | 1.477 |
| AD-39684.1 | 0.321 | 0.186 | 0.954 | 1.222 | 0.0952 | 0.1895 | 0.253 | 1.088 |
| AD-39690.1 | 0.825 | 0.828 | 0.832 | 0.852 | 0.0019 | 0.0139 | 0.826 | 0.842 |
| AD-39696.1 | 1.071 | 0.996 | 1.087 | 0.883 | 0.0532 | 0.1443 | 1.034 | 0.985 |
| AD-39702.1 | 0.360 | 0.334 | 1.050 | 1.285 | 0.0179 | 0.1660 | 0.347 | 1.167 |
| AD-39708.1 | 0.757 | 0.969 | 1.049 | 1.091 | 0.1494 | 0.0299 | 0.863 | 1.070 |
| AD-39714.1 | 0.953 | 1.056 | 0.779 | 0.639 | 0.0731 | 0.0995 | 1.004 | 0.709 |
| AD-39720.1 | 0.210 | 0.182 | 0.404 | 0.691 | 0.0199 | 0.2030 | 0.196 | 0.548 |
| AD-39726.1 | 0.774 | 0.968 | 0.921 | 1.068 | 0.1372 | 0.1037 | 0.871 | 0.995 |
| AD-39685.1 | 1.022 | 1.358 | 1.053 | 0.997 | 0.2378 | 0.0399 | 1.190 | 1.025 |
| AD-39691.1 | 0.494 | 0.380 | 0.913 | 0.967 | 0.0806 | 0.0379 | 0.437 | 0.940 |
| AD-39697.1 | 0.892 | 1.333 | 0.937 | 1.344 | 0.3125 | 0.2883 | 1.113 | 1.140 |
| AD-39703.1 | 1.071 | 1.141 | 0.983 | 0.864 | 0.0497 | 0.0842 | 1.106 | 0.924 |
| AD-39709.1 | 0.905 | 1.086 | 0.944 | 1.079 | 0.1284 | 0.0953 | 0.995 | 1.011 |
| AD-39715.1 | 0.347 | 0.385 | 1.398 | 1.013 | 0.0265 | 0.2722 | 0.366 | 1.205 |
| AD-39721.1 | 0.911 | 0.781 | 1.128 | 1.119 | 0.0918 | 0.0066 | 0.846 | 1.123 |
| AD-39727.1 | 0.267 | 0.271 | 0.911 | 1.038 | 0.0028 | 0.0894 | 0.269 | 0.975 |
| AD-39686.1 | 0.897 | 0.490 | 0.781 | 0.633 | 0.2877 | 0.1044 | 0.694 | 0.707 |
| AD-39692.1 | 0.936 | 0.879 | 0.814 | 0.802 | 0.0403 | 0.0083 | 0.908 | 0.808 |
| AD-39698.1 | 1.257 | 1.375 | 0.829 | 0.876 | 0.0834 | 0.0333 | 1.316 | 0.853 |
| AD-39704.1 | 1.211 | 1.189 | 0.909 | 0.735 | 0.0155 | 0.1234 | 1.200 | 0.822 |
| AD-39710.1 | 1.389 | 1.136 | 0.881 | 0.862 | 0.1789 | 0.0130 | 1.263 | 0.871 |
| AD-39716.1 | 0.787 | 0.943 | 0.991 | 0.963 | 0.1099 | 0.0199 | 0.865 | 0.977 |
| AD-39722.1 | 1.147 | 1.049 | 0.872 | 0.716 | 0.0688 | 0.1108 | 1.098 | 0.794 |
| AD-39728.1 | 0.846 | 0.974 | 0.891 | 0.928 | 0.0902 | 0.0264 | 0.910 | 0.909 |

(continued)

| | 10nM KRAS | | .1nM KRAS | | 10nM | .1nM | 10nM | .1nM |
|---|---|---|---|---|---|---|---|---|
| Duplex ID | bio1 | bio2 | bio1 | bio2 | SD | SD | AVG | AVG |
| AD-39683.1 | 0.374 | 0.307 | 0.996 | 0.982 | 0.0469 | 0.0098 | 0.341 | 0.989 |
| AD-39687.1 | 1.015 | 0.775 | 1.035 | 1.642 | 0.1695 | 0.4296 | 0.895 | 1.338 |
| AD-39689.1 | 0.340 | 0.328 | 0.769 | 0.762 | 0.0090 | 0.0047 | 0.334 | 0.765 |
| AD-39693.1 | 1.262 | 0.832 | 0.855 | 0.967 | 0.3040 | 0.0791 | 1.047 | 0.911 |
| AD-39699.1 | 1.173 | 1.113 | 1.113 | 1.307 | 0.0423 | 0.1375 | 1.143 | 1.210 |
| AD-39705.1 | 0.622 | 0.582 | 0.730 | 0.807 | 0.0282 | 0.0542 | 0.602 | 0.768 |
| AD-39711.1 | 1.996 | 0.864 | 0.720 | 0.829 | 0.8006 | 0.0770 | 1.430 | 0.774 |
| AD-39717.1 | 0.551 | 0.523 | 1.136 | 1.064 | 0.0197 | 0.0511 | 0.537 | 1.100 |
| AD-39723.1 | 1.179 | 0.695 | 0.877 | 0.873 | 0.3428 | 0.0025 | 0.937 | 0.875 |
| AD-39729.1 | 1.083 | 0.864 | 0.898 | 0.873 | 0.1547 | 0.0178 | 0.973 | 0.885 |
| AD-39688.1 | 0.341 | 0.492 | 0.700 | 0.782 | 0.1064 | 0.0577 | 0.416 | 0.741 |
| AD-39694.1 | 0.230 | 0.327 | 0.923 | 1.087 | 0.0685 | 0.1164 | 0.279 | 1.005 |
| AD-39700.1 | 0.529 | 0.760 | 0.600 | 0.671 | 0.1631 | 0.0497 | 0.645 | 0.635 |
| AD-39706.1 | 0.180 | 0.217 | 0.447 | 0.522 | 0.0263 | 0.0528 | 0.198 | 0.484 |
| AD-39712.1 | 0.330 | 0.394 | 0.798 | 0.777 | 0.0452 | 0.0147 | 0.362 | 0.787 |
| AD-39718.1 | 0.672 | 0.798 | 0.688 | 0.724 | 0.0889 | 0.0255 | 0.735 | 0.706 |
| AD-39724.1 | 0.599 | 1.136 | 0.643 | 0.680 | 0.3804 | 0.0261 | 0.868 | 0.661 |
| AD-39730.1 | 0.996 | 1.459 | 0.848 | 0.784 | 0.3271 | 0.0448 | 1.228 | 0.816 |
| AD-39736.1 | 1.085 | 1.121 | 0.733 | 0.883 | 0.0254 | 0.1064 | 1.103 | 0.808 |
| AD-39742.1 | 0.518 | 0.561 | 0.651 | 0.703 | 0.0308 | 0.0374 | 0.539 | 0.677 |
| AD-39748.1 | 0.445 | 0.609 | 0.772 | 0.788 | 0.1163 | 0.0113 | 0.527 | 0.780 |
| AD-39754.1 | 0.549 | 0.823 | 0.691 | 0.894 | 0.1942 | 0.1438 | 0.686 | 0.793 |
| AD-39760.1 | 0.233 | 0.210 | 0.417 | 0.747 | 0.0158 | 0.2332 | 0.221 | 0.582 |
| AD-39766.1 | 0.700 | 0.882 | 0.849 | 0.836 | 0.1291 | 0.0095 | 0.791 | 0.843 |
| AD-39772.1 | 0.323 | 0.379 | 0.797 | 0.870 | 0.0393 | 0.0519 | 0.351 | 0.833 |
| AD-39731.1 | 0.804 | 0.222 | 0.452 | 0.417 | 0.4116 | 0.0251 | 0.513 | 0.434 |
| AD-39737.1 | 0.450 | 0.347 | 0.737 | 0.768 | 0.0726 | 0.0219 | 0.399 | 0.753 |
| AD-39743.1 | 0.375 | 0.533 | 0.794 | 0.852 | 0.1112 | 0.0415 | 0.454 | 0.823 |
| AD-39695.1 | 0.610 | 0.492 | 0.831 | 0.821 | 0.0836 | 0.0070 | 0.551 | 0.826 |
| AD-39749.1 | 0.292 | 0.121 | 0.545 | 0.695 | 0.1205 | 0.1059 | 0.206 | 0.620 |
| AD-39755.1 | 0.226 | 0.275 | 0.874 | 1.170 | 0.0350 | 0.2093 | 0.251 | 1.022 |
| AD-39761.1 | 0.821 | 0.841 | 0.942 | 1.101 | 0.0141 | 0.1124 | 0.831 | 1.022 |
| AD-39767.1 | 0.360 | 0.374 | 0.730 | 0.711 | 0.0097 | 0.0134 | 0.367 | 0.720 |
| AD-39773.1 | 0.223 | 0.236 | 0.802 | 1.159 | 0.0096 | 0.2520 | 0.229 | 0.980 |
| AD-39732.1 | 0.446 | 0.662 | 1.593 | 1.181 | 0.1532 | 0.2914 | 0.554 | 1.387 |
| AD-39738.1 | 0.257 | 0.147 | 1.369 | 1.482 | 0.0776 | 0.0801 | 0.202 | 1.426 |
| AD-39744.1 | 0.259 | 0.200 | 1.097 | 0.871 | 0.0414 | 0.1592 | 0.230 | 0.984 |

(continued)

| | 10nM KRAS | | .1nM KRAS | | 10nM | .1nM | 10nM | .1nM |
|---|---|---|---|---|---|---|---|---|
| Duplex ID | bio1 | bio2 | bio1 | bio2 | SD | SD | AVG | AVG |
| AD-39750.1 | 0.182 | 0.302 | 0.642 | 0.782 | 0.0853 | 0.0988 | 0.242 | 0.712 |
| AD-39756.1 | 0.568 | 0.548 | 0.808 | 0.993 | 0.0144 | 0.1308 | 0.558 | 0.900 |
| AD-39762.1 | 0.684 | 0.916 | 0.783 | 0.929 | 0.1641 | 0.1031 | 0.800 | 0.856 |
| AD-39768.1 | 0.929 | 1.239 | 1.000 | 1.033 | 0.2194 | 0.0239 | 1.084 | 1.016 |
| AD-39774.1 | 0.391 | 0.447 | 1.087 | 1.087 | 0.0399 | 0.0003 | 0.419 | 1.087 |
| AD-39733.1 | 0.605 | 0.569 | 1.020 | 1.212 | 0.0251 | 0.1355 | 0.587 | 1.116 |
| AD-39739.1 | 0.950 | 1.271 | 0.959 | 1.030 | 0.2265 | 0.0501 | 1.111 | 0.994 |
| AD-39701.1 | 0.798 | 1.193 | 1.221 | 1.018 | 0.2794 | 0.1437 | 0.996 | 1.120 |
| AD-39745.1 | 0.257 | 0.212 | 1.116 | 1.226 | 0.0317 | 0.0779 | 0.234 | 1.171 |
| AD-39751.1 | 0.260 | 0.248 | 1.037 | 2.286 | 0.0085 | 0.8828 | 0.254 | 1.662 |
| AD-39757.1 | 0.319 | 0.566 | 1.098 | 0.949 | 0.1753 | 0.1053 | 0.443 | 1.023 |
| AD-39763.1 | 0.235 | 0.300 | 0.667 | 0.531 | 0.0463 | 0.0961 | 0.267 | 0.599 |
| AD-39769.1 | 0.637 | 0.531 | 0.526 | 0.826 | 0.0749 | 0.2120 | 0.584 | 0.676 |
| AD-39775.1 | 0.472 | 0.526 | 0.760 | 1.002 | 0.0382 | 0.1711 | 0.499 | 0.881 |
| AD-39734.1 | 0.915 | 0.920 | 0.751 | 0.837 | 0.0035 | 0.0606 | 0.917 | 0.794 |
| AD-39740.1 | 0.323 | 0.302 | 0.406 | 0.632 | 0.0145 | 0.1593 | 0.312 | 0.519 |
| AD-39746.1 | 0.553 | 0.702 | 0.949 | 1.145 | 0.1058 | 0.1382 | 0.627 | 1.047 |
| AD-39752.1 | 0.440 | 0.577 | 0.855 | 0.860 | 0.0965 | 0.0030 | 0.509 | 0.857 |
| AD-39758.1 | 1.129 | 1.095 | 0.952 | 0.830 | 0.0238 | 0.0863 | 1.112 | 0.891 |
| AD-39764.1 | 1.125 | 0.898 | 1.205 | 1.405 | 0.1606 | 0.1409 | 1.011 | 1.305 |
| AD-39770.1 | 0.647 | 0.314 | 0.672 | 0.461 | 0.2356 | 0.1486 | 0.480 | 0.566 |
| AD-39776.1 | 1.013 | 0.970 | 1.035 | 0.739 | 0.0304 | 0.2092 | 0.992 | 0.887 |
| AD-39735.1 | 0.284 | 0.201 | 0.613 | 0.976 | 0.0588 | 0.2564 | 0.242 | 0.794 |
| AD-39741.1 | 0.177 | 0.188 | 0.526 | 0.806 | 0.0074 | 0.1977 | 0.182 | 0.666 |
| AD-39747.1 | 1.047 | 0.926 | 1.247 | 1.483 | 0.0854 | 0.1666 | 0.986 | 1.365 |
| AD-39753.1 | 1.082 | 0.823 | 1.034 | 1.662 | 0.1833 | 0.4437 | 0.953 | 1.348 |
| AD-39759.1 | 0.327 | 0.292 | 0.638 | 0.412 | 0.0252 | 0.1600 | 0.309 | 0.525 |
| AD-39765.1 | 1.099 | 0.596 | 1.270 | 1.027 | 0.3554 | 0.1714 | 0.847 | 1.148 |
| AD-39771.1 | 0.238 | 0.334 | 0.801 | 0.460 | 0.0679 | 0.2407 | 0.286 | 0.631 |
| AD-39778.1 | 0.217 | 0.250 | 0.502 | 0.354 | 0.0235 | 0.1042 | 0.233 | 0.428 |
| AD-39784.1 | 0.698 | 0.598 | 1.036 | 0.944 | 0.0708 | 0.0655 | 0.648 | 0.990 |
| AD-39790.1 | 0.150 | 0.260 | 0.614 | 0.787 | 0.0777 | 0.1221 | 0.205 | 0.700 |
| AD-39796.1 | 0.635 | 0.610 | 0.731 | 0.846 | 0.0178 | 0.0813 | 0.622 | 0.788 |
| AD-39802.1 | 0.349 | 0.150 | 0.898 | 0.970 | 0.1408 | 0.0509 | 0.249 | 0.934 |
| AD-39808.1 | 0.734 | 0.972 | 1.079 | 0.955 | 0.1680 | 0.0881 | 0.853 | 1.017 |
| AD-39814.1 | 1.070 | 1.294 | 0.979 | 0.968 | 0.1588 | 0.0074 | 1.182 | 0.974 |
| AD-39820.1 | 0.381 | 0.209 | 0.786 | 0.687 | 0.1218 | 0.0701 | 0.295 | 0.737 |

(continued)

| | 10nM KRAS | | .1nM KRAS | | 10nM | .1nM | 10nM | .1nM |
|---|---|---|---|---|---|---|---|---|
| Duplex ID | bio1 | bio2 | bio1 | bio2 | SD | SD | AVG | AVG |
| AD-39779.1 | 0.744 | 0.889 | 1.038 | 0.942 | 0.1020 | 0.0683 | 0.817 | 0.990 |
| AD-39785.1 | 0.182 | 0.218 | 0.509 | 0.506 | 0.0255 | 0.0022 | 0.200 | 0.507 |
| AD-39791.1 | 0.214 | 0.308 | 0.556 | 0.519 | 0.0667 | 0.0260 | 0.261 | 0.537 |
| AD-39797.1 | 0.341 | 0.326 | 0.788 | 0.543 | 0.0100 | 0.1730 | 0.333 | 0.665 |
| AD-39803.1 | 0.229 | 0.210 | 0.808 | 0.648 | 0.0135 | 0.1133 | 0.220 | 0.728 |
| AD-39809.1 | 0.624 | 0.829 | 1.060 | 1.031 | 0.1450 | 0.0211 | 0.727 | 1.046 |
| AD-39815.1 | 0.441 | 0.449 | 0.862 | 0.852 | 0.0057 | 0.0072 | 0.445 | 0.857 |
| AD-39821.1 | 0.582 | 0.592 | 0.722 | 0.608 | 0.0071 | 0.0806 | 0.587 | 0.665 |
| AD-39780.1 | 0.748 | 0.784 | 0.973 | 0.976 | 0.0252 | 0.0025 | 0.766 | 0.974 |
| AD-39786.1 | 0.376 | 0.455 | 0.466 | 0.640 | 0.0557 | 0.1226 | 0.416 | 0.553 |
| AD-39792.1 | 1.195 | 1.019 | 0.908 | 1.168 | 0.1249 | 0.1835 | 1.107 | 1.038 |
| AD-39798.1 | 0.429 | 0.342 | 0.933 | 0.936 | 0.0615 | 0.0020 | 0.386 | 0.934 |
| AD-39804.1 | 0.551 | 0.588 | 0.942 | 0.959 | 0.0259 | 0.0119 | 0.570 | 0.950 |
| AD-39810.1 | 0.605 | 0.532 | 0.989 | 0.956 | 0.0518 | 0.0236 | 0.568 | 0.972 |
| AD-39764.1 | 1.112 | 1.027 | 0.961 | 0.746 | 0.0602 | 0.1522 | 1.070 | 0.854 |
| AD-39816.1 | 0.805 | 0.645 | 1.014 | 0.839 | 0.1130 | 0.1236 | 0.725 | 0.927 |
| AD-39770.1 | 0.420 | 0.371 | 0.858 | 0.887 | 0.0345 | 0.0201 | 0.396 | 0.873 |
| AD-39822.1 | 0.288 | 0.313 | 0.589 | 0.494 | 0.0178 | 0.0671 | 0.301 | 0.542 |
| AD-39781.1 | 0.276 | 0.379 | 0.404 | 0.305 | 0.0725 | 0.0701 | 0.328 | 0.354 |
| AD-39787.1 | 0.982 | 1.065 | 0.675 | 0.803 | 0.0584 | 0.0909 | 1.024 | 0.739 |
| AD-39793.1 | 0.165 | 0.291 | 0.487 | 0.488 | 0.0893 | 0.0007 | 0.228 | 0.487 |
| AD-39799.1 | 0.444 | 0.540 | 0.686 | 0.746 | 0.0675 | 0.0423 | 0.492 | 0.716 |
| AD-39805.1 | 0.295 | 0.250 | 0.474 | 0.421 | 0.0320 | 0.0371 | 0.273 | 0.448 |
| AD-39811.1 | 0.646 | 0.792 | 0.892 | 0.882 | 0.1030 | 0.0070 | 0.719 | 0.887 |
| AD-39817.1 | 0.583 | 0.824 | 0.848 | 0.846 | 0.1708 | 0.0012 | 0.703 | 0.847 |
| AD-39823.1 | 1.229 | 1.075 | 1.080 | 0.859 | 0.1083 | 0.1564 | 1.152 | 0.970 |
| AD-39782.1 | 0.605 | 0.757 | 1.162 | 0.883 | 0.1076 | 0.1972 | 0.681 | 1.022 |
| AD-39788.1 | 0.257 | 0.289 | 0.665 | 0.534 | 0.0227 | 0.0926 | 0.273 | 0.599 |
| AD-39794.1 | 0.667 | 0.699 | 1.196 | 1.102 | 0.0229 | 0.0668 | 0.683 | 1.149 |
| AD-39800.1 | 0.888 | 0.734 | 1.201 | 0.908 | 0.1092 | 0.2072 | 0.811 | 1.054 |
| AD-39806.1 | 0.505 | 0.637 | 0.630 | 0.849 | 0.0935 | 0.1549 | 0.571 | 0.739 |
| AD-39812.1 | 0.281 | 0.390 | 0.521 | 0.597 | 0.0771 | 0.0538 | 0.335 | 0.559 |
| AD-39818.1 | 0.999 | 1.049 | 0.789 | 0.936 | 0.0347 | 0.1044 | 1.024 | 0.863 |
| AD-39824.1 | 1.086 | 1.053 | 1.370 | 1.034 | 0.0235 | 0.2377 | 1.070 | 1.202 |
| AD-39783.1 | 0.460 | 0.731 | 0.736 | 0.822 | 0.1916 | 0.0609 | 0.595 | 0.779 |
| AD-39789.1 | 0.859 | 1.454 | 1.139 | 1.308 | 0.4213 | 0.1195 | 1.157 | 1.224 |
| AD-39795.1 | 1.165 | 0.901 | 1.015 | 0.916 | 0.1866 | 0.0698 | 1.033 | 0.965 |

(continued)

| | 10nM KRAS | | .1nM KRAS | | 10nM | .1nM | 10nM | .1nM |
|---|---|---|---|---|---|---|---|---|
| Duplex ID | bio1 | bio2 | bio1 | bio2 | SD | SD | AVG | AVG |
| AD-39801.1 | 1.222 | 1.191 | 1.098 | 0.843 | 0.0216 | 0.1801 | 1.207 | 0.971 |
| AD-39807.1 | 0.776 | 0.807 | 1.007 | 1.002 | 0.0215 | 0.0036 | 0.791 | 1.004 |
| AD-39813.1 | 0.923 | 1.206 | 1.008 | 0.901 | 0.1998 | 0.0753 | 1.065 | 0.955 |
| AD-39819.1 | 0.952 | 1.021 | 1.024 | 0.916 | 0.0484 | 0.0765 | 0.986 | 0.970 |
| AD-39825.1 | 0.648 | 0.864 | 1.028 | 0.981 | 0.1529 | 0.0336 | 0.756 | 1.004 |
| AD-39776.1 | 0.795 | 1.019 | 0.891 | 1.046 | 0.1581 | 0.1094 | 0.907 | 0.969 |
| AD-39831.1 | 0.648 | 0.601 | 1.018 | 1.034 | 0.0334 | 0.0119 | 0.624 | 1.026 |
| AD-39837.1 | 0.799 | 0.984 | 0.969 | 0.903 | 0.1308 | 0.0463 | 0.891 | 0.936 |
| AD-39843.1 | 0.325 | 0.385 | 0.695 | 0.572 | 0.0427 | 0.0866 | 0.355 | 0.634 |
| AD-39849.1 | 0.702 | 1.075 | 0.903 | 0.969 | 0.2638 | 0.0469 | 0.889 | 0.936 |
| AD-39855.1 | 0.309 | 0.490 | 1.202 | 0.800 | 0.1280 | 0.2840 | 0.399 | 1.001 |
| AD-39861.1 | 0.762 | 1.162 | 1.020 | 1.000 | 0.2825 | 0.0141 | 0.962 | 1.010 |
| AD-39867.1 | 0.873 | 1.162 | 1.219 | 0.918 | 0.2040 | 0.2126 | 1.017 | 1.069 |
| AD-39826.1 | 1.358 | 1.043 | 1.148 | 0.961 | 0.2226 | 0.1320 | 1.201 | 1.055 |
| AD-39832.1 | 0.792 | 1.337 | 1.214 | 0.873 | 0.3849 | 0.2413 | 1.065 | 1.043 |
| AD-39838.1 | 0.429 | 0.270 | 0.793 | 0.530 | 0.1128 | 0.1859 | 0.349 | 0.661 |
| AD-39844.1 | 0.572 | 0.789 | 0.833 | 0.692 | 0.1536 | 0.0997 | 0.680 | 0.762 |
| AD-39850.1 | 0.535 | 0.231 | 0.505 | 0.529 | 0.2152 | 0.0169 | 0.383 | 0.517 |
| AD-39856.1 | 0.323 | 0.377 | 0.835 | 0.784 | 0.0384 | 0.0359 | 0.350 | 0.810 |
| AD-39862.1 | 0.405 | 0.429 | 0.835 | 0.615 | 0.0167 | 0.1552 | 0.417 | 0.725 |
| AD-39868.1 | 0.386 | 0.484 | 0.683 | 0.643 | 0.0697 | 0.0284 | 0.435 | 0.663 |
| AD-39735.1 | 0.168 | 0.255 | 0.439 | 0.294 | 0.0616 | 0.1026 | 0.211 | 0.366 |
| AD-39827.1 | 0.202 | 0.313 | 0.616 | 0.413 | 0.0787 | 0.1432 | 0.258 | 0.515 |
| AD-39833.1 | 0.190 | 0.492 | 1.007 | 1.026 | 0.2134 | 0.0135 | 0.341 | 1.017 |
| AD-39839.1 | 0.790 | 1.002 | 1.227 | 1.327 | 0.1503 | 0.0708 | 0.896 | 1.277 |
| AD-39845.1 | 0.192 | 0.213 | 0.391 | 0.218 | 0.0147 | 0.1220 | 0.202 | 0.304 |
| AD-39851.1 | 0.779 | 0.493 | 1.243 | 0.929 | 0.2022 | 0.2220 | 0.636 | 1.086 |
| AD-39857.1 | 0.602 | 1.444 | 0.992 | 0.692 | 0.5960 | 0.2120 | 1.023 | 0.842 |
| AD-39863.1 | 0.482 | 0.135 | 0.906 | 0.632 | 0.2449 | 0.1937 | 0.308 | 0.769 |
| AD-39869.1 | 0.357 | 0.025 | 0.799 | 0.558 | 0.2347 | 0.1708 | 0.191 | 0.678 |
| AD-39828.1 | 1.185 | 1.085 | 1.176 | 0.952 | 0.0707 | 0.1585 | 1.135 | 1.064 |
| AD-39834.1 | 1.140 | 1.273 | 1.315 | 1.075 | 0.0938 | 0.1698 | 1.207 | 1.195 |
| AD-39840.1 | 0.251 | 0.541 | 0.766 | 0.591 | 0.2050 | 0.1239 | 0.396 | 0.679 |
| AD-39846.1 | 0.635 | 0.646 | 1.044 | 0.984 | 0.0077 | 0.0425 | 0.641 | 1.014 |
| AD-39852.1 | 0.434 | 0.555 | 0.707 | 0.719 | 0.0857 | 0.0085 | 0.494 | 0.713 |
| AD-39858.1 | 0.169 | 0.192 | 0.363 | 0.268 | 0.0160 | 0.0670 | 0.180 | 0.316 |
| AD-39864.1 | 1.612 | 1.161 | 0.825 | 0.919 | 0.3189 | 0.0665 | 1.387 | 0.872 |

(continued)

| | 10nM KRAS | | .1nM KRAS | | 10nM | .1nM | 10nM | .1nM |
|---|---|---|---|---|---|---|---|---|
| Duplex ID | bio1 | bio2 | bio1 | bio2 | SD | SD | AVG | AVG |
| AD-39870.1 | 0.321 | 0.186 | 0.072 | 0.170 | 0.0952 | 0.0695 | 0.253 | 0.121 |
| AD-39829.1 | 0.283 | 0.133 | 0.353 | 0.266 | 0.1060 | 0.0611 | 0.208 | 0.310 |
| AD-39835.1 | 1.265 | 0.792 | 0.460 | 0.641 | 0.3343 | 0.1277 | 1.029 | 0.551 |
| AD-39841.1 | 0.345 | 0.267 | 0.416 | 0.369 | 0.0552 | 0.0335 | 0.306 | 0.392 |
| AD-39853.1 | 0.257 | 0.268 | 0.640 | 0.527 | 0.0081 | 0.0799 | 0.262 | 0.584 |
| AD-39859.1 | 0.316 | 0.290 | 0.773 | 0.886 | 0.0182 | 0.0801 | 0.303 | 0.830 |
| AD-39865.1 | 0.228 | 0.216 | 0.456 | 0.424 | 0.0084 | 0.0231 | 0.222 | 0.440 |
| AD-39871.1 | 0.218 | 0.338 | 0.502 | 0.571 | 0.0849 | 0.0489 | 0.278 | 0.537 |
| AD-39830.1 | 0.284 | 0.293 | 0.770 | 0.716 | 0.0063 | 0.0379 | 0.289 | 0.743 |
| AD-39836.1 | 0.267 | 0.232 | 0.615 | 0.580 | 0.0249 | 0.0254 | 0.250 | 0.598 |
| AD-39842.1 | 0.242 | 0.259 | 0.707 | 0.654 | 0.0121 | 0.0373 | 0.250 | 0.681 |
| AD-39848.1 | 0.515 | 0.591 | 1.071 | 1.012 | 0.0536 | 0.0416 | 0.553 | 1.042 |
| AD-39854.1 | 0.853 | 1.128 | 1.035 | 1.059 | 0.1943 | 0.0169 | 0.991 | 1.047 |
| AD-39860.1 | 0.969 | 1.119 | 0.965 | 0.833 | 0.1058 | 0.0936 | 1.044 | 0.899 |
| AD-39866.1 | 0.247 | 0.395 | 0.733 | 0.834 | 0.1045 | 0.0711 | 0.321 | 0.784 |
| AD-39992.1 | 0.507 | 0.837 | 0.985 | 0.990 | 0.2334 | 0.0032 | 0.672 | 0.988 |
| AD-39998.1 | 0.384 | 0.549 | 0.745 | 0.676 | 0.1167 | 0.0486 | 0.466 | 0.711 |
| AD-40004.1 | 0.509 | 0.664 | 1.090 | 1.042 | 0.1096 | 0.0340 | 0.587 | 1.066 |
| AD-40010.1 | 0.212 | 0.245 | 0.954 | 0.885 | 0.0232 | 0.0487 | 0.228 | 0.919 |
| AD-40016.1 | 0.261 | 0.514 | 0.731 | 1.050 | 0.1790 | 0.2254 | 0.387 | 0.891 |
| AD-40022.1 | 0.784 | 1.221 | 0.955 | 0.939 | 0.3089 | 0.0112 | 1.003 | 0.947 |
| AD-40028.1 | 0.907 | 1.194 | 1.062 | 0.976 | 0.2026 | 0.0608 | 1.050 | 1.019 |
| AD-40034.1 | 0.367 | 0.483 | 0.902 | 0.718 | 0.0819 | 0.1298 | 0.425 | 0.810 |
| AD-39999.1 | 0.227 | 0.339 | 0.910 | 0.837 | 0.0789 | 0.0511 | 0.283 | 0.873 |
| AD-40005.1 | 0.219 | 0.202 | 0.419 | 0.421 | 0.0121 | 0.0012 | 0.211 | 0.420 |
| AD-40011.1 | 0.470 | 0.529 | 0.731 | 0.637 | 0.0413 | 0.0663 | 0.499 | 0.684 |
| AD-40017.1 | 0.209 | 0.402 | 0.873 | 0.806 | 0.1364 | 0.0475 | 0.306 | 0.839 |
| AD-40029.1 | 0.793 | 0.821 | 0.904 | 0.834 | 0.0199 | 0.0496 | 0.807 | 0.869 |
| AD-39846.1 | 0.591 | 0.468 | 0.690 | 0.823 | 0.0867 | 0.0942 | 0.529 | 0.757 |
| AD-40035.1 | 0.872 | 1.166 | 1.079 | 1.090 | 0.2083 | 0.0081 | 1.019 | 1.085 |
| AD-39852.1 | 0.439 | 0.346 | 0.784 | 0.995 | 0.0659 | 0.1492 | 0.393 | 0.890 |
| AD-39994.1 | 0.396 | 0.517 | 0.936 | 1.097 | 0.0854 | 0.1137 | 0.457 | 1.016 |
| AD-40000.1 | 0.574 | 0.734 | 1.100 | 1.100 | 0.1133 | 0.0004 | 0.654 | 1.100 |
| AD-40006.1 | 0.402 | 0.389 | 0.831 | 0.800 | 0.0094 | 0.0223 | 0.395 | 0.815 |
| AD-40012.1 | 0.211 | 0.435 | 0.704 | 0.929 | 0.1588 | 0.1593 | 0.323 | 0.816 |
| AD-40018.1 | 0.692 | 1.163 | 0.808 | 0.933 | 0.3325 | 0.0883 | 0.928 | 0.871 |
| AD-40024.1 | 0.182 | 0.428 | 0.772 | 0.716 | 0.1739 | 0.0399 | 0.305 | 0.744 |

(continued)

| | 10nM KRAS | | .1nM KRAS | | 10nM | .1nM | 10nM | .1nM |
|---|---|---|---|---|---|---|---|---|
| Duplex ID | bio1 | bio2 | bio1 | bio2 | SD | SD | AVG | AVG |
| AD-40030.1 | 0.437 | 0.651 | 0.825 | 0.874 | 0.1516 | 0.0344 | 0.544 | 0.849 |
| AD-40036.1 | 0.092 | 0.216 | 0.452 | 0.532 | 0.0876 | 0.0567 | 0.154 | 0.492 |
| AD-39995.1 | 0.290 | 0.686 | 0.918 | 0.618 | 0.2803 | 0.2122 | 0.488 | 0.768 |
| AD-40001.1 | 0.672 | 0.758 | 0.709 | 1.090 | 0.0607 | 0.2698 | 0.715 | 0.899 |
| AD-40007.1 | 0.785 | 0.477 | 0.780 | 0.928 | 0.2177 | 0.1048 | 0.631 | 0.854 |
| AD-40013.1 | 0.503 | 0.901 | 0.782 | 1.056 | 0.2814 | 0.1936 | 0.702 | 0.919 |
| AD-40019.1 | 0.264 | 0.125 | 0.615 | 0.731 | 0.0979 | 0.0818 | 0.194 | 0.673 |
| AD-40025.1 | 0.864 | 1.208 | 0.968 | 1.047 | 0.2430 | 0.0562 | 1.036 | 1.007 |
| AD-40031.1 | 0.268 | 0.286 | 0.995 | 0.805 | 0.0124 | 0.1344 | 0.277 | 0.900 |
| AD-40037.1 | 0.579 | 0.647 | 0.689 | 1.211 | 0.0484 | 0.3693 | 0.613 | 0.950 |
| AD-39996.1 | 0.338 | 0.400 | 0.618 | 0.680 | 0.0438 | 0.0442 | 0.369 | 0.649 |
| AD-40002.1 | 0.993 | 0.812 | 0.746 | 0.851 | 0.1281 | 0.0747 | 0.903 | 0.798 |
| AD-40008.1 | 0.227 | 0.154 | 0.309 | 0.434 | 0.0516 | 0.0879 | 0.190 | 0.372 |
| AD-40014.1 | 0.612 | 0.631 | 0.702 | 0.613 | 0.0136 | 0.0630 | 0.622 | 0.658 |
| AD-40020.1 | 0.386 | 0.409 | 0.962 | 1.008 | 0.0164 | 0.0324 | 0.398 | 0.985 |
| AD-40026.1 | 0.229 | 0.130 | 0.370 | 0.498 | 0.0702 | 0.0901 | 0.180 | 0.434 |
| AD-40032.1 | 0.421 | 0.454 | 0.979 | 0.954 | 0.0232 | 0.0176 | 0.437 | 0.966 |
| AD-40038.1 | 0.491 | 0.674 | 0.851 | 1.008 | 0.1291 | 0.1106 | 0.582 | 0.930 |
| AD-39997.1 | 0.644 | 0.860 | 0.749 | 0.878 | 0.1531 | 0.0917 | 0.752 | 0.814 |
| AD-39858.1 | 0.146 | 0.293 | 0.681 | 0.569 | 0.1043 | 0.0792 | 0.219 | 0.625 |
| AD-40009.1 | 0.191 | 0.382 | 0.912 | 1.055 | 0.1351 | 0.1012 | 0.287 | 0.984 |
| AD-40015.1 | 0.897 | 0.856 | 0.778 | 1.065 | 0.0292 | 0.2034 | 0.876 | 0.922 |
| AD-40021.1 | 0.225 | 0.282 | 0.850 | 0.658 | 0.0401 | 0.1358 | 0.254 | 0.754 |
| AD-40027.1 | 0.466 | 1.083 | 0.916 | 0.854 | 0.4358 | 0.0437 | 0.774 | 0.885 |
| AD-40033.1 | 0.278 | 0.311 | 0.426 | 0.588 | 0.0230 | 0.1150 | 0.295 | 0.507 |
| AD-40039.1 | 0.257 | 0.447 | 0.666 | 0.739 | 0.1348 | 0.0511 | 0.352 | 0.703 |
| AD-40045.1 | 0.263 | 0.147 | 0.526 | 0.945 | 0.0821 | 0.2962 | 0.205 | 0.735 |
| AD-40051.1 | 0.162 | 0.126 | 0.665 | 0.837 | 0.0255 | 0.1209 | 0.144 | 0.751 |
| AD-40057.1 | 0.150 | 0.176 | 0.641 | 0.791 | 0.0178 | 0.1066 | 0.163 | 0.716 |
| AD-40063.1 | 0.220 | 0.287 | 0.650 | 0.977 | 0.0468 | 0.2311 | 0.253 | 0.813 |
| AD-40069.1 | 0.431 | 0.559 | 0.729 | 0.922 | 0.0907 | 0.1363 | 0.495 | 0.825 |
| AD-40075.1 | 0.399 | 0.613 | 0.834 | 0.972 | 0.1511 | 0.0971 | 0.506 | 0.903 |
| AD-40081.1 | 0.287 | 0.351 | 0.499 | 0.612 | 0.0453 | 0.0795 | 0.319 | 0.556 |
| AD-40040.1 | 0.861 | 0.850 | 0.888 | 1.077 | 0.0077 | 0.1337 | 0.856 | 0.983 |
| AD-40046.1 | 0.386 | 0.397 | 0.607 | 0.634 | 0.0083 | 0.0191 | 0.392 | 0.621 |
| AD-39864.1 | 0.930 | 0.933 | 0.810 | 1.018 | 0.0022 | 0.1468 | 0.931 | 0.914 |
| AD-40052.1 | 0.272 | 0.216 | 0.526 | 0.852 | 0.0396 | 0.2299 | 0.244 | 0.689 |

(continued)

| | 10nM KRAS | | .1nM KRAS | | 10nM | .1nM | 10nM | .1nM |
|---|---|---|---|---|---|---|---|---|
| Duplex ID | bio1 | bio2 | bio1 | bio2 | SD | SD | AVG | AVG |
| AD-40058.1 | 0.398 | 0.305 | 0.928 | 1.079 | 0.0655 | 0.1071 | 0.351 | 1.003 |
| AD-40064.1 | 0.460 | 0.208 | 0.909 | 1.083 | 0.1777 | 0.1230 | 0.334 | 0.996 |
| AD-40070.1 | 0.116 | 0.454 | 0.447 | 0.885 | 0.2390 | 0.3095 | 0.285 | 0.666 |
| AD-40076.1 | 0.396 | 1.498 | 0.634 | 1.054 | 0.7792 | 0.2969 | 0.947 | 0.844 |
| AD-40082.1 | 0.584 | 0.553 | 0.760 | 1.053 | 0.0218 | 0.2073 | 0.569 | 0.906 |
| AD-40041.1 | 0.196 | 0.241 | 0.862 | 0.978 | 0.0322 | 0.0819 | 0.218 | 0.920 |
| AD-40047.1 | 0.289 | 0.214 | 0.699 | 1.096 | 0.0535 | 0.2806 | 0.251 | 0.897 |
| AD-40053.1 | 0.973 | 1.094 | 0.717 | 1.079 | 0.0854 | 0.2560 | 1.033 | 0.898 |
| AD-40059.1 | 1.064 | 0.985 | 0.720 | 1.088 | 0.0562 | 0.2601 | 1.025 | 0.904 |
| AD-40065.1 | 0.855 | 0.863 | 0.589 | 1.020 | 0.0057 | 0.3048 | 0.859 | 0.805 |
| AD-40071.1 | 0.242 | 0.346 | 0.846 | 0.826 | 0.0739 | 0.0142 | 0.294 | 0.836 |
| AD-40077.1 | 0.249 | 0.187 | 0.649 | 0.738 | 0.0437 | 0.0633 | 0.218 | 0.693 |
| AD-40083.1 | 0.286 | 0.339 | 0.807 | 1.130 | 0.0373 | 0.2279 | 0.312 | 0.968 |
| AD-40042.1 | 0.549 | 0.288 | 1.205 | 1.288 | 0.1845 | 0.0587 | 0.418 | 1.246 |
| AD-40048.1 | 0.413 | 0.345 | 0.713 | 0.940 | 0.0483 | 0.1610 | 0.379 | 0.826 |
| AD-40054.1 | 0.278 | 0.258 | 0.685 | 0.825 | 0.0146 | 0.0989 | 0.268 | 0.755 |
| AD-40060.1 | 0.288 | 0.230 | 0.781 | 0.811 | 0.0410 | 0.0217 | 0.259 | 0.796 |
| AD-40066.1 | 0.779 | 0.829 | 0.950 | 1.284 | 0.0357 | 0.2361 | 0.804 | 1.117 |
| AD-40072.1 | 0.273 | 0.169 | 0.579 | 0.963 | 0.0738 | 0.2716 | 0.221 | 0.771 |
| AD-40078.1 | 0.747 | 0.841 | 1.177 | 1.410 | 0.0666 | 0.1645 | 0.794 | 1.294 |
| AD-40084.1 | 1.064 | 0.972 | 0.944 | 1.036 | 0.0647 | 0.0651 | 1.018 | 0.990 |
| AD-40043.1 | 0.340 | 0.313 | 0.804 | 0.795 | 0.0190 | 0.0065 | 0.327 | 0.800 |
| AD-40049.1 | 0.179 | 0.159 | 0.339 | 0.312 | 0.0141 | 0.0191 | 0.169 | 0.326 |
| AD-40055.1 | 0.243 | 0.248 | 0.811 | 0.817 | 0.0034 | 0.0044 | 0.245 | 0.814 |
| AD-40061.1 | 0.153 | 0.229 | 0.357 | 0.519 | 0.0537 | 0.1146 | 0.191 | 0.438 |
| AD-40067.1 | 0.260 | 0.141 | 0.715 | 0.692 | 0.0837 | 0.0156 | 0.201 | 0.703 |
| AD-40073.1 | 0.241 | 0.156 | 0.833 | 0.858 | 0.0597 | 0.0180 | 0.199 | 0.846 |
| AD-40079.1 | 0.236 | 0.236 | 0.694 | 0.991 | 0.0002 | 0.2099 | 0.236 | 0.842 |
| AD-40085.1 | 0.188 | 0.210 | 0.558 | 0.519 | 0.0155 | 0.0276 | 0.199 | 0.539 |
| AD-40044.1 | 0.229 | 0.126 | 0.342 | 0.506 | 0.0728 | 0.1157 | 0.178 | 0.424 |
| AD-40050.1 | 1.016 | 0.566 | 0.933 | 1.088 | 0.3183 | 0.1097 | 0.791 | 1.011 |
| AD-40056.1 | 0.274 | 0.244 | 0.801 | 1.155 | 0.0215 | 0.2506 | 0.259 | 0.978 |
| AD-40062.1 | 0.536 | 0.653 | 0.900 | 1.143 | 0.0822 | 0.1719 | 0.595 | 1.022 |
| AD-40068.1 | 0.175 | 0.131 | 0.218 | 0.438 | 0.0313 | 0.1551 | 0.153 | 0.328 |
| AD-40074.1 | 0.366 | 0.462 | 0.911 | 0.734 | 0.0674 | 0.1256 | 0.414 | 0.823 |
| AD-40080.1 | 0.273 | 0.296 | 0.844 | 0.932 | 0.0166 | 0.0623 | 0.284 | 0.888 |
| AD-40071.1 | 0.341 | 0.214 | 0.691 | 0.912 | 0.0902 | 0.1562 | 0.277 | 0.801 |

(continued)

| | 10nM KRAS | | .1nM KRAS | | 10nM | .1nM | 10nM | .1nM |
|---|---|---|---|---|---|---|---|---|
| Duplex ID | bio1 | bio2 | bio1 | bio2 | SD | SD | AVG | AVG |
| AD-40077.1 | 0.192 | 0.180 | 0.483 | 0.404 | 0.0083 | 0.0563 | 0.186 | 0.444 |
| AD-40083.1 | 0.145 | 0.238 | 0.514 | 0.872 | 0.0657 | 0.2536 | 0.192 | 0.693 |
| AD-40042.1 | 0.403 | 0.288 | 0.626 | 0.747 | 0.0814 | 0.0856 | 0.346 | 0.686 |
| AD-35523.3 | 0.228 | 0.350 | 1.165 | 1.104 | 0.0862 | 0.0434 | 0.289 | 1.135 |
| AD-35529.1 | 0.367 | 0.368 | 1.099 | 0.831 | 0.0004 | 0.1893 | 0.368 | 0.965 |
| AD-35535.4 | 0.466 | 0.362 | 0.563 | 0.600 | 0.0735 | 0.0264 | 0.414 | 0.582 |
| AD-35541.4 | 0.585 | 0.358 | 1.003 | 0.713 | 0.1606 | 0.2052 | 0.472 | 0.858 |
| AD-35547.4 | 0.491 | 0.546 | 0.732 | 0.626 | 0.0393 | 0.0753 | 0.518 | 0.679 |
| AD-35553.4 | 0.785 | 0.690 | 1.043 | 0.744 | 0.0677 | 0.2115 | 0.738 | 0.893 |
| AD-35559.4 | 0.387 | 0.437 | 0.779 | 0.776 | 0.0357 | 0.0021 | 0.412 | 0.778 |
| AD-35565.4 | 0.549 | 0.568 | 0.930 | 1.163 | 0.0132 | 0.1647 | 0.559 | 1.046 |
| AD-35524.4 | 0.358 | 0.399 | 0.997 | 0.915 | 0.0289 | 0.0576 | 0.379 | 0.956 |
| AD-35530.4 | 0.212 | 0.406 | 1.129 | 0.981 | 0.1374 | 0.1046 | 0.309 | 1.055 |
| AD-35536.4 | 0.463 | 0.272 | 1.028 | 1.131 | 0.1352 | 0.0727 | 0.368 | 1.080 |
| AD-35542.4 | 0.573 | 0.537 | 0.804 | 0.919 | 0.0255 | 0.0808 | 0.555 | 0.861 |
| AD-35548.4 | 0.944 | 0.574 | 0.886 | 0.728 | 0.2619 | 0.1113 | 0.759 | 0.807 |
| AD-35554.4 | 0.490 | 0.393 | 1.021 | 0.679 | 0.0682 | 0.2416 | 0.442 | 0.850 |
| AD-35560.2 | 1.199 | 0.386 | 0.796 | 0.610 | 0.5748 | 0.1310 | 0.793 | 0.703 |
| AD-35566.4 | 1.082 | 0.981 | 0.747 | 0.840 | 0.0714 | 0.0652 | 1.031 | 0.794 |
| AD-35525.2 | 0.645 | 0.767 | 0.956 | 0.627 | 0.0864 | 0.2323 | 0.706 | 0.792 |
| AD-35531.4 | 0.425 | 0.328 | 0.755 | 0.776 | 0.0683 | 0.0144 | 0.376 | 0.765 |
| AD-35537.4 | 0.824 | 0.823 | 0.710 | 0.628 | 0.0004 | 0.0578 | 0.824 | 0.669 |
| AD-35543.2 | 0.232 | 0.367 | 0.980 | 0.845 | 0.0954 | 0.0949 | 0.300 | 0.912 |
| AD-35549.4 | 1.492 | 0.378 | 1.057 | 0.665 | 0.7877 | 0.2769 | 0.935 | 0.861 |
| AD-35555.4 | 0.382 | 0.460 | 1.133 | 1.096 | 0.0554 | 0.0260 | 0.421 | 1.115 |
| AD-35561.4 | 1.037 | 0.685 | 1.036 | 0.854 | 0.2488 | 0.1285 | 0.861 | 0.945 |
| AD-35567.4 | 1.437 | 0.698 | 1.098 | 0.909 | 0.5222 | 0.1337 | 1.068 | 1.004 |
| AD-35526.4 | 1.354 | 0.995 | 0.597 | 0.754 | 0.2540 | 0.1111 | 1.174 | 0.675 |
| AD-35532.4 | 0.645 | 0.312 | 1.117 | 0.944 | 0.2357 | 0.1222 | 0.479 | 1.031 |
| AD-35535.4 | 0.694 | 0.362 | 0.856 | 1.093 | 0.2353 | 0.1672 | 0.528 | 0.974 |
| AD-35538.4 | 0.280 | 0.358 | 1.121 | 0.733 | 0.0551 | 0.2744 | 0.319 | 0.927 |
| AD-35544.4 | 1.562 | 0.806 | 0.895 | 1.104 | 0.5351 | 0.1477 | 1.184 | 1.000 |
| AD-35550.4 | 0.988 | 0.683 | 0.745 | 1.159 | 0.2155 | 0.2926 | 0.836 | 0.952 |
| AD-35556.4 | 0.597 | 0.298 | 1.087 | 0.890 | 0.2119 | 0.1388 | 0.448 | 0.989 |
| AD-35562.4 | 0.412 | 0.652 | 0.796 | 0.857 | 0.1700 | 0.0436 | 0.532 | 0.826 |
| AD-35568.4 | 0.324 | 0.544 | 0.986 | 1.171 | 0.1556 | 0.1304 | 0.434 | 1.079 |
| AD-35527.4 | 0.218 | 0.272 | 1.079 | 1.097 | 0.0380 | 0.0122 | 0.245 | 1.088 |

(continued)

| | 10nM KRAS | | .1nM KRAS | | 10nM | .1nM | 10nM | .1nM |
|---|---|---|---|---|---|---|---|---|
| Duplex ID | bio1 | bio2 | bio1 | bio2 | SD | SD | AVG | AVG |
| AD-35533.4 | 0.488 | 0.226 | 1.236 | 1.089 | 0.1848 | 0.1038 | 0.357 | 1.162 |
| AD-35539.4 | 0.821 | 0.660 | 1.065 | 1.155 | 0.1140 | 0.0637 | 0.740 | 1.110 |
| AD-35545.4 | 1.206 | 0.787 | 0.990 | 1.097 | 0.2968 | 0.0755 | 0.997 | 1.043 |
| AD-35551.4 | 0.860 | 0.404 | 1.011 | 1.078 | 0.3220 | 0.0473 | 0.632 | 1.044 |
| AD-35563.4 | 1.183 | 0.977 | 0.908 | 1.277 | 0.1461 | 0.2606 | 1.080 | 1.092 |
| AD-35528.4 | 0.955 | 0.770 | 1.094 | 0.757 | 0.1309 | 0.2387 | 0.863 | 0.926 |
| AD-35540.4 | 0.560 | 0.541 | 1.061 | 1.052 | 0.0132 | 0.0065 | 0.550 | 1.056 |
| AD-35546.4 | 0.716 | 0.605 | 1.121 | 0.996 | 0.0789 | 0.0886 | 0.660 | 1.058 |
| AD-35541.4 | 0.847 | 0.358 | 1.046 | 1.002 | 0.3458 | 0.0314 | 0.603 | 1.024 |
| AD-35552.4 | 0.468 | 0.529 | 1.057 | 1.002 | 0.0431 | 0.0391 | 0.499 | 1.029 |
| AD-35558.4 | 0.424 | 0.342 | 1.241 | 1.045 | 0.0578 | 0.1385 | 0.383 | 1.143 |
| AD-35564.4 | 0.485 | 0.438 | 0.980 | 0.968 | 0.0329 | 0.0085 | 0.461 | 0.974 |
| AD-35570.4 | 0.579 | 0.413 | 0.839 | 1.108 | 0.1175 | 0.1900 | 0.496 | 0.973 |
| AD-35571.4 | 0.476 | 0.348 | 0.818 | 0.951 | 0.0907 | 0.0943 | 0.412 | 0.885 |
| AD-35577.4 | 0.300 | 0.393 | 0.844 | 0.882 | 0.0660 | 0.0267 | 0.347 | 0.863 |
| AD-35583.4 | 1.305 | 0.679 | 0.970 | 1.085 | 0.4424 | 0.0813 | 0.992 | 1.027 |
| AD-35589.4 | 1.799 | 1.425 | 1.223 | 1.155 | 0.2646 | 0.0480 | 1.612 | 1.189 |
| AD-35595.4 | 0.504 | 0.417 | 0.976 | 1.013 | 0.0619 | 0.0261 | 0.461 | 0.995 |
| AD-35601.4 | 0.834 | 0.338 | 1.076 | 0.938 | 0.3513 | 0.0973 | 0.586 | 1.007 |
| AD-35607.4 | 0.597 | 0.419 | 1.000 | 1.074 | 0.1256 | 0.0523 | 0.508 | 1.037 |

**Table 6. IC50 data: KRAS fold change values**

| SD = standard deviation, Avg = average | | | | |
|---|---|---|---|---|
| **Duplex** | **IC50_bio1** | **IC50_bio2** | **AvgIC50** | **SD** |
| **AD-39731.1** | 0.020 | 0.003 | 0.012 | 0.0122 |
| **AD-39706.1** | 0.004 | 0.000 | 0.002 | 0.0028 |
| **AD-39720.1** | 0.012 | 0.002 | 0.007 | 0.0070 |
| **AD-39741.1** | 0.002 | 0.000 | 0.001 | 0.0015 |
| **AD-39738.1** | 0.002 | 0.001 | 0.002 | 0.0008 |
| **AD-39870.1** | 0.159 | 0.069 | 0.114 | 0.0636 |
| **AD-39845.1** | 0.056 | 0.015 | 0.036 | 0.0294 |
| **AD-39829.1** | 0.133 | 0.027 | 0.080 | 0.0750 |
| **AD-39778.1** | 0.135 | 0.040 | 0.088 | 0.0670 |
| **AD-39865.1** | 0.179 | 0.038 | 0.109 | 0.0996 |
| **AD-39793.1** | 0.003 | 0.078 | 0.041 | 0.0526 |
| **AD-39785.1** | 0.004 | 0.014 | 0.009 | 0.0068 |

(continued)

| SD = standard deviation, Avg = average | | | | |
|---|---|---|---|---|
| **Duplex** | **IC50_bio1** | **IC50_bio2** | **AvgIC50** | **SD** |
| **AD-40068.1** | 0.010 | 0.002 | 0.006 | 0.0052 |
| **AD-40008.1** | 0.041 | 0.011 | 0.026 | 0.0217 |
| **AD-40026.1** | 0.014 | 0.009 | 0.012 | 0.0036 |
| **AD-40061.1** | 0.014 | 0.004 | 0.009 | 0.0068 |
| **AD-40036.1** | 0.040 | 0.013 | 0.027 | 0.0196 |
| **AD-40057.1** | 0.030 | 0.111 | 0.071 | 0.0577 |
| **AD-35581.4** | 0.017 | 0.027 | 0.022 | 0.0071 |
| **AD-35616.4** | 0.032 | 0.044 | 0.038 | 0.0091 |
| **AD-35588.4** | 0.036 | 0.060 | 0.048 | 0.0164 |
| **AD-35604.4** | 0.088 | 0.373 | 0.231 | 0.2015 |
| **AD-35592.4** | 0.086 | 0.266 | 0.176 | 0.1270 |
| **AD-35685.1** | 0.036 | 0.019 | 0.027 | 0.0120 |
| **AD-38159.1** | 0.093 | 0.096 | 0.095 | 0.0027 |
| **AD-38139.1** | 0.092 | 0.354 | 0.223 | 0.1849 |
| **AD-38172.1** | 0.130 | 0.180 | 0.155 | 0.0352 |
| **AD-38134.1** | 0.120 | 0.199 | 0.159 | 0.0557 |
| **AD-38161.1** | 0.109 | 0.173 | 0.141 | 0.0448 |
| **AD-38131.1** | 0.134 | 0.374 | 0.254 | 0.1699 |
| **AD-38128.1** | 0.014 | 0.070 | 0.042 | 0.0394 |
| **AD-38151.1** | 0.093 | 0.315 | 0.204 | 0.1571 |
| **AD-38127.1** | 0.064 | 0.089 | 0.077 | 0.0177 |
| **AD-38154.1** | 0.041 | 0.284 | 0.163 | 0.1721 |
| **AD-38133.1** | 0.036 | 0.203 | 0.120 | 0.1182 |
| **AD-38170.1** | 0.140 | 0.332 | 0.236 | 0.1352 |
| **AD-38167.1** | 0.247 | 0.262 | 0.255 | 0.0104 |
| **AD-39735.1** | 0.060 | 0.034 | 0.047 | 0.0186 |
| **AD-39858.1** | 0.040 | 0.037 | 0.039 | 0.0016 |
| **AD-39781.1** | 0.009 | 0.016 | 0.013 | 0.0043 |
| **AD-39841.1** | 0.420 | 0.242 | 0.331 | 0.1255 |
| **AD-39805.1** | 0.057 | 0.073 | 0.065 | 0.0114 |
| **AD-39869.1** | 0.182 | 0.142 | 0.162 | 0.0281 |
| **AD-39790.1** | 0.143 | 0.059 | 0.101 | 0.0595 |
| **AD-40044.1** | 0.019 | 0.057 | 0.038 | 0.0265 |
| **AD-40049.1** | 0.017 | 0.039 | 0.028 | 0.0154 |
| **AD-40005.1** | 0.013 | 0.066 | 0.039 | 0.0372 |
| **AD-40077.1** | 0.049 | 0.241 | 0.145 | 0.1358 |
| **AD-40085.1** | 0.039 | 0.488 | 0.264 | 0.3173 |

(continued)

| SD = standard deviation, Avg = average | | | | |
|---|---|---|---|---|
| **Duplex** | **IC50_bio1** | **IC50_bio2** | **AvgIC50** | **SD** |
| **AD-40019.1** | 0.103 | 0.340 | 0.222 | 0.1677 |
| **AD-40083.1** | 0.108 | 0.426 | 0.267 | 0.2253 |
| **AD-35609.4** | 0.055 | 0.055 | 0.055 | 0.0001 |
| **AD-35679.1** | 0.113 | 0.209 | 0.161 | 0.0673 |
| **AD-35600.1** | 0.046 | 0.027 | 0.037 | 0.0134 |
| **AD-35606.1** | 0.045 | 0.043 | 0.044 | 0.0018 |
| **AD-38136.1** | 0.035 | 0.043 | 0.039 | 0.0061 |
| **AD-38163.1** | 0.019 | 0.016 | 0.018 | 0.0017 |
| **AD-38155.1** | 0.236 | 0.220 | 0.228 | 0.0118 |
| **AD-38145.1** | 0.111 | 0.230 | 0.171 | 0.0842 |
| **AD-38138.1** | 0.260 | 0.675 | 0.468 | 0.2933 |
| Bio1: biological replicates 1 and 2; residual mRNA level relative to control. | | | | |

**TABLE 6.1. Efficacy of particular KRAS siRNAs.**

| Various KRAS siRNAs were tested for efficacy in RKO cells in vitro. Results are provided in Table 6.1, below. Provided are the residual gene activity at 10 nM (Column 2) and 0.1 nM (Column 3). For example, for AD-39720.1, there was a residual gene activity of 0.2, or 20% (indicating 80% gene knockdown) at 10 nM, and 0.55 or 55% (45% gene knockdown) at 0.1 nM. IC50 of knockdown in nM in MiaPaca 2 cells is also provided. | | | |
|---|---|---|---|
| **K-RAS siRNA** | **10nM RK0** | **0.1nM RKO** | **knockdown IC50 MiaPaca 2** |
| AD-35600.1 | 0.3 | 0.27 | 0.0934 |
| AD-35609.4 | 0.17 | 0.41 | 0.037049 |
| AD-38136.1 | 0.07 | 0.14 | 0.0346 |
| AD-38159.1 | 0.14 | 0.22 | 0.01861 |
| AD-38163.1 | 0.09 | 0.16 | 0.0383 |
| AD-39720.1 | 0.2 | 0.55 | 0.005496 |
| AD-39735.1 | 0.21 | 0.37 | 0.0473 |
| AD-39741.1 | 0.18 | 0.67 | 0.0394 |
| AD-39760.1 | | | 0.0806 |
| AD-39778.1 | 0.23 | 0.43 | 0.040545 |
| AD-39822.1 | | | 1.3242 |
| AD-39845.1 | 0.2 | 0.3 | 0.020622 |
| AD-39870.1 | 0.25 | 0.12 | 0.026542 |
| AD-40008.1 | 0.19 | 0.37 | 0.033536 |
| AD-40021.1 | | | 0.1217 |
| AD-40061.1 | 0.19 | 0.44 | 0.009348 |
| AD-40068.1 | 0.15 | 0.33 | 0.009477 |
| AD-40072.1 | | | 0.2838 |

**TABLE 6.2. Efficacy of particular KRAS siRNAs.**

Table 6.2, below, provides additional data pertaining to activity of particular KRAS siRNAs. The generic target sequence is provided. Proliferation was tested in vitro in MiaPaca2 cells, which are KRAS mutant and are therefore K-RAS dependent. IC50 was studied, using test concentrations from 0 to 10 nM. IC50 data are provided. For example, in the first row, siRNA AD-39822.1 has an IC50 of 1.3242 nM.

Proliferation was tested in both BXPC3 and NCIH28 cells, which are both Wild type for KRAS and are therefore KRAS independent. IC50 was studied, using test concentrations of 0 to 10 nM. In these studies, >10 nM IC50 indicates that the efficacy of KRAS siRNAs was specific to KRAS (little or no substantial off-target effects).

| AlnyID | Nicknames | Guide 19mer generic | SEQ ID NO: | Proliferation BXPC3 | Proliferation NCIH28 | IC50 MiaPaca2 |
|---|---|---|---|---|---|---|
| AD-35600. 1 | NA | TCTCTATAATGGTGAA TAT | 2742 | >10 | >10 | 0.0934 |
| AD-35609.4 | NA | AAATACACAAAGAAA GCCC | 2743 | >10 | >10 | 0.037049395 |
| AD-38136.1 | NA | TTTATTTCCTACTAGG ACC | 2744 | >10 | >10 | 0.0346 |
| AD-38159.1 | hs KRAS 528 A22S26 | TTTCCTACTAGGACC ATAG | 2745 | >10 | >10 | 0.01861 |
| AD-38163.1 | NA | TTTAATTTGTTCTCTA TAA | 2746 | >10 | >10 | 0.0383 |
| AD-39720.1 | NA | AGCTCCAACTACCAC AAGT | 2747 | >10 | >10 | 0.005495521 |
| AD-39735.1 | hs KRAS 321 A22S26 | TCAATTACTACTTGCT TCC | 2748 | >10 | >10 | 0.0473 |
| AD-39741.1 | hs KRAS 322 A22S26 | ATCAATTACTACTTGC TTC | 2749 | >10 | >10 | 0.0394 |
| AD-39760.1 | NA | ATTAGCTGTATCGTC AAGG | 2750 | >10 | >10 | 0.0806 |
| AD-39778.1 | NA | TTCTCCATCAATTACT ACT | 2751 | >10 | >10 | 0.040544532 |
| AD-39822.1 | NA | TCGAGAATATCCAAG AGAC | 2752 | >10 | >10 | 1.3242 |
| AD-39845.1 | NA | GATTTAGTATTATTTA TGG | 2753 | >10 | >10 | 0.020622331 |
| AD-39870.1 | NA | ATATCTTCAAATGATT TAG | 2754 | >10 | >10 | 0.026542015 |
| AD-40008.1 | NA | TCTAGAAGGCAAATC ACAT | 2755 | >10 | >10 | 0.033536261 |
| AD-40021.1 | NA | TGTCTACTGTTCTAGA AGG | 2756 | >10 | >10 | 0.1217 |
| AD-40061.1 | NA | AATTCCATAACTTCTT GCT | 2757 | >10 | >10 | 0.00934829 |
| AD-40068.1 | NA | AATAAAAGGAATTCC ATAA | 2758 | >10 | >10 | 0.009476662 |

(continued)

| AlnyID | Nicknames | Guide 19mer generic | SEQ ID NO: | Proliferation BXPC3 | Proliferation NCIH28 | IC50 MiaPaca2 |
|---|---|---|---|---|---|---|
| AD-40072.1 | NA | ATAACTTCTTGCTAAG TCC | 2759 | >10 | >10 | 0.2838 |

## EQUIVALENTS

A composition of embodiment 1 is a composition comprising a RNAi agent comprising a sense strand and an antisense strand, wherein the antisense strand comprises at least 15 contiguous nucleotides differing by 0, 1, 2, or 3 nucleotides from the antisense strand of a RNAi agent specific to KRAS provided in Table 1.

The composition of embodiment 1, wherein the composition further comprises a second RNAi agent to KRAS.

The composition of embodiment 1, wherein the antisense strand is 30 or fewer nucleotides in length.

The composition of embodiment 1, wherein the sense strand and the antisense strand form a duplex region 15 to 30 nucleotide pairs in length.

The composition of embodiment 1, wherein the antisense strand and the sense strand are independently 19 to 23 nucleotides in length.

The composition of embodiment 1, wherein the RNAi agent comprises a modification that causes the RNAi agent to have increased stability in a biological sample or environment.

The composition of embodiment 1, wherein the RNAi agent comprises at least one modified backbone and/or at least one 2'-modified nucleotide.

The composition of embodiment 1, wherein the RNAi agent comprises:

a) at least one 5'-uridine-adenine-3' (5'-ua-3') dinucleotide, wherein the uridine is a 2'-modified nucleotide; and/or
b) at least one 5'-uridine-guanine-3' (5'-ug-3') dinucleotide, wherein the 5'-uridine is a 2'-modified nucleotide; and/or
c) at least one 5'-cytidine-adenine-3' (5'-ca-3') dinucleotide, wherein the 5'-cytidine is a 2'-modified nucleotide; and/or
d) at least one 5'-uridine-uridine-3' (5'-uu-3') dinucleotide, wherein the 5'-uridine is a 2'-modified nucleotide.

The composition of embodiment 1, wherein the RNAi agent comprises a 2'-modification selected from the group consisting of: 2'-deoxy, 2'-deoxy-2'-fluoro, 2'-O-methyl, 2'-O-methoxyethyl (2'-O-MOE), 2'-O-aminopropyl (2'-O-AP), 2'-O-dimethylaminoethyl (2'-O-DMAOE), 2'-O-dimethylaminopropyl (2'-O-DMAP), 2'-O-dimethylaminoethyloxyethyl (2'-O-DMAEOE), and 2'-O-N-methylacetamido (2'-O-NMA).

The composition of embodiment 1, wherein the RNAi agent comprises a blunt end.

The composition of embodiment 1, wherein the RNAi agent comprises an overhang having 1 to 4 unpaired nucleotides.

The composition of embodiment 1, wherein the RNAi agent comprises an overhang at the 3'-end of the antisense strand of the RNAi agent.

The composition of embodiment 1, wherein the RNAi agent is ligated to one or more agent selected from: one or more diagnostic compound, reporter group, cross-linking agent, nuclease-resistance conferring moiety, natural or unusual nucleobase, lipophilic molecule, cholesterol, lipid, lectin, steroid, uvaol, hecigenin, diosgenin, terpene, triterpene, sarsasapogenin, Friedelin, epifriedelanol-derivatized lithocholic acid, vitamin, carbohydrate, dextran, pullulan, chitin, chitosan, synthetic carbohydrate, oligo lactate 15-mer, natural polymer, low- or medium-molecular weight polymer, inulin, cyclodextrin, hyaluronic acid, protein, protein-binding agent, integrin-targeting molecule, polycationic, peptide, polyamine, peptide mimic, and/or transferrin.

The composition of embodiment 1, wherein the RNAi agent is capable of inhibiting expression of KRAS by at least about 60% at a concentration of 10 nM in RKO cells in vitro.

The composition of embodiment 1, wherein the RNAi agent is capable of inhibiting expression of KRAS by at least about 70% at a concentration of 10 nM in RKO cells in vitro.

The composition of embodiment 1, wherein the RNAi agent is capable of inhibiting expression of KRAS by at least about 80% at a concentration of 10 nM in RKO cells in vitro.

The composition of embodiment 1, wherein the RNAi agent is capable of inhibiting expression of KRAS by at least about 90% at a concentration of 10 nM in RKO cells in vitro.

The composition of embodiment 1, wherein the RNAi agent has an EC50 of no more than about 0.1 nM.

The composition of embodiment 1, wherein the RNAi agent has an EC50 of no more than about 0.01 nM.

The composition of embodiment 1, wherein the RNAi agent has an EC50 of no more than about 0.001 nM.

A composition of embodiment 2 comprising a RNAi agent comprising a first strand and a second strand, wherein the first strand and second strand comprise at least 15 contiguous nucleotides differing by 0, 1, 2, or 3 nucleotides from the first and second strand, respectively, of a RNAi agent specific to KRAS provided in Table 1.

The composition of embodiment 2, wherein the composition comprises a second RNAi agent to KRAS.

The composition of embodiment 2, wherein the second strand is 30 or fewer nucleotides in length.

The composition of embodiment 2, wherein the first strand and the second strand form a duplex region 15 to 30 nucleotide pairs in length.

The composition of embodiment 2, wherein the first strand and the second strand are independently 19 to 23 nucleotides in length.

The composition of embodiment 2, wherein the RNAi agent comprises a modification that causes the RNAi agent to have increased stability in a biological sample or environment.

The composition of embodiment 2, wherein the RNAi agent comprises a phosphorothioate and/or a 2'-modified nucleotide.

The composition of embodiment 2, wherein the RNAi agent comprises:

at least one 5'-uridine-adenine-3' (5'-ua-3') dinucleotide, wherein the uridine is a 2'-modified nucleotide; and/or at least one 5'-uridine-guanine-3' (5'-ug-3') dinucleotide, wherein the 5'-uridine is a 2'-modified nucleotide;
and/or at least one 5'-cytidine-adenine-3' (5'-ca-3') dinucleotide, wherein the 5'-cytidine is a 2'-modified nucleotide;
and/or at least one 5'-uridine-uridine-3' (5'-uu-3') dinucleotide, wherein the 5'-uridine is a 2'-modified nucleotide.

The composition of embodiment 2, wherein the RNAi agent comprises one or more 2'-modifications selected from the group consisting of:
2'-deoxy, 2'-deoxy-2'-fluoro, 2'-O-methyl, 2'-O-methoxyethyl (2'-O-MOE), 2'-O-aminopropyl (2'-O-AP), 2'-O-dimethylaminoethyl (2-O-DMAOE), 2'-O-dimethylaminopropyl (2'-O-DMAP), 2'-O-dimethylaminoethyloxyethyl (2'-O-DMAEOE), and 2'-O-N-methylacetamido (2'-O-NMA).

The composition of embodiment 2, wherein the RNAi agent comprises a blunt end.

The composition of embodiment 2, wherein the RNAi agent comprises an overhang having 1 to 4 unpaired nucleotides.

The composition of embodiment 2, wherein the RNAi agent comprises an overhang at the 3'-end of the antisense strand.

The composition of embodiment 2, wherein the RNAi agent is ligated to one or more agents, the agent selected from a: one or more diagnostic compound, reporter group, cross-linking agent, nuclease-resistance conferring moiety, natural or unusual nucleobase, lipophilic molecule, cholesterol, lipid, lectin, steroid, uvaol, hecigenin, diosgenin, terpene, triterpene, sarsasapogenin, Friedelin, epifriedelanol-derivatized lithocholic acid, vitamin, carbohydrate, dextran, pullulan, chitin, chitosan, synthetic carbohydrate, oligo lactate 15-mer, natural polymer, low- or medium-molecular weight polymer, inulin, cyclodextrin, hyaluronic acid, protein, protein-binding agent, integrin-targeting molecule, polycationic, peptide, polyamine, peptide mimic, and/or transferrin.

The composition of embodiment 2, wherein the RNAi agent is capable of inhibiting expression of KRAS by at least about 60% at a concentration of 10 nM in RKO cells in vitro.

The composition of embodiment 2, wherein the RNAi agent is capable of inhibiting expression of KRAS by at least about 70% at a concentration of 10 nM in RKO cells in vitro.

The composition of embodiment 2, wherein the RNAi agent is capable of inhibiting expression of KRAS by at least about 80% at a concentration of 10 nM in RKO cells in vitro.

The composition of embodiment 2, wherein the RNAi agent is capable of inhibiting expression of KRAS by at least about 90% at a concentration of 10 nM in RKO cells in vitro.

The composition of embodiment 2, wherein the RNAi agent has an EC50 of no more than about 0.1 nM.

The composition of embodiment 2, wherein the RNAi agent has an EC50 of no more than about 0.01 nM in MiaPaca2 cells.

The composition of embodiment 2, wherein the RNAi agent has an EC50 of no more than about 0.001 nM in MiaPaca2 cells.

A method of embodiment 3 is a method comprising a method of treating a KRAS-related disease in an individual, comprising the step of administering to the individual a therapeutically effective amount of a composition comprising a RNAi agent comprising a sense strand and an antisense strand, wherein the antisense strand comprises at least 15 contiguous nucleotides differing by 0, 1, 2, or 3 nucleotides from the antisense strand of a RNAi agent specific to KRAS provided in Table 1.

The method of embodiment 3, wherein the KRAS-related disease is a proliferative disease, cardio-facio-cutaneous (CFC) syndrome or Noonan syndrome..

The method of embodiment 3, wherein the KRAS-related disease is a proliferative disease, cardio-facio-cutaneous (CFC) syndrome or Noonan syndrome.

The method of embodiment 3, wherein the method further comprises the step of administering an additional treatment for a proliferative disease, cardio-facio-cutaneous (CFC) syndrome or Noonan syndrome.

The method of embodiment 3, wherein the method further comprises the step of administering an additional treatment for a proliferative disease, including without limitation a solid or liquid cancer, adenocarcinoma, colorectal cancer, advanced and/or metastatic colorectal cancer, colon cancer, lung, non-small cell lung cancer and lung adenocarcinoma, acute myelogenous lung, bladder, brain, breast, cervical, endometrial, gastric, head and neck, kidney, leukemia, myelodysplastic syndrome, myeloid leukemia, liver, melanoma, ovarian, pancreatic, prostate, testicular, thyroid cancers, or cardio-facio-cutaneous (CFC) syndrome or Noonan syndrome.

The method of embodiment 3, wherein the composition comprises a second RNAi agent to KRAS.

The method of embodiment 3, wherein the method further comprises the step of administering an additional RNAi agent to KRAS.

The method of embodiment 3, further comprising the administration of an additional treatment.

The method of embodiment 3, wherein the additional treatment is a composition.

The method of embodiment 3, wherein the additional treatment is a method.

The method of embodiment 3, wherein the additional treatment and the RNAi agent can be administered in any order.

A method of embodiment 4 is a method comprising a method of inhibiting the expression of KRAS in an individual, comprising the step of administering to the individual a therapeutically effective amount of a composition comprising a RNAi agent comprising a sense strand and an antisense strand, wherein the antisense strand comprises at least 15 contiguous nucleotides differing by 0, 1, 2, or 3 nucleotides from the antisense strand of a RNAi agent specific to KRAS provided in Table 1.

The method of embodiment 4, wherein the individual is afflicted with or susceptible to a KRAS-related disease.

The method of embodiment 4, wherein the KRAS-related disease is a proliferative disease, cardio-facio-cutaneous (CFC) syndrome or Noonan syndrome.

The method of embodiment 4, wherein the KRAS-related disease is a proliferative disease.

The method of embodiment 4, wherein the KRAS-related disease is a proliferative disease, including without limitation a solid or liquid cancer, adenocarcinoma, colorectal cancer, advanced and/or metastatic colorectal cancer, colon cancer, lung, non-small cell lung cancer and lung adenocarcinoma, acute myelogenous lung, bladder, brain, breast, cervical, endometrial, gastric, head and neck, kidney, leukemia, myelodysplastic syndrome, myeloid leukemia, liver, melanoma, ovarian, pancreatic, prostate, testicular, thyroid cancers, or cardio-facio-cutaneous (CFC) syndrome or Noonan syndrome

The method of embodiment 4, further comprising the administration of an additional treatment.

The method of embodiment 4, wherein the additional treatment is a composition.

The method of embodiment 4, wherein the additional treatment is a method.

The method of embodiment 4, wherein the additional treatment and the RNAi agent can be administered in any order.

A composition of embodiment 5 is a composition comprising a medicament for use in an RNAi formulation comprising a RNAi agent comprising a sense strand and an antisense strand, wherein the antisense strand comprises at least 15 contiguous nucleotides differing by 0, 1, 2, or 3 nucleotides from the antisense strand of a RNAi agent specific to KRAS provided in Table 1.

Any composition above in a pharmaceutically effective formulation.

The composition according to embodiment 5, for use in a method of treating a KRAS-related disease in an individual, the method comprising the step of administering to the individual a therapeutically effective amount of a composition according to embodiment 1.

The use of a composition according to embodiment 5, in the manufacture of a medicament for the treatment of a KRAS-related disease.

The use of a composition according to embodiment 5, in the manufacture of a medicament for the treatment of a proliferative disease, including without limitation a solid or liquid cancer, adenocarcinoma, colorectal cancer, advanced and/or metastatic colorectal cancer, colon cancer, lung, non-small cell lung cancer and lung adenocarcinoma, acute myelogenous lung, bladder, brain, breast, cervical, endometrial, gastric, head and neck, kidney, leukemia, myelodysplastic syndrome, myeloid leukemia, liver, melanoma, ovarian, pancreatic, prostate, testicular, thyroid cancers, or cardio-facio-cutaneous (CFC) syndrome or Noonan syndrome.

The use of embodiment 5, wherein the KRAS-related disease is a proliferative disease, cardio-facio-cutaneous (CFC) syndrome or Noonan syndrome.

The composition of embodiment 1, wherein all the pyrimidines are 2' O-methyl-modified nucleotides.

The composition of embodiment 1, wherein all the pyrimidines are 2' O-methyl-modified nucleotides.

Unless defined otherwise, the technical and scientific terms used herein have the same meaning as that usually understood by a specialist familiar with the field to which the disclosure belongs.

Unless indicated otherwise, all methods, steps, techniques and manipulations that are not specifically described in detail can be performed and have been performed in a manner known per se, as will be clear to the skilled person. Reference is for example again made to the standard handbooks and the general background art mentioned herein and to the further references cited therein.

**CLAUSES**

1. A composition comprising a RNAi agent comprising a sense strand and an antisense strand, wherein the antisense strand comprises at least 15 contiguous nucleotides differing by 0, 1, 2, or 3 nucleotides from the antisense strand of a RNAi agent specific to KRAS provided in Table 1.
2. The composition of clause 1, wherein the composition further comprises a second RNAi agent to KRAS.
3. The composition of clause 1, wherein the RNAi agent comprises at least one modified backbone and/or at least one 2'-modified nucleotide.
4. The composition of clause 1, wherein the RNAi agent is ligated to one or more agent selected from: diagnostic compound, reporter group, cross-linking agent, nuclease-resistance conferring moiety, natural or unusual nucleobase, lipophilic molecule, cholesterol, lipid, lectin, steroid, uvaol, hecigenin, diosgenin, terpene, triterpene, sarsasapogenin, Friedelin, epifriedelanol-derivatized lithocholic acid, vitamin, carbohydrate, dextran, pullulan, chitin, chitosan, synthetic carbohydrate, oligo lactate 15-mer, natural polymer, low- or medium-molecular weight polymer, inulin, cyclodextrin, hyaluronic acid, protein, protein-binding agent, integrin-targeting molecule, polycationic, peptide, polyamine, peptide mimic, and/or transferrin.
5. A composition comprising a RNAi agent comprising a first strand and a second strand, wherein the first strand and second strand comprise at least 15 contiguous nucleotides differing by 0, 1, 2, or 3 nucleotides from the first and second strand, respectively, of a RNAi agent specific to KRAS provided in Table 1.
6. The composition of clause 1, wherein the composition comprises a second RNAi agent to KRAS.
7. The composition of clause 1, wherein the RNAi agent comprises a phosphorothioate and/or a 2'-modified nucleotide.
8. The composition of clause 1, wherein the RNAi agent is ligated to one or more agents, the agent selected from a: diagnostic compound, reporter group, cross-linking agent, nuclease-resistance conferring moiety, natural or unusual nucleobase, lipophilic molecule, cholesterol, lipid, lectin, steroid, uvaol, hecigenin, diosgenin, terpene, triterpene, sarsasapogenin, Friedelin, epifriedelanol-derivatized lithocholic acid, vitamin, carbohydrate, dextran, pullulan, chitin, chitosan, synthetic carbohydrate, oligo lactate 15-mer, natural polymer, low- or medium-molecular weight polymer, inulin, cyclodextrin, hyaluronic acid, protein, protein-binding agent, integrin-targeting molecule, polycationic, peptide, polyamine, peptide mimic, and/or transferrin.
9. A method of treating a KRAS-related disease in an individual, comprising the step of administering to the individual a therapeutically effective amount of a composition comprising a RNAi agent comprising a sense strand and an antisense strand, wherein the antisense strand comprises at least 15 contiguous nucleotides differing by 0, 1, 2, or 3 nucleotides from the antisense strand of a RNAi agent specific to KRAS provided in Table 1.
10. The method of clause 9, wherein the KRAS-related disease is a proliferative disease, cardio-facio-cutaneous (CFC) syndrome or Noonan syndrome.
11. The method of clause 9, wherein the method further comprises the step of administering an additional treatment for a proliferative disease, cardio-facio-cutaneous (CFC) syndrome or Noonan syndrome.
12. The method of clause 11, wherein the method further comprises the step of administering an additional RNAi agent to KRAS.
13. A method of inhibiting the expression of KRAS in an individual, comprising the step of administering to the individual a therapeutically effective amount of a composition comprising a RNAi agent comprising a sense strand and an antisense strand, wherein the antisense strand comprises at least 15 contiguous nucleotides differing by 0, 1, 2, or 3 nucleotides from the antisense strand of a RNAi agent specific to KRAS provided in Table 1.
14. The method of clause 13, wherein the KRAS-related disease isa proliferative disease, cardio-facio-cutaneous (CFC) syndrome or Noonan syndrome.
15. A medicament for use in an RNAi formulation comprising a RNAi agent comprising a sense strand and an antisense strand, wherein the antisense strand comprises at least 15 contiguous nucleotides differing by 0, 1, 2, or 3 nucleotides from the antisense strand of a RNAi agent specific to KRAS provided in Table 1.
16. Any composition above in a pharmaceutically effective formulation.
17. The composition according to clause 1, for use in a method of treating a KRAS-related disease in an individual, the method comprising the step of administering to the individual a therapeutically effective amount of a composition according to claim 1.

18. The use of a composition according to clause 1, in the manufacture of a medicament for the treatment of a KRAS-related disease.
19. The composition of clause 1, wherein all the pyrimidines are 2' O-methyl-modified nucleotides.
20. The composition of clause 5, wherein all the pyrimidines are 2' O-methyl-modified nucleotides.
21. The composition of clause 1, wherein the RNAi agent has an EC50 of no more than about 1 nM in MiaPaca2 cells.
22. The composition of clause 1, wherein the RNAi agent is capable of inhibiting expression of KRAS by at least about 70% at a concentration of 10 nM in RKO cells in vitro.

## Claims

**1.** A composition comprising a RNAi agent comprising a first strand and a second strand, wherein the first strand is about 17-23 nucleotides in length and comprises at least 15 contiguous nucleotides of the sequence of SEQ ID NO: 554 or a modified or unmodified variant thereof, and the second strand is about 17-23 nucleotides in length and comprises at least 15 continguous nucleotides of the sequence of SEQ ID NO: 127 or a modified or unmodified variant thereof.

**2.** A composition comprising a RNAi agent comprising a first strand and a second strand, wherein the first strand and second strand are each independently 19-23 nucleotides in length, and the first strand comprises the nucleotide sequence of SEQ ID NO: 554 or a modified or unmodified variant thereof, and the second strand comprises the nucleotide sequence of SEQ ID NO: 127 or a modified or unmodified variant thereof.

**3.** The composition of claim 1 or 2, wherein the composition comprises a second RNAi agent to KRAS.

**4.** The composition of claim 1 or 2, wherein the RNAi agent comprises one or more phosphorothioates.

**5.** The composition of claim 1 or 2, wherein the RNAi agent comprises one or more 2'-modified nucleotides.

**6.** The composition of claim 1 or 2, wherein the RNAi agent comprises one or more phosphorothioates and one or more 2'-modified nucleotides.

**7.** The composition of claim 5 or 6 wherein the one or more 2'-modified nucleotides are independently 2'-deoxy, 2'-deoxy-2'-fluoro, 2'-O-methyl, 2'-O-methoxyethyl (2-O-MOE), 2'-O-aminopropyl (2'-O-AP), 2'-O-dimethylaminoethyl (2-O-DMAOE), 2-O-dimethylaminopropyl (2'-O-DMAP), 2'-O-dimethylaminoethyloxyethyl (2'-O-DMAEOE), or 2'-O-N-methylacetamido (2'-O-NMA).

**8.** The composition of any of claims 5-7 wherein all the pyrimidines are 2'-O-methyl-modified nucleotides.

**9.** The composition of any of claims 1-7, wherein the RNAi agent is ligated to one or more agents, the agent selected from a: diagnostic compound, reporter group, cross-linking agent, nuclease-resistance conferring moiety, natural or unusual nucleobase, lipophilic molecule, cholesterol, lipid, lectin, steroid, uvaol, hecigenin, diosgenin, terpene, triterpene, sarsasapogenin, Friedelin, epifriedelanol-derivatized lithocholic acid, vitamin, carbohydrate, dextran, pullulan, chitin, chitosan, synthetic carbohydrate, oligo lactate 15-mer, natural polymer, low- or medium-molecular weight polymer, inulin, cyclodextrin, hyaluronic acid, protein, protein-binding agent, integrin-targeting molecule, polycationic, peptide, polyamine, peptide mimic, and/or transferrin.

**10.** A medicament comprising a RNAi agent comprising a first strand and a second strand, wherein the first strand is about 17-23 nucleotides in length and comprises at least 15 contiguous nucleotides of the sequence of SEQ ID NO: 554 or a modified or unmodified variant thereof, and the second strand is about 17-23 nucleotides in length and comprises at least 15 continguous nucleotides of the sequence of SEQ ID NO: 127 or a modified or unmodified variant thereof.

**11.** Any composition above in a pharmaceutically effective formulation.

**12.** The composition according to claim 1 or 2, for use in treatment of a KRAS-related disease in an individual.

**13.** A composition of claim 1 or 2, wherein the first strand comprises SEQ ID NO: 1408 and the second strand comprises SEQ ID NO: 981.

**14.** A composition of claim 1 or 2, wherein the first strand is SEQ ID NO: 1408 and the second strand is SEQ ID NO: 981.

U002

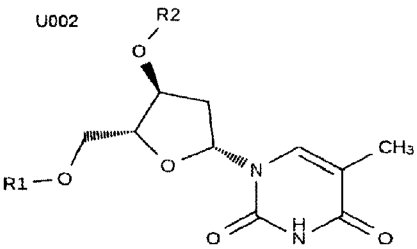

U003

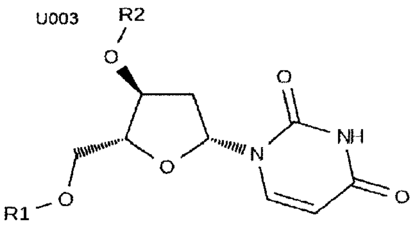

U004

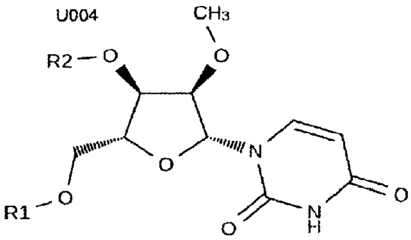

U005

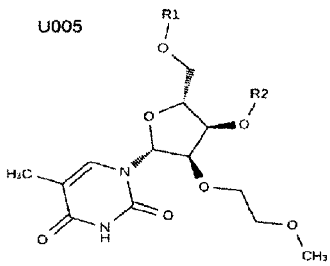

C004

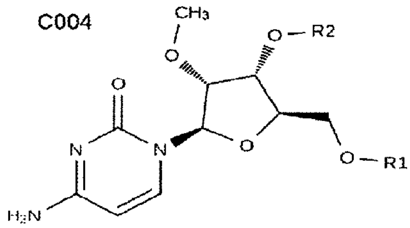

C005

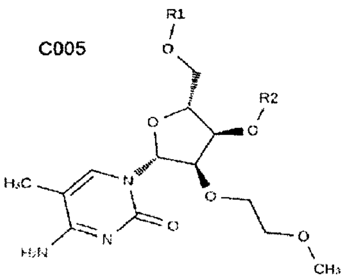

A004

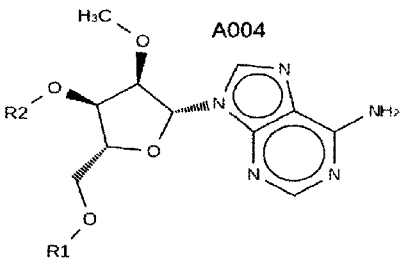

A005

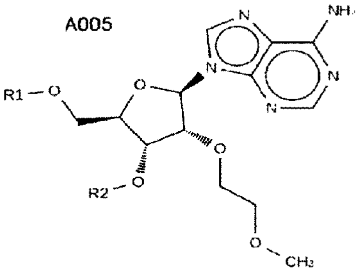

G004

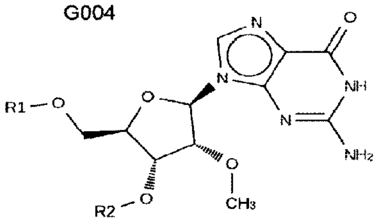

G005

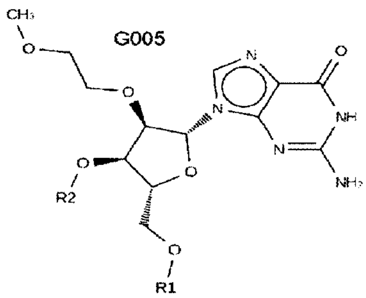

FIG.1

Europäisches Patentamt
European Patent Office
Office européen des brevets

# EUROPEAN SEARCH REPORT

Application Number
EP 20 17 8501

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (IPC) |
| X<br><br><br>A | WO 2009/108217 A2 (INTRADIGM CORP [US]; XIE FRANK Y [US]; YANG XIAODONG [US]; LIU YING [U) 3 September 2009 (2009-09-03)<br>* sequences 437-442 *<br>* the whole document *<br>----- | 1-12<br><br><br>13,14 | INV.<br>C12N15/113<br>A61P35/00<br>A61K31/713 |
| X<br><br><br><br><br><br><br><br><br>A | STEFANIE DERER ET AL: "Oncogenic KRAS impairs EGFR antibodies' efficiency by C/EBPbeta-dependent suppression of EGFR expression",<br>NEOPLASIA (NEW YORK, N.Y.),<br>vol. 14, no. 3, 1 March 2012 (2012-03-01), page 190, XP055136911,<br>Canada<br>DOI: 10.1593/neo.111636<br>* the whole document *<br>----- | 1-12<br><br><br><br><br><br><br><br><br>13,14 | |
| X<br><br><br><br><br><br><br><br><br>A | BARBIE DAVID A ET AL: "Systematic RNA interference reveals that oncogenic KRAS-driven cancers require TBK1",<br>NATURE, NATURE PUBLISHING GROUP UK, LONDON,<br>vol. 462, no. 7269,<br>21 October 2009 (2009-10-21), pages 108-112, 2pp, XP009133782,<br>ISSN: 0028-0836, DOI: 10.1038/NATURE08460<br>* the whole document *<br>-& DAVID A. BARBIE ET AL: "Supplementary Information - Systematic RNA interference reveals that oncogenic KRAS-driven cancers require TBK1",<br>NATURE,<br>vol. 462, no. 7269,<br>21 October 2009 (2009-10-21), pages 108-112, XP055735498,<br>ISSN: 0028-0836, DOI: 10.1038/nature08460<br>* table S5 *<br>-----<br>-/-- | 1-12<br><br><br><br><br><br><br><br><br>13,14 | TECHNICAL FIELDS SEARCHED (IPC)<br>C12N<br>A61K |

The present search report has been drawn up for all claims

| Place of search | Date of completion of the search | Examiner |
|---|---|---|
| The Hague | 30 September 2020 | Spindler, Mark-Peter |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 1 of 2

Europäisches Patentamt
European Patent Office
Office européen des brevets

# EUROPEAN SEARCH REPORT

Application Number
EP 20 17 8501

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2010/115202 A2 (DICERNA PHARMACEUTICALS INC [US]; BROWN BOB D [US]) 7 October 2010 (2010-10-07) * the whole document * ----- | 1-14 | |
| A | WO 2010/115206 A2 (DICERNA PHARMACEUTICALS INC [US]; BROWN BOB D [US]) 7 October 2010 (2010-10-07) * the whole document * ----- | 1-14 | |
| A | WO 2005/040379 A2 (SIRNA THERAPEUTICS INC [US]; MCSWIGGEN JAMES [US]) 6 May 2005 (2005-05-06) * the whole document * ----- | 1-14 | |
| A | WO 2011/110671 A2 (MAX PLANCK GESELLSCHAFT [DE]; KUMAR GURUMURTHY RAJENDRA [DE]; MAEURER) 15 September 2011 (2011-09-15) * figures 1, 2; example 1; table 1a * * the whole document * ----- | 1-14 | |
| | | | TECHNICAL FIELDS SEARCHED (IPC) |
| A | WO 2008/109516 A2 (MDRNA INC [US]; QUAY STEVEN C [US]; MCSWIGGEN JAMES [US]; VAISH NAREND) 12 September 2008 (2008-09-12) * claims 1-24; sequence 1862 * * the whole document * ----- | 1-14 | |

The present search report has been drawn up for all claims

2

| Place of search | Date of completion of the search | Examiner |
|---|---|---|
| The Hague | 30 September 2020 | Spindler, Mark-Peter |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

# ANNEX TO THE EUROPEAN SEARCH REPORT ON EUROPEAN PATENT APPLICATION NO. EP 20 17 8501

This annex lists the patent family members relating to the patent documents cited in the above-mentioned European search report. The members are as contained in the European Patent Office EDP file on
The European Patent Office is in no way liable for these particulars which are merely given for the purpose of information.

30-09-2020

| Patent document cited in search report | Publication date | Patent family member(s) | Publication date |
|---|---|---|---|
| WO 2009108217 A2 | 03-09-2009 | CA 2704737 A1 | 03-09-2009 |
| | | EP 2190995 A2 | 02-06-2010 |
| | | JP 2010538678 A | 16-12-2010 |
| | | US 2010286241 A1 | 11-11-2010 |
| | | WO 2009108217 A2 | 03-09-2009 |
| WO 2010115202 A2 | 07-10-2010 | NONE | |
| WO 2010115206 A2 | 07-10-2010 | AU 2010232410 A1 | 27-10-2011 |
| | | CA 2757613 A1 | 07-10-2010 |
| | | CN 102575254 A | 11-07-2012 |
| | | CN 103555722 A | 05-02-2014 |
| | | CN 104651362 A | 27-05-2015 |
| | | DK 2756845 T3 | 12-06-2017 |
| | | EP 2414374 A2 | 08-02-2012 |
| | | ES 2628144 T3 | 01-08-2017 |
| | | JP 6010458 B2 | 19-10-2016 |
| | | JP 2012531888 A | 13-12-2012 |
| | | KR 20120034610 A | 12-04-2012 |
| | | US 2011021604 A1 | 27-01-2011 |
| | | US 2013123342 A1 | 16-05-2013 |
| | | US 2015057337 A1 | 26-02-2015 |
| | | US 2018044680 A1 | 15-02-2018 |
| | | WO 2010115206 A2 | 07-10-2010 |
| WO 2005040379 A2 | 06-05-2005 | CA 2542835 A1 | 19-05-2005 |
| | | CA 2543013 A1 | 19-05-2005 |
| | | EP 1675949 A2 | 05-07-2006 |
| | | EP 1675953 A2 | 05-07-2006 |
| | | EP 1682661 A2 | 26-07-2006 |
| | | JP 2007522793 A | 16-08-2007 |
| | | JP 2008506351 A | 06-03-2008 |
| | | WO 2005040379 A2 | 06-05-2005 |
| | | WO 2005044981 A2 | 19-05-2005 |
| | | WO 2005045035 A2 | 19-05-2005 |
| WO 2011110671 A2 | 15-09-2011 | EP 2544671 A2 | 16-01-2013 |
| | | US 2013116302 A1 | 09-05-2013 |
| | | WO 2011110671 A2 | 15-09-2011 |
| WO 2008109516 A2 | 12-09-2008 | CA 2679388 A1 | 12-09-2008 |
| | | EP 2121925 A2 | 25-11-2009 |
| | | JP 2010519912 A | 10-06-2010 |
| | | US 2010055783 A1 | 04-03-2010 |
| | | WO 2008109516 A2 | 12-09-2008 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2007107162 A
- WO 2005021749 A
- WO 2007128477 A
- WO 2008147824 A
- WO 0044914 A
- WO 0168836 A, Beach
- CA 2359180
- US 20090209626 A, Khvorova
- WO 02100435A1 A
- WO 03015757A1 PCT
- WO 04029213A2 PCT
- WO 2011076807 PCT
- US 5962016 A
- US 5030453 A
- US 6680068 B
- US 20040208921 A
- WO 04002453 A1
- US 20040204377 A
- US 20030012812 A
- US 20060240093
- US 20070135372 A
- WO 2009082817 A
- WO 2011076807 A


### Non-patent literature cited in the description


- **BOS et al.** *Cancer Res.,* 1989, vol. 49, 4682-4689
- **FORBES et al.** *Br. J. Cancer,* 2006, vol. 94, 318-322
- **ELBASHIR et al.** *Nature,* 2001, vol. 411, 494-498
- **ELBASHIR et al.** *EMBO J.,* 2001, vol. 20, 6877-6888
- **KRAYNACK et al.** *RNA,* 2006, vol. 12, 163-176
- **GOODSELL et al.** *Oncologist,* 1999, vol. 4 (3), 263-4
- **DOWNWARD.** *Nat. Rev. Cancer 3,* 2003, vol. 1, 11-22
- **MALUMBRES et al.** *Nat. Rev. Cancer,* 2003, vol. 3 (6), 459-65
- **CHANG et al.** *Proc. Natl. Acad. Sci. U.S.A.,* 1982, vol. 79 (16), 4848-52
- **HARVEY.** *Nature,* 1964, vol. 204 (4963), 1104-5
- **KIRSTEN et al.** *Bibl Haematol.,* 1970, vol. 36, 246-9
- **SANTOS et al.** *Nature,* 1982, vol. 298 (5872), 343-7
- **PARADA et al.** *Nature,* 1982, vol. 297 (5866), 474-8
- **DER et al.** *Proc. Nat. Acad. Sci.,* 1982, vol. 79, 3637-3640
- **CHANG et al.** *Proc. Nat. Acad. Sci.,* 1982, vol. 79, 4848-4852
- **MCCOY et al.** *Nature,* 1983, vol. 302, 79-81
- **MCGRATH et al.** *Nature,* 1983, vol. 304, 501-506
- **CARTA et al.** *Am. J. Hum. Genet.,* 2006, vol. 79, 129-135
- **GRIMMOND et al.** *Urol. Res.,* 1992, vol. 20, 121-126
- **BURMER et al.** *Proc. Natl. Acad. Sci.,* 1989, vol. 86, 2403-2407
- **ZENKER et al.** *J. Med. Genet.,* 2007, vol. 44, 131-135
- **ROTBLAT et al.** *Methods Enzymol,* 2008, vol. 439, 467-89
- **BLUM et al.** *Cancer Research,* 2005, vol. 65 (3), 999-1006
- **LODISH et al.** Cancer. Molecular cell biology. W.H. Freeman, 2000
- **SIOUD.** *J. Mol. Biol.,* 2005, vol. 348, 1079-1090
- **YAMATO et al.** *cancer Gene Ther.,* 2011, vol. 18, 587-597
- **HUTVAGNER et al.** *Science,* 2001, vol. 293, 834
- **SHARP et al.** *Genes Dev.,* 2001, vol. 15, 485
- **BERNSTEIN et al.** *Nature,* 2001, vol. 409, 363
- **NYKANEN et al.** *Cell,* 2001, vol. 107, 309
- **ELBASHIR et al.** *Genes Dev.,* 2001, vol. 15, 188
- **HUMMER et al.** *J. Am. Soc. Nephrol.,* 2005, vol. 16, 3160-3166
- **RANDRIANARISON et al.** *Am. J. Physiol. Lung Cell. Mol. Physiol.,* 2007, vol. 294, 409-416
- **CAO et al.** *Am. J. Physiol. Renal Physiol.,* 2006
- **GAMBLING et al.** *Kidney Intl.,* 2004, vol. 65, 1774-1781

- **PARRISH et al.** *Molecular Cell,* 2000, vol. 6, 1077-1087
- **HENSCHEL et al.** DEQOR: a web-based tool for the design and quality control of siRNAs. *Nucleic Acids Research,* 2004, vol. 32, W113-W120
- **USMAN ; CEDERGREN.** *TIBS,* 1992, vol. 17, 34
- **USMAN et al.** *Nucleic Acids Symp. Ser.,* 1994, vol. 31, 163
- **BURGIN et al.** *Biochemistry,* 1996, vol. 35, 14090
- **SOUTSCHEK et al.** *Nature,* 2004, vol. 432, 173-178
- **DOWLER et al.** *Nucl. Acids Res.,* 2006, vol. 34, 1669-1675
- **GAULTIER et al.** *Nucleic Acids. Res.,* 1987, vol. 15, 6625-6641
- **INOUE et al.** *Nucleic Acids Res.,* 1987, vol. 15, 6131-6148
- **INOUE et al.** *FEBS Lett.,* 1987, vol. 215, 327-330
- **YAMATO et al.** *Cancer Gene Ther.,* 2011, vol. 18, 587-597
- **SUN et al.** *Nature Biotech.,* 2008, vol. 26, 1379-1382
- **CHU ; RANA.** *RNA,* 2008, vol. 14, 1714-1719
- **OVCHARENKO D.** Efficient delivery of siRNAs to human primary cells. *Ambion TechNotes,* 2003, vol. 10 (5), 15-16
- **SONG et al.** *Nat Med.,* 10 February 2003
- **CAPLEN et al.** *Proc. Natl. Acad. Sci. (USA),* 2001, vol. 98, 9742-9747
- **MCCAFFREY et al.** *Nature,* vol. 414, 34-39
- **SONG et al.** *Nat Biotech.,* 2005, vol. 23, 709-717
- **SCHIFFELERS et al.** *Nucl. Acids Res.,* 2004, vol. 32 (el49), 141-1 10
- **BOS.** *Cancer Res.,* 1989, vol. 49 (17), 4682-9
- **ENGELMAN et al.** *Nature Med.,* 2008, vol. 14, 1351-1356
- **HAIGIS et al.** *Nature Gen.,* 2008, vol. 40, 600-608
- **KARAPETIS et al.** *N. E. J. Med.,* 2008, vol. 359, 1757-1765
- **KIARIS et al.** *Int. J. Oncol.,* 1995, vol. 7, 413-421
- **LIU et al.** *Nature,* 1987, vol. 330, 186-188
- **RIELY et al.** *Clin. Cancer Res.,* 2008, vol. 14, 5731
- **RIELY et al.** *Proc. Am. Thorac. Soc.,* 2009, vol. 6, 201-205
- **ADACHI et al.** *Seizure: Eur. J. Epilepsy,* 2012, vol. 21, 55-60
- **KIM et al.** *J. Korean Soc. Neonatol.,* 2011, vol. 18, 374-378
- **YUN et al.** *Science,* 2009, vol. 325, 1555-1559
- **MEYLAN et al.** *Nature,* 2009, vol. 462, 104-107
- **BARBIE et al.** *Nature,* 2009, vol. 462, 108-112